# EUROPEAN PATENT SPECIFICATION

(11) **EP 1 831 173 B1**
(45) Date of publication and mention of the grant of the patent: **26.01.2011**
(21) Application number: 05854585.6
(22) Date of filing: 15.12.2005
(51) Int. Cl.: C07D 217/16, C07D 401/12, C07D 403/12, C07D 417/12, C07D 217/14, C07D 409/04, C07D 401/04, C07D 413/12, C07D 401/14, C07D 405/12, A61K 31/472, A61K 31/4725, A61P 25/24

(54) **TETRAHYDROISOQUINOLINE COMPOUNDS FOR TREATMENT OF CNS DISORDERS**
TETRAHYDROISOCHINOLINVERBINDUNGEN ZUR BEHANDLUNG VON ZNS-ERKRANKUNGEN
COMPOSES DE TETRAHYDROISOQUINOLINE POUR LE TRAITEMENT DE TROUBLES DU SYSTEME NERVEUX CENTRAL

(30) Priority: 17.12.2004 US 637173 P
(43) Date of publication of application: 12.09.2007
(73) Proprietor: Janssen Pharmaceutica NV, 2340 Beerse (BE)
(72) Inventor: CARRUTHERS, Nicholas, I., Poway, California 92064 (US); GOMEZ, Leslie, A., San Diego, California 92129 (US); JABLONOWSKI, Jill, A., San Diego, California 92129 (US); KEITH, John, M., San Diego, California 92128 (US); LETAVIC, Michael, A., San Diego, California 92130 (US); LY, Kiev, S., San Diego, California 92122 (US); MILLER, Jennifer, M., B., Kenmore, Washington 98028 (US); STOCKING, Emily, M., Encinitas, California 92024 (US); WOLIN, Ronald, L., San Diego, California 92127 (US)
(74) Representative: Warner, James Alexander
(86) International application number: PCT/US2005/045905
(87) International publication number: WO 2006/066197

(56) References cited:
- EP-A- 0 400 319
- WO-A-01/32624
- US-A- 4 113 869
- J.E. RIDINGS ET. AL.: "A Qualitative Assessment of Developmental Toxicity Within a Series of Structurally Related Dopamine Mimetics." TOXICOLOGY, vol. 76, 1992, page 197-207, XP002378678

## Description

### Field of the Invention

There is provided by the present invention compounds that are modulators of the histamine H₃ receptor and the serotonin transporter. More particulafly, there is provided by the present invention tetrahydroisoquinoline compounds to treat disorders and conditions mediated by the histamine H₃ receptor and the serotonin transporter. As a consequence of these activities the compounds of the present invention will have therapeutic utility for the treatment of depression and a range of related disorders.

### Background of the Invention

Depression is a chronic illness with an estimated lifetime prevalence of 17%. The total annual cost of depression in the USA is estimated at $44 billion. As such, it represents a major health problem with a serious pharmacoeconomic impact (Griffiths, R.I. et al. Pharmacoeconomics 1999, 15(5), 495-505). Although the biochemical basis of depression is not completely elucidated, the most commonly accepted hypothesis states that depression occurs when monoaminergic neurotransmission in the brain is impaired. This theory is largely based on the observation that compounds that improve noradrenergic and/or serotoninergic neurotransmission often have beneficial effects in depression. Such an improvement in monoaminergic neurotransmission can be achieved in several ways. The biological effect of noradrenaline is terminated by two mechanisms: reuptake from the synaptic cleft into the neuron via the norepinephrine transporter (NET), and degradation by monoamine oxidase (MAO). For serotonin, reuptake in the neuron via the serotonin transporter (SERT) likewise limits its availability in the synaptic cleft.

Currently, clinical treatment of depression relies mainly on four types of drugs: 1) MAO inhibitors; 2) tricyclic antidepressants (TCA); 3) selective serotonin reuptake inhibitors (SSRI); and 4) other drugs such as reboxetine and venlafaxine. MAOs have long been used as second-line drugs because of their potentially dangerous side effects, and more recently, reversible MAO-A selective inhibitors with improved profiles have been described (Bonnet, U. CNS Drug Rev. 2002, 8(3), 283-308). TCAs such as amitryptiline display complex pharmacological activities. They inhibit reuptake of noradrenaline and serotonin via their respective transporters, but also have affinity at muscarinic and histamine H₁ receptors. Thus, their efficacy in treating depression is counterbalanced by numerous unwanted side effects. The SSRIs, which represent the largest and most successful group of antidepressants, show a higher selectivity for the SERT than for the NET, although the exact affinity ratio varies from drug to drug. This class of drugs is characterized by a milder side-effect profile than the MAO-inhibitors or the TCAs. Other drugs have been described, such as reboxetine, which preferentially targets the NET, and venlafaxine, which has dual activity at the SERT and NET (Olver, J.S. et al. CNS Drugs 2001, 15(12), 941- 954).

Although remarkable progress has been made in the treatment of depression, there remains opportunity for improvement. The delay between start of treatment and subjective improvement is a case in point. Most drugs do not cause an improvement in the Hamilton Rating Scale for Depression until after several weeks of treatment, potentially leaving the patient subject to severe mental anguish during this time. Currently available drugs have a limited response rate and in most clinical trials only about 30% of patients show clinical improvement (Menza, M.A. et al. J. Clin. Psych. 2000, 61 (5), 378-381). Psychiatrists frequently have to evaluate several drugs for individual patients before a satisfactory therapeutic response is observed. Consequently, there is a significant therapeutic need for drugs with a faster onset of action, improved side effect profiles and higher response ratio.

In order to appreciate the rationale for a combined SERT/H₃ antagonist, it is necessary to understand the physiology of the histamine H₃ receptor. This receptor was described in 1983 (Arrang, J.-M. et al. Nature (London) 1983, 302(5911), 832-837) as a presynaptic, auto-inhibitory receptor on histaminergic neurons with a characteristic pharmacology. Activation of the H₃ receptor was shown to decrease the amount of histamine released from the nerve terminals and to inhibit the activity of histidine decarboxylase, the rate-limiting enzyme in the synthesis of histamine. The cloning and characterization of the human H₃ receptor made it possible to explore its pharmacology (Lovenberg, T.W. et al. Molec. Pharmacol. 1999, 55(6), 1101-1107). It is now known that the H₃ receptor is expressed on a variety of neurons and thus, when activated, decreases the release of a number of other neurotransmitters including noradrenaline, dopamine, and acetylcholine (Hill, S.J. et al. Pharmacol. Rev. 1997, 49(3), 253-278). For the purpose of this discussion, we will focus on its known effects on the release of the neurotransmitters involved in depression, noradrenaline and serotonin. Although the serotoninergic cell bodies are found in the dorsal raphe nucleus while the histaminergic cells are located in the tuberomammillary nucleus of the hypothalamus, both systems have extensive projections throughout the brain. In several regions, such as the suprachiasmatic nucleus (Laitinen, K.S.M. et al. Eur. J. Pharmacol. 1995, 285(2), 159-164) and striatum both neurotransmitters are present. It is known that activation of the H₃ receptor leads to a decreased release of serotonin, for instance in rat cortex slices (Fink, K. et al. Naunyn-Schmiedeberg's Arch. Pharmacol. 1990, 342(5), 513-519; Schlicker, E. et al. Naunyn-Schmiedeberg's Arch. Pharmacol. 1988, 337(5), 588-590). Functional antagonists of the H₃ receptor lead to an increased release of noradrenaline in the central (mouse cortex slices, Leurs, R. et al. J. Pharmacol. Exp. Ther. 1996, 276(3), 1009-1015; the rat hippocampus, Alvez-Rodrigues, A. et al. Brain Res. 1998, 788(1-2), 179-186) and peripheral nervous system (human myocardial nerves, Hatta, E. et al. J. Pharmacol. Exp. Ther. 1997, 283(2), 494-500; guinea-pig intestinal sympathetic nerves, Blandizzi, C. et al. Br. J. Pharmacol. 2000, 129(7), 1387-1396). However, there is little evidence that H₃ receptor antagonists alone are capable of increasing serotonin levels *in vivo* to those required for antidepressant effects. Microdialysis studies of the effect of H₃ antagonists on serotonin levels in the brain of live animals are lacking. There are sparse reports indicating that thioperamide, an H₃ receptor antagonist, may have an antidepressant effect *per se* in the mouse or rat forced swim test (Lamberti, C. et al. Br. J. Pharmacol. 1998, 123(7), 1331-1336; Perez-Garcia, C. et al. Psychopharmacology 1999, 142(2), 215-220).

The rationale for combining H₃ receptor blockade and SERT activity in one single molecule is the expectation that both mechanisms will contribute synergistically to enhanced concentrations of serotonin in the synaptic cleft. Antagonism at the H₃ receptor will provide increased release of serotonin-containing vesicles into the synaptic cleft, and concomitant blockade of the SERT will decrease the neuronal reuptake of these neurotransmitter molecules. Thus, higher concentrations of serotonin will be achieved, leading to an enhanced therapeutic effect.

Among the prominent vegetative symptoms of depression are disturbed sleep and the daytime fatigue associated with it. Polysomnographic investigations have shown severe disturbances in the sleep architecture of depressed patients. Among the typical abnormalities observed are: discontinuous sleep, decreased slow-wave sleep, shorter latency to REM sleep and an increased intensity and duration of REM sleep (Riemann, D. et al. Neuropsychobiology 2002, 45(Suppl. 1), 7-12). It is believed that suppression of REM sleep is involved in antidepressant efficacy. This is illustrated by the dramatic success of overnight deprivation of (REM) sleep (Riemann et al. 2002). Another non-pharmacological treatment for depression, electroconvulsant therapy, likewise decreases REM sleep. Virtually all of the available antidepressant drugs, regardless of their neurochemical mechanism of action, suppress REM sleep, nefazodone (a 5-HT_{2A} antagonist) being the exception (Sharpley, A.L., Cowen, P.J. Biol. Psych. 1995, 37(2), 85-98). Antidepressant drugs also affect slow-wave-sleep, although in a less clear manner: H₃ antagonists share this REM-sleep suppressing property and one of the main biological effects of histamine H₃ antagonists is to improve wakefulness. Administration of H₃ antagonists has been shown to decrease REM and non-REM sleep in several animal species. For example, the H₃ antagonist carboperamide induces waking in rats (Monti, J.M. et al. Neuropsychopharmacology 1996, 15(1), 31-35). Another H₃ antagonist, thioperamide, decreased both REM and non-REM sleep in rats (Monti, J.M. et al. Eur. J. Pharmacol. 1991, 205(3), 283-287) and cats (Lin, J.-S. et al. Brain Res. 1990, 523(2), 325-330). It is of interest to note that although H₃ antagonists promote wakefulness, they do so much less patently than amphetamine derivatives. They may thus be considered mild stimulants. The daytime correlate of disturbed sleep is fatigue. Indeed, fatigue and lethargy are prominent symptoms of depression, and there is considerable interest in the use of stimulants to augment antidepressant therapy (Menza et al., 2000). However, most of the available stimulants, like the amphetamine derivatives and methylphenidate; carry a considerable risk of abuse and are not ideal therapeutic choices. Modafinil, a wake-promoting compound of unknown mechanism with a lower addictive potential, is marketed for the treatment of narcolepsy. In a small series of patients it was shown that addition of a low dose of modafinil to traditional antidepressant therapy resulted in a faster onset of action. Fatigue was particularly responsive to this therapy, but the cognitive and physical subscales of the Hamilton Rating Scale for Depression also improved (Menza et al., 2000). The behavioral profile of H₃ antagonists (suppression of sleep with no stimulation of locomotor activity and limited addictive potential) is much like that of modafinil. Therefore, a combined H₃/SERT modulating compound would provide symptomatic relief for the fatigue during the first weeks of treatment, before the mood-elevating effect of the SERT modulator can be noticed.

Depression is also associated with a number of cognitive symptoms such as impaired memory and concentration difficulties. H₃ antagonists have been shown to improve memory in a variety of memory tests, including the elevated plus maze in mice (Miyazaki, S. et al. Life Sci. 1995, 57(23), 2137-2144), a two-trial place recognition task (Orsetti, M. et al. Behav. Brain Res. 2001, 124(2), 235-242), the passive avoidance test in mice (Miyazaki, S. et al. Meth. Find. Exp. Clin. Pharmacol. 1995, 17(10), 653-658) and the radial maze in rats (Chen, Z. Acta Pharmacol. Sin. 2000, 21(10), 905-910). Also, in the spontaneously hypertensive rat, an animal model for the learning impairments in attention-deficit disorders, H₃ antagonists were shown to improve memory (Fox, G.B. et al. Behav. Brain Res. 2002, 131(1-2), 151-161). Although no human studies are available, the evidence indicates that a combined SERT/H₃ modulator will provide additional benefit in combating the cognitive impairments associated with depression.

Compounds that have H₃ preceptor activity and SERT activity have been disclosed in U.S. Patent Appl. No. 2007-0065125 (June 17, 2005) and U.S. Patent Appl. No. 2006-0287292 (June 17, 2005), which are both hereby incorporated by reference.

Tetrahydroisoquinoline compounds have been described for various uses in U.S. Patent No. 4,113,869 (Sept. 12, 1978), Eur. Patent Appl. No. EP1113007 (July 4, 2001), and Intl. Patent Appl. No. W0200132624 (May 10, 2001).

In summary, the combination of H₃ receptor antagonism with SERT activity will result in the production of drugs with an improved antidepressant profile compared to a SERT modulator alone. These drugs will be especially efficacious in ameliorating the symptoms of fatigue, disturbed sleep and memory loss associated with depression.

### Summary of the Invention

The invention features a compound of Formula (I): 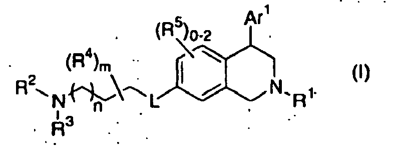 wherein :
L is -0- and n is 1 or 2; or L is -C≡C- or -CH₂CH₂- and n is 0 or 1;
R¹ is -H; or is -C₁₋₆alkyl, -C₃₋₆alkenyl, -C₃₋₆alkynyl, -C₃₋₇cycloalkyl, -C₁₋₆alkylC₃₋₇cycloalkyl, -COOC₁₋₆alkyl, or -COObenzyl, each optionally mono-, di-, or tri-substituted with R^{a};
   where R^{a} is selected from -OH, -OC₁₋₆alkyl, phenyl optionally substituted with -OC₁₋₄alkyl or halo, -CN, -NO₂, -N(R^{b})R^{c}, -C(O)N(R^{b})R^{c}, -N(R^{b})C(O)R^{b}, -N(R^{b})SO₂C₁₋₆alkyl, -C(O)C₁₋₆alkyl, -S(O)₀₋₂-C₁₋₆alkyl, -SO₂N(R^{b})R^{c}, -SCF₃, halo, -CF₃, -OCF₃, -COOH; and -COOC₁₋₆alkyl; wherein R^{b} and R^{c} are each independently -H or -C₁₋₆alkyl;
R² and R³ are each independently selected from the group consisting of: -H;
   A) -C₁₋₆alkyl, -C₃₋₆alkenyl, -C₃₋₆alkynyl, -C₃₋₇cycloalkyl, -C₁₋₆alkylC₃₋₇cycloalkyl, -CH(C₃₋₈cycloalkyl)₂, -CH(phenyl)₂, benzyl, and -C(O)OC₁₋₄alkyl, wherein each alkyl, cycloalkyl, or benzyl is optionally substituted with -OH, -OC₁₋₄alkyl, -CN, -NH₂, -NH(C₁₋₄alkyl), -N(C₁₋₄alkyl)₂, halo, -CF₃, -OCF₃, -COOH, or -COOC₁₋₆alkyl;
   B) phenyl or pyridyl, optionally fused at two adjacent carbon ring members to a three- or four-membered hydrocarbon moiety to form a fused five- or six-membered aromatic ring, which moiety has one carbon atom replaced by >O, >S, >NH, >N(C₁₋₄alkyl), or >NC(O)OC₁₋₄alkyl, and which moiety has up to one additional carbon atom optionally replaced by -N=;
   C) naphthyl,
   D) a 4-8 membered heterocyclic ring, said heterocyclic ring having a carbon atom which is the point of attachment, having 1 or 2 heteroatom members selected from the group consisting of >O, >S(O)₀₋₂, >NH, >N(C₁₋₄alkyl), and >NC(O)OC₁₋₄alkyl, and having 0 or 1 double bonds; and
   E) a monocyclic aromatic hydrocarbon group having five or six ring atoms, having a carbon atom which is the point of attachment, having one carbon atom replaced by >O, >S, >NH, >N(C₁₋₄alkyl), or >NC(O)OC₁₋₄alkyl, having up to one additional carbon atom optionally replaced by -N=, and optionally benzofused or pyridofused;
   where each of B)-E) are optionally mono-, di-, or tri-substituted with a moiety selected from the group consisting of -C₁₋₄alkyl, -OH, -C₁₋₄alkylOH, -OC₁₋₆alkyl, -CN, -NO₂, -N(R^{d})R^{e} -C(O)N(R^{d})R^{e}, -N(R^{d})C(O)R^{d}, -N(R^{d})SO₂C₁₋₆alkyl, -C(O)C₁₋₆alkyl, -S(O)₀₋₂-C₁₋₆alkyl, -SO₂N(R^{d})R^{e}, -SCF₃, halo, -CF₃, -OCF₃, -COOH, -COOC₁₋₆alkyl, -OC(O)N(R^{d})R^{e}, and -OC(O)OC₁₋₆alkyl;
   where R^{d} and R^{e} are each independently -H or -C₁₋₆alkyl;
or, alternatively,
R² and R³ may be taken together with the nitrogen to which they are attached to form tetrahydro-pyrimidin-2-ylidenyl, or a 4-8 membered heterocyclic ring, said heterocyclic ring having 0 or 1 additional heteroatom members separated from the nitrogen of attachment by at least one carbon member and selected from the group consisting of >O, >S(O)₀₋₂, >NH, and >NR^{f}, having 0 or 1 double bonds, having 0, 1 or 2 carbon members separated from the nitrogen of attachment by at least one carbon member which is a carbonyl, optionally benzo or pyrido fused, optionally having one carbon member that forms a bridge, and having 0-5 carbon member substituents R",
   where R^{f} is selected from the group consisting of:
   i) -C₁₋₆alkyl optionally substituted with R^{z}, -C₃₋₆alkenyl, -C₃₋₆alkynyl, -C(O)N(R^{g})R^{h}, -C(O)Rⁱ, -S(O)₀₋₂-C₁₋₆alkyl, and -COOC₁₋₆alkyl, where R^{z} is fluoro, -CN, -OH, -OC₁₋₄alkyl, -C₃₋₇cycloalkyl, or -CF₃; where R^{g} and R^{h} are each independently -H or -C₁₋₆alkyl; and where Rⁱ is -C₁₋₆alkyl, -C₃₋₈cycloalkyl, phenyl, or 5- or 6-membered aromatic heterocyclyl, where each alkyl, cycloalkyl, phenyl or heterocyclyl is optionally mono-, di-, or tri-substituted with -C₁₋₄alkyl, -OH, -OC₁₋₆alkyl, -CF₃, -CN, or halo;
   ii) -(CH₂)₀₋₁-RingA, where Ring A is phenyl or a 5- or 6-membered carbon-linked aromatic heterocyclyl, optionally substituted with R^{aa};
      where R^{aa} is -OH, -C₁₋₆alkyl, -OC₁₋₆alkyl, phenyl, -CN, -NO₂, -N(R^{g})R^{h} -C(O)N(R^{g})R^{h}, -N(R^{g})C(O)R^{h}, -N(R^{h})SO₂C₁₋₆alkyl, -C(O)C₁₋₆alkyl, -S(O)₀₋₂-C₁₋₆alkyl, -SO₂N(R^{g})R^{h}, -SCF₃, halo, -CF₃, -OCF₃, -OCHF₂, -COOH, and -COOC₁₋₆alkyl; and
   iii) -(CH₂)₀₋₁-RingB, where Ring B is -C₃₋₇cycloalkyl with one or two carbon ring members optionally replaced with >O, or >NH, and optionally substituted with -C₁₋₄alkyl, fluoro, -CN, -OH, -OC₁₋₄alkyl, or -CF₃;
   where R^{ff} is selected from the group consisting of -C₁₋₆alkyl optionally mono-or di-substituted with R^{z}, -C₂₋₆alkenyl, -C₂₋₆alkynyl, -C₃₋₇cycloalkyl, -CH(phenyl)₂, halo, -OH, -OC₁₋₆alkyl, -OC₂₋₃alkylO-, -CN, -NO₂, -N(R^{g})R^{h}, -C(O)N(R^{g})R^{h}, -N(R^{g})C(O)R^{g}, -N(R^{g})SO₂C₁₋₆alkyl, -C(O)Rⁱ, -S(O)₀₋₂-C₁₋₆alkyl, -SO₂N(R^{g})R^{h}, -SCF₃, -CF₃, -OCF₃, -COOH, and -COOC₁₋₆alkyl;
R⁴ is -OH, -OC₁₋₆alkyl, -CF₃, -C₁₋₆alkyl, or halo; two R⁴ substituents may be taken together to form methylene or ethylene; or one of R⁴ is taken together with R² to form methylene, ethylene, propylene, or -CH₂CH₂O-, wherein each is optionally substituted with -OH, -OC₁₋₆alkyl, -SC₁₋₆alkyl, -CF₃, -C₁₋₆alkyl, amino, or halo;
m is 0, 1, or 2;
R⁵ is selected from the group consisting of -C₁₋₆alkyl, -OH, -OC₁₋₆alkyl, -SC₁₋₆alkyl, and halo;
Ar¹ is an aryl or heteroaryl ring selected from the group consisting of:
   a) phenyl, optionally mono-, di-, or tri-substituted with R^{j} and optionally di-substituted on adjacent carbons with -OC₁₋₄alkyleneO- optionally mono-or di-substituted with fluoro, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
      where R^{j} is selected from the group consisting of
      1) -OH, -C₁₋₆alkyl, -OC₁₋₆alkyl optionally mono-, di-, or tri-substituted with halo, -C₂₋₆alkenyl, -OC₃₋₆alkenyl, -C₂₋₆alkynyl optionally substituted with trimethylsilyl, -OC₃₋₆alkynyl, -C₃₋₆cycloalkyl, -OC₃₋₆cycloalkyl, -CN, -NO₂, -N(R^{k})R^{l}, -N(R^{k})C(O)R^{l}, -N(R^{k})SO₂C₁₋₆alkyl, -C(O)C₁₋₆alkyl, -S(O)₀₋₂-C₁₋₆alkyl, -C(O)N(R^{m})Rⁿ, -SO₂N(R^{m})Rⁿ, -SCF₃, halo, -CF₃, -COOH, -COOC₁₋₆alkyl, and -COOC₃₋₇cycloalkyl;
         where R^{k} and R^{l} are each independently -H or -C₁₋₆alkyl;
         where R^{m} and Rⁿ are each independently -H or -C₁₋₆alkyl, or R^{m} and Rⁿ taken together with their nitrogen of attachment form a 4-8 membered heterocyclic ring having 1 or 2 heteroatom members selected from >O, >S(O)₀₋₂, >NH, and >NC₁₋₆alkyl, having 0 or 1 double bonds, having 0 or 1 carbonyl members;
      2) -G-Ar², where G is a bond, -O-, or -S-, and Ar² is phenyl or is a monocyclic aromatic hydrocarbon group having five or six ring atoms, having one carbon atom replaced by >O, >S, >NH, or >N(C₁₋₄alkyl), having up to one additional carbon atom optionally replaced by -N=, each optionally mono-, di-, or tri-substituted with R^{p};
         where R^{p} is a substituent independently selected from the group consisting of: -OH, -C₁₋₆alkyl, -OC₁₋₆alkyl, phenyl, -CN, -NO₂, -N(R^{q})R^{r}, -C(O)N(R^{q})R^{r}, -N(R^{q})C(O)R^{r}, -N(R^{q})SO₂C₁₋₆alkyl, -C(O)C₁₋₆alkyl, -S(O)₀₋₂-C₁₋₆alkyl, -SO₂N(R^{q})R^{r}, -SCF₃, halo, -CF₃, -OCF₃, -OCHF₂, -COOH, and -COOC₁₋₆alkyl;
         wherein R^{q} and R^{r} are each independently selected from -H, -C₁₋₆alkyl, and -C₂₋₆alkenyl; and
      3) a 4-8 membered saturated or partially saturated heterocyclic ring, having 1 or 2 heteroatom members selected from >O, >S(O)₀₋₂, >NH, and >NC₁₋₆alkyl, having 0 or 1 carbonyl members, said ring optionally mono-, di-, or tri-substituted with R^{p};
   b) phenyl or pyridyl fused at two adjacent carbon ring members to a three membered hydrocarbon moiety to form a fused five membered aromatic ring, which moiety has one carbon atom replaced by >O, >S, >NH, or >N(C₁₋₄alkyl), and which moiety has up to one additional carbon atom optionally replaced by -N=, the fused rings optionally mono-, di-, or tri-substituted with R^{t};
      where R^{t} is a substituent independently selected from the group consisting of: -OH, -C₁₋₆alkyl, -OC₁₋₆alkyl, phenyl, -CN, -NO₂, -N(R^{u})R^{v}, -C(O)N(R^{u})R^{v}, -N(R^{u})C(O)R^{v}, -N(R^{u})SO₂C₁₋₆alkyl, -C(O)C₁₋₆alkyl, -S(O)₀₋₂-C₁₋₆alkyl, -SO₂N(R^{u})R^{v}, -SCF₃, halo, -CF₃, -OCF₃, -OCHF₂, -COOH, and -COOC₁₋₆alkyl;
      where R^{u} and R^{v} are each independently -H or -C₁₋₆alkyl;
   c) phenyl fused at two adjacent ring members to a four membered hydrocarbon moiety to form a fused six membered aromatic ring, which moiety has one or two carbon atoms replaced by -N=, the fused rings optionally mono-, di-, or tri-substituted with R^{t};
   d) naphthyl, optionally mono-, di-, or tri-substituted with R^{t};
   e) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atom optionally replaced by -N=, optionally mono- or di-substituted with R^{j} and optionally benzofused or pyridofused at two adjacent carbon atoms, where the benzofused or pyridofused moiety is optionally mono-, di-, or tri-substituted with R^{t}; and
   f) a monocyclic aromatic hydrocarbon group having six ring atoms, having a carbon atom which is the point of attachment, having one or two carbon atoms replaced by -N=, optionally mono- or di-substituted with R^{j} and optionally benzofused or pyridofused at two adjacent carbon atoms, where the benzofused or pyridofused moiety is optionally mono- or di-substituted with R^{t};
   and enantiomers, diastereomers, hydrates, solvates and pharmaceutically acceptable salts, esters and amides thereof;
   with the following proviso:
   when n is 1, L is -O-, Ar is unsubstituted phenyl, and R² and R³ are both methyl, then R⁴ is not -OH.
   for use in the treatment or prevention of a CNS disorder selected from the group consisting of: sleep/wake and arousal/vigilance disorders, insomnia, jet lag, disturbed sleep, attention deficit hyperactivity disorders (ADHD), attention-deficit disorders, learning and memory disorders, learning impairment, memory impairment, memory loss, cognitive dysfunction, migraine, neurogenic inflammation, dementia, mild cognitive impairment, pre-dementia, Alzheimer's disease, epilepsy, narcolepsy with or without associated cataplexy, cataplexy, disorders of sleep/wake homeostasis, idiopathic somnolence, excessive daytime sleepiness (EDS), circadian rhythym disorders, sleep/fatigue disorders, fatigue, drowsiness associated with sleep apnea, sleep impairment due to perimenopausal hormonal shifts, Parkinson's-related fatigue, MS-related fatigue, depression-related fatigue, chemotherapy-induced fatigue, work-related fatigue, eating disorders, obesity, motion sickness, vertigo, schizophrenia, substance abuse, bipolar disorders, manic disorders, and depression in mammals.

Isomeric forms of the compounds of Formula (I), and of their pharmaceutically acceptable salts, esters, and amides, are encompassed within the present invention, and reference herein to one of such isomeric forms is meant to refer to at least one of such isomeric forms. One of ordinary skill in the art will recognize that compounds according to this invention may exist, for example in a single isomeric form whereas other compounds may exist in the form of a regioisomeric mixture.

The invention also features pharmaceutical compositions containing such compounds and the use of such compounds and compositions in the treatment or prevention of disease states mediated by the histamine H₃ receptor and the serotonin transporter.

Compounds of the present invention are useful in combination with other therapeutic agents as a combination therapy method, including use in combination with H₁ receptor antagonists, H₂ receptor antagonists, H₃ receptor antagonists, and neurotransmitter modulators such as serotonin-norepinephrine reuptake inhibitors, selective serotonin reuptake inhibitors (SSRIs), no-adrenergic reuptake inhibitors, non-selective serotonin re-uptake inhibitors (NSSRIs), and modafinil.

Additional features and advantages of the invention will become apparent from the detailed description and examples below, and the appended claims.

### Detailed Description

Particular preferred compounds of the invention comprise a compound of Formula (I), or an enantiomer, diastereomer, hydrate, solvate thereof, or a pharmaceutically acceptable salt, amide or ester thereof, wherein n, m, R¹⁻⁵, and Ar¹ have any of the meanings defined hereinabove and equivalents thereof, or at least one of the following assignments and equivalents thereof. Such assignments may be used where appropriate with any of the definitions, claims or embodiments defined herein:
Preferably, L is -O- and n is 1.
Preferably, L is -C≡C- and n is 0.
Preferably, L is -CH₂CH₂- and n is 0.
Preferably, R¹ is -C₁₋₄alkyl.

In a preferred embodiment, R¹ is selected from the group consisting of hydrogen, methyl, ethyl, propyl, isopropyl, butyl, t-butyl, pentyl, hexyl, benzyl, vinyl, allyl, propargyl, cyclopropyl, cyclobutyl, cyclopentyl, cyclohexyl, cyclopropylmethyl, -COOCH₃, -COO-t-butyl, and -COObenzyl.

More preferably, R¹ is methyl; ethyl, propyl, t-butyl, allyl, propargyl, or benzyl.

Even more preferably, R¹ is hydrogen or methyl.

Preferably, when R² is methyl, R³ is not methyl.

Preferably, R² and R³ are hydrogen, or, optionally substituted, are independently selected from the group consisting of:
A) methyl, ethyl, propyl, isopropyl, sec-butyl, butyl, pentyl, hexyl, vinyl, allyl, propargyl, cyclopropyl, cyclobutyl, cyclopentyl, cyclohexyl, cyclopropylmethyl, dicyclopropylmethyl, benzhydryl, benzyl, -C(O)O-t-butyl,
B) phenyl, pyridyl, 4-, 5-, 6- or 7-benzoxazolyl, 4-, 5-, 6- or 7-benzothiophenyl, 4-, 5-, 6- or 7-benzofuranyl, 4-, 5-, 6- or 7-indolyl, 4-, 5-, 6- or 7-benzthiazolyl, 4-, 5-, 6- or 7-benzimidazolyl, 4-, 5-, 6- or 7-indazolyl, imidazo[1,2-a]pyridin-5, 6, 7 or 8-yl, pyrazolo[1,5-a]pyridin-4, 5, 6 or 7-yl, 1H-pyrrolo[2,3-b]pyridin-4,5 or 6-yl, 1H-pyrrolo[3,2-c]pyridin-4, 6 or 7-yl, 1H-pyrrolo[2,3-c]pyridin-4, 5 or 7-yl, 1H-pyrrolo[3,2-b]pyridin-5, 6 or 7-yl,
C) naphthyl,
D) azetidinyl, pyrrolidinyl, piperidinyl, tetrahydropyranyl, and
E) furanyl, oxazolyl, isoxazolyl, 1,2,3-oxadiazolyl, 1,2,4-oxadiazolyl, 1,2,5-oxadiazolyl, 1,3,4-oxadiazolyl, thiophenyl, thiazolyl, isothiazolyl, pyrrolyl, imidazolyl; pyrazolyl, 1,2,3-triazolyl, 1,2,4-triazolyl, 3-indoxazinyl, 2-benzoxazolyl, 2- or 3-benzothiophenyl, 2- or 3-benzofuranyl, 2- or 3-indolyl, 2-benzthiazolyl, 2-benzimidazolyl, and 3-indazolyl.

More preferably, R² and R³ are independently selected from the group consisting of methyl, ethyl, propyl, isopropyl, sec-butyl, hydroxyethyl, 2-hydroxy-2-methylpropyl, allyl, cyclopropyl, cyclobutyl, cyclopentyl, dicyclopropylmethyl, pyrrolidinyl, piperidinyl, N-methylpiperidinyl, tetrahydropyranyl; 2-benzothiazolyl, and methoxyethyl.

Even more preferably, R² and R³ are each independently hydrogen, ethyl, isopropyl, methoxyethyl, 2-benzothiazolyl, cyclopropyl, cyclobutyl, or cyclopentyl.

In a preferred embodiment, R² and R³, optionally substituted, are taken together with the nitrogen to which they are attached to form tetrahydro-pyrimidin-2-ylidenyl, or a ring selected from the group consisting of azetidinyl, pyrrolidinyl, piperidinyl, morpholinyl, thiomorpholinyl, piperazinyl, homopiperidinyl, 1,3-dihydro-isoindol-2-yl, 5,6-dihydro-4H-pyrimidin-1-yl, and 1,1-dioxo-1λ⁶-thiomorpholin-4-yl.

Preferably, R^{f}, optionally substituted, is methyl, ethyl, isopropyl, allyl, cyclopropyl, tert-butoxycarbonyl, tetrahydrofuranylmethyl, [1,3]-dioxolan-ylmethyl, thiazolyl, thiophenyl, thiophenylmethyl, pyridinyl, phenyl, acetyl, isobutyryl, cyclopropanecarbonyl, cyclobutanecarbonyl, pyridinyl, pyridine-carbonyl, 1H-pyrrole-carbonyl, and 1H-imidazole-carbonyl.

In an alternative embodiment, R² and R³ are taken together with the nitrogen to which they are attached to form a 4-8 membered heterocyclic ring, said heterocyclic ring selected from piperidine, pyrrolidine, and morpholine, said ring substituted with 1 or 2 substituents R^{ff}.

Preferably, R^{ff} is selected from the group consisting of methyl, ethyl, isopropyl, butyl, hexyl, -CHF₂, benzhydryl, -CF₃, vinyl, allyl, propargyl, cyclopropyl, cyclopentyl, cyclopropylmethyl, cyclobutylethyl, bromo, chloro, fluoro, iodo, -OH, hydroxymethyl, hydroxyethyl, methoxy, ethoxy, isopropoxy, pentyloxy, -O(CH₂)₂O-, -O(CH₂)₃O-, -CN, amino, methylamino, dimethylamino, diethylamino, diethylcarbamoyl, methanesulfanyl, methanesulfonyl, methanesulfonamido, -C(O)Rⁱ, -COOH, and ethoxycarbonyl.

More preferably, R^{ff} is selected from the group consisting of methyl, fluoro, -OH, -CF₃, hydroxymethyl, hydroxyethyl, benzhydryl, dimethylamino, ethoxycarbonyl, cyano, and -O(CH₂)₂O-.

Preferably, Rⁱ is selected from the group consisting of methyl, pyridyl, isopropyl, cyclobutyl, cyclopropyl, N-methylpyrrolyl, and 1-methylimidazolyl.

More preferably, R² and R³ are taken together with the nitrogen to which they are attached to form azetidinyl, 3,3-difluoroazetidinyl, 3-benzhydryl-azetidinyl, 2-methylpyrrolidinyl, 3-hydroxypyrrolidinyl, 3-dimethylaminopyrrolidinyl, 2,5-dimethylpyrrolidinyl, 2-trifluoromethylpyrrolidinyl, 2-hydroxymethylpyrrolidinyl, 3,3-difluoropyrrolidinyl, piperidinyl, 4-fluoropiperidinyl, 3-fluoropiperidinyl, 3,3-difluoropiperidinyl, 4,4-difluoropiperidinyl, 3-trifluoromethylpiperidinyl, 4-trifluoromethylpiperidinyl, 1,4-dioxa-8-aza-spiro[4.5]dec-8-yl, 4-cyanopiperidinyl, 4-carboethoxypiperidinyl, 3-hydroxypiperidinyl, 4-hydroxypiperidinyl, 2-hydroxymethylpiperidinyl, 3-hydroxymethylpiperidinyl, 4-hydroxymethylpiperidinyl, 3-hydroxyethylpiperidinyl, 4-hydroxyethylpiperidinyl, morpholinyl, 2-methylmorpholin-4-yl, 3-methylmorpholin-4-yl, 3-hydroxymethylmorpholin-4-yl, 2-hydroxymethylmorpholin-4-yl, 4-methyl-piperazin-1-yl, 4-ethyl-piperazin-1-yl, 4-isopropyl-piperazin-yl, 4-allyl-piperazin-1-yl, 4-cyclopropyl-piperazin-1-yl, 4-(2-hydroxyethyl)piperazin-1-yl, 4-(2-methoxyethyl)-piperazin-1-yl, 4-(tert-butoxycarbonyl)piperazin-1-yl, 4-(tetrahydrofuran-2-ylmethyl)piperazin-1-yl, 4-[1,3]-dioxolan-2-ylmethyl-piperazin-1-yl, 4-thiazol-2-yl-piperazin-1-yl, 4-(2-thiophenyl)piperazinyl, 4-thiophen-2-ylmethyl-piperazin-1-yl, 4-(pyridin-4-yl-)-piperazin-1-yl, 4-phenylpiperazin-1-yl, 4-(2-hydroxyphenyl)piperazinyl, 4-(4-trifluoromethyl-phenyl)-piperazin-1-yl, 4-(4-cyanophenyl)piperazin-1-yl, 4-acetytpiperazin-1-yl, 4-isobutyryl-piperazin-1-yl, 4-cyclopropanecarbonyl-piperazin-1-yl), 4-cyclobutanecarbonyl-piperazin-1-yl, 4-pyridin-4-yl-piperazin-1-yl, 4-(pyridine-4-carbonyl)-piperazin-1-yl, 4-(1-methyl-1H-pyrrote-2-carbonyl)-piperazin-1-yl, 4-(1-methyl-1H-imidazole-4-carbonyl)-piperazin-1-yl, 1,1-dioxo-1λ⁶-thiomorpholin-4-yl, 2,6-dimethylmorpholin-4-yl, and 1,3-dihydro-isoindol-2-yl, 5,6-dihydro-4H-pyrimidin-1-yl.

Even more preferably, R² and R³ are taken together with the nitrogen to which they are attached to form piperidinyl, 4-fluoropiperidinyl, 4,4-difluoropiperidinyl, morpholinyl, or 3-methylmorpholin-4-yl.

Preferably, R⁴ is hydroxy, methoxy, ethoxy, isopropoxy, pentyloxy, -CF₃, methyl, ethyl, propyl, isobutyl, pentyl, chloro, or fluoro.

More preferably, R⁴ is hydroxy, methyl, methoxy, fluoro, or -CF₃.

Preferably, two R⁴ are taken together to form methylene.

Preferably, R² and one of R⁴ are taken together to form methylene, ethylene, or -CH₂CH₂O.

Preferably, m is 0 or 1.

Preferably, R⁵ is methyl, ethyl, isopropyl, hexyl, hydroxy, methoxy, ethoxy, isopropoxy, methylsulfanyl, bromo, chloro, fluoro, or iodo.

More preferably, R⁵ is methyl, hydroxy, or fluoro.

Preferably, Ar¹, optionally substituted, is selected from the group consisting of:
a) phenyl, 5-, 6-, 7-, 8-benzo-1,4-dioxanyl, 4-, 5-, 6-, 7-benzo-1,3-dioxolyl, 4-, 5-, 6-, 7-indolinyl, 4-, 5-, 6-, 7-isoindolinyl, 1,2,3,4-tetrahydro-quinolin-4, 5, 6 or 7-yl, 1,2,3,4-tetrahydro-isoquinolin-4, 5, 6 or 7-yl,
b) 4-, 5-, 6- or 7-benzoxazolyl, 4-, 5-, 6- or 7-benzothiophenyl, 4-, 5-, 6- or 7-benzofuranyl, 4-, 5-, 6- or 7-indolyl, 4-, 5-, 6- or 7-benzthiazolyl, 4-, 5-, 6- or 7-benzimidazolyl, 4-, 5-, 6- or 7-indazolyl, imidazo[1,2-a]pyridin-5, 6, 7 or 8-yl, pyrazolo[1,5-a]pyridin-4, 5, 6 or 7-yl, 1H-pyrrolo[2,3-b]pyridin-4, 5 or 6-yl, 1H-pyrrolo[3,2-c]pyridin-4, 6 or 7-yl, 1H-pyrrolo[2,3-c]pyridin-4, 5 or 7-yl, 1H-pyrrolo[3,2-b]pyridin-5, 6 or 7-yl,
c) 5-, 6-, 7- or 8-isoquinolinyl, 5-, 6-, 7- or 8-quinolinyl, 5-, 6-, 7- or 8-quinoxalinyl, 5-, 6-, 7- or 8-quinazolinyl,
d) naphthyl,
e) furanyl, oxazolyl, isoxazolyl, 1,2,3-oxadiazolyl, 1,2,4-oxadiazolyl, 1,2,5-oxadiazolyl, 1 ,3,4-oxadiazolyl, thiophenyl, thiazolyl, isothiazolyl, pyrrolyl, imidazolyl, pyrazolyl, 1,2,3-triazolyl, 1,2,4-triazolyl, 3-indoxazinyl, 2-benzoxazolyl, 2- or 3-benzothiophenyl, 2- or 3-benzofuranyl, 2- or 3-indolyl, 2-berizthiazolyl, 2-benzimidazolyl, 3-indazolyl, and
f) pyridinyl, pyridinyl-N-oxide, pyrazinyl, pyrimidinyl, pyridazinyl, 1-, 3- or 4-isoquinolinyl, 2-, 3- or 4-quinolinyl, 2- or 3-quinoxaliriyl, 2- or 4-quinazolinyl, [1,5], [1,6], [1,7], or [1,8]naphthyridin-2-, 3-, or 4-yl, [2,5], [2,6], [2,7], [2,8]naphthyridin-1-3-, or 4-yl.

More preferably, Ar¹, optionally substituted, is selected from the group consisting of phenyl, pyridyl, pyrazinyl, thiazolyl, pyrazolyl, and thiophenyl.

Preferably, G is a-bond or -S-.

Preferably, Ar² is selected from the group consisting of phenyl, pyrrolyl, furanyl, thiophenyl, pyrazolyl, imidazolyl, oxazolyl, thiazolyl, isoxazolyl, triazolyl, tetrazolyl, pyridinyl, pyrimidinyl, or pyrazinyl.

Even more preferably, Ar¹ is selected from the group consisting of phenyl, 2-methoxyphenyl, 3-methoxyphenyl, 4-methoxyphenyl, 4-ethoxyphenyl, 2,4-dimethoxyphenyl, 2,5-dimethoxyphenyl, 3,4-dimethoxyphenyl, 3,5-dimethoxyphenyl, 3,4,5-trimethoxyphenyl, 2-methylphenyl, 3-methylphenyl, 4-methylphenyl, 4-ethylphenyl, 3-ethynylphenyl, 4-ethynylphenyl, 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 3-iodophenyl, 4-iodophenyl, 2-trifluoromethylphenyl, 3-trifluoromethylphenyl, 4-trifluoromethylphenyl, 3-trifluoromethoxyphenyl, 4-trifluoromethoxyphenyl, 4-difluoromethoxyphenyl, 3-cyanophenyl, 4-cyanophenyl, 3-acetylphenyl, 4-acetylphenyl, 3,4-difluorophenyl, 3,4-dichlorophenyl, 2,3-difluorophenyl, 2,3-dichlorophenyl, 2,4-difluorophenyl, 2,4-dichlorophenyl, 2,5-dichlorophenyl, 3,5-dichlorophenyl, 3-nitrophenyl, 4-nitrophenyl, 3-chloro-4-fluorophenyl, 3-chloro-4-methoxyphenyl, 3-chloro-4-difluoromethoxyphenyl, 3-fluoro-4-chlorophenyl, 2-fluoro-4-methoxyphenyl, 3-fluoro-4-methoxyphenyl, benzo[1,3]dioxol-4 or 5-yl, 2-hydroxyphenyl, 3-hydroxyphenyl, 4-hydroxyphenyl, 4-hydroxy-2-methylphenyl, 4-hydroxy-3-fluorophenyl, 3,4-dihydroxyphenyl, 4-aminophenyl, 4-dimethylaminophenyl, 4-morpholin-4-yl-phenyl, 4-carbamoylphenyl, 4-fluoro-3-methylphenyl, 4-methanesulfanylphenyl, 4-methanesulfinylphenyl, 4-methanesulfonylphenyl, 4-trifluoromethanesulfanylphenyl, thiophen-2-yl, thiophen-3-yl, 2-pyridinyl, 3-pyridinyl, 4-pyridinyl, 2-chloro-5-pyridinyl, 2-dimethylamino-5-pyridinyl, 6-methoxy-pyridin-3-yl, 6-methylsulfanyl-pyridin-3-yl, 2-hydroxy-5-pyridinyl, 6-pyrazol-1-yl-pyridin-3-yl, 6-bromo-pyridin-3-yl, 6-ethynyl-pyridin-3-yl, 6-trimethylsilanylethynyl-pyridin-3-yl, 6-phenylsulfanyl-pyridin-3-yl, 6-imidazol-1-yl-pyridin-3-yl, 6-(1H-imidazol-2-ylsulfanyl)-pyridin-3-yl, 6-(pyrimidin-2-ylsulfanyl)-pyridin-3-yl, oxazol-5-yl, thiazol-5-yl, thiazol-2-yl, 2H-pyrazol-3-yl, pyrazin-2-yl, 1-naphthyl, 2-naphthyl, 4-imidazol-1-ylphenyl, 4-pyrazol-1-ylphenyl, 1H-indol-5-yl, 1H-benzimidazol-5-yl, benzo[b]thiophen-7-yl, and 4-biphenyl.

In a particular embodiment, Ar¹, optionally substituted with halo, is 4-methoxyphenyl, 4-methanesulfanylphenyl, or 4-chlorophenyl.

Preferably, when (a) n is 1, (b) L is -O-, (c) Ar is unsubstituted phenyl or phenyl substituted with fluoro, chloro, nitro, trifluoromethyl, methyl, or methoxy, and (d) R² and R³ are hydrogen, methyl, or ethyl, then R⁴ is not -OH.

Alternatively, when (a) n is 1, (b) L is -O-, (c) Ar is optionally substituted phenyl, and (d) R² and R³ are hydrogen, methyl, or ethyl, then R⁴ is not -OH.

Alternatively, R⁴ is not -OH.

Compounds of the invention also include compounds of Formula (II): 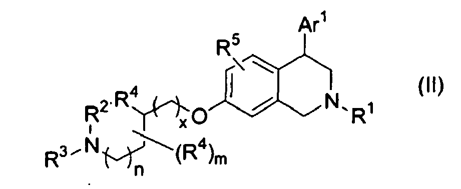 wherein
n, m, R¹, R⁵, and Ar¹ are defined as for Formula (I);
x is 0 or 1;
where n + x is 1 or 2;
R³ is hydrogen, or is selected from the group consisting of:
   A) -C₁₋₆alkyl, -C₃₋₆alkenyl, -C₃₋₆alkynyl, -C₃₋₇Cycloalkyl, -C₁₋₆alkylC₃₋₇cycloalkyl, -CH(C₃₋₈cycloalkyl)₂, -CH(phenyl)₂, benzyl, and -C(O)OC₁₋₄alkyl, wherein each alkyl, cycloalkyl, or benzyl is optionally substituted with -OH,-OC₁₋₄alkyl, -CN, -NH₂, -NH(C₁₋₄alkyl), -N(C₁₋₄alkyl)₂, halo, -CF₃, -OCF₃, -COOH, or -COOC₁₋₆alkyl;
   B) phenyl or pyridyl, optionally fused at two adjacent carbon ring members to a three- or four-membered hydrocarbon moiety to form a fused five- or six-membered aromatic ring, which moiety has one carbon atom replaced by >O, >S, >NH, >N(C₁₋₄alkyl), or >NC(O)OC₁₋₄alkyl, and which moiety has up to one additional carbon atom optionally replaced by -N=;
   C) naphthyl,
   D) a 4-8 membered heterocyclic ring, said heterocyclic ring, having a carbon atom which is the point of attachment, having 1 or 2 heteroatom members selected from the group consisting of >O, >S(O)₀₋₂, >NH_{;} >N(C₁₋₄alkyl), and >NC(O)OC₁₋₄alkyl, and having 0 or 1 double bonds; and
   E) a monocyclic aromatic hydrocarbon group having five or six ring atoms, having a carbon atom which is the point of attachment, having one carbon atom replaced by >O, >S, >NH, >N(C₁₋₄alkyl), or >NC(O)OC₁₋₄alkyl, having up to one additional carbon atom optionally replaced by -N=, and optionally benzofused or pyridofused;
where each of B)-E) are optionally mono-, di-, or tri-substituted with a moiety selected from the group consisting of -C₁₋₄alkyl, -OH, -C₁₋₄alkylOH, -OC₁₋₆alkyl, -CN, -NO₂, -N(R^{d})R^{e}, -C(O)N(R^{d})R^{e}, -N(R^{d})C(O)R^{d}, -N(R^{d})SO₂C₁₋₆alkyl, -C(O)C₁₋₆alkyl, -S(O)₀₋₂-C₁₋₆alkyl, -SO₂N(R^{d})R^{e}, -SCF₃, halo, -CF₃, -OCF₃, -COOH, -COOC₁₋₆alkyl, -OC(O)N(R^{d})R^{e}, and -OC(O)OC₁₋₆alkyl;
where R^{d} and R^{e} are each independently -H or -C₁₋₆alkyl;
-R²-R⁴- is methylene, ethylene, propylene, or -CH₂CH₂O-, wherein each is optionally substituted with -OH, -OC₁₋₆alkyl, -SC₁₋₆alkyl, -CF₃, -C₁₋₆alkyl, amino, or halo;
R⁴ is -OH, -OC₁₋₆alkyl, -CF₃, -C₁₋₆alkyl, or halo,
and enantiomers, diastereomers, hydrates, solvates and pharmaceutically acceptable salts, esters and amides thereof.

Preferably, with regard to Formula (II), n is 2 and x is 0. More preferably, n is 1 and x is 0. Even more preferably, n is 1 and x is 1.

It is understood that some compounds referred to herein are chiral and/or have geometric isomeric centers, for example *E-* and *Z*- isomers. The present invention encompasses all such optical, including stereoisomers and racemic mixtures, diastereomers, and geometric isomers that possess the activity that characterizes the compounds of this invention. Compounds of the invention may exist as single enantiomers, mixtures of enantiomers, or racemic mixtures. In certain embodiments, the absolute configuration of a single enantiomers may be unknown. In addition, certain compounds referred to herein can exist in solvated as well as unsolvated forms. It is understood that this invention encompasses all such solvated and unsolvated forms that possess the activity that characterizes the compounds of this invention.

Compounds according to the present invention that have been modified to be detectable by some analytic technique are also within the scope of this invention. The compounds of the present invention may be labeled with radioactive elements such as ¹²⁵I, ¹⁸F, ¹¹C, ⁶⁴Cu, and the like for use in imaging or for radioactive treatment of patients. An example of such compounds is an isotopically labeled compound, such as an ¹⁸F isotopically labeled compound that may be used as a probe in detection and/or imaging techniques, such as positron emission tomography (PET) and single-photon emission computed tomography (SPECT). Preferably, compounds of the present invention labeled with ¹⁸F or ¹¹C may be used as a positron emission tomography (PET) molecular probe for studying disorders mediated by the histamine H₃ receptor and the serotonin transporter. Another example of such compounds is an isotopically labeled compound, such as a deuterium and/or tritium labeled compound that maybe used in reaction kinetic studies. Alternatively, ³H or ¹⁴C-labelled compounds may be useful in biodistribution studies. The compounds described herein may be reacted with an appropriate functionalized radioactive reagents using conventional chemistry to provide radiolabeled compound.

The present disclosure includes within its scope prodrugs of the compound of this invention. In general, such prodrugs will be functional derivatives of the compounds that are readily convertible *in vivo* into the required compound. Thus, in the methods of treatment of the present invention, the term "administering" shall encompass the treatment of the various disorders described with a compound of Formula (I) or (II) or with a compound that converts to a compound of Formula (I) or (II) *in vivo* after administration to the patient. Conventional procedures for the selection and preparation of suitable prodrug derivatives are described, for example, in "Design of Prodrugs", ed. H. Bundgaard, Elsevier, 1985. In addition to salts, the invention provides the esters, amides, and other protected or derivatized forms of the described compounds.

Preferred compounds, which are tetrahydroisoquinoline compounds, are selected from the group consisting of:

| **EX** | **CHEMICAL NAME** |
|---|---|
| **1** | 7-[3-(4-Fluoro-piperidin-1-yl)-propoxy]-4-(4-methoxy-phenyl)-2-methyl-1,2,3,4-tetrahydro-isoquinoline; |
| **1A** | 7-[3-(4-Fluoro-piperidin-1-yl)-propoxy]-4-(4-methoxy-phenyl)-2-methyl-1,2,3,4-tetrahydro-isoquinoline (enantiomer 1); |
| **1B** | 7-[3-(4-Fluoro-piperidin-1-yl)-propoxy]-4-(4-methoxy-phenyl)-2-methyl-1,2,3,4-tetrahydro-isoquinoline (enantiomer 2); |
| **2** | 1-(4-{3-[4-(4-Methoxy-phenyl)-2-methyl-1,2,3,4-tetrahydro-isoquinolin-7-yloxy]-propyl}-piperazin-1-yl)-ethanone; |
| **3** | Diethyl-{3-[4-(4-methoxy-phenyl)-2-methyl-1,2,3,4-tetrahydro-isoquinolin-7-yloxy]-propyl}-amine; |
| **4** | (4-{3-[4-(4-Methoxy-phenyl)-2-methyl-1,2,3,4-tetrahydro-isoquinolin-7-yloxy]-propyl}-piperazin1-yl)-pyridin-4-yl-methanone; |
| **5** | 1-(4-{3-[4-(4-Methoxy-phenyl)-2-methyl-1,2,3,4-tetrahydro-isoquinolin-7-yloxy]-propyl}-piperazin-1-yl)-2-methyl-propan-1-one; |
| **6** | Cyclobutyl-(4-{3-[4-(4-methoxy-phenyl)-2-methyl-1,2,3,4-tetrahydro-isoquinolin-7-yloxy]-propyl}-piperazin-1-yl)-methanone; |
| **7** | Cyclopropyl-(4-{3-[4-(4-methoxy-phenyl)-2-methyl-1,2,3,4-tetrahydro-isoquinolin-7-yloxy]-propyl}-piperazin-1-yl)-methanone; |
| **8** | 7-[3-(4,4-Difluoro-piperidin-1-yl)-propoxy]-4-(4-methoxy-phenyl)-2-methyl-1,2,3,4-tetrahydro-isoquinoline; |
| **9** | 4-(4-Methoxy-phenyl)-2-methyl-7-(3-morpholin-4-yl-propoxy)-1,2,3,4-tetrahydro-isoquinoline; |
| **10** | (1-{3-[4-(4-Methoxy-phenyl)-2-methyl-1,2,3,4-tetrahydro-isoquinolin-7-yloxy]-propyl}-pyrrolidin-3-yl)-dimethyl-amine; |
| **11** | 7-[3-((2R,5R)-trans-Dimethyl-pyrrolidin-1-yl)-propoxy]-4-(4-methoxy-phenyl)-2-methyl-1,2,3,4-tetrahydro-isoquinoline; |
| **12** | 4-{3-[4-(4-Methoxy-phenyl)-2-methyl-1,2,3,4-tetrahydro-isoquinolin-7-yloxy]-propyl}-piperazine-1-carboxylic acid ethyl ester; |
| **13** | (4-{3-[4-(4-Methoxy-phenyl)-2-methyl-1,2,3,4-tetrahydro-isoquinolin-7-yloxy]-propyl}-piperazin-1-yl)-(1-methyl-1H-pyrrol-2-yl)-methanone; |
| **14** | (4-{3-[4-(4-Methoxy-phenyl)-2-methyl-1,2,3,4-tetrahydro-isoquinolin-7-yloxy]-propyl}-piperazin-1-yl)-(1-methyl-1H-imidazol-4-yl)-methanone; |
| **15** | (1,3-Dimethyl-tetrahydro-pyrimidin-2-ylidene)-{3-[4-(4-methoxy-phenyl)-2-methyl-1,2,3,4-tetrahydro-isoquinolin-7-yloxy]-propyl}-amine; |
| **16** | 7-[3-(1,3-Dihydro-isoindol-2-yl)-propoxy]-4-(4-methoxy-phenyl)-2-methyl-1,2,3,4-tetrahydro-isoquinoline; |
| **17** | Bis-(2-methoxy-ethyl)-{3-[4-(4-methoxy-phenyl)-2-methyl-1,2,3,4-tetrahydro-isoquinolin-7-yloxy]-propyl}-amine; |
| **18** | 7-[3-(5,6-Dihydro-4H-pyrimidin-1-yl)-propoxy]-4-(4-methoxy-phenyl)-2-methyl-1,2,3,4-tetrahydro-isoquinoline; |
| **19** | Benzothiazol-2-yl-{3-[4-(4-methoxy-phenyl)-2-methyl-1,2,3,4-tetrahydro-isoquinolin-7-yloxy]-propyl}-methyl-amine; |
| **20** | 1-{3-[4-(4-Methoxy-phenyl)-2-methyl-1,2,3,4-tetrahydro-isoquinolin-7-yloxy]-propyl}-piperidin-3-ol; |
| **21** | 1-{3-[4-(4-Methoxy-phenyl)-2-methyl-1,2,3,4-tetrahydro-isoquinolin-7-yloxy]-propyl}-piperidin-4-ol; |
| **22** | (1-{3-[4-(4-Methoxy-phenyl)-2-methyl-1,2,3,4-tetrahydro-isoquinolin-7-yloxy]-propyl}-piperidin-4-yl)-methanol; |
| **23** | (1-{3-[4-(4-Methoxy-phenyl)-2-methyl-1,2,3,4-tetrahydro-isoquinolin-7-yloxy]-propyl}-piperidin-3-yl)-methanol; |
| **24** | (1-{3-[4-(4-Methoxy-phenyl)-2-methyl-1,2,3,4-tetrahydro-isoquinolin-7-yloxy]-propyl}-piperidio-2-yl)-methanol; |
| **25** | 4-(4-Methoxy-phenyl)-2-methyl-7-[3-(3-trifluoromethyl-piperidin-1-yl)-propoxy]-1,2,3,4-tetrahydro-isoquinoline; |
| **26** | 2-(1-{3-[4-(4-Methoxy-phenyl)-2-methyl-1,2,3,4-tetrahydro-isoquinolin-7-yloxy]-propyl}-piperidin-4-yl)-ethanol; |
| **27** | 7-[3-(1,1-Dioxo-1λ⁶-thiomorpholin-4-yl)-propoxy]-4-(4-methoxy-phenyl)-2-methyl-1,2,3,4-tetrahydro-isoquinoline; |
| **28** | 4-(4-Methoxy-phenyl)-2-methyl-7-[3-((2S)-trifluoromethyl-pyrrolidin-1-yl)-propoxy]-1,2,3,4-tetrahydro-isoquinoline; |
| **29** | 7-[3-(3,3-Difluoro-piperidin-1-yl)-propoxy]-4-(4-methoxy-phenyl)-2-methyl-1,2,3,4-tetrahydro-isoquinoline; |
| **29A** | 7-[3-(3,3-Difluoro-piperidin-1-yl)-propoxy]-4-(4-methoxy-phenyl)-2-methyl-1,2,3,4-tetrahydro-isoquinoline (enantiomer 1); |
| **29B** | 7-[3-(3,3-Difluoro-piperidin-1-yl)-propoxy]-4-(4-methoxy-phenyl)-2-methyl-1,2,3,4-tetrahydro-isoquinoline (enantiomer 2); |
| **30** | (1-{3-[4-(4-Methoxy-phenyl)-2-methyl-1,2,3,4-tetrahydro-isoquinolin-7-yloxy]-propyl}-pyrrolidin-(2R)-yl)-methanol; |
| **31** | 1-{3-[4-(4-Methoxy-phenyl)-2-methyl-1,2,3,4-tetrahydro-isoquinolin-7-yloxy]-propyl}-(R)-pyrrolidin-3-ol; |
| **32** | 7-[3-(2,6-Dimethyl-morpholin-4-yl)-propoxy]-4-(4-methoxy-phenyl)-2-methyl-1,2,3,4-tetrahydro-isoquinoline; |
| **33** | 1-{3-[4-(4-Methoxy-phenyl)-2-methyl-1,2,3,4-tetrahydro-isoquinolin-7-yloxy]-propyl}-piperidine-4-carboxylic acid ethyl ester; |
| **34** | 7-(3-Azetidin-1-yl-propoxy)-4-(4-methoxy-phenyl)-2-methyl-1,2,3,4-tetrahydro-isoquinoline; |
| **35** | 4-(4-Methoxy-phenyl)-2-methyl-7-[3-(2-methyl-pyrrolidin-1-yl)-propoxy]-1,2,3,4-tetrahydro-isoquinoline; |
| **36** | 2-(1-{3-[4-(4-Methoxy-phenyl)-2-methyl-1,2,3,4-tetrahydro-isoquinolin-7-yloxy]-propyl}-piperidin-2-yl)-ethanol; |
| **37** | 1-{3-[4-(4-Methoxy-phenyl)-2-methyl-1,2,3,4-tetrahydro-isoquinolin-7-yloxy]-propyl}-piperidine-4-carbonitrile; |
| **37A** | 1-{3-[4-(4-Methoxy-phenyl)-2-methyl-1,2,3,4-tetrahydro-isoquinolin-7-yloxy]-propyl}-piperidine-4-carbonitrile (enantiomer 1); |
| **37B** | 1-{3-[4-(4-Methoxy-phenyl)-2-methyl-1,2,3,4-tetrahydro-isoquinolin-7-yloxy]-propyl}-piperidine-4-carbonitrile (enantiomer 2); |
| **38** | 4-(4-Methoxy-phenyl)-2-methyl-7-[3-(4-trifluoromethyl-piperidin-1-yl)-propoxy]-1,2,3,4-tetrahydro-isoquinoline; |
| **39** | 7-[3-(3-Fluoro₋piperidin-1-yl)-propoxy]-4-(4-methoxy-phenyl)-2-methyl-1,2,3,4-tetrahydro-isoquinoline; |
| **40** | Ethyl-(2-methoxy-ethyl)-{3-[4-(4-methoxy-phenyl)-2-methyl-1,2,3,4-tetrahydro-isoquinolin-7-yloxy]-propyl}-amine; |
| **41** | 7-[3-(1,4-Dioxa-8-aza-spiro[4.5]dec-8-yl)-propoxy]-4-(4-methoxy-phenyl)-2-methyl-1,2,3,4-tetrahydro-isoquinoline; |
| **42** | 1-[4-(4-Methoxy-phenyl)-2-methyl-1,2,3,4-tetrahydro-isoquinolin-7-yloxy]-3-piperidin-1-yl-propan-2-ol; |
| **43** | 7-[2-Fluoro-3-(4-fluoro-piperidin-1-yl)-propoxy]-4-(4-methoxy-phenyl)-2-methyl-1,2,3,4-tetrahydro-isoquinoline; |
| **44** | 7-[3-(4-Fluoro-piperidin-1-yl)-propoxy]-4-(3-methoxy-phenyl)-2-methyl-1,2,3,4-tetrahydro-isoquinoline; |
| **45A** | 7-[3-(4-Fluoro-piperidin-1-yl)-propoxy]-4-(3-methoxy-phenyl)-2-methyl-1,2,3,4-tetrahydro-isoquinoline (enantiomer 1); |
| **45B** | 7-[3-(4-Fluoro-piperidin-1-yl)-propoxy]-4-(3-methoxy-phenyl)-2-methyl-1,2,3,4-tetrahydro-isoquinoline (enantiomer 2); |
| **46** | 4-(3-Chloro-phenyl)-7-[3-(4-fluoro-piperidin-1-yl)-propoxy]-2-methyl-1,2,3,4-tetrahydro-isoquinoline; |
| **47** | 4-(4-Chloro-phenyl)-7-[3-(4-fluoro-piperidin-1-yl)-propoxy]-2-methyl-1,2,3,4-tetrahydro-isoquinoline; |
| **48** | 4-(3-Fluoro-phenyl)-7-[3-(4-fluoro-piperidin-1-yl)-propoxy]-2-methyl-1,2,3,4-tetrahydro-isoquinoline; |
| **49** | 7-[3-(4-Fluoro-piperidin-1-yl)-propoxy]-2-methyl-4-(4-trifluoromethoxy-phenyl)-1,2,3,4.tetrahydro-isoquinoline; |
| **50** | 4-(4-Difluoromethoxy-phenyl)-7-[3-(4-fluororpiperidin-1-yl)-prcpoxy]-2-methyl-1,2,3,4-tetrahydro-isoquinoline; |
| **51** | 7-[3-(4-Fluoro-piperidin-1-yl)-propoxy]-4-(4-methanesulfonyl-phenyl)-2-methyl-1,2,3,4-tetrahydro-isoquinoline; |
| **52** | 7-[3-(4-Fluoro-piperidin-1-yl)-propoxy]-2-methyl-4-(4-methylsulfanyl-phenyl)-1,2,3,4-tetrahydro-isoquinoline; |
| **53** | 4-(3-Chloro-4-methoxy-phenyl)-7-[3-(4-fluoro-piperidin-1-yl)-propoxy]-2-methyl-1,2,3,4-tetrahydro-isoquinoline; |
| **54** | 4-(2,4-Dichloro-phenyl)-7-[3-(4-fluoro-piperidin-1-yl)-propoxy]-2-methyl-1,2,3,4-tetrahydro-isoquinoline; |
| **55** | 4-(2,5-Dichloro-phenyl)-7-[3-(4-fluoro-piperidin-1-yl)-propoxy]-2-methyl-1,2,3,4-tetrahyfro-isoquinoline; |
| **56** | 4-(3,5-Dichloro-phenyl)-7-[3-(4-fluoro-piperidin-1-yl)-propoxy]-2-methyl-1,2,3,4-tetrahydro-isoquinoline; |
| **57** | 2-Methyl-7-(3-piperidin-1-yl-propoxy)-4-thiophen-3-yl-1,2,3,4-tetrahydro-isoquirioline; |
| **58** | 4-(3,4-Dichloro-phenyl)-7-[3-(4-fluoro-piperidin-1-yl)-propoxy]-2-methyl-1,2,3,4-tetrahydro-isoquinoline; |
| **59** | 2-Methyl-7-(3-piperidin-1-yl-propoxy)-4-pyridin-3-yl-1,2,3,4-tetrahydro-isoquinoline; |
| **60** | 2-Methyl-7-(3-piperidin-1-yl-propoxy)-4-(trifluoromethyl-phenyl)-1,2,3,4-tetrahydro-isoquinoline; |
| **61** | 4-(4-Methoxy-phenyl)-2-methyl-7-(3-piperidin-1-yl-propoxy)-1,2,3,4-tetrahydro-isoquinoline (racemic); |
| **62** | 4-(4-Methoxy-phenyl)-2-methyl-7-(3-piperidin-1-yl-propoxy)-1,2,3,4-tetrahydro-isoquinoline (enantiomers 1); |
| **63** | 4-(4-Methoxy-phenyl)-2-methyl-7-(3-piperidin-1-yl-propoxy)-1,2,3,4-tetrahydro-isoquinoline (enantiomer 2); |
| **64** | 2-tert-Butyl-4-(4-methoxy-phenyl)-7-(3-piperidin-1-yl-propoxy)-1,2,3,4-tetrahydro-isoquinoline; |
| **65** | 2-Benzyl-4-(4-methoxy-phenyl)-7-(3-piperidin-1-yl-propoxy)-1,2,3,4-tetrahydro-isoquinoline; |
| **66** | 2-Ethyl-4-(4-methoxy-phenyl)-7-(3-piperidin-1-yl-propoxy)-1,2,3,4-tetrahydro-isoquinoline; |
| **67** | 4-(4-Methoxy-phenyl)-7-(3-piperidin-1-yl-propoxy)-2-propyl-1,2,3,4-tetrahydro-isoquinoline; |
| **68** | 2-Isopropyl-4-(4-methoxy-phenyl)-7-(3-piperidin-1-yl-propoxy)-1,2,3,4-tetrahydro-isoquinoline; |
| **69** | 4-(4-Methoxy-phenyl)-7-(3-piperidin-1-yl-propoxy)-1,2,3,4-tetrahydro-isoquinoline; |
| **70** | 3-[4-(4-Methoxy-phenyl)-7-(3-piperidin-1-yl-propoxy)-3,4-dihydro-1H-isoquinolin-2-yl]-propan-1-ol; |
| **71** | 2-[4-(4-Methoxy-phenyl)-7-(3-piperidin-1-yl-propoxy)-3,4-dihydro-1H-isoquinolin-2-yl]-ethanol; |
| **72** | 2-(2-Fluoro-ethyl)-4-(4-methoxy-phenyl)-7-(3-piperidin-1-yl-propoxy)-1,2,3,4-tetrahydro-isoquinoline; |
| **73** | 2-Cyclopropyl-4-(4-methoxy-phenyl)-7-(3-piperidin-1-yl-propoxy)-1,2,3,4-tetrahydro-isoquinoline; |
| **74** | 4-(4-Methoxy-phenyl)-7-(3-morpholin-4-yl-propoxy)-2-(1-phenyl-ethyl)-1,2,3,4-tetrahydro-isoquinoline; |
| **75** | 4-(4-Methoxy-phenyl)-7-(3-morpholin-4-yl-propoxy)-2-(1-phenyl-ethyl)-1,2,3,4-tetrahydro-isoquinoline (diastereomer 1); |
| **75A** | 4-(4-Methoxy-phenyl)-7-(3-morpholin-4-yl-propoxy)-2-(1-phenyl-ethyl)-1,2,3,4-tetrahydro-isoquinoline (diastereomer 2); |
| **76** | 4-(3,4-Dichloro-phenyl)-2-methyl-7-(3-piperidin-1-yl-propoxy)-1,2,3,4-tetrahydro-isoquinoline; |
| **77** | 4-(3,4-Dichloro-phenyl)-2-methyl-7-(3-morpholin-4-yl-propoxy)-1,2,3,4-tetrahydro-isoquinoline; |
| **78** | 4-(4-Methoxy-phenyl)-2-methyl-7-(piperidin-4-ylmethoxy)-1,2,3,4-tetrahydro-isoquinoline; |
| **79** | 4-(3-Chloro-4-methoxy-phenyl)-2-methyl-7-(piperidin-4-ylmethoxy)-1,2,3,4-tetrahydro-isoquinoline; |
| **80** | 2-Methyl-4-(4-methylsulfanyl-phenyl)-7-(piperidin-4-ylmethoxy)-1,2,3,4-tetrahydro-isoquinoline; |
| **81** | 4-(3-Fluoro-4-methoxy-phenyl)-2-methyl-7-(piperidin-4-ylmethoxy)-1,2,3,4-tetrahydro-isoquinoline; |
| **82** | 4-(2-Fluoro-4-methoxy-phenyl)-2-methyl-7-(piperidin-4-ylmethoxy)-1,2,3,4-tetrahydro-isoquinoline; |
| **83** | 7-(1-Isopropyl-piperidin-4-ylmethoxy)-4-(4-methoxy-phenyl)-2-methyl-1,2,3,4-tetrahydro-isoquinoline; |
| **84** | 4-(4-Methoxy-phenyl)-2-methyl-7-(1-methyl-piperidin-4-ylmethoxy)-1,2,3,4-tetrahydro-isoquinoline; |
| **85** | 4-(4-Methoxy-phenyl)-2-methyl-7-(1-propyl-piperldin-4-ylmethoxy)-1,2,3,4-tetrahydro-isoquiholine; |
| **86** | 7-(1-Cyclopentyl-piperidin-4-ylmethoxy)-4-(4-methoxy-phenyl)-2-methyl-1,2,3,4-tetrahydro-isoquinoline; |
| **87** | 7-(1-Ethyl-piperidin-4-ylmethoxy)-4-(4-methoxy-phenyl)-2-methyl-1,2,3,4-tetrahydro-isoquinoline; |
| **88** | 4-(3-Chloro-4-methoxy-phenyl)-7-(1-isopropyl-piperidin-4-ylmethoxy)-2-methyl-1,2,3,4-tetrahydro-isoquinoline; |
| **89** | 4-(3-Fluoro-4-methoxy-phenyl)-7-(1-isopropyl-piperidin-4-ylmethoxy)-2-methyl-1,2,3,4-tetrahydro-isoquinoline; |
| **90** | 4-(2-Fluoro-4-methoxy-phenyl)-7-(1-isopropyl-piperidin-4-ylmethoxy)-2-methyl-1,2,3,4-tetrahydro-isoquinoline; |
| **91** | 7-(1-Isopropyl-piperidin-4-ylmethoxy)-4-(3-methoxy-phenyl)-2-methyl-1,2,3,4-tetrahydro-isoquinoline; |
| **92** | 4-(3-Methoxy-phenyl)-2-methyl-7-(1-methyl-piperidin-4-ylmethoxy)-1,2,3,4-tetrahydro-isoquinoline; |
| **93** | 4-(3-Methoxy-phenyl)-2-methyl-7-(1-propyl-piperidin-4-ylmethoxy)-1,2,3,4-tetrahydro-isoquinoline; |
| **94** | 7-(1-Cyclopentyl-piperidin-4-ylmethoxy)-4-(3-methoxy-phenyl)-2-methyl-1,2,3,4-tetrahydro-isoquinoline; |
| **95** | 7-(1-Ethyl-piperidin-4-ylmethoxy)-4-(3-methoxy-phenyl)-2-methyl-1,2,3,4-tetrahydro-isoquinoline; |
| **96** | 4-(3-Methoxy-phenyl)-2-methyl-7-(piperidin-4-yloxy)-1,2,3,4-tetrahydro-isoquinoline; |
| **97** | 4-(3-Methoxy-phenyl)-2-methyl-7-(1-methyl-piperidin-4-yloxy)-1,2,3,4-tetrahydro-isoquinoline; |
| **98** | 7-(1-Isopropyl-piperidin-4-yloxy)-4-(3-methoxy-phenyl)-2-methyl-1,2,3,4-tetrahydro-isoquinoline; |
| **99** | 7-(1-Cyclobutyl-piperidin-4-yloxy)-4-(3-methoxy-phenyl)-2-methyl-1,2,3,4-tetrahydro-isoquinoline; |
| **100** | 7-(1-Ethyl-piperidin-4-yloxy)-4-(3-methoxy-phenyl)-2-methyl-1,2,3,4-tetrahydro-isoquinoline; |
| **101** | 7-(1-Cyclopentyl-piperidin-4-yloxy)-4-(3-methoxy-phenyl)-2-methyl-1,2,3,4-tetrahydro-isoquinoline; |
| **102** | 4-(3-Methoxy-phenyl)-2-methyl-7-(1-propyl-piperidin-4-yloxy)-1,2,3,4-tetrahydro-isoquinoline; |
| **103** | 7-(1-Isopropyl-piperidin-4-ylmethoxy)-2-methyl-4-(4-methylsulfanyl-phenyl)-1,2,3,4-tetrahydro-isoquinoline; |
| **104** | 7-(1-Cyclobutyl-piperidin-4-ylmethoxy)-2-methyl-4-(4-methylsulfanyl-phenyl)-1,2,3,4-tetrahydro-isoquinoline; |
| **105** | 7-(1-Ethyl-piperidin-4-ylmethoxy)-2-methyl-4-(4-methylsulfanyl-phenyl)-1,2,3,4-tetrahydro-isoquinoline; |
| **106** | 4-(3-Chloro-4-methoxy-phenyl)-7-(1-cyclobutyl-piperidin-4-ylmethoxy)-2-methyl-1,2,3,4-tetrahydroisoquinoline; |
| **107** | 4-(3-Chloro-4-methoxy-phenyl)-7-(1-ethyl-piperidin-4-ylmethoxy)-2-methyl-1,2,3,4-tetrahydro-isoquinoline; |
| **108** | 4-(3-Chloro-4-methoxy-phenyl)-2-methyl-7-(1-propyl-piperidin-4-ylmethoxy)-1,2,3,4-tetrahydro-isoquinoline; |
| **109** | 2-Methyl-4-(4-methylsulfanyl-phenyl)-7-(1-propyl-piperidin-4-ylmethoxy)-1,2,3,4-tetrahydro-isoquinoline; |
| **110** | 7-(1-Isobutyl-piperidin-4-ylmethoxy)-4-(4-methoxy-phenyl)-2-methyl-1,2,3,4-tetrahydro-isoquinoline; |
| **111** | 7-(1-Cyclobutyl-piperidin-4-ylmethoxy)-4-(3-fluoro-4-methoxy-phenyl)-2-methyl-1,2,3,4-tetrahydro-isoquinoline; |
| **112** | 4-(3-Fluoro-4-methoxy-phenyl)-2-methyl-7-(1-propyl-piperidin-4-ylmethoxy)-1,2,3,4-tetrahydro-isoquinoline; |
| **113** | 7-(1-Cyclobutyl-piperidin-4-ylmethoxy)-4-(2-fluoro-4-methoxy-phenyl)-2-methyl-1,2,3,4-tetrahydro-isoquinoline; |
| **114** | 4-(2-Fluoro-4-methoxy-phenyl)-2-methyl-7-(1-propyl-piperidin-4-ylmethoxy)-1,2,3,4-tetrahydro-isoquinoline; |
| **115** | 4-(2-Fluoro-4-methoxy-phenyl)-7-(1-isobutyl-piperidin-4-ylmethoxy)-2-methyl-1,2,3,4-tetrahydro-isoquinoline; |
| **116** | 7-[1-(2-Fluoro-ethyl)-piperidin-4-ylmethoxy]-4-(4-methoxy-phenyl)-2-methyl-1,2,3,4-tetrahydro-isoquinoline; |
| **117** | 7-(1-isopropyl-piperidin-4-yloxy)-4-(4-methoxy-phenyl)-2-methyl-1,2,3,4-tetrahydro-isoquinoline; |
| **118** | 7-(1-Isopropyl-piperidin-4-yloxy)-2-methyl-4-(4-methylsulfanyl-phenyl)-1,2,3,4-tetrahydro-isoquinoline; |
| **119** | 4-(2-Fluoro-4-methoxy-phenyl)-7-(1-isopropyl-piperidin-4-yloxy)-2-methyl-1,2,3,4-tetrahydro-isoquinoline; |
| **120** | 4-(3-Fluoro-4-methoxy-phenyl)-7-(1-isopropyl-piperidin-4-yloxy)-2-methyl-1,2,3,4-tetrahydro-isoquinoline; |
| **121** | 4-(4-Methoxy-phenyl)-2-methyl-7-[1-(tetrahydro-pyran-4-yl)-piperidin-4-yloxy]-1,2,3,4-tetrahydro-isoquinoline; |
| **122** | 2,2,2-Trifluoro-1-{4-[4-(4-methoxy-phenyl)-2-methyl-1,2,3,4-tetrahydro-isoquinolin-7-yloxymethyl]-piperidin-1-yl}-ethanone; |
| **123** | 4-(4-Methoxy-phenyl)-2-methyl-7-[1-(2,2,2-trifluoro-ethyl)-piperidin-4-ylmethoxy]-1,2,3,4-tetrahydroisoquinoline; |
| **124** | 7-[3-(4-Fluoro-piperidin-1-yl)-propoxy]-2-methyl-4-phenyl-1,2,3,4-tetrahydro-isoquinoline; |
| **125** | 4-(2-Fluoro-phenyl)-7-[3-(4-fluoro-piperidin-1-yl)-propoxy]-2-methyl-1,2,3,4-tetrahydro-isoquinoline; |
| **126** | 7-[3-(4-Fluoro-piperidin-1-yl)-propoxy]-2-methyl-4-p-tolyl-1,2,3,4-tetrahydro-isoquinoline; |
| **127** | 2-Benzyl-7-[3-(4-fluoro-piperidin-1-yl)-propoxy]-4-(4-methoxy-phenyl)-1,2,3,4-tetrahydro-isoquinoline; |
| **128** | 7-[3-(4-Fluoro-piperidin-1-yl)-propoxy]-2-methyl-4-(3-trifluoromethoxy-phenyl)-1,2,3,4-tetrahydro-isoquinoline; |
| **129** | 7-[3-(3,3-Difluoro-pyrrolidin-1-yl)-propoxy]-4-(4-methoxy-phenyl)-2-methyl-1,2,3,4-tetrahydro-isoquinoline; |
| **130** | 4-(4-Methoxy-phenyl)-2-methyl-7-[3-(3S-methyl-morpholin-4-yl)-propoxy]-1,2,3,4-tetrahydro-isoquinoline; |
| **131** | Dicyclopropylmethyl-{3-[4-(4-methoxy-phenyl)-2-methyl-1,2,3,4-tetrahydro-isoquinolin-7-yloxy]-propyl}-amine; |
| **132** | 4-(2-Chloro-phenyl)-7-[3-(4-fluoro-piperidin-1-yl)-propoxy]-2-methyl-1,2,3,4-tetrahydro-isoquinoline; |
| **133** | 2-Methyl-7-[3-(3S-methyl-morpholin-4-yl)-propoxy]-4-(4-morpholin-4-yl-phenyl)-1,2,3,4-tetrahydro-isoquinoline; |
| **134** | 7-[3-(4-Fluoro-piperidin-1-yl)-propoxy]-2-methyl-4-(4-morpholin-4-yl-phenyl)-1,2,3,4-tetrahydro-isoquinoline; |
| **135** | 4-{2-Methyl-7-[3-(3S-methyl-morpholin-4-yl)-propoxy]-1,2,3,4-tetrahydro-isoquinolin-4-yl}-benzonitrile; |
| **136** | 4-{7-[3-(4-Fluoro-piperidin-1-yl)-propoxy]-2-methyl-1,2,3,4-tetrahydro-isoquinolin-4-yl}-benzonitrile; |
| **137** | 7-[3-(3-Benzhydryl-azetidin-1-yl)-propoxy]-4-(4-methoxy-phenyl)-2-methyl-1,2,3,4-tetrahydro-isoquinoline; |
| **138** | 2-Methyl-4-(4-methylsulfanyl-phenyl)-7-(3-morpholin-4-yl-propoxy)-1,2,3,4-tetrahydro-isoquinoline; |
| **139** | 4-(2-Fluoro-4-methoxy-phenyl)-7-[3-(4-fluoro-piperidin-1-yl)-propoxy]-2-methyl-1,2,3,4-tetrahydro-isoquinoline (racemate); |
| **140** | 4-(2-Fluoro-4-methoxy-phenyl)-7-[3-(4-fluoro-piperidin-1-yl)-propoxy]-2-methyl-1,2,3,4-tetrahydro-isoquinoline (enantiomer 1); |
| **141** | 4-(2-Fluoro-4-methoxy-phenyl)-7-[3-(4-fluoro-piperidin-1-yl)-propoxy]-2-methyl-1,2,3,4-tetrahydro-isoquinoline (enantiomer 2); |
| **142** | 4-(3-Fluoro-4-methoxy-phenyl)-7-[3-(4-fluoro-piperidin-1-yl)-propoxy]-2-methyl-1,2,3,4-tetrahydro-isoquinoline (racemate); |
| **143** | 4-(3-Fluoro-4-methoxy-phenyl)-7-[3-(4-fluoro-piperidin-1-yl)-propoxy]-2-methyl-1,2,3,4-tetrahydro-isoquinoline (enantiomer 1); |
| **144** | 4-(3-Fluoro-4-methoxy-phenyl)-7-[3-(4-fluoro-piperidin-1-yl)-propoxy]-2-methyl-1,2,3,4-tetrahydro-isoquinoline (enantiomer 2); |
| **145** | 4-(4-Ethoxy-phenyl)-7-[3-(4-fluoro-pipendin-1-yl)-propoxy]-2-methyl-1,2,3,4-tetrahydro-isoquinoline; |
| **146** | 2-Ethyl-7-[3-(4-fluoro-piperidin-1-yl)-propoxy]-4-(4-methoxy-phenyl)-1,2,3,4-tetrahydro-isoquinoline; |
| **147** | 7-[3-(4-Fluoro-piperidin-1-yl)-propoxy]-4-(4-methoxy-phenyl)-1,2,3,4-tetrahydro-isoquinoline; |
| **148** | 4-(4-Methoxy-phenyl)-2-methyl-7-(peridin-4-yloxy)-1,2,3,4-tetrahydro-isoquinoline; |
| **149** | 2-Methyl-4-(4-methylsulfanyl-phenyl)-7-(piperidin-4-yloxy)-1,2,3,4-tetrahydro-isoquinoline; |
| **150** | 4-(2-Fluoro-4-methoxy-phenyl)-2-methyl-7-(piperidin-4-yloxy)-1,2,3,4-tetrahydro-isoquinoline; |
| **151** | 4-(3-Fluoro-4-methoxy-phenyl)-2-methyl-7-(piperidin-4-yloxy)-1,2,3,4-tetrahydro-isoquinoline; |
| **152** | 7-[1-(2-Fluoro-ethyl)-piperidin-4-yloxy]-4-(4-methoxy-phenyl)-2-methyl-1,2,3,4-tetrahydro-isoquinoline; |
| **153** | 7-[3-(4-Fluoro-piperidin-1-yl)-propoxy]-2-methyl-4-(4-methylsulfanyl-phenyl)-1,2,3,4-tetrahydro-isoquinoline (enantiomer 1); |
| **154** | 7-[3-(4-Fluoro-piperidin-1-yl)-propoxy]-2-methyl-4-(4-methylsulfanyl-phenyl)-1,2,3,4-tetrahydro-isoquinoline (enantiomer 2); |
| **155** | 4-(4-Methoxy-phenyl)-2-methyl-7-[3-(3S-methyl-morpholin-4-yl)-propoxy]-1,2,3,4-tetrahydro-isoquinoline (enantiomer 1); |
| **156** | 4-(4-Methoxy-phenyl)-2-methyl-7-[3-(3S-methyl-morpholin-4-yl)-propoxy]-1,2,3,4-tetrahydro-isoquinoline (enantiomer 2); |
| **157** | 2-Methyl-4-(4-methylsulfanyl-phenyl)-7-(3-morpholin-4-yl-propoxy)-1,2,3,4-tetrahydro-isoquinoline (enantiomer 1); |
| **158** | 2-Methyl-4-(4-methylsulfanyl-phenyl)-7-(3-morpholin-4-yl-propoxy)-1,2,3,4-tetrahydro-isoquinoline (enantiomer 2); |
| **159** | 7-[3-(4-Fluoro-piperidin-1-yl)-propoxy]-4-(4-methylsulfanyl-phenyl)-1,2,3,4-tetrahydro-isoquinoline; |
| **160** | 4-(4-Methylsulfanyl-phenyl)-7-(3-morpholin-4-yl-propoxy)-1,2,3,4-tetrahydro-isoquinoline; |
| **161** | 7-[3-(3S-Methyl-morpholin-4-yl)-propoxy]-4-(4-methylsulfanyl-phenyl)-1,2,3,4-tetrahydro-isoquinoline; |
| **163** | 4-(4-Methoxy-phenyl)-7-(3-morpholin-4-yl-propoxy)-1,2,3,4-tetrahydro-isoquinoline; |
| **164** | 7-[3-(4-Fluoro-piperidin-1-yl)-propoxy]-4-(4-methanesulfinyl-phenyl)-2-methyl-1,2,3,4-tetrahydro-isoquinoline; |
| **165** | 4-(4-Methanesulfinyl-phenyl)-2-methyl-7-(3-morpholin-4-yl-propoxy)-1,2,3,4-tetrahydro-isoquinoline; |
| **165A** | 4-(4-Methanesulfinyl-phenyl)-2-methyl-7-(3-morpholin-4-yl-propoxy)-1,2,3,4-tetrahydro-isoquinoline (enantiomer 1); |
| **167** | 4-(4-Methanesulfonyl-phenyl)-2-methyl-7-(3-morpholin-4-yl-propoxy)-1,2,3,4-tetrahydro-isoquinoline; |
| **168** | 7-[3-(4-Fluoro-piperidin-1-yl)-propoxy]-4-(4-methoxy-phenyl)-2,6-dimethyl-1,2,3,4-tetrahydro-isoquinoline; |
| **169** | 7-[3-(4-Fluoro-piperidin-1-yl)-propoxy]-4-(4-methoxy-phenyl)-2,8-dimethyl-1,2,3,4-tetrahydro-isoquinoline; |
| **170** | 7-[3-(4-Fluoro-piperidn-1-yl)-propoxy]-4-(4-methoxy-phenyl)-2-methyl-1,2,3,4-tetrahydro-isoquinolin-6-ol; |
| **171** | 7-[3-(4-Fluoro-piperidin-1-yl)-propoxy]-4-(4-methoxy-phenyl)-2-methyl-1,2,3,4-tetrahydro-isoquinolin-8-ol; |
| **172** | 2,8-Dimethyl-4-(4-methylsulfanyl-phenyl)-7-(3-morpholin-4-yl-propoxy)-1,2,3,4-tetrahydro-isoquinoline; |
| **173** | 2,6-Dimethyl-4-(4-methylsulfanyl-phenyl)-7-(3-morpholin-4-yl-propoxy)-1,2,3,4-tetrahydro-isoquinoline; |
| **174** | 2-Methyl-4-(4-methylsulfanyl-phenyl)-7-(3-morpholin-4-yl-propoxy)-1,2,3,4-tetrahydro-isoquinolin-8-ol; |
| **175** | 2-Methyl-4-(4-methylsulfanyl-phenyl)-7-(3-morpholin-4-yl-propoxy)-1,2,3,4-tetrahydro-isoquinolin-6-ol; |
| **176** | 8-Fluoro-2-methyl-4-(4-methylsulfanyl-phenyl)-7-(3-morpholin-4-yl-propoxy)-1,2,3,4-tetrahydro-isoquinoline; |
| **177** | 6-Fluoro-2-methyl-4-(4-methylsulfanyl-phenyl)-7-(3-morpholin-4-yl-propoxy)-1,2,3,4-tetrahydro-isoquinoline; |
| **178** | 7-[1-(2-Fluoro-ethyl)-piperidin-4-ylmethoxy]-2-methyl-4-(4-methylsulfanyl-phenyl)-1,2,3,4-tetrahydro-isoquinoline; |
| **179** | 7-[1-(2-Fluoro-ethyl)-piperidin-4-yloxy]-2-methyl-4-(4-methylsulfanyl-phenyl)-1,2,3,4-tetrahydro-isoquinoline; |
| **180** | 4-(4-Methoxy-phenyl)-7-[3-(3S-methyl-morpholin-4-yl)-propoxy]-1,2,3,4-tetrahydro-isoquinoline; |
| **181** | 4-(4-Methanesulfinyl-phenyl)-2-methyl-7-[3-(3S-methyl-morpholin-4-yl)-propoxy]-1,2,3,4-tetrahydro-isoquinoline; |
| **182** | 7-[3-(3,3-Difluoro-azetidin-1-yl)-propoxy]-4-(4-methoxy-phenyl)-2-methyl-1,2,3,4-tetrahydro-isoquinoline; |
| **183** | (4-{3-[4-(4-Methoxy-phenyl)-2-methyl-1,2,3,4-tetrahydro-isoquinolin-7-yloxy]-propyl}-morpholin-3-yl)-methanol; |
| **183A** | (4-{3-[4-(4-Methoxy-phenyl)-2-methyl-1,2,3,4-tetrahydro-isoquinolin-7-yloxy]-propyl}-morpholin-3S-yl)-methanol; |
| **184** | (4-{3-[4-(4-Methoxy-phenyl)-2-methyl-1,2,3,4-tetrahydro-isoquinolin-7-yloxy]-propyl}-morpholin-2-yl)-methanol; |
| **185** | {3-[2-Methyl-4-(4-methylsulfanyl-phenyl)-1,2,3,4-tetrahydro-isoquinolin-7-yloxy]-propyl}-(2H-pyrazol-3-yl)-amine; |
| **186** | 4-(6-Bromo-pyridin-3-yl)-7-[3-(4-fluoro-piperidin-1-yl)-propoxy]-2-methyl-1,2,3,4-tetrahydro-isoquinoline; |
| **187** | 7-[3-(4-Fluoro-piperidin-1-yl)-propoxy]-2-methyl-4-(6-methylsulfanyl-pyridin-3-yl)-1,2,3,4-tetrahydro-isoquinoline; |
| **188** | (5-{7-[3-(4-Fluoro-piperidin-1-yl)-propoxy]-2-methyl-1,2,3,4-tetrahydro-isoquinolin-4-yl}-pyridin-2-yl)-dimethyl-amine; |
| **189** | 7-[3-(4-Fluoro-piperidin-1-yl)-propoxy]-2-methyl-4-(6-trimethylsilanylethynyl-pyridin-3-yl)-1,2,3,4-tetrahydro-isoquinoline; |
| **190** | 4-(6-Ethynyl-pyridin-3-yl)-7-[3-(4-fluoro-piperidin-1-yl)-propoxy]-2-methyl-1,2,3,4-tetrahydro-isoquinoline; |
| **191** | 7-[3-(4-Fluoro-piperidin-1-yl)-propoxy]-2-methyl-4-(6-phenylsulfanyl-pyridin-3-yl)-1,2,3,4-tetrahydro-isoquinoline; |
| **192** | 7-[3-(4-Fluoro-piperidin-1-yl)-propoxy]-4-(6-imidazol-1-yl-pyridin-3-yl)-2-methyl-1,2,3,4-tetrahydro-isoquinoline; |
| **193** | 7-[3-(4-Fluoro-piperidin-1-yl)-propoxy]-4-(6-methoxy-pyridin-3-yl)-2-methyl-1,2,3,4-tetrahydro-isoquinoline; |
| **194** | 7-[3-(4-Fluoro-piperidin-1-yl)-propoxy]-2-methyl-4-(6-pyrazol-1-yl-pyridin-3-yl)-1,2,3,4-tetrahydro-isoquinoline; |
| **195** | 7-[3-(4-Fluoro-piperidin-1-yl)-propoxy]-4-[6-(1H-imidazol-2-ylsulfanyl)-pyridin-3-yl]-2-methyl-1,2,3,4-tetrahydro-isoquinoline; |
| **196** | 7-[3-(4-Fluoro-piperidin-1-yl)-propoxy]-2-methyl-4-[6-(pyrimidin-2-ylsulfanyl)-pyridin-3-yl]-1,2,3,4-tetrahydro-isoquinoline; |
| **197** | 4-(4-Methoxy-phenyl)-2-methyl-7-[3-(4-methyl-piperazin-1-yl)-propoxy]-1,2,3,4-tetrahydro-isoquinoline; |
| **198** | 7-[3-(4-Ethyl-piperazin-1-yl)-propoxy]-4-(4-methoxy-phenyl)-2-methyl-1,2,3,4-tetrahydro-isoquinoline; |
| **199** | 7-[3-(4-Isopropyl-piperazin-1-yl)-propoxy]-4-(4-methoxy-phenyl)-2-methyl-1,2,3,4-tetrahydro-isoquinoline; |
| **200** | 4-(4-Methoxy-phenyl)-2-methyl-7-[3-(4-thiazol-2-yl-piperazin-1-yl)-propoxy]-1,2,3,4-tetrahydro-isoquinoline; |
| **201** | 4-(4-Methoxy-phenyl)-2-methyl-7-[3-(4-thiophen-2-ylmethyl-piperazin-1-yl)-propoxy]-1,2,3,4-tetrahydro-isoquinoline; |
| **202** | 2-(4-{3-[4-(4-Methoxy-phenyl)-2-methyl-1,2,3,4-tetrahydro-isoquinolin-7-yloxy]-propyl}-piperazin-1-yl)-ethanol; |
| **203** | 2-(4-{3-[4-(4-Methoxy-phenyl)-2-methyl-1,2,3,4-tetrahydro-isoquinolin-7-yloxy]-propyl}-piperazin-1-yl)-phenol; |
| **204** | 4-(4-Methoxy-phenyl)-2-methyl-7-[3-(4-pyridin-4-yl-piperazin-1-yl)-propoxy]-1,2,3,4-tetrahydro-isoquinoline; |
| **205** | 4-(4-Methoxy-phenyl)-2-methyl-7-{3-[4-(4-trifluoromethyl-phenyl)-piperazin-1-yl]-propoxy}-1,2,3,4-tetrahydro-isoquinoline; |
| **206** | 7-[3-(4-Allyl-piperazin-1-yl)-propoxy]-4-(4-methoxy-phenyl)-2-methyl-1,2,3,4-tetrahydro-isoquinoline; |
| **207** | 7-[3-(4-[1,3]-Dioxolan-2-ylmethyl-piperazin-1-yl)-propoxy]-4-(4-methoxy-phenyl)-2-methyl-1,2,3,4-tetrahydro-isoquinoline; |
| **208** | 4-(4-{3-[4-(4-Methoxy-phenyl)-2-methyl-1,2,3,4-tetrahydro-isoquinolin-7-yloxy]-propyl}-piperazin-1-yl)-benzonitrile; |
| **209** | 7-{3-[4-(2-Methoxy-ethyl)-piperazin-1-yl]-propoxy}-4-(4-methoxy-phenyl)-2-methyl-1,2,3,4-tetrahydro-isoquinoline; |
| **210** | 4-(4-Methoxy-phenyl)-2-methyl-7-{3-[4-(tetrahydro-furan-2-ylmethyl)-piperazin-1-yl]-propoxy}-1,2,3,4-tetrahydro-isoquinoline; |
| **211** | 4-{3-[4-(4-Methoxy-phenyl)-2-methyl-1,2,3,4-tetrahydro-isoquinolin-7-yloxy]-propyl}-piperazine-1-carboxylic acid tert-butyl ester; |
| **212** | 7-[3-(4-Cyclopropyl-piperazin-1-yl)-propoxy]-4-(4-methoxy-phenyl)-2-methyl-1,2,3,4-tetrahydro-isoquinoline; |
| **213** | 4-(4-Bromo-phenyl)-7-[3-(4-fluoro-piperidin-1-yl)-propoxy]-2-methyl-1,2,3,4-tetrahydro-isoquinoline; |
| **214** | 4-(4-Difluoromethoxy-phenyl)-2-methyl-7-(3-morpholin-4-yl-propoxy)-1,2,3,4-tetrahydro-isoquinoline; |
| **215** | 2-Methyl-7-[3-(35-methyl-morpholin-4-yl)-propoxy]-4-(4-methylsulfanyl-phenyl)-1,2,3,4-tetrahydro-isoquinoline; |
| **216** | 7-[3-(4,4-Difluoro-piperidin-1-yl)-propoxy]-2-methyl-4-(4-methylsulfanyl-phenyl)-1,2,3,4-tetrahydro-isoquinoline; |
| **217** | 7-[3-(4-Fluoro-piperidin-1-yl)-propoxy]-2-methyl-4-(4-trifluoromethylsulfanyl-phenyl)-1,2,3,4-tetrahydro-isoquinoline; |
| **218** | 2-Methyl-7-(3-morpholin-4-yl-propoxy)-4-(4-trifluoromethylsulfanyl-phenyl)-1,2,3,4-tetrahydro-isoquinoline; |
| **219** | 2-Methyl-7-[3-(3S-methyl-morpholin-4-yl)-propoxy]-4-(4-trifluoromethylsulfanyl-phenyl)-1,2,3,4-tetrahydro-isoquinoline; |
| **220** | 4-(2-Fluoro-4-methoxy-phenyl)-2-methyl-7-[3-(3S-methyl-morpholin-4-yl)-propoxy]-1,2,3,4-tetrahydro-isoquinoline; |
| **221** | 4-(3-Fluoro-4-methoxy-phenyl)-2-methyl-7-[3-(3S-methyl-morpholin-4-yl)-propoxy]-1,2,3,4-tetrahydro-isoquinoline; |
| **222** | 2-Methyl-7-(3-piperidin-1-yl-propoxy)-4-(4-trifluoromethoxy-phenyl)-1,2,3,4-tetrahydro-isoquinoline; |
| **223** | {3-[4-(4-Methoxy-phenyl)-2-methyl-1,2,3,4-tetrahydro-isoquinolin-7-yloxy]-propyl}-(tetrahydro-pyran-4-yl)-amine; |
| **224** | Cyclopropyl-{3-[4-(4-methoxy-phenyl)-2-methyl-1,2,3,4-tetrahydro-isoquinolin-7-yloxy]-propyl}-(1-methyl-piperidin-4-yl)-amine; |
| **225** | (2-Methoxy-ethyl)-{3-[4-(4-methoxy-phenyl)-2-methyl-1,2,3,4-tetrahydro-isoquinolin-7-yloxy]-propyl}-amine; |
| **226** | 3-{3-[4-(4-Methoxy-phenyl)-2-methyl-1,2,3,4-tetrahydro-isoquinolin-7-yloxy]-propylamino}-propan-1-ol; |
| **227** | Allyl-{3-[4-(4-methoxy-phenyl)-2-methyl-1,2,3,4-tetrahydro-isoquinolin-7-yloxy]-propyl}-amine; |
| **228** | Isobutyl-{3-[4-(4-methoxy-phenyl)-2-methyl-1,2,3,4-tetrahydro-isoquinolin-7-yloxy]-propyl}-amine; |
| **229** | Cyclopropyl-{3-[4-(4-methoxy-phenyl)-2-methyl-1,2,3,4-tetrahydro-isoquinolin-7-yloxy]-propyl}-amine; |
| **230** | Isopropyl-{3-[4-(4-methoxy-phenyl)-2-methyl-1,2,3,4-tetrahydro-isoquinolin-7-yloxy]-propyl}-amine; |
| **231** | {3-[4-(4-Methoxy-phenyl)-2-methyl-1,2,3,4-tetrahydro-isoquinolin-7-yloxy]-propyl}-propyl-amine; |
| **232** | Ethyl-{3-[4-(4-methoxy-phenyl)-2-methyl-1,2,3,4-tetrahydro-isoquinolin-7-yloxy]-propyl}-amine; |
| **233** | Isopropyl-{3-[4-(4-methoxy-phenyl)-2-methyl-1,2,3,4-tetrahydro-isoquinolin-7-yloxy]-propyl}-methyl-amine; |
| **234** | Cyclopropyl-{3-[4-(4-methoxy-phenyl)-2-methyl-1,2,3,4-tetrahydro-isoquinolin-7-yloxy]-propyl}-methyl-amine; |
| **235** | Dicyclopropyl-{3-[4-(4-methoxy-phenyl)-2-methyl-1,2,3,4-tetrahydro-isoquinolin-7-yloxy]-propyl}-amine; |
| **236** | 7-(1-Isopropyl-azetidin-3-ylmethoxy)-2-methyl-4-(4-methylsulfanyl-phenyl)-1,2,3,4-tetrahydro-isoquinoline; |
| **237** | 7-(1-Cyclopropyl-azetidin-3-ylmethoxy)-2-methyl-4-(4-methylsulfanyl-phenyl)-1,2,3,4-tetrahydro-isoquinoline; |
| **238** | 7-(1-Cyclobutyl-azetidin-3-ylmethoxy)-2-methyl-4-(4-methylsulfanyl-phenyl)-1,2,3,4-tetrahydro-isoquinoline; |
| **239** | 7-[1-(2-Fluoro-ethyl)-azetidin-3-ylmethoxy]-2-methyl-4-(4-methylsulfanyl-phenyl)-1,2,3,4-tetrahydro-isoquinoline; |
| **240** | 4-(4-Methoxy-phenyl)-2-methyl-7-[2-(1-methyl-pyrrolidin-2-yl)-ethoxy]-1,2,3,4-tetrahydro-isoquinoline; |
| **240A** | 4-(4-Methoxy-phenyl)-2-methyl-7-[2-(1-methyl-pyrrolidin-2-yl)-ethoxy]-1,2,3,4-tetrahydro-isoquinoline (diastereomer 1); |
| **240B** | 4-(4-Methoxy-phenyl)-2-methyl-7-[2-(1-methyl-pyrrolidin-2-yl)-ethoxy]-1,2,3,4-tetrahydro-isoquinoline (diastereomer 2); |
| **241** | 4-(3-Methoxy-phenyl)-2-methyl-7-(piperidin-4-ylmethoxy)-1,2,3,4-tetrahydro-isoquinoline; |
| **242** | {4-[4-(4-Methoxy-phenyl)-2-methyl-1,2,3,4-tetrahydro-isoquinolin-7-yloxymethyl]-piperidin-1-yl}-acetonitrile; |
| **243** | 2-Methyl-7-(1-methyl-piperidin-4-yloxy)-4-(4-methylsulfanyl-phenyl)-1,2,3,4-tetrahydro-isoquinoline; |
| **244** | 2-Mefhyl-4-(4-methylsulfanyl-phenyl)-7-[1-(3,3,3-trifluoro-propyl)-piperidin-4-yloxy]-1,2,3,4-tetrahydro-isoquinoline; |
| **245** | {4-[2-Methyl-4-(4-methylsulfanyl-phenyl)-1,2,3,4-tetrahydro-isoquinolin-7-yloxy]-piperidin-1-yl}-acetonitrile; |
| **246** | 2-Methyl-4-(4-methylsulfanyl-phenyl)-7-[1-(tetrahydro-pyran-4-yl)-piperidin-4-yloxy]-1,2,3,4-tetrahydro-isoquinoline; |
| **247** | 7-(1-Cyclopropyl-piperidin-4-yloxy)-2-methyl-4-(4-methylsulfanyl-phenyl)-1,2,3,4-tetrahydro-isoquinoline; |
| **248** | 7-(1-Cyclobutyl-piperidin-4-ylmethoxy)-4-(4-methoxy-phenyl)-2-methyl-1,2,3,4-tetrahydro-isoquinoline; |
| **249** | 7-(1-Cyclopropyl-piperidin-4-ylmethoxy)-4-(4-methoxy-phenyl)-2-methyl-1,2,3,4-tetrahydro-isoquinoline; |
| **250** | 1-{4-[4-(4-Methoxy-phenyl)-2-methyl-1,2,3,4-tetrahydro-isoquinolin-7-yloxymethyl]-piperidin-1-yl}-2-methyl-propan-2-ol; |
| **251** | 4-(4-Methoxy-phenyl)-2-methyl-7-(4-methyl-morpholin-2-ylmethoxy)-1,2,3,4-tetrahydro-isoquinoline; |
| **252** | 4-(4-Methoxy-phenyl)-2-methyl-7-(morpholin-2S-ylmethoxy)-1,2,3,4-tetrahydro-isoquinoline; |
| **253** | 4-(4-Methoxy-phenyl)-2-methyl-7-(morpholin-2R-ylmethoxy)-1,2,3,4-tetrahydro-isoquinoline; |
| **254** | 7-(4-Isopropyl-morpholin-2-ylmethoxy)-4-(4-methoxy-phenyl)-2-methyl-1,2,3,4-tetrahydro-isoquinoline; |
| **255** | 7-(4-isopropyl-morpholin-2S-ylmethoxy)-4-(4-methoxy-phenyl)-2-methyl-1,2,3,4-tetrahydro-isoquinoline; |
| **256** | 7-(4-Isopropyl-morpholin-2R-ylmethoxy)-4-(4-methoxy-phenyl)-2-methyl-1,2,3,4-tetrahydro-isoquinoline; |
| **256A** | 7-(4-Isopropyl-morpholin-2R-ylmethoxy)-4-(4-methoxy-phenyl)-2-methyl-1,2,3,4-tetrahydro-isoquinoline (diastereomer 1); |
| **256B** | 7-(4-Isopropyl-morpholin-2R-ylmethoxy)-4-(4-methoxy-phenyl)-2-methyl-1,2,3,4-tetrahydro-isoquinoline (diastereomer 2); |
| **257** | 7-(4-Ethyl-morpholin-2-ylmethoxy)-4-(4-methoxy-phenyl)-2-methyl-1,2,3,4-tetrahydro-isoquinoline; |
| **258** | 7-[4-(2-Fluoro-ethyl)-morpholin-2-ylmethoxy]-4-(4-methoxy-phenyl)-2-methyl-1,2,3,4-tetrahydro-isoquinoline; |
| **259** | 7-(4-Cyclopropyl-morpholin-2-ylmethoxy)-4-(4-methoxy-phenyl)-2-methyl-1,2,3,4-tetrahydro-isoquinoline; |
| **260** | 7-(4-Cyclopropyl-morpholin-2S-ylmethoxy)-4-(4-methoxy-phenyl)-2-methyl-1,2,3,4-tetrahydro-isoquinoline; |
| **260A** | 7-(4-Cyclopropyl-morpholin-2S-ylmethoxy)-4-(4-methoxy-phenyl)-2-methyl-1,2,3,4-tetrahydro-isoquinoline (diastereomer 1); |
| **260B** | 7-(4-Cyclopropyl-morpholin-2S-ylmethoxy)-4-(4-methoxy-phenyl)-2-methyl-1,2,3,4-tetrahydro-isoquinoline (diastereomer 2); |
| **261** | 7-(4-Cyclopropyl-morpholin-2R-ylmethoxy)-4-(4-methoxy-phenyl)-2-methyl-1,2,3,4-tetrahydro-isoquinoline; |
| **261A** | 7-(4-Cyclopropyl-morpholin-2R-ylmethoxy)-4-(4-methoxy-phenyl)-2-methyl-1,2,3,4-tetrahydro-isoquinoline (diastereomer 1); |
| **261B** | 7-(4-Cyclopropyl-morpholin-2R-ylmethoxy)-4-(4-methoxy-phenyl)-2-methyl-1,2,3,4-tetrahydro-isoquinoline (diastereomer 2); |
| **262** | 7-(4-Isopropyl-morpholin-2-ylmethoxy)-2-methyl-4-(4-methylsulfanyl-phenyl)-1,2,3,4-tetrahydro-isoquinoline; |
| **263** | 7-(4-Cyclopropyl-morpholin-2-ylmethoxy)-2-methyl-4-(4-methylsulfanyl-phenyl)-1,2,3,4-tetrahydro-isoquinoline; |
| **264** | 2-Methyl-4-(4-methylsulfanyl-phenyl)-7-(morpholin-2-ylmethoxy)-1,2,3,4-tetrahydro-isoquinoline; |
| **265** | 4-(4-Methoxy-phenyl)-2,6-dimethyl-7-(3-piperdin-1-yl-propoxy)-1,2,3,4-tetrahydro-isoquinoline; |
| **266** | 4-(4-Methoxy-phenyl)-2,6-dimethyl-7-(3-morpholin-4-yl-propoxy)-1,2,3,4-tetrahydro-isoquinoline; |
| **267** | 4-(3-Fluoro-4-methoxy-phenyl)-2,6-dimethyl-7-(3-morpholin-4-yl-propoxy)-1,2,3,4-tetrahydro-isoquinoline; |
| **268** | 4-(3-Fluoro-4-methoxy-phenyl)-2,8-dimethyl-7-(3-morpholin-4-yl-propoxy)-1,2,3,4-tetrahydro-isoquiholine; |
| **269** | 4-(4-Methoxy-phenyl)-2,8-dimethyl-7-(3-morpholin-4-yl-propoxy)-1,2,3,4-tetrahydro-isoquinoline; |
| **270** | 2,6-Dimethyl-4-(4-methylsulfanyl-phenyl)-7-(3-piperidin-1-yl-propoxy)-1,2,3,4-tetrahydro-isoquinoline; |
| **271** | 7-(4-Piperidin-1-yl-but-1-ynyl)-4-pyridin-3-yl-1,2,3,4-tetrahydro-isoquinoline; |
| **272** | 7-(4-Piperidin-1-yl-butyl)-4-pyridin-3-yl-1,2,3,4-tetrahydro-isoquinoline; |
| **273** | 2-Methyl-7-(4-piperidin-1-yl-butyl)-4-pyridin-3-yl-1,2,3,4-tetrahydro-isoquinoline; |
| **274** | 7-[4-(4,4-Difluoro-piperidin-1-yl)-but-1-ynyl]-4-(4-methoxy-phenyl)-2-methyl-1,2,3,4-tetrahydro-isoquinoline; |
| **275** | 4-(4-Methoxy-phenyl)-2-methyl-7-(4-piperidin-1-yl-but-1-ynyl)-1,2,3,4-tetrahydro-isoquinoline; |
| **276** | 4-(4-Methoxy-phenyl)-2-methyl-7-(4-morpholin-4-yl-but-1-ynyl)-1,2,3,4-tetrahydro-isoquinoline; |
| **277** | 4-(4-Methoxy-phenyl)-2-methyl-7-(4-thiomorpholin-4-yl-but-1-ynyl)- 1,2,3,4-tetrahydro-isoquinoline; |
| **278** | 7-[4-(4-Isopropyl-piperazin-1-yl)-but-1-ynyl]-4-(4-methoxy-phenyl)-2-methyl-1,2,3,4-tetrahydro-isoquinoline; |
| **279** | 4-(4-Fluoro-phenyl)-2-methyl-7-(4-piperidin-1-yl-but-1-ynyl)-1,2,3,4-tetrahydro-isoquinotine; |
| **280** | 7-[4-(4,4-Difluoro-piperidin-1-yl)-butyl]-4-(4-methoxy-phenyl)-2-methyl-1,2,3,4-tetrahydro-isoquinoline; |
| **281** | 4-(4-Methoxy-phenyl)-2-methyl-7-(4-morpholin-4-yl-butyl)-1,2,3,4-tetrahydro-isoquinoline; |
| **282** | 4-(4-Methoxy-phenyl)-2-methyl-7-(4-thiomorpholin-4-yl-butyl)-1,2,3,4-tetrahydro-isoquinoline; |
| **283** | 7-[4-(4-Isopropyl-piperazin-1-yl)-butyl]-4-(4-methoxy-phenyl)-2-methyl-1,2,3,4-tetrahydro-isoquinoline; |

and salts thereof.

The features and advantages of the invention are apparent to one of ordinary skill in the art. Based on this disclosure, including the summary, detailed description, background, examples, and claims, one of ordinary skill in the art will be able to make modifications and adaptations to various conditions and usages. Where chemical symbols are used, it is understood that they are read from left to right, and that otherwise their spatial orientation has no significance.

The compounds as described above may be made according to processes within the skill of the art and/or that are described in the schemes and examples that follow. To obtain the various compounds herein, starting materials may be employed that carry the ultimately desired substituents though the reaction scheme with or without protection as appropriate. This may be achieved by means of conventional protecting groups, such as those described in "protective Groups in Organic Chemistry", ed. J.F.W. McOmie, Plenum Press, 1973; and T.W. Greene & P.G.M. Wuts, "Protective Groups in Organic Synthesis", 3rd ed., John Wiley & Sons, 1999. The protecting groups may be removed at a convenient subsequent stage using methods known from the art. Alternatively, it may be necessary to employ, in the place of the ultimately desired substituent, a suitable group that may be carried through the reaction scheme and replaced as appropriate with the desired substituent. Such compounds, precursors, or prodrugs are also within the scope of the invention. Reactions may be performed between the melting point and the reflux temperature of the solvent, and preferably between 0 °C and the reflux temperature of the solvent.

**Table of Acronyms and Abbreviations**

| **Term** | **Acronym or Abbreviation** |
|---|---|
| n-Butanol | n-BuOH |
| 1,2-Dichloroethane | DCE |
| Dichloromethane | DCM |
| Diisopropylethylamine | DIPEA |
| Ethylene glycol dimethyl ether | DME |
| N,N-Dimethylformamide | DMF |
| Ethyl acetate | EtOAc |
| Ethanol | EtOH |
| Methanol | MeOH |
| Triethylamine | TEA |
| Tetrahydrofuran | THF |

The tetrahydroisoquinoline compounds of Formulae (I) and (II) may be prepared by a humber of reaction schemes. Access to compounds of Formulae (I) and (II) is described in Schemes A-C. Persons skilled in the art will recognize that certain compounds are more advantageously produced by one scheme as compared to the other. Additional applicable methodologies are described in U.S. Patent Appl. No. 60/691,958 (June 17, 2005) and U.S. Patent Appl. No. 60/692,003 (June 17, 2005).

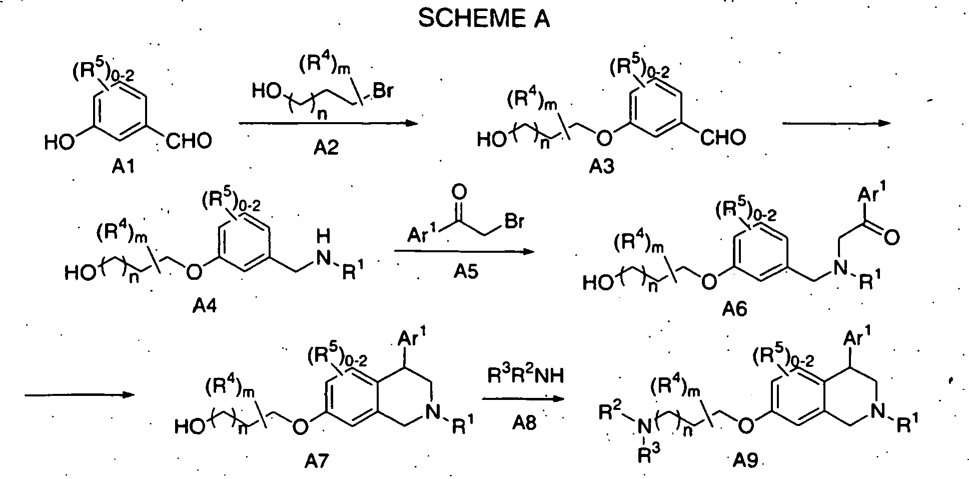

Referring to Scheme A, reagents of formulae A1, A2, and A5 are commercially, available, or are prepared according to known methods. 3-Hydroxybenzaldehyde derivatives A1 are reacted with alcohols A2 according to a Williamson ether synthesis protocol to form ethers A3, using a suitable base such as K₂CO₃, Na₂CO₃, or NaH, in a solvent such as acetonitrile, with or without catalytic Kl or Nal. Alternatively; ethers of formula A3 may be prepared under Mitsunobu conditions where A2 contains a protected hydroxyl in place of the bromide substituent. Reductive amination of the aldehyde functionality of compounds A3 will provide compounds of formula A4. The aldehyde can be treated with a suitable R¹-containing amine, with or without the addition of an activating agent such as a protic or Lewis acid, and with an appropriate reducing agent such as NaBH₄, NaCNBH₃, or NaB(OAc)₃H. Preferred conditions include NaBH₄ in methanol. Alkylation of amines A4 with alpha-haloketones A5 to form ketones A6 is accomplished in the presence of a tertiary amine base such as TEA or DIPEA, in a suitable solvent such as THF or DCM. Cyclization to generate tetrahydroisoquinolines A7 involves effecting cyclization to a tetrahydroisoquinolinium salt by exposure to a suitable protic or Lewis acid, such as methanesulfonic acid (MSA), trifluoroacetic acid (TFA), AlCl₃, TiCl₄, or BF₃. OEt₂ with or without a solvent such as DCM. Preferred conditions are neat MSA or MSA in DCM. The intermediate salt may be reduced using standard reducing agents such as NaCNBH₃ in an acidic methanol medium. Alternatively, ketones A6 may first be reduced by known methods, including NaBH₄, to their corresponding alcohols. Treatment of the intermediate alcohols with MSA in DCM provides cyclic species A7. Finally, the pendant primary alcohol group in compounds A7 may be converted to the corresponding amines A9 by activation to form an appropriate leaving group (such as a mesylate or bromide), followed by displacement of the leaving group with suitable amine A8. The displacement may be performed using a suitable base such as Na₂CO₃, in a polar solvent such as n-BuOH, with or without catalytic KI or Nal. Alternatively, amines A9 may be prepared through oxidation of the alcohol and reductive amination of the resulting aldehyde.

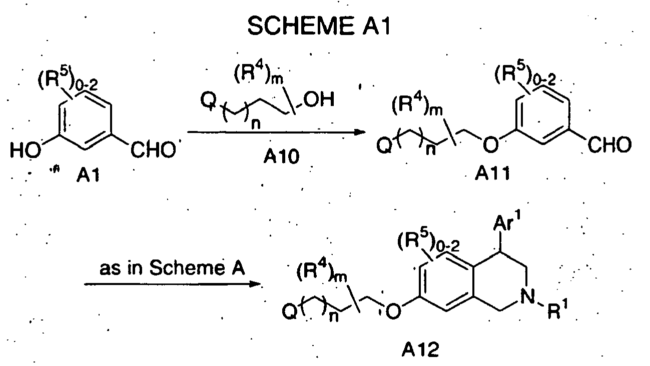

Referring to Scheme A1, benzaldehydes A1 may alternately be alkylated under, for example, Mitsunobu conditions, with suitable alcohols A10, where Q is -NR²R³ or a protected amino group or surrogate. Ethers A11 may be processed into compounds A12 as described in Scheme A.

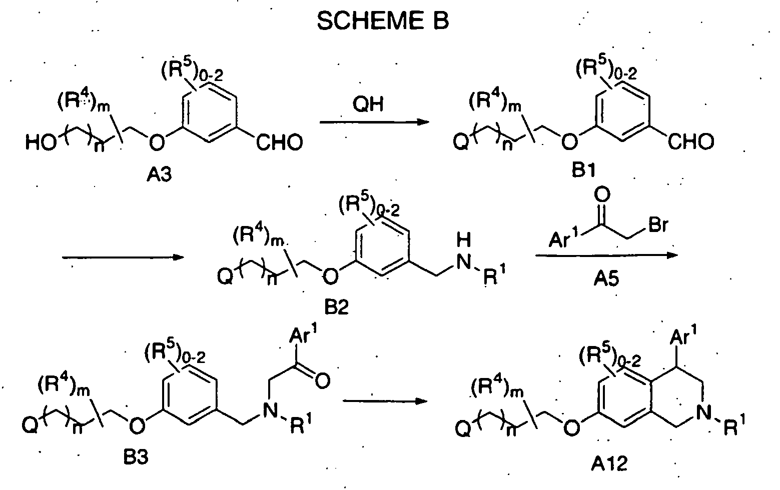

Referring to Scheme B, ethers of formula A3 may first be converted as described in Scheme A to the corresponding optionally protected amines B1. Benzaldehydes B1 may then be transformed into diamines B2 using reductive amination protocols as in Scheme A. Alkylation to form ketones B3, and cyclization to produce compounds of formula A12 are accomplished as shown for Scheme A.

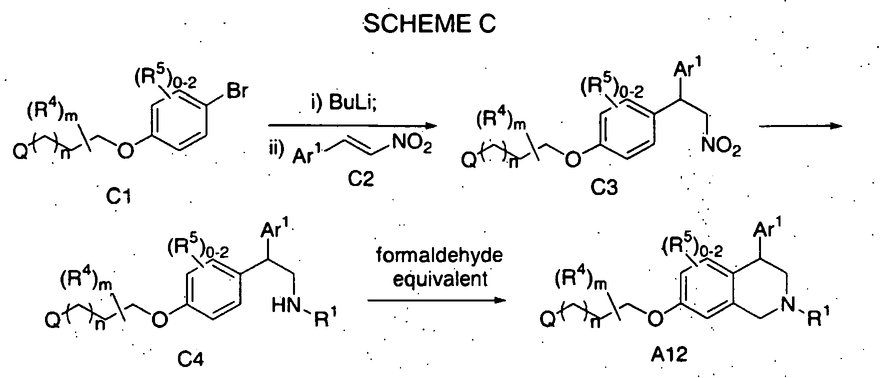

Referring to Scheme C, compounds of formula A12 are alternately prepared using a Pictet-Spengler protocol. Intermediates of formula C1 may be prepared by a variety of methods including those described in Schemes A and B. Halogen-metal exchange of the aryl bromides C1, followed by reaction with nitroalkenes C2 will provide compounds of formula C3. Reduction of the nitro group to an amine using procedures well-known to those skilled in the art, and subsequent alkylation or reductive amination will give rise to amines C4. Reaction of amines C4 with a formaldehyde equivalent such as formaldehyde, formic acid, or a formaldehyde equivalent, under Pictet-Spengler conditions, leads to formation of the tetrahydroisoquinoline system of compounds A12.

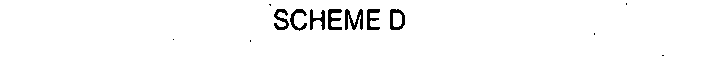

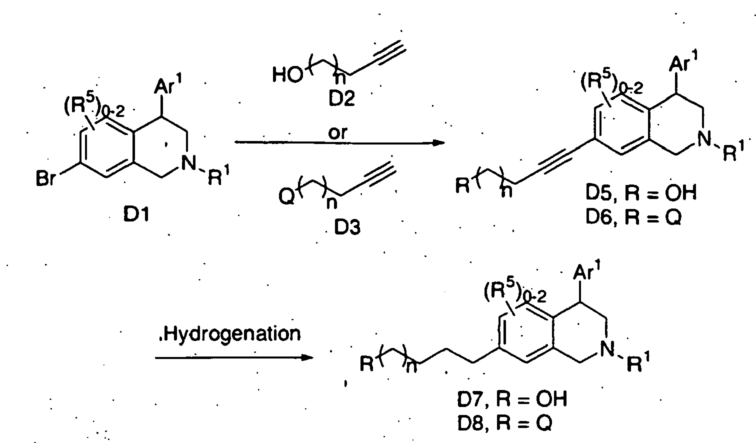

Referring to Scheme D, aryl bromides D1 are available according to the methods described in Schemes A-C. Palladium coupling with suitable alkynes such as functionalized alkynes D2 (optionally hydroxyl protected) or D3 is accomplished in the presence of a palladium catalyst such as PdCl₂(PPh₃)₂ or Pd(PPh₃)₄, a phosphine ligand such as PPh3, optional additives such as copper(I) iodide and diethylamine, in a solvent such as DMF or DME, at temperatures between room temperature and the reflux temperature of the solvent, or using a high pressure reaction vessel. Where alkynes D5 are produced, they may be converted into compounds of Formula (I) according to Scheme A. Alkynes D5 or D6 may be hydrogenated in the presence of a palladium catalyst, such as Pd/BaSO₄ or Pd(OH)₂, in a solvent such as methanol or ethanol, to give alkanes D7 and D8. Alkanes D7 may be converted into compounds of Formula (I) according to Scheme A.

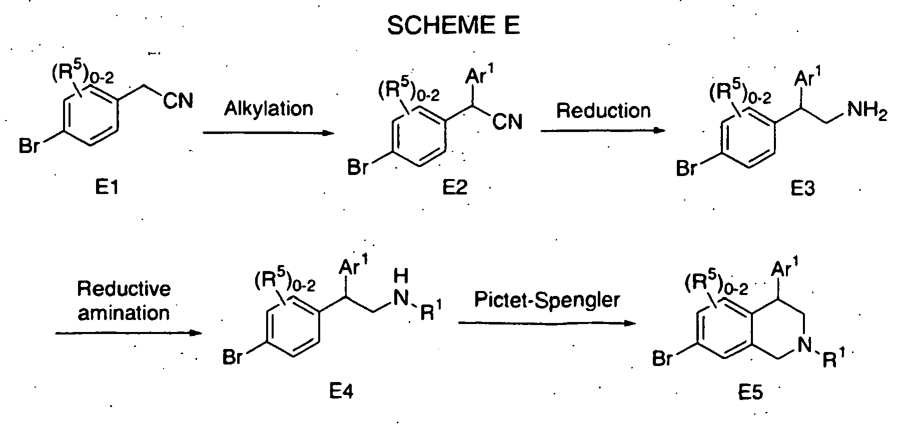

Compounds of Formula (I) may also be prepared according to Scheme E. Aryl bromides E1, which are commercially available or readily available using known methods, are alkylated by nucleophilic aromatic substitution by a) treatment of E1 with a strong base such as NaH or BuLi, in a solvent such as THF, at temperatures between 0 ºC and the reflux temperature of the solvent; and b) treatment of the resulting anion with a suitable electrophilic Ar¹-F or Ar¹-Cl reagent, such as 1-fluoro-4-nitro-benzene, 2-chloropyridine, or 4-fluoropyridine. Reduction of nitriles E2 to amines E3 may be accomplished using a suitable reducing agent, such as hydrogen with a catalyst, LiAlH₄, or NaBH₄ (activated with I₂ or Raney nickel), in a solvent such as THF, at temperatures ranging from room temperature to the reflux temperature of the solvent. Amines E3 may be protected as a C₁₋₄alkyl carbamate, such as a methyl carbamate (not shown), or may be alkylated using reductive amination-protocols to give amines E4. Amines E3, their carbamate-protected analogs, or amines E4 each are suitable reagents for the Pictet-Spengler cyclization, which is accomplished as described in Scheme C. Cyclized products E5 may be transformed into compounds of Formula (I) using methods described in the preceeding schemes, particularly Schemes A and D.

For each scheme above, where group Q is -NR²R³, compounds A12, D6, and D8 are within the scope of Formula (I). Where group Q is a protected amino group or surrogate, one skilled in the art will recognize that Q may be transformed into -NR²R³ using general deprotection methods, optionally followed by alkylation or reductive amination, at any of several points in the synthetic sequence.

Compounds prepared according to the schemes described above may be obtained as single enantiomers, diastereomers, or regioisomers, or as racemic mixtures or mixtures of enantiomers, diastereomers, or regioisomers. Where regioisomeric or diastereomeric mixtures are obtained, isomers may be separated using conventional methods such as chromatography or crystallization. Where racemic (1:1) and non-racemic (not 1:1) mixtures of enantiomers are obtained, single enantiomers may be isolated using conventional separation methods known to one skilled in the art. Particularly useful separation methods may include chiral chromatography, recrystallization, diastereomeric salt formation, or derivatization into diastereomeric adducts followed by separation.

For therapeutic use, salts of the compounds of the present invention are those that are pharmaceutical acceptable. However, salts of acids and bases that are non-pharmaceutically acceptable may also find use, for example, in the preparation or purification of a pharmaceutically acceptable compound. All salts, whether pharmaceutical acceptable or not are included within the ambit of the present invention.

Pharmaceutically acceptable salts, esters, and amides of compounds according to the present invention refer to those salt, ester, and amide forms of the compounds of the present invention which would be apparent to the pharmaceutical chemist, *i.e*., those which are non-toxic and which would favorably affect the pharmacokinetic properties of said compounds of the present invention. Those compounds having favorable pharmacokinetic properties would be apparent to the pharmaceutical chemist, *i.e.,* those which are non-toxic and which possess such pharmacokinetic properties to provide sufficient palatability, absorption, distribution, metabolism and excretion. Other factors, more practical in nature, which are also important in the selection, are cost of raw materials, ease of crystallization, yield, stability, hygroscopicity and flowability of the resulting bulk drug.

Examples of acids that may be used in the preparation of pharmaceutically acceptable salts include the following: acetic acid, 2,2-dichloroacetic acid, acylated amino acids, adipic acid, alginic acid, ascorbic acid, L-aspartic acid, benzenesulfonic acid, benzoic acid, 4-acetamidobenzoic acid, boric acid, (+)-camphoric acid, camphorsulfonic acid, (+)-(1S)-camphor-10-sulfonic acid, capric acid, caproic acid, caprylic acid, cinnamic acid, citric acid, cyclamic acid, cyclohexanesulfamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucuronic acid, L-glutamic acid, α-oxo-glutaric acid, glycolic acid, hippuric acid, hydrobromic acid, hydrochloric acid, hydroiodic acid, (+)-L-lactic acid, (±)-DL-lactic acid, lactobionic acid, lauric acid, maleic acid, (-)-L-malic acid, malonic acid, (±)-DL-mandelic acid, methanesulfonic acid, naphthalene-2-sulfonic acid, naphthalene-1,5-disuifonic acid, 1-hydroxy-2-naphthoic acid, nicotinic acid, nitric acid, oleic acid, orotic acid, oxalic acid, palmitic acid, pamoic acid, perchloric acid, phosphoric acid, L-pyroglutamic acid, saccharic acid, salicylic acid, 4-amino-salicylic acid, sebacic acid, stearic acid, succinic acid, sulfuric acid, tannic acid, (+)-L-tartaric acid, thiocyanic acid, *p*-toluenesulfonic acid, undecylenic acid, and valeric acid.

Compounds of the present invention containing acidic protons may be converted into their therapeutically active non-toxic metal or amine addition salt forms by treatment with appropriate organic and inorganic bases. Appropriate base salt forms comprise, for example, the ammonium salts; the alkali and earth alkaline metal salts (e.g. lithium, sodium, potassium, magnesium, calcium salts, which may be prepared by treatment with, for example, magnesium hydroxide, calcium hydroxide, potassium hydroxide, zinc hydroxide, or sodium hydroxide); and amine salts made with organic bases (e.g. primary, secondary and tertiary aliphatic and aromatic amines such as L-arginine, benethamine, benzathine, choline, deanol, diethanolamine, diethylamine, dimethylamine, dipropylamine, diisopropylamine, 2-(diethylamino)-ethanol, ethanolamine, ethylamine, ethylenediamine, isopropylamine, N-methyl-glucamine, hydrabamine, 1*H-*imidazole, L-lysine, morpholine, 4-(2-hydroxyethyl)-morpholine, methylamine, piperidine, piperazine, propylamine, pyrrolidine, 1-(2-hydroxyethyl)-pyrrolidine, pyridine, quinuclicine, quinoline, isoquinoline, secondary amines, triethanolamine, trimethylamine, triethylamine, *N*-methyl-D-glucamine, 2-amino-2-(hydroxymethyl)-1,3-propanediol, and tromethamine). See, *e.g*., S.M. Berge, et al., "Pharmaceutical Salts", J. Pharm. Sci., 1977, 66:1-19, which is incorporated herein by reference.

Pharmaceutically acceptable esters and amides are those that are within a reasonable benefit/risk ratio, pharmacologically effective and suitable for contact with the tissues of patients without undue toxicity, irritation, or allergic response. Representative pharmaceutically acceptable amides of the invention include those derived from ammonia, primary C₁₋₆alkyl amines and secondary di(C₁₋₆alkyl) amines. Secondary amines include 5- or 6-membered heterocyclic or heteroaromatic ring moieties containing at least one nitrogen atom and optionally between 1 and 2 additional heteroatoms. Preferred amides are derived from ammonia, C₁₋₃alkyl primary amines, and di(C₁₋₂alkyl)amines.

Representative pharmaceutically acceptable esters of the invention include C₁₋₇alkyl, C₅₋₇cycloalkyl, phenyl, substituted phenyl, and phenylC₁₋₆alkyl- esters. Preferred esters include methyl esters. Furthermore, examples of suitable esters include such esters where one or more carboxyl substituents is replaced with *p*-methoxybenzyloxy-carbonyl, 2,4,6-trimethylbenzyloxycarbonyl, 9-anthryloxycarbonyl, CH₃SCH₂COO-, tetrahydrofur-2-yloxycarbonyl, tetrahydropyran-2-yloxy-carbonyl, fur-2-yloxycarbonyl, benzoylmethoxycarbonyl, *p*-nitrobenzyloxy-carboriyl, 4-pyridylmethoxycarbonyl, 2,2,2-trichloroethoxy-carbonyl, 2,2,2-tribromoethoxycarbonyl, t-butyloxycarbonyl, t-amyloxycarbonyl, diphenylmethoxycarbonyl, triphenylmethoxycarbonyl, adamantyloxycarbonyl, 2-benzyloxyphenyloxycarbonyl, 4-methylthiophenyloxycarbonyl, or tetrahydropyran-2-yloxycarbonyl.

The compounds of the present invention are modulators of the histamine H₃ receptor arid of the serotonin transporter, and as such, the compounds are useful in the treatment of histamine H₃ and serotonin-mediated disease states. Compounds of the present invention possess serotonin transporter and H₃ receptor modulating activity. As such modulators, the compounds may act as antagonists or agonists. The effect of an antagonist may also be produced by an inverse agonist.

The compounds of the present invention are useful for treating or preventing neurologic or CNS disorders including steep/wake and arousal/vigilance disorders (e.g. insomnia, jet lag, and disturbed sleep), attention deficit hyperactivity disorders (ADHD), attention-deficit disorders, learning and memory disorders, learning impairment, memory impairment, memory loss, cognitive dysfunction, migraine, neurogenic inflammation, dementia, mild cognitive impairment (pre-dementia), Alzheimer's disease, epilepsy, narcolepsy with or without associated cataplexy, cataplexy, disorders of sleep/wake homeostasis, idiopathic somnolence, excessive daytime sleepiness (EDS), circadian rhythym disorders, sleep/fatigue disorders, fatigue, drowsiness associated with sleep apnea, sleep impairment due to perimenopausal hormonal shifts, Parkinson's-related fatigue, MS-related fatigue, depression-retated fatigue, chemotherapy-induced fatigue, work-related fatigue, eating disorders, obesity, motion sickness, vertigo, schizophrenia, substance abuse, bipolar disorders, manic disorders, and depression. Said methods comprise the step of administering to a mammal suffering therefrom an effective amount of at least one compound of the present invention.

Particularly, as modulators of the histamine H₃ receptor and the serotonin transporter, the compounds of the present invention may be used in the treatment or prevention of depression, disturbed sleep, fatigue, lethargy, cognitive impairment, memory impairment, memory loss, learning impairment, and attention-deficit disorders.

The present invention also contemplates use for treating our preventing a disease or condition mediated by the histamine H₃ receptor and the serotonin transporter with a combination therapy, comprising administering at least one compound of "the present invention in combination with one or more therapeutic agents. Suitable therapeutic agents include: H₁ receptor antagonists, H₂ receptor antagonists, H₃ receptor antagonists, and neurotransmitter modulators such as serotonin-norepinephrine reuptake inhibitors, selective serotonin reuptake inhibitors (SSRIs), noradrenergic reuptake inhibitors, non-selective serotonin re-uptake inhibitors (NSSRIs), and modafinil. In a particular embodiment, a combination therapy method includes administering at least one compound of present invention and administering modafinil, for example, for the treatment of narcolepsy, excessive daytime sleepiness (EDS), Alzheimer's disease, depression, attention-deficit disorders, MS-related fatigue, post-anesthesia grogginess, cognitive impairment, schizophrenia, spasticity associated with cerebral palsy, age-related memory decline, idiopathic somnolence, or jet-lag

Compounds of the present invention may be administered in pharmaceutical compositions to treat patients (humans and other mammals) with disorders mediated by the H₃ receptor and serotonin transporter. Thus, the invention features pharmaceutical compositions containing at least one compound of the present invention and a pharmaceutically acceptable carrier. A composition of the invention may further include at least one other therapeutic agent (for example, a combination formulation or combination of differently formulated active agents for use in a combination therapy method).

The present invention also features methods of using or preparing or formulating such pharmaceutical compositions. The pharmaceutical compositions can be prepared using conventional pharmaceutical excipients and compounding techniques known to those skilled in the art of preparing dosage forms. It is anticipated that the compounds of the invention can be administered by oral, parenteral, rectal; topical, or ocular routes, or by inhalation. Preparations may also be designed to give slow release of the active ingredient. The preparation may be in the form of tablets, capsules, sachets, vials, powders, granules, lozenges, powders for reconstitution, liquid preparations, or suppositories. Preferably, compounds may be administered by intravenous infusion or tropical administration, but more preferably by oral administration.

For oral administration, the compounds of the invention can be provided in the form of tablets or capsules, or as a solution, emulsion, or suspension. Tablets for oral use may include the active ingredient mixed with pharmaceutically acceptable excipients such as inert diluents, disintegrating agents, binding agents, lubricating agents, sweetening agents, flavoring agents, coloring agents and preservatives agents. Suitable inert fillers include sodium and calcium carbonate, sodium and calcium phosphate, lactose, starch, sugar, glucose, methyl cellulose, magnesium stearate, mannitol, sorbitol, and the like; typical liquid oral excipients include ethanol, glycerol, water and the like. Starch, polyvinyl-pyrrolidone, sodium starch glycolate, microcrystalline cellulose, and alginic acid are suitable disintegrating agents. Binding agents may include starch and gelatin. The lubricating agent, if present, will generally be magnesium stearate, stearic acid or talc. If desired, the tablets may be coated with a material such as glyceryl monostearate or glyceryl distearate to delay absorption in the gastrointestinal tract, or may be coated with an enteric coating. Capsules.for oral use include hard gelatin capsules in which the active ingredient is mixed with a solid, semi-solid, or liquid diluent, and soft gelatin capsules wherein the active ingredient is mixed with water, an oil such as peanut oil or olive oil, liquid paraffin, a mixture of mono and di-glycerides of short chain fatty acids, polyethylene glycol 400, or propylene glycol.

Liquids for oral administration may be suspensions, solutions, emulsions or syrups or may be presented as a dry product for reconstitution with water or other suitable vehicles before use. Compositions of such liquid may contain pharmaceutically-acceptable excipients such as suspending agents (for example, sorbitol, methyl cellulose, sodium alginate, gelatin, hydroxyethylcellulose, carboxymethylcellulose, aluminium stearate gel and the like); non-aqueous vehicles, which include oils (for example, almond oil or fractionated coconut oil), propylene glycol, ethyl alcohol or water; preservatives (for example; methyl or propyl p-hydroxybenzoate or sorbic acid); wetting agents such as lecithin; and, if needed, flavoring or coloring agents.

The compounds of this invention may also be administered by non-oral routes. The compositions may be formulated for rectal administration as a suppository. For parenteral use, including intravenous, intramuscular, intraperitoneal, or subcutaneous routes, the compounds of the invention will generally be provided in sterile aqueous solutions or suspensions, buffered to an appropriate pH and isotonicity or in parenterally acceptable oil. Suitable aqueous vehicles include Ringer's solution and isotonic sodium chloride. Such forms will be presented in unit dose form such as ampules or disposable injection devices, in multi-dose forms such as vials from which the appropriate dose may be withdrawn, or in a solid form or pre-concentrate that can be used to prepare an injectable formulation. Another mode of administration of the compounds of the invention may utilize a patch formulation to affect transdermal delivery. The compounds of this invention may also be administered by inhalation, via the nasal or oral routes using a spray formulation consisting of the compound of the invention and a suitable carrier.

Methods are known in the art for determining effective doses for therapeutic and prophylactic purposes for the pharmaceutical compositions or the drug combinations of the present invention, whether or not formulated in the same composition. The specific dosage level required for any particular patient will depend on a number of factors, including severity of the condition being treated, the route of administration, and the weight of the patient. For therapeutic purposes, "effective dose" or "effective amount" refers to that amount of each active compound or pharmaceutical agent, alone or in combination, that elicits the biological or medicinal response in a tissue system, animal, or human that is being sought by a researcher, veterinarian, medical doctor, or other clinician, which includes alleviation of the symptoms of the disease or disorder being treated. For prophylactic purposes (i.e., inhibiting the onset or progression of a disorder); the term "effective dose" or "effective amount" refers to that amount of each active compound or pharmaceutical agent, alone or in combination, that inhibits in a subject the onset or progression of a disorder as being sought by a researcher, veterinarian, medical doctor, or other clinician, the delaying of which disorder is mediated, at least in part, by the modulation of the histamine H₃ receptor and/or the serotonin transporter. Thus, the present invention provides combinations of two or more drugs wherein, for example, (a) each drug is administered in an independently therapeutically or prophylactically effective amount; (b) at least one drug in the combination is administered in an amount that is sub-therapeutic or sub-prophylactic if administered alone, but is therapeutic or prophylactic when administered in combination with the second or additional drugs according to the invention; or (c) both drugs are administered in an amount that is sub-therapeutic or sub-prophylactic if administered alone, but are therapeutic or prophylactic when administered together. Combinations of three or more drugs are analogously possible. Methods of combination therapy include co-administration of a single formulation containing all active agents; essentially contemporaneous administration of more than one formulation; and administration of two or more active agents separately formulated.

It is anticipated that the daily dose (whether administered as a single dose or as divided doses) will be in the range 0.01 to 1000 mg per day, more usually from 1 to 500 mg per day, and most usually from 10 to 200 mg per day. Expressed as dosage per unit body weight, a typical dose will be expected to be between 0.0001 mg/kg and 15 mg/kg, especially between 0.01 mg/kg and 7 mg/kg, and most especially between 0.15 mg/kg and 2.5 mg/kg.

Preferably, oral doses range from about 0.05 to 200 mg/kg, daily, taken in 1 to 4 separate doses. Some compounds of the invention may be orally dosed in the range of about 0.05 to about 50 mg/kg daily, others may be dosed at 0.05 to about 20 mg/kg daily, while still others may be dosed at 0.1 to about 10 mg/kg daily. Infusion doses can range from about 1 to 1000 µg/kg/min of inhibitor, admixed with a pharmaceutical carrier over a period ranging from several minutes to several days. For topical administration compounds of the present invention may be mixed with a pharmaceutical carrier at a concentration of about 0.1% to about 10% of drug to vehicle.

### EXAMPLES

In order to illustrate the invention, the following examples are included. These examples do not limit the invention. They are only meant to suggest a method of practicing the invention. Those skilled in the art may find other methods of practicing the invention, which are obvious to them. However, those methods are deemed to be within the scope of this invention.

Where solutions or mixtures are "concentrated", they are typically concentrated under reduced pressure using a rotary evaporator.

Normal phase flash column chromatography (FCC) was typically performed with RediSep® silica gel columns using 2 M NH₃ in MeOH/DCM as eluent.

Preparative Reversed-Phase high performance liquid chromatography (HPLC) was typically performed using a Gilson® instrument with a YMC-Pack ODS-A, 5 µm, 75x30 mm column, a flow rate of 25 mL/min, detection at 220 and 254 nm, with a 15% to 99% acetonitrile/water/0.05% TFA gradient.

Analytical Reversed-Phase HPLC was typically performed using 1) a Hewlett Packard Series 1100 instrument with an Agilent ZORBAX® Bonus RP, 5 µm, 4.6x250 mm column; a flow rate of 1 mL/min, detection at 220 and 254 nm, with a 1% to 99% acetonitrile/water/0-05% TFA gradient; or 2) a Hewlett Packard HPLC instrument with an Agilent ZORBAX® Eclipse XDB-C8, 5 µm, 4.6x150 mm column, a flow rate of 1 mL/min, detection at 220 and 254 nm, with a 1% to 99% acetonitrile/water/0.05% TFA gradient.

Chiral chromatography was typically performed using the following methods. Preparative supercritical fluid chromatography (SFC) was performed using a Thar Technologies® instrument with a Chiracel AD, 10 µm, 250x20 mm column, a flow rate of 37 g/min, detection at 220 and 254 nm, a pressure of 150 bar, a temperature of 35 ºC, and an isocratic 30% isopropanol/70% CO₂ mobile phase. Analytical SFC was typically performed using a Jasco® instrument with a Chiracel AD, 10 µm, 250x4.6 mm column, a flow rate of 1 g/min, detection at 220 and 254 nm, a pressure of 150 bar, a temperature of 35 ºC, and an isocratic 30% isopropanol/70% CO₂ mobile phase. Chiral HPLC was performed using a a Chiracel AD-H, 21x250 mm, 5 µM (Chiral Technologies) column with a flow rate of 8 mL/min, and an eluent of 0.2% Et₂NH/EtOH.

Mass spectra were obtained on an Agilent series 1100 MSD using electrospray ionization (ESI) in either positive or negative modes as indicated. Calculated mass corresponds to the exact mass.

Thin-layer chromatography was performed using Merck silica gel 60 F₂₅₄ 2.5 cm x 7.5 cm 250 µm or 5.0 cm x 10.0 cm 250 µm pre-coated silica gel plates. Preparative thin-layer chromatography was performed using EM Science silica gel 60 F₂₅₄ 20 cm x 20 cm 0.5 mm pre-coated plates with a 20 cm x 4 cm concentrating zone.

NMR spectra were obtained on either a Bruker model DPX400 (400 MHz), DPX500 (500 MHz), DRX600 (600 MHz) spectrometer. The format of the ¹H NMR data below is: chemical shift in ppm down field of the tetramethylsilane reference (multiplicity, coupling constant J in Hz, integration).

Where a potential chiral center is designated with a solid bond (not bold or hashed), the structure is meant to refer to a racemic mixture, a mixture of enantiomers, or a single enantiomer as described. Where a single enantiomer is described without enantiomeric designation at the chiral center, it is understood that the absolute configuration of the single enantiomer is unknown.

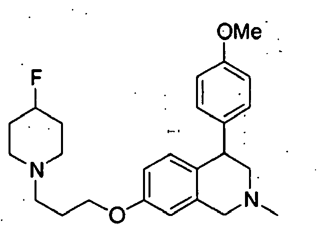

### Example 1 (racemic), 1A (enantiomer 1), and 1B (enantiomer 2); 7-[3-(4-Fluoro-piperidin-1-yl)-propoxy]-4-(4-methoxy-phenyl)-2-methyl-1,2,3,4-tetrahydro-isoquinoline.

Step 1; 3-(3-Hydroxy-propoxy)-benzaldehyde. To a mixture of 3-hydroxybenzaldehyde (40.0 g, 328 mmol) and K₂CO₃ (68.0 g, 491 mmol) in acetonitrile (650 mL) was added 3-bromo-propan-1-ol (54.6 g, 393 mmol) and the mixture was heated at reflux for 2 d. The mixture was allowed to cool to room temperature (rt), a white solid was removed by filtration, and the filtrate was concentrated. The crude material was purified by FCC to give the desired product as a clear oil (53.4 g, 90%). MS (ESI): mass calcd for C₁₀H₁₂O₃, 180.2; m/z found, 181 [M+H]⁺. ¹H NMR (CDCl₃): 9.95 (s, 1H), 7.46-7.41 (m, 2H), 7.39 (s, 1H), 7.18-7.15 (m, 1H), 4.17 (t, J = 6.0, 2H), 3.87-3.85 (m, 2H), 2.09-2.03 (m, 2H), 1.96-1.87 (m, 1H).

Step 2; 3-(3-Methylaminomethyl-phenoxy)-propan-1-ol. To a solution of 3-(3-hydroxy-propoxy)-benzaldehyde (30.0 g, 167 mmol) and aqueous MeNH₂ (40% wt, 27.2 mL, 350 mmol) in MeOH (330 mL) at 0 °C was added NaBH₄ (12.0 g, 316 mmol) portion-wise. The reaction mixture was stirred at 0 °C for 30 min. The ice-water bath was then removed and the reaction was stirred at rt overnight. The mixture was concentrated and the residue was stirred in 1 N NaOH (300 mL) for 2 h. The product was extracted with DCM and the combined organic layers were washed with brine, dried over Na₂SO₄, and concentrated to give the product as a clear oil (32.5 g, 100%). MS (ESI): mass calcd for C₁₁H₁₇NO₂, 195.2; m/z found, 196.4 [M+H]⁺. ¹H NMR (CDCl₃): 7.24-7.21 (m, 1H), 6.91-6.88 (m, 1H), 6.89 (d, J = 7.5, 1H), 6.80 (dd, J = 8.1, 2.1, 1H), 4.12 (t, J = 6.1, 2H), 3.82 (t, J = 5.9, 2H), 3.74 (5, 2H), 2.44 (s, 3H), 2.07-2.00 (m, 2H).

Step 3; 2-{[3-(3-Hydroxy-propoxy)-benzyl]-methyl-amino}-1-(4-methoxy-phenyl)-ethanone. To a solution of 3-(3-methylaminomethyl-phenoxy)-propan-1-ol (43.5 g, 166 mmol) and DIPEA (32.5 g, 250 mmol) in THF (415 mL) was added 2-bromo-4'-methoxy-acetophenone (40.0 g, 175 mmol). The mixture was stirred for 45 min at rt and then most of the THF was removed *in vacuo.* The mixture was diluted with water (200 mL) and extracted with DCM. The combined organic layers were washed with brine, dried over Na₂SO₄, and concentrated to give a light yellow oil (62 g) that was carried forward without purification. MS (ESI): mass calcd for C₂₀H₂₅NO₄, 343.42; m/z found, 344.5 [M+H]⁺.

Step 4; 7-(3-Hydroxy-propoxy)-4-(4-methoxy-phenyl)-2-methyl-isoquinolinium salt. 2-{[3-(3-Hydroxy-propoxy)-benzyl]-methyl-amino}-1-(4-methoxy-phenyl)-etharione (62 g, 181 mmol) was stirred in methanesulfonic acid (MSA, 60 mL) at 60 °C overnight. The mixture was poured into cold 1 N NaOH (500 mL), made basic with 6 N NaOH, and extracted with DCM (3x). The combined organic layers were washed with brine, dried over Na₂CO₃, and concentrated to give the desired product (62.3 g), which was carried forward without purification. MS (ESI): mass calcd for C₂₀H₂₂NO₃⁺, 324.39; m/z found, 324.5 [M+H]⁺.

Step 5; 3-[4-(4-Methoxy-phenyl)-2-methyl-1,2,3,4-tetrahydro-isoquinolin-7-yloxy]-propan-1-ol. To a solution of 7-(3-hydroxy-propoxy)-4-(4-methoxy-phenyl)-2-methyl-isoquinolinium salt. (2.5 g, 7.7 mmol) in MeOH (100 mL) was added NaCNBH₃ (1.5 g, 23.1 mmol) in one portion. Bromocresol green (3 mg, 0.004 mmol) was added followed by 1.25 M methanolic HCl until a color change from orange-brown or green to yellow was obtained. The mixture was stirred an additional 30 min with periodic addition of methanolic HCl to maintain a yellow color. The mixture was concentrated and the residue was diluted with water, made basic with 1 N NaOH, and extracted with DCM (3x). The combined organic layers were washed with brine, dried over Na₂CO₃, and concentrated. The crude product was purified by FCC to give the desired product as an orange semi-solid (1.2 g, 28% over 3 steps). MS (ESI): mass calcd for C₂₀H₂₅NO₃, 327.42; m/z found, 328.4 [M+H]⁺. ¹H NMR (acetone-d₆): 7.17 (d, J = 11.5, 2H), 6.92 (d, J = 11.5, 2H), 6.85-6.72 (m, 3H), 4.67-4.58 (m, 2H), 4.54 (br s, 1H), 4.09 (t, J = 5.3, 2H), 3.80 (s, 3H), 3.83-3.76 (m, 1H), 3.71 (t, J = 6.1, 2H), 3.46 (br s, 1H), 3.07 (s, 3H), 1.97-1.92 (m, 2H).

Step 6: Methanesulfonic acid 3-[4-(4-methoxy-phenyl)-2-methyl-1,2,3,4-tetrahydro-isoquinolin-7-yloxy]-propyl ester. Methanesulfonyl chloride (0.20 g, 1.75 mmol) was added dropwise to a solution of 3-[4-(4-methoxy-phenyl)-2-methyl-1,2,3,4-tetrahydro-isoquinolin-7-yloxy]-propan-1-ol (0.52 g, 1.59 mmol) and TEA (0.24 g, 2.38 mmol) in DCM (8 mL) at 0 °C. The mixture was brought to rt and stirred for 20 min. The mixture was then diluted with DCM (30 mL), washed with a satd. aq. NaHCO₃ (15 mL), water (15 mL), and brine (15 mL), dried over MgSO₄, and concentrated to give the crude product (0.60 g, 93%). MS (ESI): mass calcd for C₁₈H₂₆N₂O, 405.51; m/z found, 406.4 [M+H]⁺.

Step 7. A mixture of 4-fluoropiperidine hydrochloride (128 mg, 0.92 mmol) and sodium *t*-butoxide (60 mg, 0.62 mmol) in *n*-BuOH (1 mL) was added to a mixture of methanesulfonic acid 3-[4-(4-methoxy-phenyl)-2-methyl-1,2,3,4-tetrahydro-isoquinolin-7-yloxy]-propyl ester (250 mg, 0.62 mmol), Na₂CO₃ (98 mg, 0.92 mmol), and KI (5 mg, 0.031 mmol) in *n*-BuOH (1.2 mL) and the mixture was heated overnight at 50-80 °C. The mixture was cooled to rt, filtered, and the filtrate was concentrated. The crude product was purified by FCC and then reverse phase chromatography to give the desired product (98 mg, 25%) as a TFA salt. MS (ESI): mass calcd for C₂₅H₃₃FN₂O₂, 412.54; m/z found, 413.5 [M+H]⁺. ¹H NMR (acetone-d₆): 7.19 (d, J = 8.6, 2H), 6.95 (d, J = 8.5, 2H), 6.86-6.75 (m, 3H), 5.04 (d, J = 48, 1H), 4.71-4.56 (m, 3H), 4.12 (t, J = 4.9, 2H), 3.89-3.80 (m, 1H), 3.81 (s, 3H), 3.64-3.61 (m, 2H), 3.50 (br s, 1H), 3.42 (t, J = 7.2, 2H), 3.29-3.20 (m, 2H), 3.12 (s, 3H), 3.36-3.31 (m, 3H), 2.26-2.17 (m, 3H).

The enantiomers were separated (SFC HPLC) to provide Example 1A (first eluting) and Example 1 B (second eluting).

The following Examples 2-41 were prepared by a sequence similar to that described in Example 1.

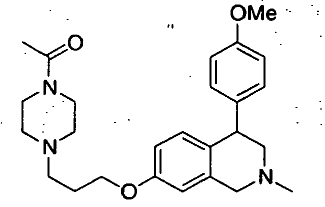

### Example 2; 1-(4-{3-[4-(4-Methoxy-phenyl)-2-methyl-1,2,3,4-tetrahydro-isoquinolin-7-yloxy]-propyl}-piperazin-1-yl)-ethanone.

Yield: 48.0 mg (7%) as the free base. MS (ESI): mass calcd for C₂₆H₃₅N₃O₃, 437.27; m/z found, 438.5 [M+H]⁺. ¹H NMR (acetone-d₆): 4.09 (d, J = 8.7; 2H), 6.82 (d, J = 8.7, 2H), 6.77 (d, 8.5, 1H), 6.64-6.56 (m, 2H), 4.17-4.14 (m, 1H), 3.98 (t, J = 6.3, 2H), 3.80 (s, 3H), 3.72-3.68 (m, 1H), 3.62 (t, J = 4.9, 1H), 3.58-3.54 (m, 1H), 3.46 (t, J = 4.9, 2H), 3.00-2.97 (m, 1H), 2.55-2.50 (m, 3H), 2.50-2.39 (m, 5H), 2.42 (s, 3H), 2.09 (s, 3H), 1.98-1.90 (m, 2H).

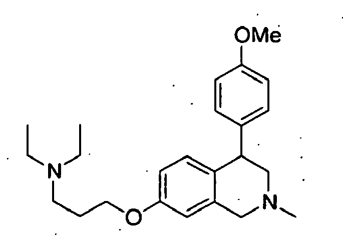

### Example 3; Diethyl-{3-[4-(4-methoxy-phenyl)-2-methyl-1,2,3,4-tetrahydro-isoquinolin-7-yloxy]-propyl}-amine.

Yield: 275.0 mg (30%) as a TFA salt. MS (ESI): mass calcd for C₂₄H₃₄N₂O₂, 382.26; m/z found, 383.5 [M+H]⁺. ¹H NMR (acetone-d₆): 7.20 (d, J = 8.6, 2H), 6.95 (d, J = 8.5, 2H), 6.88-6.75 (m, 3H), 3.72-4.48 (m, 3H), 4.16 (t, J = 5.6, 2H), 3.85-3.77 (m, 1H), 3.81 (s, 3H), 3.49-3.30 (m, 7H), 3.06 (s, 3H), 2.34-2.27 (m, 2H), 1.37 (t, J = 7.3, 6H).

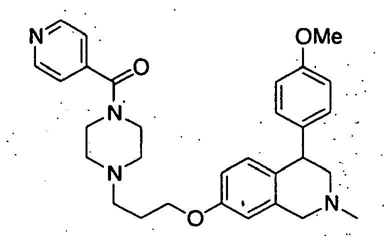

### Example 4; (4-{3-[4-(4-Methoxy-phenyl)-2-methyl-1,2,3,4-tetrahydro-isoquiriolin-7-yloxy]-propyl}-piperazin-1-yl)-pyridin-4-yl-methanone.

Yield: 58.6 mg (11%) as a TFA salt. MS (ESI): mass calcd for C₃₀H₃₆N₄O₃, 500.28; m/z found, 501.5 [M+H]⁺. ¹H NMR (acetone-d₆): 8.72-8.70 (m, 2H), 7.62-7.60 (m, 2H), 7.05 (d, J = 8.7, 2H), 6.80 (d, J = 8.5, 2H), 6.71-6.63 (m, 3H), 4.55-4.43 (m, 3H), 4.00 (t, J = 5.6, 2H), 3.81 (br s, 4H), 3.67 (s, 3H), 3.40-3.30 (m, 6H), 2.98 (s, 3H), 2.24-2.18 (m, 2H).

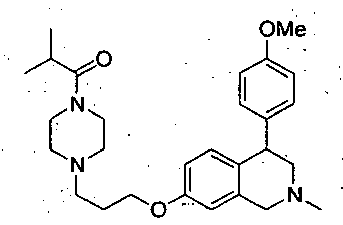

### Example 5; 1-(4-{3-[4-(4-Methoxy-phenyl)-2-methyl-1,2,3,4-tetrahydro-isoquinolin-7-yloxy]-propyl}-piperazin-1-yl)-2-methyl-propan-1-one.

Yield: 0.9 mg (5%) as a TFA salt. MS (ESI): mass calcd for C₂₈H₃₉N₃O₃, 465.63; m/z found, 466.6 [M+H]⁺. ¹H NMR (acetone-d₆): 7.05 (d, J = 8.4, 2H), 6.81 (d, J = 8.5, 2H), 6.72-6.62 (m, 3H), 4.55-4.40 (m, 3H), 3.00 (t, J = 5.0, 2H), 3.71-3.65 (m, 1H), 3.63 (s, 3H), 3.38-3.27 (m, 1H), 3.29 (t, J = 7.8, 2H), 2.99 (s, 3H), 2.85-2.79 (m, 1H), 2.24-2.18 (m, 2H), 0.97-0.90 (m, 6H).

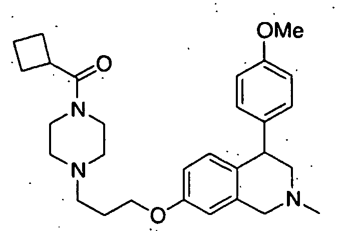

### Example 6; Cyclobutyl-(4-{3-[4-(4-methoxy-phenyl)-2-methyl-1,2,3,4-tetrahydro-isoquinolin-7-yloxy]-propyl}-piperazin-1-yl)-methanone.

Yield: 73.0 mg (17%) as a TFA salt. MS (ESI): mass calcd for C₂₉H₃₉N₃O₃, 477.64; m/z found, 478.6 [M+H]⁺. ¹H NMR (acetone-d₆): 7.05 (d, J = 8.6, 2H), 6.81 (d, J = 8.6, 2H), 6.71-6.63 (m, 3H), 4.54-4.38 (m, 3H), 4.00 (t, J = 4.6, 2H), 3.71-3.62 (m, 1H), 3.63 (s, 3H), 3.34-3.26 (m, 5H), 2.95 (s, 3H), 2.21-2.13 (m, 4H), 2.05-2.00 (m, 2H), 1.93-1.81 (m, 1H), 1.67-1.64 (m, 1H).

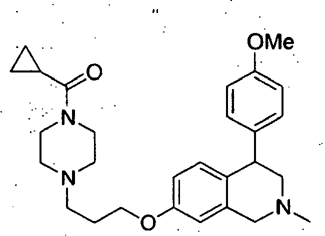

### Example 7; Cyclopropyl-(4-{3-[4-(4-methoxy-phenyl)-2-methyl-1,2,3,4-tetrahydro-isoquinolin-7-yloxy]-propyl}-piperazin-1-yl)-methanone.

Yield: 87.4 mg (20%) as a TFA salt. MS (ESI): mass calcd for C₂₈H₃₇N₃O₃, 477.64; m/z found, 478.6 [M+H]⁺. ¹H NMR (acetone-d₆): 7.05 (d, J = 8.5, 2H), 6.81 (d, 8.5, 2H), 6.72-6.61 (m, 3H), 4.56-4.41 (m, 4H), 4.01 (t, J = 5.2, 2H), 3.72-3.53 (m, 2H), 3.67 (s, 3H), 3.42-3.30 (m, 2H), 3.30 (t, J = 7.8, 2H), 2.99 (s, 3H), 2.25-2.17 (m, 2H), 1.88-1.82 (m, 1H), 0.71-.069 (m, 2H), 0.65-0.61 (m, 2H).

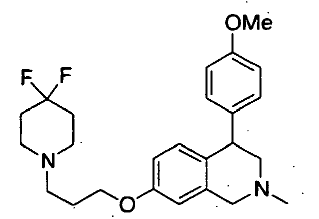

### Example 8; 7-[3-(4,4-Difluoro-piperidin-1-yl)-propoxy]-4-(4-methoxy-phenyl)-2-methyl-1,2,3,4-tetrahydro-isoquinoline.

Yield: 79.8 mg (20%) as a TFA salt. MS (ESI): mass calcd for C₂₅H₃₂F₂N₂O₂, 430.24; m/z found, 431.5 [M+H]⁺. ¹H NMR (acetone-d₆): 7.19 (d, J = 8.6, 2H), 6.95 (d, J = 8.6, 2H), 6.86-6.78 (m, 3H), 4.73-4.56 (m, 3H), 4.15 (t, J = 5.3, 2H), 3.85-3.77 (m, 2H), 3.81 (s, 3H), 3.50-3.43 (m, 3H), 3.43 (br s, 1H), 3.14 (s, 3H), 2.61-2.38 (m, 4H), 2.47-2.34 (m, 2H).

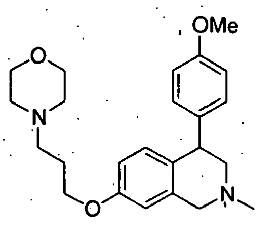

### Example 9; 4-(4-Methoxy-phenyl)-2-methyl-7-(3-morpholin-4-yl-propoxy)-1,2,3,4-tetrahydro-isoquinoline.

Yield: 86.0 mg (22%) as a TFA salt. MS (ESI): mass calcd for C₂₄H₃₂N₂O₃, 396.2; m/z found, 397.5 [M+H]⁴. ¹H NMR (acetone-d₆): 7.06 (d, J = 8.6, 2H), 6.81 (d, J = 8.6, 2H), 6.72-6.63 (m, 3H), 4.57-4.36 (m, 3H), 4.00 (t, J = 5.2, 2H), 3.93-3.87 (m, 2H), 3.84-3.75 (m, 2H), 3.72-3.67 (m, 1H), 3.67 (s, 3H), 3.53-3.46 (m, 2H), 3.33 (br s, 1H), 3.27 (t, J = 8.0, 2H), 3.10-3.01 (m, 2H), 2.96 (s, 3H), 2.22-2.16 (m, 2H).

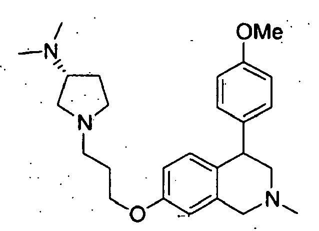

### Example 10; (1-{3-[4-(4-Methoxy-phenyl)-2-methyl-1,2,3,4-tetrahydro-isoquinolin-7-yloxy]-propyl}-pyrrolidin-3-yl)-dimethyl-amine.

Yield: 72.5 mg (18%) as a TFA salt. MS (ESI): mass calcd for C₂₆H₃₇N₂O₂, 423.29; m/z found, 424.6 [M+H]⁺. ¹H NMR (acetone-d₆): 7.18 (d, J = 8.6, 2H), 6.93 (d, J = 8.6, 2H), 6.84-6.73 (m, 3H), 4.65-4.43 (m, 3H), 4.31 (br s, 1H), 4.13 (t, J = 5.8, 2H), 4.10 (br s, 1H), 4.01 (br s, 1H), 3.80 (s, 3H), 3.80-3.76 (m, 1H), 3.72 (br s, 2H), 3.54-3.51 (m, 2H), 3.44 (br s, 1H), 2.70 (m, 1H), 3.06 (s, 3H), 3.04 (s, 6H), 2.60-2.56 (m, 1H), 2.32-2.27 (m, 2H).

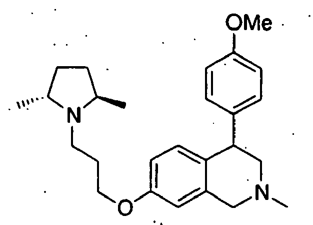

### Example 11; 7-[3-((2R,5R)-trans-Dimethyl-pyrrolidin-1-yl)-propoxy]-4-(4-methoxy-phenyl)-2-methyl-1,2,3,4-tetrahydro-isoquinoline.

Yield: 39.9 mg (10%) as a TFA salt. MS (ESI): mass calcd for C₂₆H₃₆N₂O₂, 408.28; m/z found, 409.5 [M+H]⁺. ¹H NMR (acetone-d₆): 7.19 (d, J = 8.6, 2H), 6.93 (d, J = 8.7, 2H), 6.88-6.73 (m, 3H), 4.66-4.44 (m, 3H), 4.18-4.09 (m, 3H), 3.80 (s, 3H), 3.76 (br s, 1H), 3.68-3.57 (m, 1H), 3.45-3.39 (m, 2H), 3.20-3.14 (m, 1H), 3.06 (s, 3H), 2.41-2.32 (m, 3H), 2.28-2.22 (m, 1H), 1.92-1.84 (m, 1H), 1.80-1.72 (m, 1H), 1.51-1.47 (m, 4H), 1.35-1.32 (m, 2H).

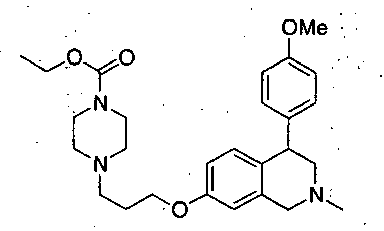

### Example 12; 4-{3-[4-(4-Methoxy-phenyl)-2-methyl-1,2,3,4-tetrahydro-isoquinolin-7-yloxy]-propyl}-piperazine-1-Carboxylic acid ethyl ester.

Yield: 60.8 mg (12%) as a TFA salt. MS (ESI): mass calcd for C₂₇H₃₇N₃O₄, 467.28; m/z found, 468.6 [M+H]⁺. ¹H NMR (acetone-d₆): 7.06 (d, J = 8.6, 2H), 6.81 (d, J = 8.6, 2H), 6.72-6.63 (m, 3H), 4.57-4.34 (m, 3H), 4.01-3.96 (m, 4H), 3.71-3.69 (m, 1H), 3.67 (s, 3H), 3.36-3.27 (m, 5H), 2.96 (s, 3H), 2.22-2.19 (m, 2H), 1.09 (t, J = 7.1, 3H).

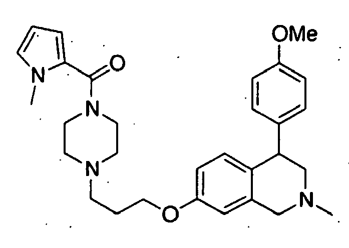

### Example 13; (4-{3-[4-(4-Methoxy-phenyl)-2-methyl-1,2,3,4-tetrahydro-isoquinolin-7-yloxy]-propyl}-piperazin-1-yl)-(1-methyl-1H-pyrrol-2-yl)-methanone.

Yield: 61.7 mg (12%) as a TFA salt. MS (ESI): mass calcd for C₃₀H₃₈N₄O₃, 502.29; m/z found, 503.6 [M+H]⁺. ¹H NMR (acetone-d₆): 7.06 (d, J = 8.6, 2H), 6.81 (d, J = 8.7, 2H), 6.74-6.72 (m, 2H), 6.69-6.61 (m, 2H), 6.32-6.30 (m, 1H), 5.93-5.91 (m, 1H), 4.53-4.40 (m, 4H), 4.02-4.00 (m, 2H), 3.68 (s, 4H), 3.63 (s, 4H), 3.35-3.27 (m, 5H), 2.93 (s, 3H), 2.24-2.19 (m, 2H).

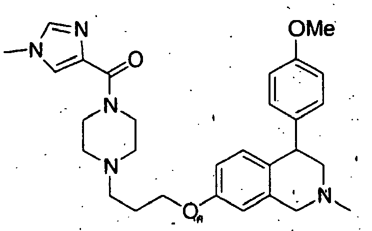

### Example 14; (4-{3-[4-(4-Methoxy-phenyl)-2-methyl-1,2,3,4-tetrahydro-isoquinolin-7-yloxy]-propyl}-piperazin-1-yl)-(1-methyl-1H-imidazol-4-yl)-methanone.

Yield: 17.0 mg (3%) as a TFA salt. MS (ESI): mass calcd for C₂₉H₃₇N₅O₃, 503.29; m/z found, 504.5 [M+H]⁺. ¹H NMR (acetone-d₆): 7.94 (br s, 1H), 7.69 (br s, 1H), 7.05 (d, J = 8.6, 2H), 6.81 (d, J = 8.4, 2H), 6.76-6.61 (m, 3H), 4.52-4.41 (m, 3H), 4.01-3.99 (m, 2H), 3.77 (s, 3H), 3.73-3.71 (m, 1H), 3.67 (s, 4H), 3.53-3.07 (m, 10H), 2.95 (s, 3H), 2.25-2.20 (m, 2H).

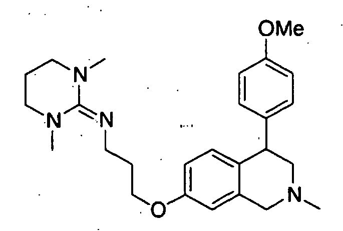

### Example 15; (1,3-Dimethyl-tetrahydro-pyrimidin-2-ylidene)-{3-[4-(4-methoxy-phenyl)-2-methyl-1,2,3,4-tetrahydro-isoquinolin-7-yloxy]-propyl}-amine.

Yield: 2.1 mg (0.5%) as a TFA salt. MS (ESI): mass calcd for C₂₆H₃₆N₄O₂, 436.28; m/z found, 437.5 [M+H]⁺. ¹H NMR (acetone-d₆): 7.18 (d, J = 8.6, 2H), 6.93 (d, J = 8.7, 2H), 6.81-6.76 (m, 3H), 4.65-4.41 (m, 3H), 4.13 (t, J = 5.3, 2H), 3.80 (s, 3H), 3.81 (br s, 1H), 3.45-3.38 (m, 7H), 3.26 (s, 1H), 3.05 (s, 3H), 2.95 (s, 3H), 2.48-2.45 (m, 1H), 2.34-2.27 (m, 2H), 2.07-2.02 (m, 3H).

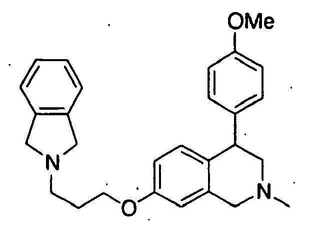

### Example 16; 7-[3-(1,3-Dihydro-isoindol-2-yl)-propoxy]-4-(4-methoxy-phenyl)-2-methyl-1,2,3,4-tetrahydro-isoquinoline.

Yield: 98 mg (20%) as a TFA salt. MS (ESI): mass calcd for C₂₈H₃₂N₂O₂, 428.25; m/z found, 429.5 [M+H]⁺. ¹H NMR (acetone-d₆): 7.33-7.23 (m, 4H), 7.06 (d, J = 8.2, 2H), 6.81 (d, J = 8.2, 2H), 6.75-6.62 (m, 3H), 4.94 (br s, 2H), 4.71-4.39 (m, 5H), 4.08-4.00 (m, 2H), 3.72-3.66 (m, 1H), 6.68 (s, 3H), 3.35 (br s, 1H), 3.00 (s, 3H), 2.32-2.24 (m, 2H), 1.83-1.80 (m, 2H).

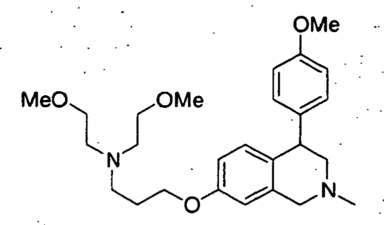

### Example 17; Bis-(2-methoxy-ethyl)-{3-[4-(4-methoxy-phenyl)-2-methyl-1,2,3,4-tetrahydro-isoquinolin-7-yloxy]-propyl}-amine.

Yield: 41.1 mg (8%) as a TFA salt. MS (ESI): mass calcd for C₂₆H₃₈N₂O₄, 442.28; m/z found, 443.5 [M+H]⁺. ¹H NMR (acetone-d₆): 7.06 (d, J = 8.5, 2H), 6.81 (d, J = 8.6, 2H), 6.74-6.63 (m, 3H), 4.59-4.31 (m, 3H), 4.04 (t, J = 5.0, 2H), 3.73-3.70 (m, 4H), 3.68 (s, 4H), 3.48-3.44 (m, 6H), 3.35 (br s, 1H), 3.21 (s, 6H), 2.93 (s, 3H), 2.25-2.19 (m, 2H).

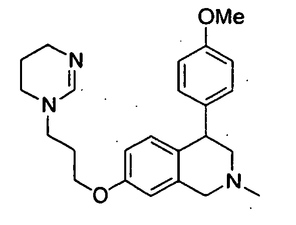

### Example 18; 7-[3-(5,6-Dihydro-4H-pyrimidin-1-yl)-propoxy]-4-(4-methoxy-phenyl)-2-methyl-1,2,3,4-tetrahydro-isoquinoline.

Yield: 3.8 mg (1%) as a TFA salt. MS (ESI): mass calcd for C₂₄H₃₁N₃O₂, 393.24; m/z found, 394.5 [M+H]⁺. ¹H NMR(acetone-d₆): 8.11-8.08 (m, 1H), 7.07 (d, J = 8.6, 2H), 6.81 (d, J = 8.7, 2H), 6.76-6.60 (m, 3H), 4.54-4.39 (m, 3H), 4.07-4.03 (m, 2H), 3.72-3.63 (m, 3H), 3.68 (s, 3H), 3.52-3.49 (m, 2H), 3.40-3.30 (m, 3H), 2.91 (s, 3H), 2.13-2.09 (m, 2H), 2.02-1.98 (m, 2H).

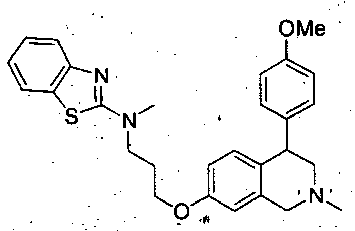

### Example 19; Benzothiazol-2-yl-{3-[4-(4-methoxy-phenyl)-2-methyl-1,2,3,4-tetrahydro-isoquinolin-7-yloxy]-propyl}-methyl-amine.

Yield: 3.2 mg (1%) as a TFA salt. MS (ESI): mass calcd for C₂₈H₃₁N₃O₂S, 473.21; m/z found, 474.5 [M+H]⁺. ¹H NMR (acetone-d₆): 7.85 (d, J = 8.1, 1H), 7.53 (d, J = 8.2, 1H), 7.43 (t, J = 7.4, 1H), 7.28 (t, J = 7.5, 1H), 7.06 (d, J = 8.6, 2H), 6.77 (d, J = 8.0, 2H), 6.72-6.58 (m, 3H), 4.58-4.46 (m, 4H), 4.35 (br s, 1H), 4.08 (t, J = 5.3, 2H), (m, 1H), 3.68 (s, 3H), 3.68-3.60 (m, 1H), 3.31 (br s, 1H), 3.06 (s, 2H), 2.93 (s, 3H), 2.25-2.18 (m, 2H).

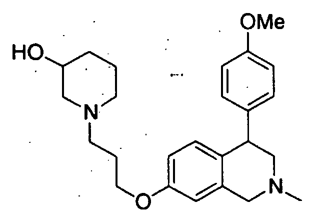

### Example 20; 1-{3-[4-(4-Methoxy-phenyl)-2-methyl-1,2,3,4-tetrahydro-isoquinolin-7-yloxy]-propyl}-piperidin-3-ol.

Yield: 65.0 mg (15%) as a TFA salt and mixture of diastereomers. MS (ESI): mass calcd for C₂₅H₃₄N₂O₃, 410.26; m/z found, 411.5 [M+H]⁺. ¹H NMR (acetone-d₆): 7.06 (d, J = 8.6, 2H), 6.81 (d, J = 8.5, 2H), 6.75-6.63 (m, 3H), 4.57-4.35 (m, 3H), 4.14 (br s, 0.5H), 4.06-3.98 (m, 2H), 3.97-3.87 (m, 0.5H), 3.68 (s, 3H), 3.69-3.63 (m, 1H), 3.61-3.58 (m, 1H), 3.52-3.43 (m, 1H), 3.40-3.22 (m, 3H), 3.19-3.14 (m, 0.5H), 3.05-2.96 (m, 0.5H), 2.95-2.93 (m, 3H), 2.79-2.73 (m, 0.5H), 2.60-2.53 (m, 0.5H), 2.25-2.14 (m, 3H), 1.83-1.59 (m, 3H), 1.33-1.25 (m, 0.5H).

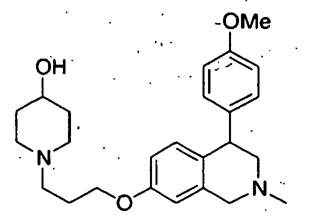

### Example 21; 1-{3-[4-(4-Methoxy-phenyl)-2-methyl-1,2,3,4-tetrahydro-isoquinolin-7-yloxy]-propyl}-piperidin-4-ol.

Yield: 36.0 mg (13%) as a TFA salt. MS (ESI): mass calcd for C₂₅H₃₄N₂O₃, 410.26; m/z fpund, 411.5 [M+H]⁺. ¹H NMR (acetone-d₆): 7.19 (d, J = 8.6, 2H), 6.93 (d, J = 8.6, 2H), 6.85-6.76 (m, 3H), 4.72-4.46 (m, 4H), 4.12-4.11 (m, 3H), 3.89-3.84 (m, 1H), 3.80 (s, 3H), 3.68-3.66 (m, 1H), 3.48-3.44 (m, 2H), 3.34-3.29 (m, 3H), 3.04 (s, 4H), 2.36-2.28 (m, 2H), 2.17-2.09 (m, 2H), 1.93-1.87 (m, 2H).

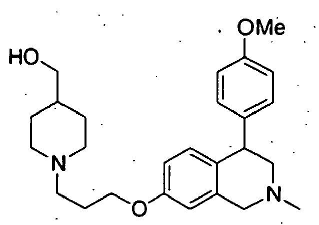

### Example 22; (1-{3-[4-(4-Methoxy-phenyl)-2-methyl-1,2,3,4-tetrahydro-isoquinolin-7-yloxy]-propyl}-piperidin-4-yl)-methanol.

Yield: 10.0 mg (13%) as a TFA salt. MS (ESI): mass calcd for C₂₆H₃₆N₂O₃, 424.27; m/z found, 425.5 [M+H]⁺. ¹H NMR (acetone-d₆): 7.19 (d, J = 8.6, 2H), 6.94 (d, J = 8.6, 2H), 6.85-6.75 (m, 3H), 4.66-4.45 (m, 3H), 4.13-4.10 (m, 2H), 3.80 (s, 3H), 3.82-3.78 (m, 1H), 3.75-3.68 (m, 2H), 3.48-3.43 (m, 4H), 3.38-3.34 (m, 2H), 3.06 (s, 3H), 3.05-2.94 (m, 2H), 2.34-2.30 (m, 2H), 2.01-1.96 (m, 2H), 1.33-1.28 (m, 1H), 1.76-1.63 (m, 2H).

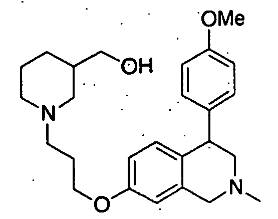

### Example 23; (1-{3-[4-(4-Methoxy-phenyl)-2-methyl-1,2,3,4-tetrahydro-isoquinolin-7-yloxy]-propyl}-piperidin-3-yl)-methanol.

Yield: 110.0 mg (20%) as a TFA salt. MS (ESI): mass calcd for C₂₆H₃₆N₂O₃, 424.27; m/z found, 425.5 [M+H]⁺. ¹H NMR (acetone-d₆): 7.07 (d, J = 8.6, 2H), 6.82 (d, J = 8.6, 2H), 6.74-6.63 (m, 3H), 4.57-4.36 (m, 4H), 4.04-4.00 (m, 2H), 3.69-3.62 (m, 1H), 3.69 (s, 3H), 3.60-3.55 (m, 2H), 3.48-3.45 (m, 1H), 3.35-3.31 (m, 2H), 3.25-3.20 (m, 2H), 2.96 (s, 3H), 2.82-2.73 (m, 1H), 2.67-2.58 (m, 1H), 2.24-2.19 (m, 2H), 2.11-2.02 (m, 1H); 1.85-1.82 (m, 2H), 1.72-1.69 (m, 1H), 1.22-1.87 (m, 1H).

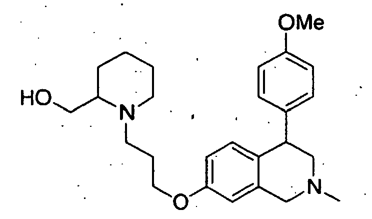

### Example 24; (1-{3-[4-(4-Methoxy-phenyl)-2-methyl-1,2,3,4-tetrahydro-isoquinolin-7-yloxy]-propyl}-piperidin-2-yl)-methanol.

Yield: 46.5 mg (8%) as a TFA salt. MS (ESI): mass calcd for C₂₆H₃₆N₂O₃, 424.27; m/z found, 425.5 [M+H]⁺. ¹H NMR (acetone-d₆): 7.17 (d, J = 8.5, 2H), 6.92 (d, J = 8.5, 2H), 6.80-6.71 (m, 3H), 4.67-4.47 (m, 3H), 4.16-4.11 (m, 2H), 4.05-3.99 (m, 1H), 3.79 (s, 3H), 3.83-3.75 (m, 2H), 3.68-3.62 (m, 2H), 3.53-3.33 (m, 3H), 3.15-3.21 (m, 1H), 3.06 (s, 3H), 2.41-2.32 (m, 2H), 2.07-2.00 (m, 1H), 1.93-1.78 (m, 5H), 1.65-1.54 (m, 1H).

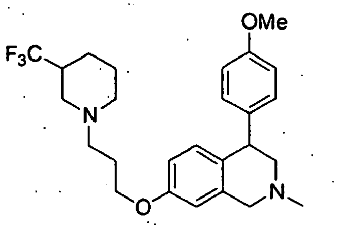

### Example 25; 4-(4-Methoxy-phenyl)-2-methyl-7-[3-(3-trifluoromethyl-piperldin-1-yl)-propoxy]-1,2,3,4-tetrahydro-isoquinoline.

Yield: 104.0 mg (18%) as a TFA salt. MS (ESI): mass calcd for C₂₆H₃₃F₃N₂O₂, 462.25; m/z found, 463.5 [M+H]⁺. ¹H NMR (acetone-d₆): 7.12 (d, J = 8.6, 2H), 6.88 (d, J = 8.6, 2H), 6.79-6.68 (m, 3H), 4.63-4.43 (m, 3H), 4.13-4.05 (m, 2H), 3.79-3.74 (m, 2H), 3.74 (s, 3H), 3.70-3.66 (m, 1H), 3.51-3.34 (m, 3H); 3.15-3.07 (m, 1H), 3.07-2.97 (m, 2H), 2.99 (s, 3H), 2.34-2.25 (m, 2H), 2.10-2.04 (m, 1H), 1.89-1.94 (m, 2H), 1.61-1.50 (m, 1H).

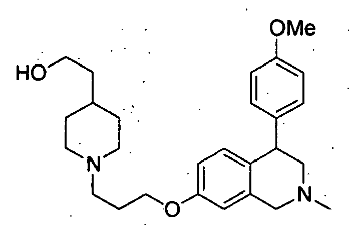

### Example 26; 2-(1-{3-[4-(4-Methoxy-phenyl)-2-methyl-1,2,3,4-tetrahydro-isoquinolin-7-yloxy]-propyl}-piperidin-4-yl)-ethanol.

Isolated as a TFA salt. MS (ESI): mass calcd for C₂₇H₃₈N₂O₃, 438.29; m/z found, 439.5 [M+H]⁺. ¹H NMR (acetone-d₆): 7.12 (d, J = 8.5, 2H), 6.87 (d, J = 8.5, 2H), 6.79-6.67 (m, 3H), 4.60-4.36 (m, 4H), 4.07-4.03 (m, 2H), 3.77-3.69 (m, 1H), 3.74 (s, 3H), 3.61-3.55 (m, 2H), 3.50 (t, J = 4.2, 2H), 3.41-3.34 (m, 2H), 3.27-3.23 (m, 2H), 2.99 (s, 3H), 2.95-2.76 (m, 2H), 2.26-2.17 (m, 1H), 1.99-1.85 (m, 2H), 1.75-1.69 (m, 1H), 1.64-1.56 (m, 2H), 1.44-1.39 (m, 2H).

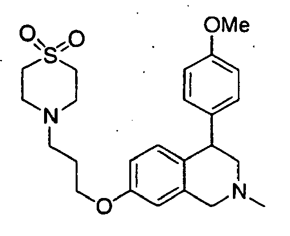

### Example 27; 7-[3-(1,1-Dioxo-1λ⁶-thiomorpholin-4-yl)-propoxy]-4-(4-methoxy-phenyl)-2-methyl-1,2,3,4-tetrahydro-isoquinoline.

Yield: 69.0 mg (12%),as a TFA salt. MS (ESI): mass calcd for C₂₄H₃₂N₂O₄S, 444.21; m/z found, 445.5 [M+H]⁺. ¹H NMR (acetone-d₆): 7.15 (d, J = 8.7, 2H), 6.90-(d, J = 8.6, 2H), 6.82=6.70 (m, 3H), 4.63-4.51 (m, 3H), 4.10 (t, J = 4.9, 2H), 3.81-3.74 (m, 1H), 3.77 (s, 3H), 3.65-3.62 (m, 4H), 3.47-3.40 (m, 5H), 3.29 (t, J = 7.6, 2H), 3.06 (s, 3H), 2.24-2.18 (m, 2H).

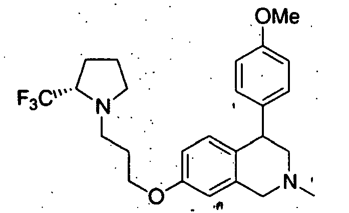

### Example 28; 4-(4-Methoxy-phenyl)-2-methyl-7-[3-((2S)-trifluoromethyl-pyrrolidin-1-yl)-propoxy]-1,2,3,4-tetrahydro-isoquinoline.

Yield: 2.5 mg (0.5%) as a TFA salt. MS (ESI): mass calcd for C₂₅H₃₁F₃N₂O₂, 448.23; m/z found, 449.5 [M+H]⁺. ¹H NMR (acetone-d₆): 7.15 (d, J = 8.6, 2H), 6.90 (d, J = 8.4, 2H), 6.81-6.70 (m, 3H), 4.78-4.57 (m, 4H), 4.03 (t, J = 6.1, 2H), 3.86-3.82 (m, 1H), 3.77 (s, 3H), 3.46 (br s, 1H), 3.41-3.30 (m, 1H), 3.36-3.19 (m, 1H), 3.10-3.04 (m, 1H), 3.10 (s, 3H), 2.82-2.71 (m, 1H), 2.51-2.42 (m, 1H), 1.96-1.91 (m, 2H), 1.90-1.72 (m, 3H).

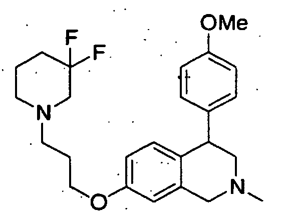

### Example 29 (racemic), 29A (enantiomer 1), and 29B (enantiomer 2); 7-[3-(3,3-Difluoro-piperidin-1-yl)-propoxy]-4-(4-methoxy-phenyl)-2-methyl-1,2,3,4-tetrahydro-isoquinoline.

Yield: 59.0 mg (11%) as a TFA salt. MS (ESI): mass calcd for C₂₅H₃₂F₂N₂O₂, 430.24; m/z found, 431.5 [M+H]⁺. ¹H NMR (acetone-d₆): 7.16 (d, J = 8.6, 2H), 6.92 (d, J = 8.5, 2H), 6.82-6.71 (m, 3H), 4.67-4.58 (m, 3H), 4.10 (t, J = 5.4, 2H), 3.84-3.75 (m, 1H), 3.78 (s, 3H), 3.66 (t, J = 10.9, 2H), 3.46-3.36 (m, 5H), 3.08 (s, 3H), 2.37-2.28 (m, 2H), 2.23-2.11 (m, 2H), 2.11-2.07 (m, 2H).

The enantiomers were separated (SFC HPLC) to provide Example 29A (first eluting) and Example 29B (second eluting).

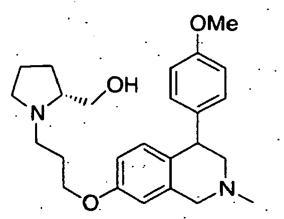

### Example 30; (1-{3-[4-(4-Methoxy-phenyl)-2-methyl-1,2,3,4-tetrahydro-isoquinolin-7-yloxy]-propyl}-pyrrolidin-(2R)-yl)-methanol.

Yield: 68.0 mg (12%) as a TFA salt. MS (ESI): mass calcd for C₂₅H₃₄N₂O₃, 410.26; m/z found, 411.5 [M+H]⁺. ¹H NMR (acetone-d₆): 7.19 (d, J = 8.6, 2H), 6.94 (d, J = 8.5, 2H), 6.86-6.77 (m, 3H), 4.79-4.53 (m, 5H), 4.16-4.11 (m, 2H), 3.91-3.69 (m, 9H), 3.48-3.37 (m, 2H), 3.34-3.29 (m, 1H), 3.09 (s, 3H), 2.39-2.24 (m, 3H), 2.12-2.05 (m, 1H), 1.96-1.89 (m, 1H).

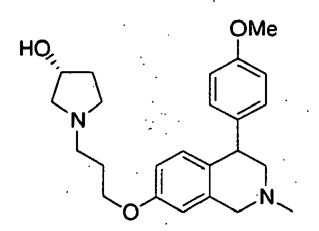

### Example 31; 1-{3-[4-(4-Methoxy-phenyl)-2-methyl-1,2,3,4-tetrahydro-isoquinolin-7-yloxy]-propyl}-(R)-pyrrolidin-3-ol.

Yield: 56.0 mg (10%) as a TFA salt. MS (ESI): mass calcd for C₂₄H₃₂N₂O₃, 396.24; m/z found, 397.5 [M+H]⁺. ¹H NMR (acetone-d₆): 7.18 (d, J = 8.6, 2H), 6.93 (d, J = 8.6, 2H), 6.85-6.75 (m; 3H), 4.64-4.51 (m, 4H), 4.35 (br s, 2H), 3.96-3.84 (m, 1H), 3.84-3.72 (m, 2H), 4.04 (s, 3H), 3.70-3.55 (m, 3H), 3.52-3.38 (m, 3H), 3.34-3.23 (m, 2H), 3.26 (s, 3H), 2.33-2.26 (m, 2H).

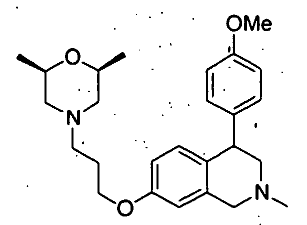

### Example 32; 7-[3-(2,6-Dimethyl-morpholin-4-yl)-propoxy]-4-(4-methoxy-phenyl)-2-methyl-1,2,3,4-tetrahydro-isoquinoline.

Yield: 118.0 mg (21 %) as a TFA salt. MS (ESI): mass calcd for C₂₆H₃₆N₂O₃, 424.27; m/z found, 425.5 [M+H]⁺. ¹H NMR (acetone-d₆): 7.19 (d, J = 8.6, 2H), 6.95 (d, J = 8.6, 2H), 6.85-6.75 (m, 3H), 4.76-4.51 (m, 3H), 4.14-4.11 (m, 2H), 4.06-3.99 (m, 2H), 3.85-3.77 (m, 1H), 3.81 (s, 3H), 3.60 (d, J = 11.8, 2H), 3.52-3.44 (m, 1H), 3.40-3.36 (m, 2H), 3.10 (s, 3H), 2.70 (t, J = 11.5, 2H), 2.37-2.31 (m, 2H), 1.25-1.18 (d, J = 6.3, 6H).

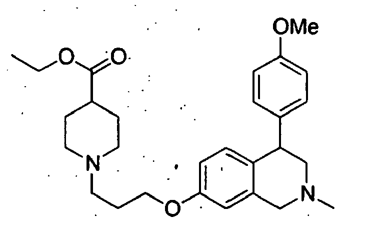

### Example 33; 1-{3-[4-(4-Methoxy-phenyl)-2-methyl-1,2,3,4-tetrahydro-isoquinolin-7-yloxy]-propyl}-piperidine-4-carboxylic acid ethyl ester.

Yield: 56.0 mg (9%) as a TFA salt. MS (ESI): mass calcd for C₂₈H₃₈N₂O₄, 466.28; m/z found, 467.5 [M+H]⁺. ¹H NMR (acetone-d₆): 7.19 (d, J = 8.5, 2H), 6.95 (d, J = 8.4, 2H), 6.86.6.75 (m, 3H), 4.68-4.54 (m, 3H), 4.20-4.12 (m, 4H), 3.87-3.74 (m, 5H), 3.75-3.61 (m, 1H), 3.52-3.43 (m, 1H), 3.38-3.31 (m, 2H), 3.14-3.06 (m, 5H), 2.73 (m, 1H), 2.36-2.30 (m, 2H), 2.24-2.16 (m, 3H), 2.10-2.02 (m, 1H), 1.26-1.18 (m, 3H).

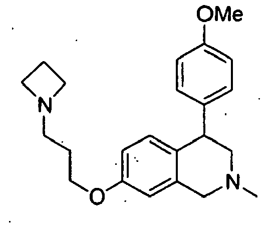

### Example 34; 7-(3-Azetidin-1-yl-propoxy)-4-(4-methoxy-phenyl)-2-methyl-1,2,3,4-tetrahydro-isoquinoline.

Yield: 2.0 mg (0.4%) as a TFA salt. MS (ESI): mass calcd for C₂₃H₃₀N₂O₂, 366.23; m/z found, 367.5 [M+H]⁺. ¹H NMR (acetone-d₆): 7.16 (d, J = 8.5, 2H), 6.90 (d, J = 8.6, 2H), 6.81-6.72 (m, 3H), 4.58-4.34 (m, 3H), 4.32-4.24 (m, 2H), 4.10-4.01 (m, 5H), 3.81 (s, 3H), 3.81-3.72 (m, 1H), 3.41-3.32 (m, 3H), 2.95 (s, 3H), 2.60-2.55 (m, 1H), 2.36 (br s, 1H), 2.09-2.03 (m, 1H).

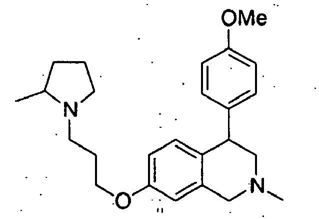

### Example 35; 4-(4-Methoxy-phenyl)-2-methyl-7-[3-(2-methyl-pyrrolidin-1-yl)-propoxy]-1,2,3,4-tetrahydro-isoquinoline.

Yield: 78.0 mg (25%) as a TFA salt. MS (ESI): mass calcd for C₂₅H₃₄N₂O₂, 394.26; m/z found, 395.5 [M+H]⁺. ¹H NMR (acetone-d₆): 7.16 (d, J = 8.6, 2H), 6.91 (d, J = 8.5, 2H), 6.83-6.70 (m, 3H), 4.65-4.45 (m, 4H), 4.78-4.10 (m, 2H), 3.92-3.88 (m, 1H), 3.80-3.73 (m, 1H), 3.77 (s, 3H), 3.63-3.57 (m, 1H), 3.53-3.39 (m, 2H), 3.22-3.18 (m, 2H), 3.07 (s, 3H), 2.32-2.27 (m, 3H), 2.09.2.03 (m, 2H), 1.83-1.73 (m, 1H), 1.49-1.46 (m, 2H).

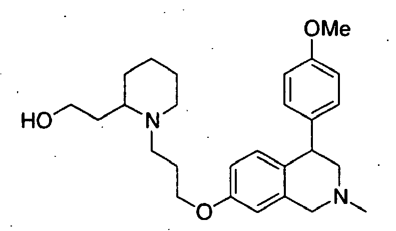

### Example 36; 2-(1-{3-[4-(4-Methoxy-phenyl)-2-methyl-1,2,3,4-tetrahydro-isoquinolin-7-yloxy]-propyl}-piperidin-2-yl)-ethanol.

Isolated as a TFA salt. MS (ESI): mass calcd for C₂₇H₃₈N₂O₃, 438.29; m/z found, 439.6 [M+H]⁺. ¹H NMR (acetone-d₆): 7.17 (d, J = 8.7, 2H), 6.88 (d, J = 8.7, 2H), 6.81-6.72 (m, 3H), 4.66-4.51 (m, 3H), 4.16-4.08 (m, 2H), 3.83-3.73 (m, 3H), 3.78 (s, 3H), 3.69-3.61 (m, 2H), 3.49-3.31 (m, 4H), 3.16 (br s, 1H), 3.06 (s, 3H), 2.33-2.28 (m, 2H), 2.22-2.12 (m, 1H), 2.07-2.03 (m, 1H), 1.93-178 (m, 5H), 1.65-1.58 (m, 1H).

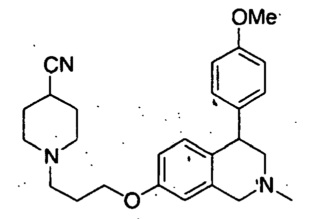

### Example 37 (racemic), 37A (enantiomer 1), and 37B (enantiomer 2); 1-{3-[4-(4-Methoxy-phenyl)-2-methyl-1,2,3,4-tetrahydro-isoquinolin-7-yloxy]-propyl}-piperidine-4-carbonitrile.

Yield: 75.2 mg (14%) as a TFA salt. MS (ESI): mass calcd for C₂₆H₃₃N₃O₂, 419.26; m/z found, 420.5 [M+H]⁺. ¹H NMR (acetone-d₆): 7.18 (d, J = 8.6, 2H), 6.93 (d, J = 8.6, 2H), 6.84-6.71 (m, 3H), 4.66-4.50 (m, 3H), 4.14-4.08 (m, 2H), 3.84-3.65 (m, 3H), 3.80 (s, 3H), 3.45-3.36 (m, 4H), 3.21-3.11 (m, 2H), 3.07 (s, 3H), 2.39-2.18 (m, 6H).

The enantiomers were separated (SFC HPLC) to provide Example 37A (first eluting) and Example 37B (second eluting).

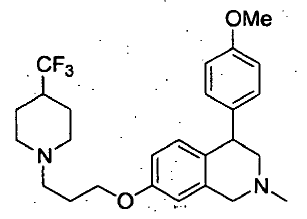

### Example 38; 4-(4-Methoxy-phenyl)-2-methyl-7-[3-(4-trifluoromethyl-piperidin-1-yl)-propoxy]-1,2,3,4-tetrahydro-isoquinoline.

Yield: 56.2 mg (9%) as a TFA salt. MS (ESI): mass calcd for C₂₆H₃₃F₃N₂O₂, 462.25; m/z found, 463.5 [M+H]⁺. ¹H NMR (acetone-d₆): 7.18 (d, J = 8.6, 2H), 6.93 (d, J = 8.7, 2H), 6.84-6.71 (m, 3H), 4.63-4.43 (m, 3H), 4.14-4.09 (m, 2H), 3.84-3.76 (m, 3H), 3.80 (s, 3H), 3.45-3.39 (m, 1H), 3.38-3.32 (m, 2H), 3.14-3.08 (m, 2H), 3.08 (s, 3H), 2.69-2.65 (m, 1H), 2.34-2.29 (m, 2H), 2.16-2.06 (m, 4H).

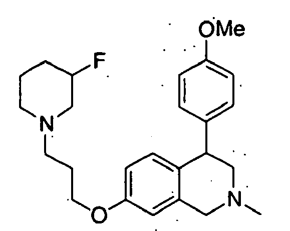

### Example 39; 7-[3-(3-Fluoro-piperidin-1-yl)-propoxy]-4-(4-methoxy-phenyl)-2-methyl-1,2,3,4-tetrahydro-isoquinoline.

Yield: 77.4 mg (14%) as a TFA salt. MS (ESI): mass calcd for C₂₅H₃₃FN₂O₂, 412.25; m/z found, 413.5 [M+H]⁺. ¹H NMR (acetone-d₆): 7.18 (d, J = 8.6, 2H), 6.94 (d, J = 8.6, 2H), 6.84-6.71 (m, 3H), 5.11 (br s, 1H), 4.63-4.51 (m, 3H), 4.13 (t, J = 4.3, 2H), 3.93 (br s, 1H), 3.84-3.76 (m, 2H), 3.80 (s, 3H), 3.50-3.41 (m, 4H), 3.22-3.14 (m, 1H), 3.12 (s, 3H), 2.35-2.30 (m, 2H), 2.29-2.12 (m, 2H), 1.92-1.82 (m, 2H).

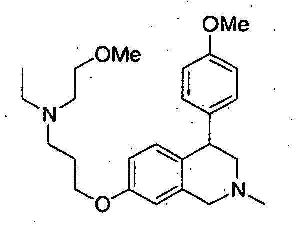

### Example 40; Ethyl-(2-methoxy-ethyl)-{3-[4-(4-methoxy-phenyl)-2-methyl-1,2,3,4-tetrahydro-isoquinolin-7-yloxy]-propyl}-amine.

Yield: 49.7 mg (9%) as a TFA salt. MS (ESI): mass calcd for C₂₅H₃₆N₂O₃, 412.27; m/z found, 413.5 [M+H]⁺. ¹H NMR (acetone-d₆): 7.18 (d, J = 8.6, 2H), 6.94 (d, J = 8.6, 2H), 6.86-6.71 (m, 3H), 4.67-4.51 (m, 3H), 4.14 (t, J = 5.1, 2H), 3.83 (t, J = 4.8, 2H), 3.76 (s, 4H), 3.52-3.39 (m, 7H), 3.34 (s, 3H), 3.06 (s, 3H), 2.33-2.28 (m, 2H), 1.37 (t, J = 7.3, 3H).

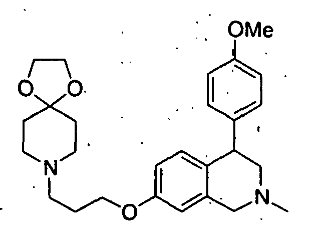

### Example 41; 7-[3-(1,4-Dioxa-8-aza-spiro[4.5]dec-8-yl)-propoxy]-4-(4-methoxy-phenyl)-2-methyl-1,2,3,4-tetrahydro-isoquinoline.

Yield: 61.2 mg (10%) as a TFA salt. MS (ESI): mass calcd for C₂₇H₃₆N₂O₄, 452.27; m/z found, 453.5 [M+H]⁺. ¹H NMR (acetone-d₆): 7.16 (d, J = 8.6, 2H), 6.91 (d, J = 8.5, 2H), 6.87-6.70 (m, 3H), 4.67-4.51 (m, 3H), 4.09 (t, J = 2.8, 2H), 4.01-3.91 (m, 1H), 3.97 (s, 3H), 3.82-3.74 (m, 1H), 3.77 (s, 3H), 3.68-3.63 (m, 2H), 3.50-3.35 (m, 3H), 3.19-3.12 (m, 2H), 3.06 (s, 3H), 2.32-2.26 (m, 2H), 2.19-2.13 (m, 2H), 1.91-1.85 (m, 2H).

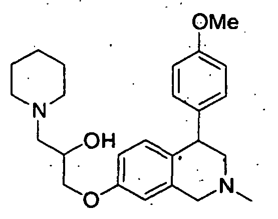

### Example 42; 1-[4-(4-Methoxy-phenyl)-2-methyl-1,2,3,4-tetrahydro-isoquinolin-7-yloxy]-3-piperidin-1-yl-propan-2-ol.

Step 1; 3-Oxiranylmethoxy-benzaldehyde. To a mixture of 3-hydroxybenzaldehyde (10.0 g, 81.9 mmol) and K₂CO₃ (17.0 g, 123.0 mmol) in acetonitrile (235 mL) was added epichlorohydrin (15.2 g, 163.8 mmol) and the mixture was heated at reflux overnight. The mixture was allowed to cool to rt, was diluted with EtOAc, washed with 1 N NaOH and brine, dried (MgSO₄), and concentrated. The crude material was purified by FCC (EtOAc/hexanes) to give the desired product as a clear oil (8.49 g, 58%). ¹H NMR (CDCl₃): 9.98 (s, 1H), 7.50-7.44 (m, 2H), 7.41-7.40 (m, 1H), 7.24-7.21 (m, 1H), 4.33 (dd, J=11.0, 2.8, 1H), 4.00-3.97 (m, 1H), 3.40-3.37 (m, 1H), 2.94-2.93 (m, 1H), 2.79-2.78 (m, 1H).

Step 2; 3-(2-Hydroxy-3-piperidin-1-yl-propoxy)-benzaldehyde. A mixture of 3-oxiranylmethoxy-benzaldehyde (0.59 g, 3.31 mmol) and piperidine (0.34 mL, 3.48 mmol) in EtOH (10 mL) was heated at reflux overnight. The reaction mixture was allowed to cool to rt and then was concentrated. The crude material was purified by FCC (MeOH/DCM) to give the desired product as a yellow oil (0.63 g, 72%). MS (ESI): mass calcd for C₁₅H₂₁NO₃, 263.15; m/z found, 264.4 [M+H]⁺. ¹H NMR (CDCl₃): 9.98 (s, 1H), 7.48-7.41 (m, 3H), 7.23-7.21 (m, 1H), 4.13-4.08 (m, 1H), 4.06-4.00 (m, 2H), 2.76-2.57 (m, 2H), 2.53-2.45 (m, 2H), 2.42-2.31 (m, 2H), 1.64-1.57 (m, 4H), 1.49-1.45 (m, 2H).

Step 3; 1-(3-Methylaminomethyl-phenoxy)-3-piperidin-1yl-propan-2-ol. Prepared in a similar manner as 3-(3-methylaminomethyl-phenoxy)-propan-1-ol to give the desired product as a clear oil (0.59 g, 89%). MS (ESI): mass calcd for C₁₆H₂₆N₂O₂, 278.20; m/z found, 279.5 [M+H]⁺. ¹H NMR (CDCl₃): 7.22 (t, J = 7.8, 1H), 6.91-6.88 (m, 2H), 6.83-6.80 (m, 1H), 4.09-4.05 (m, 1H), 3.98-3.97 (m, 2H), 3.72 (s, 2H), 2.65-2.56 (m, 2H), 2.51-2.45 (m, 2H), 2.45 (s, 3H), 2.42-2.32 (m, 2H), 1.65-1.54 (m, 5H), 1.48-1.43 (m, 2H).

Step 4; 2-{[3-(2-Hydroxy-3-piperidin-1-yl-propoxy)-benzyl]-methyl-amino}-1-(4-methoxy-phenyl)-ethanone. Prepared in a similar manner as 2-{[3-(3-hydroxy-propoxy)-benzyl]-methyl-amino}-1-(4-methoxy-phenyl)-ethanone to give the desired product as a yellow oil (0.83 g, >100% crude), which was carried forward without purification. MS (ESI): mass calcd for C₂₅H₃₄N₂O₄, 426.25; m/z found, 427.5 [M+H]⁺. ¹H NMR (CDCl₃): 7.97 (d, J = 6.9, 2H), 7.21 (t, J = 7.6, 1H), 6.93-6.88 (m, 4H), 6.84-6.82 (m, 1H), 4.11-4.07 (m, 1H), 3.95-3.93 (m, 2H), 3.89-3.84 (m, 1H), 3.86 (s, 3H), 3.61 (s, 2H), 3.57 (s, 2H), 2.65-2.60 (m, 2H), 2.52-2.46 (m, 2H), 2.34 (s, 3H), 2.04 (s, 1H), 1.64-1.58 (m, 4H), 1.48-1.45 (m, 2H).

Step 5; 7-(2-Hydroxy-3-piperidin-1-yl-propoxy)-4-(4-methoxy-phenyl)-2-methyl-isoquinolinium salt. Prepared in a similar manner as 7-(3-hydroxy-propoxy)-4-(4-methoxy-phenyl)-2-methyl-isoquinolinium salt to give the desired product as a yellow oil (0.67 g, 86% crude), which was carried forward without purification. MS (ESI): mass calcd for C₂₅H₃₁N₂O₃, 407.23; m/z found, 407.5 [M]⁺.

Step 6. Prepared in a similar manner as 3-[4-(4-methoxy-phenyl)-2-methyl-1,2,3,4-tetrahydro-isoquinolin-7-yloxy]-propan-1-ol to give the desired product as a TFA salt (61.5 mg, 12%). MS (ESI): mass calcd for C₂₅H₃₄N₂O₃, 410.26; m/z found, 411.5 [M+H]⁺. ¹H NMR (CDCl₃): 7.19 (d, J = 8.5, 2H), 6.95 (d, J = 8.4, 2H), . 6.83-6.76 (m, 3H), 4.58-4.56 (m, 2H), 4.56-4.51 (m, 2H), 4.10-4.06 (m, 1H), 4.02-3.99 (m, 1H), 3.85-3.82 (m, 1H), 3.81 (s, 3H), 3.77-3.74 (m, 2H), 3.52-3.44 (m, 2H), 3.37-3.32 (m, 1H), 3.15-3.06 (m, 2H), 3.10 (s, 3H), 1.98-1.90 (m, 4H), 1.85-1.82 (m, 1H), 1.57-1.53 (m, 1H).

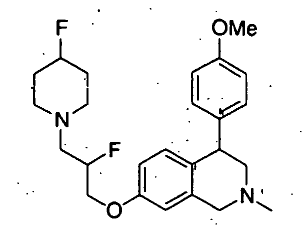

### Example 43; 7-[2-Fluoro-3-(4-fluoro-piperidin-1-yl)-propoxy]-4-(4-methoxy-phenyl)-2-methyl-1,2,3,4-tetrahydro-isoquinoline.

Step 1; 3-(3-Benzyloxy-2-hydroxy-propoxy)-benzaldehyde. A mixture of 3-hydroxybenzaldehyde (1.12 g, 9.14 mmol), K₂CO₃ (1.30 g, 9.14 mmol), and 2-benzyloxymethyl-pxirane (1.00 g, 6.09 mmol) in acetonitrile (25 mL) was heated at reflux overnight. The mixture was cooled to rt and filtered to remove a white solid. The filtrate was concentrated, diluted with EtOAc, washed with 1 N NaOH (2x) and brine, dried (MgSO₄), and concentrated. The crude material was purified by FCC to give the desired product as a clear oil (1.22 g, 70%). MS (ESI): mass calcd for C₁₇H₁₈O₄, 286.12; m/z found, 287.0 [M+H]⁺. ¹H NMR (CDCl₃): 9.97 (s, 1H), 7.49-7.43 (m, 2H), 7.40-7.39 (m, 1H), 7.37-7.29 (m, 5H), 7.20-7.18 (m, 1H), 4.59 (s, 2H), 4.23-4.22 (m, 1H), 4.13-4.0.9 (m, 2H), 3.70-3.63 (m, 2H), 2.55 (d, J = 5.0, 1H).

Step 2; 3-(3-Benzyloxy-2-fluoro-propoxy)-benzaldehyde. A mixture of 3-(3-benzyloxy-2-hydroxy-propoxy)-benzaldehyde (250 mg, 0.87 mmol), perfluorobutanesulphonyl fluoride (0.31 mL, 1.75 mmol), triethylamine trihydrofluoride (0.28 mL, 1.75 mmol), TEA (0.73 mL, 5.24 mmol) and THF (2.5 mL). The mixture was stirred at rt overnight, diluted with EtOAc, washed with water (2x), satd. aq. NaHCO₃, and brine, dried (MgSO₄), and concentrated to give the desired product as a bright yellow oil (0.22 g, 88%). GCMS (EI): mass calcd for C₁₇H₁₇FO₃, 288.12; m/z found, 288.0 [M]⁺. ¹H NMR (CDCl₃): 9.95 (s, 1H), 7.50-7.44 (m, 2H), 7.40-7.39 (m, 1H), 7.37-7.28 (m, 5H), 7.21-7.19 (m, 1H), 5.07-4.93 (m, 1H), 4.61 (s, 2H), 4.30-4.27 (m, 1H), 4.26-4.23 (m, 1H), 3.81 (d, J = 4.5, 1H), 3.76 (d, J = 4.8, 1H).

Step 3; [3-(3-Benzyloxy-2-fluoro-propoxy)-benzyl]-methyl-amine. Prepared in a similar manner as 3-(3-methylaminomethyl-phenoxy)-propan-1-ol to give the desired product as a clear oil (0.20 g, 87%). MS (ESI): mass calcd for C₁₈H₂₂FNO₂, 303.16; m/z found, 304.4 [M+H]⁺. ¹H NMR (CDCl₃): 7.36-7.32 (m, 4H), 7.31-7.28 (m, 1H), 7.23 (t, J = 7.9, 1H), 6.92-6.89 (m, 2H), 6.81-6.79 (m, 1H), 5.04-4.91 (m, 1H), 4.60 (s, 2H), 4.20 (dd, J = 20.9, 5.4, 2H), 3.79 (dd, J = 21.9, 4.6, 2H), 3.72 (s, 2H), 2.45 (s, 3H), 1.41-1.33 (br s, 1H).

Step 4: 2-Fluoro-3-(3-methylaminomethyl-phenoxy)-propan-1-ol. To a nitrogen-purged solution of [3-(3-benzyloxy-2-fluoro-propoxy)-benzyl]-methyl-amine (0.16 g, 0.53 mmol) in EtOAc (1 mL) was added 10% Pd/C (160 mg, 0.18 mmol). The mixture was stirred under an atmosphere of H₂ overnight and then was filtered though diatomaceous earth. The filtrate was concentrated and purified by FCC (MeOH/DCM) to give a yellow oil (24.0 mg, 21%). MS (ESI): mass calcd for C₁₁H₁₆FNO₂, 213.12; m/z found, 214.4 [M+H]⁺. ¹H NMR (CDCl₃): 7.23 (t, J = 7.8, 1H), 6.94-6.88 (m, 2H), 6.83-6.80 (m, 1H), 4.91-4.81 (m, 1H), 4.19 (dd, J = 20.9, 4.9, 2H), 3.90-3.84 (m, 2H), 3.72 (s, 2H), 2.43 (s, 3H).

Step 5; 2-{[3-(2-Fluoro-3-hydroxy-propoxy)-benzyl]-methyl-amino}-1-(4-methoxy-phenyl)-ethanone. Prepared in a similar manner as 2-{[3-(3-hydroxy-propoxy)-benzyl]-methyl-amino}-1-(4-methoxy-phenyl)-ethanone to give the desired product as a yellow oil (0.41 g, >100% crude), which was carried forward without purification. MS (ESI): mass calcd for C₂₇H₃₀FNO₄, 361.17; m/z found, 362.4 [M+H]⁺.

Step 6; 7-(2-Fluoro-3-hydroxy-propoxy)-4-(4-methoxy-phenyl)-2-Methyl-isoquinolinium salt. Prepared in a similar manner as 7-(3-hydroxy-propoxy)-4-(4-methoxy-phenyl)-2-methyl-isoquinolinium salt to give the desired product (0.39 g, 100% crude), which was carried forward without purification. MS (ESI): mass calcd for C₂₅H₃₀FN₂O₂⁺, 342.15; m/z found, 342.5 [M]⁺.

Step 7. Prepared in a similar manner as 3-[4-(4-methoxy-phenyl)-2-methyl-1,2,3,4-tetrahydro-isoquinolin-7-yloxy]-propan-1-ol to give the desired product (70.0 mg, 12%). MS (ESI): mass calcd for C₂₅H₃₂F₂N₂O₂, 430.24; m/z found, 431.5 [M+H]⁺. ¹H NMR (acetone-d₆): 7.12 (d, J = 8.7, 2H), 8.7, 2H), 6.76-6.68 (m, 3H), 4.99-4.89 (m, 1H), 4.68-4.55 (m, 1H), 4.23-4.08 (m, 3H), 3.76 (s, 3H), 3.58 (s, 2H), 2.87-2.84 (m, 1H), 2.82 (s, 2H), 2.78 (br s, 1H), 2.76-2.74 (m, 1H), 2.72-2.65 (m, 2H), 2.52-2.44 (m, 3H), 2.33 (s, 3H), 1.93-1.85 (m, 2H), 1.78-1.70 (m, 2H).

The following Examples 44-56 were prepared by a sequence similar to that used in Example 1.

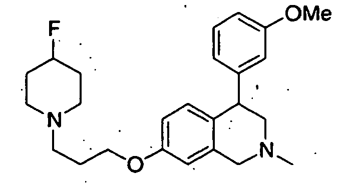

### Examples 44 (racemate), 45A (enantiomer 1), and 45B (enantiomer 2); 7-[3-(4-Fluoro-piperidin-1-yl)-propoxy]-4-(3-methoxy-phenyl)-2-methyl-1,2,3,4-tetrahydro-isoquinoline.

Step 1; 2-{[3-(3-Hydroxy-propoxy)-benzyl]-methyl-amino}-1-(3-methoxy-phenyl)-ethanone. Yield (5.12 mmol scale): 2.11 g (>100%) of crude product. MS (ESI): mass calcd for C₂₀H₂₅NO₄, 343.2; m/z found, 344.5 [M+H]⁺.

Step 2; 7-(3-Hydroxy-propoxy)-4-(3-methoxy-phenyl)-2-methyl-isoquinolinium salt. Yield (5.12 mmol scale): 1.82 g (97%) of crude product. MS (ESI): mass calcd for C₂₀H₂₂NO₃⁺, 324.2; m/z found, 324.4 [M]⁺.

Step 3: 3-[4-(3-Methoxy-phenyl)-2-methyl-1,2,3,4-tetrahydro-isopuinolin-7-yloxy]-prpopan-1-ol. Yield (5.06 mmol scale): 0.78 g (47%) after FCC followed by reverse phase HPLC purification. The compound was characterized as a TFA salt. MS (ESI): mass calcd for C₂₀H₂₃NO₃, 327.2; m/z found, 328.5 [M+H]⁺. ¹H NMR (MeOH-d₄): 7.29-7.26 (m, 1H), 6.88-6.87 (m, 1H), 6.81 (br s, 5H), 4.91 (s, 3H), 4.58-4.50 (m, 3H), 4.09-4.05 (m, 2H), 3.79-3.71 (m, 1H), 3.75 (s, 3H), 3.72 (t, J = 6.3, 2H), 3.45 (br s, 1H), 3.05 (br s, 3H), 1.99-1.92 (m, 2H).

Step 4; Methanesulfonic acid 3-[4-(3-methoxy-phenyl)-2-methyl-1,2,3,4-tetrahydro-isoquinolin-7-yloxyl-propyl ester. Yield (2.11 mmol scale): 925.4 mg (>100%) of crude product. MS (ESI): mass calcd for C₂₀H₂₇NO₅S, 405.2; m/z found, 406.4 [M+H]⁺.

Step 5; 7-[3-(4-Fluoro-piperidin-1-yl)-propoxy]-4-(3-methoxy-phenyl)-2-methyl-1,2,3,4-tetrahydro-isoquinoline. Yield (2.11 mmol scale): 378.7 mg (28%) after purification by HPLC. The compound was characterized as a TFA salt. MS (ESI): mass calcd for C₂₅H₃₃FN₂O₂, 412.2; m/z found, 413.5 [M+H]⁺. ¹H NMR (MeOH-d₄): 7.29-7.26 (m, 1H), 6.91-6.79 (m, 6H), 4.97 (s, 4H), 4.56-4.50 (m, 3H), 4.09 (t, J = 5.4, 2H), 3.79 (br s, 1H), 3.76 (s, 3H), 3.54 (d, J = 11.6, 2H), 3.36-3.33 (m, 2H), 3.26-3.21 (m, 2H), 3.06 (br s, 3H), 2.28-2.08 (m, 6H). The enantiomers were separated (SFC HPLC) to give 45A (first eluting enantiomer) and 45B (second eluting enantiomer).

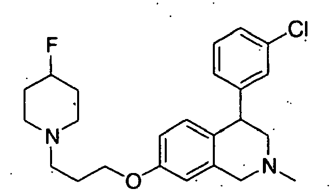

### Example 46; 4-(3-Chloro-phenyl)-7-[3-(4-fluoro-piperidin-1-yl)-propoxy]-2-methyl-1,2,3,4-tetrahydro-isoquinoline.

Step 1; 1-(3-Chloro-phenyl)-2-{[3-(3-hydroxy-propoxy)-penzyl]-methyl-amino}-ethanone. Yield (5.12 mmol scale): 2.23 g (>100%) of crude product. MS (ESI): mass calcd for C₁₉H₂₂CINO₃, 347.1; m/z found, 348.4 [M+H]⁺.

Step 2; 4-(3-Chloro-phenyl)-7-(3-hydroxy-propoxy)-2-methyl-isoquinolinium salt. Yield (5.12 mmol scale): 1.90 g (>100%) of crude product. MS (ESI): mass calcd for C₁₉H₁₉CINO₂⁺, 328.1; m/z found, 328.4 [M]⁺.

Step 3; 3-[4-(3-Chloro-phenyl)-2-methyl-1,2,3,4-tetrahydro-isoquinolin-7-yloxy] propan-1-ol. Yield (5.12 mmol scale): 0.45 g (26%), which was characterized as the TFA salt after purification by HPLC. MS (ESI): mass calcd for C₁₉H₂₂CINO₂, 331.1; m/z found, 332.4 [M+H]⁺. ¹H NMR (MeOH-d₄): 7.38-7.32 (m, 2H), 7.26 (br s, 1H), 7.19 (br d, J = 6.5, 1H), 6.88-6.72 (m, 3H), 4.89 (s, 3.5H), 4.58-4.45 (m, 4H), 4.11-4.06 (m, 2H), 3.85-3.77 (m, 1H), 3.72 (t, J = 6.5, 2H), 3.46 (br s, 1H), 3.05 (s, 3H), 1.99-1.92 (m, 2H).

Step 4; Methanesulfonic acid 3-[4-(3-chloro-phenyl)-2-methyl-1,2,3,4-tetrahydro-isoquinolin-7-yloxy]-propyl ester. Yield (1.08 mmol scale): 498.7 mg (>100%) of crude product. MS (ESI): mass calcd for C₂₀H₂₄CINO₄S, 409.1; m/z found, 410.4 [M+H]⁺.

Step 5; 4-(3-Chloro-phenyl)-7-[3-(4-fluoro-piperidin-1-yl)-propoxy]-2-methyl-1,2,3,4-tetrahydro-isoquinoline. Yield (1.08 mmol scale): 163.5 mg (23%) isolated as a TFA salt following purification by HPLC. MS (ESI): mass calcd for C₂₄H₃₀CIFN₂O, 416.2; m/z found, 417.4 [M+H]⁺. ¹H NMR (MeOH-d₄): 7.40-7.29 (m, 2H), 7.26 (br s, 1H), 7.19 (d, J = 6.4, 1H), 6.85 (br s, 2H), 6.78 (br s, 1H), 5.00 (s, 3H), 4.93-4.75 (m, 1H), 4.59-4.56 (m, 3H), 4.10 (t, J = 5.4, 2H), 3.81 (br s, 1H), 3.70-3.66 (br m. 1H), 3.55 (d. J = 12.0, 2H), 3.46 (br s, 1H), 3.35 (t, J = 7.8, 2H), 3.27-3.22 (m, 2H), 3.22-3.11 (m, 1H), 3.06 (s, 3H), 2.29-1.92 (m, 6H).

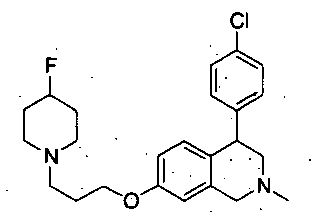

### Example 47; 4-(4-Chloro-phenyl)-7-[3-(4-fluoro-piperidin-1-yl)-propoxy]-2-methyl-1,2,3,4-tetrahydro-isoquinoline.

Step 1; 1-(4-Chloro-phenyl)-2-{[3-(3-hydroxy-propoxy)-benzyl]-methyl-amino}-ethanone. Yield (5.12 mmol scale): 2.26 g (>100%) of crude product. MS (ESI): mass calcd for C₁₉H₂₂CINO₃, 347.1; m/z found, 348.4 [M+H]⁺.

Step 2; 4-(4-Chloro-phenyl)-7-(3-hydroxy-propoxy)-2-methyl-isoquinolinium salt. Yield (5.12 mmol scale): 2.06 g (>100%) of crude product. MS (ESI): mass calcd for C₁₉H₁₉CINO₂⁺, 328.1; m/z found, 328.3 [M]⁺.

Step 3; 3-[4-(4-Chloro-phenyl)-2-methyl-1.2,3.4-tetrahydro-isoquinolin-7-yloxy]-propan-1-ol. Yield (5.12 mmol scale): 584.3 mg (34%), which was characterized as a TFA salt after purification by HPLC. MS (ESI): mass calcd for C₁₉H₂₂CINO₂, 331.1; m/z found, 332.4 [M+H]⁺. ¹H NMR (acetone-d₆): 7.23 (d, J = 8.3, 2H), 7.08 (d, J = 8.4, 2H), 6.65-6.58 (m, 2H), 6.52 (br s, 1H), 4.80 (br s; 1.5H), 4.50-4.47 (m, 1H), 4.41 (t, J = 6.3, 2H), 3.93-3.83 (m, 2H), 3.67-3.59 (m, 1H), 3.51 (t, J = 6.2, 2H), 3.28 (br s, 1H), 2.86 (s, 3H), 1.84-1.81 (m, 1H), 1.74-1.68 (m, 2H).

Step 4; Methanesulfonic acid 3-[4-(4-chloro-phenyl)-2-methyl-1,2,3,4-tetrahydro-isoquinolin-7-yloxy]-propyl ester. Yield (1.36 mmol scale): 601.9 mg (>100%) of crude product. MS (ESI): mass calcd for C₂₀H₂₄ClNO₄S, 409.1; m/z found, 410.3 [M+H]⁺.

Step 5; 4-(4-Chloro-phenyl)-7-[3-(4-fluoro-piperidin-1-yl)-propoxy]-2-methyl-1,2,3,4-tetrahydro-isoquinoline. Yield (1.36 mmol scale): 8.7 mg (1%) characterized as a TFA salt following purification by HPLC. MS (ESI): mass calcd for C₂₄H₃₀CIFN₂O, 416.2; m/z found, 417.5 [M+H]⁺. ¹H NMR (acetone-d₆): 7.41 (d, J = 8.5, 2H), 7.30 (d, J = 8.5, 2H), 6.86-6.82 (m, 2H), 6.76 (br s, 1H), 4.73-4.70 (m, 1H), 4.13 (t, J = 5.0, 2H), 3.89 (br s 1H), 3.61 (br d, J = 10.5, 2H), 3.53 (br s, 1H), 3.40 (t, J = 7.0, 2H), 3.26-3.16 (br m, 2H), 3.10 (s, 3H), 2.33 (br s, 3H), 2.25-2.10 (m, 2H).

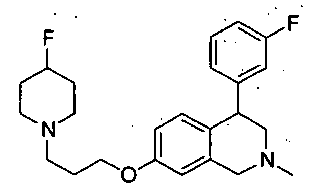

### Example 48; 4-(3-Fluoro-phenyl)-7-[3-(4-fluoro-piperidin-1-yl)-propoxy]-2-methyl-1,2,3,4-tetrahydro-isoquinoline.

Step 1; 1-(3-Fluoro-phenyl)-2-{[3-(3-hydroxy-propoxy)-benzyl]-methyl-amino}-ethanone. Yield (4.19 mmol scale): 1.56 g (>100%) of crude product. MS (ESI): mass calcd for C₁₉H₂₂FNO₃, 331.2; m/z found, 332.4 [M+H]⁺.

Step 2; 4-(3-Fluoro-phenyl)-7-(3-hydroxy-propoxy)-2-methyl-isoquinolinium salt. Yield (4.19 mmol scale): 1.43 g (94%) of crude product. MS (ESI): mass calcd for C₁₉H₁₉FNO₂⁺, 312.1; m/z found, 312.3 [M]⁺.

Step 3: 3-[4-(3-Fluoro-phenyl)-2-methyl-1,2,3,4-tetrahydro-isoquinolin-7-yloxy]-propan-1-ol. Yield (4.19 mmol scale): 348.3 mg (25%); characterized as a TFA salt. MS (ESI): mass calcd for C₁₉H₂₂FNO₂, 315.2; m/z found, 316.4 [M+H]⁺. ¹H NMR (acetone-d₆): 7.23-7.19 (m, 1H), 6.93 (d, J = 7.6, 1H), 6.89.6.82 (m, 2H), 6.65-6.59 (m, 2H), 6.54 (br s, 1H), 4.52-4.49 (m, 1H), 4.40 (t, J = 6.3, 2H), 4.36 (br s, 1H), 3.95-3.83 (m, 2H), 3.63 (br s, 1H), 3.48 (t, J = 6.1, 2H), 3.30 (br s, 1H), 2.85 (s, 3H), 1.85-1.81 (m, 3H), 1.75-1.69 (m, 2H), 1.21-1.14 (m, 1H).

Step 4; Methanesulfonic acid 3-[4-(3-fluoro-phenyl)-2-methyl-1,2,3,4-tetrahydro-isoquinolin-7-yloxy]-propyl ester. Yield (0.859 mmol scale): 364.8 mg (>100%) of crude product. MS (ESI): mass calcd for C₂₀H₂₄FNO₄S, 393.1; m/z found, 394.4 [M+H]⁺.

Step 5; 4-(3-Fluoro-phenyl)-7-[3-(4-fluoro-piperidin-1-yl)-propoxy]-2-methyl-1,2,3,4-tetrahydro-isoquinoline. Yield (0.859 mmol scale): 2.8 mg (0.5%) as a TFA salt. MS (ESI): mass calcd for C₂₄H₃₀F₂N₂O, 400.2; m/z found, 401.5 [M+H]⁺. ¹H NMR (acetone-d₆): 7.45-7.40 (m, 1H), 7.15 (d, J = 7.5, 1H), 7.11-7.04 (m, 2H), 6.86-6.75 (m, 3H), 4.74-4.71 (m, 1H), 4.56 (br s, 2H), 4.12-4.10 (m, 2H), 3.83 (br s, 1H), 3.58 (br s, 2H), 3.49 (br s, 2H), 3.36 (t, J = 8.0, 2H), 3.21 (br s, 2H), 3.04 (s, 3H), 2.33-2.26 (m, 4H), 2.17-2.12 (m, 2H).

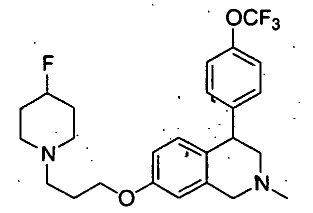

### Example 49; 7-[3-(4-Fluoro-piperidin-1-yl)-propoxy]-2-methyl-4-(4-trifluoromethoxy-phenyl)-1,2,3,4-tetrahydro-isoquinoline.

Step 1; 2-{[3-(3-Hydroxy-propoxy)-benzyl]-methyl-amino}-1-(4-trifluoromethoxyphenyl)-ethanone. Yield (5.12 mmol scale): 2.37 g (>100%) of crude product. MS (ESI): mass calcd for C₂₀H₂₂F₃NO₄, 397.2; m/z found, 398.4 [M+H]⁺.

Step 2; 7-(3-Hydroxy-propoxy)-2-methyl-4-(4-trifluoromethoxy-phenyl)-isoquinolinium salt. Yield (5.12 mmol scale): 2.06 g (94%) of crude product. MS (ESI): mass calcd for C₂₀H₁₉F₃NO₃⁺, 378.1; m/z found, 378.3 [M]⁺.

Step 3; 3-[2-Methyl-4-(4-trifluoromethoxy-phenyl)-1,2,3,4-tetrahydro-isoquinolin-7-yloxy]-propan-1-ol. Yield (5.12 mmol scale): 1.19 mg (59%), which was characterized as a TFA salt. MS (ESI): mass calcd for C₂₀H₂₂F₃NO₃, 381.2; m/z found, 382.4 [M+H]⁺. ¹H NMR (acetone-d₆): 7.21-7.19 (m, 2H), 7.12 (d, J = 8.2, 2H), 6.65-6.58 (m, 2H), 6.51 (br d, J = 7.8, 1H), 4.56-4.52 (m, 1H), 4.42-4.30 (m, 1H), 3.88-3.85 (m, 2H), 3.66-3.62 (m, 2H), 3.48 (t, J = 6.0, 2H), 3.28 (br s, 1H), 2.84 (s, 3H), 1.85-1.81 (m, 2H), 1.74-1.69 (m, 2H).

Step 4; Methanesulfonic acid 3-[2-methyl-4-(4-trifluoromethoxy-phenyl)-1,2,3,4-tetrahydro-isoquinolin-7-yloxy]-propyl ester. Yield (2.82 mmol scale): 1.50 g (>100%) of crude product.

Step 5; 7-[3-(4-Fluoro-piperidin-1-yl)-propoxy]-2-methyl-4-(4-trifluoromethoxyphenyl)-1,2,3,4-tetrahydro-isoquinoline. Yield (2.82 mmol scale): 333.4 mg (17%) as a TFA salt. MS (ESI): mass calcd for C₂₄H₃₀F₂N₂O, 400.2; m/z found, 401.5 [M+H]⁺. ¹H NMR (acetone-d₆): 7.41 (d, J = 8.5, 2H), 7.33 (d, J = 7.5, 2H), 6.87-6.76 (m, 3H), 5.03 (br d, J = 48, 1H), 4.78-4.75 (m, 1H), 4.65 (br s, 2H), 4.12 (br s, 2H), 3.93-3.90 (m, 1H), 3.77 (br s, 1H), 3.63 (br d, J = 11.0, 2H), 3.43 (br s, 2H), 3.31-3.21 (m, 2H), 3.13 (s, 3H), 2.47-2.25 (m, 3H), 2.24-2.10 (m, 2H).

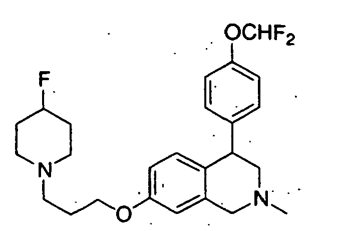

### Example 50; 4-(4-Difluoromethoxy-phenyl)-7-[3-(4-fluoro-piperidin-1-yl)-propoxy]-2-methyl-1,2,3,4-tetrahydro-isoquinoline.

Step 1; 1-(4-Difluoromethoxy-phenyl)-2-{[3-(3-hydroxy-propoxy)-benzyl]-methyl-amino}-ethanone. Yield: (5.12 mmol scale): 2.21 g (>100%) of crude product. MS (ESI): mass calcd for C₂₀H₂₃F₂NO₄, 379.2; m/z found, 380.4 [M+H]⁺.

Step 2; 4-(4-Difluoromethoxy-phenyl)-7-(3-hydroxy-propoxy)-2-methyl-isoquinolinium salt. Yield (5.12 mmol scale): 1.63 g (78%) of crude product. MS (ESI): mass calcd for C₂₀H₂₀F₂NO₃⁺, 360.1; m/z found, 360.4 [M]⁺.

Step 3; 3-[4-(4-Difluoromethoxy-phenyl)-2-methyl-1,2,3,4-tetrahydro-isoquinolin-7-yloxy]-proyan-1-ol. Yield (4.12 mmol scale): 1.00 g (67%), which was characterized as a TFA salt. MS (ESI): mass calcd for C₂₀H₂₃F₂NO₃, 363.2; m/z found, 364.4 [M+H]⁺. ¹H NMR (MeOH-d₄): 7.28 (d, J = 7.0, 2H), 7.15 (d, J = 8.5, 2H), 6.97-6.68 (t, J = 74, 1H), 6.86-6.70 (m; 3H), 4.99 (br s, 2H), 4.61 -4.55 (m, 3H), 4.06 (t, J = 6.0, 2H), 3.79 (br s, 1H), 3.72 (t, J = 6.5, 2H), 3.44 (brs, 1H), 3.06 (s, 3H), 1.98-1.93 (m, 2H).Step 4; Methanesulfonic acid 3-[4-(4-difluoromethoxy-phenyl)-2-methyl-1,2,3,4-tetrahydro-isoquinolin-7-yloxy]propyl ester. Yield (0.660 mmol scale): 357.8 mg (>100%) of crude product. MS (ESI): mass calcd for C₂₁H₂₅F₂NO₅S, 441.1; m/z found, 442.4 [M+H]⁺.

Step 5; 4-(4-Difluoromethoxy-phenyl)-7-[3-(4-fluoro-piperidin-1-yl)-propoxy]-2-methyl-1,2,3,4-tetrahydro-isoquinoline. Yield (0.660 mmol scale): 202.8 mg (44%) as a TFA salt MS (ESI): mass calcd for C₂₅H₃₁F₃N₂O₂, 448.2; m/z found, 449.5 [M+H]⁺. ¹H NMR (MeOH-d₄): 7.21 (d, J = 7.5, 2H), 7.08 (d, J = 8.5, 2H), 6.78 (br s, 2H), 6.76 (t, J = 74, 1H), 6.72 (br s, 1H), 5.00-4.80 (br m, 3H), 4.54-4.51 (m, 3H), 4.04 (t, J = 5.5, 2H), 3.77-3.70 (br m, 1H), 3.64-3.61 (br m, 1H), 3.49 (d, J = 12.0, 2H), 3.39 (br s, 1H), 3.31-3.27 (m, 3H), 3.21-3.16 (m, 1H), 3.00 (s, 3H), 2.20-2.14 (m, 6H).

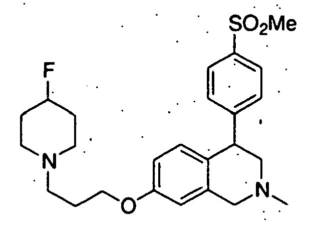

### Example 51; 7-[3-(4-Fluoro-piperidin-1-yl)-propoxy]-4-(4-methanesulfonyl-phenyl)-2-methyl-1,2,3,4-tetrahydro-isoquinoline.

Step 1; 2-{[3-(3-Hydroxy-propoxy)-benzyl]-methyl-amino}-1-(4-methanesulfonyl-phenyl)-ethanone. Yield (3.60 mmol scale): 1.73 g (>100%) of crude product. MS (ESI): mass calcd for C₂₀H₂₅NO₅S, 391.2; m/z found, 392.4 [M+H]⁺.

Step 2; 7-(3-Hydroxy-propoxy)-4-(4-methanesulfonyl-phenyl)-2-methyl-isoquinolinium salt. Yield (3.60 mmol scale): 1.45 g (99%) of crude product. MS (ESI): mass calcd for C₂₀H₂₂NO₄S⁺, 372.4; m/z found, 372.4 [M]⁺.

Step 3; 3-[4-(4-Methanesulfonyl-phenyl)-2-methyl-1,2,3,4-tetrahydro-isoquinolin-7-yloxy]-propan-1-ol. Yield (3.60 mmol scale): 810.8 mg (60%), which was characterized as a TFA salt. MS (ESI): mass calcd for C₂₀H₂₅NO₄S, 375.2; m/z found, 376.4 [M+H]⁺. ¹H NMR (acetone-d₆): 7.73 (d, J = 8.1, 2H), 7.34 (d, J = 8.3, 2H), 6.67-6.59 (m, 2H), 6.51 (br s, 1H), 5.25 (br s, 2H), 4.65-4.62 (m, 1H), 4.40 (t, J = 6.3, 2H), 3.87 (t, J = 6.1, 2H), 3.72-3.68 (m, 1H), 3.48 (t, J = 6.1, 2H), 3.35 (br s, 1H), 2.90 (s, 3H), 2.88 (s, 3H), 1.82-1.81 (m, 1H), 1.74-1.69 (m, 2H).

Step 4; Methanesulfonic acid 3-[4-(4-methanesulfonyl-phenyl)-2-methyl-1,2,3,4-tetrahydro-isoquinolin-7-yloxy]-propyl ester. Yield (1.98 mmol scale): 762.7 mg (>100%) of crude product.

Step 5; 7-[3-(4-Fluoro-piperidin-1-yl)-propoxy]-4-(4-methanesulfonyl-phenyl)-2-methyl-1,2,3,4-tetrahydro-isoquinoline. Yield (.1.98 mmol scale): 127.5 mg (9%) as a TFA salt. MS (ESI): mass calcd for C₂₅H₃₃FN₂O₃S, 460.2; m/z found, 461.5 [M+H]⁺. ¹H NMR (acetone-d₆): 7.95 (d, J = 8.5, 2H), 7.55 (d, J = 8.0, 2H), 6.88 (br s, 1H), 6.83 (br d, J = 8.0, 1H), 6.74 (br s, 1H), 5.03 (br d, J = 48, 1H), 4.88-4.84 (m, 1H), 4.64 (br s, 2H), 4.12 (t, J = 5.5, 2H), 3.94-3.91 (m, 1H), 3.61 (br d, J = 10.5, 2H), 3.40 (t, J = 7.0, 2H), 3.30-3.17 (m, 3H), 3.13 (s, 3H), 3.09 (s, 3H), 2.36-2.26 (m, 3H), 2.24-2.10 (m, 3H).

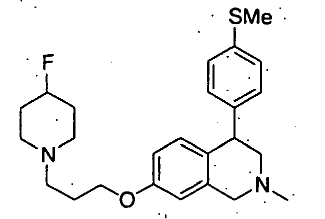

### Example 52; 7-[3-(4-Fluoro-piperidin-1-yl)-propoxy]-2-methyl-4-(4-methylsulfanyl-phenyl)-1,2,3,4-tetrahydro-isoquinoline.

Step 1; 2-{[3-(3-Hydroxy-propoxy)-benzyl]-methyl-amino}-1-(4-methylsulfanyl-phenyl)-ethanone. Yield (5.12 mmol scale): 2.37 g (>100%) of crude product. MS (ESI): mass calcd for C₂₀H₂₅NO₃S, 359.2; m/z found, 360.4 [M+H]⁺.

Step 2; 7-(3-Hydroxy-propoxy)-2-methyl-4-(4-methylsulfanyl-phenyl)-isoquinolinium salt. Yield (5.12 mmol scale): 2.07 g (98%) of crude product. MS (ESI): mass calcd for C₂₀H₂₂NO₂S⁺, 340.1; m/z found, 340.4 [M]⁺.

Step 3; 3-[2-Methyl-4-(4-methylsulfanyl-phenyl)-1,2,3,4-tetrahydro-isoquinolin-7-yloxy]-propan-1-ol. Yield (5.12 mmol scale): 352.4 mg (14%), which was characterized as a TFA salt. MS (ESI): mass calcd for C₂₀H₂₅NO₂S, 343.2; m/z found, 344.4 [M+H]⁺. ¹H NMR (MeOH-d₄): 7.25 (d, J = 8.0, 2H), 7.15 (br d, J = 6.0, 2H), 6.81 (br s, 2H), 6.78 (br s, 1H), 4.90 (s, 3H), 4.58-4.49 (m, 3H), 4.06 (t, J = 6.0, 2H), 3.77 (br s, 1H), 3.72 (t, J = 6.5, 2H), 3.41 (br s, 1H), 3.05 (s, 3H), 2.45 (s, 3H), 1.99-1.94 (m, 2H).

Step 4; Methanesulfonic acid 3-[2-methyl-4-(4-methylsulfanyl-phenyl)-1,2,3,4-tetrahydro-isoquinolin-7-yloxy]-propyl ester. Yield (0.563 mmol scale): 305.1 mg (>100%) of crude product. MS (ESI): mass calcd for C₂₁H₂₇NO₄S₂, 421.1; m/z found, 422.4 [M+H]⁺.

Step 5; 7-[3-(4-Fluoro-piperidin-1-yl)-propoxy]-2-methyl-4-(4-methylsulfanyl-phenyl)-1,2,3,4-tetrahydro-isoquinoline. Yield (0.563 mmol scale): 145.9 mg (39%) as a TFA salt. MS (ESI): mass calcd for C₂₅H₃₃FN₂OS, 428.2; m/z found, 429.5 [M+H]⁺. ¹H NMR (MeOH-d₄): 7.25 (d, J = 8.5, 2H), 7.15 (br d, J = 7.0, 2H), 6.83 (br s, 2H), 6.79 (br s, 1H), 4.92 (s, 3H), 4.54-4.49 (m, 3H), 4.09 (t, J = 5.5, 2H), 3.77 (br s, 1H), 3.68 (br d, unresolved), 3.54 (d, J = 11.0, 2H), 3.42 (br s, 1H), 3.36-3.33 (m, 2H), 3.24 (t, J = 12.5, 2H), 3.13-3.00 (m, 1H), 3.05 (s, 3H), 2.45 (s, 3H), 2.31-2.14 (m, 5H), 2.13-1.90 (m, 1H).

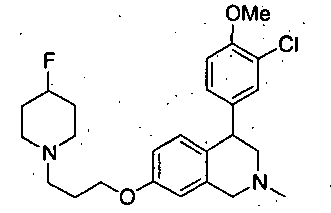

### Example 53; 4-(3-Chloro-4-methoxy-phenyl)-7-[3-(4-fluoro-piperidin-1-yl)-propoxy]-2-methyl-1,2,3,4-tetrahydro-isoquinoline.

Step 1; 1-(3-Chloro-4-methoxy-phenyl)-2-{[3-(3-hydroxy-propoxy)-benzyl]-methyl-amino}-ethanone. Yield (3.61 mmol scale): 2.10 g (>100%) of crude product. MS (ESI): mass calcd for C₂₀H₂₄CINO₄, 377.1; m/z found, 378.4 [M+H]⁺.

Step 2; 4-(3-Chloro-4-methoxy-phenyl)-7-(3-hydroxy-propoxy)-2-methyl-isoquinolinium salt. Yield (3.61 mmol scale): 1.84 g (>100%) of crude product. MS (ESI): mass calcd for C₂₀H₂₁CINO₃⁺, 358.1; m/z found, 358.4 [M]⁺.

Step 3; 3-[4-(3-Chloro-4-methoxy-phenyl)-2-methyl-1,2,3,4-tetrahydro-isoquinolin-7-yloxy]-propan-1-ol. Yield (3.61 mmol scale): 352.5 mg (21%) as a TFA salt. MS (ESI): mass calcd for C₂₀H₂₄CINO₃, 361.1; m/z found, 362.4 [M+H]⁺. ¹H NMR (MeOH-d₄): 7.24 (br s, 1H), 7.16 (br s, 1H), 7.04 (d, J = 8.5, 1H), 6.81 (br s, 3H), 4.96 (br s, 2.5H), 4.57-4.48 (m, 3H), 4.06 (t, J = 6.5, 2H), 3.85 (s, 3H), 3.80 (br s, 1H), 3.72 (t, J = 6.0, 2H), 3.41 (br s 1H), 3.04 (s, 3H), 1.97-1.93 (m, 2H).

Step 4; Methanesulfonic acid 3-[4-(3-chloro-4-methoxy-phenyl)-2-methyl-1,2,3,4-tetrahydro-isoquinolin-7-yloxy]-propyl ester. Yield (0.663 mmol scale): 344.6 mg (>100%) of crude product. MS (ESI): mass calcd for C₂₁H₂₆CINO₅S, 439.1; m/z found, 440.4 [M+H]⁺.

Step 5; 4-(3-Chloro-4-methoxy-phenyl)-7-[3-(4-fluoro-piperidin-1-yl)-propoxy]-2-methyl-1,2,3,4-tetrahydro-isoquinoline. Yield (0.663 mmol scale): 205.9 mg (46%) as a TFA salt. MS (ESI): mass calcd for C₂₅H₃₂CIFN₂O₂, 446.2; m/z found, 447.5 [M+H]⁺. ¹H NMR (MeOH-d₄): 7.22 (br s, 1H), 7.16 (d, J = 7.5, 1H), 7.04 (d, J = 8.5, 1H), 6.84-6.77 (m, 3H), 5.27 (s, 6H), 5.02-4.74 (m, 1H), 4.57-4.48 (m, 3H), 4.09 (br s, 2H), 3.96 (s, 1H), 3.86 (s, 3H), 3.80-3.77 (m, 1H), 3.70-3.65 (m, 1H), 3.54 (d, J = 12.0, 2H), 3.43-3.33 (m, 4H), 3.26-3.20 (m, 2H), 3.15-3.10 (m, 1H), 3.05 (s, 3H), 2.31-1.97 (m, 8H).

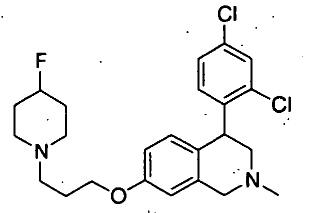

### Example 54; 4-(2,4-Dichloro-phenyl)-7-[3-(4-fluoro-piperidin-1-yl)-propoxy]-2-methyl-1,2,3,4-tetrahydro-isoquinoline.

Step 1; 1-(2,4-Dichloro-phenyl)-2-{[3-(3-hydroxy-propoxy)-benzyl]-methyl-amino}-ethanone. Yield (7.02 mmol scale): 3.15 g (>100%) of crude product. MS (ESI): mass calcd for C₁₉H₂₁CI₂NO₃, 381.1; m/z found, 382.3 [M+H]⁺, 384.3 [M+H]⁺.

Step 2; 4-(2,4-Dichloro-phenyl)-7-(3-hydroxy-propoxv)-2-methyl-isoquinolinium salt. Yield (7.02 mmol scale): 2.78 g (99%) of crude product. MS (ESI): mass calcd for C₁₉H₁₈CI₂NO₂⁺, 362.1; m/z found, 362.3 [M]⁺, 364.3 [M]⁺.

Step 3; 3-[4-(2,4-Dichloro-phenyl)-2-methyl-1,2,3,4-tetrahydro-isoquinolin-7-yloxy]-propan-1-ol. Yield (6.97 mmol scale): 1.537 g (46%) as a TFA salt. MS (ESI): mass calcd for C₁₉H₂₁CI₂NO₂, 365.1; m/z found, 366.3 [M+H]⁺, 368.3 [M+H]⁺. ¹H NMR (MeOH-d₄): 7.56 (d, J = 2.0, 1H), 7.32 (d, J = 8.0, 1H), 7.13 (br s, 1H), 6.87-6.80 (m, 2H), 6.75 (br s, 1H), 5.06 (br s, 1H), 4.89 (s, 3H), 4.58-4.46 (m, 2H), 4.08 (t, J = 6.5, 2H), 3.84-3.81 (m, 1H), 3.72 (t, J = 6.5, 2H), 3.52 (br s, 1H), 3.08 (s, 3H), 1.99-1.94 (m, 2H).

Step 4; Methanesulfonic acid 3-[4-(2,4-dichloro-phenyl)-2-methyl-1,2,3,4-tetrahydro-isoquinolin-7-yloxy]-propyl ester. Yield (3.02 mmol scale): 1.62 g (>100%) of crude product. MS (ESI): mass calcd for C₂₀H₂₃CI₂NO₄S, 443.1; m/z found, 443.3 [M+H]⁺, 445.3 [M+H]⁺.

Step 5; 4-(2,4-Dichloro-phenyl)-7-[3-(4-fluoro-piperidin-1-yl)-propoxy]-2-methyl-1,2,3,4-tetrahydro-isoquinoline. Yield (3.02 mmol scale): 1.13 g (55%) as a TFA salt. MS (ESI): mass calcd for C₂₄H₂₉CI₂FN₂O, 450.2; m/z found, 451.4 [M+H]⁺, 453.4 [M+H]⁺. ¹H NMR (MeOH-d₄): 7.53 (d, J = 2.0, 1H), 7.29 (br s, 1H), 7.16 (br s, 1H), 6.86 (br s, 2H), 6.74 (br s, 1H), 5.09 (s, 3H), 5.02-4.75 (m, 1H), 4.57 (br s, 2H), 4.11 (t, J = 5.5, 2H), 3.84-3.80 (m, 1H), 3.69 (br d, J = 10.0, 1H), 3.55 (d, J = 11.0, 2H), 3.36 (t, J = 8.0, 2H), 3.27-3.21 (m, 2H), 3.17-3.03 (m, 1H), 3.08 (s, 3H), 2.34-2.96 (m, 6H).

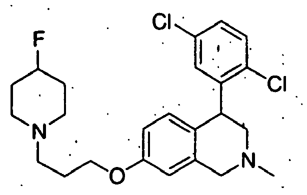

### Example 55; 4-(2,5-Dichloro-phenyl)-7-[3-(4-fluoro-piperidin-1-yl)-propoxy]-2-methyl-1,2,3,4-tetrahydro-isoquinoline.

Step 1; 1-(2,5-Dichloro-phenyl)-2-{[3-(hydroxy-propoxy)-benzyl]-methyl-amino}-ethanone. Yield (5.12 mmol scale): 3.10 g (>100%) of crude product. MS (ESI): mass calcd for C₁₉H₂₁CI₂NO₃, 381.1; m/z found, 382.4 [M+H]⁺, 384.4 [M+H]⁺.

Step 2; 4-(2,5-Dichloro-phenyl)-7-(3-hydroxy-propoxy)-2-methyl-isoquinolinium salt. Yield (5.12 mmol scale): 2.22 g (>1000%) of crude product. MS (ESI): mass calcd for C₁₉H₁₈CI₂NO₂⁺, 362.1; m/z found, 362.3 [M]⁺, 364.3 [M]⁺.

Step 3; 3-[4,(2,5-Dichloro-phenyl)-2-methyl-1,2,3,4-tetrahydro-isoquinolin-7-yloxy]-propan-1-ol. Yield (5.12 mmol scale): 1.12 g (42%) as a TFA salt. MS (ESI): mass calcd for C₁₉H₂₁CI₂NO₂, 365.1; m/z found, 366.4 [M+H]⁺, 368.4 [M+H]⁺. ¹H NMR (MeOH-d₄): 7.49 (d, J = 8.5, 1H), 7.36-7.34 (m, 1H), 7.15 (br s, 1H), 6.89-6.81 (m, 2H), 6.77 (br s, 1H), 5.07 (br s, 1H), 4.88 (s, 2.5H), 4.56-4.55 (m, 2H), 4.09 (t, J = 6.0, 2H), 3.85-3.82 (m, 1H), 3.72 (t, J = 6.5, 2H), 3.55 (br s, 1H), 3.08 (s, 3H), 2.00-1.95 (m, 2H).

Step 4; Methanesulfonic acid 3-[4-(2,5-dichloro-phenyl)-2-methyl-1,2,3,4-tetrahydro-isoquinolin-7-yloxy]-propyl ester. Yield (2.08 mmol scale): 1.24 g (>100%) of crude product. MS (ESI): mass calcd for C₂₀H₂₃CI₂NO₄S, 443.1; m/z found, 443.3.

Step 5; 4-(2,5-Dichloro-phenyl)-7-[3-(4-fluoro-piperidin-1-yl)-propoxy]-2-methyl-1,2,3,4-tetrahydro-isoquinoline. Yield (2.08 mmol scale): 895.5 mg (64%) as a TFA salt. MS (ESI): mass calcd for C₂₄H₂₉CI₂FN₂O, 450.2; m/z found, 451.4 [M+H]⁺, 453.4 [M+H]⁺. ¹H NMR (MeOH-d₄): 7.47 (d, J = 8.5, 1H), 7.33-7.31 (m, 1H), 7.17 (br s, 1H), 6.87 (br s, 2H), 6.77 (br s, 1H), 5.06 (s, 3H), 5.02-4.75 (m, 1H), 4.58 (br s, 1H), 4.11 (t, J = 5.0, 2H), 3.85-3.81 (m, 1H), 3.69 (br d, J = 10.0, 1H), 3.56 (d, J = 11.5, 2H), 3.36 (t, J = 8.0, 2H), 3.27-3.22 (m, 2H), 3.16-3.02 (m, 1H), 3.08 (s, 3H), 2.40-1.95 (m, 6H).

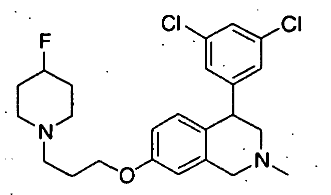

### Example 56; 4-(3,5-Dichloro-phenyl)-7-[3-(4-fluoro-piperidin-1-yl)-propoxy]-2-methyl-1,2,3,4-tetrahydro-isoquinoline.

Step 1; 1-(3,5-Dichloro-phenyl)-2-{[3-(3-hydroxy-propoxy)-benzyl]-methyl-amino}-ethanone. Yield (5.12 mmol scale): 2.31 g (>100%) of crude product. MS (ESI): mass calcd for C₁₉H₂₁CI₂NO₃, 381.1; m/z found, 382.3 [M+H]⁺, 384.3 [M+H]⁺.

Step 2; 4-(3,5-Dichloro-phenyl)-7-(3-hydroxy-propoxy)-2-methyl-isoquinolinium salt. Yield (5.12 mmol scale): 2.10 g (>100%) of crude product. MS (ESI): mass calcd for C₁₉H₁₈CI₂NO₂⁺, 362.1; m/z found, 362.3 [M]⁺, 364.3 [M]⁺.

Step 3; 3-[4-(3,5-Dichloro-phenyl)-2-methyl-1,2,3,4-tetrahydro-isoquinolin-7-yloxy]-propan-1-ol. Yield (5.12 mmol scale): 708.5 mg (27%) as the TFA salt. MS (ESI): mass calcd for C₁₉H₂₁CI₂NO₂, 365.1; m/z found, 364.4 [M+H]⁺, 366.4 [M+H]⁺. ¹H NMR (MeOH-d₄): 7.38-7.24 (m, 2H), 7.14 (d, J = 8.0, 1H), 6.96-6.67 (m, 3H), 4.99 (br s, 2H), 4.60-4.55 (m, 3H), 4.09-4.05 (m, 2H), 3.83-3.73 (m, 1H), 3.74-3.71 (m, 2H), 3.44 (br s, 1H), 3.06 (s, 3H), 1.98-1.94 (m, 2H).

Step 4; Methanesulfonic acid 3-[4-(3,5-dichloro-phenyl)-2-methyl-1,2,3,4-tetrahydro-isoquinolin-7-yloxy]-propyl ester. Yield (1.20 mmol scale): 646.9 mg (>100%) of crude product. MS (ESI): mass calcd for C₂₀H₂₃CI₂NO₄S, 443.1; m/z found, 442.4 [M+H]⁺, 444.4 [M+H]⁺.

Step 5; 4-(3,5-Dichloro-phenyl)-7-[3-(4-fluoro-piperidin-1-yl)-propoxy]-2-methyl-1,2,3,4-tetrahydro-isoquinoline. Yield (1.20 mmol scale): 175.5 mg (21 %) as a TFA salt. MS (ESI): mass calcd for C₂₄H₂₉CI₂FN₂O, 450.2; m/z found, 451.4 [M+H]⁺, 453.4 [M+H]⁺. ¹H NMR (MeOH-d₄): 7.33 (s, 1H), 7.18 (s, 2H), 6.84-6.76 (m, 3H), 4.97-4.85 (m, 4H), 4.57-4.50 (m, 3H), 4.06 (t, J = 5.5, 2H), 3.79-3.75 (m, 1H), 3.62-3.42 (m, 3H), 3.31-3.28 (m, 4H), 3.25-3.14 (m, 2H), 3.00 (s, 3H), 2.20-1.94 (m, 7H).

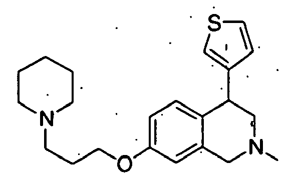

### Example 57; 2-Methyl-7-(3-piperidin-1-yl-propoxy)-4-thiophen-3-yl-1,2,3,4-tetrahydro-isoquiholine.

Step 1; 2-[(3-Methoxy-benzyl)-methyl-amino]-1-thiophen-3-yl-ethanone. A solution of (3-methoxy-benzyl)-methyl-amine (0.590 g, 3.93 mmol) and TEA (1.1 mL) in DCM (5 mL) was treated with 2-bromo-1-thiophen-3-yl-ethanone (1.11 g, 5.11 mmol). After 16 h at 23 °C, the mixture was poured onto water and extracted with DCM. The organic layer was collected, dried (Na₂SO₄), and concentrated to give the desired product (1.149 g, >100% crude). ¹H NMR (CDCl₃): 8.24 (dd, J = 2.9, 1.2, 1H), 7.57 (dd, J = 5.1, 1.2, 1H), 7.28 (dd, J = 5.1, 2.9, 1H), 7.26-7.21 (m, 1H), 6.94-6.90 (m, 2H), 6.83-6.79 (m, 1H), 3.79 (s, 3H), 3.65 (s, 2H), 3.64 (s, 2H), 2.37 (s, 3H).

Step 2; 7-Methoxy-2-methyl-4-thiophen-3-yl-1,2,3,4-tetrahydro-isoquinoline. 2-[(3-Methoxy-benzyl)-methyl-amino]-1-thiophen-3-yl-ethanone (1.14 g, 4.14 mmol) was diluted with MSA (3 mL). The mixture was stirred at 23 °C for 2 h then was poured into satd. aq. NaHCO₃ and extracted with DCM. The organic layer was collected, dried (Na₂SO₄), and concentrated, then was treated with MeOH (5 mL) and excess NaCNBH₃. A few crystals of bromocresol green were added and the pH of the mixture was adjusted to -4-5 with HCl (1.25 M in MeOH). The mixture was stirred at 23 °C for 16 h, and extracted with DCM. The organic layer was collected, dried (Na₂SO₄), and concentrated. FCC gave 7-methoxy-2-methyl-4-thiophen-3-yl-1,2,3,4-tetrahydro-isoquinoline (0.412 g, 38%). ¹H NMR (CDCl₃): 7.23 (dd, J = 5.1, 3.1, 1H), 7.03 (ddd, J = 2.9, 1.4, 0.4, 1H), 6.90 (dd, J = 5.1, 1.4, 1H), 6.87 (dd, J = 8.0, 0.4, 1H), 6.66 (dd, J = 8.4, 2.7, 1H), 6.59 (d, J = 2.7, 1H), 4.32 (dd, J = 8.0, 6.3, 1H), 3.77 (s, 3H), 3.68 (d, J = 14.9, 1H), 3.58 (d, J = 14.9, 1H), 2.97 (ddd, J = 11.5, 5.5, 1.4, 1H), 2.60 (dd, J = 11.3, 8.2, 1H), 2.43 (s, 3H).

Step 3; 2-Methyl-4-thiophen-3-yl-1,2,3,4-tetrahydro-isoquinolin-7-ol. To a 0 °C solution of 7-methoxy-2-methyl-4-thiophen-3-yl-1,2,3,4-tetrahydro-isoquinoline (0.278 g, 1.07 mmol) in DCM (3 mL) was added BBr₃ (1.0 M in DCM, 2.0 mL). After 1 h at 0 °C, satd. aq. NaHCO₃ was added and the organics were extracted into DCM. The organic layer was collected, dried (Na₂SO₄), and concentrated. FCC gave the desired compound (0.042 g, 16%). ¹H NMR (DMSO-d₆): 9.17 (s, 1H), 7.40 (dd, J = 4.9, 2.9, 1H), 7.19 (dd, J = 2.9, 1.2, 1H), 6.94 (dd, J = 4.9, 1.2, 1H), 6.67 (d, J = 8.0, 1H), 6.49 (dd, J = 8.2, 2.3, 1H), 6.46 (d, J = 2.5, 1H), 4.17 (dd, J = 6.8, 6.1, 1H), 3.50 (d, J = 15.5, 1H), 3.45 (d, J = 15.5, 1H), 2.78 (dd, J = 15.5, 5.5, 1H), 2.56 (dd, J = 11.3, 7.2, 1H), 2.30 (s, 3H).

Step 4. To a solution-of 2-methyl-4-thiophen-3-yl-1,2,3,4-tetrahydro-isoquinplin-7-ol (0.0498 g, 0.203 mmol) in THF (2 mL) was added NaH (60% dispersion in oil, 0.029 g, 0.728 mmol), followed by 1-bromo-3-chloropropane (0.075 mL). After 1 h, the mixture was treated with additional NaH (0.050 g) and 1-bromo-3-chloropropane (0.10 mL), and the mixture was heated to 50 °C for 18 h. The mixture was then cooled, diluted with satd. aq. NaHCO₃, and extracted with DCM. The organic layer was collected, dried (Na₂SO₄), and concentrated. FCC gave 7-(3-chloro-propoxy)-2-methyl-4-thiophen-3-yl-1,2,3,4-tetrahydro-isoquinoline (0.0188 g), slightly contaminated with 2-methyl-4-thiophen-3-yl-1,2,3,4-tetrahydro-isoquinolin-7-ol. The material was used crude in the next reaction. A solution of 7-(3-chloro-propoxy)-2-methyl-4-thiophen-3-yl-1,2,3,4-tetrahydroisoquinoline (0.0188 g, 0.058 mmol) in *n*-butanol (2 mL) was treated with Na₂CO₃ (0.0306 g), piperidine (0.5 mL), and KI (∼0.005 g). The resulting mixture was heated at 55 °C for 18 h, cooled, diluted with satd. aq. NaHCO₃, and extracted with DCM. The organic layer was collected, dried (Na₂SO₄), and concentrated. FCC gave the desired compound (0.020 g, 92%). MS (ESI): mass calcd for C₂₂H₃₀N₂₀S, 370.55; m/z found, 371.5 [M+H]⁺. ¹H NMR (CDCl₃): 7.23 (dd, J = 5.1, 3.1, 1H), 7.03 (dd, 2.9, 1.0, 1H), 6.89 (dd, J = 2.9, 1.0, 1H), 6.84 (d, J = 8.6, 1H), 6.65 (dd, J = 8.6, 2.7, 1H), 6.59 (d, J = 2.7, 1H), 4.13 (dd, J = 7.8, 5.9, 1H), 3.97 (d, J = 6.5, 1H), 3.95 (d, J = 6.5, 1H), 3.67 (d, J = 14.9, 1H), 3.57 (d, J = 15.1, 1H), 2.96 (ddd, 11.3, 5.5, 1.2, 1H), 2.59 (dd, J = 11.5, 8.4, 1H), 2.48-2.32 (m, 9H), 2.01-1.91 (m, 2H), 1.63-1.54 (m, 4H), 1.48-1.40 (m, 2H).

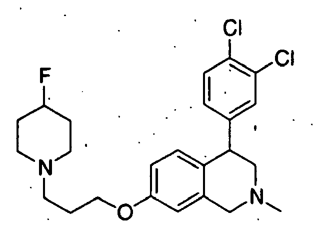

### Example 58; 4-(3,4-Dichloro-phenyl)-7-[3-(4-fluoro-piperidin-1-yl)-propoxy]-2-methyl-1,2,3,4-tetrahydro-isoquinoline.

Prepared by a sequence similar to that used in Example 1.

Step 1; 3-[4-(3,4-Dichloro-phenyl)-2-methyl-1,2.3,4-tetrahydro-isoquinolin-7-yloxy]-propan-1-ol. MS (ESI): mass calcd for C₁₉H₂₁Cl₂NO₂, 365.09; m/z found, 366.3 [M+H]⁺. ¹H NMR (CDCl₃): 7.34 (d, J = 8.2, 1H), 7.29 (d, J = 2.0, 1H), 7.03 (dd, J = 8.2, 2.2, 1H), 6.75 (d, J'= 8.4, 1H), 6.67 (dd, J = 8.4, 2.5, 1H), 6.63 (d, J = 2.5, 1H), 4.16-4.07 (m, 3H), 3.87 (d, J = 5.9, 1H), 3.85 (d, J = 5.9, 1H), 3.62 (s, 2H), 2.93 (dd, J = 11.3, 5.3, 1H), 2.54 (dd, J = 11.5, 7.4, 1H), 2.40 (s, 3H), 2.07-2.00 (m, 2H).

Step 2. MS (ESI): mass calcd for C₂₄H₂₉Cl₂FN₂O, 450.16; m/z found, 451.4 [M+H]⁺. ¹H NMR (CDCl₃): 7.33 (d, J = 8.3, 1H), 7.29 (d, J = 2.3, 1H), 7.03 (dd, J = 8.3, 2.0, 1H), 6.75 (d, J = 8.6, 1H), 6.66 (dd, J = 8.6, 2.8, 1H), 6.61 (d, J = 2.8, 1H), 4.78-4.58 (m, 1H), 4.13 (dd, J = 7.1, 5.6, 1H), 3.99 (d, J = 6.3, 1H), 3.97 (d, J = 6.3, 1H), 3.62 (s, 2H), 2.93 (dd, J = 11.4, 5.3, 1H), 265-2.48 (m, 5H), 2.40 (s, 3H), 2.41-2.35 (m, 2H), 2.00-1.83 (m, 6H).

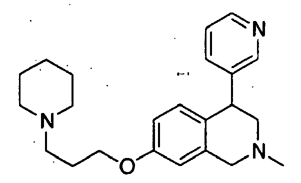

### Example 59; 2-Methyl-7-(3-piperidin-1-yl-propoxy)-4-pyridin-3-yl-1,2,3,4-tetrahydro-isoquinoline.

Step 1; 7-Methoxy-2-methyl-4-pyridin-3-yl-1,2,3,4-tetrahydro-isoquinoline.

Prepared in a manner analogous to synthesis of 3-[4-(4-methoxy-phenyl)-2-methyl-1,2,3,4-tetrahydro-isoquinolin-7-yloxy]-propan-1-ol. MS (ESI): mass calcd for C₁₆H₁₈N₂O, 254.14; m/z found, 255.4 [M+H]⁺. ¹H NMR (CDCl₃): 8.78-8.74 (m, 2H), 7.98 (d, J = 7.6, 1H), 7.66 (m, 1H), 6.86 (dd, J = 8.6, 2.5, 1H), 6.79 (d, J = 8.6, 1H), 6.72 (d, J = 2.5, 1H), 4.94-4.81 (m, 1H), 4.46 (br s, 2H), 3.88-3.70 (m, 4H), 3.01 (s, 3H).

Step 2; 2-Methyl-4-pyridin-3-yl-1,2,3,4-tetrahydro-isoquinolin-7-ol. To a solution of 7-methoxy-2-methyl-4-pyridin-3-yl-1,2,3,4-tetrahydro-isoquinoline (99 mg, 0.39 mmol) in DCM (4.0 mL) was added BBr₃ (0.78 mL, 0.78 mmol). The mixture was heated at reflux overnight. Additional BBr₃ (1 equiv.) was added; and the mixture was heated at reflux for an additional 4 h. The mixture was then poured into water, neutralized with 2 N Na₂CO₃, extracted with DCM, dried (Na₂SO₄), and concentrated to give the crude material. Purification by FCC gave the desired product (48 mg, 48%). MS (ESI): mass calcd for C₁₅H₁₆N₂O, 240.13; m/z found, 241.4 [M+H]⁺. ¹H NMR (CDCl₃): 8.38 (dd, J = 4.9, 1.6, 2H), 7.64-7.59 (m, 1H), 7.36-7.31 (m, 1H), 6.63-6.53 (m, 3H), 4.29-4.22 (m, 1H), 3.69-3.56 (m, 2H), 3.06-2.97 (m, 1H), 2.55 (dd, J = 11.5, 8.4, 1H), 2.40 (s, 3H).

Step 3; 7-(3-Chloro-propoxy-2-methyl-4-pyridin-3-yl-1,2,3,4-tetrahydro-isoquinoline. A mixture of 2-methyl-4-pyridin-3-yl-1,2,3,4-tetrahydro-isoquinolin-7-ol (40 mg, 0.17 mmol), 1-bromo-3-chloropropane (53 mg, 0.34 mmol), and K₂CO₃ (116 mg, 0.84 mmol) in acetone was heated to 100 °C in a sealed pressure tube. After 3 h, the mixture was cooled to rt, was diluted with water, and extracted with DCM (3x). The combined organic layers were washed with brine, dried (Na₂SO₄), and concentrated to give the crude product as a brown residue. Purification by FCC gave the desired product (18 mg, 33%).

Step 4. A solution of 7-(3-chloro-propoxy-2-methyl-4-pyridin-3-yl-1,2,3,4-tetrahydro-isoquinoline (18 mg, 0.06 mmol), piperidine (7.2 mg, 0.09 mmol), Na₂CO₃ (9.0 mg, 0.09 mmol), and KI (0.5 mg, 0.003 mmol) in n-butanol was heated at 80 °C and stirred overnight. The mixture was concentrated and the residue was purified by reverse phase column chromatography to give desired compound (2.5 mg, 12%) as a TFA salt. MS (ESI): mass calcd for C₂₃H₃₁N₃O, 365.25; m/z found, 366.5 [M+H]⁺. ¹H NMR (CDCl₃): 8.44 (d, J = 1.9, 1H), 8.40 (dd, J = 4.7, 1.6, 1H), 7.41-7.38 (m, 1H), 7.14-7.10 (m, 1H), 6.68 (d, J = 8.5, 1H), 6.57-6.52 (m, 2H), 4.14-4.10 (m, 1H), 4.06-3.99 (m, 1H), 3.96 (t, J = 5.7, 2H), 3.58 (d, J = 3.6, 2H), 2.91-2.86 (m, 1H), 2.75-2.71 (m, 1H), 2.56-2.48 (m, 2H), 2.34 (s, 3H), 1.61-1.36 (br m, 6H), 1.29-1.10 (m, 10H), 0.84-0.80 (m, 4H).

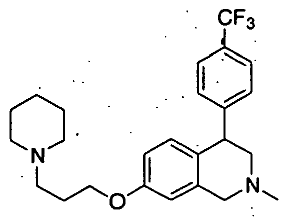

### Example 60; 2-Methyl-7-(3-piperidin-1-yl-propoxy)-4-(trifluoromethyl-phenyl)-1,2,3,4-tetrahydro-isoquinoline.

Step 1; 3-(3-Piperidin-1-yl-propoxy)-benzaldehyde. Prepared in a manner analogous to that for 3-(3-hydroxy-propoxy)-benzaldehyde, using 1-(3-hydroxypropyl)piperidine in place of 3-bromo-propan-1-ol.

Step 2; Methyl-[3-(3-piperidin-1-yl-propoxy)-benzyl]-amine. Prepared in a manner analogous to that for 3-(3-methylaminomethyl-phenoxy)-propan-1-ol.

Step 3; 1-(4-Methoxy-phenyl)-2-{methyl-[3-(3-piperidin-1-yl-propoxy)-benzyl]-amino}-ethanone. A solution of methyl-[3-(3-piperidin-1-yl-propoxy)-benzyl]-amine (150 mg, 0.57 mmol), 2-bromo-1-(4-trifluoromethyl-phenyl)-ethanone (168 mg, 0.63 mmol) and TEA (80 µL, 0.57 mmol) in DCM (5.7 mL) was stirred at rt overnight, then was diluted with waster and extracted with DCM (3x). The organic layers were combined, washed with brine, dried (Na₂SO₄), filtered, and concentrated to give the crude material. Purification using FCC gave the desired compound (84%).

Step 4; 1-(4-Methoxy-phenyl)-2-{methyl-[3-(3-piperidin-1-yl-propoxy)-benzyl]-amino}-ethanol. To a 0 °C solution of 1-(4-methoxy-phenyl)-2-{methyl-[3-(3-piperidin-1-yl-propoxy)-benzyl]-amino}-ethanone (216 mg, 0.48 mmol) in MeOH (4.8 mL) was added NaBH₄ (37 mg, 0.96 mmol). The mixture was allowed to warm to rt and was stirred at rt overnight. The mixture was diluted with water and extracted with DCM (3 x 50 mL). The organic layers were combined, washed with brine, dried (Na₂SO₄), and concentrated to give the crude material. Purification by FCC gave the desired compound (87%).

Step 5. A mixture of 1-(4-methoxy-phenyl)-2-{methyl-[3-(3-piperidin-1-yl-propoxy)-benzyl]-amino}-ethanol (146 mg, 0.32 mmol) in MSA was heated to 65 °C overnight. The mixture was cooled to rt, slowly neutralized, and basified with 2 N NaOH. The mixture was diluted with water and extracted with DCM (3 x 25 mL). The organic layers were combined and washed with brine, dried (Na₂SO₄), and concentrated to give a crude oil. Purification on an Isco using a 35 g Biotage column gave the desired compound (22%). MS (ESI): mass calcd for C₂₅H₃₁F₃N₂O, 432.24; m/z found, 433.5 [M+H]⁺. ¹H NMR (CDCl₃): 7.45 (d, J = 8.0, 2H), 7.23 (d, J = 8.8, 2H), 6.65 (d, J = 8.4; 1H), 6.58 (d, J = 2.5, 1H), 6.57-6.54 (m, 2H), 4.17 (t, J = 6.5, 1H), 3.91 (t, J = 6.3, 2H), 3.57 (s, 2H), 2.89 (dd, J = 11.5, 5.5, 1H), 2.53-2.35 (m, 8H), 2.33 (s, 3H), 1.98-1.89 (m, 2H), 1.61-1.52 (m, 4H), 1.43-1.34 (m, 2H).

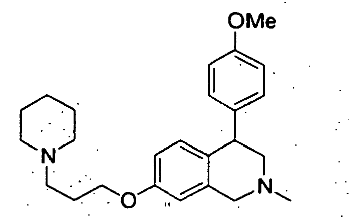

### Examples 61 (racemic); 62 (enantiomer 1); and 63 (enantiomer 2); 4-(4-Methoxy-phenyl)-2-methyl-7-(3-piperidin-1-yl-propoxy)-1,2,3,4-tetrahydro-isoquinoline.

Step 1; 2-{Methyl-[3-(3-piperidin-1-yl-propoxy)-benzyl]-amino}-1-(4-trifluoromethylphenyl)-ethanone. Prepared in a manner analogous to that for 2-{[3-(3-hydroxy-propoxy)-benzyl]-methyl-amino}-1-(4-methoxy-phenyl)-ethanone.

Step 2; 2-{Methyl-[3-(3-piperidin-1-yl-propoxy)-benzyl]-amino}-1-(4-trifluoromethylphenyl)-ethanol. Prepared in a manner analogous to Example 60.

Step 3. Prepared in a manner analogous to Example 60 to obtain the desired product as a racemic mixture. MS (ESI): mass calcd for C₂₅H₃₄N₂O₂, 394.26; m/z found, 395.5 [M+H]⁺. ¹H NMR (CDCl₃): 7.08-6.92 (m, 2H), 6.84-6.72 (m, 2H), 7.76-6.48 (m, 3H), 4.66-4.43 (m, 2H), 4.17-3.91 (m, 4H), 3.73 (s, 3H), 3.63-3.51 (m, 2H), 3.20-3.05 (m, 2H), 2.90 (s, 3H), 2.66-2.52 (m, 2H), 2.25-2.09 (m, 2H), 1.99-1.71 (m, 6H), 1.42-1.28 (m, 1H).

The two enantiomers, Examples 62 and 63, were separated by chiral HPLC. R_{T} (62): 12.9 min. R_{T} (63): 14.8 min.

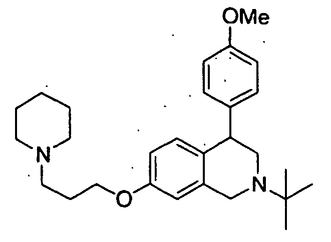

### Example 64; 2-tert-Butyl-4-(4-methoxy-phenyl)-7-(3-piperidin-1-yl-propoxy)-1,2,3,4-tetrahydro-isoquinoline.

Step 1; 3-[3-(tert-Butylamino-methyl)-phenoxyl]-propan-1-ol. To a solution of 3-(3-hydroxy-propoxy)-benzaldehyde (1.0 equiv.), *tert*-butyl amine (1.1 equiv.), and acetic acid (1.0 equiv.) in anhydrous DCE (0.5 M) was added NaB(OAc)₃H (1.5 equiv.) portionwise. The resultant mixture was stirred at rt overnight. The mixture was treated with 1 N NaOH (200 mL), stirred for 30 min, and extracted with DCM (3x). The combined organic layers were washed with brine, dried over Na₂SO₄, and concentrated. FCC gave the product (2.75 g, 70%). MS (ESI): mass calcd for C₁₄H₂₃NO₂, 237.17; m/z found, 238.4 [M+H]⁺. ¹H NMR (CDCl₃): 7.21 (t, J = 7.7, ¹H), 6.91-6.89 (m, 2H), 6.76 (dd, J = 8.3, 2.2, 1H), 4.09 (t, J = 6.1, 2H), 3.78 (t, J = 6.0, 2H), 3.68 (s, 2H), 1.99 (m, 2H), 1.17 (s, 9H).

Steps 2-5 were performed in a similar manner to that described in Example 1.

Step 2; 2-{tert-Butyl-[3-(3-hydroxy-propoxy)-benzyl]-amino}-1-(4-methoxy-phenyl)-ethanone. Yield (11.8 mmol scale): 5.33 g (>100%) of crude product. MS (ESI): mass calcd for C₂₃H₃₁NO₄, 385.23; m/z found, 386.5 [M+H]⁺.

Step 3; 2-tert-Butyl-7-(3-hydroxy-propoxy)-4-(4-methoxy-phenyl)-isoquinolinium salt. Yield (13.8 mmol scale): 3.33 g of crude product. MS (ESI): mass calcd for C₂₃H₂₈NO₃⁺, 366.5; m/z found, 366.5 [M]⁺.

Step 4; 3-[2-tert-Butyl-4-(4-methoxy-phenyl)-1,2,3,4-tetrahydro-isoauinolin-7-yloxy]-propan-1-ol. Yield (9.08 mmol scale): 3.2 g crude material that was used directly in the next step. MS (ESI): mass calcd for C₂₃H₃₁NO₃, 369.23; m/z found, 370.5 [M+H]⁺.

Step 5; Methanesulfonic acid 3-[2-tert-butyl-4-(4-methoxy-phenyl)-1,2,3,4-tetrahydro-isoquinolin-7-yloxy]-propyl ester. Yield (0.51 mmol scale): 0.28 g (>100%). MS (ESI): mass calcd for C₂₄H₃₃NO₅S, 447.21; m/z found, 448.5 [M+H]⁺.

Step 6. Yield (0.62 mmol scale): 33 mg (8% for two steps) as a TFA salt. MS (ESI): mass calcd for C₂₈H₄₀N₂O₂, 436.31; m/z found, 437.5 [M+H]⁺. ¹H NMR (CDCl₃): 7.15-7.13 (m, 2H), 6.90-6.88 (m, 2H), 6.76-6.74 (m, 1H), 6.68-6.65 (m, 2H), 4.77-4.70 (m, 2H), 4.20-4.18 (m, 1H), 4.05-4.04 (m, 2H), 3.81 (s, 3H), 3.74-3.71 (m, 1H), 3.67-3.60 (m, 2H), 3.16 (m, 2H), 2.85 (t, J = 11.9, 1H), 2.64 (m, 2H), 2.24-2.23 (m, 2H), 2.04-1.97 (m, 2H), 1.89-1.87 (m, 3H), 1.53 (s, 9H), 1.45-1.37 (m, 1H).

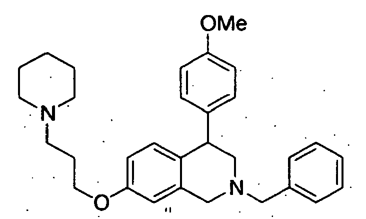

### Example 65; 2-Benzyl-4-(4-methoxy-phenyl)-7-(3-piperidin-1-yl-propoxy)-1,2,3,4-tetrahydro-isoquinoline.

Prepared by a sequence similar to that used in Example 64.

Step 1; 3-[3-(Benzylamino-methyl)-phenoxy]-propan-1-ol. Yield (16.6 mmol scale): 2.5 g (55%). MS (ESI): mass calcd for C₁₇H₂₁NO₂, 271.16; m/z found, 272.5 [M+H]⁺. ¹H NMR (CDCl₃): 7.34-7.31 (m, 4H), 7.27-7.21 (m, 2H), 6.92-6.91 (m, 2H), 6.81-6.79 (m, 1H), 4.12 (t, J = 5.9, 2H), 3.85 (t, J = 5.88, 2H), 3.80 (s, 2H), 3.77 (s, 2H), 2.03 (m, 2H).

Step 2; 2-{Benzyl-[3-(3-hydroxy-propoxy)-benzyl]-amino}-1-(4-methoxy-phenyl)-ethanone. Yield (9.32 mmol scale): 4.18 g (>100%) of crude product. MS (ESI): mass calcd for C₂₆H₂₉NO₄, 419.21; m/z found, 420.5 [M+H]⁺.

Step 3; 2-Benzyl-7-(3-hydroxy-propoxy)-4-(4-methoxy-phenyl)-isoquinolinium salt. Yield (9.32 mmol scale): 3.70 g of crude product. MS (ESI): mass calcd for C₂₆H₂₆NO₃⁺, 400.49; m/z found, 400.4 [M]⁺.

Step 4; 3-[2-Benzyl-4-(4-methoxy-phenyl)-1,2,3,4-tetrahydro-isopuinolin-7-yloxy]-propan-1-ol. 8.96 g (36%). MS (ESI): mass calcd for C₂₆H₂₉NO₃, 403.51; m/z found, 404.5 [M+H]⁺. ¹H NMR (CDCl₃): 7.45 (s, 5H), 7.05-7.03 (m, 2H), 6.85 (d, J = 8.5, 2H), 6.75 (s, 2H), 6.60 (s, 1H), 4.54 (s, 2H), 4.40-4.31 (m, 2H), 4.06 (m, 3H), 3.81-3.79 (m, 6H), 3.08 (s, 1H), 2.01-1.99 (m, 2H).

Step 5; Methanesulfonic acid 3-[2-benzyl-4-(4-methoxy-phenyl)-1,2,3,4-tetrahydro-isoquinolin-7-yloxy]-propyl ester. Yield (7.06 mmol scale): 3.87 g (>100%): MS (ESI): mass calcd for C₂₇H₃₁NO₅S, 481.60; m/z found, 482.4 [M+H]⁺.

Step 6. Yield: 1.19 mg (31 % for two steps) as a TFA salt after FCC and reverse phase HPLC. MS (ESI): mass calcd for C₃₁H₃₈N₂O₂, 470.29; m/z found, 471.5 [M+H]⁺. ¹H NMR (CDCl₃): 7.44-7.43 (m, 5H), 7.04-7.02 (m, 2H), 6.84 (d, J = 8.4, 2H), 6.76 (s, 1H), 6.68 (s, 1H), 6.54 (s, 1H), 4.51 (s, 1H), 4.34 (q, J = 12.9, 2H), 4.16 (s, 1H), 3.98 (s, 2H), 3.78 (s, 3H), 3.75 (s, 1H), 3.61 (s, 2H), 3.15 (s, 2H), 2.97 (s, 1H), 2.62-2.61 (m, 3H), 2.22 (s, 2H), 2.00-1.97 (m, 2H), 1.87-1.85 (m, 3H), 1.44-1.39 (m, 1H).

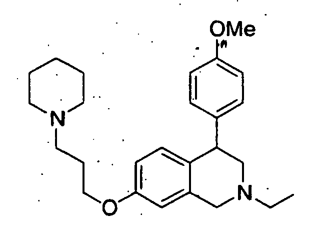

### Example 66; 2-Ethyl-4-(4-methoxy-phenyl)-7-(3-piperidin-1-yl-propoxy)-1,2,3,4-tetrahydro-isoquinoline.

Step 1; 3-[4-(4-Methoxy-phenyl)-1,2,3,4-tetrahydro-isoquinolin-7-yloxy]-propan-1-ol. To a solution of 3-[2-benzyl-4-(4-methoxy-phenyl)-1,2,3,4-tetrahydro-isoquinolin-7-yloxy]-propan-1-ol (3.79 g, 9.39 mmol) in dry EtOH (0.4 M) in a 250 mL Parr shaker reaction bottle was added Pd/C (10% wt, 4.25 g). The resulting mixture was stirred for 20 h at rt under a H₂ atmosphere (50 psi). The mixture was filtered and the filtrate concentrated. Purification by FCC gave the desired product (2.17 g, 73%). MS (ESI): mass calcd for C₁₉H₂₃NO₃, 313.17; m/z found, 314.4 [M+H]⁺. ¹H NMR (CDCl₃): 7.03-7.01 (m, 2H), 6.86-6.84 (m, 2H), 6.79-6.71 (m, 2H), 6.62 (m, 1H), 4.31-4.28 (m, 3H), 4.05-4.02 (m, 2H), 3.81-3.80 (m, 5H), 3.63-3.61 (m, 1H), 3.20-3.18 (m, 1H), 2.00-1.98 (m, 2H).

Step 2; 3-[2-Ethyl-4-(4-methoxy-phenyl)-1,2,3,4-tetrahvdro-isoquinolin-7-yloxy]-propan-1-ol. To a solution of 3-[4-(4-methoxy-phenyl)-1,2,3,4-tetrahydro-isoquinolin-7-yloxy]-propan-1-ol (330 mg, 1.05 mmol) in anhydrous DCE (0.5 M) was added dry acetaldehyde (1.1 equiv.), acetic acid (1.0 equiv.) and NaB(OAc)₃H (1.5 equiv.). The resultant solution was stirred at rt overnight. The mixture was diluted with 1 N NaOH (10 mL) and extracted with DCM (3x). The combined organics were washed with brine, dried over Na₂SO₄, and concentrated. Purification by FCC and reverse phase HPLC gave the desired product (54 mg, 15%) as a TFA salt. MS (ESI): mass calcd for C₂₁H₂NO₃, 341.20; m/z found, 342.5 [M+H]⁺. ¹H NMR (CDCl₃): 7.12-7.04 (m, 2H), 6.91-6.89 (m, 2H), 6_{.}80-6.74 (m, 2H), 6.66-6.63 (m, 1H), 4.63-4.54 (m, 2H), 4.12-4.06 (m, 3H), 3.83-3.81 (m, 5H), 3.25-2.90 (m, 5H), 2.02-2.01 (m, 2H), 1.45-1.43 (m, 3H).

Step 3; Methanesulfonic acid 3-[2-ethyl-4-(4-methoxy-phenyl)-1,2,3,4-tetrahydro-isoquinolin-7-yloxy]-propyl ester. Prepared in a similar manner to that described for methanesulfonic acid 3-[4-(4-methoxy-phenyl)-2-methyl-1,2,3,4-tetrahydro-isoquinolin-7-yloxy]-propyl ester, on a 0.158 mmol scale, to give 111 mg (>100%) of the desired product. MS (ESI): mass calcd for C₂₂H₂₉NOP₅S, 419.54; m/z found, 420.4 [M+H]⁺.

Step 4. Prepared in a similar manner to that described for 2-benzyl-4-(4-methoxy-phenyl)-7-(3-piperidin-1-yl-propoxy)-1,2,3,4-tetrahydro-isoquinoline, on a 0.26 mmol scale, to give the desired product (40 mg, 40% over two steps) as a TFA salt. MS (ESI): mass calcd for C₂₆H₃₆N₂O₂, 408.58; m/z found, 409.5 [M+H]⁺. ¹H NMR (CDCl₃): 7.11-7.06 (m, 2H), 6.88-6.87 (m, 2H), 6.79-6.77 (m, 1H), 6.70-6.68. (m, 1H), 6.63 (m, 1H), 4.68-4.61 (m, 1H), 4.23-4.02 (m, 4H), 3.81-3.78 (m, 4H), 3.63 (m, 2H), 3.28-3.26 (m, 2H), 3.18 (m, 2H), 2.96-2.91 (m, 1H), 2.65-2.63 (m, 2H), 2.24 (m, 2H), 2.05-1.87 (m, 5H), 1.45-1.43 (m, 4H).

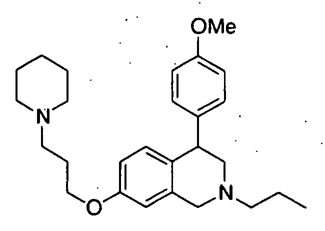

### Example 67; 4-(4-Methoxy-phenyl)-7-(3-piperidin-1-yl-propoxy)-2-propyl-1,2,3,4-tetrahydro-isoquinoline.

Prepared by a sequence similar to that used for for 2-ethyl-4-(4-methoxy-phenyl)-7-(3-piperidin-1-yl-propoxy)-1,2,3,4-tetrahydro-isoquinoline.

Step 1; 3-[4-(4-Methoxy-phenyl)-2-propyl-1,2,3,4-tetrahvdro-isoquinolin-7-yloxy]-propan-1-ol. Yield (1.05 mmol scale): 212 mg (57%) as a free base. MS (ESI): mass calcd for C₂₂H₂₉NO₃, 355.47; m/z fond, 356.5 [M+H]⁺. ¹H NMR (CDCl₃): 7.11-7.05 (m, 2H), 6.87 (d, J = 8.5, 2H), 6.80-6.76 (m, 2H), 6.74-6.65 (m, 1H), 4.72-4.61 (m, 2H), 4.15-4.06 (m, 3H), 3.85-3.77 (m, 5H), 3.14-3.11 (m, 2H), 2.95-2.90 (m, 1H), 2.03-2.02 (m, 2H), 1.90-1.86 (m, 2H), 1.03-1.00 (m, 3H).

Step 2; Methanesulfonic acid 3-[4-(4-methoxy-phenyl)-2-propyl-1,2,3,4-tetrahydro-isoquinolin-7-yloxy]-propyl ester. Yield (0.42 mmol scale): 260 mg (>100%) of the crude product. MS (ESI): mass calcd for C₂₃H₃₀NO₅S, 433.56; m/z found, 434.4 [M+H]⁺.

Step 3. Yield (0.42 mmol scale): 82 mg (30% over two steps) as a TFA salt. MS (ESI): mass calcd for C₂₇H₃₈N₂O₂, 422.60; m/z found, 423.5 [M+H]⁺. ¹H NMR (CDCl₃): 7.11-7.06 (m, 2H), 6.87 (d, J = 8.5, 2H), 6.79-6.78 (m, 1H), 6.70-6.81 (m, 1H), 6.61 (s, 1H), 4.69-4.61 (m, 1H), 4.11-4.02 (m, 3H), 3.81-3.75 (m, 4H), 3.67-3.65 (m, 3H), 3.14-3.11 (m, 4H), 2.96-2.91 (m, 1H), 2.65-2.63 (m, 2H), 2.25 (s, 2H), 2.04-1.97 (m, 2H), 1.89-1.87 (m, 5H), 1.46-1.41 (m, 1H), 1.02 (t, J = 7.4, 3H).

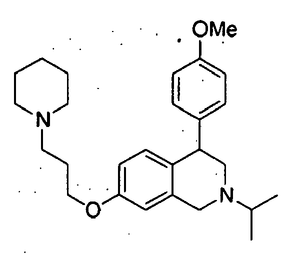

### Example 68; 2-Isopropyl-4-(4-methoxy-phenyl)-7-(3-piperidin-1-yl-propoxy)-1,2,3,4-tetrahydro-isoquinoline.

Prepared by a sequence similar to that used for for 2-ethyl-4-(4-methoxy-phenyl)-7-(3-piperidin-1-yl-propoxy)-1,2,3,4-tetrahydro-isoquinoline.

Step 1; 3-[2-Isopropyl-4-(4-methoxy-phenyl)-1,2,3,4-tetrahydro-isoauinolin-7-yloxy]-propan-1-ol. Yield (1.05 mmol scale): 239 mg (64%) crude product as a free base. MS (ESI): mass calcd for C₂₂H₂₉NO₃, 355.47; m/z found, 356.5 [M+H]⁺.

Step 2; Methanesulfonic acid 3-[2-isopropyl-4-(4-methoxy-phenyl)-1,2,3,4-tetrahydro-isoquinolin-7-yloxy]-propyl ester. Yield (0.59 mmol scale): 292 mg (>100%) of the crude product. MS (ESI): mass calcd for C₂₃H₃₁NO₅S, 433.56; m/z found, 434.4 [M+H]⁺.

Step 3. Yield (0.59 mmol scale): 119 mg (31% for two steps) as a TFA salt. MS (ESI): mass calcd for C₂₇H₃₈N₂O₂, 422.60; m/z found, 423.5 [M+H]⁺. ¹H NMR (CDCl₃): 7.82-7.80 (m, 2H), 7.58 (d, J = 8.4, 2H), 7.48-7.47 (m, 1H), 7.39-7.35 (m, 2H), 5.38-5.36 (m; 1H), 5.23-5.20 (m, 1H), 4.97-4.94 (m, 1H), 4.73-4.72 (m, 2H), 4.51 (s, 3H), 4.56-4.43 (m, 1H), 4.34-4.32 (m, 3H), 3.91-3.81 (m, 2H), 3.69-3.64 (m, 1H), 3.44-3.31 (m, 2H), 2.95-2.80 (m, 2H), 2.75-2.51 (m, 5H), 2.14-2.12 (m, 6H).

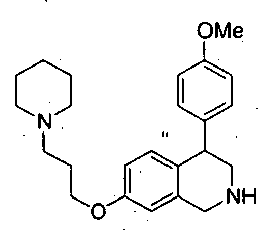

### Example 69; 4-(4-Methoxy-phenyl)-7-(3-piperidin-1-yl-propoxy)-1,2,3,4-tetrahydro-isoquinoline.

Prepared from 2-benzyl-4-(4-methoxy-phenyl)-7-(3-piperidin-1-yl-propoxy)-1,2,3,4-tetrahydro-isoquinoline as described in Example 66, Step 1, on a 6.05 mmol scale, to give 1.72 g (46%) of a TFA salt. MS (ESI): mass calcd for C₂₄H₃₂N₂O₂, 380.52; m/z found, 381.5 [M+H]⁺. ¹H NMR (CDCl₃): 7.01 (d, J = 8.7, 2H), 6.84 (d,

J = 8.7, 2H), 6.76 (d, J = 8.7, 1H), 6.67-6.65 (m, 1H), 6.60-6.59 (m, 1H), 4.37-4.29 (m, 3H), 3.99-3.97 (m, 2H), 3.78 (s, 3H), 3.63-3.58 (m, 3H), 3.19-3.16 (m, 3H), 2.70-2.68 (m, 2H), 2.21-2.16 (m, 2H), 1.91-1.84 (m, 5H), 1.43-1.41 (m, 1H).

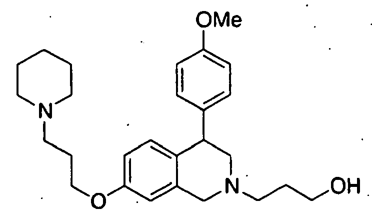

### Example 70; 3-[4-(4-Methoxy-phenyl)-7-(3-piperidin-1-yl-propoxy)-3,4-dihydro-1H-isoquinolin-2-yl]-propan-1-ol.

To a solution of 4-(4-methoxy-phenyl)-7-(3-piperidiniyl-propoxy)-1,2,3,4-tetrahydro-isoquinoline (90 mg, 0.236 mmol) in anhydrous DMF (0.3 M) were added K₂CO₃ (1.5 equiv.) and 3-bromo-propan-1-ol (1.05 equiv.). The resultant mixture was heated at 50 °C overnight. After cooling to rt, the mixture was diluted with EtOAc and washed with brine. The combined organic layers were dried over Na₂SO₄ and concentrated. Purification by FCC and reverse phase HPLC gave the desired product (19.1 mg, 3%) as a TFA salt. MS (ESI): mass calcd for C₂₇H₃₈N₂O₃, 438.6; m/z found, 439.5 [M+H]⁺. ¹H NMR (CDCl₃): 7.09-7.05 (m, 2H), 6.87 (d, J = 8.6, 2H), 6.80-6.75 (m, 1H), 6.72-6.70 (m, 1H), 6.64 (s, 1H), 4.04 (s, 2H), 3.82-3.80 (m, 6H), 3.66-3.65 (m, 2H), 3.40-3.36 (m, 2H), 3.25-3.15 (m, 2H), 2.9-2.87 (m, 2H), 2.78-2.65 (m, 4H), 2.25-2.23 (m, 2H), 2.08-1.88 (m, 8H), 1.45-1.40 (m, 2H).

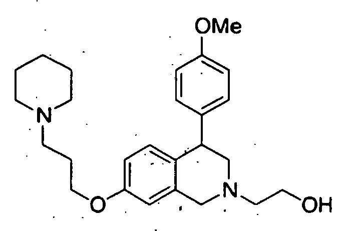

### Example 71; 2-[4-(4-Methoxy-phenyl)-7-(3-piperidin-1-yl-propoxy)-3,4-dihydro-1H-isoquinolin-2-yl]-ethanol.

Step 1; 2-[2-(tert-Butyl-dimethyl-silanyloxy)-ethyl]-4-(4-methoxy-phenyl)-7-(3-piperidin-1-yl-propoxy)-1,2,3,4-tetrahydro-isoquinoline. Prepared in a similar manner to that described for 3-[4-(4-methoxy-phenyl)-7-(3-piperidin-1-yl-propoxy)-3,4-dihydro-1H-isoquinolin-2-yl]-propan-1-ol, on a 0.23 mmol scale, to give product (74 mg, 58%). MS (ESI): mass calcd for C₃₂H₅₀N₂O₃Si, 538.8; m/z found, 539.6 [M+H]⁺.

Step 2. To a solution of 2-[2-(tert-butyl-dimethyl-silanyloxy)-ethyl]-4-(4-methoxy-phenyl)-7-(3-piperidin-1-yl-propoxy)-1,2,3,4-tetrahydro-isoquinoline (0.102 mmol, 55 mg) in anhydrous THF (5 mL) was added tetrabutylammonium fluoride (TBAF, 1 M in THF, 2 mL). The resulting solution was stirred at rt for 2 h. The solution was concentrated and purified by FCC and reverse phase HPLC to give the desired product (35 mg, 53%). MS (ESI): mass calcd for C₂₆H₃₆N₂O₃, 424.58; m/z found, 425.5 [M+H]⁺. ¹H NMR (CDCl₃): 7.06 (d, J = 8.5, 2H), 6.86 (d, J = 8.7, 2H), 6.85-6.63 (m, 3H), 4.85-4.21 (m, 4H), 4.13-4.01 (m, 4H), 3.83-3.75 (m, 4H), 3.63-3.61 (m, 2H), 3.35-3.21 (m, 2H), 3.20-3.16 (m, 2H), 2.70-2.64 (m, 2H), 2.25-2.21 (m, 2H), 2.00-1.86 (m, 5H), 1.45-1.40 (m, 1H).

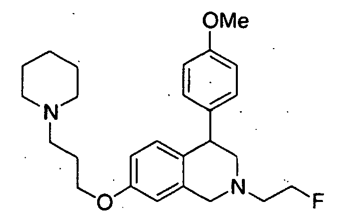

### Example 72; 2-(2-Fluoro-ethyl)-4-(4-methoxy-phenyl)-7-(3-piperidin-1-yl-propoxy)-1,2,3,4-tetrahydro-isoquinoline.

Prepared in a similar fashion to that described for 3-[4-(4-methoxy-phenyl)-7-(3-piperidin-1-yl-propoxy)-3,4-dihydro-1H-isoquinolin-2-yl]-propan-1-ol, on a 0.44 mmol scale, to give 166 mg (38%) of the product as a TFA salt. MS (ESI): mass calcd for C₂₆H₃₅FN₂O₂, 426.57; m/z found, 427.5 [M+H]⁺. ¹H NMR (CDCl₃): 7.08 (d, J = 8.5, 2H), 6.88 (d, J = 8.6, 2H), 6.84-6.82 (m; 1H), 6.73-6.71 (m, 1H), 6.62-6.61 (m, 1H), 5.00-4.99 (m, 1H), 4.91-4.88 (m, 1H), 4.61-4.51 (m, 2H), 4.05-4.03 (m, 2H), 3.86-3.83 (m, 1H), 3.81 (s, 3H), 3.67-3.66 (m, 2H), 3.62-3.60 (m, 1H), 3.56-3.55 (m, 1H), 3.21-3.19 (m, 3H), 2.67-2.65 (m, 2H), 2.26-2.23 (m, 2H), 2.05-1.88 (m, 6H), 1.43-1.41 (m, 1H).

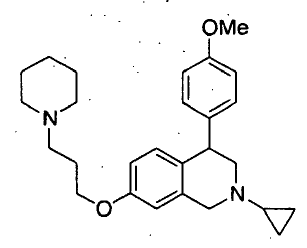

### Example 73; 2-Cyclopropyl-4-(4-methoxy-phenyl)-7-(3-piperidin-1-yl-propoxy)-1,2,3,4-tetrahydro-isoquinoline.

A solution of 4-(4-methoxy-phenyl)-7-(3-piperidin-1-yl-propoxy)-1,2,3,4-tetrahydro-isoquinoline (60 mg, 0.157 mmol), acetic acid (1.57mmol), molecular sieves (3Å), and [(1-ethoxycyclopropyl)oxy] trimethylsilane (0.96 mmol) in MeOH (0.2 M) was treated with NaCNBH₃ (0.71 mmol) and the resultant mixture stirred for 3 h at rt. The mixture was heated for 2 h at 50 °C, then cooled to rt and stirred for 2 h. The mixture was diluted with EtOAc and washed with brine. The combined organics were dried over Na₂SO₄ and concentrated. Purification by FCC and reverse phase HPLC gave the product (15 mg, 15%) as a TFA salt. MS (ESI): mass calcd for C₂₇H₃₆N₂O₂, 420.59; m/z found, 421.5 [M+H]⁺. ¹H NMR (acetone-d₆): 7.17-7.15 (m, 2H), 6.91-6.89 (m, 2H), 6.82 (s, 1H), 6.75-6.70 (m, 2H), 4.53-4.44 (m, 3H), 4.10-4.07 (m, 2H), 3.77 (s, 3H), 3.69-3.61 (m, 1H), 3.59-3.56 (m, 2H), 3.41-3.36 (m, 2H), 3.28-3.25 (m, 3H), 3.19-3.18 (m, 2H), 2.95-2.91 (m, 2H), 2.75-2.71 (m, 1H), 1.95-1.77 (m, 2H), 1.52-1.44 (m, 1H), 1.30-1.27 (m, 3H), 0.86-0.76 (m, 2H).

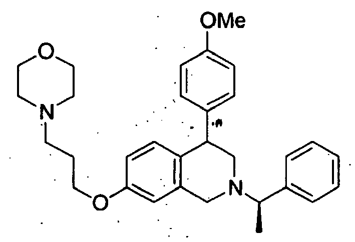

### Examples 74 and 75; 4-(4-Methoxy-phenyl)-7-(3-morpholin-4-yl-propoxy)-2-(1-phenyl-ethyl)-1,2,3,4-tetrahydro-isoquinoline.

Prepared by a sequence similar to that used for 2-benzyl-4-(4-methoxy-phenyl)-7-(3-piperidin-1-yl-propoxy)-1,2,3,4-tetrahydro-isoquinoline.

Step 1; 3-{3-[(1-Phenyl-ethylamino)-methyl]-phenoxy}-propan-1-ol. Yield (27.7 mmol scale): 7.73 g (96%) after FCC (EtOAc/hexanes). MS (ESI): mass calcd for C₁₈H₂₃NO₂, 285.2; m/z found, 286.4 [M+H]⁺. ¹H NMR (MeOH-d₄): 7.32-7.16 (m, 5H), 6.83-6.78 (m, 3H), 4.91 (s, 2H), 4.03 (t, J = 7.8, 2H), 3.80-3.71 (m, 3H), 3.57-3.47 (m, 2H), 1.99-1.91 (m, 2H), 1.37-1.35 (d, J = 8.3, 3H).

Step 2; 2-[[3-(3-Hydroxy-propoxy)-benzyl]-(1-phenyl-ethyl)-amino]-1-(4-methoxy-phenyl)-ethanone. Yield (3.50 mmol scale): 673.3 mg (45%) after FCC (EtOAc/hexanes). MS (ESI): mass calcd for C₂₇H₃₁NO₄, 433.2; m/z found, 434.5 [M+H]⁺. ¹H NMR (acetone-d₆): 7.84-7.82 (m, 2H), 7.47 (d, J = 7.4, 2H), 7.34 (t, J = 7.5, 2H), 7.25-7.22 (m, 1H), 7.16 (t, J = 7.9, 1H), 6.95-6.88 (m, 4H), 6.77-6.75 (m, 1H), 4.16-4.11 (m, 1H), 4.02 (t, J = 6.3, 2H), 3.96 (d, J = 16.4, 1H), 3.84 (s, 3H), 3.78 (d, J = 16.4, 1H), 3.73 (t, J = 6.2, 2H), 3.69-3.60 (m, 3H), 1.97-1.92 (m, 2H), 1.41 (d, J = 6.8, 3H).

Step 3; 7-(3-Hydroxy-propoxy)-4-(4-methoxy-phenyl)-2-(1-phenyl-ethyl)-isoquinolinium salt. Yield (3.50 mmol scale): 1.64 g (89%) of crude product. MS (ESI): mass calcd for C₂₇H₂₈NO₃⁺, 414.2; m/z found, 414.5 [M]⁺.

Step 4; 3-[4-(4-Methoxy-phenyl)-2-(1-phenyl-ethyl)-1,2,3,4-tetrahydro-isoquinolin-7-yloxy]-propan-1-ol. Yield (3.50 mmol scale): 210.3 mg (11%) of the desired product after FCC (EtOAc/hexanes) as a 1:1 mixture of diastereoisomers. MS (ESI): mass calcd for C₂₇H₃₁NO₃, 417.2; m/z found, 418.5 [M+H]⁺. Step 5; Methanesulfonic acid 3-[4-(4-methoxy-phenyl)-2-(1-phenyl-ethyl)-1,2,3,4-tetrahydro-isoquinolin-7-yloxy]-propyl ester. Reaction performed on a 0.328 mmol scale, to give a crude product that was taken directly on to the next step.

Step 6. Yield (0.328 mmol scale): 86.8 mg (54%) after FCC as a 1:1 mixture of diastereoisomers. MS (ESI): mass calcd for C₃₁H₃₈N₂O₃, 486.29; m/z found, 487.5 [M+H]⁺.

The isomers were separated by chiral HPLC (Chiralpak AD-H column, 5 µM, 97% hexane/IPA modified with 0.2% Et₂NH).

First eluting isomer (Example 74): MS (ESI): mass calcd for C₃₁H₃₈N₂O₃, 486.29; m/z found, 487.5 [M+H]⁺. ¹H NMR (MeOH-d₄): 7.23-7.17 (m, 5H), 6.97 (d, J = 8.6, 2H), 6.80-6.78 (m, 2H), 6.69 (d, J = 8.5, 1H), 6.63-6.60 (m, 1H), 6.57 (d, J = 2.3, 1H), 4.85 (s, 2H), 4.09-4.06 (br m, 1H), 3.94 (t, J = 6.1, 2H), 3.75 (s, 3H), 3.73-3.61 (m, 6H), 3.56-3.52 (m, 1H), 3.05-3.02 (m, 1H), 2.52-2.43 (m, 7H), 1.95-1.89 (m, 2H), 1.40 (d, J = 6.7, 3H).

Second eluting isomer (Example 75): MS (ESI): mass calcd for C₃₁H₃₈N₂O₃, 486.29; m/z found, 487.5 [M+H]⁺. ¹H NMR (MeOH-d₄): 7.31-7.27 (m, 4H), 7.23-7.21 (m, 1H), 6.96 (d, J = 8.6, 2H), 6.80-6.77 (m, 2H), 6.67-6.60 (m, 3H), 4.85 (s, 3H), 4.04-4.01 (m, 1H), 3.98-3.94 (m, 3H), 3.73 (s, 3H), 3.69-3.67 (m, 4H), 3.60-3.54 (m, 2H), 3.04-3.01 (m, 1H), 2.53-2.47 (m, 6H), 2.37-2.32 (m, 1h), 1.96-1.91 (m, 2H), 1.44 (d, J = 6.7, 3H).

The following Examples 76-77 were prepared by a sequence similar to that used in Example 1.

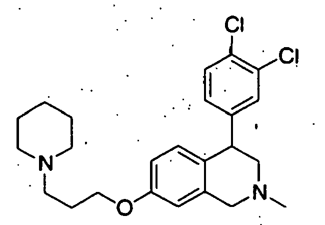

### Example 76; 4-(3,4-Dichloro-phenyl)-2-methyl-7-(3-piperidin-1-yl-propoxy)-1,2,3,4-tetrahydro-isoquinoline.

MS (ESI): mass calcd for C₂₄H₃₀Cl₂N₂O, 432.2; m/z found, 433.4, 435.4 [M+H]⁺. ¹H NMR (MeOH-d₄): 7.55 (d, J = 8.1, 1H), 7.44-7.38 (m, 1H), 7.18 (br d, J = 8.6, 1H), 6.93-6.80 (m, 3H), 4.61-4.49 (m, 3H), 4.10 (dd, J = 5.8, 5.3, 2H), 3.87-3.80 (m, 1H), 3.63-3.50 (m, 3H), 3.16-3.11 (m, 1H), 3.06 (s, 3H), 2.96 (dt, J = 12.8, 2.8, 2H), 2.28-2.18 (m, 2H), 2.02-1.93 (m, 2H), 1.90-1.68 (m, 4H), 1.59-1.47 (m, 1H).

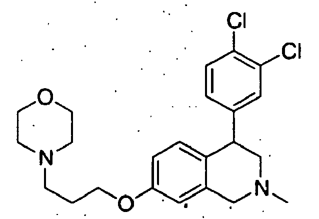

### Example 77; 4-(3,4-Dichloro-phenyl)-2-methyl-7-(3-morpholin-4-yl-propoxy)-1,2,3,4-tetrahydro-isoquinoline.

MS (ESI): mass calcd for C₂₃H₂₈Cl₂N₂O₂, 434.15; m/z found, 435.4 437.4 [M+H]⁺. ¹H NMR (MeOH-d₄): 7.55 (d, J = 8.1, 1H), 7.45-7.39 (m, 1H), 7.19 (br dd, J = 8.4, 2.0, 1H), 6.93-6.80 (m, 3H), 4.61-4.49 (m, 3H), 4.12 (dd, J = 5.6, 5.6, 3H), 4.09-4.02 (m, 1H), 3.89-3.72 (m, 3H), 3.60-3.45 (m, 3H), 3.38 (dd, J = 8.1, 7.8, 2H), 3.26-3.12 (m, 2H), 3.06 (s, 3H), 2.30-2.21 (m, 2H).

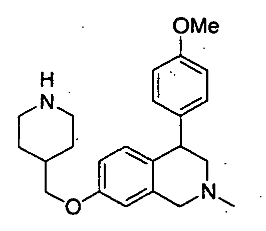

### Example 78; 4-(4-Methoxy-phenyl)-2-methyl-7-(piperidin-4-ylmethoxy)-1,2,3,4-tetrahydro-isoquinoline.

Step 1; 4-(3-Formyl-phenoxymethyl)-piperidine-1-carboxylic acid tert-butyl ester. A solution of 3-hydroxybenzaldehyde (2.45 g, 20.1 mmol) in DCM (500 mL) was treated with 4-hydroxymethyl-piperidine-1-carboxylic acid tert-butyl ester (4.45 g, 20.7 mmol), PPh₃ (5.45 g, 20.6 mmol), and diethylazodicarboxylate (DEAD; 3.15 mL, 20.0 mmol). The resulting mixture was stirred for 16 h at 23 °C. Evaporation of the solvent was followed by FCC (EtOAc/hexanes) gave the desired product (4.27 g, 67%). ¹H NMR (CDCl₃): 9.98 (s, 1H), 7.47-7.42 (m, 3H), 7.20-7.14 (m, 1H), 4.26-4.11 (m, 2H), 3.87 (d, J = 3.6, 2H), 2.84-2.69 (m, 2H), 2.03-1.93 (m, 1H), 1.83 (d, J = 12.6, 2H), 1.47 (s, 9H), 1.34-1.23 (m, 2H).

Step 2; 4-(3-Methylaminomethyl-phenoxymethyl)-piperidine-1-carboxylic acid tert-butyl ester. To a solution of 4-(3-formyl-phenoxymethyl)-piperidine-1-carboxylic acid tert-butyl ester (5.95 g, 18.6 mmol) in MeOH (100 mL) was added MeNH₂ (4 mL, 40% solution in water) and the mixture was stirred at 23 °C for 1 h. The mixture was then cooled to 0 °C and NaBH₄ (1.34 g, 35.8 mmol) was added in four portions. After 3 d at 23 °C, the mixture was concentrated, diluted with 1 M NaOH, and stirred for 1 h. The mixture was extracted with EtOAc, dried (Na₂SO₄), and concentrated to give the desired compound (5.83 g, 94%).

Step 3. To a solution of 4-(3-methylaminomethyl-phenoxymethyl)-piperidine-1-carboxylic acid tert-butyl ester (1.5 g, 4.49 mmol) in THF (35 mL) was added DIPEA (2.4 mL) and 2-bromo-1-(4-methoxy-phenyl)-ethanone (1.05 g, 4.59 mmol). The mixture was stirred at 23 °C for 15 h. The mixture was diluted with satd. aq. NaHCO₃, extracted with DCM, dried (Na₂SO₄), and concentrated. The residue was diluted with MSA (10 mL) and was stirred for 3 h at 23 °C. DCM was then added followed by 1 M aq. KOH. The mixture was extracted with DCM, dried (Na₂SO₄), and concentrated. The residue was treated in a similar manner as 7-(3-hydroxy-propoxy)-4-(4-methoxy-phenyl)-2-methyl-isoquinolinium salt to give the desired compound (0.452 g, 27%). MS (ESI): mass calcd for C₂₃H₃₀N₂O₂, 366.23; m/z found, 367.5 [M+H]⁺. ¹H NMR (CDCl₃): 7.10 (d, J = 8.8, 2H), 6.83 (d, J = 8.8, 2H), 6.77 (d, J = 8.6, 1H), 6.65-6.57 (m, 2H), 4.15 (dd, J = 8.5, 5.7, 1H), 3.79 (s, 3H), 3.77-3.67 (m, 3H), 3.56 (d, J = 15, 1H), 3.20-3.15 (m, 2H), 3.00-2.95 (m, 1H), 2.68 (ddd, J = 12.5, 12.0, 2.5, 2H), 2.49 (dd, J= 11.3, 8.9, 1H), 2.41 (s, 3 H), 1.89-1.78 (m, 2H).

The following Examples 79-82 were prepared by a sequence similar to that used in Example 78.

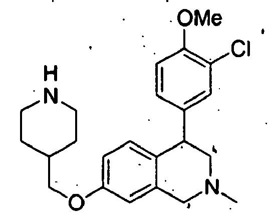

### Example 79; 4-(3-Chloro-4-methoxy-phenyl)-2-methyl-7-(piperidin-4-ylmethoxy)-1,2,3,4-tetrahydro-isoquinoline.

Yield (3.32 mmol scale): 0.583 g (44%). MS (ESI): mass calcd for C₂₃H₂₉ClN₂O₂, 400.94; m/z found, 401.4 [M+H]⁺. ¹H NMR (CDCl₃): 7.20 (d, J = 2.0, 1H), 7.04 (dd, J = 8.2, 2.3, 1H), 6.84 (d, J = 8.3, 1H), 6.76 (d, J = 8.6, 2.6, 1H), 6.59 (d, J = 2.5, 1H), 4.11 (dd, J = 7.9, 5.6, 1H), 3.88 (s, 3H), 3.75 (d, J = 6.3, 2H), 3.66 (d, J = 14.8, 1H), 3.58 (d, J = 14.9, 1H), 3.15-3.09 (m, 2H), 2.95 (dd, J = 11.6, 5.5, 1H), 2.65 (ddd, J = 12.1, 11.8, 3.6, 2H), 2.51 (dd, J = 11.3, 8.4, 1H), 2.41 (s, 3H), 1.96-1.77 (m, 3H), 1.33-1.20 (m, 2H).

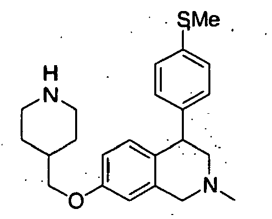

### Example 80; 2-Methyl-4-(4-methylsulfanyl-phenyl)-7-(piperidin-4-ylmethoxy)-1,2,3,4-tetrahydro-isoquinoline.

Yield (3.47 mmol scale): 0.552 g (42%). MS (ESI): mass calcd for C₂₃H₃₀N₂O₂S, 382.56; m/z found, 383.5 [M+H]⁺. ¹H NMR (CDCl₃): 7.18 (d, J = 8.4, 1H), 7.11 (d, J = 8.3, 1H), 6.76 (d, J = 8.3, 1H), 6.65-6.57 (m, 2H), 4.16 (dd, J = 7.7, 6.0, 1H), 3.74 (d, J = 6.4, 2H), 3.69 (d, J = 14.6, 1H), 3.58 (d, J = 14.8, 1H), 3.16-3.08 (m 2H), 2.97 (ddd, 11.4, 5.5, 1.0, 1H), 2.64 (ddd, 12.1, 12.1, 2.5, 2H), 2.55-2.47 (m, 1H), 2.47 (s, 3H), 2.41 (s, 3H), 1.96-1.77 (m, 3H), 1.32-1.19 (m, 2H).

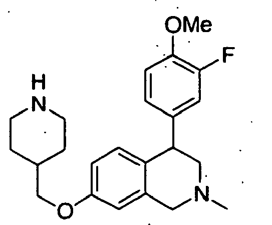

### Example 81; 4-(3-Fluoro-4-methoxy-phenyl)-2-methyl-7-(piperidin-4-ylmethoxy)-1,2,3,4-tetrahydro-isoquinoline.

Yield (3.05 mmol scale): 0.379 g (32%). MS (ESI): mass calcd for C₂₃H₂₉FN₂O₂, 384.22; m/z found, 385.5 [M+H]⁺. ¹H NMR (CDCl₃): 6.94 (m, 3H), 6.77 (d, J = 8.6, 1H), 6.64 (dd, J = 8.7, 2.8, 1H), 6.59 (d, J = 2.7, 1H), 4.12 (dd, J = 7.4, 6.0, 1H), 3.87 (s, 3H), 3.75 (d, J = 6.6, 2H), 3.66 (d, J = 14.9, 1H), 3.58 (d, J = 14.9, 1H), 3.15-3.08 (m, 2H), 2.95 (ddd, J = 11.3, 4.5, 1.0, 1H), 2.65 (ddd, J = 12.4, 12.1, 2.6, 2H), 2.52 (dd, J = 11.3, 8.1, 1H), 2.41 (s, 3H), 1.95-1.78 (m, 3H), 1.33-1.20 (m, 2H).

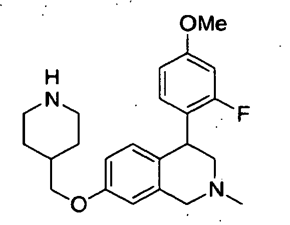

### Example 82; 4-(2-Fluoro-4-methoxy-phenyl)-2-methyl-7-(piperidin-4-ylmethoxy)-1,2,3,4-tetrahydro-isoquinoline.

Yield (3.20 mmol scale): 0.492 g (40%). MS (ESI): mass calcd for C₂₃H₂₉FN₂O₂, 384.49; m/z found, 385.5 [M+H]⁺. ¹H NMR (CDCl₃): 6.93 (dd, J = 8.5, 8.3, 1H), 6.79 (d, J = 8.6, 1H), 6.68-6.55 (m, 4H), 4.80 (dd, J = 6.4, 6.2, 1H), 3.77 (s, 3H), 3.75 (d, J = 6.3, 2H), 3.62 (dd, J = 13.4, 11.4, 2H), 3.17-3.09 (m, 2H), 2.92 (dd, J = 11.3, 5.5, 1H), 2.70-2.55 (m, 3H), 2.40 (s, 3H), 1.97-1.77 (m 3H), 1.34-1.21 (m, 2H).

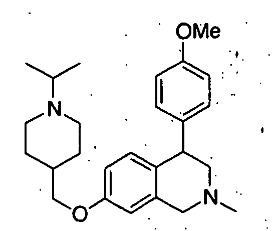

### Example 83; 7-(1-Isopropyl-piperidin-4-ylmethoxy)-4-(4-methoxy-phenyl)-2-methyl-1,2,3,4-tetrahydro-isoquinoline.

A solution of 4-(4-methoxy-phenyl)-2-methyl-7-(piperidin-4-ylmethoxy)-1,2,3,4-tetrahydro-isoquinoline (0.100 g, 0.273 mmol) in DCM (5 mL) was treated with acetone (0.1 mL), acetic acid (0.030 mL), and NaB(OAc)₃H (0.081 g, 0.382 mmol). After 15 h, 1 M NaOH was added and the reaction was stirred for 0.5 h. The mixture was extracted with DCM, the organic layer was dried (Na₂SO₄) and concentrated to give a residue which was purified by FCC to give 7-(1-isopropyl-piperidin-4-ylmethoxy)-4-(4-methoxy-phenyl)-2-methyl-1,2,3,4-tetrahydro-isoquinoline (9.1 mg, 8%).

The following Examples 84-121 were prepared by a sequence similar to that used in Example 83, except where otherwise noted.

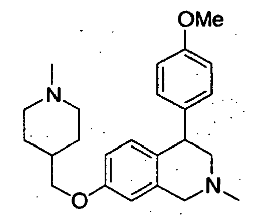

### Example 84; 4-(4-Methoxy-phenyl)-2-methyl-7-(1-methyl-piperidin-4-ylmethoxy)-1,2,3,4-tetrahydro-isoquinoline.

Yield (0.280 mmol scale): 5 mg (5%). MS (ESI): mass calcd for C₂₄H₃₂N₂O₂, 380.25; m/z found, 381.5 [M+H]⁺. ¹H NMR (CDCl₃): 7.09 (d, J = 8.6, 2H), 6.83 (d, J = 8.8, 2H), 6.76 (d, J = 8.1, 1H), 6.62 (dd, J = 8.3, 2.5, 1H)< 6.58 (d, J =- 2.3, 1H), 4.19-4.13 (m, 1H), 3.79 (s, 3H), 3.76 (d, J = 6.3, 2H), 3.71 (d, J = 15.2, 1H), 3.56 (d, J = 14.9, 1H), 2.98 (ddd, J = 11.1, 5.3, 1.0, 1H), 2.92-2.85 (m, 1H), 2.49 (dd, J = 11.3, 8.8, 1H), 2.41 (s, 3H), 2.28 (s, 3H), 1.96 (ddd, J = 14.4, 11.9, 2.0, 1H), 1.88-1.73 (m, 3H), 1.46-1.35 (m, 3H).

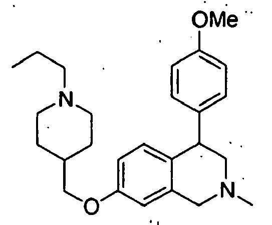

### Example 85; 1-(4-Methoxy-phenyl)-2-methyl-7-(1-propyl-piperidin-4-ylmethoxy)-1,2,3,4-tetrahydro-isoquinoline.

Yield (0.293 mmol scale): 34.2 mg (29%). MS (ESI): mass calcd for C₂₆H₃₆N₂O₂, 408.28; m/z found, 409.5 [M+H]⁺. ¹H NMR (CDCl₃): 7.09 (d, J = 8.6, 2H), 6.82 (d, J = 8.8, 2H), 6.76 (d, J = 8.6, 1H), 6.62 (dd, J = 8.3, 2.5, 1H), 6.58 (d, J =2.5, 1H), 4.15 (dd, J = 8.3, 5.6, 1H), 3.80-3.73 (m, 5H), 3.70 (d, J = 14.9, 1H), 3.56 (d, J = 14.9, 1H), 3.02-2.92 (m, 3H), 2.49 (dd, J = 11.4, 8.8, 1H), 2.41 (s, 3H), 2.33-2.25 (m; 2H), 1.94 (ddd, J = 13.4, 11.3, 1.8, 2H), 1.86-1.74 (m, 3H), 1.57-1.47 (m, 2H), 1.46-1.37 (m, 2H), 0.90 (t, J = 7.3, 3H).

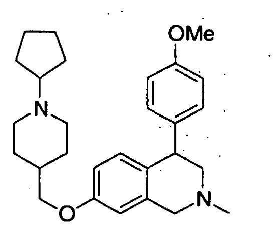

### Example 86; 7-(1-Cyclopentyl-piperidin-4-ylmethoxy)-4-(4-methoxy-phenyl)-2-methyl-1,2,3,4-tetrahydro-isoquinoline.

Yield (0.293 mmol scale): 7.7 mg (6%). MS (ESI): mass calcd for C₂₈H₃₈N₂O₂, 434.29; m/z found, 435.5 [M+H]⁺. ¹H NMR (CDCl₃): 7.09 (d, J = 8.8, 2H), 6.82 (d, J = 8.8, 2H), 6.76 (d, J = 8.6, 1H), 6.62 (dd, J = 8.3, 2.5, 1H), 6.58 (d, J = 2.5, 1H), 4.15 (dd, J = 8.3, 5.3, 1H), 3.79 (s, 3H), 3.75 (d, J = 6.1, 1H), 3.70 (d, J = 14.9, 1H), 3.56 (d, J = 14.9, 1H), 3.10-3.02 (m, 2H), 2.98 (ddd, J = 11.4, 5.6, 1.0, 1H), 2.52-2.42 (m, 2H), 2.41 (s, 3H), 1.99-1.34 (m, 15H).

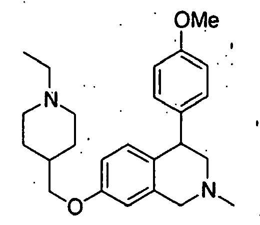

### Example 87; 7-(1-Ethyl-piperidin-4-ylmethoxy)-4-(4-methoxy-phenyl)-2-methyl-1,2,3,4-tetrahydro-isoquinoline.

Yield (0.293 mmol scale): 7.1 mg (6%). MS (ESI): mass calcd for C₂₅H₃₄N₂O₂, 394.26; m/z found, 395.5 [M+H]⁺. ¹H NMR (CDCl₃): 7.09 (d, J = 8.8, 2H), 6.82 (d, J = 8.8, 2H), 6.76 (d, J = 8.6, 1H), 6.62 (dd, J = 8.6, 2.8, 1H), 6.59 (d, J = 2.5, 1H), 4.15 (dd, J = 8.6, 5.8 1H), 3.79 (s, 3H), 3.76 (d, J = 6.3, 2H), 3.70 (d, J = 14.9, 1H), 3.56 (d, J = 14.9, 1H), 3.02-2.94 (m, 3H), 2.49 (dd, J = 11.3, 8.8, 1H), 2.43-2.37 (m, 5H), 1.97-1.80 (m, 5H), 1.47-1.37 (m, 2H), 1.10 (t, J = 7.3, 3H).

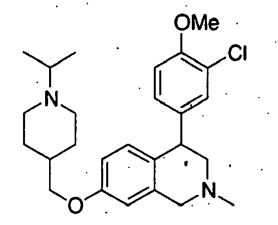

### Example 88; 4-(3-Chloro-4-methoxy-phenyl)-7-(1-isopropyl-piperidin-4-ylmethoxy)-2-methyl-1,2,3,4-tetrahydro-isoquinoline.

Yield (0.293 mmol scale): 57.3 mg (44%). ¹H NMR (CDCl₃): 7.20 (d, J = 2.1, 1H), 7.03 (dd, J = 8.5, 2.2, 1H), 6.84 (d, J = 8.2, 1H), 6.76 (d, J = 8.4, 1H), 6.64 (dd, J = 8.6, 2.5, 1H), 6.59 (d J = 2.6, 1H), 4.11 (dd, J = 6.9, 6.4, 1H), 3.88 (s, 3H), 3.76 (d, J = 6.3, 2H), 3.67 (d, J = 14.5, 1H), 3.58 (d, J = 15.0, 1H), 2.98-2.89 (m, 3H), 2.76-2.68 (m, 1H), 2.50 (dd, J = 11.4, 8.3, 1H), 2.41 (s, 3H), 2.19-2.11 (m, 2H), 1.89-1.70 (m, 3H), 1.43-1.33 (m, 2H), 1.05 (d, J = 6.6, 6H).

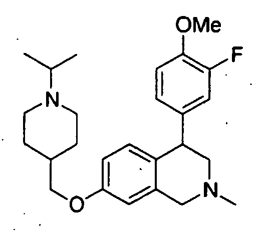

### Example 89; 4-(3-Fluoro-4-methoxy-phenyl)-7-(1-isopropyl-piperidin-4-ylmethoxy)-2-methyl-1,2,3,4-tetrahydro-isoquinoline.

Yield (0.318 mmol scale): 68.2 mg (50%). MS (ESI): mass calcd for C₂₆H₃₅FN₂O₂, 426.57; m/z found, 427.5 [M+H]⁺. ¹H NMR (CDCl₃): 6.95-6.82 (m, 3H), 6.76 (d, J = 8.7, 1H), 6.64 (dd, J = 8.6, 2.8, 1H), 6.59 (d, J = 2.5, 1H), 4.12 (dd, J = 7.1, 6.2, 1H), 3.87 (s, 3H), 3.75 (d, J = 6.6, 2H), 3.65 (d,'J = 14.9, 1H), 3.58 (d J = 14.6, 1H), 3.02-2.88 (m, 3H), 2.71 (m, 1H), 2.51 (dd, J = 11.4, 8.1, 1H), 2.40 (s, 3H), 2.19-2.10 (m, 2H), 1.96-1.80 (m, 3H), 1.45-1.30 (m, 2H), 1.05 (d, J = 6.6, 6H).

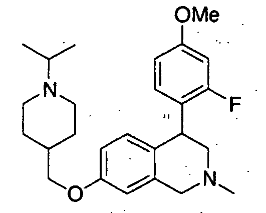

### Example 90; 4-(2-Fluoro-4-methoxy-phenyl)-7-(1-isopropyl-piperidin-4-ylmethoxy)-2-methyl-1,2,3,4-tetrahydro-isoquinoline.

Yield (0.372 mmol scale): 79.3 mg (50%). MS (ESI): mass calcd for C₂₆H₃₅FN₂O₂, 426.27; m/z found, 427.5 [M+H]⁺. ¹H NMR (CDCl₃): 6.92 (t, J = 8.6, 1H), 6.79 (d, J = 8.7, 1H), 6.66-6.54 (m, 4H), 4.48 (dd, J = 6.4, 6.2, 1H), 3.79-3.73 (m, 5H), 3.63 (d, J = 13.1, 1H), 3.60 (d, J = 13.1, 1H), 2.96-2.88 (m, 3H), 2.71 (h, J = 6.5, 1H), 2.58 (dd, J = 11.3, 7.5, 1H), 2.40 (s, 3H), 2.20-2.10 (m, 2H), 1.96-1.72 (m, 3H), 1.42-1.32 (m, 2H), 1.05 (d, J = 6.6, 6H).

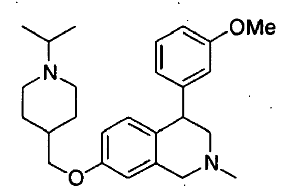

### Example 91; 7-(1-Isopropyl-piperidin-4-ylmethoxy)-4-(3-methoxy-phenyl)-2-methyl-1,2,3,4-tetrahydro-isoquinoline.

Yield (0.245 mmol scale): 51.2 mg (51 %). MS (ESI): mass calcd for C₂₆H₃₆N₂O₂, 408.28; m/z found, 409.5 [M+H]⁺. ¹H NMR (CDCl₃): 7.22-7.15 (m, 1H), 6.80-6.73 (m, 4H), 6.62 (dd, J = 8.3, 2.5, 1H), 6.59 (d, J = 2.5, 1H), 4.18 (dd, J = 6.8, 5.6, 1H), 3.77 (s, 3H), 3.75 (d, J = 6.6, 2H), 3.71 (d, J = 14.9, 1H), 3.56 (d, J = 15.1, 1H), 3.01 (ddd, 11.4, 5.6, 1.3, 1H), 2.95-2.89 (m, 2H), 2.71 (h, J = 6.6, 1H), 2.53 (dd, J = 11.6, 8.8, 1H), 2.41 (s, 3H), 2.14 (ddd, J = 14.1, 11.8, 1.8, 2H), 1.89-1.81 (m, 1H), 1.81-1.71 (m, 1H), 1.43-1.31 (m, 3H), 1.05 (d, J = 6.6, 6H).

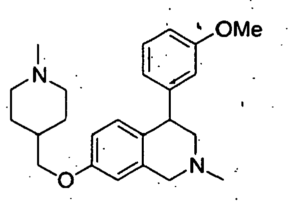

### Example 92; 4-(3-Methoxy-phenyl)-2-methyl-7-(1-methyl-piperidin-4-ylmethoxy)-1,2,3,4-tetrahydro-isoquinoline.

Yield (0.283 mmol scale): 0.030 g (28%). MS (ESI): mass calcd for C₂₄H₃₂N₂O₂, 380.25; m/z found, 381.5 [M+H]⁺. ¹H NMR (CDCl₃): 7.20-7.17 (m, 1H), 6.80-6.72 (m, 4H), 6.62 (dd, J = 8.6, 2.8, 1H), 6.58 (d, J = 2.5, 1H), 4.17 (dd, J = 8.3, 5.8, 1H), 3.76 (m, 5H), 3.70 (d, J = 14.9, 1H), 3.54 (d, J = 152, 1H), 3.00 (ddd, J = 11.9, 5.8, 1.3, 1H), 2.92-2.85 (m, 2H), 2.53 (dd, J = 11.6, 8.8, 1H), 2.41 (s, 3H), d.28 (s, 3H), 1.95 (ddd, J = 14.1, 11.9, 2.3, 2H), 1.87-1.71 (m, 3H), 1.47-1.36 (m, 2H).

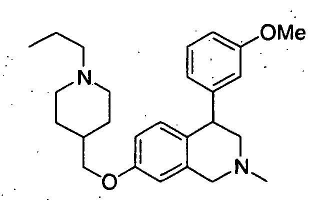

### Example 93; 4-(3-Methoxy-phenyl)-2-methyl-7-(1-propyl-piperidin-4-ylmethoxy)-1,2,3,4-tetrahydro-isoquinoline.

Yield (0.273 mmol scale): 14.6 mg (13%). MS (ESI): mass calcd for C₂₆H₃₆N₂O₂, 408.28; m/z found, 409.5 [M+H]⁺. ¹H NMR (CDCl₃): 7.20 (dd, J = 7.6, 7.3, 1H), 6.80-6.73 (m, 4H), 6.62 (dd, J = 8.3, 2.5, 1H), 6.59 (d, J = 2.5, 1H), 4.18 (dd, J = 8.1, 6.1, 1H), 3.77 (s, 3H), 3.77-3.73 (m, 2H), 3.71 (d, J = 14.9, 1H), 3.56 (d, J = 15.2, 1H), 3.04-2.94 (m, 3H), 2.53 (dd, J = 11.4, 8.8, 1H), 2.41 (s, 3H), 2.32-2.25 (m, 2H), 1.99-1.74 (m, 5H), 1.58-1.47 (m, 2H), 1.46-1.37 (m, 2H), 0.90 (t, J = 7.3, 3H).

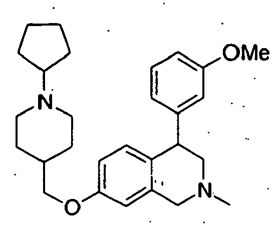

### Example 94; 7-(1-Cyclopentyl-pipendin-4-ylmethoxy)-4-(3-methoxy-phenyl)-2-methyl-1,2,3,4-tetrahydro-isoquinoline.

Yield (0.273 mmol scale): 32.8 mg (28%). MS (ESI): mass calcd for C₂₈H₃₈N₂O₂, 434.29; m/z found, 435.5 [M+H]⁺. ¹H NMR (CDCl₃): 7.20 (dd, J = 7.8, 7.8, 1H), 6.80-6.73 (m, 4H), 6.63 (dd, J = 8.3, 2.5, 1H), 6.58 (d, J = 2.5, 1H), 4.18 (dd, J = 8.3, 6.1, 1H), 3.77 (s, 3H), 3.77-3.73 (m, 2H), 3.70 (d, J = 14.9, 1H), 3.56 (d, J = 15.2, 1H), 3.11-3.04 (m, 2H), 3.01 (ddd, J = 11.6, 5.6, 1.3, 1H), 2.53 (dd, J = 11.4, 8.8, 1H), 2.50-2.44 (m, 1H), 2.41 (s, 3H), 2.00-1.36 (m, 16H).

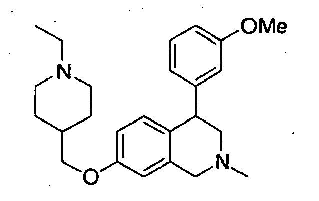

### Example 95; 7-(1-Ethyl-piperidin-4-ylmethoxy)-4-(3-methoxy-phenyl)-2-methyl-1,2,3,4-tetrahydro-isoquinoline.

Yield (0.273 mmol scale): 16.8 mg (16%). ¹H NMR (CDCl₃): 7.20 (dd, J = 7.8, 7.8, 1H), 6.81-6.72 (m, 4H), 6.62 (dd, J = 8.6, 2.8, 1H), 6.59 (d, J = 2.5, 1H), 4.18 (dd, J = 8.3, 5.8, 1H), 3.79-3.74 (m, 5H), 3.70 (d, J = 14.9, 1H), 3.57 (d, J = 15.7, 1H), 3.04-2.96 (m, 3H), 2.53 (dd, J = 11.4, 8.8, 1H), 2.45-2.37 (m, 5H), 1.98-1.75 (m, 5H), 1.47-1.37 (m, 2.H), 1.10 (t, J = 7.1, 3H).

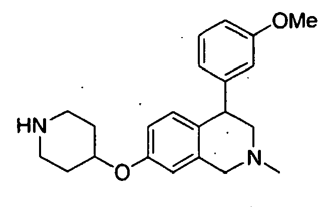

### Example 96; 4-(3-Methoxy-phenyl)-2-methyl-7-(piperidin-4-yloxy)-1,2,3,4-tetrahydro-isoquinoline.

The title compound was prepared using methods similar to those in Example 78. MS (ESI): mass calcd for C₂₂H₂₈N₂O₂, 352.22; m/z found, 353.5 [M+H]⁺. ¹H NMR (CDCl₃): 7.16-7.11 (m, 1H), 6.73-6.66 (m, 4H), 6.59-6.53 (m, 2H), 4.3-4.2 (m, 1H), 4.10 (dd, J = 5.7, 8.4, 1H), 3.70 (s, 3H), 3.62 (d, J = 14.9, 1H), 3.49 (d, J = 14.9, 1H), 3.12-3.01 (m, 2H), 2.97-2.89 (m, 1H), 2.91-2.60 (m, 2H), 2.46 (dd, J = 11.4, 8.8, 1H), 2.34 (s, 3H), 1.99-1.88 (m; 2H), 1.65-1.51 (m, 2H).

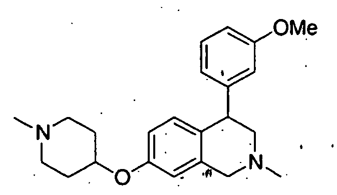

### Example 97; 4-(3-Methoxy-phenyl)-2-methyl-7-(1-methyl-piperidin-4-yloxy)-1,2,3,4-tetrahydro-isoquinoline.

MS (ESI): mass calcd for C₂₃H₃₀N₂O₂, 366.23; m/z found, 367.5 [M+H]⁺. ¹H NMR (CDCl₃): 7.20 (t, J = 9.7, 1H), 6.81-6.73 (m, 4H), 6.65-6.58 (m, 2H), 4.38 (br s, 1H), 4.20-4.14 (m, 1H), 3.76 (s, 3H), 3.71-3.67 (d, J = 14.8, 1H), 3.59-3.53 (d, J = 14.8, 1H), 3.04-2.98 (m, 1H), 2.87-2.78 (m, 2H), 2.67-2.49 (m, 3H), 2.46 (s, 3H), 2.45 (s, 3H), 2.24-2.10 (m, 2H), 1.98-1.85 (m, 2H), 1.70-1.54 (m, 1H), 1.35-1.61 (m, 6H), 0.92-0.75 (m, 2H).

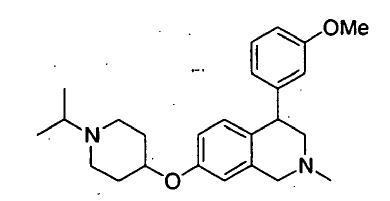

### Example 98; 7-(1-Isopropyl-piperidin-4-yloxy)-4-(3-methoxy-phenyl)-2-methyl-1,2,3,4-tetrahydro-isoquinoline.

MS (ESI): mass calcd for C₂₅H₃₄N₂O₂, 394.26; m/z found, 395.5 [M+H]⁺. ¹H NMR (CDCl₃): 7.27-7.21 (m, 1H), 6.84-6.77 (m, 4H), 6.70-6.63 (m, 2H), 4.35-4.25 (m, 1H), 4.24-4.17 (m, 1H), 3.81 (s, 3H), 3.73 (d, J = 14.9, 1H), 3.59 (d, J = 14.9, 1H), 3.08-3.01 (m, 1H), 2.89-2.76 (m, 3H), 2.57 (dt, J = 11.5, 8.8, 1H), 2.50-2.40 (m, 5H), 2.12-2.00 (m, 2H), 1.91-1.79 (m, 2H), 1.11 (d, J = 6.65, 6H).

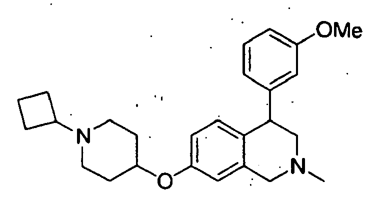

### Example 99; 7-(1-Cyclobutyl-piperidin-4-yloxy)-4-(3-methoxy-phenyl)-2-methyl-1,2,3,4-tetrahydro-isoquinoline.

MS (ESI): mass calcd for C₂₆H₃₄N₂O₂, 406.26; m/z found, 407.5 [M+H]⁺. ¹H NMR (CDCl₃): 7.23-7.17 (m, 1H), 6.81-6.71 (m, 4H), 6.65-6.57 (m, 2H), 4.40-4.27 (m, 1H), 4.20-4.13 (m, 1H), 3.76 (s, 3H), 3.69 (d, J = 14.8, 1H), 3.55 (d, J = 14.8, 1H), 3.03-2.97 (m, 1H), 2.92-2.77 (m, 1H), 2.74-2.60 (m, 2H), 2.57-2.50 (m, 1H), 2.43-2.39 (m, 4H), 2.18-1.99 (m, 6H), 1.81-1.72 (m, 2H), 1.72-1.59 (m, 1H), 1.38-1.17 (m, 1H).

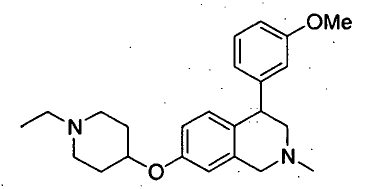

### Example 100; 7-(1-Ethyl-piperidin-4-yloxy)-4-(3-methoxy-phenyl)-2-methyl-1,2,3,4-tetrahydro-isoquinoline.

MS (ESI): mass calcd for C₂₄H₃₂N₂O₂, 380.25; m/z found, 381.5 [M+H]⁺. ¹H NMR (CDCl₃): 7.22-7.18 (m, 1H), 6.81-6.72 (m, 4H), 6.65-6.58 (m, 2H), 4.51-4.35 (br m, 1H), 4.19-1.12 (m, 1H), 3.76 (s, 3H), 3.69 (d, J = 14.8, 1H), 3.55 (d, J = 14.8, 1H), 3.04-2.98 (m, 1H), 2.95-2.86 (m, 1H), 2.80-2.68 (m, 2H), 2:53 (dd, J = 11.5, 8.8, 1H), 2.42 (s, 3H), 2.36-2.23 (m, 2H), 2.04-1.93 (m, 2H), 1.33-1.22 (m, 3H).

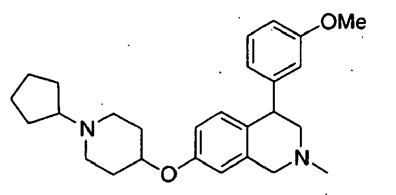

### Example 101; 7-(1-Cyclopentyl-piperidin-4-yloxy)-4-(3-methoxy-phenyl)-2-methyl-1,2,3,4-tetrahydro-isoquinoline.

MS (ESI): mass calcd for C₂₇H₃₆N₂O₂, 420.28; m/z found, 421.5 [M+H]⁺. ¹H NMR (CDCl₃): 7.22-7.17 (m, 1H), 6.81-6.71 (m, 4H), 6.64-6.57 (m, 2H), 4.44-4.34 (br m, 1H), 1.20-4.13 (m, 1H), 3.76 (s, 3H), 3.69 (d, J = 14.8, 1H), 3.55 (d, J = 14.8, 1H), 3.04-2.96 (m, 1H), 2.96-2.86 (m, 2H), 2.56-2.50 (m, 2H), 2.53 (dd, J = 11.5, 8.8; 1H), 2.41 (s, 3H), 2.33-2.16 (m, 2H), 2.02-1.87 (m, 4H), 1.83-1.64 (m, 4H), 1.63-1.49 (m, 2H), 1.35-1.18 (m, 1H).

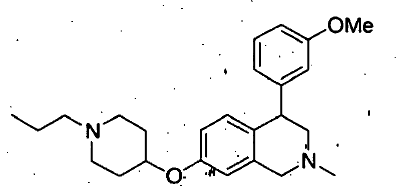

### Example 102; 4-(3-Methoxy-phenyl)-2-methyl-7-(1-propyl-piperidin-4-yloxy)-1,2,3,4-tetrahydro-isoquinoline.

MS (ESI): mass calcd for C₂₅H₃₄N₂O₂, 394.26; m/z found, 395.5 [M+H]⁺. ¹H NMR (CDCl₃): 7.22-7.18 (m, 1H), 7.80-6.73 (m, 4H), 6.65-6.59 (m, 2H), 4.33-4.24 (br m, 1H), 4.20-4.14 (m, 1H), 3.77 (s, 3H), 3.69 (d, J = 14.8, 1H), 3.69 (d, J = 14.8, 1H), 3.04-2.97 (m, 1H), 2.82-2.72 (br m, 2H), 2.53 (dd, J = 11.5, 8.8), 2.41 (s, 3H), 3.86-2.30 (m, 4H), 2.08-1.97 (m, 2H), 1.90-7.77 (m, 2H), 1.62-1.49 (m, 2H), 0.91 (t, J =7.4, 3H).

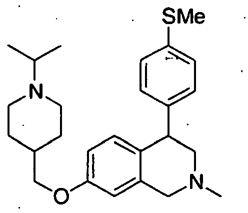

### Example 103; 7-(1-Isopropyl-piperidin-4-ylmethoxy)-2-methyl-4-(4-methylsulfanyl-phenyl)-1,2,3,4-tetrahydro-isoquinoline.

MS (ESI): mass calcd for C₂₆H₃₆N₂O₂, 424.25; m/z found, 425.5 [M+H]⁺: ¹H NMR (CDCl₃): 7.21-7.16 (m, 2H), 7.12-7.08 (m, 2H), 6.75 (d, J= 8.5, 1H), 6.63 (dd, J = 8.5, 2.7, 1H), 6.60-6.59 (m, 1H), 4.18-4.12 (m, 1H), 3.78-3.73 (m, 2H), 3.68 (d, J = 14.8, 1H), 3.58 (d, J = 14.8, 1H), 3.02-2.87 (m, 3H, 2.74-2.67 (m, 1H), 2.55-2.48 (m, 1H), 2.43 (s, 3H), 2.40 (s, 3H), 2.18-2.16(m, 1H), 2.15-2.10 (m, 1H), 1.65-1.20 (m, 5H), 1.45-1.31 (m, 2H), 1.31 (m, 1H), 1.05 (d, J =.6.6, 4H).

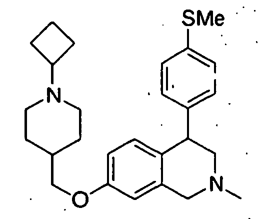

### Example 104; 7-(1-Cyclobutyl-piperidin-4-ylmethoxy)-2-methyl-4-(4-methylsulfanyl-phenyl)-1,2,3,4-tetrahydro-isoquinoline.

MS (ESI): mass calcd for C₂₇H₃₆N₂O₂, 436.25; m/z found, 437.5 [M+H]⁺. ¹H NMR (CDCl₃): 7.20-7.16 (m, 2H), 7.12-7.08 (m, 2H), 6.75 (d, J = 8.5, 1H), 6.12(dd, J = 8.5, 2.5, 1H), 6.59-6.57 (m, 1H), 4.17-4.13 (m, 1H), 3.75 (d, J = 6.3, 2H), 3.68 (d, J = 14.8, 1H), 3.57 (d, J = 14.8, 1H), 2.99-2.94 (m, 1H), 2.94-2.88 (m, 2H), 2.72-2.64 (m, 1H), 2.54-2.47 (m, 1H), 2.46 (s, 3H), 2.40 (s, 3H), 2.08-1.99 (m, 2H), 1.93-1.59 (m, 10H), 1.43-1.32 (m, 2H).

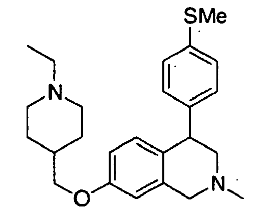

### Example 105; 7-(1-Ethyl-piperidin-4-ylmethoxy)-2-methyl-4-(4-methylsulfanyl-phenyl)-1,2,3,4-tetrahydro-isoquinoline.

MS (ESI): mass calcd for C₂₅H₃₅N₂O₂, 410.24; m/z found, 411.5 [M+H]⁺. ¹H NMR (CDCl₃): 7.21-7.15 (m, 2H), 7.13-7.07 (m, 2H), 6.75 (d, J = 8.5, 1H), 6.62 (dd, J = 8.5, 2.5, 1H), 6.60-6.57 (m, 1H), 4.18-4.12 (m, 1H), 3.75 (d, J = 6.1, 2H), 3.68 (d, J = 14.8, 1H), 3.57 (d, J = 14.8, 1H), 3.01-2.93 (m, 3H), 2.54-2.47 (m, 1H), 2.46 (s, 3H), 2.44-2.42 (m, 4H), 2.17 (s, 2H), 1.96-1.73 (m, 5H), 1.46-1.34 (m, 2H), 1.13-1.06 (m, 3H).

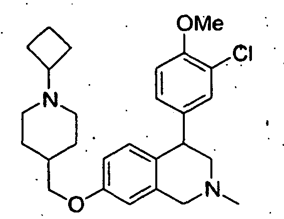

### Example 106; 4-(3-Chloro-4-methoxy-phenyl)-7-(1-cyclobutyl-piperidin-4-ylmethoxy)-2-methyl-1,2,3,4-tetrahydro-isoquinoline.

MS (ESI): mass calcd for C₂₇H₃₅ClN₂O₂, 454.24; m/z found, 455.5 [M+H]⁺. ¹H NMR (CDCl₃): 7.19 (d, J = 2.2, 1H), 7.03 (dd, J = 8.5, 2.2, 1H), 6.83 (d, J = 8.5, 1H), 6.75 (d, J = 8.5, 1H), 6.63 (dd, J = 8.5, 2.7, 1H), 6.60-6.57 (m, 1H), 4.14-4.08 (m, 1H), 3.87 (s, 3H), 3.76 (d, J = 6.0, 2H), 3.65 (d, J = 14.8, 1H), 3.58 (d, J = 14.8, 1H), 2.98-2.87 (m, 3H), 2.74-2.63 (m, 1H), 2.50 (dd, J = 11.5, 8.2, 1H), 2.40 (s, 3H), 2.09-1.99 (m, 2H), 1.96-1.57 (m, 10H), 1.45-1.31 (m, 2H).

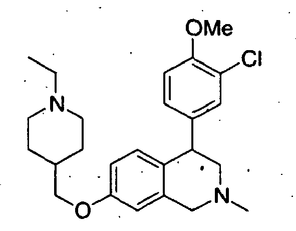

### Example 107; 4-(3-Chloro-4-methoxy-phenyl)-7-(1-ethyl-piperidin-4-ylmethoxy)-2-methyl-1,2,3,4-tetrahydro-isoquinoline.

MS (ESI): mass calcd for C₂₅H₃₃ClN₂O₂, 428.22; m/z found, 429.5 [M+H]⁺. ¹H NMR (CDCl₃): 7.19 (d, J = 1.9, 1H), 7.03 (dd, J = 8.2, 2.2, 1H), 6.83 (d, J = 8.5, 1H), 6.76 (d, J = 8.5, 1H), 6.63 (dd, J = 8.5, 2.5, 1H), 6.60-6.57 (m, 1H), 4.14-4.08 (m, 1H), 3.87 (s, 3H), 3.76 (d, J = 6.3, 2H), 3.66 (d, J = 14.8, 1H), 3.58 (d, J = 14.8, 1H), 3.04-2.90 (m, 3H), 2.53-2.46 (m, 1H), 2.45-2.37 (m, 5H), 1.98-1.88 (m, 2H), 1.88-1.71 (m, 4H), 1.47-1.34 (m, 2H), 1.12-1.06 (m, 3H).

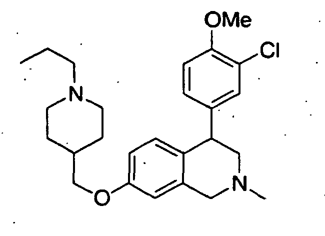

### Example 108; 4-(3-Chloro-4-methoxy-phenyl)-2-methyl-7-(1-propyl-piperidin-4-ylmethoxy)-1,2,3,4-tetrahydro-isoquinoline.

MS (ESI): mass calcd for C₂₆H₃₅ClN₂O₂, 442.24; m/z found, 443.5 [M+H]⁺. ¹H NMR (CDCl₃): 7.20 (d, J = 2.2, 1H), 7.03 (dd, J = 8.5, 2.2, 1H), 6.84 (d, J = 8.5, 1H), 6.76 (d, J = 8.5, 1H), 6.63 (dd, J = 8.5, 2.5, 1H), 6.60-6.57 (m, 1H), 4.14-4.08 (m, 1H), 3.87 (s, 3H), 3.76 (d, J = 6.3, 2H), 3.66 (d, J = 14.8, 1H), 3.58 (d, J = 14.8, 1H), 3.01-2.91 (m, 3H), 2.50 (dd, J = 11.2, 8.2, 1H), 2.40 (s, 3H), 2.46-2.34 (m, 2H), 2.00-1.89 (m, 2H), 1.87-1.72 (m, 3H), 1.64-1.47 (m, 5H), 1.46-1.33 (m, 2H), 0.90 (t, J = 7.4, 3H).

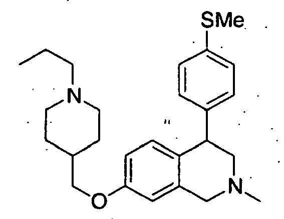

### Example 109; 2-Methyl-4-(4-methylsulfanyl-phenyl)-7-(1-propyl-piperidin-4-ylmethoxy)-1,2,3,4-tetrahydro-isoquinoline.

MS (ESI): mass calcd for C₂₆H₃₆N₂OS, 424.25; m/z found, 425.5 [M+H]⁺. ¹H NMR (CDCl₃): 7.22-7.15 (m, 2H), 7.14-1.07 (m, 2H), 6.76 (d, J = 8.6, 1H), 6.62 (d, J = 2.7 H, 1H), 6.60-6.57 (m, 1H), 4.16 (dd, J = 8.0, 5.9, 1H), 3.76 (d, J = 6.3, 2H), 3.68 (d, J = 14.9, 1H), 3.57 (d, J = 14.9, 1H), 3.01-2.92 (m, 3H), 2.56-2.44 (m, 4H), 2.41 (s, 3H), 2.34-2.24 (m, 2H), 2.01-1.89 (m, 2H), 1.85-1.76 (m, 4H), 1.60-1.48 (m, 2H), 1.47-1.30 (m, 2H), 0.90 (t, J = 7.4, 3H).

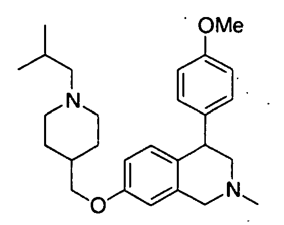

### Example 110; 7-(1-Isobutyl-piperidin-4-ylmethoxy)-4-(4-methoxy-phenyl)-2-methyl-1,2,3,4-tetrahydro-isoquinoline.

MS (ESI): mass calcd for C₂₇H₃₈N₂O₂, 422.29; m/z found, 423.5 [M+H]⁺. ¹H, NMR (CDCl₃): 7.15-6.97 (m, 2H), 6.95-6.83 (m, 2H), 6.83-6.66 (m, 2H), 6.67-6.51 (m, 1H), 4.91-4.45 (m, 1H), 4.41-3.98 (m, 1H), 3.98-3.62 (m, 8H), 3.62-3.34 (m, 1H), 3.23-2.91 (m, 4H), 2.91-2.80 (m, 2H), 2.78-2.58 (m, 2H), 2.29-1.75 (m, 6H), 1.12-0.97 (m, 6H).

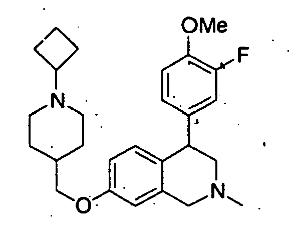

### Example 111; 7-(1-Cyclobutyl-piperidin-4-ylmethoxy)-4-(3-fluoro-4-methoxy-phenyl)-2-methyl-1,2,3,4-tetrahydro-isoquinoline.

MS (ESI): mass calcd for C₂₇H₃₅FN₂O₂, 438.27; m/z found, 439.5 [M+H]⁺: ¹H NMR (CDCl₃): 7.11-6.52 (m, 6H), 1.95-1.43 (m, 2H), 4.43-4.11 (m, 1H), 3.99-3.65 (m, 6H), 3.65-3.44 (m, 2H), 3.43-3.24 (m, 1H), 3.19-2.83 (m, 4H), 2.67-2.33 (m, 4H), 2.33-2.14 (m, 2H), 2.11-1.94 (m, 3H), 1.94-1.58 (m, 4H).

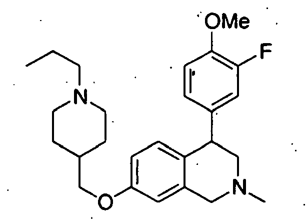

### Example 112; 4-(3-Fluoro-4-methoxy-phenyl)-2-methyl-7-(1-propyl-piperidin-4-ylmethoxy)-1,2,3,4-tetrahydro-isoquinoline.

MS (ESI): mass calcd for C₂₆H₃₅FN₂O₂, 426.27; m/z found, 427.5 [M+H]⁺. ¹H NMR (CDCl₃): 7.13-6.56 (m, 6H), 4.74-4.47 (m, 2H), 4.39-4.07 (m, 1H), 3.97-3.58 (m, 8H), 3.16-2.88 (m, 6H), 2.83-2.57 (m, 2H), 2.19-1.94 (m, 3H), 1.93-1.66 (m, 4H), 1.04-0.90 (m, 3H).

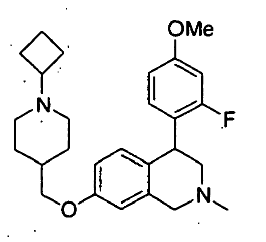

### Example 113; 7-(1-Cyclobutyl-piperidin-4-ylmethoxy)-4-(2-fluoro-4-methoxy-phenyl)-2-methyl-1,2,3,4-tetrahydro-isoquinoline.

MS (ESI): mass calcd for C₂₇H₃₅FN₂O₂, 438.27; m/z found, 439.5 [M+H]⁺. ¹H NMR (CDCl₃): 7.1,6-6.51 (m, 6H), 4.92-4.81 (m, 2H), 4.35-3.99 (m, 1H), 3.88-3.45 (m, 8H), 3.45-3.12 (m, 2H), 3.00 (s, 3H), 2.63-2.33 (m, 4H), 2.31-2.15 (m, 2H), 2.13-1.93 (m, 3H), 1.93-1.61 (m, 4H).

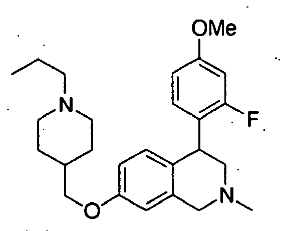

### Example 114; 4-(2-Fluoro-4-methoxy-phenyl)-2-methyl-7-(1-propyl-piperidin-4-ylmethoxy)-1,2,3,4-tetrahydro-isoquinoline.

MS (ESI): mass calcd for C₂₆H₃₆FN₂O₂, 426.27; m/z found, 427.5 [M+H]⁺. ¹H NMR (CDCl₃): 7.16-6.87 (m, 1H), 6.85-6.54 (m, 5H), 4.96-4.55 (m, 2H), 4.29-3.88 (m, 1H), 3.88-3.48 (m, 8H), 3.45-3.11 (m, 1H), 3.11-2.89 (m, 5H), 2.86-2.63 (m, 2H), 2.13-1.93 (m, 3H), 1.93-1.68 (m, 4H), 1.06-0.92 (m, 3H).

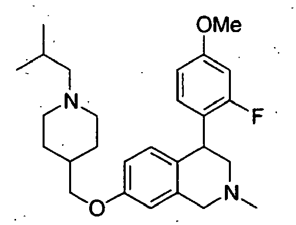

### Example 115; 4-(2-Fluoro-4-methoxy-phenyl)-7-(1-isobutyl-piperidin-4-ylmethoxy)-2-methyl-1,2,3,4-tetrahydro-isoquinoline.

MS (ESI): mass calcd for C₂₇H₃₇FN₂O₂, 440.28; m/z found, 441.5 [M+H]⁺. ¹H NMR (CDCl₃): 7.14-6.89 (m, 1H), 6.85-6.53 (m, 5H), 4.92-4.53 (m, 1H), 4.26-4.01 (m, 1H), 3.98-3.37 (m, 8H), 3.33-3.10 (m, 1H), 3.10-2.92 (m, 3H), 2.92-2.81 (m, 2H), 2.81-2.65 (m, 2H), 2.26-1.69 (m, 6H), 1.03 (d, J = 6.5, 6H).

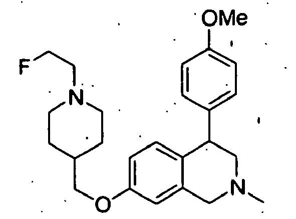

### Example 116; 7-[1-(2-Fluoro-ethyl)-piperidin-4-ylmethoxy]-4-(4-methoxy-phenyl)-2-methyl-1,2,3,4-tetrahydro-isoquinoline.

MS (ESI): mass calcd for C₂₅H₃₃FN₂O₂, 412.25; m/z found, 413.4 [M+H]⁺. ¹H NMR (CDCl₃): 7.16-6.97 (m, 2H), 6.94-6.67 (m, 4H), 6.67-6.55 (m, 1H), 4.97-4.87 (m, 1H), 4.87-4.75 (m, 1H), 4.74-4.50 (m, 1H), 4.23-4.01 (m, 1H), 7.07-3.90 (m, 7H), 3.59-3.23 (m, 3H), 3.13-2.93 (m, 4H), 2.92-2.75 (m, 2H), 2.31-1.70 (m, 5H).

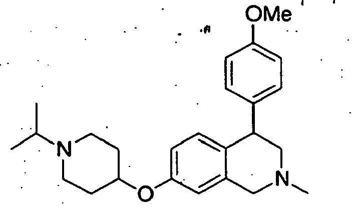

### Example 117; 7-(1-Isopropyl-piperidin-4-yloxy)-4-(4-methoxy-phenyl)-2-methyl-1,2,3,4-tetrahydro-isoquinoline.

Yield (0.16 mmol scale): 43 mg (69%). MS (ESI): mass calcd for C₂₅H₃₄N₂O₂, 394.3; m/z found, 395.5 [M+H]⁺. ¹H NMR (CDCl₃): 7.10 (d, J = 8.7, 2H), 6.82 (d, J = 8.7, 2H), 6.76 (d, J = 8.3, 1H), 6.63 (dd, J = 8.4, 2.6, 1H), 6.61 (d, J = 2.3, 1H), 4.26-4.20 (m, 1H), 4.15 (dd, J = 8.5, 5.7, 1H), 3.78 (s, 3H), 3.70 (d, J = 14.8, 1H), 3.55 (d, J = 14.8, 1H), 2.99-2.96 (m, 1H), 2.80-2.70 (m, 3H), 2.49 (dd, J = 11.4, 8.8, 1H), 2.40 (s, 3H), 2.40-2.32 (m, 2H), 2.04-1.95 (m, 2H), 1.83-1.74 (m, 2H), 1.05 (d, J = 6.6, 6H).

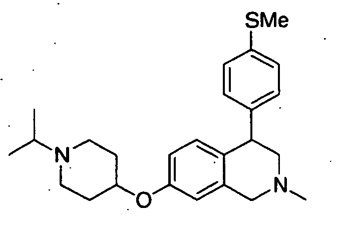

### Example 118; 7-(1-Isopropyl-piperidin-4-yloxy)-2-methyl-4-(4-methylsulfanyl-phenyl)-1,2,3,4-tetrahydro-isoquinoline.

Yield (0.43 mmol scale): 94 mg (53%). MS (ESI): mass calcd for C₂₅H₃₄N₂OS, 410.2; m/z found, 411.5 [M+H]⁺. ¹H NMR (CDCl₃): 7.17 (d, J = 8.3, 2H), 7.10 (d, J = 8.3, 2H), 6.74 (d, J = 8.4, 1H), 6.63 (dd, J = 8.4, 2.6, 1H), 6.60 (d, J =2.4, 1H), 4.26-4.20 (m, 1H), 4.14 (dd, J = 7.8, 6.0, 1H), 3.66 (d, J = 14.8, 1H), 3.56 (d, J = 14.8, 1H), 2.97-2.94 (m, 1H), 2.79-2.70 (m, 3H), 2.50 (dd, J = 11.4, 8.5, 1H), 2.44 (s, 3H), 2.39 (s, 3H), 2.39-2.34 (m, 2H), 2.00-1.92 (m, 2H), 1.83-1.75 (m, 2H), 1.04 (d, J = 6.6, 6H).

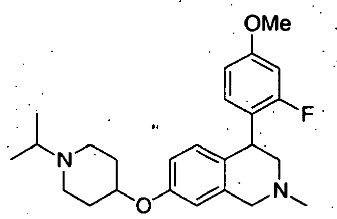

### Example 119; 4-(2-Fluoro-4-methoxy-phenyl)-7-(1-isopropyl-piperidin-4-yloxy)-2-methyl-1,2,3,4-tetrahydro-isoquinoline.

Yield (0.43 mmol scale): 87 mg (50%). MS (ESI): mass calcd for C₂₅H₃₃FN₂O₂, 412.3; m/z found, 413.5 [M+H]⁺. ¹H NMR (CDCl₃): 6.93 (dd, J = 8.7, 8.7, 1H), 6.78 (d, J = 8.5, 1H), 6.66 (dd, J = 8.5, 2.5, 1H), 6.62-6.59 (m, 2H), 6.57 (dd, J = 8.5, 2.7, 1H), 4.47 (dd, J = 6.3, 6.3, 1H), 4.26-4.22 (m, 1H), 3.76 (s, 3H), 3.60 (br s, 2H), 2.92 (m, 1H), 2.80-2.72 (m, 3H), 2.58 (dd, J = 11.3, 7.5, 1H), 2.42 (s, 3H), 2.42-2.36 (m, 2H), 2.04-1.92 (m, 2H), 1.85-1.75 (m, 2H), 1.06 (d, J = 6.6, 6H).

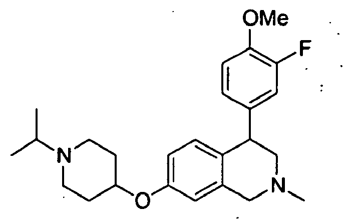

### Example 120; 4-(3-Fluoro-4-methoxy-phenyl)-7-(1-isopropyl-piperidin-4-yloxy)-2-methyl-1,2,3,4-tetrahydro-isoquinoline.

Yield (0.38 mmol scale): 129 mg (82%). MS (ESI): mass calcd for C₂₅H₃₃FN₂O₂, 412.3; m/z found, 413.5 [M+H]⁺. ¹H NMR (CDCl₃): 6.91-6.83 (m, 3H), 6.75 (d, J = 8.5, 1H), 6.63 (dd, J = 8.5, 2.5, 1H), 6.60 (d, J = 2.5, 1H), 4.25-4.20 (m, 1H), 4.09 (dd, J = 6.8, 6.8, 1H), 3.83 (s, 3H), 3.62 (d, J = 14.9, 1H). 3.56 (d, J = 14.9, 1H), 2.92 (dd, J = 11.4, 5.5, 1H), 2.78-2.69 (m, 3H), 2.50 (dd, J = 11.4, 8.0, 1H), 2.38 (s, 3H), 2.38-2.33 (m, 2H), 2.02-1.92 (m, 2H), 1.81-1.75 (m, 2H), 1.06 (d, J = 6.6, 6H).

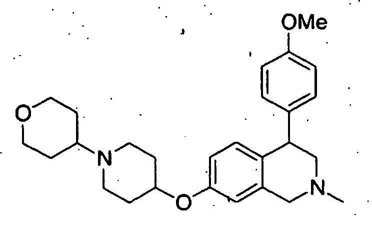

### Example 121; 4-(4-Methoxy-phenyl)-2-methyl-7-[1-(tetrahydro-pyran-4-yl)-piperidin-4-yloxy]-1,2,3,4-tetrahydro-isoquinoline.

Yield (0.15 mmol scale): 41 mg (64%). MS (ESI): mass calcd for C₂₇H₃₆N₂O₃, 436.3; m/z found, 437.5 [M+H]⁺. ¹H NMR (CDCl₃): 7.10 (d, J = 8.6, 2H), 6.82 (d, J = 8.7, 2H), 6.76 (d, J = 8.4, 1H), 6.63 (dd, J = 8.5, 2.5, 1H), 6.60 (d, J = 2.2, 1H), 4:27-4.23 (m, 1H), 4.17-4.12 (m, 1H), 4.02 (dd, J = 11.0, 4.0, 2H), 3.80 (s, 3H), 3.69 (d, J = 14.8, 1H), 3.55 (d, J = 14.8, 1H), 3.40-3.35 (m, 2H), 2.97 (dd J = 11.5, 5.5, 1H), 2.86-2.77 (m, 2H), 2.51-2.41 (m, 4H), 2.40 (s, 3H), 2.03-1.94 (m, 2H), 1.83-1.71 (m, 4H), 1.63 (dd J = 12.0, 4.3, 1H), 1.58 (dd J = 12.1, 4.2, 1H).

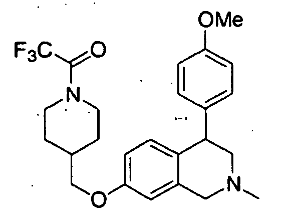

### Example 122; 2,2,2-Trifluoro-1-{4-[4-(4-methoxy-phenyl)-2-methyl-1,2,3,4-tetrahydro-isoquinolin-7-yloxymethyl]-piperidin-1-yl}-ethanone.

A solution of 4-(4-methoxy-phenyl)-2-methyl-7-(piperidin-4-ylmethoxy)-1,2,3,4-tetrahydro-isoquinoline (0.117 g, 0.319 mmol) in DCM (3 mL) was treated with trifluoroacetic anhydride (0.25 mL). The mixture was aged for 1 h and then was concentrated. FCC gave the title compound (0.119 g, 81%). MS (ESI): mass calcd for C₂₅H₂₉F₃N₂O₃, 462.21; m/z found, 463.5 [M+H]⁺. ¹H NMR (CDCl₃): 7.09 (d, J = 8.6, 2H), 6.83 (d, J = 8.8, 2H), 6.78 (d, J = 8.1, 1H), 6.61 (dd, J = 8.6, 2.8, 1H), 6.58 (d, J = 2.5, 1H), 4.63-4.56 (m, 1H), 4.18-4.04 (m, 2H), 3.83-3.75 (m, 2H), 3.79 (s, 3H), 3.70 (d, J = 14.4, 1H), 3.56 (d, J = 14.9, 1H), 3.17 (ddd, J = 13.7, 13.3, 2.5, 1H), 2.99 (ddd, J = 11.6, 5.5, 1.3, 1H), 2.81 (ddd, J = 12.8, 12.6, 2.0, 1H), 2.49 (dd, J = 11.4, 8.8 1H), 2.41 (s, 3H), 2.16-1.92 (m, 3H), 1.45-1.32. (m, 2H).

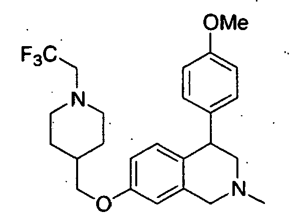

### Example 123; 4-(4-Methoxy-phenyl)-2-methyl-7-[1-(2,2,2.trifluoro-ethyl)-piperidin-4-ylmethoxy]-1,2,3,4-tetrahydroisoquinoline.

A solution of 2,2,2-trifluoro-1-{4-[4-(4-methoxy-phenyl)-2-methyl-1,2,3,4-tetrahydro-isoquinolin-7-yloxymethyl]-piperidin-1-yl}-ethanone (0.102 g, 0.221 mmol) in THF (5 mL) was treated with LiAlH₄ (1 M in THF, 1 mL). The mixture was stirred at 23 °C for 1 h then at 58 °C for 18 h. The mixture was cooled, diluted with satd. aq. NH₄Cl, diluted with 1 M NaOH and extracted with DCM. The organic layers were concentrated. FCC gave the desired product (0.0515 g, 52%). MS (ESI): mass calcd for C₂₅H₃₁F₃N₂O₂, 448.23; m/z found, 449.5 [M+H]⁺. ¹H NMR (CDCl₃): 7.10 (d, J = 8.8, 2H), 6.83 (d, J = 8.8, 2H), 6.77 (d, J = 8.6, 1H), 6.61 (dd, J = 8.6, 2.8, 1H), 6.58 (d, J = 2.5, 1H), 4.19-4.12 (m, 1H), 3.79 (s, 3H), 3.75 (d, 6.1, 2H), 3.70 (d, J = 14.7, 1H), 3.56 (d, J = 14.9, 1H), 3.04 (m, 5H), 2.49 (dd, 11.4, 8,8, 1H), 2.41 (s, 3H), 2.41-2.33 (m, 2H), 1.85-1.74 (m, 3H), 1.49-1.39 (m, 2H).

The following Examples 124-127 were prepared by a sequence similar to that used for 2-benzyl-4-(4-methoxy-phenyl)-7-(3-piperidin-1-yl-propoxy)-1,2,3,4-tetrahydroisoquinoline.

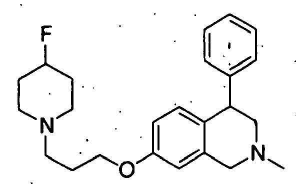

### Example 124; 7-[3-(4-Fluoro-pipehdin-1-yl)-propoxy]-2-methyl-4-phenyl-1,2,3,4-tetrahydro-isoquinoline.

Yield (0.672 mmol scale): 22 mg (5% over two steps) as a TFA salt. MS (ESI): mass calcd for C₂₄H₃₁FN₂O, 382.51; m/z found, 383.5 [M+H]⁺. ¹H NMR (acetone-d₆): 7.27-7.14 (m, 5H), 6.73-6.59 (m, 3H), 5.05-4.76 (m, 1H), 4.55-4.31 (m, 3H), 4.00-3.97 (m, 2H), 3.71-3.66 (m, 1H), 3.44-3.32 (m, 3H), 3.25-3.21 (m, 2H), 3.10-3.07 (m, 2H), 2.92 (s, 3H), 2.22-2.00 (m, 6H),

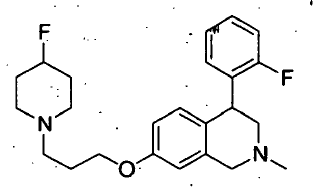

### Example 125; 4-(2-Fluoro-phenyl)-7-[3-(4-fluoro-piperidin-1-yl)-propoxy]-2-methyl-1,2,3,4-tetrahydro-isoquinoline.

Yield (1.9 mmol scale): 434 mg (36% over two steps) as a TFA salt. MS (ESI): mass calcd for C₂₄H₃₀F₂N₂O, 400.5; m/z found, 401.5 [M+H]⁺. ¹H NMR (acetone-d₆): 7.46-7.15 (m, 4H), 6.88-6.75 (m, 3H), 4.98-4.94 (m, 2H), 4.74-4.53 (m, 2H), 4.15-4.12 (m, 2H), 3.92-3.88 (m, 1H), 3.85-3.72 (m, 1H), 3.63-3.61 (m, 2H), 3.42-3.93 (m, 2H), 3.26-3.22 (m, 2H), 3.14 (s, 3H), 2.33-2.07 (m, 6H).

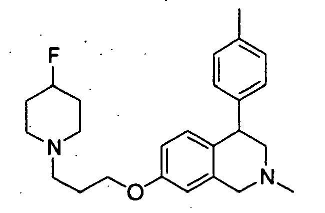

### Example 126: 7-[3-(4-Fluoro-piperidin-1-yl)-propoxy]-2-methyl-4-p-tolyl-1,2,3,4-tetrahydro-isoquinoline.

Yield (1.15 mmol scale): 88 mg (12% over two steps) as a TFA salt. MS (ESI): mass calcd for C₂₅H₃₃FN₂O, 396.54; m/z found, 397.5 [M+H]⁺. ¹H NMR (CDCl₃): 7.16-7.15 (m, 2H), 7.06-7.04 (m, 2H), 6.89-6.67 (m, 2H), 6:61 (s, 1H), 5.05-4.93 (m, 1H), 4.77-4.55 (m, 2H), 4.24-4.02 (m, 3H), 3.83-3.68 (m, 1H) 3.56-3.53 (m, 3H) 3.24-3.21 (m, 2H), 3.08-3.02 (m, 2H), 2.96 (s, 3H), 2.44-2.14 (m, 9H).

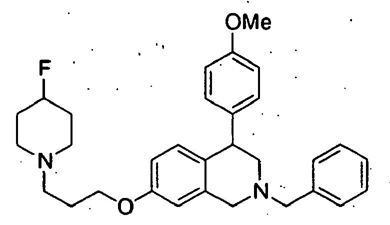

### Example 127; 2-Benzyl-7-[3-(4-fluoro-piperidin-1-yl)-propoxy]-4-(4-methoxy-phenyl)-1,2,3,4-tetrahydro-isoquinoline.

Yield (0.43 mmol scale): 50 mg (21% over two steps) as a TFA salt. MS (ESI): mass calcd for C₃₁H₃₇N₂O₂, 488.64; m/z found, 489.5 [M+H]⁺. ¹H NMR (CDCl₃): 7.44 (s, 5H), 7.04-7.03 (m, 2H), 6.86-6.84 (m, 2H), 6.77-6.69 (m, 2H), 6.56 (s, 1H), 5.03-4.93 (m, 1H), 4.52-4.36 (m, 4H), 4.14-3.83 (m, 3H), 3.79-3.64 (m, 4H), 3.55-3.53 (m, 2H), 3.23-3.21 (m, 2H), 3.07-3.03 (m, 2H), 2.92-2.85 (m, 1H), 3.38-2.00 (m, 6H).

The following Examples 128-145 were prepared by a sequence similar to that used in Example 1, unless otherwise noted.

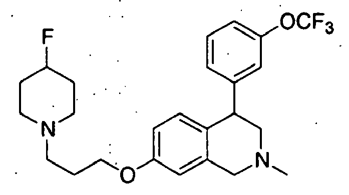

### Example 128; 7-[3-(4-Fluoro-piperidin-1-yl)-propoxy]-2-methyl-4-(3-trifluoromethoxy-phenyl)-1,2,3,4-tetrahydro-isoquinoline.

Yield (0.856 mmol scale): 0.184 g (46%). MS (ESI): mass calcd for C₂₅H₃₀F₄N₂O₂, 466.22; m/z found, 467.5 [M+H]⁺. ¹H NMR (CDCl₃): 7.33-7.27 (m, 1H), 7.14-7.04 (m, 3H), 6.75 (d, J = 8.6, 1H), 6.65 (dd, J = 8.4, 2.5, 1H), 6.61 (d, J = 2.5, 1H), 4.77-4.58 (m, 1H), 4.20 (dd, J = 7.4, 5.7, 1H), 3.98 (t, J = 6.5, 2H), 3.67 (d, J = 14.9, 1H), 3.60 (d, J = 15.3, 1H), 2.98 (m, 1H), 2.65-2.48 (m, 5H), 2.43-2.33 (m, 5H), 1.99-1.83 (m, 6H).

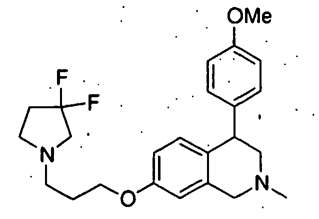

### Example 129; 7-[3-(3,3-Difluoro-pyrrolidin-1-yl)-propoxy]-4-(4-methoxy-phenyl)-2-methyl-1,2,3,4,tetrahydro-isoquinoline.

Yield: 135.0 mg (14%) as a TFA salt. MS (ESI): mass calcd for C₂₄H₃₀F₂N₂O₂; 416.23; m/z found, 417.5 [M+H]⁺. ¹H NMR (acetone-d₆): 7.16 (d, J = 8.7, 2H), 6.92 (d, J = 8.6, 2H), 6.83-6.73 (m, 3H), 4.66-4.49 (m, 3H), 4.10 (t, J = 5.9, 2H), 3.97 (t, J = 12.0, 2H), 3.81-3.75 (m, 3H), 3.78 (s, 3H), 3.57-3.43 (m, 3H), 3.08 (s, 3H), 2.73-2.65 (m, 2H), 2.30-2.25 (m, 2H).

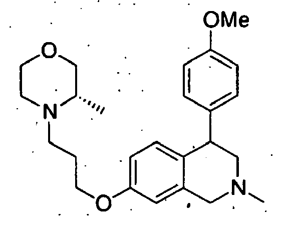

### Example 130; 4-(4-Methoxy-phenyl)-2-methyl-7-[3-(3S-methyl-morpholin-4-yl)-propoxy]-1,2,3,4-tetrahydro-isoquinoline.

Yield: 88.0 mg (9%) as a TFA salt. MS (ESI): mass calcd for C₂₅H₃₄N₂O₃, 410.26; m/z found, 411.5 [M+H]⁺. ¹H NMR (acetone-d₆): 7.16 (d, J = 8.7, 2H), 6.92 (d, J = 8.6, 2H), 6.84-6.73 (m, 3H), 4.65-4.50 (m, 3H), 4.13 (br s; 2H), 4.02-3.91 (m, 3H), 3.84-3.77 (m, 2H), 3.78 (s, 3H), 3.72-3.59 (m, 2H), 3.51-3.34 (m, 15H), 3.33-3.20 (m, 2H), 3.19-3.17 (m, 1H), 3.08 (s, 3H), 2.34-2.23 (m, 2H), 1.47-1.42 (m, 1H), 1.38-1.35 (m, 2H).

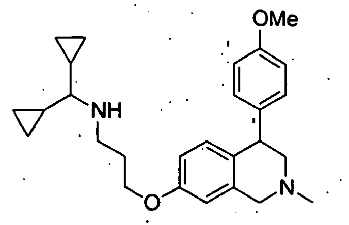

### Example 131; Dicyclopropylmethyl-{3-[4-(4-methoxy-phenyl)-2-methyl-1,2,3,4-tetrahydro-isoquinolin-7-yloxy]-propyl}-amine.

Yield: 87.0 mg (9%) as a TFA salt. MS (ESI): mass calcd for C₂₇H₃₆N₂O₂, 420.28; m/z found, 421.5 [M+H]⁺. ¹H NMR (acetone-d₆): 7.17 (d, J = 8.5, 2H), 6.92 (d, J = 8.5, 2H), 6.84-6.72 (m, 3H), 4.77-4.53 (m, 4H), 4.15-4.13 (m, 2H), 3.82-3.74 (m, 1H), 3.78 (s, 3H), 3.53-3.41 (m, 3H), 3.06 (s, 3H), 2.32-2.27 (m, 2H), 2.25-2.18 (m, 1H), 1.26-1.23 (m, 2H), 0.67-0.61 (m, 4H), 0.56-0.53 (m, 2H), 0.48-0.46 (m, 2H).

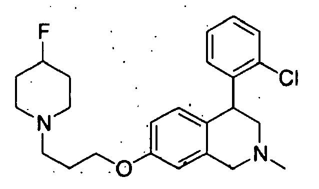

### Example 132; 4-(2-Chloro-phenyl)-7-[3-(4-fluoro-piperidin-1-yl)-propoxy]-2-methyl-1,2,3,4-tetrahydro-isoquinoline.

Prepared as for Example 1 to give 245 mg (32%) as a TFA salt. MS (ESI): mass calcd for C₂₄H₃₀CIFN₂O, 416.20; m/z.found, 417.4 [M+H]⁺. ¹H NMR (acetone-d₆): 7.50-7.48 (m, 1H), 7.36-7.29 (m, 2H), 7.19 (br s, 1H), 6.86 (br s, 1H), 6.83-6.80 (m, 1H), 6.72-6.69 (m, 1H), 5.17 (br s, 1H), 4.98 (d, J = 47.9, 1H), 4.65-4.55 (m, 2H), 4.11 (t, J = 5.8, 2H), 3.86-3.82 (m, 1H), 3.75-3.70 (m, 1H), 3.64-3.57 (m, 2H), 3.38-3.35 (m, 2H), 3.23-3.18 (m, 2H), 3.10 (s, 3H), 2.33-2.04 (m, 6H).

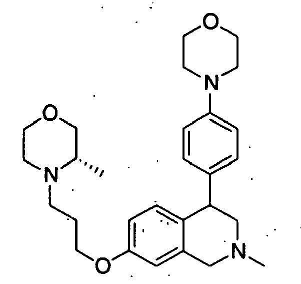

### Example 133; 2-Methyl-7-[3-(3S-methyl-morpholin-4-yl)-propoxy]-4-(4-morpholin-4-yl-phenyl)-1,2,3,4-tetrahydro-isoquinoline.

Yield: 31.9 mg (6%) as a TFA salt. MS (ESI): mass calcd for C₂₈H₃₉N₃O₃, 465.30; m/z found, 466.5 [M+H]⁺. ¹H NMR (acetone-d₆): 7.13 (s, J'= 8.7, 2H), 7.04 (d, J = 8.8, 2H), 6.83-6.68 (m, 3H), 4.62-4.69 (m, 1H), 4.59-4.55 (m, 2H), 4.16-4.09 (m, 2H), 4.03-4.91 (m, 3H), 4.87-4.84 (m, 2H), 3.77 (t, J = 4.7, 4H), 3.71-3.63 (m, 2H), 3.50-3.32 (m, 3H), 3.28-3.19 (m, 1H), 3.16 (t, J = 4.9, 4H), 3.13 (s, 3H), 2.38-2.26 (m, 2H), 1.50-1.46 (m, 1H), 1.39-1.37 (m, 2H).

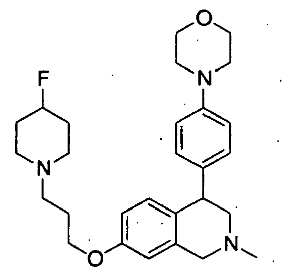

### Example 134; 7-[3-(4-Fluoro-piperidin-1-yl)-propoxy]-2-methyl-4-(4-morpholin-4-yl-phenyl)-1,2,3,4-tetrahydro-isoquinoline.

Yield: 155.6 mg (29%) as a TFA salt. MS (ESI): mass calcd for C₂₈H₃₈FN₃O₂, 467.29; m/z found, 468.5 [M+H]⁺. ¹H NMR (acetone-d₆): 7.13 (d, J = 8.6, 2H), 7.00 (d, J = 8.3, 2H), 6.85-6.72 (m, 3H), 5.02 (d, J = 47.7, 1H), 4.72-4.60 (m, 1H), 4.58-4.54 (m, 2H), 4.14-4.05 (m, 2H), 3.90-3.76 (m, 5H), 3.63-3.60 (m, 2H), 3.47-3.39 (m, 3H), 3.31-3.19 (m, 2H), 3.18-3.15 (m, 4H), 3.11 (s, 3H), 2.38-2.28 (m, 3H), 2.20-2.07 (m, 3H).

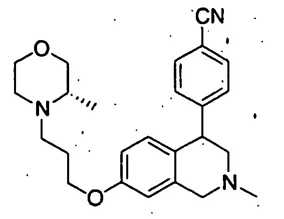

### Example 135; 4-{2-Methyl-7-[3-(3S-methyl-morpholin-4-yl)-propoxy]-1,2,3,4-tetrahydro-isoquinolin-4-yl}-benzonitrile.

Yield: 45.0 mg (7%) as a TFA salt. MS (ESI): mass calcd for C₂₅H₃₁N₃O₂, 405.24; m/z found, 406.5 [M+H]⁺. ¹H NMR (acetone-d₆): 7.80 (d, J = 8.2, 2H), 7.50 (d, J = 8.3, 2H), 3.89 (br s, 1H), 6.85-6.82 (m, 1H), 6.75-6.72 (m, 1H), 4.85-4.81 (m, 1H), 4.62 (br s, 2H), 4.20-4.09 (m, 2H), 4.02-3.89 (m, 4H), 3.72-3.56 (m, 4H), 3.46-3.27 (m, 3H), 3.09 (s, 3H), 2.36-2.24 (m, 2H), 1.46-1.35 (m, 3H).

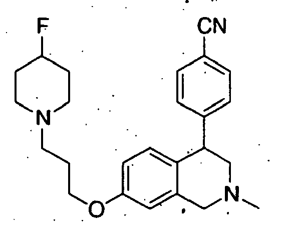

### Example 136; 4-{7-[3-(4-Fluoro-piperidin-1-yl)-propoxy]-2-methyl-1,2,3,4-tetrahydro-isoquinolin-4-yl}-benzonitrile.

Yield: 121.0 mg (19%) as a TFA salt. MS (ESI): mass calcd for C₂₅H₃₀FN₃O, 407.24; m/z found, 408.5 [M+H]⁺. ¹H NMR (acetone-d₆): 7.80 (d, J = 8.1, 2H), 7.50 (d, J = 8.2, 2H), 6.87 (s, 1H), 6.84-6.80 (m, 1H), 6.75-6.71 (m, 1H), 5.02 (d, J = 47.8, 1H), 4.84-4.80 (m, 1H), 4.63-4.52 (m, 2H), 4.16-4.11 (m, 2H), 3:92-3.88 (m, 1H), 3.78-3.71 (m, 1H), 3.61-3.55 (m, 2H), 3.40-3.36 (m, 2H), 3.24-3.19 (m, 2H), 3.07 (s, 3H), 2.36-2.02 (m, 6H).

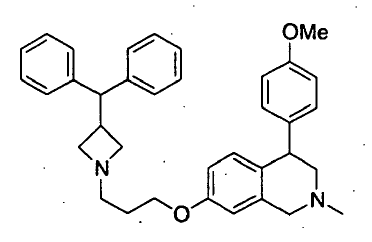

### Example 137; 7-[3-(3-Benzhydryl-azetidin-1-yl)-propoxy]-4-(4-methoxy-phehyl)-2-methyl-1,2,3,4-tetrahydro-isoquinoline.

The title compound may be prepared according the methods described in the preceding examples.

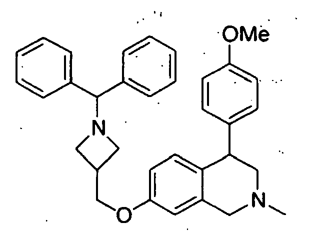

### Example 137A; 7-(1-Benzhydryl-azetidin-3-ylmethoxy)-4-(4-methoxy-phenyl)-2-methyl-1,2,3,4-tetrahydro-isoquinoline.

Yield: 6.1 mg (0.2%) as a TFA salt. MS (ESI): mass calcd for C₃₄H₃₆N₂O₂, 504.28; m/z found, 505.5 [M+H]⁺. ¹H NMR (acetone-d₆): 7.70 (d, J = 8.6, 4H), 7.45-7.32 (m, 6H), 7.17 (d, J = 8.5, 2H), 6.93 (d, J = 8.5, 4H), 6.83-6.76 (m, 1H), 5.77 (br s, 1H), 4.68-4.47 (m, 3H), 4.44-4.31 (m, 2H), 4.29-4.16 (m, 3H), 4.12-4.03 (m, 2H), 3.79 (s, 3H), 3.48-3.39 (m, 2H), 3.06 (s, 3H).

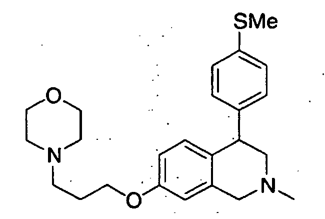

### Example 138; 2-Methyl-4-(4-methylsulfanyl-phenyl)-7-(3-morpholin-4-yl-propoxy)-1,2,3,4-tetrahydro-isoquinoline.

Yield (0.466 mmol scale): 0.110 g (57%). MS (ESI): mass calcd for C₂₄H₃₂N₂O₂S, 412.22; m/z found, 413.5 [M+H]⁺. ¹H NMR (CDCl₃): 7.18 (d, J = 8.3, 2H), 7.10 (d, J = 8.3, 2H), 6.76 (d, J = 8.6, 1H), 6.66-6.59 (m, 2H), 4.16 (dd, J = 8.6, 6.3, 1H), 4.00-3.95 (m, 2H), 3.76-3.65 (m, 5H), 3.58 (d, J = 15.2, 1H), 2.97 (ddd, J = 11.6, 5.6, 1.0, 1H), 2.55-2.45 (m, 10H), 2.41 (s, 3H), 1.98-1.91 (m, 2H).

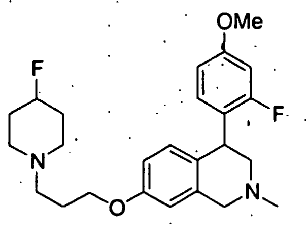

### Examples 139 (racemate), 140 (enantiomer 1) and 141 (enantiomer 2); 4-(2-Fluoro-4-methoxy-phenyl)-7-[3-(4-fluoro-piperidin-1-yl)-propoxy]-2-methyl-1,2,3,4-tetrahydro-isoquinoline.

Step 1. 3-[4-(2-Fluoro-4-methoxy-phenyl)-2-methyl-1,2,3,4-tetrahydro-isoquinolin-7-yloxy]-propan-1-ol. Yield (5.94 mmol scale): 0.98 g (48%). MS (ESI): mass calcd for C₂₀H₂₄FNO₃, 345.2; m/z found, 346.4 [M+H]⁺. ¹H NMR (CDCl₃): 6.92 (dd, J = 8.5, 8.5, 1H), 6.80 (d, J = 8.5, 1H), 6.67 (dd, J = 8.5, 2.7, 1H), 6.63-6.57 (m, 3H), 4,48 (dd, J = 6.5, 6.5, 1H), 4.09 (t, J = 5.9, 2H), 3.85 (t, J = 5.9, 2H), 3.77 (s, 3H), 3.62 (s, 2H), 2.93 (dd, J = 11.4, 5.5, 1H), 2.58 (dd, J = 11.4, 7.7, 1H), 2.40 (s, 3H), 2.02 (m, 2H), 1.75 (br s, 1H).

Step 2. Yield (1.52 mmol scale): 242 mg (37%) as a mixture of enantiomers. MS (ESI): mass calcd for C₂₅H₃₂F₂N₂O₂, 430.2; m/z found, 431.5 [M+H]⁺. ¹H NMR (CDCl₃): 6.92 (dd, J = 8.6, 8.6, 1H), 6.79 (d, J = 8.5, 1H), 6.63 (dd, J = 8.5, 2.6, 1H), 6.62-6.59 (m, 2H), 6.57 (dd, J = 8.5, 2.6, 1H), 4.74-4.60 (m, 1H), 4.47 (dd, J = 6.4, 6.4, 1H), 3.97 (t, J = 6.3, 2H), 3.76 (s, 3H), 3.61 (br s, 2H), 2.92 (dd, J = 11.4, 5.5, 1H), 2.65-2.60 (m, 3H), 2.52 (t, J = 7.2, 2H), 2.40 (s, 3H), 2.38-2.65 (m, 2H), 1.98-1.83 (m, 6H). The enantiomers were separated (SFC HPLC) to give Example 140 (first eluting) and Example 141 (second eluting).

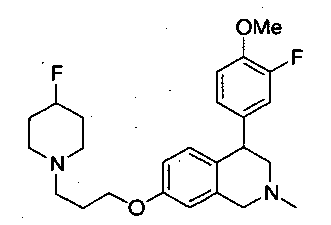

### Examples 142 (racemic), 143 (enantiomer 1) and 144 (enantiomer 2); 4-(3-Fluoro-4-methoxy-phenyl)-7-[3-(4-fluoro-piperidin-1-yl)-propoxy]-2-methyl-1,2,3,4-tetrahydro-isoquinoline.

Step 1. 3-[4-(3-Fluoro-4-methoxy-phenyl)-2-methyl-1,2,3,4-tetrahydro-isoquinolin-7-yloxy]-propan-1-ol. Yield (9.1.2 mmol scale): 1.55 g (49%). MS (ESI): mass calcd for C₂₀H₂₄FNO₃, 345.2; m/z found, 346.5 [M+H]⁺. ¹H NMR (CDCl₃): 6.91-6.85 (m, 3H), 6.78 (d, J = 8.5, 1H), 6.66 (dd, J = 8.5, 2.6, 1H), 6.61 (d, J = 2.6, 1H), 4.14-4.11 (m, 1H), 4.09 (t, J = 5.9, 2H), 3.87 (s, 3H), 3.85 (t, J = 5.9, 2H), 3.65 (d, J = 14.9, 1H), 3.58 (d, J = 14.9, 1H), 2.97-2.93 (m, 1H), 2.50 (dd, J = 11.4, 8.2, 1H), 2.40 (s, 3H), 2.02 (quint, J = 5.9, 2H), 1.66 (br s, 1H).

Step 2. Yield (1.40 mmol scale): 351 mg (58%) as a mixture of enantiomers. MS (ESI): mass calcd for C₂₅H₃₂F₂N₂O₂, 430.2; m/z found, 431.5 [M+H]⁺. ¹H NMR (CDCl₃): 6.94 (d, J = 8.8, 1H), 6.88 (d, J = 2.2, 1H), 6.86 (dd, J = 8.5, 8.5, 1H), 6.76 (d, J= 8.5, 1H), 6.64 (dd, J = 8.5, 2.5, 1H), 6.59 (d, J = 2.3, 1H), 4.76-4.59 (m, 1H), 4.11 (dd, J = 6.7, 6.7, 1H), 3.97 (t, J = 6.3, 2H) 3.84 (s, 3H), 3.64 (d, J = 14.9, 1H), 3.58 (d, J = 14.9, 1H), 2.94 (dd, J = 11.5, 5.5, 1H), 2.67-2.56 (m, 2H), 2.54 (t, J = 7.3, 2H), 2.51 (dd, J = 11.4, 8.1, 1H), 2.49-2.40 (m, 2H), 2.39 (s, 3H), 1.99-1.83 (m, 6H). The enantiomers were separated (SFC HPLC) to provide Example 143 (first eluting) and Example 144 (second eluting)

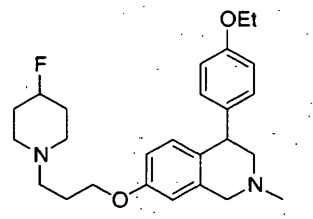

### Example 145; 4-(4-Ethoxy-phenyl)-7-[3-(4-fluoro-piperidin-1-yl)-propoxy]-2-methyl-1,2,3,4-tetrahydro-isoquinoline.

Step 1. 3-[4-(4-Ethoxy-phenyl)-2-methyl-1,2,3,4-tetrahydro-isoquinolin-7-yloxy]-propan-1-ol. Yield (4.76 mmol scale): 0.15 g (9%). MS (ESI): mass calcd for C₂₁H₂₇NO₃, 341.2; m/z found, 342.4 [M+H]⁺. ¹H NMR (CDCl₃): 7.07 (d, J = 8.6, 2H), 6.81 (d, J = 8.6, 2H), 6.77 (d, J = 8.5, 1H), 6.63 (dd, J = 8.5, 2.5, 1H), 6.60 (d, J = 2.5, 1H), 4.16 (dd, J = 8.9, 5.8, 1H), 4.04 (t, J = 6.0, 2H), 4.00 (q, J = 7.0, 2H), 3.80 (t, J = 5.9, 2H), 3.71 (d, J = 14.9, 2H), 3.54 (d, J = 14.8, 2H), 3.01-2.98 (m, 1H), 2.48 (dd, J = 11.4, 9.2, 1H), 2.41 (s, 3H), 2.04-1.96 (m, 3H), 1.40 (t, J = 7.0, 1H).

Step 2. Yield (0.28 mmol scale): 64.2 mg (53%). MS (ESI): mass calcd for C₂₆H₃₅FN₂O₂, 426.3; m/z found, 427.5 [M+H]⁺. ¹H NMR (CDCl₃): 7.07 (d, J = 8.7, 2H), 6.81 (d, J = 8.7, 2H), 6.77 (d, J = 8.5, 1H), 6.63 (dd, J = 8.5, 2.6, 1H), 6.59 (d, J = 2.5, 1H), 4.75-4.58 (m, 1H), 4.155 (dd, J = 8.6, 5.9, 1H), 4.01 (q, J = 7.0, 2H) 3.96 (t, J = 6.3, 2H), 3.70 (d, J = 14.81, 1H), 2.98 (m, 1H), 2.65-2.54 (m, 2H), 2.52-2.48 (m, 3H), 2.41 (s, 3H), 2.41-2.29 (m, 2H), 1.99-1.81 (m, 6H) 1.40 (t, J **=** 7.0, 3H).

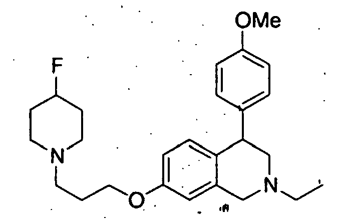

### Example 146; 2-Ethyl-7-[3-(4-fluoro-piperidin-1-yl)-propoxy]-4-(4-methoxy-phenyl)-1,2,3,4-tetrahydro-isoquinoline.

Prepared in a similar manner to 2-ethyl-4-(4-methoxy-phenyl)-7-(3-piperidin-1-yl-propoxy)-1,2,3,4-tetrahydro-isoquinoline to give the desired product (74 mg, 0.11 mmol) as a TFA salt (0.54 mmol scale; 27% over two steps). MS (ESI): mass calcd for C₂₆H₃₅FN₂O₂, 426.57; m/z found, 427.5 [M+H]⁺. ¹H NMR (CDCl₃): 7.09-7.03 (m, 2H), 6.88-6.86 (m, 2H), 6.79-6.63 (m, 3H), 5.02-4.93 (m, 1H), 4.80-4.56 (m, 2H), 4.24-4.02 (m, 3H), 3.80-3.76 (m, 4H), 3.65- 3.61 (m, 1H), 3.56-3.52 (m, 2H), 3.28-3.23 (m, 4H), 3.08-2.93 (m, 3H), 2.36-2.20 (m, 6H), 1.44-1.39 (m, 3H).

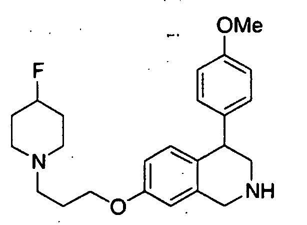

### Example 147: 7-[3-(4-Fluoro-piperidin-1-yl)-propoxy]-4-(4-methoxy-phenyl)-1,2,3,4-tetrahydro-isoquinoline.

Prepared from 2-benzyl-7-[3-(4-fluoro-piperidin-1-yl)-propoxy]-4-(4-methoxy-phenyl)-1,2,3,4-tetrahydro-isoquinoline as described in Example 66, Step 1, on a 0.046 mmol scale to give 10.2 mg (35%) of the desired product as a TFA salt. MS (ESI): mass calcd for C₂₄H₃₀FN₂O₂, 398.51; m/z found, 399.5 [M+H]⁺. ¹H NMR (CDCl₃): 7.05-7.03 (m, 2H), 6.88-6.83 (m, 3H), 6.74-6.73 (m, 1H), 6.67-6.66 (m, 1H), 5.13-4.93 (m, 1H), 4.44-4.29 (m, 3H), 4.08-4.06 (m, 2H), 3.80 (s, 3H), 3.72-3.65 (m, 1H), 3.60-3.58 (m, 2H), 3.28-3.27 (m, 3H), 3.15-3.05 (m, 2H), 2.51-2.20 (m, 7H).

The following Examples 148-151 were prepared by a sequence similar to that used in Example 78.

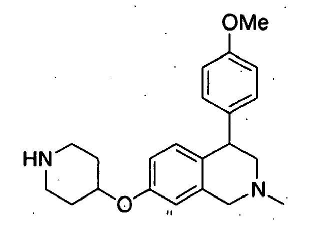

### Example 148; 4-(4-Methoxy-phenyl)-2-methyl-7-(piperidin-4-yloxy)-1,2,3,4-tetrahydro-isoquinoline.

Yield (4.06 mmol scale): 0.88 g (58%). MS (ESI): mass calcd for C₂₂H₂₈N₂O₂. 352.2; m/z found, 353.5 [M+H]⁺. ¹H NMR (CDCl₃): 7.10 (d, J = 8.6, 2H), 6.82 (d, J = 8.6, 2H), 6.76 (d, J = 8.4, 1H), 6.63 (dd, J = 8.4, 2.5, 1H), 6.61 (d, J = 2.5, 1H), 4.34-4.26 (m, 1H), 4.14 (dd, J = 8.3, 5.9, 1H), 3.78 (s, 3H), 3.69 (d, J = 14.8, 1H), 3.55 (d, J = 14.8, 1H), 3.14-3.08 (m, 2H), 2.97 (dd, J = 11.4, 5.6, 1H), 2.73-2.68 (ddd, J = 12.5, 9.4, 3.1, 2H), 2.49 (dd, J = 11.4, 8.8, 1H), 2.40 (s, 3H), 2.32 (br s, 1H), 2.00-1.87 (m, 4H), 1.67-1.58 (m, 2H).

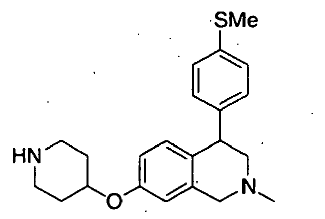

### Example 149; 2-Methyl-4-(4-methylsulfanyl-phenyl)-7-(piperidin-4-yloxy)-1,2,3,4-tetrahydro-isoquinoline.

Yield (1.38 mmol scale): 0.178 g (35%). MS (ESI): mass calcd for C₂₂H₂₈N₂OS, 368.2; m/z found, 369.4 [M+H]⁺. ¹H NMR (CDCl₃): 7.15 (d, J = 8.4, 2H), 7.08 (d, J = 8.5, 2H), 6.73 (d, J = 8.2, 1H), 6.62-6.59 (m, 2H), 4.35-4.27 (m, 1H), 4.13 (dd, J = 7.8, 6.0, 1H), 3.65 (d, J = 14.9, 1H), 3.54 (d, J = 14.9, 1H), 3.13-3.01 (m, 3H), 2.94 (dd, J = 11.4, 5.6, 1H), 2.72 (ddd, J = 12.3, 8.7, 3.2, 2H), 2.28 (dd, J = 11.4, 8.5, 1H), 2.43 (s, 3H), 2.38 (s, 3H), 2.00-1.96 (m, 2H), 1.69-1.61 (m, 2H).

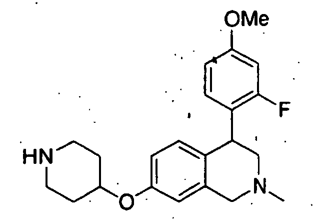

### Example 150; 4-(2-Fluoro-4-methoxy-phenyl)-2-methyl-7-(piperidin-4-yloxy)-1,2,3,4-tetrahydro-isoquinoline.

Yield (1.12 mmol scale): 0.170 g (41%). MS (ESI): mass calcd for C₂₂H₂₇FN₂O₂, 370.2; m/z found 371.4 [M+H]⁺. ¹H NMR (CDCl₃): 6.93 (dd, J = 8.7, 8.7, 1H), 6.78 (d, J = 8.5, ¹H), 6.65 (dd, J = 8.5, 2.6, 1H), 6.62-6.54 (m, 3H), 4.47 (dd, J = 6.3, 6.3, 1H), 4.26-4.22 (m, 1H), 3.76 (s, 3H), 3.60 (br s, 2H), 3.16-3.10 (m, 2H), 2.91 (dd, J = 11.3, 5.5, 1H), 2.73 (ddd, J = 12.4, 9.1, 3.1, 2H), 2.58 (dd, J = 11.3, 7.6, 1H), 2.41 (br s, 1H), 2.39 (s, 3H), 2.03-1.97 (m, 2H), 1.70-1.61 (m, 2H).

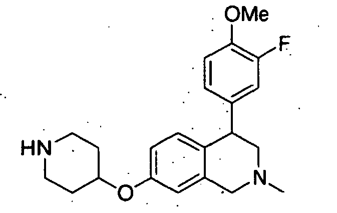

### Example 151; 4-(3-Fluoro-4-methoxy-phenyl)-2-methyl-7-(piperidin-4-yloxy)-1,2,3,4-tetrahydro-isoquinoline.

Yield (1.19 mmol scale): 0.157 g (40%). MS (ESI): mass calcd for C₂₂H₂₇FN₂O₂, 370.2; m/z found, 371.5 [M+H]⁺. ¹H NMR (CDCl₃): 6.90-6.82 (m, 3H), 6.74 (d, J = 8.5, 1H), 6.63 (dd, J = 8.5, 2.6, 1H), 6.59 (d, J = 2.4, 1H), 4.32-4.27 (m, 1H), 4.09 (dd, J = 6.9, 6.9, 1H), 3.83 (s, 3H), 3.62 (d, J = 14.9, 1H), 3.55 (d, J = 14.9, 1H), 3.13-3.08 (m, 2H), 2.92 (dd, J = 11.3, 5.6, 1H), 2.70 (ddd, J = 12.4, 9.2, 3.1, 2H), 2.49 (dd, J = 11.4, 8.1, 1H), 2.38 (s, 3H), 2.30 (br s, 1H), 2.00-1.95 (m, 2H), 1.67-1.58 (m, 2H).

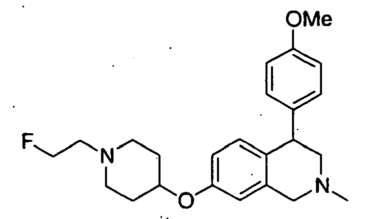

### Example 152; 7-[1-(2-Fluoro-ethyl)-piperidin-4-yloxy]-4-(4-methoxy-phenyl)-2-methyl-1,2,3,4-tetrahydro-isoquinoline.

To a mixture of 4-(4-methoxy-phenyl)-2-methyl-7-(piperidin-4-yloxy)-1,2,3,4-tetrahydro-isoquinoline (0.064 g, 0.18 mmol) and K₂CO₃ (0.038 g, 0.27 mmol) in DMF 1 mL was added 1-bromo-2-fluoroethane (14 µL, 0.19 mmol and the resulting mixture was heated at reflux overnight. The mixture was allowed to cool to rt, diluted with brine, and extracted with EtOAc (3x). The combined organic layers were dried (Na₂SO₄) and concentrated. The crude material was purified by FCC to give the desired product (36.3 mg, 50%). MS (ESI): mass calcd for C₂₄H₃₁FN₂O₂, 398.2; m/z found, 399.5 [M+H]⁺. ¹H NMR (CDCl₃): 7.10 (d, J = 8.7, 2H), 6.83 (d, J = 8.7, 2H), 6.76 (d, J = 8.4, 1H), 6.63 (dd, J = 8.5, 2.5, 1H), 6.60 (d, J = 2.2, 1H), 4.62 (t, J = 4.9, 1H), 4.52 (t, J = 4.9, 1H), 4.30-4.25 (m, 1H), 4.16-4.14 (m, 1H), 3.79 (s, 3H), 3.69 (d, J = 14.8, 1H), 3.55 (d, J = 14.8, 1H), 2.99-2.96 (m, 1H), 2.84-2.80 (m, 2H), 2.74 (t, J = 4.9, 1H), 2.69 (t, J = 4.9, 1H), 2.49 (dd, J = 11.4, 8.8, 1H), 2.46-2.39 (m, 2H), 2.41 (s, 3H), 2.01-1.96 (m, 2H), 1.88-1.80 (m, 2H).

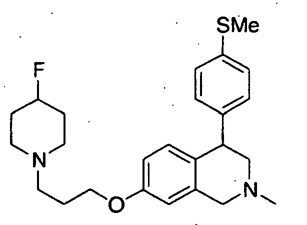

### Examples 153 (enantiomer 1) and 154 (enantiomer 2); 7-[3-(4-Fluoro-piperidin-1-yl)-propoxy]-2-methyl-4-(4-methylsulfanyl-phenyl)-1,2,3,4-tetrahydro-isoquinoline.

The enantiomers were separated (SFC HPLC) from racemic material (Example 51) to provide Example 153 (first eluting) and Example 154 (second eluting). Each compound was diluted with DCM and treated with 1.25 M HCl in MeOH then concentrated to give the HCl salts prior to analytical and biological testing.

Example 153: [α]₅₈₉ = -13.89 (*c* 9.36 mg/mL, MeOH). Example 154: [α]₅₈₉ = +12.51 (*c* 9.27 mg/mL, MeOH).

The compounds in Examples 155 through 158 were obtained as described in Examples 153 and 154.

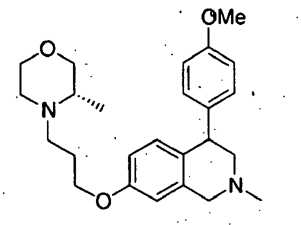

### Example 155 (enantiomer 1) and 156 (enantiomer 2); 4-(4-Methoxy-phenyl)-2-methyl-7-[3-(3S-methyl-morpholin-4-yl)-propoxy]-1,2,3,4-tetrahydro-isoquinoline.

The enantiomers were obtained from the racemic product of Example 130 by SFC HPLC to provide Example 155 (first eluting) and Example 156 (second eluting). Each compound was treated with 1.25 M HCl in MeOH and then concentrated to give the HCl salts prior to analytical and biological testing.

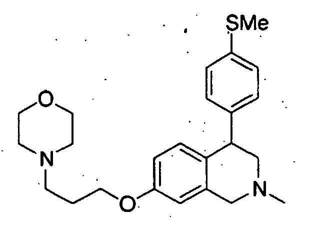

### Example 157 (enantiomer 1) and 158 (enantiomer 2); 2-Methyl-4-(4-methylsulfanyl-phenyl)-7-(3-morpholin-4-yl-propoxy)-1,2,3,4-tetrahydro-isoquinoline:

The enantiomers were obtained from the racemic product of Example 138 by SFC HPLC to provide Example 157 (first eluting) and Example 158 (second eluting). Each compound was diluted with DCM and treated with 1.25 M HCl in MeOH then concentrated to give the HCl salts prior to analytical and biological testing.

Example 157 (HCl salt): [α]₅₈₉ = +9.89 (*c* 10.8 mg/mL, MeOH); mp: 251-255 °C (dec).

The compounds in Examples 159-171 can be prepared according to procedures described in the preceding examples.

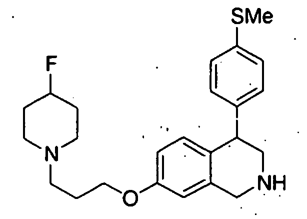

### Example 159; 7-[3-(4-Fluoro-piperidin-1-yl)-propoxy]-4-(4-methylsulfanyl-phenyl)-1,2,3,4-tetrahydro-isoquinoline.

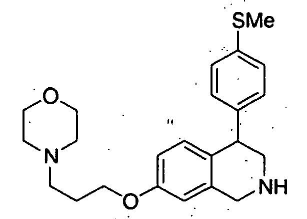

### Example 160; 4-(4-Methylsulfanyl-phenyl)-7-(3-morpholin-4-yl-propoxy)-1,2,3,4-tetrahydro-isoquinoline.

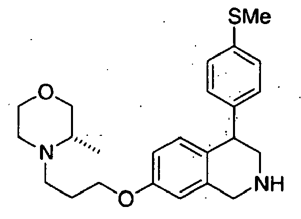

### Example 161; 7-[3-(3S-Methyl-morpholin-4-yl)-propoxy]-4-(4-methylsulfanyl-phenyl)-1,2,3,4-tetrahydro-isoquinoline.

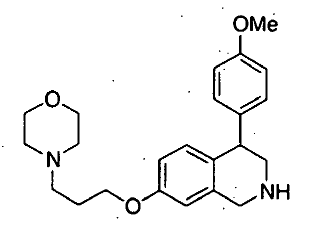

### Example 163; 4-(4-Methoxy-phenyl)-7-(3-morpholin-4-yl-propoxy)-1,2,3,4-tetrahydro-isoquinoline.

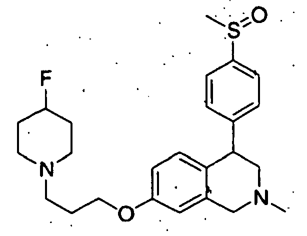

### Example 164; 7-[3-(4-Fluoro-piperidin-1-yl)-propoxy]-4-(4-methanesulfinylphenyl)-2-methyl-1,2,3,4-tetrahydro-isoquinoline.

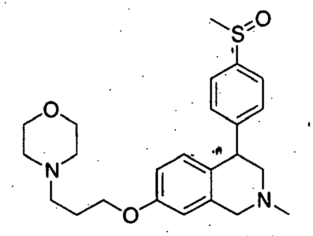

### Example 165; 4-(4-Methanesulfinylphenyl)-2-methyl-7-(3-morpholin-4-yl-propoxy)-1,2,3,4-tetrahydro-isoquinoline.

### Example 165A; 4-(4-Methanesulfinyl-phenyl)-2-methyl-7-(3-morpholin-4-yl-propoxy)-1,2,3,4-tetrahydro-isoquinoline (enantiomer 1).

To a solution of 2-methyl-4-(4-methylsulfanyl-phenyl)-7-(3-morpholin-4-yl-propoxy)-1,2,3,4-tetrahydro-isoquinoline (enantiomer 2) (Example 158; 33 mg, 0.068 mmol) and isopropyl 2-iodoxy-benzoate (IBX; 13.2 mg, 0.0408 mmol) in DMF (0.85 mL) was heated at 120 °C for 1 h and then allowed to stir at rt overnight. Additional IBX (10 mg, 0.031) was added and the mixture was heated at 120 °C for 2 h. After cooling to rt, the mixture was diluted with water, basified with satd. aq. NaHCO₃, and extracted with DCM (3x). The combined organic layers were dried (Na₂SO₄) and concentrated to give 12.5 mg (43%) of the desired product. MS (ESI): mass calcd for C₂₄H₃₂N₂O₃S, 428.21; m/z found, 429.4 [M+H]⁺. ¹H NMR (CDCl₃): 7.56 (d, J = 8.2, 2H), 7.34 (d, J = 7.9, 2H), 6.73 (d, J = 8.3, 1H), 6.67-6.60 (m, 2H), 3.99 (t, J = 6.2, 1H), 3.80-3.66 (m, 6H), 3.06-3.00 (m, 1H), 2.72 (s, 3H), 2.65-2.50 (m, 8H), 2.45 (s, 4H), 2.02-1.95 (m, 2H).

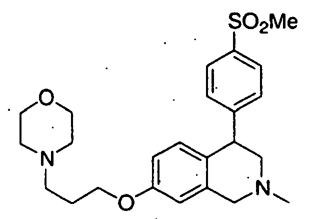

### Example 167; 4-(4-Methanesulfonyl-phenyl)-2-methyl-7-(3-morpholin-4-yl-propoxy)-1,2,3,4-tetrahydro-isoquinoline.

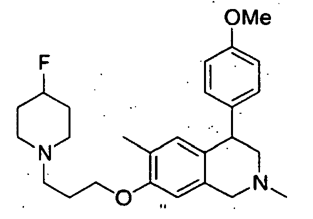

### Example 168; 7-[3-(4-Fluoro-piperidin-1-yl)-propoxy]-4-(4-methoxy-phenyl)-2,6-dimethyl-1,2,3,4-tetrahydro-isoquinoline.

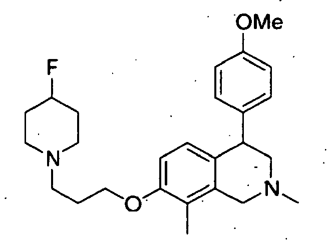

### Example 169; 7-[3-(4-Fluoro-piperidin-1-yl)-propoxy]-4-(4-methoxy-phenyl)-2,8-dimethyl-1,2,3,4-tetrahydro-isoquinoline.

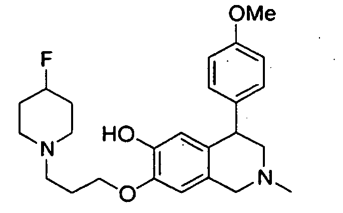

### Example 170; 7-[3-(4-Fluoro-piperidin-1-yl)-propoxy]-4-(4-methoxy-phenyl)-2-methyl-1,2,3,4-tetrahydro-isoquinolin-6-ol.

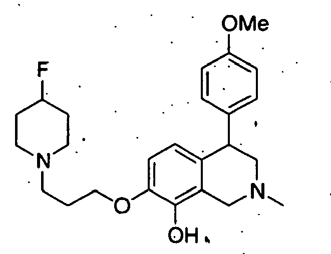

### Example 171; 7-[3-(4-Fluoro-piperidin-1-yl)-propoxy]-4-(4-methoxy-phenyl)-2-methyl-1,2,3,4-tetrahydro-isoquinolin-8-ol.

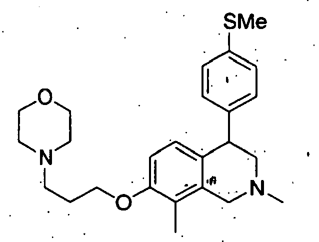

### Example 172; 2,8-Dimethyl-4-(4-methylsulfanyl-phenyl)-7-(3-morpholin-4-yl-propoxy)-1,2,3,4-tetrahydro-isoquinoline.

The title compound was prepared using methods similar to those described in Example 1. Yield: 27.6 mg (17% over 3 steps). MS (ESI): mass calcd for C₂₅H₃₄N₂O₂S, 426.23; m/z found, 427.3 [M+H]⁺. ¹H NMR (CDCl₃): 7.18 (d, J = 8.4, 2H), 7.10 (d, J = 8.3, 2H), 6.67-6.60 (m, 2H), 4.20-4.15 (m, 1H), 3.97 (t, J = 6.3, 2H), 3.74-3.65 (m, 5H), 3.47 (d, J = 15.3, 1H), 2.96-2.90 (m, 1H), 2.56-2.50 (m, 3H), 2.50-2.44 (m, 10H), 2.12 (s, 3H), 2.00-1.93 (m, 2H).

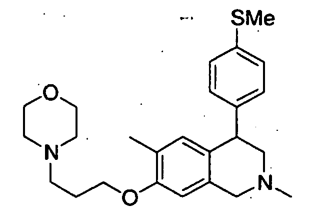

### Example 173; 2,6-Dimethyl-4-(4-methylsulfanyl-phenyl)-7-(3-morpholin-4-yl-propoxy)-1,2,3,4-tetrahydro-isoquinoline.

The title compound was prepared using methods similar to those described in Example 1. Yield: 35 mg (7%). MS (ESI): mass calcd for C₂₅H₃₄N₂O₂S, 426.23; m/z found, 427.5 [M+H]⁺. ¹H NMR (CDCl₃): 7.19 (d, J = 8.4, 2H), 7.11 (d, J = 8.3, 2H), 6.61 (s, 1H), 6.51 (s, 1H), 4.14-4.08 (m, 1H), 4.00 (t, J = 6.2, 2H), 3.75-3.70 (m, 4H), 3.68-3.54 (m, 2H), 2.79-2.91 (m, 1H), 2.56-2.45 (m, 8H), 2.40 (s, 3H), 2.05 (s, 3H), 2.02-1.94 (m, 2H), 1.56 (s, 2H).

The compounds in Examples 174-177, 180-181, 183, and 185 may be prepared according to procedures described in the preceding examples.

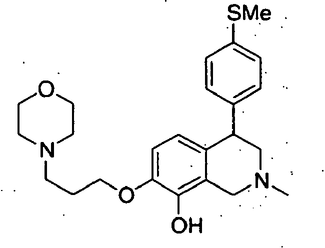

### Example 174; 2-Methyl-4-(4-methylsulfanyl-phenyl)-7-(3-morpholin-4-yl-propoxy)-1,2,3,4-tetrahydro-isoquinolin-8-ol.

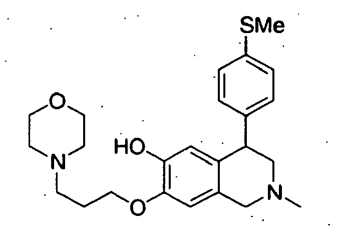

### Example 175; 2-Methyl-4-(4-methylsulfanyl-phenyl)-7-(3-morpholin-4-yl-propoxy)-1,2,3,4-tetrahydro-isoquinolin-6-ol.

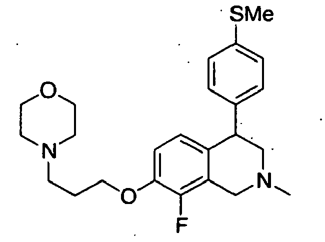

### Example 176; 8-Fluoro-2-methyl-4-(4-methylsulfanyl-phenyl)-7-(3-morpholin-4-yl-propoxy)-1,2,3,4-tetrahydro-isoquinoline.

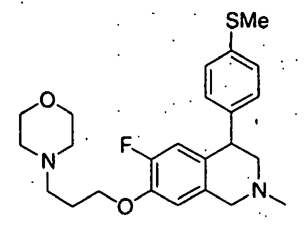

### Example 177; 6-Fluoro-2-methyl-4-(4-methylsulfanyl-phenyl)-7-(3-morpholin-4-yl-propoxy)-1,2,3,4-tetrahydro-isoquinoline.

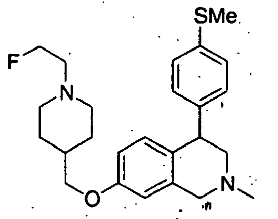

### Example 178; 7-[1-(2-Fluoro-ethyl)-piperidin-4-ylmethoxy]-2-methyl-4-(4-methylsulfanyl-phenyl)-1,2,3,4-tetrahydro-isoquinoline.

The title compound was prepared using methods similar to those described in Example 83. Yield: 11 mg (6%). MS (ESI): mass calcd for C₂₅H₃₃FN₂OS, 428.23; m/z found, 429.5 [M+H]⁺. ¹H NMR (CDCl₃): 7.23 (d, J = 8.3, 2H), 7.14-7.02 (m, 2H), 6.88-6.70 (m, 2H), 6.66-6.58 (m, 1H), 4.96-4.92 (m, 1H), 4.84-4.80 (m, 1H), 4.71-4.58 (m, 1H), 4.13-4.04 (m, 1H), 3.88-3.74 (m, 4H), 3.57-3.44 (m, 1H), 3.45-3.40 (m, 1H), 3.39-3.33 (m, 1H), 3.04-2.98 (m, 1H), 2.97 (s, 3H), 2.87-2.75 (m, 2H), 2.49 (s, 3H), 2.12-1.98 (m, 3H), 1.98-1.82 (m, 3H).

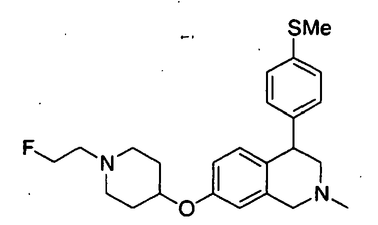

### Example 179; 7-[1-(2-Fluoro-ethyl)-piperidin-4-yloxy]-2-methyl-4-(4-methylsulfanyl-phenyl)-1,2,3,4-tetrahydro-isoquinoline.

The title compound was prepared using methods similar to those described in Example 152. Yield: 79 mg (40%). MS (ESI): mass calcd for C₂₄H₃₁FN₂OS, 414.21; m/z found, 415.5 [M+H]⁺. ¹H NMR (CDCl₃): 7.18 (d, J = 8.3,2H), 7.11 (d, J = 8.3, 2H), 6.75 (d, J = 8.5, 1H), 6.65-6.61 (m, 2H), 4.62 (t, J = 4.8, 1H), 4.54 (t, J = 4.8, 1H), 4.30-4.25 (m, 1H), 4.17-4.14 (m, 1H), 3.68 (d, J= 14.8, 1H), 3.57 (d, J = 14.8, 1H), 2.99-2.95 (m, 1H), 2.82-2.76 (m, 2H), 2.74 (t, J = 4.8, 1H), 2.70 (t, J = 4.8, 1H), 2.53-2.48 (m, 1H), 2.46 (s, 3H), 2.40 (s, 3H), 2.43-2.38 (m, 1H), 2.02-1.96 (m, 3H),1.87-1.80 (m, 2H).

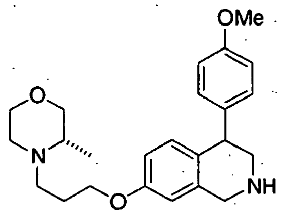

### Example 180; 4-(4-Methoxy-phenyl)-7-[3-(3S-methyl-morpholin-4-yl)-propoxy]-1,2,3,4-tetrahydro-isoquinoline.

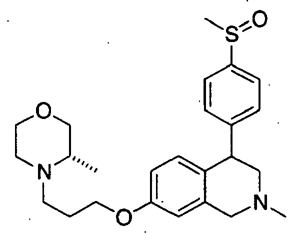

### Example 181; 4-(4-Methanesulfinyl-phenyl)-2-methyl-7-[3-(3S-methyl-morpholin-4-yl)-propoxy]-1,2,3,4-tetrahydro-isoquinoline.

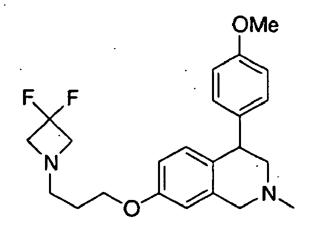

### Example 182; 7-[3-(3,3-Difluoro-azetidin-1-yl)-propoxy]-4-(4-methoxy-phenyl)-2-methyl-1,2,3,4-tetrahydro-isoquinoline.

The title compound was prepared using methods similar to those described in Example 1. Yield: 120 mg (13%) as a TFA salt. MS (ESI): mass calcd for C₂₃H₂₈F₂N₂O₂, 402.21; m/z found, 403.4 [M+H]⁺. ¹H NMR (acetone-d₆): 7.17 (d, J = 8.6, 2H), 6.92 (d, J = 8.7, 2H), 6.84-6.73 (m, 3H), 4.77 (t, J = 11.4, 4H), 4.58-4.44 (m, 3H), 4.12 (t, J = 6.0, 2H), 3.82-3.74 (m, 1H), 3.78 (s, 3H), 3.60 (t, J = 7.4, 2H), 3.50-3.42 (m, 1H), 3.06 (s, 3H), 2.19-2.1.4 (m, 2H).

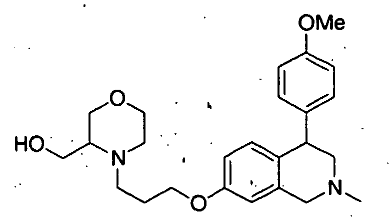

### Example 183; (4-{3-[4-(4-Methoxy-phenyl)-2-methyl-1,2,3,4-tetrahydro-isoquinolin-7-yloxy]-propyl}-morpholin-3-yl)-methanol.

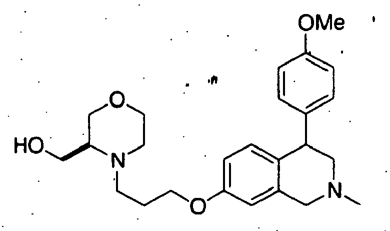

### Example 183A; (4-{3-[4-(4-Methoxy-phenyl)-2-methyl-1,2,3,4-tetrahydro-isoquinolin-7-yloxy]-propyl}-morpholin-35-yl)-methanol.

The title compound was prepared using (S)-morpholin-3-yl-methanol to give the product as a mixture of diastereomers. Yield: 103 mg (11 %) as a TFA salt. MS (ESI): mass calcd for C₂₅H₃₄N₂O₄, 426.25; m/z found, 427.4 [M+H]⁺. ¹H NMR (acetone-d₆): 7.17 (d, J = 8.6, 2H), 6.92 (d, J = 8.6, 2H), 6.85-6.73 (m, 3H), 4.64-4.45 (m, 3H), 4.18-4.06 (m, 2H), 4.05-4.01 (m, 4H), 3.94-3.89 (m, 3H), 3.82-3.74 (m, 2H), 3.78 (s, 3H), 3.67-3.59 (m, 1H), 3.52-3.44 (m, 3H), 3.39-3.30 (m, 1H), 3.06 (s, 3H), 2.37-2.30 (m, 2H).

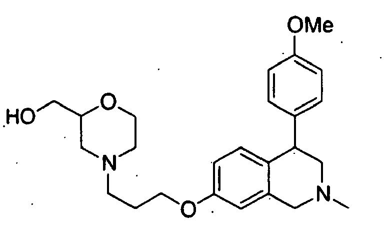

### Example 184; (4-{3-[4-(4-Methoxy-phenyl)-2-methyl-1,2,3,4-tetrahydro-isoquinolin-7-yloxy]-propyl}-morpholin-2-yl)-methanol.

Yield: 145 mg (16%) as a TFA salt. MS (ESI): mass calcd for C₂₅H₃₄N₂O₄, 426.25; m/z found, 427.4 [M+H]⁺. ¹H NMR (acetone-d₆): 7.16 (d, J = 8.6, 2H), 6.92 (d, J = 8.5, 2H), 6.80-6.75 (m, 3H), 4.66-4.48 (m, 3H), 4.13-4.05 (m, 3H), 3.99-3.92 (m, 2H), 3.82-3.74 (m, 1H), 3.78 (s, 3H), 3.66-3.56 (m, 4H), 3.49-3.38 (m, 3H), 3.11-3.03 (m, 1H), 3.07 (s, 1H), 2.98-2.94 (m, 1H), 2.34-2.29 (m, 2H).

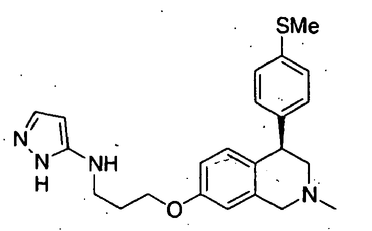

### Example 185; {3-[2-Methyl-4-(4-methylsulfanyl-phenyl)-1,2,3,4-tetrahydro-isoquinolin-7-yloxy]-propyl}-(2H-pyrazol-3-yl)-amine.

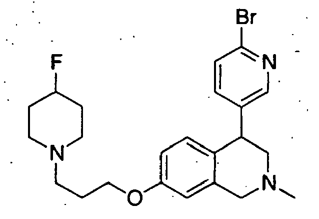

### Example 186; 4-(6-Bromo=pyridin-3-yl)-7-[3-(4-fluoro-piperidin-1-yl)-propoxy]-2-methyl-1,2,3,4-tetrahydro-isoquinoline.

Prepared by a sequence similar to that described in Example 1. Yield: 75.0 mg (20%) as a TFA salt. MS (ESI): mass calcd for C₂₃H₂₉BrFN₃O, 461.15; m/z found, 462.4, 464.4 [M+H]⁺. ¹H NMR (acetone-d₆): 8.35 (s, 1H), 7.59 (s, 1H), 6.9.1-6.80 (m, 3H), 5.03 (d, J = 47.7, 1H), 4.80-4.76 (m, 1H), 4.55 (bs, 2H), 4.13 (t, 5.6, 2H), 3.92-3.90 (m, 1H), 3.68-3.71 (m, 1H), 3.51-3.61 (m, 2H), 3.37-3.41 (m, 2H), 3.20-3.25 (m, 3H), 3.09 (s, 3H), 2.30-2.34 (m, 4H), 2.09-2.20 (m, 3H).

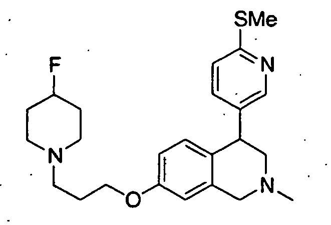

### Example 187; 7-[3-(4-Fluoro-piperidin-1-yl)-propoxy]-2-methyl-4-(6-methylsulfanyl-pyridin-3-yl)-1,2,3,4-tetrahydro-isoquinoline.

A solution of 4-(6-brormo-pyridin-3-yl)-7-[3-(4-fluoro-piperidin-1-yl)-propoxy]-2-methyl-1,2,3,4-tetrahydro-isoquinoline (250 mg, 0.54 mmol) and MeSNa (42.0 mg, 0.59 mmol) in DMF (2 mL) was heated at 80 °C for 3 h and then cooled to rt. The mixture was diluted with EtOAc and washed with water and brine. The organic layer was dried (MgSO₄) and concentrated. The crude product was purified by reverse phase chromatography and treated with MeOH-HCl to give 27 mg (9%) as an HCl salt. MS (ESI): mass calcd for C₂₄H₃₂FN₃OS, 429.23; m/z found, 430.5 [M+H]⁺. ¹H NMR (acetone-d₆): 8.39-8.30 (m, 1H), 7.48-7.44 (m, 1H), 7.26 (d, J = 8.3, 1H), 6.87-6.79 (m, 3H), 5.03 (d, J = 47.7, 1H), 4.73-4.50 (m, 3H), 4.14-4.12 (m, 2H), 3.95-3.90 (m, 1H), 3.76-3.74 (m, 1H), 3.50-3.63 (m, 3H), 3.44-3.41 (m, 2H), 3.28-3.16 (m, 2H), 3.11 (s, 3H), 2.53 (s, 3H), 2.33-2.21 (m, 4H), 2.20-2.13 (m, 3H).

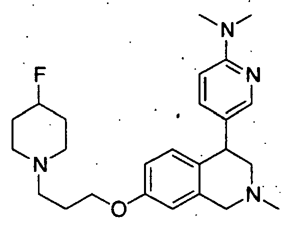

### Example 188; (5-{7-[3-(4-Fluoro-piperidin-1-yl)-propoxy]-2-methyl-1,2,3,4-tetrahydro-isoquinolin-4-yl}-pyridin-2-yl)-dimethyl-amine.

A solution of 4-(6-bromo-pyridin-3-yl)-7-[3-(4-fluoro-piperidin-1-yl)-propoxy]-2-methyl-1,2,3,4-tetrahydro-isoquinoline (250 mg, 0.54 mmol) and diethanolamine (0.13 mL, 1.35 mmol) in DMF (0.5 mL) was heated at 120°C for 4 days. The mixture was then diluted with water and extracted with EtOAc and DCM. The combined organic layers were dried (MgSO₄) and concentrated. The residue was diluted with MeOH (4 mL) and treated with NaCNBH₃ (2 scoops) and bromocresol green (spatula tip). After 5 min, MeOH-HCl was added until a color change was observed. Additional MeOH-HCl was added, as needed, to maintain color change. After 5 min, the mixture was diluted with water, made basic with 1 N NaOH, and extracted with EtOAc. The combined organic layers were washed with brine, dried over MgSO₄, and concentrated. Purification by reverse phase HPLC gave 6.0 mg (3%) of product. MS (ESI): mass calcd for C₂₅H₃₅FN₄O, 426.28; m/z found, 427.4 [M+H]⁺. ¹H NMR (acetone-d₆): 7.99 (d, J = 2.5, 1H), 7.26 (dd, J = 8.8, 2.4, 1H), 6.76-6.74 (m, 1H), 6.66-6.64 (m, 2H), 6.49 (d, J = 8.8, 1H), 4.63-4.52 (m, 1H), 3.99 (t, J = 6.4, 3H), 3.60-3.50 (m, 2H), 3.01 (s, 6H), 2.59-2.52 (m, 3H), 2.52-2.41 (m, 4H), 2.32 (s, 5H), 1.91-1.85 (m, 5H), 1.75-1.68 (m, 3H).

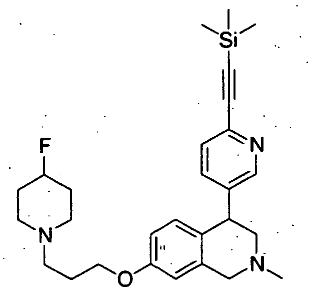

### Example 189; 7-[3-(4-Fluoro-piperidin-1-yl)-propoxy]-2-methyl-4-(6-trimethylsilanylethynyl-pyridin-3-yl)-1,2,3,4-tetrahydro-isoquinoline.

A mixture of 4-(6-bromo-pyridin-3-yl)-7-[3-(4-fluoro-piperidin-1-yl)-propoxy]-2-methyl-1,2,3,4-tetrahydro-isoquinoline (250 mg, 0.54 mmol), (Ph₃P)₂PdCl₂ (9.0 mg, 0.0135 mmol), and Cul (3 mg, 0.0135 mmol) in iPr₂NH (0.9 mL) was warmed to 30 °C and treated with (trimethylsilyl)acetylene (0.083 mL, 0.59 mmol). After 4 h, the mixture was cooled to rt, diluted with water, and extracted with DCM. The combined organic layers were washed with brine, dried (MgSO₄), and concentrated (180 mg, 69%). The crude material was carried forward without purification. MS (ESI): mass calcd for C₂₈H₃₈FN₃OSi, 479.28; m/z found, 480.5 [M+H]⁺. ¹H NMR (acetone-d₆): 8.51 (s, 1H), 7.65 (dd, J = 20.7, 2.3, 1H), 7.50 (d, J = 8.0, 1H), 6.87-6.82 (m, 2H), 6.76-6.74 (m, 1H), 5.14-4.98 (m, 1H), 4.81-4.77 (m, 1H), 4.63-4.59 (m, 3H), 4.13-4.11 (m, 2H), 3.91-3.87 (m, 1H), 3.80-3.71 (m, 1H), 3.59-3.52 (m, 3H), 3.38-3.35 (m, 2H), 3.21-3.17 (m, 2H), 3.06 (s, 3H), 2.32-2.25 (m, 4H), 2.25-2.19 (m, 2H), 0.24 (s, 9H).

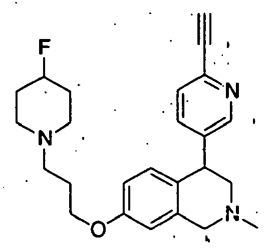

### Example 190; 4-(6-Ethynyl-pyridin-3-yl)-7-[3-(4-fluoro-piperidin-1-yl)-propoxy]-2-methyl-1,2,3,4-tetrahydro-isoquinoline.

To a solution of (5-{7-[3-(4-fluoro-piperidin-1-yl)-propoxy]-2-methyl-1,2,3,4-tetrahydro-isoquinolin-4-yl}-pyridin-2-yl)-dimethyl-amine (150 mg, 0.31 mmol) in 2:1 MeOH/DCM (1 mL) was added KOH (35 mg, 0.62 mmol). The mixture was stirred at rt for 2 h, diluted with water, and extracted with DCM. The combined organic layers were washed with brine, dried (MgSO₄), and concentrated. Purification by reverse phase chromatography gave 69 mg (55%) as a TFA salt. MS (ESI): mass calcd for C₂₅H₃₀FN₃O, 407.24; m/z found, 408.5 [M+H]⁺. ¹H NMR (acetone-d₆): 8.48 (s, 1H), 7.68 (dd, J = 8.0, 2.1, 1H), 7.57 (d, J = 9.7, 1H), 6.88-6.52 (m, 4H), 5.03 (d, J = 47.2, 1H), 4.82-4.79 (m, 1H), 4.68-4.58 (m, 2H), 4.14-4.12 (m, 2H), 4.08-4.03 (m, 1H), 3.79-3.72 (m, 1H), 3.61-3.54 (m, 2H), 3.43-3.32 (m, 2H), 3.24-3.18 (m, 3H), 3.07-3.02 (m, 4H), 2.38-2.25 (m, 5H), 2.22-2.10 (m, 3H).

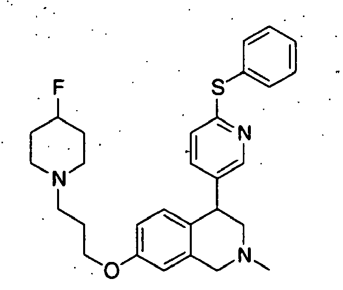

### Example 191; 7-[3-(4-Fluoro-piperidin-1-yl)-propoxy]-2-methyl-4-(6-phenylsulfanyl-pyridin-3-yl)-1,2,3,4-tetrahydro-isoquinoline.

Prepared as described for 7-[3-(4-fluoro-piperidin-1-yl)-propoxy]-2-methyl-4-(6-methylsulfanyl-pyridin-3-yl)-1,2,3,4-tetrahydro-isoquinoline to give 1.18 mg (30%) as a TFA salt. MS (ESI): mass calcd for C₂₉H₃₄FN₃OS, 491.24; m/z found, 492.5 [M+H]⁺. ¹H NMR (acetone-d₆): 8.34 (s, 1H), 7.61-7.58 (m, 2H), 7.48-7.43 (m, 4H), 6.91 (d, J = 8.3, 1H), 6.85-6.79 (m, 3H), 5.03 (d, J = 47.7, 1H), 4.71-4.57 (m, 3H), 4.12 (t, J = 5.6, 2H), 3.91-3.88 (m, 1H), 3.76-3.55 (m, 3H), 3.40 (t, J = 7.5, 2H), 3.29-3.17 (m, 2H), 3.09 (s, 3H), 2.34-2.25 (m, 3H), 2.23-2.11 (m, 3H).

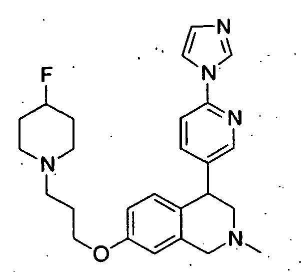

### Example 192; 7-[3-(4-Fluoro-piperidin-1-yl)-propoxy]-4-(6-imidazol-1-yl-pyridin-3-yl)-2-methyl-1,2,3,4-tetrahydro-isoquinoline.

A mixture of 4-(6-bromo-pyridin-3-yl)-7-[3-(4-fluoro-piperidin-1-yl-propoxy]-2-methyl-1,2,3,4-tetrahydro-isoquinoline (250 mg, 0.54 mmol), CuO (4.0 mg, 0.054 mmol), K₂CO₃ (112 mg, 0.81 mmol), and imidazole (110 mg, 1.62 mmol) in pyridine (0.5 mL) was heated at reflux in a sealed pressure tube for 18 h and then was cooled to rt. The mixture was filtered and concentrated, and the residue was purified by reverse phase chromatography to give 124 mg (29%) as a TFA salt. MS (ESI): mass calcd for C₂₆H₃₂FN₅O, 449.26; m/z found, 450.4 [M+H]⁺. ¹H NMR (acetone-d₆): 10.15 (br s, 1H), 9.54 (s, 1H), 8.51 (s, 1H), 8.33 (s, 1H), 8.01 (d, J = 8.5, 1H), 7.92 (d, J = 7.6, 1H), 7.70 (br s, 1H), 6.86-6.78 (m, 2H), 6.71 (br s, 1H), 5.03-4.85 (m, 1H), 4.75-4.67 (m, 2H), 4.53-4.39 (m, 2H), 4.05-4.00 (m, 2H), 3.61 (br s, 1H), 3.64-3.52 (m, 1H), 3.45-3.42 (m, 1H), 3.25-3.20 (m, 2H), 3.16-3.02 (m, 2H), 2.95 (s, 3H), 2.21-1.83 (m, 7H).

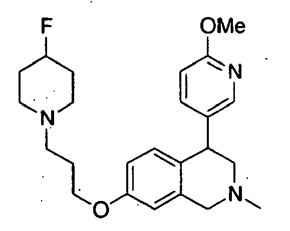

### Example 193; 7-[3-(4-Fluoro-piperidin-1-yl)-propoxy]-4-(6-methoxy-pyridin-3-yl)-2-methyl-1,2,3,4-tetrahydro-isoquinoline.

A mixture of 4-(6-bromo-pyridin-3-yl)-7-[3-(4-fluoro-piperidin-1-yl)-propoxy]-2-methyl-1,2,3,4-tetrahydro-isoquinoline (250 mg, 0.54 mmol) and NaOMe (204 mg, 3.78 mmol) in DMSO (0.7 ml) was heated at 140 °C for 3 days. Purification by reverse phase chromatography gave 18 mg (8%) of product. MS (ESI): mass calcd for C₂₄H₃₂FN₃O₂, 413.25; m/z found, 414.4 [M+H]⁺. ¹H NMR (acetone-d₆): 8.03 (d, J = 2.3, 1H), 7.46 (dd, J = 2.5, 8.5, 1H), 6.75 (d, J = 9.4, 1H), 6.67-6.62 (m, 3H), 4.66-4.54 (m, 1H), 4.09 (t, J = 5.5, 1H), 3.99 (t, J = 6.4, 2H), 3.83 (s, 3H), 3.64 (d, J = 14.9, 1H), 3.48 (d, J = 14.9, 1H), 2.82-2.79 (m, 2H), 2.59-2.56 (m, 3H), 2.45 (t, J = 7.0, 2H), 2.32 (s, 3H), 2.32-2.28 (m, 2H), 1.91-1.86 (m, 4H), 1.74-1.72 (m, 2H).

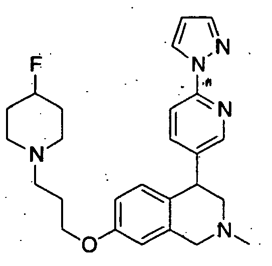

### Example 194; 7-[3-(4-Fluoro-piperidin-1-yl)-propoxy]-2-methyl-4-(6-pyrazol-1-yl-pyridin-3-yl)-1,2,3,4-tetrahydro-isoquinoline.

Prepared according to the procedure described for 7-[3-(4-fluoro-piperidin-1-yl)-propoxy]-4-(6-imidazol-1-yl-pyridin-3-yl)-2-methyl-1,2,3,4-tetrahydro-isoquinoline to give 120 mg (28%) as a TFA salt. MS (ESI): mass calcd for C₂₆H₃₂FN₅O, 449.26; m/z found, 450.4 [M+H]⁺. ¹H NMR (acetone-d₆): 8.59 (s, 1H), 8.41 (s, 1H), 7.94-7.90 (m, 1H), 7.81 (s, 1H), 7.75-7.73 (m, 1H), 6.86-6.80 (m, 2H), 6.75-6.71 (m, 1H), 6.58-6.55 (m, 1H), 4.98 (d, J = 47.2, 1H), 4.73-7.50 (m, 3H), 4.02-4.00 (m, 2H), 3.93-3.43 (m, 9H), 3.29-3.22 (m, 2H), 3.1-4-2.90 (m, 4H), 2.24-2.20 (m, 1H), 2.11-1.83 (m, 5H).

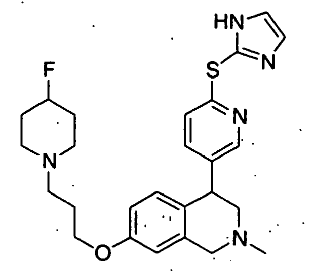

### Example 195; 7-[3-(4-Fluoro-piperidin-1-yl)-propoxy]-4-[6-(1H-imidazol-2-ylsulfanyl)-pyridin-3-yl]-2-methyl-1,2,3,4-tetrahydro-isoquinoline.

A mixture of 4-(6-bromo-pyridin-3-yl)-7-[3-(4-fluoro-piperidin-1-yl)-propoxy]-2-methyl-1,2,3,4-tetrahydro-isoquinoline (200 mg, 0.43 mmol), 2-mercaptoimidazole (130 mg, 1.30 mmol), and K₂CO₃ (179 mg, 1.30 mmol) in DMA (0.5 mL) was heated at 135 °C for 18 h. After cooling to rt, the mixture was diluted with DCM, filtered, and concentrated. Purification by reverse phase chromatography gave 35 mg (10%) as a TFA salt. MS (ESI): mass calcd for C₂₆H₃₂FN₅OS, 481.23; m/z found, 482.4 [M+H]⁺. ¹H NMR (acetone-d₆): 8.35-8.34 (m, 1H), 7.64 (s, 2H), 7.60 (dd, J = 2.3, 8.3, 1H), 7.31 (d, J = 8.3, 1H), 6.86-6.77 (m, 3H), 5.07-4.93 (m, 1H), 4.77-4.73 (m, 1H), 4.62-4.55 (m, 2H), 4.11 (t, J = 6.0, 2H), 3.90-3.86 (m, 1H), 3.61-3.55 (m, 3H), 3.39-3.29 (m, 2H), 3.23-3.18 (m, 2H), 3.06 (s, 3H), 2.34-2.13 (m, 7H).

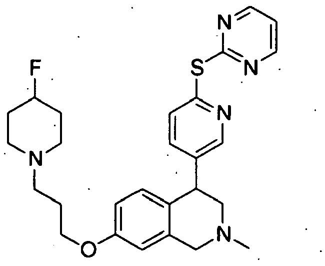

### Example 196; 7-[3-(4-Fluoro-piperidin-1-yl)-propoxy]-2-methyl-4-[6-(pyrimidin-2-ylsulfanyl)-pyridin-3-yl]-1,2,3,4-tetrahydro-isoquinoline.

Prepared as described for 7-[3-(4-fluoro-piperidin-1-yl)-propoxy]-4-[6-(1H-imidazol-2-ylsulfanyl)-pyridin-3-yl]-2-methyl-1,2,3,4-tetrahydro-isoquinoline (35. mg, 22%). MS (ESI): mass calcd for C₂₇H₃₂FN₅OS, 493.23; m/z found, 494.4 [M+H]⁺. ¹H NMR (acetone-d₆): 8.58-8.55 (m, 2H), 8.48 (d, J = 2.1, 1H), 7.75 (d, J = 8.1, 1H), 7.64 (d, J = 8.3, 2.4, 1H), 6.74-6.64 (m, 2H), 4.70-4.51 (m, 1H), 4.24-4.16 (m, 1H), 4.00 (t, J = 6.4, 2H), 3.75 (d, J = 15.0, 1H), 3.45 (d, J = 15.0, 1H), 2.87-2.76 (m, 2H); 2.78-2.66 (m, 1H), 2.63-2.50 (m, 2H), 2.46 (t, J = 7.0, 2H), 2.33 (s, 3H), 2.31-2.23 (m, 2H), 1.94-1.79 (m, 4H), 1.79-1.65 (m, 2H).

The compounds in Examples 197-236 were prepared using methods similar to those described for Example 1.

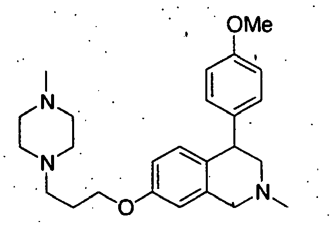

### Example 197; 4-(4-Methoxy-phenyl)-2-methyl-7-[3-(4-methyl-piperazin-1-yl)-propoxy]-1,2,3,4-tetrahydro-isoquinoline.

Yield: 31 mg (12%). MS (ESI): mass calcd for C₂₅H₃₅N₃O₂, 409.27; m/z found, 410.4 [M+H]⁺. ¹H NMR (acetone-d₆): 7.10 (d, J = 8.7, 2H), 6.81 (d, J = 8.7, 2H), 6.70 (d, J = 8.2, 1H), 6.66-6.62 (m, 2H), 4.10-4.06 (m, 1H), 3.98 (t, J = 6.4, 2H), 3.76 (s, 3H), 3.56 (s, 2H), 2.87-2.84 (m, 1H), 2.51-2.48 (m, 1H), 2.50-2.21 (m, 8H), 2.44 (t, J = 7.0, 2H), 2.32 (s, 3H), 2.16 (s, 3H), 1.91-1.87 (m, 2H).

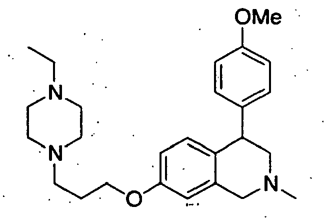

### Example 198; 7-[3-(4-Ethyl-piperazin-1-yl)-propoxy]-4-(4-methoxy-phenyl)-2-methyl-1,2,3,4-tetrahydro-isoquinoline.

Yield: 85 mg (28%) as an HCl salt. MS (ESI): mass calcd for C₂₆H₃₇N₃O₂, 423.29; m/z found, 424.4 [M+H]⁺. ¹H NMR (acetone-d₆): 7.11 (d, J = 8.7, 2H), 6.81 (d, J = 8.7, 2H), 6.71 (d, J = 8.2, 1H), 6.64-6.61 (m, 2H), 4.08-4.05 (m, 1H), 3.99 (t, J = 6.4, 1H), 3.74 (s, 3H), 2.85-2.82 (m, 1H), 2.50-2.27 (m, 16H), 1.90-1.83 (m, 2H), 0.99 (t, J = 7.2, 3H).

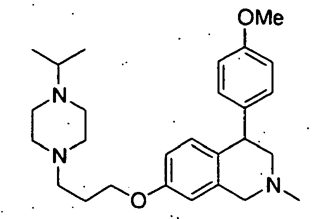

### Example 199; 7-[3-(4-Isopropyl-piperazin-1-yl)-propoxy]-4-(4-methoxy-phenyl)-2-methyl-1,2,3,4-tetrahydro-isoquinoline. Yield: 54 mg (17%) as an HCl salt. MS (ESI): mass calcd for C₂₇H₃₉N₃O₂, 437.30;

m/z found, 438.5 [M+H]⁺. ¹H NMR (acetone-d₆): 7.10 (d, J = 8.6, 2H), 6.81 (d, J = 8.7, 2H), 6.71 (d, J = 8.3, 1H), 6.64-6.61 (m, 2H), 4.08-4.05 (m, 1H), 3.99 (t, J = 6.4, 2H), 3.74 (s, 3H), 3.55 (s, 2H), 2.85-2.82 (m, 1H), 2.59-2.54 (m, 1H), 2.50-2.34 (m, 10H, 2.31 (s, 3H), 1.90-1.84 (m, 2H), 0.96 (d, J = 6.5, 6H).

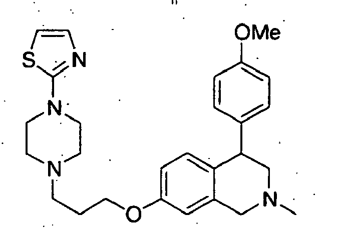

### Example 200; 4-(4-Methoxy-phenyl)-2-methyl-7-[3-(4-thiazol-2-yl-piperazin-1-yl)-propoxy]-1,2,3,4-tetrahydro-isoquinoline.

Yield: 44 mg (15%). MS (ESI): mass calcd for C₂₇H₃₄N₄O₂S, 478.24; m/z found, 479.4 [M+H]⁺. ¹H NMR (acetone-d₆): 7.13-7.10 (m, 3H), 6.81 (d, J = 8.7, 2H), 6.72-6.69 (m, 2H), 6.66-6.63 (m, 2H), 4.08-4.05 (m, 1H), 4.04 (t, J = 6.4, 2H), 3.74 (s, 3H), 3.55 (s, 2H), 3.44-3.42 (m, 4H), 2.86-2.82 (m, 1H), 2.80-2.76 (m, 1H), 2.56-2.47 (m, 6H), 2.31 (s, 3H), 1.95-1.93 (m, 2H).

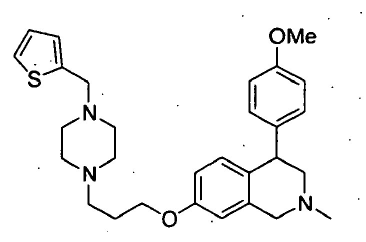

### Example 201; 4-(4-Methoxy-phenyl)-2-methyl-7-[3-(4-thiophen-2-ylmethyl-piperazin-1-yl)-propoxy]-1,2,3,4-tetrahydro-isoquinoline.

Yield: 56 mg (18%). MS (ESI): mass calcd for C₂₉H₃₇N₃O₂S, 491.26; m/z found, 479.4 [M+H]⁺. ¹H NMR (acetone-d₆): 7.32-7.30 (m, 1H), 7.10 (d, J = 8.7, 2H), 6.93-6.91 (m, 2H), 6.81 (d, J = 8.7, 2H), 6.70 (d, J = 8.2, 1H), 6.64-6.61 (m, 2H), 4.08-4.05 (m, 1H), 3.99 (t, J = 6.4, 1H), 3.74 (s, 3H), 3.66 (s, 2H), 3.55 (s, 2H), 2.85-2.81 (m, 1H), 2.50-2.40 (m, 10H), 2.31 (s, 3H), 1.90-1.85 (m, 2H).

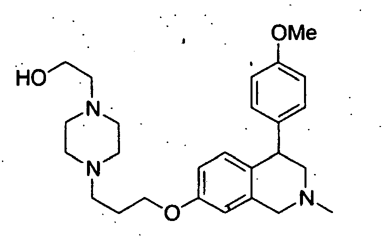

### Example 202; 2-(4-{3-[4-(4-Methoxy-phenyl)-2-methyl-1,2,3,4-tetrahydro-isoquinolin-7-yloxy]-propyl}-piperazin-1-yl)-ethanol.

Yield: 48 mg (18%). MS (ESI): mass calcd for C₂₆H₃₇N₃O₃, 439.28; m/z found, 440.4 [M+H]⁺. ¹H NMR (acetone-d₆): 7.11 (d, J = 8.7, 2H), 6.81 (d, J = 8.7, 2H), 6.71 (d, J = 8.2, 1H), 6.64-6.61 (m, 2H), 4.08-4.05 (m, 1H), 3.99 (t, J = 6.4, 2H), 3.74 (s, 3H), 3.55-3.51 (m, 4H), 3.29 (br s, 1H), 2.85-2.82 (m, 1H), 2.54-2.31 (m, 3H), 2.31 (s, 3H), 1.90-1.85 (m, 2H).

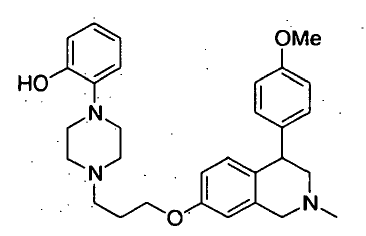

### Example 203; 2-(4-{3-[4-(4-Methoxy-phenyl)-2-methyl-1,2,3,4-tetrahydro-isoquinolin-7-yloxy]-propyl}-piperazin-1-yl)-phenol.

Yield: 94 mg (31 %). MS (ESI): mass calcd for C₃₀H₃₇N₃O₃, 487.28; m/z found, 488.4 [M+H]⁺. ¹H NMR (acetone-d₆): 7.13-7.08 (m, 3H), 6.96-6.93 (m, 1H), 6.85-6.77 (m, 4H), 6.73-6.70 (m, 1H), 6.67-6.64 (m, 2H), 4.09-4.06 (t, J = 6.4, 2H), 3.74 (s, 3H), 3.59 (s, 2H), 2.90 (t, J = 4.7, 4H), 2.86-2.83 (m, 1H), 2.63-2.57 (m, 4H), 2.53 (t, J = 7.0, 2H), 2.51-2.47 (m, 1H), 2.31 (s, 3H), 1.96-1.91 (m, 2H).

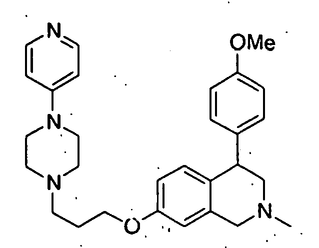

### Example 204; 4-(4-Methoxy-phenyl)-2-methyl-7-[3-(4-pyridin-4-yl-piperazin-1-yl)-propoxy]-1,2,3,4-tetrahydro-isoquinoline.

Yield: 44 mg (13%) as an HCl salt. MS (ESI): mass calcd for C₂₉H₃₆N₄O₂, 472.28; m/z found, 473.4 [M+H]⁺. ¹H NMR (acetone-d₆): 8.19-8.16 (m, 2H), 7.11 (d, J = 8.7, 2H), 6.81 (d, J = 8.6, 2H), 6.77-6.70 (m, 3H), 6.65-6.63 (m, 2H), 4.08-4.05 (m, 1H), 4.03 (t, J = 6.3, 2H), 3.74 (s, 3H), 3.55 (s, 2H), 3.55-3.27 (m, 4H), 2.85-2.81 (m, 1H), 2.55-2.44 (m, 7H), 2.31 (s, 3H), 1.96-1.911 (m, 2H).

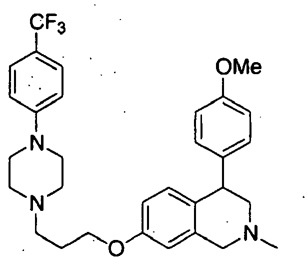

### Example 205; 4-(4-Methoxy-phenyl)-2-methyl-7-{3-[4-(4-trifluoromethyl-phenyl)-piperazin-1-yl]-propoxy}-1,2,3,4-tetrahydro-isoquinoline.

Yield: 89 mg (27%). MS (ESI): mass calcd for C₃₁H₃₆F₃N₃O_{2,} 539.28; m/z found, 540.4 [M+H]⁺. ¹H NMR (acetond-d₆): 7.49 (d, J = 8.9, 2H), 7.11 (d, J = 8.6, 2H), 7.05 (d, J = 8.9, 2H), 6.81 (d, J = 8.6, 2H), 6.71 (d, J = 8.2, 1H), 6.66-6.64 (m, 2H), 4.08-4.06 (m, 1H), 4.03 (t, J = 6.4, 2H), 3.74 (s, 3H), 3.55 (s, 2H), 3.31-3.29 (m, 4H), 2.85-2.81 (m, 1H), 2.58-2.55 (m, 4H), 2.53 (t, J = 7.0, 2H), 2.50-2.47 (m, 1H), 2.31 (s, 3H), 1.97-1.92 (m, 2H).

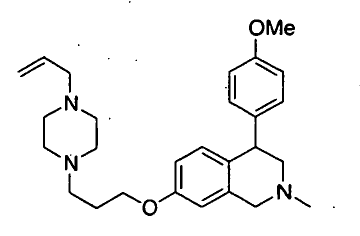

### Example 206; 7-[3-(4-Allyl-piperazin-1-yl)-propoxy]-4-(4-methoxy-phenyl)-2-methyl-1,2,3,4-tetrahydro-isoquinoline.

Yield: 97 mg (36%). ¹H NMR (acetone-d₆): 7.10 (d, J = 8.6, 2H), 6.81 (d, J = 8.6, 2H), 6.71 (d, J = 8.2, 1H), 6.64-6.61 (m, 2H), 5.83-5.75 (m, 1H), 5.169-5.12 (m, 1H), 5.07-5.04 (m, 1H), 4.08-4.05 (m, 1H), 3.98 (t, J = 6.4, 2H), 2.85-2.81 (m, 1H), 2.52-2.25 (m, 14H), 1.90-1.85 (m, 2H).

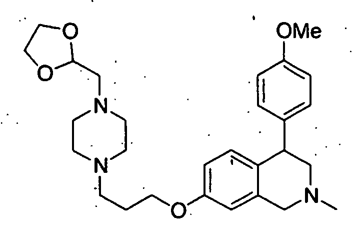

### Example 207; 7-[3-(4-[1,3]-Dioxolan-2-ylmethyl-piperazin-1-yl)-propoxy]-4-(4-methoxy-phenyl)-2-methyl-1,2,3,4-tetrahydro-isoquinoline.

Yield: 60 mg (20%). MS (ESI): mass calcd for C₂₈H₃₉N₃O₄, 481.29; m/z found, 482.4 [M+H]⁺. ¹H NMR (acetone-d₆): 7.11 (d, J = 8.6, 2H), 6.81 (d, J = 8.7, 2H), 6.71 (d, J = 8.2, 1H), 6.65-6.62 (m, 2H), 4.88 (t, J = 4.3, 1H), 4.08-4.05 (m, 1H), 3.98 (t, J = 6:4, 2H), 3.89-3.84 (m, 2H), 3.78-3.75 (m, 2H), 3.74 (s, 3H), 3.55 (s, 2H), 2.85-2.82 (m, 1H), 2.58-2.35 (m, 13H), 2.31 (s, 3H), 1.90-1.84 (m, 2H).

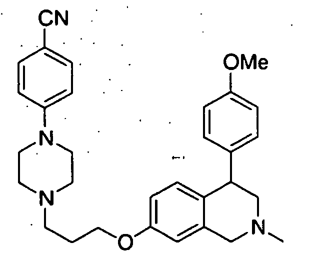

### Example 208; 4-(4-{3-[4-(4-Methoxy-phenyl)-2-methyl-1,2,3,4-tetrahydro-isoquinolin-7-yloxy]-propyl}-piperazin-1-yl)-benzonitrile.

Yield: 104 mg (34%) as a TFA salt. MS (ESI): mass calcd for C₃₁H₃₆N₄O₂, 496.28; m/z found, 497.4 [M+H]⁺. ¹H NMR (acetone-d₆): 7.51 (d, J = 9.1, 2H), 6.81 (d, J = 8.7, 2H), 6.71 (d, J = 8.2, 1H), 6.65-6.63 (m, 2H), 4.08-4.05 (m, 1H), 4.03 (t, J = 6.3, 2H), 3.74 (s, 3H), 3.55 (s, 2H), 3.36-3.34 (m, 4H), 2.85-2.81 (m, 1H), 2.57-2.47 (m, 7H), 2.31 (s, 3H), 1.97-1.91 (m, 2H).

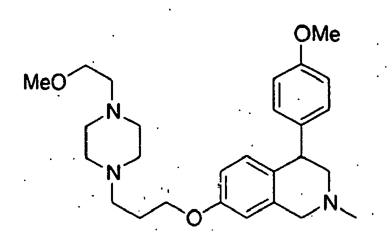

### Example 209; 7-{3-[4-(2-Methoxy-ethyl)-piperazin-1-yl]-propoxy}-4-(4-methoxy-phenyl)-2-methyl-1,2,3,4-tetrahydro-isoquinoline.

Yield: 71 mg (25%) as a TFA salt. MS (ESI): mass calcd for C₂₇H₃₉N₃O₃, 453.30; m/z found, 454.4 [M+H]⁺. ¹H NMR (acetone-d₆): 7.11 (d, J = 8.7, 2H), 6.81 (d, J = 8.7, 2H), 6.70 (d, J = 8.2, 1H), 6.65-6.62 (m, 2H), 4.08-4.05 (m, 1H), 3.98 (t, J = 6.4, 2H), 3.75 (s, 3H), 3.55 (s, 2H), 3.43 (t, J = 6.0, 2H), 3.24 (s, 3H), 2.85-2.81 (m, 2H), 2.50-2.31 (m, 12H), 2.27 (s, 3H), 1.90-1.84 (m, 2H).

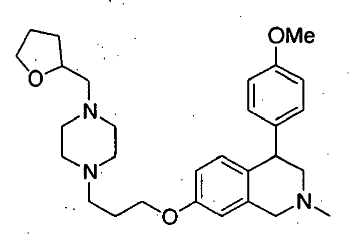

### Example 210; 4-(4-Methoxy-phenyl)-2-methyl-7-{3-[4-(tetrahydro-furan-2-ylmethyl)-piperazin-1-yl]-propoxy}-1,2,3,4-tetrahydro-isoquinoline.

Yield: 129 mg (43%) as a TFA salt_{.} MS (ESI): mass calcd for C₂₉H₄₁N₃O₃, 479.31; m/z found, 480.4 [M+H]⁺. ¹H NMR (acetone-d₆): 7.10 (d, J = 8.7, 2H), 6.81 (d, J = 8.7, 2H), 6.71 (d, J = 8.2, 1H), 6.65-6.62 (m, 2H), 4.08-4.05 (m, 1H), 3.98 (t, J = 6.4, 2H), 3.94-3.87 (m, 1H), 3.75-3.72 (m, 4H), 3.62-3.59 (m, 1H), 3.55 (s, 2H), 2.85-2.82 (m, 1H), 2.55-2.32 (m, 13H), 2.31 (s, 3H), 1.95-1.85 (m, 3H), 1.81-1.72 (m, 2H), 1.54-1.46 (m, 1H).

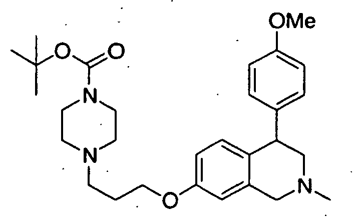

### Example 211; 4-{3-[4-(4-Methoxy-phenyl)-2-methyl-1,2,3,4-tetrahydro-isoquinokulin-7-yloxy]-propyl}-piperazine-1-carboxylic acid tert-butyl ester.

Yield: 80 mg (26%) as a TFA salt. MS (ESI): mass calcd for C₂₉H₄₁N₃O₄, 495.31; m/z found, 496.4 [M+H]⁺. ¹H NMR (acetone-d₆): 7.11 (d, J = 8.7, 2H), 6.81 (d, J = 8.7, 2H), 6.71 (d, J = 8.2, 1H), 6.64-6.62 (m, 2H), 4.08-4.05 (m, 1H), 4.00 (t, J = 6.4, 2H), 3.74 (s, 3H), 3.55 (s, 2H), 3.39-3.34 (m, 4H), 2.85-2.82 (m, 1H), 2.50-2.45 (m, 3H), 2.35-2.33 (m, 4H), 2.31 (s, 3H), 1.93-1.87 (m, 2H), 1.41 (s, 9H).

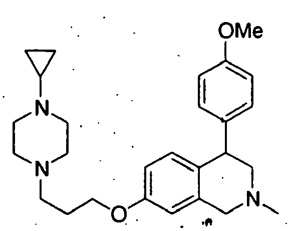

### Example 212; 7-[3-(4-Cyclopropyl-piperazin-1-yl)-propoxy]-4-(4-methoxy-phenyl)-2-methyl-1,2,3,4-tetrahydro-isoquinoline.

Yield: 15 mg (6%) as a TFA salt. MS (ESI): mass calcd for C₂₇H₃₇N₃O₂, 435.29; m/z found, 436.4 [M+H]⁺. ¹H NMR (acetone-d₆): 7.11 (d, J = 8.7, 2H), 6.81 (d, J = 8.7, 2H), 6.71 (d, J.= 8.2, 1H), 6.65-6.62 (m, 2H), 4.08-4.05 (m, 1H), 3.99 (t, J = 6.4, 2H), 3.74 (s, 3H), 3.55 (s, 2H), 2.85-2.82 (m, 2H), 2.58-2.30 (m, 13H), 1.89-1.84 (m, 2H), 1.56-1.53 (m, 1H), 0.37-0.35 (m, 2H), 0.27-0.25 (m, 2H).

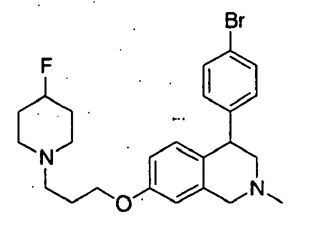

### Example 213; 4-(4-Bromo-phenyl)-7-[3-(4-fluoro-piperidin-1-yl)-propoxy]-2-methyl-1,2,3,4-tetrahydro-isoquinoline.

Yield: 2.56 g (31 %) as a TFA salt. MS (ESI): mass calcd for C₂₄H₃₀BrFN₂O, 460.15; m/z found, 461.4, 463.4 [M+H]⁺. ¹H NMR (acetone-d₆): 7.55 (d, J = 8.2, 2H), 7.23 (d, J = 8.4, 2H), 6.89-6.70 (m, 3H), 5.03 (d, J = 47.6, 1H), 4.72-4.54 (m, 3H), 4.16-4.08 (m, 2H), 3.93-3.86 (m, 1H), 3.84-3.47 (m, 3H), 3.42-3.38 (m, 2H), 3.30-3.18 (m, 2H), 3.07 (s, 3H), 2.37-2.28 (m, 3H), 2.25-2.13 (m, 3H).

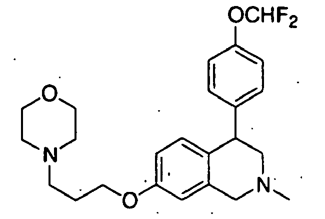

### Example 214; 4-(4-Difluoromethoxy-phenyl)-2-methyl-7-(3-morpholin-4-yl-propoxy)-1,2,3,4-tetrahydro-isoquinoline.

Yield: 1.63 g (95%). MS (ESI): mass calcd for C₂₄H₃₀F₂N₂O₃, 432.22; m/z found, 433.4 [M+H]⁺. ¹H NMR (acetone-d₆): 7.28 (d, J = 8.6, 2H), 7.09 (d, J = 8.6, 2H), 6.75 (d, J = 8.3, 1H), 6.70-6.66 (m, 2H), 4.17 (t, J = 5.8, 1H), 4.03 (t, J = 6.4, 2H), 3.66-3.57 (m, 4H), 3.55-3.51 (m, 2H), 2.88-2.82 (m, 1H), 2.75-2.72 (m, 2H), 2.61-2.56 (m, 1H), 2.48 (t, J = 7.0, 2H), 2.42-2.36 (m, 3H), 2.34 (s, 3H), 1.95-1.89 (m, 2H).

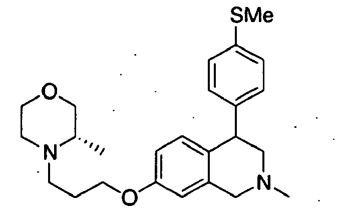

### Example 215; 2-Methyl-7-[3-(3S-methyl-morpholin-4-yl)-propoxy]-4-(4-methylsulfanyl-phenyl)-1,2,3,4-tetrahydro-isoquinoline.

Yield: 30.1 mg (4%). MS (ESI): mass calcd for C₂₅H₃₄N₂O₂S, 426.23; m/z found, 427.5 [M+H]⁺. ¹H NMR (CDCl₃): 7.18 (d, J = 8.3, 2H), 7.10 (d, J = 8.3, 2H), 6.76 (d, J = 8.5, 1H), 6.65-6.59 (m, 2H), 4.19-4.13 (m, 1H), 4.00-3.95 (m, 2H), 3.83-3.78 (m, 1H), 3.72-3.55 (m, 4H); 3.27-3.21 (m, 1H), 3.01-2.88 (m, 2H), 2.77 (dt, J = 2.6, 11.8, 1H), 2.54-2.30 (m, 9H), 1.98-1.75 (m, 3H), 0.97 (d, J = 6.3, 3H).

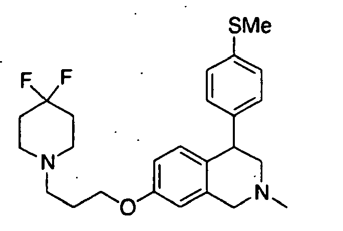

### Example 216; 7-[3-(4,4-Difluoro-piperidin-1-yl)-propoxy]-2-methyl-4-(4-methylsulfanyl-phenyl)-1,2,3,4-tetrahydro-isoquinoline.

Yield: 330.7 mg (37%). MS (ESI): mass calcd for C₂₅H₃₂F₂N₂OS, 446.22; m/z found, 447.5 [M+H]⁺. ¹H NMR (CDCl₃): 7.19 (d, J = 8.4, 2H), 7.10 (d, J = 8.3, 2H), 6.76 (d, J = 8.5, 1H), 6.65-6.59 (m, 2H), 4.19-4.13 (m, 1H), 3.98 (t, J = 6.3, 2H), 3.69 (d, J = 14.9, 1H), 3.58 (d, J = 14.9, 1H), 3.01-2.94 (m, 1H), 2.60-2.48 (m, 7H), 2.47 (s, 3H), 2.41 (s, 3H), 2.05-1.90 (m, 6H).

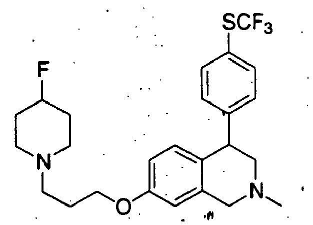

### Example 217; 7-[3-(4-Fluoro-piperidin-1-yl)-propoxy]-2-methyl-4-(4-trifluoromethylsulfanyl-phenyl)-1,2,3,4-tetrahydro-isoquinoline.

Yield: 11.5 mg (19%). MS (ESI): mass calcd for C₂₅H₃₀F₄N₂OS, 482.20; m/z found, 483.4 [M+H]⁺. ¹H NMR (CDCl₃): 7.53 (d, J = 8.2, 2H), 7.23 (d, J = 8.2, 2H), 6.73 (d, J. 8.5, 1H), 6.66-6.60 (m, 2H), 4.73-4.58 (m, 1H), 4.21 (t, J = 6.5, 1H), 3.97 (t, J = 6.3, 2H), 3.62 (s, 2H), 2.97-2.92 (m, 1H), 2.63-2.52 (m, 3H), 2.50 (t, J = 7.2, 2H), 2.38 (s, 3H), 2.40-2.34 (m, 2H), 1.97-1.82 (m, 6H).

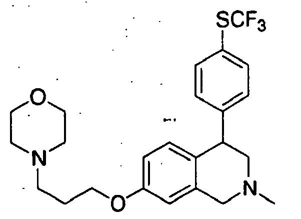

### Example 218; 2-Methyl-7-(3-morpholin-4-yl-propoxy)-4-(4-trifluoromethylsulfanyl-phenyl)-1,2,3,4-tetrahydro-isoquinoline.

Yield: 341 mg (58%). MS (ESI): mass calcd for C₂₄H₂₉F₃N₂O₂S, 466.19; m/z found, 467.4 [M+H]⁺. ¹H NMR (CDCl₃): 7.51 (d, J = 8.1, 2H), 7.21 (d, J = 8.2, 2H), 6.71 (d, J = 8.5, 1H), 6.65-6.59 (m, 2H), 6.40 (t, J = 6.4, 1H), 3.96 (t, J = 6.3, 2H), 3.68 (t, J = 4.6, 4H), 3.60 (s, 2H), 2.95-2.90 (m, 1H), 2.57-2.51 (m, 1H), 2.48 (t, J = 7.1, 2H), 2.45-2.40 (m, 4H), 2.37 (s, 3H), 1.95-1.89 (m, 2H).

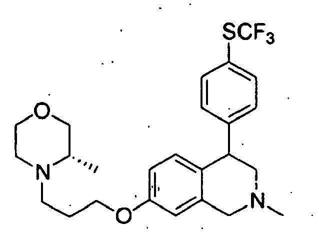

### Example 219; 2-Methyl-7-[3-(3S-methyl-morpholin-4-yl)-propoxy]-4-(4-trifluoromethylsulfanyl-phenyl)-1,2,3,4-tetrahydro-isoquinoline.

Yield: 154 mg (25%). MS (ESI): mass calcd for C₂₅H₃₁F₃N₂O₂S, 480.21; m/z found, 481.5 [M+H]⁺. ¹H NMR (CDCl₃): 7.54 (d, J = 8.1, 2H), 7.23 (d, J = 8.2, 2H), 6.73 (d, J = 8.5, 1H), 6.66-6.60 (m, 2H), 4.21 (t, J = 6.5, 1H), 3.98-3.94 (m, 2H), 3.81-3.76 (m, 1H), 3.67-3.60 (m, 4H), 3.25-3.20 (m, 1H), 2.97-2.88 (m, 2H), 2.77-2.73 (m, 1H), 2.58-2.53 (m, 1H), 2.45-2.30 (m, 6H), 1.96-1.85 (m, 2H), 0.96 (d, J = 6.3, 3H).

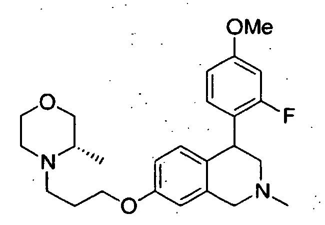

### Example 220; 4-(2-Fluoro-4-methoxy-phenyl)-2-methyl-7-[3-(3S-methyl-morpholin-4-yl)-propoxy]-1,2,3,4-tetrahydro-isoquinoline.

Yield: 10.2 mg (2%). MS (ESI): mass calcd for C₂₅H₃₃FN₂O₃, 428.25; m/z found, 429.5 [M+H]⁺. ¹H NMR (CDCl₃): 7.10 (d, J = 8.3, 1H), 7.02 (dd, J = 12.8, 2.2, 1H), 6.97 (d, J = 2.6, 1H), 6.95-6.92 (m, 1H), 6.85 (d, J = 8.6, 1H), 6.79 (dd, J = 8.3, 2.7, 1H), 5.63 (s, 1H), 5.13 (s, 1H), 4.05 (t, J = 6.0, 2H), 3.87 (s; 3H), 3.81 (dt, J = 11.2, 2.8, 1H), 3.69-3.64 (m, 2H), 3.43 (s, 2H), 3.27-3.22 (m, 1H), 2.98-2.91 (m, 1H), 2.87 (dt, J = 11.7, 2.6, 1H), 2.47-2.32 (m, 3H), 2.25 (s, 3H), 2.08 (s, 1H), 2.00-1.90 (m, 2H), 0.98 (d, J = 3.6, 3H).

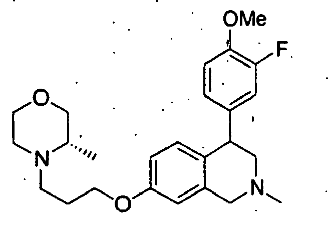

### Example 221; 4-(3-Fluoro-4-methoxy-phenyl)-2-methyl-7-[3-(3S-methyl-morpholin-4-yl)-propoxy]-1,2,3,4-tetrahydro-isoquinoline.

MS (ESI): mass calcd for C₂₅H₃₃FN₂O₃, 428.25; m/z found, 429.4 [M+H]⁺. ¹H NMR (CDCl₃): 6.92-6.84 (m, 3H), 6.77 (d, J = 8.5, 1H), 6.64 (dd, J = 2.6, 8.5, 1H), 6.60 (d, J = 2.5, 1H), 4.12 (t, J = 6.8, 1H), 3.97 (td, J = 6.1, 1.8, 2H), 3.86 (s, 3H), 3.80 (dt, J = 11.2, 2.8, 1H), 3.68-3.55 (m, 3H), 3.26-3.21 (m, 1H), 2.97-2.89 (m, 2H), 2.76 (dt, J = 11.8, 2.7, 1H), 2.54-2.48 (m, 1H), 2.47-2.31 (m, 6H), 1.98-1.84 (m, 2H), 1.80-1.70 (m, 1H), 0.97 (d, J = 6.3, 3H).

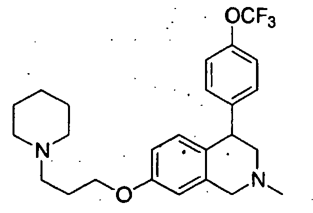

### Example 222; 2-Methyl-7-(3-piperidin-1-yl-propoxy)-4-(4-trifluoromethoxy-phenyl)-1,2,3,4-tetrahydro-isoquinoline.

Yield: 20.7 mg (84%). MS (ESI): mass calcd for C₂₅H₃₁F₃N₂O₂, 448.23; m/z found, 450.5 [M+H]⁺. ¹H NMR (CDCl₃): 7.27-7.68 (s, 1H), 7.32-7.24 (m, 5H), 6.62-6.57 (s, 1H), 4.74-4.67 (m, 1H), 4.66-4.54 (m, 1H), 4.38 (t, J = 5.4, 2H), 4.36-4.24 (m, 1H), 3.81-3.74 (m, 1H), 3.68 (t, J = 11.1, 2H), 3.30-3.11 (m, 3H), 3.02 (s, 3H), 2.75-2.61 (m, 2H), 2.29-2.20 (m, 2H), 2.08-1.86 (m, 5H), 1.50-1.38 (m, 1H).

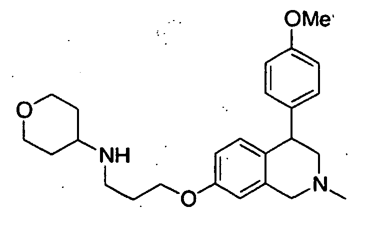

### Example 223; {3-[4-(4-Methoxy-phenyl)-2-methyl-1,2,3,4-tetrahydro-isoquinolin-7-yloxy]-propyl}-(tetrahydro-pyran-4-yl)-amine.

Yield: 28 mg (21%). MS (ESI): mass calcd for C₂₅H₃₄N₂O₃, 410.26; m/z found, 411.4 [M+H]⁺. ¹H NMR (CDCl₃): 7.09 (d, J = 8.6, 2H), 6.83 (d, J = 8.6, 2H), 6.77 (d, J = 8.5, 1H), 6.77 (m. 2H), 4.19-4.13 (m, 1H), 4.03-3.94 (m, 4H), 3.79 (s, 3H), 3.70 (d, J = 14.9, 1H), 3.56 (d, J = 14.8, 1H), 3.44-3.35 (m, 2H), 3.02-2.95 (m, 1H), 2.83 (t, J = 6.9, 2H), 2.72-2.63 (m, 1H), 2.53-2.46 (m, 1H), 2.41 (s, 3H), 1.98-1.90 (m, 2H), 1.87-1.74 (m, 3H), 1.46-1.36 (m, 2H).

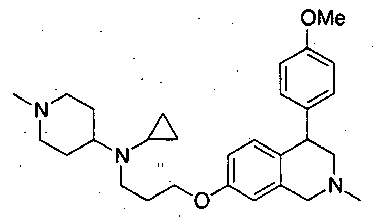

### Example 224; Cyclopropyl-{3-[4-(4-methoxy-phenyl)-2-methyl-1,2,3,4-tetrahydro-isoquinolin-7-yloxy]-propyl}-(1-methyl-piperidin-4-yl)-amine.

Yield: 5 mg (5%). MS (ESI): mass calcd for C₂₉H₄₁N₃O₂, 463.32; m/z found, 464.5 [M+H]⁺. ¹H NMR (CDCl₃): 7.10 (d, J = 8.6, 2H), 6.83 (d, J = 8.7, 2H), 6.76 (d, J = 8.3, 1H), 6.64-6.57 (m, 2H), 4.18-4.13 (m, 1H), 3.92 (t, J = 6.3, 2H), 3.79 (s, 3H), 3.70 (d, J = 14.8, 1H), 3.56 (d, J = 14.9, 1H), 3.02-2.96 (m, 1H), 2.93-2.86 (m, 2H), 2.81 (t, J = 7.0, 2H), 2.60-2.45 (m, 2H), 2.41 (s, 3H), 2.26 (s, 3H), 1.96-1.85 (m, 4H), 1.76-1.62 (m, 6H), 0.50-0.45 (m, 2H), 0.40-0.35 (m, 2H).

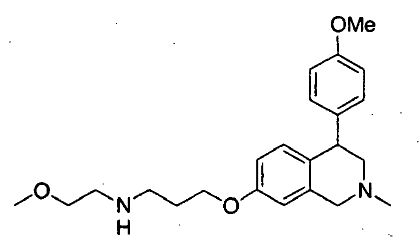

### Example 225; (2-Methoxy-ethyl)-{3-[4-(4-methoxy-phenyl)-2-methyl-1,2,3,4-tetrahydro-isoquinolin-7-yloxy]-propyl}-amine.

Yield: 301.8 mg (86%). MS (ESI): mass calcd for C₂₃H₃₂N₂O₃, 384.24; m/z found, 385.5 [M+H]⁺. ¹H NMR (CDCl₃): 7.09 (d, J = 8.6, 2H), 6.82 (d, J = 7.8, 2H), 6.76 (d, J = 8.4, 1H), 6.65-6.59 (m, 2H), 4.00 (t, J = 6.2, 2H), 2.79-3.75 (m, 3H), 3.70 (d, J = 14.9, 1H), 3.55 (d, J = 14.9, 1H), 3.50 (t, J = 4.8, 2H), 3.36-3.34 (m, 4H), 3.01-2.95 (m, 1H), 2.81-2.76 (m, 4H), 2.52-2.46 (m, 1H), 2.42-2.37 (m, 3H), 1.99-1.91 (m, 2H).

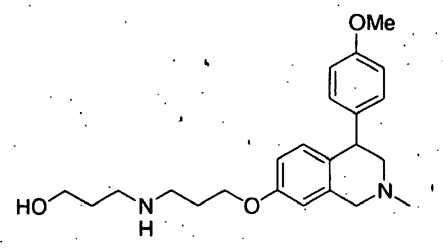

### Example 226; 3-{3-[4-(4-Methoxy-phenyl)-2-methyl-1,2,3,4-tetrahydro-isoquinolin-7-yloxy]-propylamino}-propan-1-ol.

Yield: 178 mg (51 %). MS (ESI): mass calcd for C₂₃H₃₂N₂O₃, 384.24; m/z found, 385.5 [M+H]⁺. ¹H NMR (CDCl₃): 7.09 (d, J = 8.4, 2H), 6.82 (d, J = 8.0, 2H), 6.77 (d, J = 8.4, 1H), 6.65-6.58 (m, 2H), 4.19-4.13 (m, 1H), 3.99 (t, J = 6.1, 2H), 3.81-3.76 (m, 5H), 3.71 (d, J = 14.9, 1H), 3.56 (d, J = 14.9, 1H), 3.44-3.42 (m, 1H), 3.02-2.92 (m, 2H), 2.90-2.84 (m, 2H), 2.82-2.77 (m, 2H), 2.52-2.45 (m, 1H), 2.41 (s, 3H), 1.98-1.90 (m, 2H), 1.73-1.65 (m, 2H).

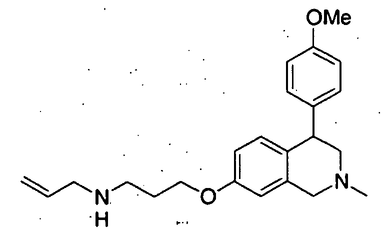

### Example 227; Allyl-{3-[4-(4-methoxy-phenyl)-2-methyl-1,2,3,4-tetrahydro-isoquinolin-7-yloxy]-propyl}-amine.

Yield: 152.3 mg (45%). MS (ESI): mass calcd for C₂₃H₃₀N₂O₂, 366.23; m/z found, 367.5 [M+H]⁺. ¹H NMR (CDCl₃): 7.09 (d, J = 8.7, 2H), 6.82 (d, J = 8.7, 2H), 6.76 (d, J = 8.5, 1H), 6.65-6.58 (m, 2H), 5.96-5.85 (m, 1H), 5.21-5.14 (m, 1H), 5.11-5.06 (m, 1H), 4.18-4.13 (m, 1H); 4.00 (t, J = 6.2, 2H), 3.77 (s, 3H), 3.69 (d, J = 14.8, 1H), 3.55 (d, J = 14.8, 1H), 3.27-3.24 (m, 2H), 3.00-2.94 (m, 1H), 2.79 (t, J = 6.9, 2H), 2.52-2.46 (m, 1H), 2,40 (s, 3H), 1.95 (quintet, J = 6.6, 2H).

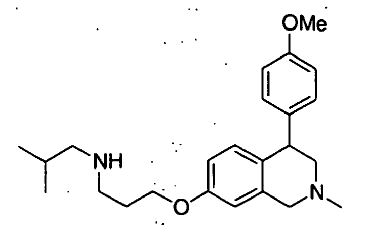

### Example 228; Isobutyl-{3-[4-(4-methoxy-phenyl)-2-methyl-1,2,3,4-tetrahydro-isoquinolin-7-yloxy]-propyl}-amine.

Yield: 238.5 mg (68%). MS (ESI): mass calcd for C₂₄H₃₄N₂O₂, 382.26; m/z found, 383.4 [M+H]⁺. ¹H NMR (CDCl₃): 7.09 (d, J = 8.7, 2H), 6.82 (d, J = 8.7, 2H), 6.77 (d, J = 8.5, 1H), 6.65-6.58 (m, 2H), 4.18-4.12 (m, 1H), 4.00 (t, J = 6.2, 2H), 3.78 (s, 3H), 3.70 (d, J = 14.8, 1H), 3.56 (d, J = 14.9, 1H), 3.01-2.95 (m, 1H), 2.77 (t, J = 6.9, 2H), 2.52-2.46 (m, 1H), 2.43-2.40 (m, 5H), 1.95 (quintet, J = 6.6, 2H), 1.80-1.70 (m, 1H), 0.90 (d, J = 6.6, 6H).

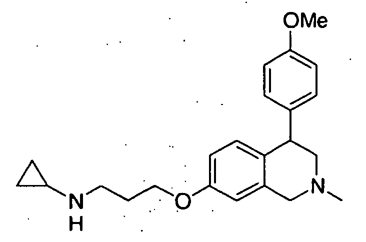

### Example 229; Cyclopropyl-{3-[4-(4-methoxy-phenyl)-2-methyl-1,2,3,4-tetrahydro-isoquinolin-7-yloxy]-propyl}-amine.

Yield: 193.8 mg (58%). MS (ESI): mass calcd for C₂₃H₃₀N₂O₂, 366.23; m/z found, 367.5 [M+H]⁺. ¹H NMR (CDCl₃): 7.09 (d, J = 8.7, 2H), 6.82 (d, J = 8.7, 2H), 6.77 (d, J = 8.5, 1H), 6.66-6.59 (m, 2H), 4.18-4.13 (m, 1H), 3.99 (t, J = 6.2, 2H), 3.78 (s, 3H), 3.79-3.76 (m, 1H), 3.70 (d, J = 14.8, 1H), 3.56 (d, J = 14.9, 1H), 3.01-2.95 (m, 1H), 2.88 (t, J = 7.0, 2H), 2.52-2.46 (m, 1H), 2.41 (s, 3H), 2.16-2.10 (m, 1H), 1.95 (quintet, J = 6.7, 2H), 0.45-0.41 (m, 2H), 0.34-0.30 (m, 2H).

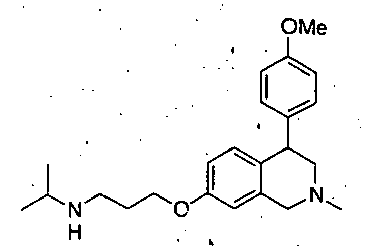

### Example 230; Isopropyl-{3-[4-(4-methoxy-phenyl)-2-methyl-1,2,3,4-tetrahydro-isoquinolin-7-yloxy]-propyl}-amine.

Yield: 112.5 mg (33%). MS (ESI): mass calcd for C₂₃H₃₂N₂O₂, 368.25; m/z found, 369.5 [M+H]⁺: ¹H NMR (CDCl₃): 7.09 (d, J = 8.7, 2H), 6.82 (d, J = 8.6, 2H), 6.77 (d, J = 8.5, 1H), 6.65-6.58 (m, 2H), 4.18-4.12 (m, 1H), 4.00 (t, J = 6.1, 2H), 3.78 (s, 3H), 3.70 (d, J = 14.9, 1H), 3.56 (d, J = 14.9, 1H), 3.01-2.95 (m, 1H), 2.84-2.74 (m, 3H), 2.52-2.46 (m, 1H), 2.41 (s, 3H), 1.94 (quintet, J = 6.7, 2H), 1.06 (d, J = 6.2, 6H).

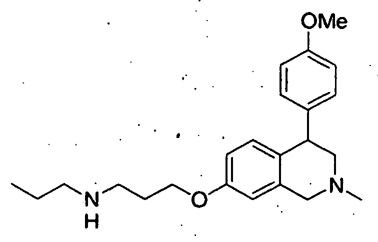

### Example 231; {3-[4-(4-Methoxy-phenyl)-2-methyl-1,2,3,4-tetrahydro-isoquinolin-7-yloxy]-propyl}-propyl-amine.

Yield: 235.4 mg (70%). MS (ESI): mass calcd for C₂₃H₃₂N₂O₂, 368.25; m/z found, 369.5 [M+H]⁺. ¹H NMR (CDCl₃): 7.09 (d, J = 8.6, 2H), 6.82 (d, J = 8.6, 2H), 6.77 (d, J = 8.5, 1H), 6.65-6.54 (m, 2H), 4.18-4.12 (m, 1H), 4.00 (t, J = 6.2, 2H), 3.78 (s, 3H), 3.70 (d, J = 14.9, 1H), 3.56 (d, J = 14.8, 1H), 3.01-2.95 (m, 1H), 2.78 (t, J = 7.0, 2H), 2.58 (t, J = 7.2, 2H), 2.52-2.46 (m, 1H), 2.41 (s, 3H), 1.95 (quintet, J = 6.6, 2H), 1.52 (m, 2H), 0.92 (t, J = 7.4, 3H).

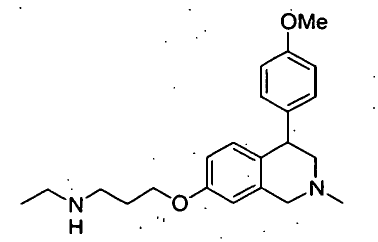

### Example 232; Ethyl-{3-[4-(4-methoxy-phenyl)-2-methyl-1,2,3,4-tetrahydro-isoquinolin-7-yloxy]-propyl}-amine.

Yield: 7.7 mg (4%). MS (ESI): mass calcd for C₂₂H₃₀N₂O₂, 354.23; m/z found, 355.5 [M+H]⁺. ¹H NMR (CDCl₃): 7.10 (d, J = 8.7, 2H), 6.83 (d, J = 8.7, 2H), 6.77 (d, J = 8.4, 1H), 6.66-6.59 (m, 2H), 4.18-4.13 (m, 1H), 4.01 (t, J = 6.2, 2H), 3.79 (s, 3H), 3.70 (d, J = 14.9, 1H), 3.56 (d, J = 14.8, 1H), 3.01-2.95 (m, 1H), 2.80 (t, J = 7.0, 2H), 2.68 (q, J = 7.2, 2H), 2.52-2.46 (m, 1H), 2.41 (s, 3H), 1.98 (quintet, J = 6.4, 2H), 1.12 (t, J = 7.1, 3H).

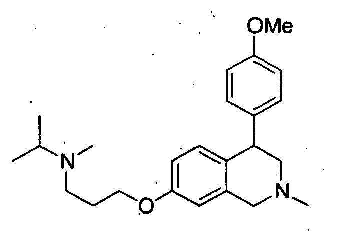

### Example 233; Isopropyl-{3-[4-(4-methoxy-phenyl)-2-methyl-1,2,3,4-tetrahydro-isoquinolin-7-yloxy]-propyl}-methyl-amine.

Yield: 15 mg (7%). MS (ESI): mass calcd for C₂₄H₃₄N₂O₂, 382.26; m/z found, 383.4 [M+H]⁺. ¹H NMR (CDCl₃): 7.09 (d, J = 8.7, 2H), 6.82 (d, J = 8.7, 2H), 6.76 (d, J = 8.5, 1H), 6.66-6.59 (m, 2H), 4.18-4.13 (m, 1H), 3.97 (t, J = 6.3, 2H), 3.78 (s, 3H), 3.70 (d, J = 14.8, 1H), 3.56 (d, J = 14.8, 1H), 3.01-2.95 (m, 1H), 2.86-2.78 (m, 1H), 2.55-2.46 (m, 3H), 2.41 (s, 3H), 2.21 (s, 3H), 1.94-1.86 (m, 2H), 0.99 (d, J = 6.6, 6H).

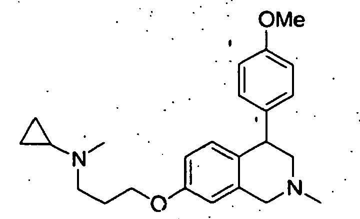

### Example 234; Cyclopropyl-{3-[4-(4-methoxy-phenyl)-2-methyl-1,2,3,4-tetrahydro-isoquinolin-7-yloxy]-propyl}-methyl-amine.

Yield: 25.2 mg (40%) as a TFA salt. MS (ESI): mass calcd for C₂₄H₃₂N₂O₂, 380.25; m/z found, 381.4 [M+H]⁺. ¹H NMR (CDCl₃): 7.17-6.99 (m, 2H), 6.93-6.69 (m, 4H), 6.63 (br s, 1H), 4.92-4.54 (m, 2H); 4.17-3.99 (m, 3H), 3.86-3.69 (m, 4H), 3.60-3.21 (m, 2H), 3.06-2.88 (m, 6H); 2.75-2.20 (m, 4H), 1.53-1.15 (m, 2H), 0.92 (br s, 2H).

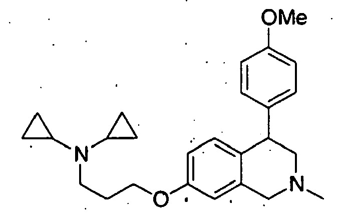

### Example 235; Dicyclopropyl-{3-[4-(4-methoxy-phenyl)-2-methyl-1,2,3,4-tetrahydro-isoquinolin-7-yloxy]-propyl}-amine.

Yield: 35.4 mg (32%) as a TFA salt. MS (ESI): mass calcd for C₂₄H₃₂N₂O₂, 380.25; m/z found; 381.4 [M+H]⁺. ¹H NMR (CDCl₃): 7.14-7.04 (m, 2H), 6.92-6.70 (m, 4H), 664 (br s, 1H), 4.73-4.54 (m, 1H), 4.18-4.00 (m, 3H), 3.84-3.73 (m, 4H), 3.59-3.32 (m, 3H), 3.10-2.92 (m, 4H), 2.62-2.53 (m, 2H), 2.46-2.28 (m, 2H), 1.40-1.28 (m, 4H), 0.97-0.81 (m, 4H).

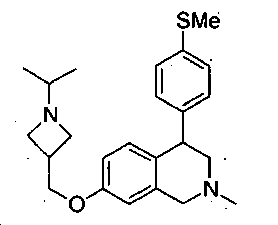

### Example 236; 7-(1-Isopropyl-azetidin-3-ylmethoxy)-2-methyl-4-(4-methylsulfanyl-phenyl)-1,2,3,4-tetrahydro-isoquinoline.

Step 1; 3-Hydroxymethyl-azetidine-1-carboxylic acid tert-butyl ester. To a 0 °C solution of azetidine-1,3-dicarboxylic acid mono-tert-butyl ester (0.50 g, 2.48 mmol) and TEA (0.30 mL, 2.98 mmol) in THF (25 mL) was added isobutylchloroformate (0.39 mL, 2.98 mmol). The mixture was stirred at rt for 1 h, filtered, and concentrated to approximately half volume. The resultant solution was cooled to 0 °C and was treated with NaBH₄ (188 mg, 4.96 mmol) and water (12 mL). After 2 h, the mixture was allowed to warm to rt and was stirred overnight. The mixture was diluted with EtOAc, washed with water and brine, dried over MgSO₄, and concentrated to give 310 mg (67%) of crude material, which was carried forward without purification. MS (ESI): mass calcd for C₉H₁₇NO₃, 187.12; m/z found, 210.4 [M+Na]⁺. ¹H NMR (CDCl₃): 3.98 (t, J = 8.6, 2H), 3.76-3.73 (m, 2H), 3.68-3.66 (m, 2H), 2.70-2.66 (m, 1H), 2.03-1.99 (m, 1H), 1.42 (s, 9H).

Step 2; 3-(3-Formyl-phenoxymethyl)-azetidine-1-carboxylic acid tort-butyl ester. A mixture of 3-hydroxymethyl-azetidine-1-carboxylic acid tert-butyl ester (3.35 g, 18.96 mmol), 3-hydroxybenzaldehyde (3.47 g, 28.44 mmol), DEAD (4.95 g, 28.44 mmol), resin bound-PPh₃ (9.50 g, 28.44 mmol), and DCM (95 mL) was placed on a shaker for 2 days. The mixture was then filtered and the filtrate was concentrated. The residue was purified by FCC to give 1.35 g (24%) of product. ¹H NMR (CDCl₃): 9.96 (s, 1H), 7.47-7.42 (m, 2H), 7.38-7.36 (m, 1H); 7.18-7.16 (m, 1H), 4.14 (d, J = 6.6, 2H), 4.10 (t, J = 8.6, 2H), 3.81-3.78 (m, 2H), 3.00-2.97 (m, 1H), 1.44 (s, 9H).

Step 3; 3-(3-Methylaminomethyl-phenoxymethyl)-azetidine-1-carboxylic acid tert-butyl ester. Prepared as described in Example 1, Step 2. Yield: 2.33 g (83%). MS (ESI): mass calcd for C₁₇H₂₆N₂O₃, 306.19; m/z found, 307.5 [M+H]⁺. ¹H NMR (CDCl₃): 7.23 (t, J = 7.8, 1H), 6.91-6.87 (m, 2H), 6.78-6.76 (m, 1H), 4.08-4.03 (m, 4H), 3.79-3.75 (m, 2H), 3.67 (s, 2H), 2.95-2.92 (m, 1H), 2.44 (s, 3H), 1.43 (s, 9H). Step 4; 3-[3-({Methyl-[2-(4-methylsulfanyl-phenyl)-2-oxo-ethyl]-amino}-methyl)-phenoxymethyl]-azetidine-1-carboxylic acid tert-butyl ester. Prepared as described in Example 1, Step 3. MS (ESI): mass calcd for C₂₆H₃₄N₂O₄S, 470.22; m/z found, 471.5 [M+H]⁺.

Step 5. 7-(1-Isopropyl-azetidin-3-vlmethoxy)-2-methyl-(4-methylsulfanyl-phenyl)-isoguinolinium. Prepared as described in Example 1, Step 4. Yield: 1.3 g (48% over 2 steps).

Step 6. The title compound was prepared according to the methods described in Example 1, Step 5, with the reaction performed in the presence of acetone. Yield: 210 mg (9%) as a TFA salt. MS (ESI): mass calcd for C₂₄H₃₂N₂OS, 396.22; m/z found, 397.3 [M+H]⁺. ¹H NMR (acetone-d₆): 7.24 (d, J = 8.3, 2H), 7.14 (d, J = 8.2, 2H), 6.86-6.79 (m, 2H), 6.67 (d, J = 8.1, 1H), 4.45-4.29 (m, 3H), 4.25-4.20 (m, 1H), 4.19-4.02 (m, 4H), 3.92-3.82 (m, 2H), 3.65-3.56 (m, 1H), 3.17-3.06 (m, 1H), 3.02-2.92 (m, 1H), 2.85 (br s, 3H), 2.45 (s, 3H), 1.11 (d, J = 6.3, 6H).

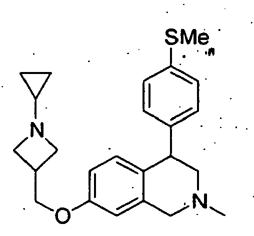

### Example 237; 7-(1-Cyclopropyl-azetidin-3-ylmethoxy)-2-methyl-4-(4-methylsulfanyl-phenyl)-1,2,3,4-tetrahydro-isoquinoline.

Step 1; 2-Bromo-1-(4-methylsulfanyl-phenyl)-ethanone. To a water bath-cooled solution of 1-(4-methylsulfanyl-phenyl)-ethanone (200.3 g, 1.205 mmol) in MeOH (720 mL) was added Br₂ (61.5 mL, 1.20 mmol) dropwise over 1 h via an additional funnel. After addition was complete, water was added and the mixture was stirred vigorously. Vacuum filtration gave the desired product (285.5 g, 97%) as a solid. MS (ESI): mass calcd. for C₉H₉BrOS, 243.96; m/z found, 245.2, 247.2 [M+H]⁺. ¹H NMR (acetone-d₆): 7.98 (d, J = 8.7, 2H), 7.40 (d, J = 8.7, 2H), 4.72 (s, 2H), 2.59 (s, 3H).

Step 2; 2-Methylamino-1-(4-methylsulfanyl-phenyl)-ethanol. To a solution of EtOH (400 mL) and 40% aq. MeNH₂ (63.5 mL, 816 mmol) was added slowly 2-bromo-1-(4-methylsulfanyl-phenyl)-ethanone (10.08 g, 40.79 mmol) with vigorous stirring over 20 min. NaBH₄ (4.764 g, 122.4 mmol) was then added portionwise. After 18 h, the mixture was concentrated and purified by FCC to give the desired product (6.34 g, 78%). MS (ESI): mass calcd. for C₁₀H₁₅NOS, 197.09; m/z found, 198.1 [M+H]⁺. ¹H NMR (MeOD): 7.31 (d, J = 8.2, 2H), 7.26 (d, J = 8.4, 2H), 4.76-4.72 (m, 1H), 2.76-2.72 (m, 1H), 2.70-2.65 (m, 1H), 2.47 (s, 3H), 2.41 (s, 3H).

Step 3; 3-({[2-Hydroxy-2-(4-methylsulfanyl-phenyl)-ethyl]-methyl-amino}-methyl)-phenol. To a mixture of 2-methylamino-1-(4-methylsulfanyl-phenyl)-ethanol (3.40 g, 17.2 mmol), 3-hydroxy-benzaldehyde (2.81 g, 22.4 mmol), and acetic acid (0.99 mL, 17 mmol) in THF (50 mL) at 0 °C was added NaB(OAc)₃H (8.57 g, 43.1 mmol) and the mixture was stirred at rt for 3 days. The mixture was concentrated and the residue taken up in 1 N NaOH and extracted with DCM. The combined organic layers were dried (Na₂SO₄) and concentrated. Purification by FCC gave the desired product (3.51 g, 78%) as a crystalline solid. MS (ESI): mass calcd. for C₁₇H₂₁NO₂S, 303.13; m/z found, 304.3 [M+H]⁺. ¹H NMR (MeOD): 7.24 (d, J = 8.2, 2H), 7.20 (d, J = 8.3, 2H), 7.13 (t, J = 7.8, 1H); 6.80 (s, 1H), 6.77 (d, J = 7.4, 1H), 6.72-6.71 (m, 1H), 4.78-4.74 (m, 1H), 3.56 (d, J = 13.0, 1H), 3.48 (d, J = 13.0, 1H), 2.66-2.61 (m, 1H), 2.51-2.47 (m, 1H), 2.41 (s, 3H), 2.28 (s, 3H).

Step 4; 2-Methyl-4-(4-methylsulfanyl-phenyl)-1,2,3,4-tetrahydro-isoquinolin-7-ol. Prepared in an analogous fashion to Example 1, Steps 4 and 5, with heating at 50 °C to give the desired product (3.08 g, 67%). MS (ESI): mass calculated for C₁₇H₁₉NOS, 285.12; m/z found, 286.3 [M+H]⁺. ¹H NMR (MeOD): 7.21 (d, J = 6.9, 2H), 7.10 (d, J = 7.1, 2H), 6.63 (d, J = 9.1, 1H), 6.58-6.54 (m, 2H), 4.24-4.18 (m, 1H), 3.82 (d, J = 14.9, 1H), 3.61 (d, J = 14.9, 1H), 3.15-3.10 (m, 1H), 2.57 (t, J = 11.1, 1H), 2.48 (s, 3H), 2.46 (s, 3H).

Step 5; 3-(Toluene-4-sulfonyloxymethyl)-azetidine-1-carboxylic acid tert-butyl ester. To a solution of 3-hydroxymethyl-azetidine-1-carboxylic acid tert-butyl ester (250 mg, 1.3 mmol) in pyridine (2.2 mL) at 0 °C was added *p*-toluenesulfonyl chloride (380 mg, 2.0 mmol). After 18 h, the mixture was poured into Et₂O and 2 N HCl. The mixture was then washed with Et₂O (3x) and the combined organic layers were washed with brine, dried, (MgSO₄), and concentrated. The crude material was purified by FCC (EtOAc/hexanes) to give 240 mg (55%) of a yellow solid. ¹H NMR (CDCl₃): 7.78 (d, J = 8.3, 2H), 7.35 (d, J = 8.0, 2H), 4.12 (d, J = 6.9, 2H), 3.95 (t, J = 8.6, 2H), 3.59-3.54 (m, 2H), 2.86-2.77 (m, 1H), 2.45 (s, 3H), 1.40 (s, 9H).

Step 6; 3-[2-Methyl-4-(4-methylsulfanyl-phenyl)-1,2,3,4-tetrahydro-isoquinolin-7-yloxymethyl]-azetidine-1-carboxylic acid tert-butyl ester. A mixture of 2-methyl-4-(4-methylsulfanyl-phenyl)-1,2,3,4-tetrahydro-isoquinolin-7-ol (260 mg, 0.91 mmol), 3-(toluene-4-sulfonyloxymethyl)-azetidine-1-carboxylic acid tert-butyl ester (373 mg, 1.09 mmol), Cs₂CO₃ (889 mg, 2.73 mmol), and DMSO (9 mL) was heated at 50 °C for 4 h. The mixture was cooled to rt and poured into Et₂O. The organic layer was washed with 1 N NaOH and brine, dried over MugSO₄, and concentrated. The crude material was purified by FCC to give 350 mg (85%) of product. MS (ESI): mass calcd for C₂₆H₃₄N₂O₃S, 454.23; m/z found, 455.4 [M+H]⁺. ¹H NMR (CDCl₃): 7.18 (d, J = 8.3, 2H), 7.09 (d, J = 8.3, 2H), 6.76 (d, J = 8.5, 1H), 6.64-6.58 (m, 2H), 4.17-4.13 (m, 1H), 4.07-4.02 (m, 4H), 3.78-3.74 (m, 2H), 3.68 (d, J = 14.9, 1H), 3.57 (d, J = 14.9; 2.99-2.87 (m, 2H), 2.53-2.47 (m, 1H), 2.46 (s, 3H), 2.40 (s, 3H), 1.43 (s, 9H).

Step 7; 7-(Azetidin-3-ylmethoxy)-2-methyl-4-(4-methylsulfanyl-phenyl)-1,2,3,4-tetrahydro-isoquinoline. To a solution of 3-[2-methyl-4-(4-methylsulfanyl-phenyl)-1,2,3,4-tetrahydro-isoquinolin-7-yloxymethyl]-azetidine-1-carboxylic acid tert-butyl ester (150 mg, 0.33 mmol) in DCM (2 mL) was added TFA (0.5 mL). After 18 h, the mixture was concentrated and the residue was diluted with MeOH (8 mL) and and treated with Dowex 550A (OH) anion exchange resin. After 1.5 h, the mixture was filtered, and the filtrate was concentrated to give 125 mg (100%) of product. MS (ESI): mass calcd for C₂₁H₂₆N₂OS, 354.52; m/z found, 355.4 [M+H]⁺. H NMR (CDCl₃): 7.17 (d, J = 8.3, 2H), 7.09 (d, J = 8.3, 2H), 6.77 (d, J = 8.0, 1H), 6.66-6.63 (m, 2H), 4.18-4.13 (m, 1H), 4.08-4.03 (m, 4H), 3.93-3.90 (m, 2H), 3.69 (d, J = 14.9, 1H), 3.58 (d, J = 15.0, 1H), 3.30-3.2.1 (m, 1H), 2.99-2.95 (m, 1H), 2.52-2.48 (m, 1H), 2.46 (s, 3H), 2.40 (s, 3H).

Step 8. To a solution of 7-(azetidin-3-ylmethoxy)-2-methyl-4-(4-methylsulfanyl-phenyl)-1,2,3,4-tetrahydro-isoquinoline (40 mg, 0.11 mmol) in MeOH (1.1 mL) was added acetic acid (0.06 mL, 1.1 mmol); 4Å powdered molecular sieves (- 25 mg), 1-ethoxycyclopropyltrimethylsilane (0.12 mL, 0.66 mmol), and NaCNBH₃ (31 mg, 0.50 mmol). After 18 h at 50°C the mixture was diluted with DCM, washed with 1 N NaOH and brine, dried over MgSO₄, and concentrated. The crude material was purified by reverse phase chromatography to give 15.2 mg (35%) of product. MS (ESI): mass calcd for C₂₄H₃₀N₂OS, 394.21; m/z found, 395.4 [M+H]⁺. ¹H NMR (CDCl₃): 7.17 (d, J = 8.3, 2H), 7.09 (d, J = 8.3, 2H), 6.75 (d, J = 8.3, 1H), 6.64-5.59 (m, 2H), 4.17-4.13 (m, 1H), 4.02 (d, J = 6.5, 2H), 3.68 (d, J = 14.8, 1H), 3.57 (d, J = 14.9, 1H), 3.53-3.49 (m, 2H), 3.20-3.16 (m, 2H), 2.99-2.94 (m, 2H), 3.20-3.16 (m, 2H), 2.99-2.94 (m, 1H), 2.90-2.84 (m, 1H), 2.52-2.47 (m, 1H), 2.46 (s, 3H), 2.40 (s, 3H), 1.89-1.85 (m, 1H), 0.39-0.32 (m, 4H).

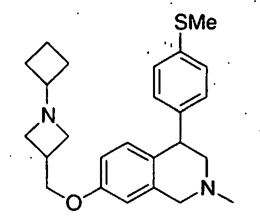

### Example 238; 7-(1-Cyclobutyl-azetidin-3-ylmethoxy)-2-methyl-4-(4-methylsulfanyl-phenyl)-1,2,3,4-tetrahydro-isoquinoline.

To a solution of 7-(azetidin-3-ylmethoxy)-2-methyl-4-(4-methylsulfanyl-phenyl)-1,2,3,4-tetrahydro-isoquinoline (40 mg, 0.11 mmol) and cyclobutanohe (9.0 µL, 0.13 mmol) in THF (0.6 mL) at 0 °C was added NaB(OAC)₃H (35 mg, 0.17 mmol) and the mixture was stirred overnight at rt. The mixture was diluted with DCM, washed with 1 N NaOH and brine, dried (MgSO₄), and concentrated. The residue was purified by reverse phase chromatography to give 10.1 mg (22%) of product. MS (ESI): mass calcd for C₂₅H₃₂N₂OS, 408.22; m/z found, 409.4 (M+H)⁺. ¹H NMR (CDCl₃): 7.18 (d, J = 8.3, 2H), 7.09 (d, J = 8.3, 2H), 6.77 (d, J = 8.5, 1H), 6.59-6.64 (m, 2H), 4.18-4.13 (m, 1H), 4.00 (d, J = 5.4, 2H), 3.78-3.65 (m, 3H), 3.57 (d, J = 14.9, 3.40 (br s, 3H), 3.12-3.02 (m, 1H), 2.99-2.95 (m, 1H), 2.54-2.47 (m, 1H), 2.46 (s, 3H), 2.41 (s, 3H), 2.10-1.97 (m, 4H), 1.87-1.77 (m, 1H), 1.75-1.68 (m, 1H).

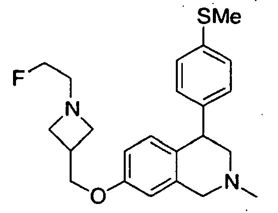

### Example 239; 7-[1-(2-Fluoro-ethyl)-azetidin-3-ylmethoxy]-2-methyl-4-(4-methylsulfanyl-phenyl)-1,2,3,4-tetrahydro-isoquinoline.

A mixture of 7-(azetidin-3-ylmethoxy)-2-methyl-4-(4-methylsulfanyl-phenyl)-1,2,3,4-tetrahydro-isoquinoline (40 mg, 0.11 mmol), 1-bromo-2-fluoroethane (10 µL, 0.13 mmol), and K₂CO₃ (46 mg, 0.33 mmol) in acetonitrile (1 mL) was heated at 50 °C overnight. The mixture was filtered and concentrated, and the residue was purified by reverse phase chromatography to give 12.0 mg (27%) of product. MS (ESI): mass calcd for C₂₃H₂₉FN₂OS, 400.20; m/z found, 401.4 [M+H]⁺. ¹H NMR (CDCl₃): 7.18 (d, J = 8.3, 2H), 7.09 (d, J = 8.3, 2H), 6.77 (d, J = 8.5, 1H), 6.65-6.60 (m, 2H), 4.57-4.54 (m, 1H), 4.48-4.44 (m, 1H), 4.21-4.16 (m, 1H), 4.03 (d, J = 5.9, 2H), 3.78-3.60 (m, 4H), 3.42-3.30 (m, 2H), 3.06-3.00 (m, 2H), 2.96-2.85 (m, 2H), 2.58-2.52 (m, 1H), 2.48-2.43 (m, 6H).

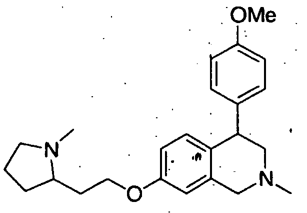

### Examples 240 (mixture), 240A (diastereomer 1) and 240B (diastereomer 2); 4-(4-Methoxy-phenyl)-2-methyl-7-[2-(1-methyl-pyrrolidin-2-yl)-ethoxy]-1,2,3,4-tetrahydro-isoquinoline.

To a solution of 2-methyl-4-(4-methylsulfanyl-phenyl)-1,2,3,4-tetrahydro-isoquinolin-7-ol (100 mg, 0.37 mmol) in DCM (4 mL) was added 2-(1-methylpyrrolidin-2-yl)-ethanol (47 mg, 0.38 mmol), PPh₃ (102 mg, 0.39 mmol), and DEAD (64 mg, 0.37 mmol). After 3 days, the mixture was concentrated and the residue was purified by FCC to give 60 mg (43%) of Example 240 as a mixture of diastereomers. MS (ESI): mass calcd for C₂₄H₃₂N₂O₂, 380.25; m/z found, 381.4 [M+H]⁺. The diastereomers were separated (SFC HPLC) to provide Example 240A (first eluting) and Example 240B (second eluting).

Example 240A. MS (ESI): mass calcd for C₂₄H₃₂N₂O₂, 380.25; m/z found, 381.4 [M+H]⁺. ¹H NMR (CDCl₃): 7.09 (d, J = 8.7, 2H), 6.83 (d, J = 8.7, 2H), 6.77 (d, J = 8.5, 1H), 6.65-6.58 (m, 2H), 4.19-4.13 (m, 1H), 4.09-4.02 (m, 1H), 4.00-3.91 (m, 1H), 3.79 (s, 3H), 3.71 (d, J = 14.9, 1H), 3.56 (d, J = 14.8, 1H), 3.32-3.25 (m, 1H), 3.02-2.96 (m, 1H), 2.60-2.49 (m, 2H), 2.47 (s, 3H), 2.42 (s, 3H), 2.37-2.22 (m, 2H), 2.13-2.04 (m, 1H), 1.96-1.74 (m, 3H), 1.73-1.62 (m, 1H).

Example 240B. MS (ESI): mass calcd for C₂₄H₃₂N₂O₂, 380.25; m/z found, 381.4 [M+H]⁺. ¹H NMR (CDCl₃): 7.16-7.04 (m, 2H), 6.91-6.72 (m, 3H), 6.67-6.54 (m, 2H), 4.25-4.10 (m, 2H), 4.05-3.89 (m, 1H), 3.79 (s, 3H), 3.75-3.64 (m, 1H), 3.62-3.50 (m, 1H), 3.12-3.93 (m, 2H), 2.75-2.47 (m, 2H), 2.41 (s, 3H), 2.37-2.27 (m, 4H), 2.25-1.44 (m, 8H).

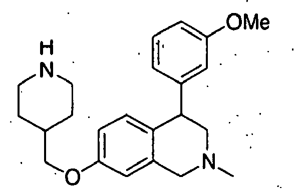

### Example 241; 4-(3-Methoxy-phenyl)-2-methyl-7-(piperidin-4-ylmethoxy)-1,2,3,4-tetrahydro-isoquinoline.

Prepared by a sequence similar to that described in Example 78 to give 950 mg (57%) of product. MS (ESI): mass calcd for C₂₃H₃₀N₂O₂, 366.23; m/z found, 367.5 [M+H]⁺.

The compounds in Examples 242-250 were prepared by a sequence similar to that described in Example 83.

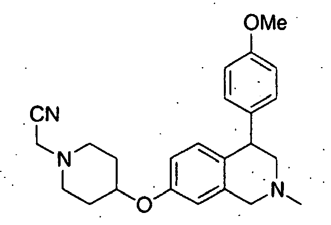

### Example 242; {4-[4-(4-Methoxy-phenyl)-2-methyl-1,2,3,4-tetrahydro-isoquinolin-7-yloxymethyl]-piperidin-1-yl}-acetonitrile.

Yield: 11 mg (4%) as a TFA salt. MS (ESI): mass calcd for C₂₄H₂₉N₃O₂, 391.23; m/z found, 392.5 [M+H]⁺. ¹H NMR (CDCl₃): 7.13-7.01 (m, 2H), 6.87 (d, J = 8.5, 2H), 6.85-6.81 (m, 1H), 6.80-6.74 (m, 1H), 6.72-6.93 (m, 1H), 5.10-4.75 (m, 1H), 4.73-4.65 (m, 1H), 4.64-4.56 (m, 2H), 4.47-4.42 (m, 1H), 4.13-4.04 (m, 1H), 3.80 (s, 3H), 3.80-3.74 (m, 2H), 3.08-3.02 (m, 2H), 2.99 (s, 3H), 2.90-2.84 (m, 2H), 2.15-2.07 (m, 2H), 2.05-1.95 (m, 3H).

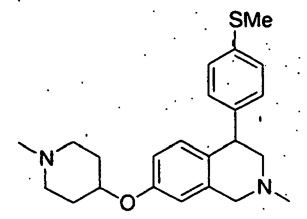

### Example 243; 2-Methyl-7-(1-methyl-piperidin-4-yloxy)-4-(4-methylsulfanyl-phenyl)-1,2,3,4-tetrahydro-isoquinoline.

Yield: 23.7 mg (50%). MS (ESI): mass calcd for C₂₃H₃₀N₂OS, 382.21; m/z found, 383.4 [M+H]⁺. ¹H NMR (CDCl₃): 7.19 (d, J = 8.3, 2H), 7.11 (d, J = 8.0, 2H), 6.76 (d, J = 8.5, 1H), 6.65-6.60 (m, 2H), 4.16 (t, J = 7.9, 1H), 3.68 (d, J = 14.9, 1H), 3.57 (d, J = 14.9, 1H), 3.00-2.96 (m, 1H), 2.74 (s,3H), 2.41 (s, 3H), 2.31 (s, 3H), 2.10-1.95 (m, 2H), 1.86-1.70 (m, 6H).

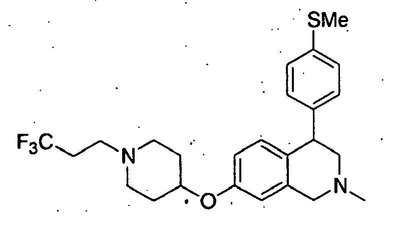

### Example 244; 2-Methyl-4-(4-methylsulfanyl-phenyl)-7-[1-(3,3,3-trifluoro-propyl)-piperidin-4-yloxy]-1,2,3,4-tetrahydro-isoquinoline.

Yield: 44 mg (33%). MS (ESI): mass calcd for C₂₅H₃₁F₃N₂OS, 464.21; m/z found, 465.4 [M+H]⁺. ¹H NMR (CDCl₃): 7.18 (d, J = 8.2, 2H), 7.11 (d, J = 8.2, 2H), 6.76 (d, J = 8.4, 1H), 6.65-6.60 (m, 2H), 4.31-4.26 (m, 1H), 4.17-4.14 (m, 1H), 3.68 (d, J = 14.8, 1H), 3.57 (d, J = 14.8, 1H), 3.00-2.95 (m, 1H), 2.75-2.70 (m, 2H), 2.64-2.60 (m, 2H), 2.53-2.54 (m, 1H), 2.47 (s, 3H), 2.41 (s, 3H), 2.37-2.29 (m, 4H), 2.00-1.94 (m, 2H), 1.85-1.79 (m, 2H).

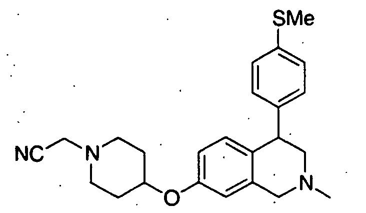

### Example 245; {4-[2-Methyl-4-(4-methylsulfanyl-phenyl)-1,2,3,4-tetrahydro-isoquinolin-7-yloxy]-piperidin-1-yl}-acetonitrile.

Yield: 61 mg (65%). MS (ESI): mass calcd for C₂₄H₂₉N₃OS, 407.20; m/z found, 408.4 [M+H]⁺. ¹H NMR (CDCl₃): 7.19 (d, J = 8.2, 2H), 7.11 (d, J = 8.2, 2H), 6.76 (d, J = 8.4, 1H), 6.65-6.61 (m, 2H), 4.33-4.28 (m, 1H). 4.16 (t, J = 7.3, 1H), 3.68 (d, J = 14.9, 1H), 3.57 (d, J = 14.9, 1H), 3.54 (s, 2H), 3.00-2.95 (m, 1H), 2.84-2.79 (m, 2H), 2.55-2.49 (m, 3H), 2.47 (s, 3H), 2.41 (s, 3H), 2.04-1.98 (m, 2H), 1.89-1.83 (m, 2H).

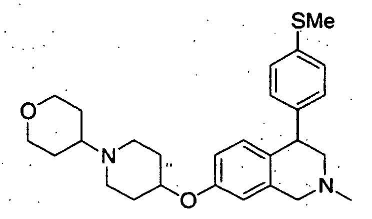

### Example 246; 2-Methyl-4-(4-methylsulfanyl-phenyl)-7-[1-(tetrahydro-pyran-4-yl)-piperidin-4-yloxy]-1,2,3,4-tetrahydro-isoquinoline.

Yield: 35 mg (32%). MS (ESI): mass calcd for C₂₇H₃₆N₂O₂S, 452.25; m/z found, 453.5 [M+H]⁺. ¹H NMR (CDCl₃): 7.18 (d, J= 8.3, 2H), 7.11 (d, J = 8.3, 2H), 6.75 (d, J = 8.5, 1H), 6.65-6.60 (m, 2H), 4.29-4.24 (m, 1H), 4.17-4.13 (m, 1H), 4.03 (dd, J = 11.1, 4.2, 2H), 3.68 (d, J = 14.9, 1H), 3.57 (d, J = 14.8, 1H), 3.41-3.35 (m, 2H), 2.99-2.95 (m, 1H), 2.85-2.80 (m, 2H), 2.53-2.48 (m, 1H), 2.47 (s, 3H), 2.45-2.41 (m, 2H), 2.41 (s, 3H); 2.01-1.95 (m, 3H), 1.84-1.74 (m, 4H), 1.65-1.56 (m, 2H).

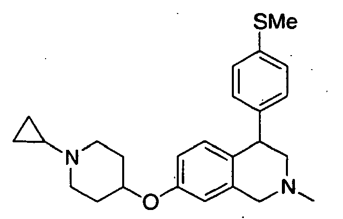

### Example 247; 7-(1-Cyclopropyl-piperidin-4-yloxy)-2-methyl-4-(4-methylsulfanyl-phenyl)-1,2,3,4-tetrahydro-isoquinoline.

Yield: 25 mg (28%). MS (ESI): mass calcd for C₂₅H₃₂N₂OS, 408.22; m/z found, 409.5 [M+H]⁺. ¹H NMR (CDCl₃): 7.18 (d, J = 8.3, 2H), 7.11 (d, J = 8.3, 2H), 6.75 (d, J = 8.4, 1H), 6.66-6.61 (m, 2H), 4.29-4.24 (m, H), 4.17-4.14 (m, 1H), 3.68 (d, J = 14.8, 1H), 3.57 (d, J = 14.8, 1H), 3.00-2.95 (m, 1H), 2.93-2.86 (m, 2H), 2.53-2.49 (m, 2H); 2.64 (s, 3H), 2.41 (s, 3H), 2.02-1.90 (m, 3H), 1.80-1.72 (m, 2H), 1.64-1.60 (m, 1H), 0.48-0.40 (m, 4H).

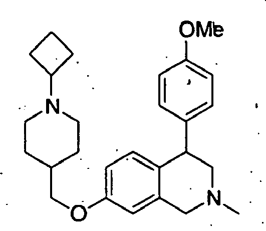

### Example 248; 7-(1-Cyclobutyl-piperidin-4-ylmethoxy)-4-(4-methoxy-phenyl)-2-methyl-1,2,3,4-tetrahydro-isoquinoline.

Yield: 28.2 mg (21%). MS (ESI): mass calcd for C₂₇H₃₆N₂O₂, 420.28; m/z found, 421.5 [M+H]⁺.

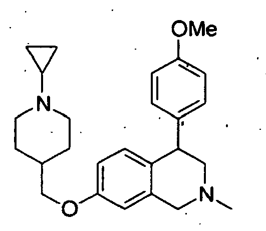

### Example 249; 7-(1-Cyclopropyl-piperidin-4-ylmethoxy)-4-(4-methoxy-phenyl)-2-methyl-1,2,3,4-tetrahydro-isoquinoline.

Yield: 330 mg (71 %). MS (ESI): mass calcd for C₂₆H₃₄N₂O₂, 406.26; m/z found, 407.5 [M+H]⁺. ¹H NMR (CDCl₃): 7.09 (d, J = 8.7, 2H), 6.82 (d, J = 8.7, 2H), 6.76 (d, J = 8.5, 1H), 6.64-6.57 (m, 2H), 4.18-4.13 (m, 1H), 3.79 (s, 3H), 3.76-3.67 (m, 2H), 3.59-3.53 (m, 1H), 3.11-3.04 (m, 2H), 3.01-2.95 (m, 1H), 2.52-2.46 (m, 1H), 2.41 (s, 3H), 2.23-2.15 (m, 2H), 1.85-1.74 (m, 4H), 1.61-1.54 (m, 1H), 1.38-1.25 (m, 2H), 0.47-0.40 (m, 4H).

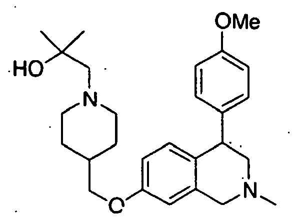

### Example 250; 1-{4-[4-(4-Methoxy-phenyl)-2-methyl-1,2,3,4-tetrahydro-isoquinolin-7-yloxymethyl]-piperidin-1-yl}-2-methyl-propan-2-ol.

Yield: 13 mg (7%) as a TFA salt. MS (ESI): mass calcd for C₂₇H₃₈N₂O₃, 438.29; m/z found, 439.4 [M+H]⁺. ¹H NMR (CDCl₃): 7.22-7.06 (m, 2H), 6.98-6.69 (m, 5H), 4.64-4.38 (m, 2H), 4.01-3.68 (m, 7H), 3.53-2.93 (m, 9H); 2.27-1.95 (m, 3H), 1.92-1.74 (m, 2H), 1.35 (br s, 7H).

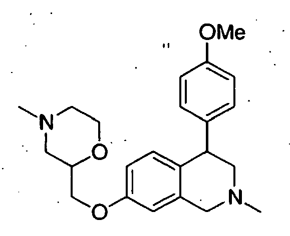

### Example 251; 4-(4-Methoxy-phenyl)-2-methyl-7-(4-methyl-morpholin-2-ylmethoxy)-1,2,3,4-tetrahydro-isoquinoline.

Step 1; 2-[4-(4-Methoxy-phenyl)-2-methyl-1,2,3,4-tetrahydro-isoquinolin-7-yloxymethyl]-morpholine-4-carboxylic acid tert-butyl ester. To a solution of 4-(4-methoxy-phenyl)-2-methyl-1,2,3,4-tetrahydro-isoquinolin-7-ol (680.0 mg, 2.52 mmol) in DCM (25 mL) was added 2-hydroxymethyl-morpholine-4-carboxylic acid tert-butyl ester (564 mg, 2.60 mmol), PPh₃ (690 mg, 2.63 mmol), and DEAD (439 mg, 2.52 mmol). After 3 days, the mixture was concentrated. Purification by FCC gave 550 mg (47%) of product as a mixture of diastereomers. MS (ESI): mass calcd for C₂₇H₃₆N₂O₅, 468.26; m/z found, 469.4 [M+H]⁺.

Step 2; 4-(4-Methoxy-phenyl)-2-methyl-7-(morpholin-2-ylmethoxy)-1,2,3,4-tetrahydro-isoquinoline. Prepared as described in Example 237, Step 7, to give 550 mg (47%) of product as a mixture of diastereomers. MS (ESI): mass calcd for C₂₂H₂₈N₂O₃, 368.21; m/z found, 369.4 [M+H]⁺.

Step 3. Prepared as described for 7-(1-cyclobutyl-azetidin-3-ylmethoxy)-2-methyl-4-(4-methylsulfanyl-phenyl)-1,2,3,4-tetrahydro-isoquinoline to give 14 mg (20%) of product as a mixture of diastereomers. MS (ESI): mass calcd for C₂₃H₃₀N₂O₃, 382.23; m/z found, 383.4 [M+H]⁺.

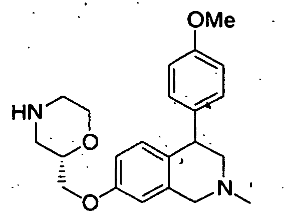

### Example 252; 4-(4-Methoxy-phenyl)-2-methyl-7-(morpholin-25-ylmethoxy)-1,2,3,4-tetrahydro-isoquinoline.

The title compound was prepared using methods similar to those described in Example 251, Steps 1 and 2, using (S)-2-hydroxymethyl-morpholine-4-carboxylic acid tert-butyl ester. Yield: 271.6 mg (82%). MS (ESI): mass calcd for C₂₂H₂₈N₂O₃, 368.21; m/z found, 369.4 [M+H]⁺.

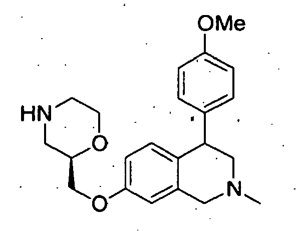

### Example 253; 4-(4-Methoxy-phenyl)-2-methyl-7-(morpholin-2R-ylmethoxy)-1,2,3,4-tetrahydro-isoquinoline.

The title compound was prepared using methods similar to those described in Example 251, .using (R)-2-hydroxymethyl-morpholine-4-carboxylic acid tert-butyl ester. Yield: 302 mg (100%). MS (ESI): mass calcd for C₂₂H₂₈N₂O₃, 368.21; m/z found, 369.3 [M+H]⁺.

The compounds in Examples 254-270 were prepared by a sequence similar to that described in Example 251.

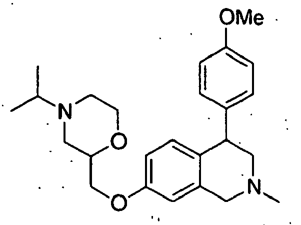

### Example 254; 7-(4-Isopropyl-morpholin-2-ylmethoxy)-4-(4-methoxy-phenyl)-2-methyl-1,2,3,4-tetrahydro-isoquinoline.

Yield: 20 mg (25%). MS (ESI): mass calcd for C₂₅H₃₄N₂O₃, 410.26; m/z found, 411.5 [M+H]⁺.

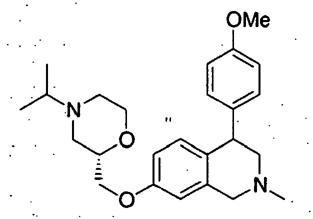

### Example 255; 7-(4-Isopropyl-morpholin-2S-ylmethoxy)-4-(4-methoxy-phenyl)-2-methyl-1,2,3,4-tetrahydro-isoquinoline.

Yield: 30 mg (31 %) as a mixture of diastereomers. MS (ESI): mass calcd for C₂₅H₃₄N₂O₃, 410.26; m/z found, 411.4 [M+H]⁺.

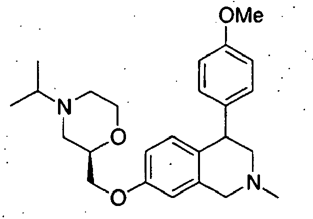

### Examples 256 (mixture), 256A (diastereomer 1), and 256B (diastereomer 2); 7-(4-Isopropyl-morpholin-2R-ylmethoxy)-4-(4-methoxy-phenyl)-2-methyl-1,2,3,4-tetrahydro-isoquinoline.

MS (ESI): mass calcd for C₂₅H₃₄N₂O₃, 410.26; m/z found, 411.4 [M+H]⁺. The diastereomers were separated by SFC HPLC to provide Example 256A (first eluting) and Example 256B (second eluting).

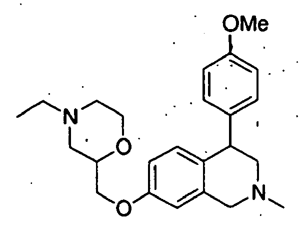

### Example 257; 7-(4-Ethyl-morpholin-2-ylmethoxy)-4-(4-methoxy-phenyl)-2-methyl-1,2,3,4-tetrahydro-isoquinoline.

Yield: 40 mg (52%) as a mixture of diastereomers. MS (ESI): mass calcd for C₂₄H₃₂N₂O₃, 396.24; m/z found, 397.4 [M+H]⁺.

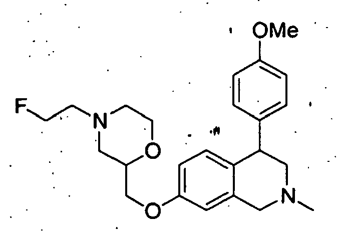

### Example 258; 7-[4-(2-Fluoro-ethyl)-morpholin-2-ylmethoxy]-4-(4-methoxy-phenyl)-2-methyl-1,2,3,4-tetrahydro-isoquinoline.

Yield: 71 mg (60%) as a mixture of diastereomers. MS (ESI): mass calcd for C₂₄H₃₁FN₂O₃, 414.23; m/z found, 415.4 [M+H]⁺.

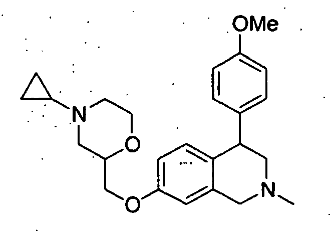

### Example 259; 7-(4-Cyclopropyl-morpholin-2-ylmethoxy)-4-(4-methoxy-phenyl)-2-methyl-1,2,3,4-tetrahydro-isoquinoline.

Yield: 63 mg (53%) as a mixture of diastereomers. MS (ESI): mass calcd for C₂₅H₃₂N₂O₃, 408.24; m/z found, 409.4 [M+H]⁺.

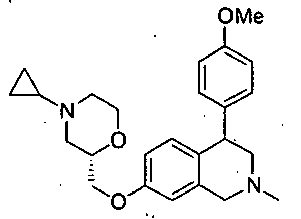

### Examples 260 (mixtures), 260A (diastereomer 1) and 260B (diastereomer 2); 7-(4-Cyclopropyl-morpholin-2S-ylmethoxy)-4-(4-methoxy-phenyl)-2-methyl-1,2,3,4-tetrahydro-isoquinoline.

Example 260. Yield: 65 mg (33%) as a mixture of diastereomers. MS (ESI): mass calcd for C₂₅H₃₂N₂O₃, 408.24; m/z found, 409.4 [M+H]⁺.

The diastereomers were separated (SFC HPLC) to provide Example 260A (first eluting) and Example 260B (second eluting).

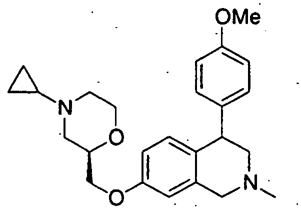

### Examples 261 (mixture), 261 A (diastereomer 1) and 261 B (diastereomer 2); 7-(4-Cyclopropyl-morpholin-2R-ylmethoxy)-4-(4-methoxy-phenyl)-2-methyl-1,2,3,4-tetrahydro-isoquinoline.

Example 261. Yield: 150 mg (68%) as a mixture of diastereomers. MS (ESI): mass calcd for C₂₅H₃₂N₂O₃, 408.24; m/z found, 409.4 [M+H]⁺.

The diastereomers were separated (SFC HPLC) to provide Example 261 A (first eluting) and Example 261B (second eluting).

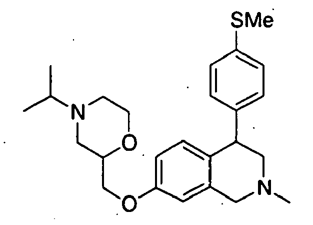

### Example 262; 7-(4-Isopropyl-morpholin-2-ylmethoxy)-2-methyl-4-(4-methylsulfanyl-phenyl)-1,2,3,4-tetrahydro-isoquinoline.

Yield: 83 mg (75%) was a mixtures of diastereomers. MS (ESI): mass calcd for C₂₅H₃₄N₂O₂S, 426.23; m/z found, 427.4 [M+H]⁺.

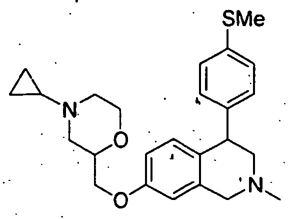

### Example 263; 7-(4-Cyclopropyl-morpholin-2-ylmethoxy)-2-methyl-4-(4-methylsulfanyl-phenyl)-1,2,3,4-tetrahydro-isoquinoline.

Yield: 15 mg (13%) as a mixture of diastereomers. MS (ESI): mass calcd for C₂₅H₃₂N₂O₂S, 424.22; m/z found, 425.3 [M+H]⁺.

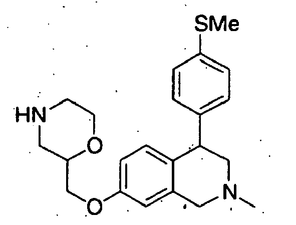

### Example 264; 2-Methyl-4-(4-methylsulfanyl-phenyl)-7-(morpholin-2-ylmethoxy)-1,2,3,4-tetrahydro-isoquinoline.

Yield: 200 mg (84%) as a mixture of diastereomers. MS (ESI): mass calcd for C₂₂H₂₈N₂O₂S, 384.19; m/z found, 385.4 [M+H]⁺.

The compounds in Examples 265-270 were prepared by a sequence similar to that described in Example 1.

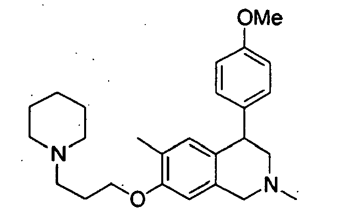

### Example 265; 4-(4-Methoxy-phenyl)-2,6-dimethyl-7-(3-piperidin-1-yl-propoxy)-1,2,3,4-tetrahydro-isoquinoline.

Yield: 40 mg (10%). MS (ESI): mass calcd for C₂₆H₃₆N₂O₂, 408.28; m/z found, 409.5 [M+H]⁺. ¹H NMR (CDCl₃): 7.10 (d, J = 8.7, 2H), 6.83 (d, J = 8.7, 2H), 6.61 (s, 1H), 6.50 (s, 1H), 4.14-4.08 (m, 1H), 3.98 (t, J = 6.2, 2H), 3.79 (s, 3H), 3.66 (d, J = 14.7, 1H), 3.56 (d, J = 14.6, 1H), 2.98-2.91 (m, 1H), 2.52-2.47 (m, 3H), 2.44-2.38 (m, 7H), 2.05 (s, 3H), 2.02-1.95 (m, 2H), 1.61 (quintet, J = 5.7, 4H), 1.48-1.40 (m, 2H).

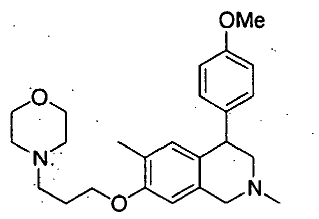

### Example 266; 4-(4-Methoxy-phenyl)-2,6-dimethyl-7-(3-morpholin-4-yl-propoxy)-1,2,3,4-tetrahydro-isoquinoline.

Yield: 200 mg (36%). MS (ESI): mass calcd for C₂₅H₃₄N₂O₃, 410.26; m/z found, 411.5 [M+H]⁺. ¹H NMR (CDCl₃): 7.10 (d, J = 8.7, 2H), 6.83 (d, J = 8.8, 2H), 6.62 (s, 1H), 6.50 (s, 1H), 4.14-4.08 (m, 1H), 4.00 (t, J = 6.2, 2H), 3.80 (s, 3H), 3.75-3.71 (m, 4H), 3.66 (d, J = 14.6, 1H), 3.56 (d, J = 14.6, 1H), 2.98-2.92 (m, 1H), 2.56-2.51 (m, 3H), 2.51-2.45 (m, 4H), 2.40 (s, 3H), 2.05 (s, 3H), 2.021.94 (m, 2H).

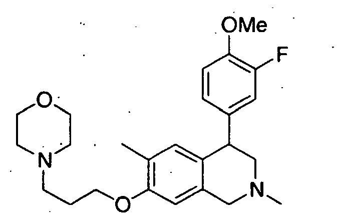

### Example 267; 4-(3-Fluoro-4-methoxy-phenyl)-2,6-dimethyl-7-(3-morpholin-4-yl-propoxy)-1,2,3,4-tetrahydro-isoquinoline.

Yield: 79.9 mg (12%). MS (ESI): mass calcd for C₂₅H₃₃FN₂O₃, 428.25; m/z found, 429.5 [M+H]⁺. ¹H NMR (CDCl₃): 6.94 (m, 3H), 6.62 (s, 1H), 6.50 (s, 1H), 4.10-4.04 (m, 1H), 4.00 (t, J = 6.2, 2H), 3.87 (s, 3H), 3.74 (t, J = 4.7, 4H), 3.60 (br s, 2H), 2.94-2.89 (m, 1H), 2.57-2.50 (m, 3H), 2.50-2.45 (m, 4H), 2.40 (s, 3H), 2.06 (s, 3H), 2.02-1.94 (m, 2H).

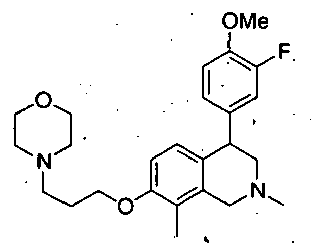

### Example 268; 4-(3-Fluoro-4-methoxy-phenyl)-2,8-dimethyl-7-(3-morpholin-4-yl-propoxy)-1,2,3,4-tetrahydro-isoquinoline.

Yield: 111 mg (3% over 3 steps). MS (ESI): mass calcd for C₂₅H₃₃FN₂O₃, 428.25; m/z found, 429.4 [M+H]⁺. ¹H NMR (CDCl₃): 6.93-6.84 (m, 3H), 6.69-6.62 (m, 2H), 4.16-4.18 (m, 1H), 3.98 (t, J = 6.3, 2H), 3.87 (s, 3H), 3.73 (t, J = 4.6, 4H), 3.64 (d, J = 15.3, 1H), 3.49 (d, J = 15.3, 1H), 2.94-2.88 (m, 1H), 2.57-2.51 (m, 3H), 2.50-2.44 (m, 6H), 2.12 (s, 3H), 2.01-1.93 (m, 2H), 1.37-1.25 (m, 1H).

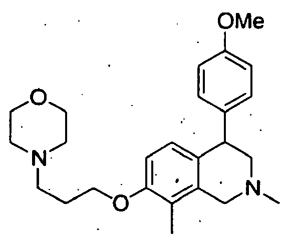

### Example 269; 4-(4-Methoxy-phenyl)-2,8-dimethyl-7-(3-morpholin-4-yl-propoxy)-1,2,3,4-tetrahydro-isoquinoline.

Yield: 8.5 mg (2%). MS (ESI): mass calcd for C₂₅H₃₄N₂O₃, 410.26; m/z found, 411.4 [M+H]⁺. ¹H NMR (CDCl₃): 7.09 (d, J = 8.7, 2H), 6.82 (d, J = 8.7, 2H), 6.66 (q, J = 8.6, 2H), 4.20-4.14 (m, 1H), 3.97 (t, J = 6.2, 2H), 3.79 (s, 3H), 3.74-3.69 (m, 5H), 3.45 (d, J = 15.3, 1H), 2.98-2.92 (m, 1H), 2.57-2.44 (m, 10H), 2.12 (s, 3H), 2.01-1.92 (m, 2H).

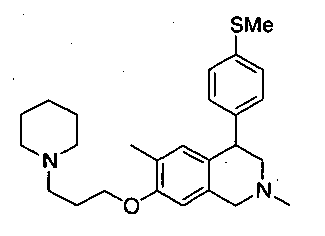

### Example 270; 2,6-Dimethyl-4-(4-methylsulfanyl-phenyl)-7-(3-piperidin-1-yl-propoxy)-1,2,3,4-tetrahydro-isoquinoline.

Yield: 25 mg (8%). MS (ESI): mass calcd for C₂₆H₃₆N₂OS, 424.25; m/z found, 425.5 [M+H]⁺. ¹H NMR (CDCl₃): 7.19 (d, J = 8.4, 2H), 7.11 (d, J = 8.3, 2H), 6.60 (s, 1H), 6.50 (s, 1H), 4.15-4.08 (m, 1H), 3.98 (t, J = 6.2, 2H), 3.61 (q, J = 15.7, 2H), 2.97-2.91 (m; 1H), 2.53-2.36 (m, 13H), 2.05 (s, 3H), 2.04-1.95 (m, 2H), 1.64-1.56 (m, 4H), 1.49-1.40 (m, 2H).

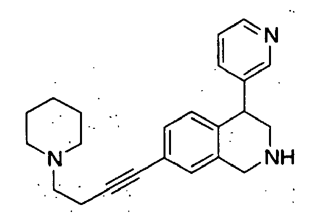

### Example 271; 7-(4-Piperidin-1-yl-but-1-ynyl)-4-pyridin-3-yl-1,2,3,4-tetrahydro-isoquinoline.

Step 1; (4-Bromo-phenyl)-pyridin-3-yl-acetonitrile. A solution of (4-bromo-phenyl)-acetonitrile (1.38g, 7.04 mmol) in THF (30 mL) was treated with NaH (60% dispersion in oil; 0.41 g, 10.7 mmol). The mixture was heated at 60 °C for 20 min, and then was treated with 3-fluoropyridine (0.44 mL, 4.99 mmol) and heated at 65 °C for 16 h. The mixture was cooled, concentrated, diluted with satd. aq. NaHCO₃, and extracted with EtOAc. The combined organic layers were concentrated and the residue was purified by FCC (EtOAc/hexanes) to give 0.53 g (39%). MS (ESI): mass calcd. for C₁₃H₉BrN₂, 271.99; m/z found, 273.3, 275.3 [M+H]⁺. ¹H NMR (CDCl₃): 8.63-8.61 (m, 2H), 7.68-7.65 (m, 1H), 7.54 (d, J = 8.6, 2H), 7.23 (d, J = 8.3, 2H), 5.14 (s, 1H).

Step 2; 2-(4-Bromo-phenyl)-2-pyridin-3-yl-ethylamine. A solution of (4-bromophenyl)-pyridin-3-yl-acetonitrile (0.50 g, 1.83 mmol) in THF (5 mL) was treated with a solution of NaBH₄ (0.166 g, 4.38 mmol) in THF (10 mL). The mixture was cooled to 0 °C and a solution of I₂ (0.78 g) in THF (5 mL) was added. After 3 h at 65 °C, the mixture was cooled to rt and MeOH (5 mL) was added dropwise. After 1 h, the mixture was diluted with 20% KOH and extracted with DCM. The organic layer was collected, dried (Na₂SO₄), and concentrated. The residue was treated with HCl in MeOH, and the resulting precipitate was washed with Et₂O. The precipitate was purified by FCC to give the title compound (0.191 g, 38%). MS (ESI): mass calcd. for C₁₃H₁₃BrN₂, 277.16; m/z found, 277.3, 279.3 [M+H]⁺. ¹H NMR (CDCl₃): 8.53 (d, 2.3, 1H), 8.49 (dd, J = 4.8, 1.7, 1H), 7.52-7.49 (m, 1H), 7.46 (d, J = 8.5, 2H), 7.26-7.23 (m, 1H), 7.13 (d, J = 8.3, 2H), 3.98 (t, J = 7.6, 1H), 3.33 (dd, J = 7.5, 1.4, 2H).

Step 3; [2-(4-Bromo-phenyl)-2-pyridin-3-yl-ethyl]-carbamic acid methyl ester. A solution of 2-(4-bromo-phenyl)-2-pyridin-3-yl-ethylamine (0.178 g, 0.642 mmol) and TEA (0.279 mL, 1.94 mmol) in DCM (2 mL) was treated with methyl chloroformate (0.060 mL, 0.777 mmol). After 2 h, the mixture was diluted with water and extracted with DCM. The organic layer was collected, dried (Na₂SO₄), and concentrated. Purification by FCC gave the title compound (0.148 g, 69%). MS (ESI): mass calcd. for C₁₅H₁₅BrN₂O₂, 335.20; m/z found, 337.30, 335.30 [M+H]⁺. ¹H NMR (CDCl₃): 8.47 (d, J = 2.3, 1H), 8.45 (dd, J = 4.9, 1.6, 2H), 7.53-7.49 (m, 1H), 7.48 (d, J = 8.6, 2H), 7.25-7.21 (m, 1H), 7.10 (d, J = 8.4, 2H), 5.20 (m, 1H), 4.21 (m, 1H), 3.62 (s, 3H).

Step 4; 7-Bromo-4-pyridin-3-yl-1,2,3,4-tetrahydro-isoquinoline. A mixture of [2-(4-bromo-phenyl)-2-pyridin-3-yl-ethyl]-carbamic acid methyl ester (0.148 g, 0.441 mmol) paraformaldehyde (0.040 mg), and conc. HCl (2 mL) was stirred at rt for 22 h, then at 35 °C for 4 h. The mixture was cooled to rt, neutralized with satd. aq. NaHCO₃, and extracted with DCM. The organic layer was collected, dried (Na₂SO4), and concentrated. Purification by FCC gave 7-bromo-4-pyridin-3-yl-3,4-dihydro-1H-isoquinoline-2-carboxylic acid methyl ester (0.150 g) which was diluted with 6 N HCl and heated at 80 °C for 14 h and then at 105 °C for 4 days. The mixture was cooled to rt, neutralized with 20% KOH, diluted with satd. aq. NaHCO₃, and extracted with DCM. The organic layer was collected, dried (Na₂SO₄), and concentrated. Purification of the residue by FCC gave an oil which was diluted with DCM and treated with HCl in Et₂O. Trituration with hexanes and concentration gave the title compound as the HCl salt (0.845 g, 61%). MS (ESI): mass calcd. for C₁₄H₁₃BrN₂, 289.17; m/z found, 291.3, 289.3 [M+H]⁺. ¹H NMR (CDCl₃): 8.49 (dd, J = 4.8, 1.8, 1H), 8.44 (d, J = 1.9, 1H), 7.39-7.32 (m, 1H), 7.29-7.19 (m, 3H), 6.74 (d, J = 8.2, 1H), 4.19-4.01 (m, 3H), 3.30 (dd, J = 12.9, 8.6, 1H), 3.05 (dd, J = 12.9, 6.2, 1H).

Step 5. A solution of 7-bromo-4-pyridin-3-yl-1,2,3,4-tetrahydro-isoquinoline hydrochloride salt (0.070 g, 0.214 mmol) and Et₂NH (1 mL) in DMF (0.3 mL) was treated with Ph₃P (0.011 g, 0.042 mmol), CuI (0.0055 g, 0.029 mmol), 1-but-3-ynyl-piperidine (0.060 mL), and dichlorobis(triphenylphosphine)palladium (II) (0.0077 g, 0.011 mmol). The mixture was heated at 128 °C for 1 h, cooled to rt, diluted with Et₂O, and filtered. The filtrate was concentrated and' purified by FCC to give the title compound (0.0487 g, 66%). MS (ESI): mass calcd. for C₂₃H₂₇N₃, 345.48; m/z found, 346.50 [M+H]⁺. ¹H NMR (CDCl₃): 8.48 (dd, J = 4.8, 1.7, 1H), 8.44 (d, J = 1.8, 1H), 7.37-7.33 (m, 1H), 7.22-7.18 (m, 1H), 7.16-7.12 (m, 2H), 6.77 (d, J = 8.1, 1H), 4.15-4.00 (m, 3H), 3.40 (dd, J = 12.7, 5.3, 1H), 3.05 (dd, J = 12.8, 6.8, 1H), 2.70-2.35 (m, 8H); 1.62-1.40 (m, 6H). Treatment with 2 N HCl in Et₂O and concentration from DCM/hexanes gave the HCl salt.

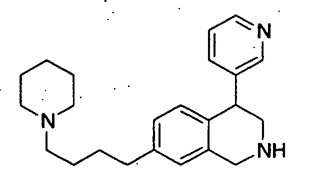

### Example 272; 7-(4-Piperidin-1-yl-butyl)-4-pyridin-3-yl-1,2,3,4-tetrahydro-isoquinoline.

A solution of 7-(4-piperidin-1-yl-but-1-ynyl)-4-pyridin-3-yl-1,2,3,4-tetrahydro-isoquinoline dihydrochloride (0.030 g, 0.072 mmol) in MeOH (2 mL) was treated with Pd/BaSO₄ (∼20 mg) and stirred under H₂ (1 atm) for 1 h. 5% Pd/C (∼25 mg) was added and the mixture was stirred under H₂ (1 atm) for an additional 1 h. The mixture was filtered and concentrated. The residue was diluted with 2 N HCl in Et₂O and concentrated to give the title compound (0.025 g, 83%) as an HCl salt. MS (ESI): mass calcd. for C₂₃H₃₁N₃, 349.51; m/z found, 350.50 [M+H]⁺. ¹H NMR (MeOD): 8.71 (m, 2H), 8.11 (d, J = 8.0, 1h), 7.78 (m, 1H), 7.23 (s, 1H), 7.16 (dd, J = 8.3, 1.2, 1H), 6.82 (d, J = 8.0, 1H), 4.78 (dd, J = 10.3, 6.5, 1H), 4.61 (d, J = 15.9, 1H), 4.47 (d, J = 15.8, 1H), 3.86 (dd, J = 12.3, 6.2, 1H), 3.60-3.50 (m, 4H), 3.15-3.05 (m, 2H), 2.95-2.85 (m, 2H), 2.00-1.40 (m, 11H).

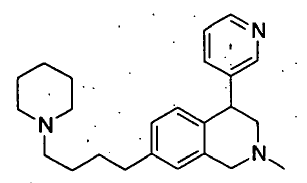

### Example 273; 2-Methyl-7-(4-piperidin-1-yl-butyl)-4-pyridin-3-yl-1,2,3,4-tetrahydro-isoquinoline.

7-(4-Piperidin-1-yl-butyl)-4-pyridin-3-yl-1,2,3,4-tetrahydro-isoquinoline dihydrochloride (0.020 mg, 0.047 mmol) was diluted with satd. aq. NaHCO₃ and the free base was extracted with DCM. The organic layer was collected, dried (Na₂SO₄), and concentrated. The resulting oil was diluted with MeOH (3 mL) and treated with paraformaldehyde (0.044 g). The mixture was heated at reflux for 45 min, cooled to rt and treated with Pd(OH)₂ (∼20 mg). After 3 days under H₂ (1 atm), the mixture was filtered and concentrated. Preparative TLC (2 M NH₃ in MeOH/DCM) gave the title compound (0.008 g, 47%). MS (ESI): mass calcd. for C₂₄H₃₃N₃, 363.54; m/z found, 364.55 [M+H]⁺. ¹H NMR (CDCl₃): 8.52 (d, J = 1.9, 1H), 8.47 (dd, J = 4.9, 1.6, 1H), 7.48 (ddd, J = 7.8, 2.0, 2.0, 1H), 7.19 (ddd, J = 8.0, 4.9, 0.8, 1H), 6.92-6.80 (m, 2H), 6.74 (d, J = 8.4, 1H), 4.24-4.19 (m, 1H), 3.65 (s, 2H), 2.96 (dd, J = 11.5, 5.5, 1H), 2.63-2.54 (m, 3H), 2.41 (s, 3H), 2.39-2.26 (m, 6H), 1.63-1.38 (m, 10H)._{.}

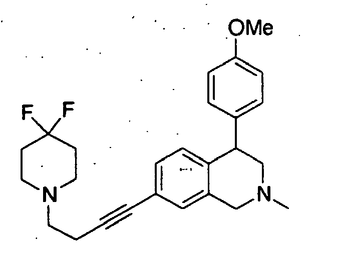

### Example 274; 7-[4-(4,4-Difluoro-piperidin-1-yl)-but-1-ynyl]-4-(4-methoxy-phenyl)-2-methyl-1,2,3,4-tetrahydro-isoquinoline.

Step 1; (3-Bromo-benzyl)-methyl-amine. The title compound was prepared using a method analogous to that described for 3-(3-methylaminomethyl-phenoxy)-propan-1-ol.

Step 2; 2-[(3-Bromo-benzyl)-methyl-amino]-1-(4-methoxy-phenyl)-ethanone. The title compound was prepared using a method analogous to that described for 2-{[3-(3-hydroxy-propoxy)-benzyl]-methyl-amino}-1-(4-methoxy-phenyl)-ethanone.

Step 3; 2-[(3-Bromo-benzyl)-methyl-amino]-1-(4-methoxy-phenyl)-ethanol. A 0 ºC solution of 2-[(3-bromo-benzyl)-methyl-amino]-1-(4-methoxy-phenyl)-ethanone (4.5 g, 13 mmol) in MeOH (65 mL) was treated with NaBH₄ (1.0 g, 27 mmol) in 4 portions. The mixture was allowed to warm to rt. After 16 h, the mixture was diluted with water and extracted with DCM (4x). The combined organic layers were washed with brine, dried (Na₂SO₄), and concentrated to give the crude product (4 g).

Step 4. 7-Bromo-4-(4-methoxy-phenyl)-2-methyl-1,2,3,4-tetrahydro-isoquinoline. A 0 ºC solution of 2-[(3-bromo-benzyl)-methyl-amino]-1-(4-methoxy-phenyl)-ethanol in DCM (230 mL) was treated with MSA (7.5 mL). After 5 min, the mixture was basified with 2 N NaOH, diluted with water, and extracted with DCM (3 x 100 mL). The combined organic layers were washed with brine, dried (Na₂SO₄), and concentrated to give an oil, which was purified by FCC (EtOAc/hexanes) and reverse phase HPLC to give the desired product (1.1 g, 29%). MS (ESI): mass calcd. for C₁₇H₁₈BrNO, 331.06; m/z found, 332.3 [M+H]⁺. ¹H NMR (MeOD): 7.48 (d, J = 1.8, 1H), 7.38 (dd, J = 8.3, 1.8, 1H), 7.17 (d, J = 8.8, 2H), 6.93 (d, J = 8.8, 2H), 6.83 (br d, J = 8.3, 1H), 4.57-4.56, m, 2H), 4.49 (dd, J = 11.4, 6.0, 1H), 3.82-3.78 (m, 1H), 3.79 (s, 3H), 3.56-3.42 (m, 1H), 3.06 (s, 3H).

Step 5; 4-[4-(4-Methoxy-phenyl)-2-methyl-1, 2,3,4-tetrahydro-isoquinolin-7-yl]-but-3-yn-1-ol. A mixture of 7-bromo-4-(4-methoxy-phenyl)-2-methyl-1,2,3,4-tetrahydro-isoquinoline (95.0 mg, 0.29 mmol), but-3-yn-1-ol (40 mg, 0.57 mmol), Ph₃P (13.4 mg, 0.05 mmol), CuI (2.7 mg, 0.10 mmol), and bis(triphenyl-phosphine)palladium(II) dichloride (10 mg, 0.01 mmol) in a high pressure reaction vessel was treated with DMF (0.5 mL) and Et₂NH (2.5 mL). The mixture was heated at 125 °C for 30 min, then was cooled to rt, diluted with Et₂O, and filtered through a pad of diatomaceous earth, washing with Et₂O. The filtrate was washed with water (2x), satd. aq. NaHCO₃, and brine, dried (Na₂SO₄), and concentrated to give a brown residue, which was purified by FCC (EtOAc/hexanes) to give the desired product (39 mg, 42%). MS (ESI): mass calcd. for C₂₁H₂₃NO₂, 321.17; m/z found, 322.5 [M+H]⁺. ¹H NMR (CDCl₃): 7.21-7.08 m, 4H, 6.91-6.81 (m, 3H), 4.28-4.20 (m, 1H), 3.86-3.80 (m, 5H), 3.76 (d, J = 14.5 Hz, 1H), 3.62 (d, J = 14.9 Hz, 1H), 3.09-3.01 (m, 1H), 2.70 (t, J = 6.3, 2H), 2.60-2.52 (m, 1H), 2.47 (s, 3H); 2.17-1.98 (m, 1H).

Step 6; Methanesulfonic acid 4-[4-(4-methoxy-phenyl)-2-methyl-1,2,3,4-tetrahydro-isoquinolin-7-yl]-but-3-ynyl ester. A solution of 4-[4-(4-methoxy-phenyl)-2-methyl-1,2,3,4-tetrahydro-isoquinolin-7-yl]-but-3-yn-1-ol (39 mg, 0.12 mmol) and TEA (19 µL, 13 mmol) in DCM (1.2 mL) was treated with MsCl (10 µL, 0.13 mmol). After 3 days, MsCl (1.1 equiv.) was added. After 1 h, the mixture was diluted with brine and extracted with DCM. The organic was dried (Na₂SO₄), and concentrated. The residue was purified by FCC (EtOAc/hexanes) to give the desired product (42 mg, 87%). MS (ESI): mass calcd. for C₂₂H₂₅NO₄S, 399.15; m/z found, 400.4 [M+H]⁺. ¹H NMR (CDCl₃): 7.08-7.05 (m, 1H), 7.04-6.98 (m, 3H), 6.78-6.72 (m, 3H), 4.30 (t, J = 6.8 Hz, 2H), 4.15-4.09 (m, 1H), 3.71 (s, 3H), 3.65 (d, J = 14.9 Hz, 1H), 3.48 (d, J = 15.1 Hz, 1H), 2.99 (s, 3H), 2.96-2.90 (m, 1H), 2.80 (t, J = 6.9 Hz, 2H), 2.48-2.40 (m, 1H), 2.35 (s, 3H).

Step 7. A solution of methanesulfonic acid 4-[4-(methoxy-phenyl)-2-methyl-1,2,3,4-tetrahydro-isoquinolin-7-yl]-but-3-ynyl ester (42 mg, 0.11 mmol) and TEA (36.7 µL) in EtOH (2 mL) was treated with 4,4-difluoropiperidine hydrochloride (25 mg, 0.16 mmol) was added. After 18 h, the mixture was heated to 50 °C. After 2 h, the mixture was heated to 75 °C. After 2 days, the mixture was cooled to rt, diluted with water, and extracted with DCM. The organic layer was washed with brine, dried (Na₂SO₄), and concentrated. The residue was purified by reverse phase HPLC to give the desired product (13 mg, 29%) as a TFA salt. MS (ESI): mass calcd. for C₂₆H₃₀N₂F₂O, 424.23; m/z found, 425.5 [M+H]⁺. ¹H NMR (CDCl₃): 7.15-7.07 (m, 2H), 7.03-6.93 (m, 2H), 6.85-6.72 (m, 3H), 4.69-4.43 (br m, 2H), 4.08-3.91 (br m, 1H), 3.73 (s, 3H), 3.20 (t, J = 6.6, 3H), 2.92-2.77 (m, 10H), 2.44-2.14 (br m, 4H).

The compounds in Examples 275-279 were prepared using methods analogous to those described for Example 274.

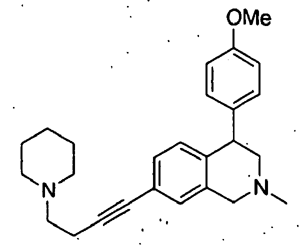

### Example 275; 4-(4-Methoxy-phenyl)-2-methyl-7-(4-piperidin-1-yl-but-1-ynyl)-1,2,3,4-tetrahydro-isoquinoline.

MS (ESI): mass calcd. for C₂₆H₃₂N₂O, 388.3; m/z found, 389.5 [M+H]⁺. ¹H NMR (MeOD): 7.35-7.27 (m, 2H), 7.18-7.14 (m, 2H), 6.96-6.88 (m, 3H), 4.63-4.45 (m, 3H), 3.86-3.76 (m, 1H), 3.79 (s, 3H), 3.66-3.58 (m, 2H), 3.55-3.41 (m, 1H), 3.37 (dd, J = 7.3, 7.1, 3H), 3.06 (s, 3H), 3.05-2.98 (m, 2H), 2.96 (dd, J = 7.3, 7.3, 2H), 2.02-1.89 (m, 2H), 1.80-1.71 (m, 3H), 1.60-1.44 (m, 1H).

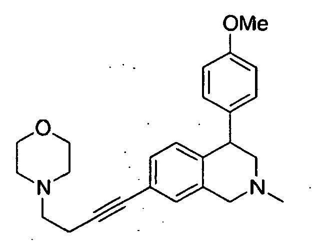

### Example 276; 4-(4-Methoxy-phenyl)-2-methyl-7-(4-morpholin-4-yl-but-1-ynyl)-1,2,3,4-tetrahydro-isoquinoline.

MS (ESI): mass calcd. for C₂₅H₃₀N₂O₂, 390.2; m/z found, 391.5 [M+H]⁺. ¹H NMR (MeOD): 7.34-7.33 (m, 1H), 7.30-7.28 (m, 1H), 7.16-7.14 (m, 2H), 6.96-6.92 (m, 2H), 6.89-6.87 (m, 1H), 4.63-4.46 (m, 3H), 4.16-3.93 (m, 2H), 3.88-3.72 (m, 3H), 3.79 (s, 3H), 3.69-3.49 (m, 2H), 3.44 (dd, J = 7.3, 7.1, 3H), 3.29-3.16 (m, 2H), 3.06 (s, 3H), 2.99 (dd, J = 7.3, 7.2, 2H).

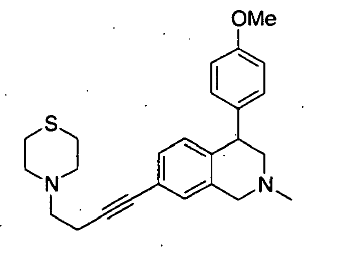

### Example 277; 4-(4-Methoxy-phenyl)-2-methyl-7-(4-thiomorpholin-4-yl-but-1-ynyl)-1,2,3,4-tetrahydro-isoquinoline.

MS (ESI): mass calcd. for C₂₅H₃₀N₂OS, 406.2; m/z found, 407.5 [M+H]⁺. ¹H NMR (MeOD): 7.36-7.22 (m, 2H), 7.19-7.08 (m, 2H), 6.96-6.81 (m, 3H), 4.62-4.43 (m, 3H), 3.86-3.72 (m, 5H), 3.61-3.36 (m, 1H), 3.33-3.23 (m, 5H), 3.11-2.85 (m, 9H).

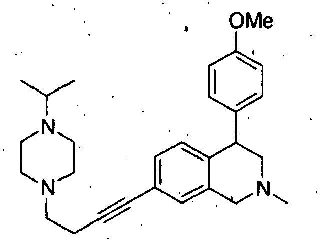

### Example 278; 7-[4-(4-Isopropyl-piperazin-1-yl)-but-1-ynyl]-4-(4-methoxy-phehyl)-2-methyl-1,2,3,4-tetrahydro-isoquinoline.

MS (ESI): mass calcd. for C₂₈H₃₇N₃O, 431.3; m/z found, 432.5 [M+H]⁺. ¹H NMR (MeOD): 7.32-7.21 (m, 2H), 7.19-7.11 (m, 2H), 6.97-6.80 (m, 3H), 4.61-4.44 (m, 3H), 3.85-3.76 (m, 1H), 3.79 (s, 3H), 3.58-3.34 (m, 6H), 3.33-3.27 (m, 2H), 3.10-3.02 (m, 1H), 3.06 (s, 3H), 3.00-2.89 (m, 3H), 2.79-2.70 (m, 2H), 1.36 (d, J = 6.6, 6H).

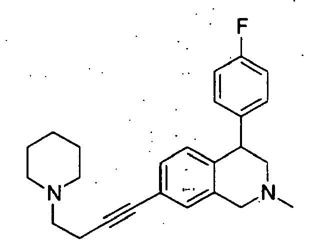

### Example 279; 4-(4-Fluoro-phenyl)-2-methyl-7-(4-piperidin-1-yl-but-1-ynyl)-1,2,3,4-tetrahydro-isoquinoline.

MS (ESI): mass calcd. for C₂₅H₂₉FN₂, 376.2; m/z found, 377.5 [M+H]⁺. ¹H NMR (MeOD): 7.37-7.33 (m, 1H), 7.32-7.23 (m, 3H), 7.13 (dd, J = 8.8, 8.6, 2H), 6.93-6.83 (m, 1H), 4.66-4.49 (m, 3H), 3.84 (dd, J = 11.4, 5.5, 1H), 3.66-3.47 (m, 2H), 3.37 (dd, J = 7.3, 7.3, 2H), 3.07 (s, 3H), 3.04-3.00 (m, 2H), 2.96 (dd, J = 7.3, 7.3, 2H), 2.03-1.91 (m, 2H), 1.90-1.70 (m, 4H), 1.59-1.46 (m, 1H)

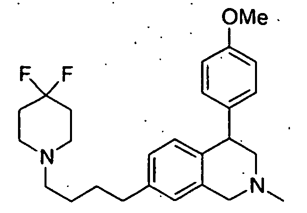

### Example 280; 7-[4-(4,4-Difluoro-piperidin-1-yl)-butyl]-4-(4-methoxy-phenyl)-2-methyl-1,2,3,4-tetrahydro-isoquinoline.

A solution of 7-[4-(4,4-difluoro-piperidin-1-yl)-but-1-ynyl]-4-(4-methoxy-phenyl)-2-methyl-1,2,3,4-tetrahydro-isoquinoline (11 mg, 0.02 mmol) in MeOH (1.0 mL) and EtOH (0.5 mL) was treated with Pd/BaSO₄ (10 mg). The system was filled with N₂, evacuated, and back-filled with H₂ (2x). After 2 h under H₂ (1 atm), the mixture was filtered through a pad of diatomaceous earth, washing with MeOH. The filtrate was concentrated, and the residue was purified by FCC to give the title compound (3.1 mg, 43%). MS (ESI): mass calcd. for C₂₆H₃₄F₂N₂O, 428.26; m/z found, 429.5 [M+H]⁺. ¹H NMR (CDCl₃): 7.04 (d, J = 8.8, 2H), 6.86-6.79 (m, 2H), 6.77 (d, J = 8.8, 2H), 6.71 (d, J = 7.6, 1H), 4.24-4.16 (m, 1H), 3.76-3.70 (m, 4H), 3.65-3.55 (m, 1H), 3.07-2.96 (m, 1H), 2.59-2.35 (m, 9H), 2.38-2.25 (m, 2H), 2.03-1.83 (m, 4H), 1.62-1.35 (m, 6H).

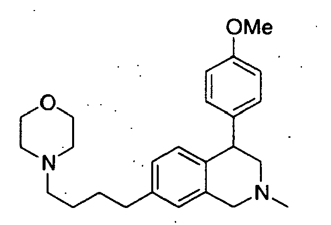

### Example 281; 4-(4-Methoxy-phenyl)-2-methyl-7-(4-morpholin-4-yl-butyl)-1,2,3,4-tetrahydro-isoquinoline.

MS (ESI): mass calcd. for C₂₅H₃₄N₂O₂, 394.3; m/z found, 395.5 [M+H]⁺. ¹H NMR (MeOD): 7.14 (d, J = 8.8, 2H), 7.12-7.09 (m, 2H), 6.93 (d, J = 8.8, 2H), 6.87, (br d, J = 8.3, 1H), 4.50-4.46 (m, 3H), 3.88-3.82 (m, 4H), 3.79 (s, 3H), 3.78-3.73 (m, 1H), 3.44 (dd, J = 11.9, 11.8, 2H), 3.28-3.16 (m, 3H), 3.11 (dd, J = 7.6, 5.6, 2H), 3.03 (s, 3H), 2.67 (dd, J = 7.6, 7.3, 2H), 1.82-1.62 (m, 4H).

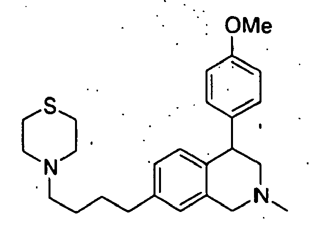

### Example 282; 4-(4-Methoxy-phenyl)-2-methyl-7-(4-thiomorpholin-4-yl-butyl)-1,2,3,4-tetrahydro-isoquinoline.

MS (ESI): mass calcd. for C₂₅H₃₄N₂OS, 410.2; m/z found, 411.5 [M+H]⁺. ¹H NMR (MeOD): 7.11 (d, J = 8.8, 2H), 7.07-7.01 (m, 2H), 6.91 (d, J = 8.8, 2H), 6.78 (d, J 8.6, 1H), 4.45-4.15 (m, 3H), 3.78 (s, 3H), 3.61-3.50 (m, 1H), 3.27-3.10 (m, 4H), 2.94-2.80 (m, 5H), 2.86 (s, 3H), 2.68-2.60 (m, 2H), 1.75-1.57 (m, 4H), 1.33-1.23 (m, 2H).

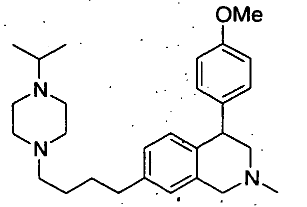

### Example 283; 7-[4-(4-Isopropyl-piperazin-1-yl)-butyl]-4-(4-methoxy-phenyl)-2-methyl-1,2,3,4-tetrahydro-isoquinoline.

MS (ESI): mass calcd. for C₂₈H₄₁N₃O, 435.3; m/z found, 436.6 [M+H]⁺. ¹H NMR (MeOD): 7.13 (d, J = 8.8, 2H), 7.06-7.04 (m, 2H), 6.91 (d, J = 8.8, 2H), 6.80 (br d, J = 8.8, 1H), 4.45-4.38 (m, 1H), 4.37-4.28 (m, 2H), 3.79 (s, 3H), 3.62 (dd, J = 12.1, 6.3, 1H), 3.31-3.21 (m, 2H), 3.19-2.95 (m, 4H), 2.94-2.87 (m, 1H), 2.92 (s, 3H), 2.63 (dd, J = 7.6, 7.0, 5H), 1.70-1.53 (m, 4H), 1.28-1.26 (m, 2H), 1.27 (d, J = 6.6, 6H).

### Biological Methods

### H₃ receptor binding

Binding of compounds to the cloned human H₃ receptor, stably expressed in SK-N-MC cells, was performed (Lovenberg, T.W. et al. J. Pharmacol. Exp. Ther. 2000, 293, 771-778). Briefly, cell pellets from SK-N-MC cells expressing the human H₃ receptor were homogenized in 50 mM Tris-HCl/5 mM EDTA and recentrifuged at 30,000 g for 30 min. Pellets were re-homogenized in 50 mM Tris/5 mM EDTA (pH 7.4). Membranes were incubated with 0.8 nM N-[³H]-α-methylhistamine plus/minus test compounds for 60 min at 25 °C and harvested by rapid filtration over GF/C glass fiber filters (pretreated with 0.3% polyethylenimine) followed by four washes with ice-cold buffer. Nonspecific binding was defined in the presence of 10 µM histamine. IC₅₀ values were determined by a single site curve-fitting program (GraphPad, San Diego, CA) and converted to Kᵢ values based on a N-[³H]-α-methylhistamine K_{d} of 800 pM and a ligand concentration of 800 pM (Cheng & Prusoff, Biochem. Pharmacol. 1973, 22,, 3099-3108). Data are presented in Table 1.

### Rat brain SERT

A rat brain without cerebellum (Zivic Laboratories, Inc.-Pittsburgh, PA) was homogenized in a 52.6 mM Tris pH 8/126.4 mM NaCl/5.26 mM KCl mixture and centrifuged at 1,000 rpm for 5 min. The supernatant was removed and recentrifuged at 15,000 rpm for 30 min. Pellets were re-homogenized in a 52.6 mM Tris pH8/126.4 mM NaCl/5.26 mM KCl mixture. Membranes were incubated with 0.6 nM [³H]-Citalopram plus/minus test compounds for 60 min at 25 °C and harvested by rapid filtration over GF/C glass fiber filters (pretreated with 0.3% polyethylenimine) followed by four washes with ice-cold buffer. Nonspecific binding was defined in the presence of 100 µM fluoxetine. IC₅₀ values were determined by a single site curve-fitting program (GraphPad, San Diego, CA) and converted to Kᵢ values based on a [³H]-Citalopram K_{d} of 0.6 nM and a ligand concentration of 0.6 nM. Data are presented in Table 1.

**Table 1.**

| **EX** | **Rat SERT** | **Human H₃** | | **EX** | **Rat SERT** | **Human H₃** |
|---|---|---|---|---|---|---|
| | **Kᵢ (nM)** | **Kᵢ (nM)** | | | **Kᵢ (nM)** | **Kᵢ (nM)** |
| **1** | 2 | 2 | | **133** | 50 | 2 |
| **1A** | 6 | 2 | | **134** | 28 | 2 |
| **1B** | 2 | 2 | | **135** | 27 | 2 |
| **2** | 13 | 70 | | **136** | 9 | 4 |
| **3** | 3 | 0.7 | | **137** | 77 | 5000 |
| **4** | 33 | 4000 | | **138** | 4 | 11 |
| **5** | 27 | 594 | | **139** | 6 | 3 |
| **6** | 15 | 534 | | **140** | 28 | 8 |
| **7** | 14 | 315 | | **141** | 3 | 3 |
| **8** | 6 | 4 | | **142** | 4 | 3 |
| **9** | 5 | 3 | | **143** | 18 | 4 |
| **10** | 4 | 1 | | **144** | 5 | 6 |
| **11** | 2 | 0.6 | | **145** | 3 | 5 |
| **12** | 6 | 59 | | **146** | 12 | 3 |
| **13** | 10 | 2400 | | **147** | 18 | 9 |
| **14** | 19 | 2000 | | **148** | 3 | 262 |
| **15** | 11 | 499 | | **149** | 4 | 240 |
| **16** | 6 | 25 | | **150** | 7 | 175 |
| **17** | 10 | 273 | | **151** | 3 | 119 |
| **18** | 1 | 74 | | **152** | 6 | 5 |
| **19** | 8 | 2000 | | **153** | 2 | 8 |
| **20** | 4 | 2 | | **154** | 5 | 13 |
| **21** | 2 | 9 | | **155** | 6 | 9 |
| **22** | 3 | 11 | | **156** | 4 | 6 |
| **23** | 2 | 2 | | **157** | 3 | 10 |
| **24** | 2 | 2 | | **158** | 2 | 11 |
| **25** | 10 | 7 | | **165A** | 91 | 2 |
| **26** | 2 | 12 | | **172** | 14 | 89 |
| **27** | 3 | 104 | | **173** | 18 | 15 |
| **28** | 12 | 140 | | **178** | 6 | 18 |
| **29** | 14 | 7 | | **179** | 3 | 5 |
| **29A** | NT | 35 | | **182** | 2 | 59 |
| **29B** | 8 | 13 | | **183A** | 1 | 6 |
| **30** | 2 | 3 | | **184** | 1 | 2 |
| **31** | 2 | 2 | | **186** | 10 | 6 |
| **32** | 8 | 21 | | **187** | 3 | 5 |
| **33** | 5 | 172 | | **188** | 37 | 22 |
| **34** | 11 | 3 | | **189** | 108 | 12 |
| **35** | 2 | 1 | | **190** | 119 | 29 |
| **36** | 2 | 7 | | **191** | 69 | 26 |
| **37** | 8 | 9 | | **192** | 9 | 3 |
| **37A** | 9 | 89 | | **193** | 5 | 3 |
| **37B** | 4 | 6 | | **194** | 7 | 1 |
| **38** | 29 | 38 | | **195** | 8 | 3 |
| **39** | 3 | 3 | | **196** | 2000 | 4 |
| **40** | 9 | 20 | | **197** | 5 | 15 |
| **41** | 6 | 14 | | **198** | 2 | 10 |
| **42** | 3 | 3 | | **199** | 2 | 8 |
| **43** | | 18 | | **200** | 3 | 227 |
| **44** | 4 | 2 | | **201** | 4 | 134 |
| **45A** | 9 | 2 | | **202** | 3 | 43 |
| **45B** | 5 | 3 | | **203** | 8 | 504 |
| **46** | 14 | 3 | | **204** | 10 | 2 |
| **47** | 27 | 10 | | **205** | 44 | 2000 |
| **48** | 31 | 6 | | **206** | 3 | 37 |
| **49** | 14 | 18 | | **207** | 15 | 78 |
| **50** | 3 | 8 | | **208** | 4 | 137 |
| **51** | 34 | 2 | | **209** | 12 | 176 |
| **52** | 3 | 6 | | **210** | 10 | 99 |
| **53** | 5 | 7 | | **211** | 27 | 823 |
| **54** | 32 | 16 | | **212** | 9 | 43 |
| **55** | 209 | 8 | | **213** | 6 | 11 |
| **56** | 42 | 20 | | **214** | 3 | 17 |
| **57** | 22 | 0.9 | | **215** | 1 | 5 |
| **58** | 8 | 9 | | **216** | 4 | 22 |
| **59** | 48 | 1 | | **217** | 58 | 42 |
| **60** | 14 | 2 | | **218** | 36 | 38 |
| **61** | 4 | 2 | | **219** | 39 | 33 |
| **62** | 10 | 0.6 | | **220** | 4 | 6 |
| **63** | 1 | 0.8 | | **221** | 11 | 9 |
| **64** | 222 | 1 | | **222** | 30 | 30 |
| **65** | 3000 | 7 | | **223** | 5 | 59 |
| **66** | 5 | 1 | | **224** | 32 | 61 |
| **67** | 104 | 0.5 | | **225** | 1 | 46 |
| **68** | 22 | 1 | | **226** | 1 | 107 |
| **69** | 9 | 1 | | **227** | 1 | 52 |
| **70** | 49 | 1 | | **228** | 1 | 38 |
| **71** | 16 | 1 | | **229** | 2 | 18 |
| **72** | 15 | 1 | | **230** | 1 | 17 |
| **73** | 83 | 2 | | **231** | 2 | 61 |
| **74** | NT | NT | | **232** | 1 | 20 |
| **75** | 79 | 14 | | **233** | 3 | 4 |
| **75A** | 2000 | 7 | | **234** | 3 | 3 |
| **76** | 11 | 5 | | **235** | 7 | 6 |
| **77** | 27 | 3 | | **236** | 6 | 10 |
| **78** | 2 | 96 | | **237** | 3 | 5 |
| **79** | 5 | 183 | | **238** | 4 | 5 |
| **80** | 3 | 130 | | **239** | 2 | 30 |
| **81** | 1 | 70 | | **240** | 3 | 4 |
| **82** | 6 | 64 | | **240A** | 2 | 3 |
| **83** | 6 | 3 | | **240B** | 5 | 21 |
| **84** | 7 | 3 | | **241** | NT | 39 |
| **85** | 7 | 8 | | **242** | 6 | 124 |
| **86** | 5 | 11 | | **243** | 5 | 22 |
| **87** | 4 | 2 | | **244** | 7 | 129 |
| **88** | 15 | 3 | | **245** | 7 | 336 |
| **89** | 5 | 2 | | **246** | 10 | 3 |
| **90** | 9 | NT | | **247** | 5 | 2 |
| **91** | 11 | 1 | | **248** | 7 | 5 |
| **92** | 12 | 2 | | **249** | 6 | 4 |
| **93** | 22 | 6 | | **250** | 4 | 34 |
| **94** | 15 | 4 | | **251** | 12 | 67 |
| **95** | 15 | 2 | | **252** | 2 | 233 |
| **96** | 14 | 182 | | **253** | 2 | 263 |
| **97** | 45 | 6 | | **254** | 9 | 9 |
| **98** | 11 | 2 | | **255** | 16 | 2 |
| **99** | 20 | 2 | | **256** | NT | NT |
| **100** | 10 | 2 | | **256A** | 12 | 7 |
| **101** | 18 | 2 | | **256B** | 8 | 7 |
| **102** | 13 | 2 | | **257** | 8 | 11 |
| **103** | 8 | 7 | | **258** | 8 | 32 |
| **104** | 5 | 11 | | **259** | 13 | 13 |
| **105** | 6 | 5 | | **260** | 29 | 3 |
| **106** | 25 | 6 | | **260A** | 17 | 2 |
| **107** | 10 | 5 | | **260B** | 26 | 29 |
| **108** | 13 | 12 | | **261** | 32 | 62 |
| **109** | 10 | 8 | | **261A** | 32 | 73 |
| **110** | 4 | 5 | | **261B** | 17 | 64 |
| **111** | 4 | 8 | | **262** | 5 | 10 |
| **112** | 5 | 9 | | **263** | 11 | 11 |
| **113** | 10 | 8 | | **264** | 6 | 3254 |
| **114** | 9 | 4 | | **265** | 13 | 2 |
| **115** | 8 | 10 | | **266** | 31 | 14 |
| **116** | 6 | 8 | | **267** | 25 | 12 |
| **117** | 9 | 1 | | **268** | 12 | 18 |
| **118** | 8 | 1 | | **269** | 23 | 44 |
| **119** | 9 | 1 | | **270** | 18 | 6 |
| **120** | 10 | 1 | | **271** | 413 | 0.7 |
| **121** | 10 | 2 | | **272** | 540 | 3 |
| **122** | 26 | 372 | | **273** | 47 | 5 |
| **123** | 18 | 346 | | **274** | 14 | 65 |
| **124** | 13 | 3 | | **275** | 5 | 3 |
| **125** | 45 | 2 | | **276** | 18 | 86 |
| **126** | 3 | 8 | | **277** | 37 | 45 |
| **127** | 5000 | 39 | | **278** | 25 | 315 |
| **128** | 40 | 12 | | **279** | 90 | 6 |
| **129** | 7 | 14 | | **280** | 4 | 96 |
| **130** | 6 | 2 | | **281** | 6 | 23 |
| **131** | 9 | 85 | | **282** | 6 | 25 |
| **132** | 19 | 4 | | **283** | 4 | 13 |

| | | | | | | |
|---|---|---|---|---|---|---|
| NT = not tested | | | | | | |

### Human SERT

Homogenized HEK293 (Human Embryonic Kidney) membranes expressing the human SERT were incubated with ³H-citalopram (SERT) at rt for 1 h in 50 mM Tris, 120 mM NaCl, 5 mM KCl (pH 7.4). Nonspecific binding was determined in the presence of 10 µM fluoxetine for the SERT. The membranes were washed and the radioactivity was counted as above. Calculations for Kᵢ at the SERT were based on a K_{d} value for ³H-citalopram and a ligand concentration of 3.1 nM. Data for compounds tested in this assay are presented in Table 2.

**Table 2.**

| **EX** | **Human SERT** | | **EX** | **Human SERT** |
|---|---|---|---|---|
| | **Kᵢ (nM)** | | | **Kᵢ (nM)** |
| **1** | 3 | | **84** | 7 |
| **1A** | 9 | | **91** | 11 |
| **1B** | 3 | | **92** | 9 |
| **2** | 56 | | **103** | 3 |
| **3** | 4 | | **104** | 3 |
| **4** | 21 | | **109** | 3 |
| **5** | 18 | | **110** | 5 |
| **6** | 15 | | **112** | 6 |
| **7** | 14 | | **116** | 7 |
| **8** | 8 | | **117** | 10 |
| **9** | 6 | | **118** | 4 |
| **10** | 5 | | **121** | 6 |
| **11** | 5 | | **124** | 56 |
| **12** | 16 | | **126** | 15 |
| **13** | 11 | | **129** | 5 |
| **14** | 18 | | **130** | 9 |
| **17** | 7 | | **134** | 30 |
| **20** | 3 | | **135** | 15 |
| **21** | 4 | | **136** | 20 |
| **22** | 5 | | **138** | 8 |
| **23** | 4 | | **139** | 6 |
| **24** | 3 | | **140** | 23 |
| **25** | 6 | | **141** | 2 |
| **26** | 5 | | **142** | 7 |
| **29** | 8 | | **145** | 5 |
| **29A** | 13 | | **146** | 12 |
| **29B** | 6 | | **148** | 5 |
| **30** | 2 | | **152** | 11 |
| **31** | 3 | | **153** | 2 |
| **32** | 9 | | **154** | 4 |
| **34** | 4 | | **156** | 10 |
| **35** | 3 | | **157** | 5 |
| **36** | 3 | | **158** | 3 |
| **37** | 6 | | **179** | 4 |
| **37A** | 13 | | **195** | 34 |
| **37B** | 6 | | **198** | 2 |
| **39** | 3 | | **199** | 2 |
| **40** | 8 | | **204** | 5 |
| **42** | 8 | | **214** | 12 |
| **44** | 7 | | **215** | 5 |
| **45A** | 7 | | **216** | 8 |
| **45B** | 6 | | **220** | 12 |
| **46** | 26 | | **222** | 18 |
| **47** | 11 | | **236** | 9 |
| **48** | 118 | | **241** | 10 |
| **50** | 3 | | **243** | 6 |
| **51** | 103 | | **247** | 7 |
| **52** | 2 | | **254** | 7 |
| **53** | 4 | | **256B** | 4 |
| **57** | 53 | | **257** | 11 |
| **59** | 160 | | **259** | 22 |
| **60** | 7 | | **271** | 3000 |
| **61** | 5 | | **272** | 3000 |
| **62** | 15 | | **273** | 158 |
| **63** | 2 | | **274** | 27 |
| **64** | 252 | | **275** | 4 |
| **66** | 4 | | **276** | 9 |
| **68** | 89 | | **277** | 13 |
| **71** | 31 | | **278** | 9 |
| **72** | 29 | | **279** | 98 |
| **76** | 9 | | **280** | 13 |
| **77** | 28 | | **281** | 8 |
| **83** | 5 | | **282** | 17 |
| | | | **283** | 7 |

### Cyclic AMP accumulation

Sublines of SK-N-MC cells were created that expressed a reporter construct and the human H₃ receptor. The reporter gene (β-galactosidase) is under the control of multiple cyclic AMP responsive elements. In 96-well plates, histamine was added directly to the cell media followed 5 min later by an addition of forskolin (5 µM final concentration). When appropriate, antagonists were added 10 min prior to agonist addition. After a 6-h incubation at 37 °C, the media was aspirated and the cells washed with 200 µL of phosphate-buffered saline followed by a second aspiration. Cells were lysed with 25 µL 0.1 x assay buffer (10 mM Na-phosphate, pH 8, 0.2 mM MgSO₄, 0.01 mM MnCl₂) and incubated at rt for 10 min. Cells were then incubated for 10 min with 100 µL of 1 x assay buffer containing 0.5% Triton and 40 mM β-mercaptoethanol. Color was developed using 25 µL of 1 mg/mL substrate solution (chlorophenolred β-D galactopyranoside; Roche Molecular Biochemicals, Indianapolis, IN). Color was quantitated on a microplate reader at absorbance 570 nM. The pA2 values were calculated by Schild regression analysis of the pEC₅₀ values and are presented for compounds tested in Table 3.

**Table 3.**

| **EX** | **pA₂** | | **EX** | **pA₂** |
|---|---|---|---|---|
| **1** | 8.6 | | **71** | 9.6 |
| **1A** | 8.4 | | **72** | 9.1 |
| **1B** | 8.9 | | **73** | 9.0 |
| **3** | 9.4 | | **76** | 8.4 |
| **8** | 8.3 | | **77** | 7.7 |
| **9** | 8.6 | | **83** | 8.9 |
| **10** | 8.4 | | **85** | 8.2 |
| **11** | 9.4 | | **146** | 8.7 |
| **20** | 9.0 | | **147** | 8.0 |
| **21** | 7.9 | | **155** | 8.5 |
| **37** | 8.2 | | **158** | 8.1 |
| **37B** | 8.3 | | **165A** | 8.5 |
| **44** | 9.1 | | **213** | 8.0 |
| **45A** | 8.9 | | **214** | 8.2 |
| **45B** | 9.1 | | **236** | 8.1 |
| **50** | 8.4 | | **237** | 7.8 |
| **51** | 9.3 | | **238** | 7.9 |
| **52** | 8.3 | | **256A** | 8.3 |
| **53** | 8.2 | | **256B** | 8.4 |
| **55** | 8.3 | | **260A** | 9.0 |
| **57** | 10.0 | | **262** | 8.2 |
| **58** | 7.5 | | **263** | 8.1 |
| **59** | 9.9 | | **265** | 8.8 |
| **60** | 8.6 | | **271** | 9.6 |
| **61** | 9.3 | | **272** | 8.5 |
| **62** | 9.3 | | **273** | 8.7 |
| **63** | 9.5 | | **275** | 8.9 |
| **69** | 9.1 | | **279** | 8.5 |

## Claims

1. A compound of formula (I); 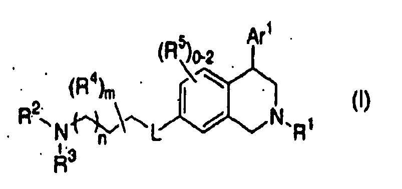 wherein
L is -0- and n is 1 or 2; or L is -C≡C- or -CH₂CH₂- and n is 0 or 1;
R¹ is -H; or is -C₁-₆alkyl, -C₃-₆alkenyl, -C₃₋₆alkynyl, -C₃₋₇cycloalkyl, -C₁-₆alkylC₃₋₇cycloalkyl, -COO₁₋₆alkyl, or -COObenzyl, each optionally, mono-, di-, or tri-substituted with R⁸;
where R⁸ is selected from -OH, -OC₁₋₆alkyl, phenyl optionally substituted with -OC₁₋₄alkyl or halo, -CN, -NO₂, -N(R^{b})R^{c}, -C(O)N(R^{b})R^{c}, -N(R^{b})C(O)R⁶, -N(R^{b})SO₂C₁₋₆alkyl, -C(O)C₁₋₆alkyl, -S(O)₀₋₂-C₁₋₆alkyl, -SO₂N(R^{b})R^{c}, -SCF₃, halo, -CF₃, -OCF₃, -COOH, and -COOC₁₋₆alkyl;
wherein R^{b} and R^{c} are each independently -H or -C₁₋₆alkyl;
R² and R³ are each independently selected from the group consisting of: -H;
A) -C₁₋₆alkyl, -C₃₋₆alkenyl, -C₃₋₆alkynyl, -C₃₋₇cycloalkyl, -C₁₋₆alkylC₃₋₇cycloalkyl, -CH(C₃₋₈cycloalkyl)₂; -CH(phenyl)₂, benzyl, and -C(O)OC₁₋₄alkyl, wherein each alkyl, cycloalkyl, or benzyl is optionally substituted with -OH, -OC₁₋₄alkyl, -CN, -NH₂, -NH(C₁₋₄alkyl), -N(C₁₋₄alkyl)₂, halo, -CF₃, -OCF₃, -COOH, or -COOC₁₋₆alkyl;
B) phenyl or pyridyl, optionally fused at two adjacent carbon ring members to a three- or four-membered hydrocarbon moiety to form a fused five- or six-membered aromatic ring, which moiety has one carbon atom replaced by >O, >S, >NH, >N(C₁₋₄alkyl), or >NC(O)OC₁₋₄alkyl, and which moiety has up to one additional carbon atom optionally replaced by -N=;
C) naphthyl,
D) a 4-8 membered heterocyclic ring, said heterocyclic ring having a carbon atom which is the point of attachment, having 1 or 2 heteroatom members selected from the group consisting of >O, >S(O)₀₋₂, >NH, >N(C₁₋₄alkyl), and >NC(O)OC₁₋₄alkyl, and having 0 or 1 double bonds; and
E) a monocyclic aromatic hydrocarbon group having five or six ring atoms, having a carbon atom which is the point of attachment, having one carbon atom replaced by. >O, >S, >NH, >N(C₁₋₄alkyl), or >NC(O)OC₁₋₄alkyl, having up to one additional carbon atom optionally replaced by -N=, and optionally benzofused or pyridofused;
where each of B)-E) are optionally mono-, di-, or tri-substituted with a moiety selected from the group consisting of -C₁₋₄alkyl, -OH, -C₁₋₄alkylOH, -OC₁₋₆₆alkyl, -CN, -NO₂, -N(R^{d})R^{e}-C(O)N(R^{d})R^{e}, -N(R^{d})C(O)R^{d}, -N(R^{d})SO₂C₁₋₆alkyl, -C(O)C₁₋₆alkyl, -S(O)₀₋₂-C₁₋₆alkyl, -SO₂N(R^{d})R^{e}, -SCF₃, halo, -CF₃, -OCF₃, -COOH, -COOC₁₋₆alkyl, -OC(O)N(R^{d})R^{e}, and -OC(O)OC₁₋₆alkyl;
where R^{d} and R^{e} are each independently -H or -C₁₋₆alkyl;
or, alternatively,
R² and R³ may be taken together with the nitrogen to which they are attached to form tetrahydro-pyrimidin-2-ylidenyl, or a 4-8 membered heterocyclic ring, said heterocyclic ring having 0 or 1 additional heteroatom members separated from the nitrogen of attachment by at least one carbon member and selected from the group consisting of >O, >S(O)₀₋₂, >NH, and >NR^{f}, having 0 or 1 double bonds, having 0, 1 or 2 carbon members separated from the nitrogen of attachment by at least one carbon member which is a carbonyl, optionally benzo or pyrido fused, optionally having one carbon member that forms a bridge, and having 0-5 carbon member substituents R^{ff},
where R^{f} is selected from the group consisting of:
i) -C₁₋₆alkyl optionally substituted with R^{z}, -C₃₋₆alkenyl, -C₃₋₆alkynyl, -C(O)N(R^{g})R^{h}, -C(O)Rⁱ, -S(O)₀₋₂-C₁₋₆alkyl, and -COOC₁₋₆alkyl,
where R^{z} is fluoro, -CN, -OH, -OC₁₋₄alkyl, -C₃₋₇cycloalkyl, or -CF₃;
where R^{g} and R^{h} are each independently -H or -C₁₋₆alkyl; and
where Rⁱ is -C₁₋₆alkyl, -C₃₋₈cycloalkyl, phenyl, or 5- or 6-membered aromatic heterocyclyl, where each alkyl, cycloalkyl, phenyl or heterocyclyl is optionally mono-, di-, for tri-substituted with -C₁₋₄alkyl, -OH, -OC₁₋₆alkyl, -CF₃, -CN, or halo;
ii) -(CH₂)₀₋₁-RingA, where Ring A is phenyl or a 5- or 6-membered carbon-linked aromatic heterocyclyl, optionally substituted with R^{aa};
where R^{aa} is -OH, C₁₋₆alkyl, -OC₁₋₆alkyl, phenyl, -CN, -NO₂, -N(R^{g})R^{h} -C(O)N(R^{g})R^{h}, -N(R^{g})C(O)R^{h}, -N(R^{h})SO₂C₁₋₆alkyl, -C(O)C₁₋₆alkyl, -S(O)₀₋₂-C₁₋₆alkyl, -SO₂N(R^{g})R^{h}, -SCF₃, halo, -CF₃, -OCF₃, -OCHF₂, -COOH, and -COOC₁₋₆alkyl; and
iii) -(CH₂)₀₋₁-RingB, where Ring B is -C₃₋₇cycloalkyl with one or two carbon ring members optionally replaced with >O, or >NH, and optionally substituted with -C₁₋₄alkyl, fluoro, -CN, -OH, -OC₁₋₄alkyl, or -CF₃;
where R^{ff} is selected from the group consisting of -C₁₋₆alkyl optionally mono-or di-substituted with R^{z}, -C₂₋₆alkenyl, -C₂₋₆alkynyl, -C₃₋₇cycloalkyl, -CH(phenyl)₂, halo, -OH, -OC₁₋₆alkyl, -OC₂₋₃alkylO, -CN, -NO₂, -N(R^{g})R^{h}, -C(O)N(R^{g})R^{h}, -N(R^{g})C(O)R^{g}, -N(R^{g})SO₂C₁₋₆alkyl, -C(O)R^{l}, -S(O)₀₋₂-C₁₋₆alkyl, -SO₂N(R^{g})R^{h}, -SCF₃, -CF₃, -OCF₃, -COOH, and -COOC₁₋₆alkyl;
R⁴ is -OH, -OC₁₋₆alkyl, -CF₃, -C₁₋₆alkyl, or halo; two R⁴ substituents may be taken together to form methylene or ethylene; or one of R⁴ is taken together with R² to form methylene, ethylene, propylene, or -CH₂CH₂O-, wherein each is optionally substituted with -OH. -OC₁₋₆alkyl, -SC₁₋₆alkyl, -CF₃, -C₁₋₆alkyl, amino, or halo;
m is 0, 1, or 2;
R⁵ is selected from the group consisting of -C₁₋₆alkyl, -OH, -OC₁₋₆alkyl, -SC₁₋₆alkyl, and halo;
Ar¹ is an aryl or heteroaryl ring selected from the group consisting of:
a) phenyl, optionally mono-, di-, or tri-substituted with R^{j} and optionally di-substituted on adjacent carbons with -OC₁₋₄alkyleneO- optionally mono-or di-substituted with fluoro, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
where R^{j} is selected from the group consisting of
1) -OH, -C₁₋₆alkyl, -OC₁₋₆alkyl optionally mono-, di-, or tri-substituted with halo, -C_{2'-6}alkenyl, -OC₃₋₆alkenyl, -C₂₋₆alkynyl optionally substituted with trimethylsilyl, -OC₃₋₆alkynyl, -C₃₋₆cycloalkyl, -OC₃₋₆cycloalkyl, -CN, -NO₂, -N(R^{k})R^{l}, -N(R^{k})C(O)R^{l}, -N(R^{k})SO₂C₁₋₆alkyl, -C(O)C₁₋₆alkyl, -S(O)₀₋₂-C₁₋₆alkyl, -C(O)N(R^{m})Rⁿ, -SO₂N(R^{m})Rⁿ, -SCF₃, halo, -CF₃, -COOH, -COOC₁₋₆alkyl, and -COOC₃₋₇cycloalkyl;
where R^{k} and R^{l} are each independently -H or -C₁₋₆alkyl;
where R^{m} and Rⁿ are each independently -H or -C₁₋₆alkyl, or R^{m} and Rⁿ taken together with their nitrogen of attachment form a 4-8 membered heterocyclic ring having 1 or 2 heteroatom members selected from >O, >S(O)₀₋₂, >NH, and >NC₁₋₆alkyl, having 0 or 1 double bonds, having 0 or 1 carbonyl members;
2) -G-Ar², where G is a bond, -O-, or -S-, and Ar² is phenyl or is a monocyclic aromatic hydrocarbon group having five or six ring atoms, having one carbon atom replaced by >O, >S, >NH, or >N(C₁₋₄alkyl), having up to one additional carbon atom optionally replaced by -N=, each optionally mono-, di-, or tri-substituted with R^{p};
where R^{p} is a substituent independently selected from the group consisting of: -OH, -C₁₋₆alkyl, -OC₁₋₆alkyl, phenyl, -CN, -NO₂, -N(R^{q})R^{r}, -C(O)N(R^{q})R^{r}, -N(R^{q})C(O)R^{r}, -N(R^{q})SO₂C₁₋₆alkyl, -C(O)C₁₋₆alkyl, -S(O)₀₋₂-C₁₋₆alkyl, -SO₂N(R^{q})R^{r}, -SCF₃, halo, -CF₃, -OCF₃, -OCHF₂, -COOH, and -COOC₁₋₆alkyl;
wherein R^{q} and R^{r} are each independently selected from -H, -C₁₋₆alkyl, and -C₂₋₆alkenyl; and
3) a 4-8 membered saturated or partially saturated heterocyclic ring, having 1 or 2 heteroatom members selected from >O, >S(O)₀₋₂, >NH, and >NC₁₋₆alkyl, having 0 or 1 carbonyl members, said ring optionally mono-, di-, or tri-substituted with R^{p};
b) phenyl or pyridyl fused at two adjacent carbon ring members to a three membered hydrocarbon moiety to form a fused five membered aromatic ring, which moiety has one carbon atom replaced by >O, >S, >NH, or >N(C₁₋₄alkyl), and which moiety has up to one additional carbon atom optionally replaced by -N=, the fused rings optionally mono-, di-, or tri-substituted with R¹;
where R^{t} is a substituent independently selected from the group consisting of: -OH, -C₁₋₆alkyl, -OC₁₋₆alkyl, phenyl, -CN, -NO₂, -N(R^{u})R^{v}, -C(O)N(R^{u})R^{v}, -N(R^{u})C(O)R^{v}, -N(R^{u})SO₂C₁₋₆alkyl, -C(O)C₁₋₆alkyl, -S(O)₀₋₂-C₁₋₆alkyl, -SO₂N(R^{u})R^{v}, -SCF₃, halo, -CF₃, -OCF₃, -OCHF₂, -COOH, and -COOC₁₋₆alkyl;
where R^{u} and R^{v} are each independently -H or -C₁₋₆alkyl;
c) phenyl fused at two adjacent ring members to a four membered hydrocarbon moiety to form a fused six membered aromatic ring, which moiety has one or two carbon atoms replaced by -N=, the fused rings optionally mono-, di-, or tri-substituted with R^{t};
d) naphthyl, optionally mono-, di-, or tri-substituted with R^{t};
e) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atom optionally replaced by -N=, optionally mono- or di-substituted with R^{j} and optionally benzofused or pyridofused at two adjacent carbon atoms, where the benzofused or pyridofused moiety is optionally mono-, di-, or tri-substituted with R^{t}; and
f) a monocyclic aromatic hydrocarbon group having six ring atoms, having a carbon atom which is the point of attachment, having one or two carbon atoms replaced by -N=, optionally mono- or di-substituted with R^{j} and optionally benzofused or pyridofused at two adjacent carbon atoms, where the benzofused or pyridofused moiety is optionally mono- or di-substituted with R^{t}; and enantiomers, diastereomers, hydrates, solvates and pharmaceutically acceptable salts, esters and amides thereof with the proviso: when n is 1, L is -O-, Ar is unsubstituted phenyl and R² and R³ are both methyl, even R⁴ is not -OH for use in the treatment or prevention of a CNS disorder selected from the group consisting of: sleep/wake and arousal/vigilance disorders, insomnia, jet lag, disturbed sleep, attention deficit hyperactivity disorders (ADHD), attention-deficit disorders, learning and memory disorders, learning impairment, memory impairment, memory loss, cognitive dysfunction, migraine, neurogenic inflammation, dementia, mild cognitive impairment, pre-dementia, Alzheimer's disease, epilepsy, narcolepsy with or without associated cataplexy, cataplexy, disorders of sleep/wake homeostasis, idiopathic somnolence, excessive daytime sleepiness (EDS), circadian rhythym disorders, sleep/fatigue disorders, fatigue, drowsiness associated with sleep apnea, sleep impairment due to perimenopausal hormonal shifts, Parkinson's-related fatigue, MS-related fatigue, depression-related fatigue, chemotherapy-induced fatigue, work-related fatigue, lethargy, eating disorders, obesity, motion slickness, vertigo, schizophrenia, substance abuse, bipolar disorders, manic disorders and depression in mammals

2. The compound of claim 1, wherein L is -O- and n is 1.

3. The compound of claim 1, wherein L is -C≡C- and n is 0.

4. The compound of claim 1, wherein L is -CH₂CH₂- and n is 0.

5. The compound of claim 1, wherein R¹ is methyl, ethyl, propyl, t-butyl, allyl, propargyl or benzyl.

6. The compound of claim 1, wherein R¹ is hydrogen or methyl.

7. The compound of claim 1, wherein when R² is methyl, R³ is not methyl.

8. The compound of claim 1, wherein R² and R³ may be taken together with the nitrogen to form a 4-8 membered heterocyclic ring, said heterocyclic ring selected from piperidine, pyrrolidine, and morpholine, said ring substituted with I or 2 substituents R^{ff}.

9. The compound of claim 1, selected from the group consisting of:
7-[3-(4-Fluoro-piperidin-1-yl)-propoxy]-4-(4-methoxy-phenyl)-2-methyl-1,2,3,4-tetrahydro-isoquinoline;
7-[3-(4-Fluoro-piperidin-1-yl)-propoxy]-4-(4-methoxy-phenyl)-2-methyl-1,2,3,4-tetrahydro-isoquinoline (enantiomer 1);
7-[3-(4-Fluoro-piperidin-1-yl)-propoxy]-4-(4-methoxy-phenyl)-2-methyl-1,2,3,4-tetrahydro-isoquinoline (enantiomer 2);
1-(4-{3-[4-(4-Methoxy-phenyl)-2-methyl-1,2,3,4-tetrahydro-isoquinolin-7-yloxy]-propyl}-piperazin-1-yl)-ethanone;
Diethyl-{3-[4-(4-methoxy-phenyl)-2-methyl-1,2,3,4-tetrahydro-isoquinolin-7-yloxy]-propyl}-amine;
(4-{3-[4-(4-Methoxy-phenyl)-2-methyl-1,2,3,4-tetrahydro-isoquinolin-7-yloxy]-propyl}-piperazin-1-yl)-pyridin-4-yl-methanone;
1-(4-{3-[4-(4-Methoxy-phenyl)-2-methyl-1,2,3,4-tetrahydro-isoquinolin-7-yloxy]-propyl}-piperazin-1-yl)-2-methyl-propan-1-one;
Cyclobutyl-(4-{3-[4-(4-methoxy-phenyl)-2-methyl-1,2,3,4-tetrahydro-isoquinolin-7-yloxy]-propyl}-piperazin-1-yl)-methanone;
Cyclopropyl-(4-{3-[4-(4-methoxy-phenyl)-2-methyl-1,2,3,4-tetrahydro-isoquinolin-7-yloxy]-propyl}-piperazin-1-yl)-methanone;
7-[3-(4,4-Difluoro-piperidin-1-yl)-propoxy]-4-(4-methoxy-phenyl)-2-methyl-1,2,3,4-tetrahydro-isoquinoline;
4-(4-Methoxy-phenyl)-2-methyl-7-(3-morpholin-4-yl-propoxy)-1,2,3,4-tetrahydro-isoquinoline;
(1-{3-[4-(4-Methoxy-phenyl)-2-methyl-1,2,3,4-tetrahydro-isoquinolin-7-yloxy]-propyl}-pyrrolidin-3-yl)-dimethyl-amine;
7-[3-((2R,5R)-trans-Dimethyl-pyrrolidin-1-yl)-propoxy]-4-(4-methoxy-phenyl)-2-methyl-1,2,3,4-tetrahydro-isoquinoline;
4-{3-[4-(4-Methoxy-phenyl)-2-methyl-1,2,3,4-tetrahydro-isoquinolin-7-yloxy]-propyl}-piperazine-1-carboxylic acid, ethyl ester;
(4-{3-[4-(4-Methoxy-phenyl)-2-methyl-1,2,3,4-tetrahydro-isoquinolin-7-yloxy]-propyl}-piperazin-1-yl)-(1-methyl-1H-pyrrol-2-yl)-methanone;
(4-{3-[4-(4-Methoxy-phenyl)-2-methyl-1,2,3,4-tetrahydro-isoquinolin-7-yloxy]-propyl}-piperazin-1-yl)-(1-methyl-1H-imidazol-4-yl)-methanone;
(1,3-Dimethyl-tetrhydro-pyrimidin-2-ylidene)-{3-[4-(4-methoxy-phenyl)-2-methyl-1,2,3,4-tetrahydro-isoquinolin-7-yloxy]-propyl}-amine;
7-[3-(1,3-Dihydro-isoindol-2-yl)-propoxy]-4-(4-methoxy-phenyl)-2-methyl-1,2,3,4-tetrahydro-isoquinoline;
Bis-(2-methoxy-ethyl)-{3-[4-(4-methoxy-phenyl)-2-methyl-1,2,3,4-tetrahydro-isoquinolin-7-yloxy]-propyl}-amine ;
7-[3-(5,6-Dihydro-4H-pyrimidin-1-yl)-propoxy]-4-(4-methoxy-phenyl)-2-methyl-1,2,3,4-tetrahydro-isoquinoline;
Benzothiazol-2-yl-{3-[4-(4-methoxy-phenyl)-2-methyl-1,2,3,4-tetrahydro-isoquinolin-7-yloxy]-propyl}-methyl-amine;
1-{3-[4-(4-Methoxy-phenyl)-2-methyl-1,2,3,4-tetrahydro-isoquinolin-7-yloxy]-propyl}-piperidin-3-ol;
1-{3-[4-(4-Methoxy-phenyl)-2-methyl-1,2,3,4-tetrahydro-isoquinolin-7-yloxy]-propyl}-piperidin-4-ol;
(1-{3-[4-(4-Methoxy-phenyl)-2-methyl-1,2,3,4-tetrahydro-isoquinolin-7-yloxy]-propyl}-pipendin-4-yl)-methanol;
(1-{3-[4-(4-Methoxy-phenyl)-2-methyl-1,2,3,4-tetrahydro-isoquinolin-7-yloxy]-propyl}-piperidin-3-yl)-methanol;
(1-{3-[4-(4-Methoxy-phenyl)-2-methyl-1,2,3,4-tetrahydro-isoquinolin-7-yloxy]-propyl}-piperidin-2-yl)-methanol;
4-(4-Methoxy-phenyl)-2-methyl-7-[3-(3-trifluoromethyl-piperidin-1-yl)-propoxy]-1,2,3,4-tetrahydro-isoquinoline;
2-(1-{3-[4-(4-Methoxy-phenyl)-2-methyl-1,2,3,4-tetrahydro-isoquinolin-7-yloxy]-propyl}-piperidin-4-yl)-ethanol;
7-[3-(1,1-Dioxo-1λ⁶-thiomorpholin-4-yl)-propoxy]-4-(4-methoxy-phenyl)-2-methyl-1,2,3,4-tetrahydro-isoquinoline;
4-(4-Methoxy-phenyl)-2-methyl-7-[3-((2S)-trifluoromethyl-pyrrolidin-1-yl)-propoxy]-1,2,3,4-tetrahydro-isoquinoline;
7-[3-(3,3-Difluoro-piperidin-1-yl)-propoxy]-4-(4-methoxy-phenyl)-2-methyl-1,2,3,4-tetrahydro-isoquinoline;
7-[3-(3,3-Difluoro-piperidin-1-yl)-propoxy]-4-(4-methoxy-phenyl)-2-methyl-1,2,3,4-tetrahydro-isoquinoline (enantiomer 1);
7-[3-(3,3-Difluoro-piperidin-1-yl)-propoxy]-4-(4-methoxy-phenyl)-2-methyl-1,2,3,4-tetrahydro-isoquinoline (enantiomer 2);
(1-{3-[4-(4-Methoxy-phenyl)-2-methyl-1,2,3,4-tetrahydro-isoquinolin-7-yloxy]-propyl}-pyrrolidin-(2R)-yl)-methanol;
1-{3-[4-(4-Methoxy-phenyl)-2-methyl-1,2,3,4-tetrahydro-isoquinolin-7-yloxy]-propyl}-(R)-pyrrolidin-3-ol;
7-[3-(2,6-Dimethyl-morpholin-4-yl)-propoxy]-4-(4-methoxy-phenyl)-2-methyl-1,2,3,4-tetrahydro-isoquinoline;
1-{3-[4-(4-Methoxy-phenyl)-2-methyl-1,2,3,4-tetrahydro-isoquinolin-7-yloxy]-propyl}-piperidine-4-carboxylic acid ethyl ester; 7-(3-Azetidin-1-yl-propoxy)-4-(4-methoxy-phenyl)-2-methyl-1,2,3,4-tetrahydro-isoquinoline; 4-(4-Methoxy-phenyl)-2-methyl-7-[3-(2-methyl-pyrrolidin-1-yl)-propoxy]-1,2,3,4-tetrahydro-isoquinoline; 2-(1-{3-[4-(4-Methoxy-phenyl)-2-methyl-1,2,3,4-tetrahydro-isoquinolin-7-yloxy]-propyl}-piperidin-2-yl)-ethanol; 1-{3-[4-(4-Methoxy-phenyl)-2-methyl-1,2,3,4-tetrahydro-isoquinolin-7-yloxy]-propyl}-piperidine-4-carbonitrile; 1-{3-[4-(4-Methoxy-phenyl)-2-methyl-1,2,3,4-tetrahydro-isoquinolin-7-yloxy]-propyl}-piperidine-4-carbonitile (enantiomer 1); 1-{3-[4-(4-Methoxy-phenyl)-2-methyl-1,2,3,4-tetrahydro-isoquinolin-7-yloxy]-propyl}-piperidine-4-carbonitrile (enantiomer 2); 4-(4-Methoxy-phenyl)-2-methyl-7-[3-(4-trifluoromethyl-piperidin-1-yl)-propoxy]-1,2,3,4-tetrahydro-isoquinoline; 7-[3-(3-Fluoro-piperidin-1-yl)-propoxy]-4-(4-methoxy-phenyl)-2-methyl-1,2,3,4-tetrahydro-isoquinoline; Ethyl-(2-methoxy-ethyl)-{3-[4-(4-methoxy-phenyl)-2-methyl-1,2,3,4-tetrahydro-isoquinolin-7-yloxy]-propyl}-amine; 7-[3-(1,4-Dioxa-8-aza-spiro[4.5]dec-8-yl)-propoxy]-4-(4-methoxy-phenyl)-2-methyl-1,2,3,4-tetrahydro-isoquinoline; 1-[4-(4-Methoxy-phenyl)-2-methyl-1,2,3,4-tetrahydro-isoquinolin-7-yloxy]-3-piperidin-1-yl-propan-2-ol; 7-[2-Fluoro-3-(4-fluoro-piperidin-1-yl)-propoxy]-4-(4-methoxy-phenyl)-2-methyl-1,2,3,4-tetrahydro-isoquinoline; 7-[3-(4-Fluoro-piperidin-1-yl)-propoxy]-4-(3-methoxy-phenyl)-2-methyl-1,2,3,4-tetrahydro-isoquinoline; 7-[3-(4-Fluoro-piperidin-1-yl)-propoxy]-4-(3-methoxy-phenyl)-2-methyl-1,2,3,4-tetrahydro-isoquinoline (enantiomer 1);
7-[3-(4-Fluoro-piperidin-1-yl)-propoxy]-4-(3-methoxy-phenyl)-2-methyl-1,2,3,4-tetrahydro-isoquinoline (enantiomer 2);
4-(3-Chloro-phehyl)-7-[3-(4-fluoro-pipeeridin-1-yl)-ropoxy]-2-methyl-1,2,3,4-tetrahydro-isoquinoline;
4-(4-Chloro-phenyl)-7-[3-(4-fluoro-piperidin-1-yl)-propoxy]-2-methyl-1,2,3,4-tetrahydro-isoquiholine;
4-(3-Fluoro-phenyl)-7-[3-(4-fluoro-piperidin-1-yl)-propoxy]-2-methyl-1,2,3,4-tetrahydro-isoquinoline;
7-[3-(4-Fluoro-piperidin-1-yl)-propoxy]-2-methyl-4-(4-trifluoromethoxy-phenyl)-1,2,3,4-tetrahydro-isoquinoline;
4-(4-Difluoromethoxy-phenyl)-7-[3-(4-fluoro-piperidin-1-yl)-propoxy]-2-methyl-1,2,3,4-tetrahydro-isoquinoline;
7-[3-(4-Fluoro-piperidin-1-yl)-propoxy]-4-(4-methahesulfonyl-phenyl)-2-methyl-1,2,3,4-tetrahydro-isoquinoline;
7-[3-(4-Fluoro-piperidin-1-yl)-propoxy]-2-methyl-4-(4-methylsulfanyl-phenyl)-1,2,3,4-tetrahydro-isoquinoline;
4-(3-Chloro-4-methoxy-phenyl)-7-[3-(4-fluoro-piperidin-1-yl)-propoxy]-2-methyl-1,2,3,4-tetrahydro-isoquinoline;
4-(2,4-Dichloro-phenyl)-7-[3-(4-fluoro-piperidin-1-yl)-propoxy]-2-methyl-1,2,3,4-tetrahydro-isoquinoline;
4-(2,5-Dichloro-phenyl)-7-[3-(4-fluoro-piperidin-1-yl)-propoxy]-2-methyl-1,2,3,4-tetrahydro-isoquinoline;
4-(3,5-Dichloro-phenyl)-7-[3-(4-fluoro-piperidin-1-yl)-propoxy]-2-methyl-1,2,3,4-tetrahydro-isoquinoline;
2-Methyl-7-(3-piperidin-1-yl-propoxy)-4-thiophen-3-yl-1,2,3,4-tetrahydro-isoquinoline;
4-(3,4-Dichloro-phenyl)-7-[3-(4-fluoro-piperidin-1-yl)-propoxy]-2-methyl-1,2,3,4-tetrahydro-isoquinoline;
2-Methyl-7-(3-piperidin-1-yl-propoxy)-4-pyridin-3-yl-1,2,3,4-tetrahydro-isoquinoline;
2-Methyl-7-(3-piperidin-1-yl-propoxy)-4-(trifluoromethyl-phenyl)-1,2,3,4-tetrahydro-isoquinoline;
4-(4-Methoxy-phenyl)-2-methyl-7-(3-piperidin-1-yl-propoxy)-1,2,3,4-tetrahydro-isoquinoline (racemic);
4-(4-Methoxy-phenyl)-2-methyl-7-(3-piperidin-1-yl-propoxy)-1,2,3,4-tetrahydro-isoquinoline (enantiomer 1); 4-(4-Methoxy-phenyl)-2-methyl-7-(3-piperidindin-1-ylpropoxy)-1,2,3,4-tetrahydro-isoquinoline (enantiomer 2); 2-tert-Butyl-4-(4-methoxy-phenyl)-7-(3-piperidin-1-yl-propoxy)-1,2,3,4-tetrahydro-isoquinoline; 2-Benzyl-4-(4-methoxy-phenyl)-7-(3-piperidin-1-yl-propoxy)-1,2,3,4-tetrahydro-isoquinoline; 2-Ethyl-4-(4-methoxy-phenyl)-7-(3-piperidin-1-yl-propoxy)-1,2,3,4-tetrahydro-isoquinoline; 4-(4-Methoxy-phenyl)-7-(3-piperidin-1-yl-propoxy)-2-propyl-1,2,3,4-tetrahydro-isoquinoline; 2-Isopropyl-4-(4-methoxy-phenyl)-7-(3-piperidin-1-yl-propoxy)-1,2,3,4-tetrahydro-isoquinoline; 4-(4-Methoxy-phenyl)-7-(3-piperidin-1-yl-propoxy)-1,2,3,4-tetrahydro-isoquinoline; 3-[4-(4-Methoxy-phenyl)-7-(3-piperidin-1-yl-propoxy)-3,4-dihydro-1H-isoquinolin-2-yl]-propan-1-ol; 2-[4-(4-Methoxy-phenyl)-7-(3-piperidin-1-yl-propoxy)-3,4-dihydro-1H-isoquinolin-2-yl]-ethanol; 2-(2-Fluoro-ethyl)-4-(4-methoxy-phenyl)-7-(3-piperidin-1-yl-propoxy)-1,2,3,4-tetrahydro-isoquinoline; 2-Cyclopropyl-4-(4-methoxy-phenyl)-7-(3-piperidin-1-yl-propoxy)-1,2,3,4-tetrahydro-isoquinoline; 4-(4-Methoxy-phenyl)-7-(3-morpholin-4-yl-propoxy)-2-(1-phenyl-ethyl)-1,2,3,4-tetrahydro-isoquinoline; 4-(4-Methoxy-phenyl)-7-(3-morpholin-4-yl-propoxy)-2-(1-phenyl-ethyl)-1,2,3,4-tetrahydro-isoquinoline (diastereomer 1); 4-(4-Methoxy-phenyl)-7-(3-morpholin-4-yl-propoxy)-2-(1-phenyl-ethyl)-1,2,3,4-tetrahydro-isoquinoline (diastereomer 2);
4-(3,4-Dichloro-phenyl)-2-methyl-7-(3-piperidin-1-yl-propoxy)-1,2,3,4-tetrahydro-isoquinoline;
4-(3,4-Dichloro-ohenyl)-2-methyl-7-(3-morpholin-4-yl-propoxy)-1,2,3,4-tetrahydro-isoquinoline;
4-(4-Methoxy-phenyl)-2-methyl-7-(piperidin-4-ylmethoxy)-1,2,3,4-tetrahydro-isoquinoline;
4-(3-Chloro-4-methoxy-phenyl)-2-methyl-7-(piperidin-4-ylmethoxy)-1,2,3,4-tetrahydro-isoquinoline;
2-Methyl-4-(4-methylsulfanyl-phenyl)-7-(piperidin-4-ylmethoxy)-1,2,3,4-tetrahydro-isoquinoline;
4-(3-Fluoro-4-methoxy-phenyl)-2-methyl-7-(piperidin-4-ylmethoxy)-1,2,3,4-tetrahydro-isoquinoline;
4-(2-Fluoro-4-methoxy-phenyl)-2-methyl-7-(piperidin-4-ylmethoxy)-1,2,3,4-tetrahydro-isoquinoline;
7-(1-Isopropyl-piperidin-4-ylmethoxy)-4-(4-methoxy-phenyl)-2-methyl-1,2,3,4-tetrahydro-isoquinoline;
4-(4-Methoxy-phenyl)-2-methyl-7-(1-methyl-piperidin-4-ylmethoxy)-1,2,3,4-tetrahydro-isoquinoline;
4-(4-Methoxy-phenyl)-2-methyl-7-(1-propyl-piperidin-4-ylmethoxy)-1,2,3,4-tetrahydro-isoquinoline;
7-(1-Cyclopentyl-piperidin-4-ylmethoxy)-4-(4-methoxy-phenyl)-2-methyl-1,2,3,4-tetrahydro-isoquinoline;
7-(1-Ethyl-piperidin-4-ylmethoxy)-4-(4-methoxy-phenyl)-2-methyl-1,2,3,4-tetrahydro-isoquinoline;
4-(3-Chloro-4-methoxy-phenyl)-7-(1-isopropyl-piperidin-4-ylmethoxy)-2-methyl-1,2,3,4-tetrahydro-isoquinoline;
4-(3-Fluoro-4-methoxy-phenyl)-7-(1-isopropyl-piperidin-4-ylmethoxy)-2-methyl-1,2,3,4-tetrahydro-isoquinoline;
4-(2-Fluoro-4-methoxy-phenyl)-7-(1-isopropyl-piperidin-4-ylmethoxy)-2-methyl-1,2,3,4-tetrahydro-isoquinoline;
7-(1-Isopropyl-piperidin-4-ylmethoxy)-4-(3-methoxy-phenyl)-2-methyl-1,2,3,4-tetrahydro-isoquinoline;
4-(3-Methoxy-phenyl)-2-methyl-7-(1-methyl-piperidin-4-ylmethoxy)-1,2,3,4-tetrahyoro-isoquinoline;
4-(3-Methoxy-phenyl)-2-methyl-7-(1-propyl-piperidin-4-ylmethoxy)-1,2,3,4-tetrahydro-isoquinoline;
7-(1-Cyclopentyl-piperidin-4-ylmethoxy)-4-(3-methoxy-phenyl)-2-methyl-1,2,3,4-tetrahydro-isoquinoline;
7-(1-Ethyl-piperidin-4-ylmethoxy)-4-(3-methoxy-phenyl)-2-methyl-1,2,3,4-tetrahydro-isoquinoline;
4-(3-Methoxy-phenyl)-2-methyl-7-(piperidin-4-yloxy)-1,2,3,4-tetrahydro-isoquinoline;
4-(3-Methoxy-phenyl)-2-methyl-7-(1-methyl-piperidin-4-yloxy)-1,2,3,4-tetrahydro-isoquinoline;
7-(1-Isopropyl-piperidin-4-yloxy)-4-(3-methoxy-phenyl)-2-methyl-1.2,3,4-tetrahydro-isoquinoline;
7-(1-Cyclobutyl-piperidin-4-yloxy)-4-(3-methoxy-phenyl)-2-methyl-1,2,3,4-tetrahydro-isoquinoline;
7-(1-Ethyl-piperidin-4-yloxy)-4-(3-methoxy-phenyl)-2-methyl-1,2,3,4-tetrahydro-isoquinoline;
7-(1-Cyclopentyl-piperidin-4-yloxy)-4-(3-methoxy-phenyl)-2-methyl-1,2,3,4-tetrahydro-isoquinoline;
4-(3-Methoxy-phenyl)-2-methyl-7-(1-propyl-piperidin-4-yloxy)-1,2,3,4-teirahydro-isoquinoline;
7-(1-Isopropyl-piperidin-4-ylmethoxy)-2-methyl-4-(4-methylsulfanyl-phenyl)-1,2,3,4-tetrahydro-isoquinoline;
7-(1-Cyclobutyl-piperidin-4-ylmethoxy)-2-methyl-4-(4-methylsulfanyl-phenyl)-1,2,3,4-tetrahydro-isoquinoline;
7-(1-Ethyl-piperidin-4-ylmethoxy)-2-methyl-4-(4-methylsulfanyl-phenyl)-1,2,3,4-tetrahydro-isoquinoline;
4-(3-Chloro-4-methoxy-phenyl)-7-(1-cyclobutyl-piperidin-4-ylmethoxy)-2-methyl-1,2,3,4-tetrahydro-isoquinoline;
4-(3-Chloro-4-methoxy-phenyl)-7-(1-ethyl-piperidin-4-ylmethoxy)-2-methyl-1,2,3,4-tetrahydro-isoquinoline;
4-(3-Chloro-4-methoxy-phenyl)-2-methyl-7-(1-propyl-piperidin-4-ylmethoxy)-1,2,3,4-tetrahydro-isoquinoline;
2-Methyl-4-(4-methylsulfanyl-phenyl)-7-(1-propyl-piperidin-4-ylmethoxy)-1,2,3,4-tetrahydro-isoquinoline;
7-(1-Isobutyl-piperidin-4-ylmethoxy)-4-(4-methoxy-phenyl)-2-methyl-1,2,3,4-tetrahydro-isoquinoline;
7-(1-Cyclobutyl-piperidin-4-ylmethoxy)-4-(3-fluoro-4-methoxy-phenyl)-2-methyl-1,2,3,4-tetrahydro-isoquinoline;
4-(3-Fluoro-4-methoxy-phenyl)-2-methyl-7-(1-propyl-iperidin-4-ylmethoxy)-1,2,3,4-tetrahydro-isoquinoline;
7-(1-Cyclobutyl-piperidin-4-ylmethoxy)-4-(2-fluoro-4-methoxy₋phenyl)-2-methyl-1,2,3,4-tetrahydro-isoquinoline;
4-(2-Fluoro-4-methoxy-phenyl)-2-methyl-7-(1-propyl-piperidin-4-ylmethoxy)-1,2,3,4-tetrahydro-isoquinoline;
4-(2-Fluoro-4-methoxy-phenyl)-7-(1-isobutyl-piperidin-4-ylmethoxy)-2-methyl-1,2,3,4-tetrahydro-isoquinoline;
7-[1-(2-Fluoro-ethyl)-piperidin-4-ylmethoxy]-4-(4-methoxy-phenyl)-2-methyl-1,2,3,4-tetrahydro-isoquinoline;
7-(1-Isopropyl-pipendin-4-yloxy)-4-(4-methoxy-phenyl)-2-methyl-1,2,3,4-tetrahydro-isoquinoline;
7-(1-Isopropyl-piperidin-4-yloxy)-2-methyl-4-(4-methylsulfanyl-phenyl)-1,2,3,4-tetrahydro-isoquinoline;
4-(2-Fluoro-4-methoxy-phenyl)-7-(1-isopropyl-piperidin-4-yloxy)-2-methyl-1,2,3,4-tetrahydro-isoquinoline;
4-(3-Fluoro-4-methoxy-phenyl)-7-(1-isopropyl-piperidin-4-yloxy)-2-methyl-1,2,3,4-tetrahydro-isoquinoline;
4-(4-Methoxy-phenyl)-2-methyl-7-[1-(tetrahydro-pyran-4-yl)-piperidin-4-yloxy]-1,2,3,4-tetrahydro-isoquinoline;
2,2,2-Trifluoro-1-{4-[4-(4-methoxy-phenyl)-2-methyl-1,2,3,4-tetrahydro-isoquinolin-7-yloxymethyl]-piperidin-1-yl}-ethanone;
4-(4-Methoxy-phenyl)-2-methyl-7-[1-(2,2,2-trifluoro-ethyl)-piperidin-4-ylmethoxy]-1,2,3,4-tetrahydroisoquinoline;
7-[3-(4-Fluoro-pipridin-1-yl)-propoxy]-2-methyl-4-phenyl-1,2,3,4-tetrahydro-isoquinoline;
4-(2-Fluoro-phenyl)-7-[3-(4-fluoro-piperidin-1-yl)-propoxy]-2-methyl-1,2,3,4-tetrahydro-isoquinoline;
7-[3-(4-Fluoro-piperidin-1-yl)-propoxy]-2-methyl-4-p-tolyl-1,2,3,4-tetrahydro-isoquinoline;
2-Benzyl-7-[3-(4-fluoro-piperidin-1-yl)-propoxy]-4-(4-methoxy-phenyl)-1,2,3,4-tetrahydro-isoquinoline;
7-[3-(4-Fluoro-piperidin-1-yl)-propoxy]-2-methyl-4-(3-trifluoromethoxy-phenyl)-1,2,3,4-tetrahydro-isoquinoline;
7-[3-(3,3-Difluoro-pyrrolidin-1-yl)-propoxy]-4-(4-methoxy-phenyl)-2-methyl-1,2,3,4-tetrahydro-isoquinoline;
4-(4-Methoxy-phenyl)-2-methyl-7-[3-(3S-methyl-morpholin-4-yl)-propoxy]-1,2,3,4-tetrahydro-isoquinoline;
Dicyclopropylmethyl-{3-[4-(4-methoxy-phenyl)-2-methyl-1,2,3,4-tetrahydro-isoquinolin-7-yloxy]-propyl}-amine;
4-(2-Chloro-phenyl)-7-[3-(4-fluoro-piperidin-1-yl)-propoxy]-2-methyl-1,2,3,4-tetrahydro-isoquinoline;
2-Methyl-7-[3-(3S-methyl-morpholin-4-yl)-propoxy]-4-(4-morpholin-4-yl-phenyl)-1,2,3,4-tetrahydro-isoquinoline;
7-[3-(4-Fluoro-pipendin-1-yl)-propoxy]-2-methyl-4-(4-morpholin-4-yl-phenyl)-1,2,3,4-tetrahydro-isoquinoline;
4-{2-Methyl-7-[3-(3S-methyl-morpholin-4-yl)-propoxy]-1,2,3,4-tetrahydro-isoquinolin-4-yl}-benzonitrile;
4-{7-[3-(4-Fluoro-piperidin-1-yl)-propoxy]-2-methyl-1,2,3,4-tetrahydro-isoquinolin-4-yl}-benzonitrile;
7-[3-(3-Benzhydryl-azetidin-1-yl)-propoxy]-4-(4-methoxy-phenyl)-2-methyl-1,2,3,4-tetrahydro-isoquinoline;
2-Methyl-4-(4-methylsulfanyl-phenyl)-7-(3-morpholin-4-yl-propoxy)-1,2,3,4-tetrahydro-isoquinoline;
4-(2-Fluoro-4-methoxy-phenyl)-7-[3-(4-fluoro-piperidin-1-yl)-propoxy]-2-methyl-1,2,3,4-tetrahydro-isoquinoline (racemate);
4-(2-Fluoro-4-methoxy-phenyl)-7-[3-(4-fluoro-piperidin-1-yl)-propoxy]-2-methyl-1,2,3,4-tetrahydro-isoquinoline (enantiomer 1);
4-(2-Fluoro-4-methoxy-phenyl)-7-[3-(4-fluoro-piperidin-1-yl)-propoxy]-2-methyl-1,2,3,4-tetrahydro-isoquinoline (enantiomer 2);
4-(3-Fluoro-4-methoxy-phenyl)-7-[3-(4-fluoro-piperidin-1-yl)-propoxy]-2-methyl-1,2,3,4-tetrahydro-isoquirioline (racemate);
4-(3-Fluoro-4-methoxy-phenyl)-7-[3-(4-fluoro-piperidin-1-yl)-propoxy]-2-methyl-1,2,3,4-tetrahydro-isoquinoline (enantiomer 1);
4-(3-Fluoro-4-methoxy-phenyl)-7-[3-(4-fluoro-piperidin-1-yl)-propoxy]-2-methyl-1,2,3,4-tetrahydro-isoquinoline (enantiomer 2);
4-(4-Ethoxy-phenyl)-7-[3-(4-fluoro-piperidin-1-yl)-propoxy]-2-methyl-1,2,3,4-tetrahydro-isoquinoline;
2-Ethyl-7-[3-(4-fluoro-piperidin-1-yl)-propoxy]-4-(4-methoxy-phenyl)-1,2,3,4-tetrahydro-isoquinoline;
7-[3-(4-Fluoro-piperdin-1-yl)-propoxy]-4-(4-methoxy-phenyl)-1,2,3,4-tetrahydro-isoquinoline;
4-(4-Methoxy-ohenyl)-2-methyl-7-(piperidin-4-yloxy)-1,2,3,4-tetrahydro-isoquinoline;
2-Methyl-4-(4-methylsulfanyl-phenyl)-7-(piperidin-4-yloxy)-1,2,3,4-tetrahydro-isoquinoline;
4-(2-Fluoro-4-methoxy-phenyl)-2-methyl-7-(piperidin-4-yloxy)-1,2,3,4-tetrahydro-isoquinoline;
4-(3-Fluoro-4-methoxy-phenyl)-2-methyl-7-(pipendin-4-yloxy)-1,2,3,4-tetrahydro-isoquinoline;
7-[1-(2-Fluoro-ethyl)-piperidin-4-yloxy]-4-(4-methoxy-phenyl)-2-methyl-1,2,3,4-tetrahydro-isoquinoline;
7-[3-(4-Fluoro-piperidin-1-yl)-propoxy]-2-methyl-4-(4-methylsulfanyl-phenyl)-1,2,3,4-tetrahydro-isoquinoline (enantiomer 1);
7-[3-(4-Fluoro-piperidin-1-yl)-propoxy]-2-methyl-4-(4-methylsulfanyl-phenyl)-1,2,3,4-tetrahydro-isoquinoline (enantiomer 2);
4-(4-Methoxy-phenyl)-2-methyl-7-[3-(3S-methyl-morpholin-4-yl)-propoxy]-1,2,3,4-tetrahydro-isoquinoline (enantiomer 1);
4-(4-Methoxy-phenyl)-2-methyl-7-[3-(3S-methyl-morpholin-4-yl)-propoxy]-1,2,3,4-tetrahydro-isoquinoline (enantiomer 2);
2-Methyl-4-(4-methylsulfanyl-phenyl)-7-(3-morpholin-4-yl-propoxy)-1,2,3,4-tetrahydro-isoquinoline (enantiomer 1);
2-Methyl-4-(4-methylsulfanyl-phenyl)-7-(3-morpholin-4-yl-propoxy)-1,2,3,4-tetrahydro-isoquinoline (enantiomer 2);
7-[3-(4-Fluoro-piperidin-1-yl)-propoxy]-4-(4-methylsulfanyl-phenyl)-1,2,3,4-tetrahydro-isoquinoline;
4-(4-Methylsulfanyl-phenyl)-7-(3-morpholin-4-yl-propoxy)-1,2,3,4-tetrahydro-isoquinoline;
7-[3-(3S-Methyl-morpholin-4-yl)-propoxy]-4-(4-methylsulfanyl-phenyl)-1,2,3,4-tetrahydro-isoquinoline;
4-(4-Methoxy-phenyl)-7-(3-morpholin-4-yl-propoxy)-1,2,3,4-tetrahydro-isoquinoline;
7-[3-(4-Fluoro-pipendin-1-yl)-propoxy]-4-(4-methanesullfinyl-phenyl)-2-methyl-1,2,3,4-tetrahydro-isoquinoline;
4-(4-Methanesulfinyl-phenyl)-2-methyl-7-(3-morpholin-4-yl-propoxy)-1,2,3,4-tetrahydro-isoquinoline;
4-(4-Methanesulfinyl-phenyl)-2-methyl-7-(3-morpholin-4-yl-propoxy)-1,2,3,4-tetrahydro-isoquinoline (enantiomer 1);
4-(4-Methanesulfonyl-phenyl)-2-methyl-7-(3-morpholin-4-yl-propoxy)-1,2,3,4-tetrahydro-isoquinoline;
7-[3-(4-Fluoro-piperidin-1-yl)-propoxy]-4-(4-methoxy-phenyl)-2,6-dimethyl-1,2,3,4-tetrahydro-isoquinoline;
7-[3-(4-Fluoro-piperidin-1-yl)-propoxy]-4-(4-methoxy-phenyl)-2,8-dimethyl-1,2,3,4-tetrahydro-isoquinoline;
7-[3-(4-Fluoro-piperidin-1-yl)-propoxy]-4-(4-methoxy-phenyl)-2-methyl-1,2,3,4-tetrahydro-isoquinolin-6-ol;
7-[3-(4-Fluoro-piperidin-1-yl)-propoxy]-4-(4-methoxy-phenyl)-2-methyl-1,2,3,4-tetrahydro-isoquinolin-8-ol;
2,8-Dimethyl-4-(4-methylsulfanyl-phenyl)-7-(3-morpholin-4-yl-oropoxy)-1,2,3,4-tetrahydro-isoquinoline;
2,6-Dimethyl-4-(4-methylsulfanyl-phenyl)-7-(3-morpholin-4-yl-propoxy)-1,2,3,4-tetrahydro-isoquinoline;
2-Methyl-4-(4-methylsulfanyl-phenyl)-7-(3-morpholin-4-yl-propoxy)-1,2,3,4-tetrahydro-isoquinolin-8-ol;
2-Methyl-4-(4-methylsulfanyl-phenyl)-7-(3-morpholin-4-yl-propoxy)-1,2,3,4-tetrahydro-isoquinolin-6-ol;
8-Fluoro-2-methyl-4-(4-methylsulfanyl-phenyl)-7-(3-morpholin-4-yl-propoxy)-1,2,3,4-tetrahydro-isoquinoline;
6-Fluoro-2-methyl-4-(4-methylsulfanyl-phenyl)-7-(3-morpholin-4-yl-propoxy)-1,2,3,4-tetrahydro-isoquinoline;
7-[1-(2-Fluoro-ethyl)-piperidin-4-ylmethoxy]-2-methyl-4-(4-methylsulfanyl-phenyl)-1,2,3,4-tetrahydro-isoquinoline;
7-[1-(2-Fluoro-ethyl)-piperidin-4-yloxy]-2-methyl-4-(4-methylsulfanyl-phenyl)-1,2,3,4-tetrahydro-isoquinoline;
4-(4-Methoxy-phenyl)-7--[3-(3S-methyl-morpholin-4-yl)-propoxy]-1,2,3,4-tetrahydro-isoquinoline;
4-(4-Methanesulfinyl-phenyl)-2-methyl-7-[3-(3S-methyl-morpholin-4-yl)-propoxy]-1,2,3,4-tetrahydro-isoquinoline;
7-[3-(3,3-Difluoro-azetidin-1-yl)-oropoxy]-4-(4-methoxy-phenyl)-2-methyl-1,2,3,4-tetrahydro-isoquinoline;
(4-{3-[4-(4-Methoxy-phenyl)-2-methyl-1,2,3,4-tetrahydro-isoquinolin-7-yloxy]-propyl}-morpholin-3-yl)-methanol;
(4-{3-[4-(4-Methoxy-phenyl)-2-methyl-1,2,3,4-tetrahydro-isoquinolin-7-yloxy]-propyl}-morpholin-3S-yl)-methariol;
(4-{3-[4-(4-Methoxy-phenyl)-2-methyl-1,2,3,4-tetrahydro-isoquinolin-7-yloxy]-propyl}-morpholin-2-yl)-methanol;
{3-[2-Methyl-4-(4-methylsulfanyl-phenyl)-1,2,3,4-tetrahydro-isoquinolin-7-yloxy]-propyl}-(2H-pyrazol-3-yl)-amine;
4-(6-Bromo-pyridin-3-yl)-7-[3-(4-fluoro-piperidin-1-yl)-propoxy]-2-methyl-1,2,3,4-tetrahydro-isoquinoline;
7-[3-(4-Fluoro-piperidin-1-yl)-propoxy]-2-methyl-4-(6-methylsulfanyl-pyridin-3-yl)-1,2,3,4-tetrahydro-isoquinoline;
(5-{7-[3-(4-Fluoro-piperidin-1-yl)-propoxy]-2-methyl-1,2,3,4-tetrahydro-isoquinolin-4-yl}-pyridin-2-yl)-dimethyl-amine;
7-[3-(4-Fluoro-piperidin-1-yl)-propoxy]-2-methyl-4-(6-trimthylsilanylethynyl-pyridin-3-yl)-1,2,3,4-tetrahydro-isoquinoline;
4-(6-Ethynyl-pyridin-3-yl)-7-[3-(4-fluoro-piperidin-1-yl)-propoxy]-2-methyl-1,2,3,4-tetrahydro-isoquinoline;
7-[3-(4-Fluoro-piperidin-1-yl)-propoxy]-2-methyl-4-(6-phenylsulfanyl-pyridin-3-yl)-1,2,3,4-tetrahydro-isoquinoline;
7-[3-(4-Fluoro-piperidin-1-yl)-propoxy]-4-(6-imidazol-1-yl-pyridin-3-yl)-2-methyl-1,2,3,4-tetrahydro-isoquinoline;
7-[3-(4-Fluoro-piperidin-1-yl)-propoxy]-4-(6-methoxy-pyridin-3-yl)-2-methyl-1,2,3,4-tetrahydro-isoquinoline;
7-[3-(4-Fluoro-piperidin-1-yl)-propoxy]-2-methyl-4-(6-pyrazol-1-yl-pyridin-3-yl)-1,2,3,4-tetrahydro-isoquinoline;
7-[3-(4-Fluoro-piperidin-1-yl)-propoxy]-4-[6-(1H-imidazol-2-ylsulfanyl)-pyridin-3-yl]-2-methyl-1,2,3,4-tetrahydro-isoquinoline;
7-[3-(4-Ftuoro-pipendin-1-yl)-propoxy]-2-methyl-4-[6-(pyrimidin-2-ylsulfanyl)-pyridin-3-yl]-1,2,3,4-tetrahydro-isoquinoline;
4-(4-Methoxy-phenyl)-2-methyl-7-[3-(4-methyl-piperazin-1-yl)-propoxy]-1,2,3,4-tetrahydro-isoquinoline;
7-[3-(4-Ethyl-piperazin-1-yl)-propoxy]-4-(4-methoxy-phenyl)-2-methyl-1,2,3,4-tetrahydro-isoquinoline;
7-[3-(4-Isopropyl-piperazin-1-yl)-propoxy]-4-(4-methoxy-phenyl)-2-methyl-1,2,3,4-tetrahydro-isoquinoline;
4-(4-Methoxy-phenyl)-2-methyl-7-[3-(4-thiazol-2-yl-piperazin-1-yl)-propoxy]-1,2,3,4-tetrahydro-isoquinoline;
4-(4-Methoxy-phenyl)-2-methyl-7-[3-(4-thiophen-2-ylmethyl-piperazin-1-yl)-propoxy]-1,2,3,4-tetrahydro-isoquinoline;
2-(4-{3-[4-(4-Methoxy-phenyl)-2-methyl-1,2,3,4-tetrahydro-isoquinolin-7-yloxy]-propyl}-piperazin-1-yl)-ethanol;
2-(4-{3-[4-(4-Methoxy-phenyl)-2-methyl-1,2,3,4-tetrahydro-isoquinolin-7-yloxy]-propyl}-piperazin-1-yl)-phenol;
4-(4-Methoxy-phenyl)-2-methyl-7-[3-(4-pyridin-4-yl-piperazin-1-yl)-propoxy]-1,2,3,4-tetrahydro-isoquinoline;
4-(4-Methoxy-phenyl)-2-methyl-7-{3-[4-(4-trifluoromethyl-phenyl)-piperazin-1-yl]-propoxy}-1,2,3,4-tetrahydro-isoquinoline;
7-[3-(4-Allyl-piperazin-1-yl)-propoxy]-4-(4-methoxy-phenyl)-2-methyl-1,2,3,4-tetrahydro-isoquiuoline;
7-[3-(4-[1,3]-Dioxolan-2-ylmethyl-piperazin-1-yl)-propoxy]-4-(4-methoxy-phenyl)-2-methyl-1,2,3,4-tetrahydro-isoquinoline;
4-(4-{3-[4-(4-Methoxy-phenyl)-2-methyl-1,2,3,4-tetrahydro-isoquinolin-7-yloxy]-propyl}-piperazin-1-yl)-benzonitrile;
7-{3-[4-(2-Methoxy-ethyl)-piperazin-1-yl]-propoxy}-4-(4-methoxy-phenyl)-2-methyl-1,2,3,4-tetrahydro-isoquinoline;
4-(4-Methoxy-phenyl)-2-methyl-7-{3-[4-(tetrahydro-furan-2-ylmethyl)-piperazin-1-yl]-propoxy}-1,2,3,4-tetrahydro-isoquinoline;
4-{3-[4-(4-Methoxy-phenyl)-2-methyl-1,2,3,4-tetrahydro-isoquinolin-7-yloxy]-propyl}-piperazine-1-carboxylic acid tert-butyl ester;
7-[3-(4-Cyclopropyl-piperazin-1-yl)-propoxy]-4-(4-methoxy-phenyl)-2-methyl-1,2,3,4-tetrahydro-isoquinoline;
4-(4-Bromo-phenyl)-7-[3-(4-fluoro-piperidin-1-yl)-propoxy]-2-methyl-1,2,3,4-tetrahydro-isoquinoline;
4-(4-Difluoromethoxy-phenyl)-2-methyl-7-(3-morpholin-4-yl-propoxy)-1,2,3,4-tetrahydro-isoquinoline;
2-Methyl-7-[3-(3S-methyl-morpholin-4-yl)-propoxy]-4-(4-methylsulfanyl-phenyl)-1,2,3,4-tetrahydro-isoquinoline;
7-[3-(4,4-Difluoro-piperidin-1-yl)-propoxy]-2-methyl-4-(4-methylsulfanyl-phenyl)-1,2,3,4-tetrahydro-isoquinoline;
7-[3-(4-Fluoro-piperidin-1-yl)-propoxy]-2-methyl-4-(4-trifluoromethylsulfanyl-phenyl)-1,2,3,4-tetrahydro-isoquinoline;
2-Methyl-7-(3-morpholin-4-yl-propoxy)-4-(4-trifluoromethylsulfanyl-phenyl)-1,2,3,4-tetrahydro-isoquinoline;
2-Methyl-7-[3-(3S-methyl-morpholin-4-yl)-propoxy]-4-(4-trifluoromethylsulfanyl-phenyl)-1,2,3,4-tetrahydro-isoquinoline;
4-(2-Fluoro-4-methoxy-phenyl)-2-methyl-7-[3-(3S-methyl-morpholin-4-yl)-propoxy]-1,2,3,4-tetrahydro-isoquinoline;
4-(3-Fluoro-4-methoxy-phenyl)-2-methyl-7-[3-(3S-methyl-morpholin-4-yl)-propoxy]-1,2,3,4-tetrahydro-isoquinoline;
2-Methyl-7-(3-piperidin-1-yl-propoxy)-4-(4-trifluoromethoxy-phenyl)-1,2,3,4-tetrahydro-isoquinoline;
{3-[4-(4-Methoxy-phenyl)-2-methyl-1,2,3,4-tetrahydro-isoquinolin-7-yloxy]-propyl}-(tetrahydro-pyran-4-yl)-amine;
Cyclopropyl-{3-[4-(4-methoxy-phenyl)-2-methyl-1,2,3,4-tetrahydro-isoquinolin-7-yloxy]-propyl}-(1-methyl-piperidin-4-yl)-amine;
(2-Methoxy-ethyl)-{3-[4-(4-methoxy-phenyl)-2-methyl-1,2,3,4-tetrahydro-isoquinolin-7-yloxy]-propyl}-amine;
3-{3-[4-(4-Methoxy-phenyl)-2-methyl-1,2,3,4-tetrahydro-isoquinoin-7-yloxy]-propylamino}-propan-1-ol;
Allyl-{3-[4-(4-methoxy-phenyl)-2-methyl-1,2,3,4-tetrahydro-isoquinolin-7-yloxy]-propyl}-amine;
Isobutyl-{3-[4-(4-methoxy-phenyl)-2-methyl-1,2,3,4-tetrahydro-isoquinolin-7-yloxy]-propyl}-amine;
Cyclopropyl-{3-[4-(4-methoxy-phenyl)-2-methyl-1,2,3,4-tetrahydro-isoquinolin-7-yloxy]-propyl}-amine;
Isopropyl-{3-[4-(4-methoxy-phenyl)-2-methyl-1,2,3,4-tetrahydro-isoquinolin-7-yloxy]-propyl}-amine;
{3-[4-(4-Methoxy-phenyl)-2-methyl-1,2,3,4-tetrahydro-isoquinolin-7-yloxy]-propyl}-propyl-amine;
Ethyl-{3-[4-(4-methoxy-phenyl)-2-methyl-1,2,3,4-tetrahydro-isoquinolin-7-yloxy]-propyl}-amine;
Isopropyl-{3-[4-(4-methoxy-phenyl)-2-methyl-1,2,3,4-tetrahydro-isoquinolin-7-yloxy]-propyl}-methyl-amine;
Cyclopropyl-{3-[4-(4-methoxy-phenyl)-2-methyl-1,2,3,4-tetrahydro-isoquinolin-7-yloxy]-propyl}-methyl-amine;
Dicyclopropyl-{3-[4-(4-methoxy-phenyl)-2-methyl-1,2,3,4-tetrahydro-isoquinolin-7-yloxy]-propyl}-amine;
7-(1-Isopropyl-azetidin-3-ylmethoxy)-2-methyl-4-(4-methylsulfanyl-phenyl)-1,2,3,4-tetrahydro-isoquinoline;
7-(1-Cyclopropyl-azetidin-3-ylmethoxy)-2-methyl-4-(4-methylsulfanyl-phenyl)-1,2,3,4-tetrahydro-isoquinoline;
7-(1-Cyclobutyl-azetidin-3-ylmethoxy)-2-methyl-4-(4-methylsulfanyl-phenyl)-1,2,3,4-tetrahydro-isoquinoline;
7-[1-(2-Fluoro-ethyl)-azetidin-3-ylmethoxy]-2-methyl-4-(4-methylsulfanyl-phenyl)-1,2,3,4-tetrahydro-isoquinoline;
4-(4-Methoxy-phenyl)-2-methyl-7-[2-(1-methyl-pyrrolidin-2-yl)-ethoxy]-1,2,3,4-tetrahydro-isoquinoline;
4-(4-Methoxy-phenyl)-2-methyl-7-[2-(1-methyl-pyrrolidin-2-yl)-ethoxy]-1,2,3,4-tetrahydro-isoquinoline (diastereomer 1);
4-(4-Methoxy-phenyl)-2-methyl-7-[2-(1-methyl-pyrrolidin-2-yl)-ethoxy]-1,2,3,4-tetrahydro-isoquinoline (diastereomer 2);
4-(3-Methoxy-phenyl)-2-methyl-7-(piperidin-4-ylmethoxy)-1,2,3,4-tetrahydro-isoquinoline;
{4-[4-(4-Methoxy-phenyl)-2-methyl-1,2,3,4-tetrahydro-isoquinolin-7-yloxymethyl]-piperidin-1-yl}-acetonitrile;
2-Methyl-7-(1-methyl-piperidin-4-yloxy)-4-(4-methylsulfanyl-phenyl)-1,2,3,4-tetrahydro-isoquinoline;
2-Methyl-4-(4-methylsulfanyl-phenyl)-7-[1-(3,3,3-trifluoro-propyl)-piperidin-4-yloxy]-1,2,3,4-tetrahydro-isoquinoline;
{4-[2-Methyl-4-(4-methylsulfanyl-phenyl)-1,2,3,4-tetrahydro-isoquinolin-7-yloxy]-piperidin-1-yl}-acetonitrile;
2-Methyl-4-(4-methylsulfanyl-phenyl)-7-[1-(tetrahydro-pyran-4-yl)-piperidin-4-yloxy]-1,2,3,4-tetrahydro-isoquinoline;
7-(1-Cyclopropyl-piperidin-4-yloxy)-2-methyl-4-(4-methylsulfanyl-phenyl)-1,2,3,4-tetrahydro-isoquinoline;
7-(1-Cyclobutyl-piperidin-4-ylmethoxy)-4-(4-methoxy-phenyl)-2-methyl-1,2,3,4-tetrahydro-isoquinoline;
7-(1-Cyclopropyl-piperidin-4-ylmethoxy)-4-(4-methoxy-phenyl)-2-methyl-1,2,3,4-tetrahydro-isoquinoline;
1-{4-[4-(4-Methoxy-phenyl)-2-methyl-1,2,3,4-tetrahydro-isoquiriolin-7-yloxymethyl]-piperidin-1-yl}-2-methyl-propan-2-ol;
4-(4-Methoxy-phenyl)-2-methyl-7-(4-methyl-morpholin-2-ylmethoxy)-1,2,3,4-tetrahydro-isoquinoline;
4-(4-Methoxy-phenyl)-2-methyl-7-(morpholin-2S-ylmethoxy)-1,2,3,4-tetrahydro-isoquinoline;
4-(4-Methoxy-phenyl)-2-methyl-7-(morpholin-2R-ylmethoxy)-1,2,3,4-tetrahydro-isoquinoline;
7-(4-Isopropyl-morpholin-2-ylmethoxy)-4-(4-methoxy-phenyl)-2-methyl-1,2,3,4-tetrahydro-isoquinoline;
7-(4-Isopropyl-morpholin-2S-ylmethoxy)-4-(4-methoxy-phenyl)-2-methyl-1,2,3,4-tetrahydro-isoquinoline;
7-(4-Isopropyl-morpholin-2R-ylmethoxy)-4-(4-methoxy-phenyl)-2-methyl-1,2,3,4-tetrahydro-isoquinoline;
7-(4-Isopropyl-morpholin-2R-ylmethoxy)-4-(4-methoxy-phenyl)-2-methyl-1,2,3,4-tetrahydro-isoquinoline (diastereomer 1);
7-(4-Isopropyl-morpholin-2R-ylmethoxy)-4-(4-methoxy-phenyl)-2-methyl-1,2,3;4-tetrahydro-isoquinoline (diastereomer 2);
7-(4-Ethyl-morpholin-2-ylmethoxy)-4-(4-methoxy-phenyl)-2-methyl-1,2,3,4-tetrahydro-isoquinoline;
7-[4-(2-Fluoro-ethyl)-morpholin-2-ylmethoxy]-4-(4-methoxy-phenyl)-2-methyl-1,2,3,4-tetrahydro-isoquinoline;
7-(4-Cyclopropyl-morpholin-2-ylmethoxy)-4-(4-methoxy-phenyl)-2-methyl-1,2,3,4-tetrahydro-isoquinoline;
7-(4-Cyclopropyl-morpholin-2S-ylmethoxy)-4-(4-methoxy-phenyl)-2-methyl-1,2,3,4-tetrahydro-isoquinoline;
7-(4-Cyclopropyl-morpholin-2S-ylmethoxy)-4-(4-methoxy-phenyl)-2-methyl-1,2,3,4-tetrahydro-isoquinoline (diastereomer 1);
7-(4-Cycloprooyl-morpholin-2S-ylmethoxy)-4-(4-methoxy-phenyl)-2-methyl-1,2,3,4-tetrahydro-isoquinoline (diastereomer 2);
7-(4-Cyclopropyl-morpholin-2R-ylmethoxy)-4-(4-methoxy-phenyl)-2-methyl-1,2,3,4-tetrahydro-isoquinoline;
7-(4-Cyclopropyl-morpholin-2R-ylmethoxy)-4-(4-methoxy-phenyl)-2-methyl-1,2,3,4-tetrahydro-isoquinoline (diastereomer 1);
7-(4-Cyclopropyl-morpholin-2R-ylmethoxy)-4-(4-methoxy-phenyl)-2-methyl-1,2,3,4-tetrahydro-isoquinoline (diastereomer 2);
7-(4-Isopropyl-morphplin-2-ylmethoxy)-2-methyl-4-(4-methylsulfanyl-phenyl)-1,2,3,4-tetrahydro-isoquinoline;
7-(4-Cyclopropyl-morpholin-2-ylmethoxy)-2-methyl-4-(4-methylsulfanyl-phenyl)-1,2,3,4-tetrahydro-isoquinoline;
2-Methyl-4-(4-methylsulfanyl-phenyl)-7-(morpholin-2-ylmethoxy)-1,2,3,4-tetrahydro-isoquinoline;
4-(4-Methoxy-phenyl)-2,6-dimethyl-7-(3-piperidin-1-yl-propoxy)-1,2,3,4-tetrahydro-isoquinoline;
4-(4-Methoxy-phenyl)-2,6-dimethyl-7-(3-morpholin-4-yl-propoxy)-1,2,3,4-tetrahydro-isoquinoline;
4-(3-Fluoro-4-methoxy-phenyl)-2,6-dimethyl-7-(3-morpholin-4-yl-propoxy)-1,2,3,4-tetrahydro-isoquinoline;
4-(3-Fluoro-4-methoxy-phenyl)-2,8-dimethyl-7-(3-morpholin-4-yl-propoxy)-1,2,3,4-tetrahydro-isoquinoline;
4-(4-Methoxy-phenyl)-2,8-dimethyl-7-(3-morpholin-4-yl-propoxy)-1,2,3,4-tetrahydro-isoquinoline;
2,6-Dimethyl-4-(4-methylsulfanyl-phenyl)-7-(3-piperidin-1-yl-propoxy)-1,2,3,4-tetrahydro-isoquinoline;
7-(4-Piperidin-1-yl-but-1-ynyl)-4-pyridin-3-yl-1,2,3,4-tetrahydro-isoquinoline;
7-(4-Piperidin-1-yl-butyl)-4-pyridin-3-yl-1,2,3,4-tetrahydro-isoquinoline; 2-Methyl-7-(4-piperidin-1-yl-butyl)-4-pyridin-3-yl-1,2,3,4-tetrahydro-isoquinoline;
7-[4-(4,4-Difluoro-piperidin-1-yl)-but-1-ynyl]-4-(4-methoxy-phenyl)-2-methyl-1,2,3,4-tetrahydro-isoquinoline;
4-(4-Methoxy-phenyl)-2-methyl-7-(4-piperidin-1-yl-but-1-ynyl)-1,2,3,4-tetrahydro-isoquinoline;
4-(4-Methoxy-phenyl)-2-methyl-7-(4-morpholin-4-yl-but-1-ynyl)-1,2,3,4-tetrahydro-isoquinoline;
4-(4-Methoxy-phenyl)-2-methyl-7-(4-thiomorpholin-4-yl-but-1-ynyl)-1,2,3,4-tetrahydro-isoquinoline;
7-[4-(4-Isopropyl-piperazin-1-yl)-but-1-ynyl]-4-(4-methoxy-phenyl)-2-methyl-1,2,3,4-tetrahydro-isoquinoline;
4-(4-Fluoro-phenyl)-2-methyl-7-(4-piperidin-1-yl-but-1-ynyl)-1,2,3,4-tetrahydro-isoquinoline;
7-[4-(4,4-Difluoro-piperidin-1-yl)-butyl]-4-(4-methoxy-phenyl)-2-methyl-1,2,3,4-tetrahydro-isoquinoline;
4-(4-Methoxy-phenyl)-2-methyl-7-(4-morpholin-4-yl-butyl)-1,2,3,4-tetrahydro-isoquinoline;
4-(4-Methoxy-phenyl)-2-methyl-7-(4-thiomorpholin-4-yl-butyl)-1,2,3,4-tetrahydro-isoquinoline;
7-[4-(4-Isopropyl-piperazin-1-yl)-butyl]-4-(4-methoxy-phenyl)-2-methyl-1,2,3,4-tetrahydro-isoquinoline;
and salts thereof.

10. A compound of formula (I): 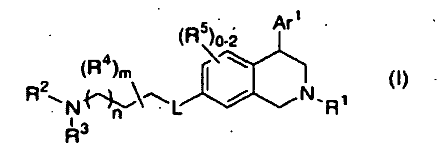 wherein
L is -O- and n is 1 or 2; or L is -C≡C- or -CH₂CH₂- and n is 0 or 1;
R¹ is -H; or is -C₁₋₆alkyl, -C₃₋₆alkenyl, -C₃₋₆alkynyl, -C₃₋₇Cycloalkyl, -C₁₋₆alkylC₃₋₇Cycloalkyl, -COOC₁₋₆alkyl, or -COObenzyl, each optionally mono-, di-, or tri-substituted with R^{a};
where R³ is selected from -OH, -OC₁₋₆alkyl, phenyl optionally substituted with -OC₁₋₄alkyl or halo, -CN, -NO₂, -N(R^{b})R^{c}, -C(O)N(R^{b})R^{c}. -N(R^{b})C(O)R^{b}, -N(R^{b})SO₂C₁₋₆alkyl, -C(O)C₁₋₆alkyl, -S(O)₀₋₂-C₁₋₆alkyl, -SO₂N(R^{b})R^{c}, -SCF₃, halo, -CF₃, -OCF₃, -COOH, and -COOC₁₋₆alkyl;
wherein R^{b} and R^{c} are each independently -H or -C₁₋₆alkyl;
R² and R³ are each independently selected from the group consisting of: -H;
A) -C₁₋₆alkyl, -C₃₋₆alkenyl, -C₃₋₆alkynyl, -C₃₋₇cycloalkyl, -C₁₋₆alkylC₃₋₇Cycloalkyl, -CH(C₃₋₈cycloalkyl)₂; -CH(phenyl)₂; benzyl, and -C(O)OC₁₋₄alkyl, wherein each alkyl, cycloalkyl, or benzyl is optionally substituted with -OH, -OC₁₋₄alkyl, -CN, -NH₂, -NH(C₁₋₄alkyl), -N(C₁₋₄alkyl)₂, halo, -CF₃, -OCF₃, -COOH, or -COOC₁₋₆alkyl,
B) phenyl or pyridyl, optionally fused at two adjacent carbon ring members to a three or four-membered hydrocarbon moiety to form a fused five- or six-membered aromatic ring, which moiety has one carbon atom replaced by >O, >S, >NH, >N(C₁₋₄alkyl), or >NC(O)OC₁₋₄alkyl, and which moiety has up to one additional carbon atom optionally replaced by -N=;
C) naphthyl,
D) a 4-8 membered heterocyclic ring, said heterocyclic ring having a carbon atom which is the point of attachment, having 1 or 2 heteroatom members selected from the group consisting of >O, >S(O)₀₋₂, >NH, >N(C₁₋₄alkyl), and >NC(O)OC₁₋₄alkyl, and having 0 or 1 double bonds; and
E) a monocyclic aromatic hydrocarbon group having five or six ring atoms, having a carbon atom which is the point of attachment, having one carbon atom replaced by >O, >S, >NH, >N(C₁₋₄alkyl), or >NC(O)OC₁₋₄alkyl, having up to one additional carbon atom optionally replaced by -N=, and optionally benzofused or pyridofused;
where each of B)-E) are optionally mono-, di-, or tri-substituted with a moiety selected from the group consisting of -C₁₋₄alkyl, -OH, -C₁₋₄alkylOH, -OC₁₋₆alkyl, -CN, -NO₂, -N(R^{d})R^{e} -C(O)N(R^{d})R^{e}, -N(R^{d})C(O)R^{d}, -N(R^{d})SO₂C₁₋₆alkyl, -C(O)C₁₋₆alkyl, -S(O)₀₋₂-C₁₋₆alkyl, -SO₂N(R^{d})R^{e}, -SCF₃, halo, -CF₃, -OCF₃, -COOH, -COOC₁₋₆alkyl, -OC(O)N(R^{d})R^{e}, and -OC(O)OC₁₋₆alkyl;
where R^{d} and R^{e} are each independently -H or -C₁₋₆alkyl;
or, alternatively,
R² and R³ may be taken together with the nitrogen to which they are attached to form tetrahydro-pyrimidin-2-ylidenyl, or a 4-8 membered heterocyclic ring, said heterocyclic ring having 0 or 1 additional heteroatom members separated from the nitrogen of attachment by at least one carbon member and selected from the group consisting of >O, >S(O)₀₋₂, >NH, and >NR^{f}, having 0 or 1 double bonds, having 0, 1 or 2 carbon members separated from the nitrogen of attachment by at least one carbon member which is a carbonyl, optionally benzo or pyrido fused, optionally having one carbon member that forms a bridge, and having 0-5 carbon member substituents R^{ff},
where R^{f} is selected from the group consisting of:
i) -C₁₋₆alkyl optionally substituted with R^{z}, -C₃₋₆alkenyl, -C₃₋₆alkynyl, -C(O)N(R^{g})R^{h}, -C(O)Rⁱ, -S(O)₀₋₂-C₁₋₆alkyl, and -COOC₁₋₆alkyl;
where R^{z} is fluoro, -CN, -OH, -OC₁₋₄alkyl, -C₃₋₇Cycloalkyl, or -CF₃;
where R^{g} and R^{h} are each independently -H or -C₁₋₆alkyl; and
where Rⁱ is -C₁₋₆alkyl, -C₃₋₈cycloalkl, phenyl, or 5- or 6-membered aromatic heterocyclyl, where each alkyl, cycloalkyl, phenyl or heterocyclyl is optionally mono-, di-, or tri-substituted with -C₁₋₄alkyl, -OH, -OC₁₋₆alkyl, -CF₃, -CN, or halo;
ii) -(CH₂)₀₋₁-Ring A, where Ring A is phenyl or a 5- or 6-membered carbon-linked aromatic heterocyclyl, optionally substituted with R^{aa};
where R^{aa} is -OH, -C₁₋₆alkyl, -OC₁₋₆alkyl, phenyl, -CN, -NO₂, -N(R^{g})R^{h} -C(O)N(R^{g})R^{h}, -N(R^{g})C(O)R^{h}, -N(R^{h})SO₂C₁₋₆alkyl, -C(O)C₁₋₆alkyl, -S(O)₀₋₂-C₁₋₆alkyl, -SO₂N(R^{g})R^{h}, -SCF₃, halo, -CF₃, -OCF₃, -OCHF₂, -COOH, and -COOC₁₋₆alkyl; and
iii) -(CH₂)₀₋₁-RingB, where Ring B is -C₃₋₇cycloalkyl with one or two carbon ring members optionally replaced with >O, or >NH, and optionally substituted with -C₁₋₄alkyl, fluoro, -CN, -OH, -OC₁₋₄alkyl, or -CF₃;
where R^{ff} is selected from the group consisting of -C₁₋₆alkyl optionally mono-or di-substituted with R^{z}, -C₂₋₆alkenyl, -C₂₋₆lkynyl, -C₃₋₇Cyclcoalkyl, -CH(phenyl)₂, halo, -OH, -OC₁₋₆alkyl, -OC₂₋₃alkylO-, -CN, -NO₂, -N(R^{g})R^{h}, -C(O)N(R^{g})R^{h}, -N(R^{g})C(O)R^{g}, -N(R^{g})SO₂C₁₋₆alkyl, -C(O)Rⁱ, -S(O)₀₋₂-C₁₋₆alkyl, -SO₂N(R^{g})R^{h}, -SCF₃, -CF₃, -OCF₃, -COOH, and -COOC₁₋₆alkyl;
R⁴ is -OH, -OC₁₋₆alkyl, -CF₃, -C₁₋₆alkyl, or halo; two R⁴ substituents may be taken together to form methylene or ethylene; or one of R⁴ is taken together with R² to form methylene, ethylene, propylene, or -CH₂CH₂O-, wherein each is optionally substituted with -OH, -OC₁₋₆alkyl, -SC₁₋₆alkyl, -CF₃, -C₁₋₆alkyl, amino, or halo;
m is 0, 1, or 2;
R⁵ is selected from the group consisting of -C₁₋₆alkyl, -OH, -OC₁₋₆alkyl, -SC₁₋₆alkyl, and halo;
Ar¹ is an aryl or heteroaryl ring selected from the group consisting of:
a) phenyl, optionally mono-, di-, or tri-substituted with R^{j} and optionally di-subsfituted on adjacent carbons with -OC₁₋₄alkyleneO- optionally mono-or di-substituted with fluoro, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
where R^{j} is selected from the group consisting of
1) -OH, -C₁₋₆alkyl, -OC₁₋₆alkyl optionally mono-, di-, or tri-substituted with halo, -C₂₋₆alkenyl, -OC₃₋₆alkenyl, -C₂₋₆alkynyl optionally substituted with trimethylsilyl, -OC₃₋₆alkynyl, -C₃₋₆cycloalkyl, -OC₃₋₆cycloalkyl, -CN, -NO₂, -N(R^{k})R^{l}, -N(R^{k})C(O)R^{l}, -N(R^{k})SO₂C₁₋₆alkyl, -C(O)C₁-₆alkyl, -S(O)₀₋₂-C₁₋₆alkyl, -C(O)N(R^{m})Rⁿ, -SO₂N(R^{m})Rⁿ, -SCF₃, halo, -CF₃, -COOH, -COOC₁-₆alkyl, and -COOC₃₋₇cycloalkyl;
where R^{k} and R^{l} are each independently -H or -C₁₋₆alkyl;
where R^{m} and Rⁿ are each independently -H or -C₁₋₆alkyl, or R^{m} and Rⁿ taken together with their nitrogen of attachment form a 4-8 membered heterocyclic ring having 1 or 2 heteroatom members selected from >O, >S(O)₀₋₂, >NH, and >NC₁₋₆alkyl, having 0 or 1 double bonds, having 0 or 1 carbonyl members;
2) -G-Ar², where G is a bond, -O-, or -S-, and Ar² is phenyl or is a monocyclic aromatic hydrocarbon group having five or six ring atoms, having one carbon atom replaced by >O, >S, >NH, or >N(C₁₋₄alkyl), having up to one additional carbon atom optionally replaced by -N=, each optionally mono-, di-, or tri-substituted with R^{p};
where R^{p} is a substituent independently selected from the group consisting of: -OH, -C₁₋₆alkyl, -OC₁₋₆alkyl, phenyl, -CN, -NO₂, -N(R^{q})R^{r}, -C(O)N(R^{q})R^{r}, -N(R^{q})C(O)R^{r}, -N(R^{q})SO₂C₁₋₆alkyl, -C(O)C₁₋₆alkyl, -S(O)₀₋₂-C₁₋₆alkyl, -SO₂N(R^{q})R^{r}, -SCF₃, halo, -CF₃, -OCF₃, -OCHF₂, -COOH, and -COOC₁₋₆alkyl;
wherein R^{q} and R^{r} are each independently selected from -H, -C₁₋₆alkyl, and -C₂₋₆alkenyl; and
3) a 4-8 membered saturated or partially saturated heterocyclic ring, having 1 or 2 heteroatom members selected from >O, >S(O)₀₋₂, >NH, and >NC₁₋₆alkyl, having 0 or 1 carbonyl members, said ring optionally mono-, di-, or tri-substituted with R^{p};
b) phenyl or pyridyl fused at two adjacent carbon ring members to a three membered hydrocarbon moiety to form a fused five membered aromatic ring, which moiety has one carbon atom replaced by >O, >S, >NH, or >N(C₁₋₄alkyl), and which moiety has up to one additional carbon atom optionally replaced by -N=, the fused rings optionally mono-, di-, or tri-substituted with R^{t};
where R^{f} is a substituent independently selected from the group consisting of: -OH, -C₁₋₆alkyl, -OC₁₋₆alkyl, phenyl, -CN, -NO₂, -N(R^{u})R^{v}, -C(O)N(R^{u})R^{v}, -N(R^{u})C(O)R^{v}, -N(R^{u})SO₂C₁₋₆alkyl, -C(O)C₁₋₆alkyl, -S(O)₀₋₂-C₁₋₆-salkyl, -SO₂N(R^{u})R^{v}, -SCF₃, halo, -CF₃, -OCF₃, -OCHF₂, -COOH, and -COOC₁₋₆alkyl;
where R^{u} and R^{v} are each independently -H or -C₁₋₆alkyl;
c) phenyl fused at two adjacent ring members to a four membered hydrocarbon moiety to form a fused six membered aromatic ring, which moiety has one or two carbon atoms replaced by -N=, the fused rings optionally mono-, di-, or tri-substituted with R^{t};
d) naphthyl, optionally mono-, di-, or tri-substituted with R^{t};
e) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atom optionally replaced by -N=, optionally mono- or di-substituted with R^{j} and optionally benzofused or pyridofused at two adjacent carbon atoms, where the benzofused or pyridofused moiety is optionally mono-, di-, or tri-substituted with R^{t}; and
f) a monocyclic aromatic hydrocarbon group having six ring atoms, having a carbon atom which is the point of attachment, having one or two carbon atoms replaced by -N=, optionally mono- or di-substituted with R^{j} and optionally benzofused or pyridofused at two adjacent carbon atoms, where the benzofused or pyridofused moiety is optionally mono- or di-substituted with R^{t}; and enantiomers, diasteromers, hydrates, solvates and pharmaceutically acceptable salts, esters and amides thereof with the proviso: when n is 1, L is -O-, Ar is unsubstituted phenyl and R² and R³ are both methyl, then R⁴ is not -OH of use in the treatment of a CNS disorder selected from the group consisting of: depression, disturbed sleep, fatigue, lethargy, cognitive impairment, memory impairment, memory loss, learning impairment, and attention-deficit disorders in mammals.

11. A compound of formula (II): 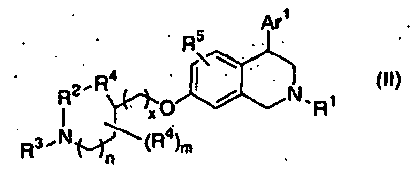 wherein
n is 1 or 2;
x is 0 or 1;
where n + x is 1 or 2;
R¹ is -H; or is -C₁₋₆alkyl, -C₃₋₆alkenyl, -C₃₋₆alkynyl, -C₃₋₇cycloalkyl, -C₁₋₆akylC₃₋₇cycloakyl, -COOC₁₋₆alkyl, or -COObenzyl, each optionally mono-, di-, or tri-substituted with R^{a};
where R^{a} is selected from -OH, -OC₁₋₆alkyl, phenyl optionally substituted with -OC₁₋₄alkyl or halo, -CN, -NO₂, -N(R^{b})R^{c}, -C(O)N(R^{b})R^{c}, -N(R^{b})C(O)R^{b}, -N(R^{b})SO₂C₁₋₆alkyl, -C(O)C₁₋₆alkyl, -S(O)₀₋₂-C₁₋₆alkyl, -SO₂N(R^{b})R^{c}, -SCF₃, halo, -CF₃, -OCF₃, -COOH, and -COOC₁₋₆alkyl;
wherein R^{b} and R^{c} are each independently -H or -C₁₋₆alkyl;
R³ is selected from the group consisting of: -H;
A) -C₁₋₆alkyl, -C₃₋₆alkenyl, -C₃₋₆alkynyl, -C₃₋₇cycloalkyl, -C₁₋₆alkylC₃₋₇cycloalkyl, -CH(C₃₋₈cycloalkyl)₂, -CH(phenyl)₂, benzyl, and -C(O)OC₁₋₄alkyl, wherein each alkyl, cycloalkyl, or benzyl is optionally substituted with -OH, -OC₁₋₄alkyl, -CN, -NH₂, -NH(C₁₋₄alkyl), -N(C₁₋₄alkyl)₂, halo, -CF₃, -OCF₃, -COOH, or -COOC₁₋₆alkyl;
B) phenyl or pyridyl, optionally fused at two adjacent carbon ring members to a three- or four-membered hydrocarbon moiety to form a fused five- or six-membered aromatic ring, which moiety has one carbon atom replaced by >O, >S, >NH, >N(C₁₋₄alkyl), or >NC(O)OC₁₋₄alkyl, and which moiety has up to one additional carbon atom optionally replaced by -N=;
C) naphthyl,
D) a 4-8 membered heterocyclic ring, said heterocyclic ring having a carbon atom which is the point of attachment, having 1 or 2 heteroatom members selected from the group consisting of >O, >S(O)₀₋₂, >NH, >N(C₁₋₄alkyl), and >NC(O)OC₁₋₄alkyl; and having 0 or 1 double bonds; and
E) a monocyclic aromatic hydrocarbon group having five or six ring atoms, having a carbon atom which is the point of attachment, having one carbon atom replaced by >O, >S, >NH, >N(C₁₋₄alkyl), or >NC(O)OC₁₋₄alkyl, having up to one additional carbon atom optionally replaced by -N=, and optionally benzofused or pyridofused;
where each of B)-E) are optionally mono-, di-, or tri-substituted with a moiety selected from the group consisting of -C₁₋₄alkyl, -OH, -C₁₋₄alkylOH, -OC₁₋₆alkyl, -CN, -NO₂, -N(R^{d})R^{e} -C(O)N(R^{d})R^{e}, -N(R^{d})C(O)R^{d}, -N(R^{d})SO₂C₁₋₆alkyl, -C(O)C₁₋₆alkyl, -S(O)₀₋₂-C₁₋₆alkyl, -SO₂N(R^{d})R^{e}, -SCF₃, halo, -CF₃, -OCF₃, -COOH, -COOC₁₋₆alkyl, -OC(O)N(R^{d})R^{e}, and -OC(O)OC₁₋₆alkyl;
where R^{d} and R^{e} are each independently -H or -C₁₋₆alkyl;
-R²-R⁴- is methylene, ethylene, propylene, or -CH₂CH₂O-, wherein each is optionally substituted, with -OH, -OC₁₋₆alkyl, -SC₁₋₆alkyl, -CF₃, -C₁₋₆alkyl, amino, or halo;
R⁴ is -OH, -OC₁₋₆alkyl, -CF₃, -C₁₋₆alkyl, or halo,
m is 0, 1, or 2;
R⁵ is selected from the group consisting of -C₁₋₆alkyl, -OH, -OC₁₋₆alkyl, -SC₁₋₆alkyl, and halo;
Ar¹ is an aryl or heteroaryl ring selected from the group consisting of:
a) phenyl, optionally mono-, di-, or tri-substituted with R^{j} and optionally di-substituted on adjacent carbons with -OC₁₋₄alkyleneO- optionally mono-or di-substituted with fluoro, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
where R^{j} is selected from the group consisting of
1) -OH, -C₁₋₆alkyl, -OC₁₋₆alkyl optionally mono-, di-, or tri-substituted with halo, -C₂₋₆alkenyl, -OC₃₋₆alkenyl, -C₂₋₆alkynyl optionally substituted with trimethylsilyl, -OC₃₋₆alkynyl, -C₃₋₆cycloalkyl, -OC₃₋₆cycloalkyl, -CN, -NO₂, -N(R^{k})R^{l}, -N(R^{k})C(O)R^{l}, -N(R^{k})SO₂C₁₋₆alkyl, -C(O)C₁₋₆alkyl, -S(O)₀₋₂-C₁₋₆alkyl, -C(O)N(R^{m})Rⁿ, -SO₂N(R^{m})Rⁿ, -SCF₃, halo, -CF₃, -COOH, -COOC₁₋₆alkyl, and -COOC₃₋₇-cycloalkyl;
where R^{k} and R^{l} are each independently -H or -C₁₋₆alkyl;
where R^{m} and Rⁿ are each independently -H or -C₁₋₆alkyl, or R^{m} and Rⁿ taken together with their nitrogen of attachment form a 4-8 membered heterocyclic ring having 1 or 2 heteroatom members selected from >O, >S(O)₀₋₂, >NH, and >NC₁₋₆alkyl, having 0 or 1 double bonds, having 0 or 1 carbonyl members;
2) -G-Ar², where G is a bond, -O-, or -S-, and Ar² is phenyl or is a monocyclic aromatic hydrocarbon group having five or six ring atoms, having one carbon atom replaced by >O, >S, >NH, or >N(C₁₋₄alkyl), having up to one additional carbon atom optionally replaced by -N=, each optionally mono-, di-, or tri-substituted with R^{p};
where R^{p} is a substituent independently selected from the group consisting of: -OH, -C₁₋₆alkyl, -OC₁₋₆alkyl, phenyl, -CN, -NO₂, -N(R^{q})R^{r}, -C(O)N(R^{q})R^{r}, -N(R^{q})C(O)R^{r}, -N(R^{q})SO₂C₁₋₆alkyl, -C(O)C₁₋₆alkyl, -S(O)₀₋₂-C₁₋₆alkyl, -SO₂N(R^{q})R^{r}, -SCF₃, halo, -CF₃, -OCF₃, -OCHF₂, -COOH, and -COOC₁₋₆alkyl;
wherein R^{q} and R^{r} are each independently selected from -H, -C₁₋₆alkyl, and -C₂₋₆alkenyl; and
3) a 4-8 membered saturated or partially saturated heterocyclic ring, having 1 or 2 heteroatom members selected from >O, >S(O)₀₋₂, >NH, and >NC₁₋₆alkyl, having 0 or 1 carbonyl members, said ring optionally mono-, di-, or tri-substituted with R^{p};
b) phenyl or pyridyl fused at two adjacent carbon ring members to a three membered hydrocarbon moiety to form a fused five membered aromatic ring, which moiety has one carbon atom replaced by >O, >S, >NH, or >N(C₁₋₄alkyl), and which moiety has up to one additional carbon atom optionally replaced by -N=, the fused rings optionally mono-, di-, or tri-substituted with R^{t};
where R^{t} is a substituent independently selected from the group consisting of: -OH, -C₁₋₆alkyl, -OC₁₋₆alkyl, phenyl, -CN, -NO₂, -N(R^{u})R^{v}, -C(O)N(R^{u})R^{v}, -N(R^{u})C(O)R^{v}, -N(R^{u})SO₂C₁₋₆alkyl, -C(O)C₁₋₆alkyl, -S(O)₀₋₂-C₁₋₆alkyl, -SO₂N(R^{u})R^{v}, -SCF₃, halo, -CF₃, -OCF₃, -OCHF₂, -COOH, and -COOC₁₋₆alkyl;
where R^{u} and R^{v} are each independently -H or -C₁₋₆alkyl;
c) phenyl fused at two adjacent ring members to a four membered hydrocarbon moiety to form a fused six membered aromatic ring, which moiety has one or two carbon atoms replaced by -N=, the fused rings optionally mono-, di-, or tri-substituted with R^{t};
d) naphthyl, optionally mono-, di-, or tri-substituted with R^{t};
e) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atom optionally replaced by -N=, optionally mono- or di-substituted with R^{j} and optionally benzofused or pyridofused at two adjacent carbon atoms, where the benzofused or pyridofused moiety is optionally mono-, di-, or tri-substituted with R^{t}; and
f) a monocyclic aromatic hydrocarbon group having six ring atoms, having a carbon atom which is the point of attachment, having one or two carbon atoms replaced by -N=, optionally mono- or di-substituted with R^{j} and optionally benzofused or pyridofused at two adjacent carbon atoms, where the benzofused or pyridofused molety is optionally mono- or di-substituted with R^{t};
and enantiomers, diastereomers, hydrates, solvates and pharmaceutically acceptable salts, esters and amides thereof for use in the treatments of a CNS disorder selected from the group consisting of: sleep/wake and arousal/vigilance disorders, insomnia jet
lag, disturbed sleep, attention deficit hyperactivity disorders (ADHD), attention-deficit disorders, learning and memory disorders, learning impairment, memory impairment, memory loss, cognitive dysfunction, migraine, neurogenic inflammation, dementia, mild cognitive impairment, pre-dementia, Alzheimer's disease; epilepsy, narcolepsy with or without associated cataplexy, cataplexy, disorders of sleep wake homeostasis, idiopathic somnolence, excessive daytime sleepiness (EDS), circadian rhythym disorders, sleep/fatigue disorders, fatigue, drowsiness associated with sleep apnea, sleep impairment due to perimenopausal hormonal shifts, Parkinson's-related fatigue, MS-related fatigue, depression-related fatigue, chemotherapy-induced fatigue, work-related fatigue, lethargy, eating disorders, obesity, motion sickness, vertigo, schizophrenia, substance abuse, bipolar disorders, manic disorders and depression in mammals

12. A compound of formula (II): 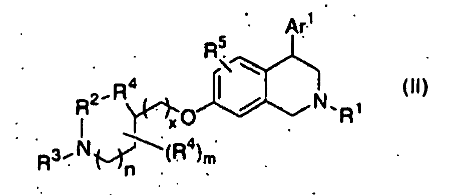 wherein
n is 1 or 2;
x is 0 or 1;
where n + x is 1 or 2;
R' is -H; or is -C₁₋₆alkyl, -C₃₋₆alkenyl, -C₃₋₆alkynyl, -C₃₋₇cycloalkyl, -C₁₋₆alkylC₃₋₇cycloalkyl, -COOC₁₋₆alkyl, or -COObenzyl, each optionally mono-, di-, or tri-substituted with R^{a};
where R^{a} is selected from -OH, -OC₁₋₆alkyl, phenyl optionally substituted with -OC₁₋₄alkyl or halo, -CN, -NO₂, -N(R^{b})R^{c}, -C(O)N(R^{b})R^{c}, -N(R^{b})C(O)R^{b}. -N(R^{b})SO₂C₁₋₆alkyl, -C(O)C₁₋₆alkyl, -S(O)₀₋₂-C₁₋₆alkyl, -SO₂N(R^{b})R^{c}, -SCF₃, halo, -CF₃, -OCF₃, -COOH, and -COOC₁₋₆alkyl;
wherein R^{b} and A^{c} are each independently -H or -C₁₋₆alkyl;
R³ is selected from the group consisting of: -H;
A) -C₁₋₆alkyl, -C₃₋₆alkenyl, -C₃₋₆alklynyl, -C₃₋₇cycloalkyl, -C₁₋₆alkylC₃₋₇cycloalkyl, -CH(C₃₋₈cycloalkyl)₂, -CH(phenyl)₂, benzyl, and -C(O)OC₁₋₄alkyl, wherein each alkyl, cycloalkyl, or benzyl is optionally substituted with -OH, -OC₁₋₄alkyl, -CN, -NH₂, -NH(C₁₋₄alkyl), -N(C₁₋₄alkyl)₂, halo, -CF₃, -OCF₃, -COOH, or -COOC₁₋₆alkyl;
B) phenyl or pyridyl, optionally fused at two adjacent carbon ring members to a three- or four-membered hydrocarbon moiety to form a fused five- or six-membered aromatic ring, which moiety has one carbon atom replaced by >O, >S, >NH, >N(C₁₋₄alkyl), or >NC(O)OC₁₋₄alkyl, and which moiety has up to one additional carbon atom optionally replaced by -N=;
C) naphthyl,
D) a 4-8 membered heterocyclic ring, said heterocyclic ring having a carbon atom which is the point of attachment, having 1 or 2 heteroatom members selected from the group consisting of >O, >S(O)₀₋₂, >NH, >N(C₁₋₄alkyl), and >NC(O)OC₁₋₄alkyl, and having 0 or 1 double bonds; and
E) a monocyclic aromatic hydrocarbon group having five or six ring atoms, having a carbon atom which is the point of attachment, having one carbon atom replaced by >O, >S, >NH, >N(C₁₋₄alkyl), or >NC(O)OC₁₋₄alkyl, having up to one additional carbon atom optionally replaced by -N=, and optionally benzofused or pyridofused;
where each of B)-E) are optionally mono-, di-, or tri-substituted with a moiety selected from the group consisting of -C₁₋₄alkyl, -OH, -C₁₋₄alkylOH, -OC₁₋₆alkyl, -CN, -NO₂, -N(R^{d})R^{e} -C(O)N(R^{d})R^{e}, -N(R^{d})C(O)R^{d}, -N(R^{d})SO₂C₁₋₆alkyl, -C(O)C₁₋₆alkyl, -S(O)₀₋₂-C₁₋₆alkyl, -SO₂N(R^{d})R^{e}, -SCF₃, halo, -CF₃, -OCF₃, -COOH, -COOC₁₋₆alkyl, -OC(O)N(R^{d})R^{e}, and -OC(O)OC₁₋₆alkyl;
where R^{d} and R^{e} are each independently -H or -C₁₋₆alkyl;
-R²-R⁴- is methylene, ethylene, propylene, or -CH₂CH₂O-, wherein each is optionally substituted with -OH, -OC₁₋₆alkyl, -SC₁₋₆alkyl, -CF₃, -C₁₋₆alkyl, amino, or halo;
R⁴ is -OH, -OC₁₋₆alkyl, -CF₃, -C₁₋₆alkyl, or halo,
m is 0, 1, or 2;
R⁵ is selected from the group consisting of -C₁₋₆alkyl, -OH, -OC₁₋₆alkyl, -SC₁₋₆alkyl, and halo;
Ar¹ is an aryl or heteroaryl ring selected from the group consisting of:
a) phenyl, optionally mono-, di-, or tri-substituted with R^{j} and optionally di-substituted on adjacent carbons with -OC₁₋₄alkyleneO- optionally mono-or di-substituted with fluoro, -(CH₂)₂₋₃NH-, -(CH₂)₁₋₂NH(CH₂)-, -(CH₂)₁₋₃N(C₁₋₄alkyl)-, or -(CH₂)₁₋₂N(C₁₋₄alkyl)(CH₂)-;
where R^{j} is selected from the group consisting of
1) -OH, -C₁₋₆alkyl, -OC₁₋₆alkyl optionally mono-, di-, or tri-substituted with halo, -C₂₋₆alkenyl, -OC₃₋₆alkenyl, -C₂₋₆alkynyl optionally substituted with trimethylsilyl, -OC₃₋₆alkynyl, -C₃₋₆cycloalkyl, -OC₃₋₆cycloalkyl, -CN, -NO₂, -N(R^{k})R^{l}, -N(R^{k})C(O)R^{l}, -N(R^{k})SO₂C₁₋₆alkyl, -C(O)C₁₋₆alkyl, -S(O)₀₋₂-C₁₋₆alkyl, -C(O)N(R^{m})Rⁿ, -SO₂N(R^{m})Rⁿ, -SCF₃, halo, -CF₃, -COOH, -COOC₁₋₆alkyl, and -COOC₃₋₇cycloalkyl;
where R^{k} and R^{l} are each independently -H or -C₁₋₆alkyl;
where R^{m} and Rⁿ are each independently -H or -C₁₋₆alkyl, or R^{m} and Rⁿ taken together with their nitrogen of attachment form a 4-8 membered heterocyclic ring having 1 or 2 heteroatom members selected from >O, >S(O)₀₋₂, >NH, and >NC₁₋₆alkyl, having 0 or 1 double bonds, having 0 or 1 carbonyl members;
2) -G-Ar², where G is a bond, -O-, or -S-, and Ar² is phenyl or is a monocyclic aromatic hydrocarbon group having five or six ring atoms, having one carbon atom replaced by >O, >S, >NH, or >N(C₁₋₄alkyl), having up to one additional carbon atom optionally replaced by -N=, each optionally mono-, di-, or tri-substituted with R^{p};
where R^{p} is a substituent independently selected from the group consisting of: -OH, -C₁₋₆alkyl, -OC₁₋₆alkyl, phenyl, -CN, -NO₂, -N(R^{q})R^{r}, -C(O)N(R^{q})R^{r}, -N(R^{q})C(O)R^{r}, -N(R^{q})SO₂C₁₋₆alkyl, -C(O)C₁₋₆alkyl, -S(O)₀₋₂-C₁₋₆alkyl, -SO₂N(R^{q})R^{r}, -SCF₃, halo, -CF₃, -OCF₃, -OCHF₂, -COOH, and -COOC₁₋₆alkyl;
wherein R^{q} and R^{r} are each independently selected from -H, -C₁₋₆alkyl, and -C₂₋₆alkenyl; and
3) a 4-8 membered saturated or partially saturated heterocyclic ring, having 1 or 2 heteroatom members selected from >O, >S(O)₀₋₂, >NH, and >NC₁₋₆alkyl, having 0 or 1 carbonyl members, said ring optionally mono-, di-, or tri-substituted with R^{p};
b) phenyl or pyridyl fused at two adjacent carbon ring members to a three membered hydrocarbon moiety to form a fused five membered aromatic ring, which moiety has one carbon atom replaced by >O, >S, >NH, or >N(C₁₋₄alkyl), and which moiety has up to one additional carbon atom optionally replaced by -N=, the fused rings optionally mono-, di-, or tri-substituted with R¹;
where R^{t} is a substituent independently selected from the group consisting of: -OH, -C₁₋₆alkyl, -OC₁₋₆alkyl, phenyl, -CN, -NO₂, -N(R^{u})R^{v}, -C(O)N(R^{u})R^{v}, -N(R^{u})C(O)R^{v}, -N(R^{u})SO₂C₁₋₆alkyl, -C(O)C₁₋₆alkyl, -S(O)₀₋₂-C₁₋₆alkyl; -SO₂N(R^{u})R^{v}, -SCF₃, halo, -CF₃, -OCF₃, -OCHF₂, -COOH, and -COOC₁₋₆alkyl;
where R^{u} and R^{v} are each independently -H or -C₁₋₆alkyl;
c) phenyl fused at two adjacent ring members to a four membered hydrocarbon moiety to form a fused six membered aromatic ring, which moiety has one or two carbon atoms replaced by -N=, the fused rings optionally mono-, di-, or tri-substituted with R^{t};
d) naphthyl, optionally mono-, di-, or tri-substituted with R^{t};
e) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atom optionally replaced by -N=, optionally mono- or di-substituted with R^{j} and optionally benzofused or pyridofused at two adjacent carbon atoms, where the benzofused or pyridofused moiety is optionally mono-, di-, or tri-substituted with R^{t}; and
f) a monocyclic aromatic hydrocarbon group having six ring atoms, having a carbon atom which is the point of attachment, having one or two carbon atoms replaced by -N=, optionally mono- or di-substituted with R^{j} and optionally benzofused or pyridofused at two adjacent carbon atoms, where the benzofused or pyridofused moiety is optionally mono- or di-substituted with R^{t};
and enantiomers, diastereomers, hydrates, solvates and pharmaceutically acceptable salts, esters and amides thereof.

13. A compound of Formula (I): 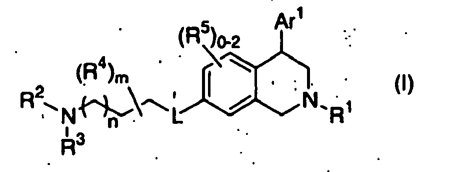 wherein
L is -O- and n is 1 or 2; or L is -C≡C- or -CH₂CH₂- and n is 0 or 1;
R¹ is -H; or is -C₁₋₆alkyl, -C₃₋₆alkenyl, -C₃₋₆alkynyl, -C₃₋₇cycloalkyl, -C₁₋₆alkylC₃₋₇cycloalkyl, -COOC₁₋₆alkyl, or -COObenzyl, each optionally mono-, di-, or tri-substituted with R^{a};
where R^{a} is selected from -OH, -OC₁₋₆alkyl, phenyl optionally substituted with -OC₁₋₄alkyl or halo, -CN, -NO₂, -N(R^{b})R^{c}, -C(O)N(R^{b})R^{c}, -N(R^{b})C(O)R^{b}, -N(R^{b})SO₂C₁₋₆alkyl, -C(O)C₁₋₆alkyl, -S(O)₀₋₂-C₁₋₆alkyl, -SO₂N(R^{b})R^{c}, -SCF₃, halo, -CF₃, -OCF₃, -COOH, and -COOC₁₋₆alkyl;
wherein R^{b} and R^{c} are each independently -H or -C₁₋₆alkyl;
R² and R³ are each independently selected from the group consisting of: -H;
A) -C₁₋₆alkyl, -C₃₋₆alkenyl, -C₃₋₆alkynyl, -C₃₋₇cycloalkyl, -C₁₋₆alkylC₃₋₇cycloalkyl, -CH(C₃₋₈cycloalkyl)₂, -CH(phenyl)₂, benzyl, and -C(O)OC₁₋₄alkyl, wherein each alkyl, cycloalkyl, or benzyl is optionally substituted with -OH, -OC₁₋₄alkyl, -CN, -NH₂, -NH(C₁₋₄alkyl), -N(C₁₋₄alkyl)₂, halo, -CF₃, -OCF₃, -COOH, or -COOC₁₋₆alkyl;
B) phenyl or pyridyl, optionally fused at two adjacent carbon ring members to a three- or four-membered hydrocarbon moiety to form a fused five- or six-membered aromatic ring, which moiety has one carbon atom replaced by >O, >S, >NH, >N(C₁₋₄alkyl), or >NC(O)OC₁₋₄alkyl, and which moiety has up to one additional carbon atom optionally replaced by -N=;
C) naphthyl,
D) a 4-8 membered heterocyclic ring, said heterocyclic ring having a carbon atom which is the point of attachment, having 1 or 2 heteroatom members selected from the group consisting of >O, >S(O)₀₋₂, >NH, >N(C₁₋₄alkyl), and >NC(O)OC₁₋₄alkyl, and having 0 or 1 double bonds; and
E) a monocyclic aromatic hydrocarbon group having five or six ring atoms, having a carbon atom which is the point of attachment, having one carbon atom replaced by >O, >S, >NH, >N(C₁₋₄alkyl), or >NC(O)OC₁₋₄alkyl, having up to one additional carbon atom optionally replaced by -N=, and optionally benzofused or pyridofused;
where each of B)-E) are optionally mono-, di-, or tri-substituted with a moiety selected from the group consisting of -C₁₋₄alkyl, -OH, -C₁₋₄alkylOH, -OC₁₋₆alkyl, -CN, -NO₂, -N(R^{d})R^{e} -C(O)N(R^{d})R^{e}, -N(R^{d})C(O)R^{d}, -N(R^{d})SO₂C₁₋₆alkyl, -C(O)C₁₋₆alkyl, -S(O)₀₋₂-C₁₋₆alkyl, -SO₂N(R^{d})R^{e}, -SCF³, halo, -CF₃, -OCF₃, -COOH, -COOC₁₋₆alkyl, -OC(O)N(R^{d})R^{e}, and OC(O)OC₁₋₆alkyl;
where R^{d} and R^{e} are each independently -H or -C₁₋₆alkyl;
or, alternatively,
R² and R³ may be taken together with the nitrogen to which they are attached to form tetrahydro-pyrimidin-2-ylidenyl, or a 4-8 membered heterocyclic ring; said heterocyclic ring having 0 or 1 additional heteroatom members separated from the nitrogen of attachment by at least one carbon member and selected from the group consisting of >O, >S(O)₀₋₂, >NH, and >NR^{f}, having 0 or 1 double bond, having 0, 1 or 2 carbon members separated from the nitrogen of attachment by at least one carbon member which is a carbonyl, optionally benzo or pyrido fused, optionally having one carbon member that forms a bridge, and having 0-5 carbon member substituents R^{ff},
where R^{f} is selected from the group consisting of:
i) -C₁₋₆alkyl optionally substituted with R², -C₃₋₆alkenyl, -C₃₋₆alkynyl, -C(O)N(R⁹)R^{h}, -C(O)Rⁱ, -S(O)₀₋₂-C₁₋₆alkyl, and -COOC₁₋₆alkyl,
where R^{z} is fluoro, -CN, -OH, -OC₁₋₄alkyl, -C₃₋₇cycloalkyl, or -CF₃;
where R^{g} and R^{h} are each independently -H or -C₁₋₆alkyl; and
where Rⁱ is -C₁₋₆alkyl, -C₃₋₈cycloalkyl, phenyl, or 5- or 6-membered aromatic heterocyclyl, where each alkyl, cycloalkyl, phenyl or heterocyclyl is optionally mono-, di-, or tri-substituted with -C₁₋₄alkyl, -OH, -OC₁₋₆alkyl, -CF₃, -CN, or halo;
ii) -(CH₂)₀₋₁-RingA, where Ring A is phenyl or a 5- or 6-membered carbon-linked aromatic heterocyclyl, optionally substituted with R^{aa};
where R^{aa} is -OH, -C₁₋₆alkyl, -OC₁₋₆alkyl, phenyl, -CN, -NO₂, -N(R^{g})R^{h} -C(O)N(R^{g})R^{h}, -N(R^{g})C(O)R^{h}, -N(R^{h})SO₂C₁₋₆alkyl, -C(O)C₁₋₆alkyl, -S(O)₀₋2-C₁₋₆alkyl, -SO₂N(R^{g})R^{h}, -SCF₃, halo, -CF₃, -OCF₃, -OCHF₂, -COOH, and -COOC₁₋₆alkyl; and
iii) -(CH₂)₀₋₁-RingB, where Ring B is -C₃₋₇cycloalkyl with one or two carbon ring members optionally replaced with >O, or >NH, and optionally substituted with -C₁₋₄alkyl, fluoro, -CN, -OH, -OC₁₋₄alkyl, or -CF₃;
where R^{ff} is selected from the group consisting of -C₁₋₆alkyl optionally mono-or di-substituted with R^{z}, -C₂₋₆alkenyl, -C₂₋₆alkynyl, -C₃₋₇cycloalkyl, -CH(phenyl)₂, halo, -OH, -OC₁₋₆alkyl, -OC₂₋₃alkylO-, -CN, -NO₂, -N(R^{g})R^{h}, -C(O)N(R^{g})R^{h}, -N(R^{g})C(O)R^{g}, -N(R^{g})SO₂C₁₋₆alkyl, -C(O)Rⁱ, -S(O)₀₋₂-C₁₋₆alkyl, -SO₂N(R^{g})R^{h}, -SCF₃, -CF₃, -OCF₃, -COOH, and -COOC₁₋₆alkyl;
R⁴ is -OH, -OC₁₋₆alkyl, -CF₃, -C₁₋₆alkyl, or halo; two R⁴ substituents may be taken together to form methylene or ethylene; or one of R⁴ is taken together with R² to form methylene, ethylene, propylene, or -CH₂CH₂O-, wherein each is optionally substituted with -OH, -OC₁₋₆alkyl, -SC₁₋₆alkyl, -CF₃, -C₁₋₆alkyl, amino, or halo;
m is 0, 1, or 2;
R⁵ is selected from the group consisting of -C₁₋₆alkyl, -OH, -OC₁₋₆alkyl, -SC₁₋₆alkyl, and halo;
Ar¹ is an aryl or heteroaryl ring selected from the group consisting of:
a) phenyl, optionally mono-, di-, or tri-substituted with R^{j} and optionally di-substituted on adjacent carbons with -OC₁₋₄alkyleneO- optionally mono-or di-substituted with fluoro, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
where R^{j} is selected from the group consisting of
1) -OH, -C₁₋₆alkyl, -OC₁₋₆alkyl optionally mono-, di-, or tri-substituted with halo, -C₂₋₆alkenyl, -OC₃₋₆alkenyl, -C₂₋₆alkynyl optionally substituted with trimethylsilyl, -OC₃₋₆alkynyl, -C₃₋₆cycloalkyl, -OC₃₋₆cycloalkyl, -CN, -NO₂, -N(R^{k})R^{l}, -N(R^{k})C(O)R^{l}, -N(R^{k})SO₂C₁₋₆alkyl, -C(O)C₁₋₆alkyl, -S(O)₀₋₂C₁₋₆alkyl, -C(O)N(R^{m})Rⁿ, -SO₂N(R^{m})Rⁿ, -SCF₃, halo, -CF₃, -COOH, -COOC₁₋₆alkyl, and -COOC₃₋₇cycloalkyl;
where R^{k} and R^{l} are each independently -H or -C₁₋₆alkyl;
where R^{m} and Rⁿ are each independently -H or -C₁₋₆alkyl; or R^{m} and Rⁿ taken together with their nitrogen of attachment form a 4-8 membered heterocyclic ring having 1 or 2 heteroatom members selected from >O, >S(O)₀₋₂, >NH, and >NC₁₋₆alkyl, having 0 or 1 double bonds, having 0 or 1 carbonyl-members;
2) -G-Ar², where G is a bond, -O-, or -S-, and Ar² is phenyl or is a monocyclic aromatic hydrocarbon group having five or six ring atoms, haying one carbon atom replaced by >O, >S, >NH, or >N(C₁₋₄alkyl), having up to one additional carbon atom optionally replaced by -N=, each optionally mono-, di-, or tri-substituted with R^{p};
where R^{p} is a substituent independently selected from the group consisting of: -OH, -C₁₋₆alkyl, -OC₁₋₆alkyl, phenyl, -CN, -NO₂, -N(R^{q})R^{r}, -C(O)N(R^{q})R^{r}, -N(R^{q})C(O)R^{r}, -N(R^{q})SO₂C₁-₆alkyl, -C(O)C₁₋₆alkyl, -S(O)₀₋₂-C₁₋₆alkyl, -SO₂N(R^{q})R^{r}, -SCF₃, halo, CF₃, -OCF₃, -OCHF₂, -COOH, and -COOC₁₋₆alkyl;
wherein R^{q} and R^{r} are each independently selected from -H, -C₁₋₆alkyl, and -C₂₋₆alkenyl; and
3) a 4-8 membered saturated or partially saturated heterocyclic ring, having 1 or 2 heteroatom members selected from >O, >S(O)₀₋₂, >NH, and >NC₁₋₆alkyl, having 0 or 1 carbonyl members, said ring optionally mono-, di-, or tri-substituted with R^{p};
b) phenyl or pyridyl fused at two adjacent carbon ring members to a three membered hydrocarbon moiety to form a fused five membered aromatic ring, which moiety has one carbon atom replaced by >O, >S, >NH, or >N(C₁₋₄alkyl), and which moiety has up to one additional carbon atom optionally replaced by -N=, the fused rings optionally mono-, di-, or tri-substituted with R^{t};
where R^{t} is a substituent independently selected from the group consisting of: -OH, -C₁₋₆alkyl, -OC₁₋₆alkyl, phenyl, -CN, -NO₂, -N(R^{u})R^{v}, -C(O)N(R^{u})R^{v}, -N(R^{u})C(O)R^{v}, -N(R^{u})SO₂C₁₋₆alkyl, -C(O)C₁-₆alkyl, -S(O)₀₋₂-C₁₋₆alkyl, -SO₂N(R^{u})R^{v}, -SCF₃, halo, -CF₃, -OCF₃, -OCHF₂, -COOH, and -COOC₁₋₆alkyl;
where R^{u} and R^{v} are each independently -H or -C₁₋₆alkyl;
c) phenyl fused at two adjacent ring members to a four membered hydrocarbon moiety to form a fused six membered aromatic ring, which moiety has one or two carbon atoms replaced by -N=, the fused rings optionally mono-, di-, or tri-substituted with R^{t};
d) naphthyl, optionally mono-, di-, or tri-substituted with R^{t};
e) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atom optionally replaced by -N=, optionally mono- or di-substituted with R^{j} and optionally benzofused or pyridofused at two adjacent carbon atoms, where the benzofused or pyridofused moiety is optionally mono-, di-, or tri-substituted with R^{t}; and
f) a monocyclic aromatic hydrocarbon group having six ring atoms, having a carbon atom which is the point of attachment, having one or two carbon atoms replaced by -N=, optionally mono- or di-substituted with R^{j} and optionally benzofused or pyridofused at two adjacent carbon atoms, where the benzofused or pyridofused moiety is optionally mono- or di-substituted with R^{t};
and enantiomers, diastereomers, hydrates, solvates and pharmaceutically acceptable salts, esters and amides thereof;
with the following proviso:
when n is 1, L is -O-, Ar is unsubstituted phenyl, and R² and R³ are both methyl,
then R⁴ is not -OH.

14. The compound of claim 13 wherein L is -O- and n is 1.

15. The compound of claim 13 wherein L is -C≡C- and n is 0.

16. The compound of claim 13 wherein L is -CH₂CH₂- and n is 0.

17. The compound of claim 13 wherein R¹ is -C₁₋₄alkyl.

18. The compound of claim 13 wherein R¹ is selected from the group consisting of hydrogen, methyl, ethyl, propyl, isopropyl, butyl, t-butyl, pentyl, hexyl, benzyl, vinyl, allyl, propargyl, cyclopropyl, cyclobutyl, cyclopentyl, cyclohexyl, cyclopropylmethyl, -COOCH₃, -COO-t-butyl, and -COObenzyl.

19. The compound of claim 13 wherein R¹ is methyl, ethyl, propyl, t-butyl, allyl, propargyl, or benzyl.

20. The compound of claim 13 wherein R¹ is hydrogen or methyl.

21. The compound of claim 13 wherein R¹ is methyl.

22. The compound of claim 13 wherein when R² is methyl, R³ is not methyl.

23. The compound of claim 13 wherein R² and R³ are hydrogen, or, optionally substituted, are independently selected from the group consisting of:
A) methyl, ethyl, propyl, isopropyl, sec-butyl, butyl, pentyl, hexyl, vinyl, allyl, propargyl, cyclopropyl, cyclobutyl, cyclopentyl, cyclohexyl, cyclopropylmethyl, dicyclopropylmethyl, benzhydryl, benzyl, -C(O)O-t-butyl,
B) phenyl, pyridyl, 4-, 5-, 6- or 7-benzoxazolyl, 4-, 5-, 6- or 7-benzothiophenyl, 4-, 5-, 6- or 7-benzofuranyl, 4-, 5-, 6- or 7-indolyl, 4-, 5-, 6- or 7-benzthiazolyl, 4-, 5-, 6- or 7-benzimidazolyl, 4-, 5-, 6- or 7-indazolyl, imidazo[1,2-a]pyridin-5, 6, 7 or 8-yl, pyrazolo[1,5-a]pyridin-4, 5, 6 or 7-yl, 1H-pyrrolo[2,3-b]pyridin-4, 5 or 6-yl, 1H-pyrrolo[3,2-c]pyridin-4, 6 or 7-yl, 1H-pyrrolo[2,3-c]pyridin-4, 5 or 7-yl, 1H-pyrrolo[3,2-b]pyridin-5, 6 or 7-yl,
C) naphthyl,
D) azetidinyl, pyrrolidinyl, piperidinyl, tetrahydropyranyl, and
E) furanyl, oxazolyl, isoxazolyl, 1,2,3-oxadiazolyl,1,2,4-oxadiazolyl, 1,2,5-oxadiazolyl, 1,3,4-oxadiazolyl, thiophenyl, thiazolyl, isothiazolyl, pyrrolyl, imidazolyl, pyrazolyl, 1,2,3-triazolyl, 1,2,4-triazolyl, 3-indoxazinyl, 2-benzoxazolyl, 2- or 3-benzothiophenyl, 2- or 3-benzofuranyl, 2- or 3-indolyl, 2-benzthiazolyl, 2-benzimidazolyl, and 3-indazolyl.

24. The compound of claim 13 wherein R² and R³ are independently selected from the group consisting of methyl, ethyl, propyl, isopropyl, sec-butyl, hydroxyethyl, 2-hydroxy-2-methylpropyl, allyl, cyclopropyl, cyclobutyl, cyclopentyl, dicyclopropylmethyl, pyrrolidinyl; piperidinyl, N-methylpiperidinyl, tetrahydropyranyl, 2-benzothiazolyl, and methoxyethyl.

25. The compound of claim 13 wherein R² and R³ are each independently hydrogen, ethyl, isopropyl, methoxyethyl, 2-benzothiazolyl, cyclopropyl, cyclobutyl, or cyclopentyl.

26. The compound of claim 13 wherein R² and R³, optionally substituted, are taken together with the nitrogen to which they are attached to form tetrahydro-pyrimidin-2-ylidenyl, or a ring selected from the group consisting of azetidinyl, pyrrolidinyl, piperidinyl, morpholinyl, thiomorpholinyl, piperazinyl, homopiperidinyl, 1,3-dihydro-isoindol-2-yl, 5,6-dihydro-4H-pyrimidin-1-yl, and 1,1-dioxo-1λ⁶-thiomorpholin-4-yl.

27. The compound of claim 13 wherein R^{f}, optionally substituted, is methyl, ethyl, isopropyl, allyl, cyclopropyl, tert-butoxycarbonyl, tetrahydrofuranylmethyl, [1,3]-dioxolan-ylmethyl, thiazolyl, thiophenyl, thiophenylmethyl, pyridinyl, phenyl, acetyl, isobutyryl, cyclopropanecarbonyl, cyclobutanecarbonyl, pyridinyl, pyridine-carbonyl, 1H-pyrrole-carbonyl, and 1H-imidazole-carbonyl.

28. The compound of claim 13 wherein R² and R³ are taken together with the nitrogen to which they are attached to form a 4-8-membered heterocyclic ring, said heterocyclic ring selected from piperidine, pyrrolidone, and morpholine, said ring substituted with 1 or 2 substituents R^{ff}.

29. The compound of claim 13 wherein R^{ff} is selected from the group consisting of methyl, ethyl, isopropyl, butyl, hexyl, -CHF₂, benzhydryl, -CF₃, vinyl, allyl, propargyl, cyclopropyl, cyclopentyl, cyclopropylmethyl, cyclobutylethyl, bromo, chloro, fluoro, iodo, -OH, hydroxymethyl, hydroxyethyl, methoxy, ethoxy, isopropoxy, pentyloxy, -O(CH₂)₂O-, -O(CH₂)₃O-, -CN, amino, methylamino, dimethylamino, diethylamino, diethylcarbamoyl, methanesulfanyl, methanesulfonyl, methanesulfonamido, -C(O)Rⁱ, -COOH, and ethoxycarbonyl.

30. The compound of claim 13 wherein R^{ff} is selected from the group consisting of methyl, fluoro, -OH, -CF₃, hydroxymethyl, hydroxyethyl, benzhydryl, dimethylamino, ethoxycarbonyl, cyano, and -O(CH₂)₂O-.

31. The compound of claim 13 wherein Rⁱ is selected from the group consisting of methyl, pyridyl, isopropyl, cyclobutyl, cyclopropyl, N-methylpyrrolyl, and 1-methylimidazolyl.

32. The compound of claim 13 wherein R² and R³ are taken together with the nitrogen to which they are attached to form azetidinyl, 3,3-difluoroazetidinyl, 3-benzhydryl-azetidinyl, 2-methylpyrrolidinyl, 3-hydroxypyrrolidinyl, 3-dimethylaminopyrrolidinyl, 2,5-dimethylpyrrolidinyl, 2-trifluoromethylpyrrolidinyl, 2-hydroxymethylpyrrolidinyl, 3,3-difluoropyrrolidinyl, piperidinyl, 4-fluoropiperidinyl, 3-fluoropiperidinyl, 3,3-difluoropiperidinyl, 4,4-difluoropioeridinyl, 3-trifluoromethylpiperidinyl, 4-trifluoromethylpiperidinyl, 1,4-dioxa-8-aza-spiro[4.5]dec-8-yl, 4-cyanopiperidinyl, 4-carboethoxypiperidinyl, 3-hydroxypiperidinyl, 4-hydroxypiperidinyl, 2-hydroxymethylpiperidinyl, 3-hydroxymothylpiperidinyl, 4-hydroxymethylpiperidinyl, 3-hydroxyethylpiperidinyl, 4-hydroxyethylpiperidinyl, morpholinyl, 2-methylmorpholin-4-yl, 3-methylmorpoolin-4-yl, 3-hydroxymethylmorpholin-4-yl, 2-hydroxymethylmorpholin-4-yl, 4-methyl-piperazin-1-yl, 4-ethyl-piperazin-1-yl, 4-isopropyl-piperazin-yl, 4-allyl-piperazin-1-yl, 4-cyclopropyl-piperazin-1-yl, 4-(2-hydroxyethyl)piperazin-1-yl, 4-(2-methoxyethyl)-piperazin-1-yl, 4-(tert-butoxycarbonyl)piperazin-1-yl, 4-(tetrahydrofuran-2-ylmethyl)piperazin-1-yl, 4-[1,3]-dioxolan-2-ylmethyl-piperazin-1-yl, 4-thiazol-2-yl-piperazin-1-yl, 4(2-thiophenyl)piperazinyl, 4-thlophen-2-ylmethyl-piperazin-1-yl, 4-(pyridin-4-yl)-piperazin-1-yl, 4-phehylpiperazin-1-yl, 4-(2-hydroxyphenyl)piperazinyl, 4-(4-trifluoromethyl-phenyl)-piperazin-1-yl, 4-(4-cyanophenyl)piperazin-1-yl, 4-acetylpiperazin-1-yl, 4-isobutyryl-piperazin-1-yl, 4-cyclopropanecarbonyl-piperazin-1-yl), 4-cyclobutanecarbonyl-piperazin-1-yl, 4-pyridin-4-yl-piperazin-1-yl, 4-(pyridine-4-carbonyl)-piperazin-1-yl, 4-(1-methyl-1H-pyrrole-2-carbonyl)-piperazin-1-yl, 4-(1-methyl-1H-imidazole-4-carbonyl)-piperazin-1-yl, 1,1-dioxo-1λ⁶-thlomorpholin-4-yl, 2,6-dimethylmorpholin-4-yl, and 1,3-dihydro-isoindol-2-yl, 5,6-dihydro-4H-pyrimidin-1-yl.

33. The compound of claim 13 wherein R² and R³ are taken together with the nitrogen to which they are attached to form piperidinyl, 4-fluoropiperldinyl, 4,4-difluoropiperidinyl, morpholinyl, or 3-methylmorpholin-4-yl.

34. The compound of claim 13 wherein R⁴ is hydroxy, methoxy, ethoxy, isopropoxy, pentyloxy, -CF₃, methyl, ethyl, propyl, isobutyl, pentyl, chloro, or fluoro.

35. The compound of claim 13 wherein R⁴ is hydroxy, methyl, methoxy, fluoro, or -CF₃.

36. The compound of claim 3 wherein two R⁴ are taken together to form methylene.

37. The compound of claim 13 wherein R² and one of R⁴ are taken together to form methylene, ethylene, or -CH₂CH₂O-.

38. The compound of claim 13 wherein m is 0.

39. The compound of claim 13 wherein R⁵ is methyl, ethyl, isopropyl, hexyl, hydroxy, methoxy, ethoxy, isopropoxy, methylsulfanyl, bromo, chloro, fluoro, or iodo.

40. The compound of claim 13 wherein R⁵ is methyl, hydroxy, or fluoro.

41. The compound of claim 13 wherein Ar¹, optionally substituted, is selected from the group consisting of:
a) phenyl, 5-, 6-. 7-, 8-benzo-1,4-dioxanyl, 4-,5-, 6-, 7-benzo-1,3-dioxolyl, 4-, 5-, 6-, 7-indolinyl, 4-, 5-, 6-, 7-isoindolinyl, 1,2,3,4-tetrahydro-ouinolin-4, 5, 6 or 7-yl, 1,2,3,4-tetrahydro-isoquinolin-4, 5, 6 or 7-yl,
b) 4-, 5-, 6- or 7-benzoxazolyl, 4-, 5-, 6- or 7-benzothiophenyl, 4-, 5-, 6- or 7-benzofuranyl, 4-, 5-, 6- or 7-indolyl, 4-, 5-, 6- or 7-benzthiazolyl, 4-, 5-, 6- or 7-benzimidazolyl, 4-, 5-, 6- or 7-indazolyl, imidazo[1,2-a]pyridin-5, 6, 7 or 8-yl, pyrazolo[1,5-a]pyridin-4, 5, 6 or 7-yl, 1H-pyrrolo[2,3-b]pyridin-4, 5 or 6-yl, 1H-pyrrolo[3,2-c]pyridin-4, 6 or 7-yl, 1H-pyrrolo[2,3-c]pyridin-4, 5 or 7-yl, 1H-pyrrolo[3,2-b]pyridin-5, 6 or 7-yl,
c) 5-, 6-, 7- or 8-isoquinolinyl, 5-, 6-, 7- or 8-quinolinyl, 5-, 6-, 7- or 8-quinoxalinyl, 5-, 6-, 7- or 8-quinazolinyl,
d) naphthyl,
e) furanyl, oxazolyl, isoxazolyl, 1,2,3-oxadiazolyl, 1,2,4-oxadiazolyl, 1,2,5-oxadiazolyl, 1,3,4-oxadiazolyl, thiophenyl, thiazolyl, isothiazolyl, pyrrolyl, imidazolyl, pyrazolyl, 1,2,3-triazolyl, 1,2,4-triazolyl, 3-indoxazinyl, 2-benzoxazolyl, 2- or 3-benzothiophenyl, 2- or 3-benzofuranyl, 2- or 3-indolyl, 2-benzthiazolyl, 2-benzimidazolyl, 3-indazolyl, and
f) pyridinyl, pyridinyl-N-oxide, pyrazinyl, pyrimidinyl, pyridazinyl, 1-, 3- or 4-isoquinolinyl, 2-, 3- or 4-quinolinyl, 2- or 3-quinoxalinyl, 2- or 4-quinazolinyl, [1,5], [1,6], [1,7], or [1,8]naphthyridin-2-, 3-, or 4-yl, [2,5], [2,6], [2,7], [2,8]naphthyridin-1-, 3-, or 4-yl.

42. The compound of claim 13 wherein Ar¹, optionally substituted, is selected from the group consisting of phenyl, pyridyl, pyrazinyl, thiazolyl, pyrazolyl, and thiophenyl.

43. The compound of claim 13 wherein G is a bond or -S-.

44. The compound of claim 13 wherein Ar² is selected from the group consisting of phenyl, pyrrolyl; furanyl, thiophenyl; pyrazolyl, imidazolyl, oxazolyl, thiazolyl, isoxazolyl, triazolyl, tetrazolyl, pyridinyl, pyrimidinyl, or pyrazinyl.

45. The compound of claim 13 wherein Ar¹ is selected from the group consisting of phenyl, 2-methoxyphenyl, 3-methoxyphenyl, 4-methoxyphenyl, 4-ethoxyphenyl, 2,4-dimethoxyphenyl, 2,5-dimethoxyphenyl, 3,4-dimethoxyphenyl, 3,5-dimethoxyphenyl, 3,4,5-trimethoxyphenyl, 2-methylphenyl, 3-methylphenyl, 4-methylphenyl, 4-ethylphenyl, 3-ethynylphenyl, 4-ethynylphenyl, 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 3-iodophenyl, 4-iodophenyl, 2-trifluoromethylphenyl, 3-trifluoromethylphenyl, 4-trifluoromethylphenyl, 3-trifluoromethoxyphenyl, 4-trifluoromethoxyphenyl, 4-difluoromethoxyphenyl, 3-cyanophenyl, 4-cyanophenyl, 3-acetylphenyl, 4-acetylphenyl, 3,4-difluorophenyl, 3,4-dichlorophenyl, 2,3-difluorophenyl, 2,3-dichlorophenyl, 2,4-difluorophenyl, 2,4-dichlorophenyl, 2,5-dichlorophenyl, 3,6-dichlorophenyl, 3-nitrophenyl, 4-nitrophenyl, 3-chloro-4-fluorophenyl, 3-chloro-4-methoxyphenyl, 3-chloro-4-difluoromethoxyphenyl, 3-fluoro-4-chlorophenyl, 2-fluoro-4-methoxyphenyl, 3-fluoro-4-methoxyphenyl, benzo[1,3]dioxol-4 or 5-yl, 2-hydroxyphenyl, 3-hydroxyphenyl, 4-hydroxyphenyl, 4-hydroxy-2-methylphenyl, 4-hydroxy-3-fluorophenyl, 3,4-dihydroxyphenyl, 4-aminophenyl, 4-dimethylaminophenyl, 4-morpholin-4-yl-phenyl, 4-carbamoylphenyl, 4-fluoro-3-methylphenyl, 4-methanesulfanylphenyl, 4-methanesulfinylphenyl, 4-methanesulfonylphenyl, 4-trifluoromethanesulfanylphenyl, thiophen-2-yl, thiophen-3-yl, 2-pyridinyl, 3-pyridinyl, 4-pyridinyl, 2-chloro-5-pyridinyl, 2-dimethylamino-5-pyridinyl, 6-methoxy-pyridin-3-yl, 6-methylsulfanyl-pyridin-3-yl, 2-hydroxy-5-pyridinyl, 6-pyrazol-1-yl-pyridin-3-yl, 6-bromo-pyridin-3-yl, 6-ethynyl-pyridin-3-yl, 6-trimethylsilanylethynyl-pyridin-3-yl, 6-phenylsulfanyl-pgridin-3-yl, 6-imidazol-1-yl-pyridin-3-yl, 6-(1H-imidazol-2-ylsulfanyl)-pyridin-3-yl, 6-(pyrimidin-2-ylsulfanyl)-pyridin-3-yl, oxazol-5-yl, thiazol-5-yl, thiazol-2-yl, 2H-pyrazol-3-yl, pyrazin-2-yl, 1-naphthyl, 2-naphthyl, 4-imidazol-1-ylphenyl, 4-pyrazol-1-ylphenyl, 1H-indol-5-yl, 1H-benzimidazol-5-yl, benzo[b]thiophen-7-yl, and 4-biphenyl.

46. The compound of claim 13 wherein Ar¹, optionally substituted with halo, is 4-methoxyphenyl, 4-methanesulfanylphenyl, or 4-chlorophenyl.

47. The compound of claim 13 wherein said pharmaceutically acceptable salt is an effective amino addition salt.

48. The compound of claim 13 wherein said pharmaceutically acceptable salt is selected from the group consisting of hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, and laurylsulfonate.

49. A compound of claim 13 isotopically-labelled to be detectable by PET or SPECT.

50. A method for studying histamine H₃ receptor- and serotonin-mediated disorders comprising the step of using an ¹⁸F-labeled or ¹¹C-labelled compound of claim 13 as a positron emission tomography (PET) molecular probe,

51. A compound of Formula (II): 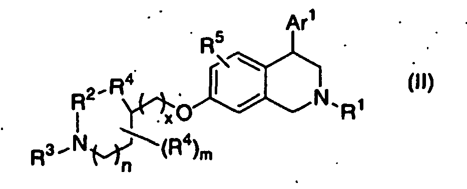 wherein
n is 1 or 2;
x is 0 or 1;
where n + x is 1 or 2;
R¹ is -H; or is -C₁₋₆alkyl, -C₃₋₆alkenyl, -C₃₋₆alkynyl, -C₃₋₇cycloalkyl, -C₁₋₆alkylC₃₋₇cycloalkyl, -COOC₁₋₆alkyl, or -COObenzyl, each optionally mono-, di-, or tri-substituted with R^{a};
where R^{a} is selected from -OH, -OC₁₋₆alkyl, phenyl optionally substituted with -OC₁₋₄alkyl or halo, -CN, -NO₂, -N(R^{b})R^{c}, -C(O)N(R^{b})R^{c}, -N(R^{b})C(O)R^{b}, -N(R^{b})SO₂C₁₋₆alkyl, -C(O)C₁₋₆alkyl, -S(O)₀₋₂-C₁₋₆alkyl, -SO₂N(R^{b})R^{c}, -SCF₃, halo, -CF₃, -OCF₃, -COOH, and, -COOC₁₋₆alkyl;
wherein R^{b} and R^{c} are each independently -H or -C₁₋₆alkyl;
R³ is selected from the group consisting of: -H;
A) -C₁₋₆alkyl, -C₃₋₆alkenyl, -C₃₋₆alkynyl, -C₃₋₇cycloalkyl, -C₁₋₆alkylC₃₋₇cycloalkyl, -CH(C₃₋₈cycloalkyl)₂, -CH(phenyl)₂; benzyl, and -C(O)OC₁₋₄alkyl, wherein each alkyl, cycloalkyl, or benzyl is optionally substituted with -OH, -OC₁₋₄alkyl, -CN, -NH₂, -NH(C₁₋₄alkyl), -N(C₁₋₄alkyl)₂, halo, -CF₃, -OCF₃, -COOH, or -COOC₁₋₆alkyl;
B) phenyl or pyridyl, optionally fused at two adjacent carbon ring members to a three- or four-membered hydrocarbon moiety to form a fused five- or six-membered aromatic ring, which moiety has one carbon atom replaced by >O, >S, >NH, >N(C₁₋₄alkyl), or >NC(O)OC₁₋₄alkyl, and which moiety has up to one additional carbon atom optionally replaced by -N=;
C) naphthyl,
D) a 4-8 membered heterocyclic ring, said heterocyclic ring having a carbon atom which is the point of attachment, having 1 or 2 heteroatom members selected from the group consisting of >O, >S(O)₀₋₂, >NH, >N(C₁₋₄alkyl), and >NC(O)OC-₁₋₄alkyl, and having 0 or 1 double bonds; and
E) a monocyclic aromatic hydrocarbon group having five or six ring atoms, having a carbon atom which is the point of attachment, having one carbon atom replaced by >O, >S, >NH, >N(C₁₋₄alkyl), or >NC(O)OC₁₋₄alkyl, having up to one additional carbon atom optionally replaced by -N=, and optionally benzofused or pyridofused;
where each of B)-E) are optionally mono-, di-, or tri-substituted with a moiety selected from the group consisting of -C₁₋₄alkyl, -OH, -C₁₋₄alkylOH, -OC₁₋₆alkyl, -CN, -NO₂, -N(R^{d})R^{e} -C(O)N(R^{d})R^{e}, -N(R^{d})C(O)R^{d}, -N(R^{d})SO₂C₁₋₆alkyl, -C(O)C₁₋₆alkyl, -S(O)₀₋₂-C₁₋₆alkyl, -SO₂N(R^{d})R^{e}, -SCF₃, halo, -CF₃, -OCF₃, -COOH, -COOC₁₋₆alkyl, -OC(O)N(R^{d})R^{e}, and -OC(O)OC₁₋₆alkyl;
where R^{d} and R^{e} are each independently -H or -C₁₋₆alkyl;
-R²-R⁴- is methylene, ethylene, propylene, or -CH₂CH₂O-, wherein each is optionally substituted with -OH, -OC₁₋₆alkyl, -SC₁₋₆alkyl, -CF₃, -C₁₋₆alkyl, amino, or halo;
R⁴ is -OH, -OC₁₋₆alkyl, -CF₃, -C₁₋₆alkyl, or halo,
m is 0, 1, or 2;
R⁵ is selected from the group consisting of -C₁₋₆alkyl, -OH, -OC₁₋₆alkyl, -SC₁₋₆alkyl, and halo;
Ar¹ is an aryl or heteroaryl ring selected from the group consisting of:
a) phenyl, optionally mono-, di-, or tri-substituted with R^{j} and optionally di-substituted on adjacent carbons with -OC₁₋₄alkyleneO- optionally mono-or di-substituted with fluoro, -(CH₂)₂₋₃NH-, -(CH₂)₁₋₂NH(CH₂)-, -(CH₂)₂₋₃N(C₁₋₄alkyl), or -(CH₂)₁₋₂N(C₁₋₄alkyl)(CH₂)-;
where R^{j} is selected from the group consisting of
1) -OH, -C₁₋₆alkyl, -OC₁₋₆alkyl optionally mono-, di-, or tri-substituted with halo, -C₂₋₆alkenyl, -OC₃₋₆alkenyl, -C₂₋₆alkynyl optionally substituted with trimethylsilyl, -OC₃₋₆alkynyl, -C₃₋₆cycloalkyl, -OC₃₋₆cycloalkyl, -CN, -NO₂, -N(R^{k})R^{l}, -N(R^{k})C(O)R^{l}, -N(R^{k})SO₂C₁₋₆alkyl, -C(O)C₁₋₆alkyl, -S(O)₀₋₂-C₁₋₆alkyl, -C(O)N(R^{m})Rⁿ, -SO₂N(R^{m})Rⁿ, -SCF₃, halo, -CF₃, -COOH, -COOC₁₋₆alkyl, and -COOC₃₋₇Cycloalkyl;
where R^{k} and R^{l} are each independently -H or -C₁₋₆alkyl;
where R^{m} and Rⁿ are each independently -H or -C₁₋₆alkyl, or R^{m} and Rⁿ taken together with their nitrogen of attachment form a 4-8 membered heterocyclic ring having 1 or 2 heteroatom members selected from >O, >S(O)₀₋₂, >NH, and >NC₁₋₆alkyl, having 0 or 1 double bonds, having 0 or 1 carbonyl members;
2) -G-Ar², where G is a bond, -O-, or -S-, and Ar² is phenyl or is a monocyclic aromatic hydrocarbon group having five or six ring atoms, having one carbon atom replaced by >O, >S, >NH, or >N(C₁₋₄alkyl), having up to one additional carbon atom optionally replaced by -N=, each optionally mono-, di-, or tri-substituted with R^{p};
where R^{p} is a substituent independently selected from the group consisting of: -OH, -C₁₋₆alkyl, -OC₁₋₆alkyl, phenyl, -CN, -NO₂, -N(R^{q})R^{f}, -C(O)N(R^{q})R^{r}, -N(R^{q})C(O)R^{r}, -N(R^{q})SO₂C₁₋₆alkyl, -C(O)C₁₋₆alkyl, -S(O)₀₋₂-C₁₋₆alkyl, -SO₂N(R^{q})R^{r}, -SCF₃, halo, -CF₃, -OCF₃, -OCHF₂, -COOH, and -COOC₁₋₆alkyl;
wherein R^{q} and R^{r} are each independently selected from -H, -C₁₋₆alkyl, and -C₂₋₆alkenyl; and
3) a 4-8 membered saturated or partially saturated heterocyclic ring, having 1 or 2 heteroatom members selected from >O, >S(O)₀₋₂, >NH, and >NC₁₋₆alkyl, having 0 or 1 carbonyl members, said ring optionally mono-, di-, or tri-substituted with R^{p};
b) phenyl or pyridyl fused at two adjacent carbon ring members to a three membered hydrocarbon moiety to form a fused five membered aromatic ring, which moiety has one carbon atom replaced by >O, >S, >NH, or >N(C₁₋₄alkyl), and which moiety has up to one additional carbon atom optionally replaced by -N=, the fused rings optionally mono-, di-, or tri-substituted with R^{t};
where R^{t} is a substituent independently selected from the group consisting of: -OH, -C₁₋₆alkyl, -OC₁₋₆alkyl, phenyl, -CN, -NO₂, -N(R^{u})R^{v}, -C(O)N(R^{u})R^{v}, -N(R^{u})C(O)R^{v}, -N(R^{u})SO₂C₁₋₆alkyl, -C(O)C₁₋₆alkyl, -S(O)₀₋₂-C₁₋₆alkyl, -SO₂N(R^{u})R^{v}, -SCF₃, halo, -CF₃, -OCF₃, -OCHF₂, -COOH, and -COOC₁₋₆alkyl;
where R^{u} and R^{v} are each independently -H or -C₁₋₆alkyl;
c) phenyl fused at two adjacent ring members to a four membered hydrocarbon moiety to form a fused six membered aromatic ring, which moiety has one or two carbon atoms replaced by -N=, the fused rings optionally mono-, di-, or tri-substituted with R^{t};
d) naphthyl, optionally mono-, di-, or tri-substituted with R^{t};
e) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atom optionally replaced by -N=, optionally mono- or di-substituted with R^{j} and optionally benzofused or pyridofused at two adjacent carbon atoms, where the benzofused or pyridofused moiety is optionally mono-, di-, or tri-substituted with R^{t}; and
f) a monocyclic aromatic hydrocarbon group having six ring atoms, having a carbon atom which is the point of attachment, having one or two carbon atoms replaced by -N=, optionally mono- or di-substituted with R^{j} and optionally benzofused or pyridofused at two adjacent carbon atoms, where the benzofused or pyridofused moiety is optionally mono- or di-substituted with R^{t};
and enantiomers, diastereomers, hydrates, solvates and pharmaceutically acceptable salts, esters and amides thereof.

52. The compound of claim 51 wherein n is 1 and x is 0.

53. The compound of claim 51 wherein n is 1 and x is 1.

54. The compound of claim 51 wherein n is 2 and x is 0.

55. The compound of claim 51 wherein R¹ is methyl.

56. The compound of claim 51 wherein R³ is hydrogen, ethyl, isopropyl, methoxyethyl, 2-benzothiazolyl, cyclopropyl, cyclobutyl, or cyclopentyl.

57. The compound of claim 51 wherein R⁴ is methyl, hydroxy, methoxy, -CF₃, methyl, fluoro, or chloro.

58. The compound of claim 51 wherein m is 0.

59. The compound of claim 51 wherein R⁵ is methyl, hydroxy, methoxy, methylsulfanyl, fluoro, or chloro.

60. The compound of claim 51 wherein Ar¹, optionally substituted with halo, is 4-methoxyphenyl, 4-methanesulfanylphenyl, or 4-chlorophenyl.

61. The compound of claim 51 wherein -R²-R⁴- is methylene.

62. The compound of claim 51 wherein -R²-R⁴- is ethylene.

63. The compound of claim 51 wherein -R²-R⁴- is -CH₂CH₂O-.

64. A compound selected from the group consisting of:
7-[3-(4-Fluoro-piperidin-1-yl)-propoxy]-4-(4-methoxy-phenyl)-2-methyl-1,2,3,4-tetrahydro-isoquinoline;
7-[3-(4-Fluoro-piperidin-1-yl)-propoxy]-4-(4-mqthoxy-phenyl)-2-methyl-1,2,3,4-tetrahydro-isoquinoline (enantiomer 1);
7-[3-(4-Fluoro-piperidin-1-yl)-propoxy]-4-(4-methoxy-phenyl)-2-methyl-1,2,3,4-tetrahydro-isoquinoline (enantiomer 2);
1-(4-{3-[4-(4-Methoxy-phenyl)-2-methyl-1,2,3,4-tetrahydro-isoquinolin-7-yloxy]-propyl}-piperazin-1-yl)-ethanone;
Diethyl-{3-[4-(4-methoxy-phenyl)-2-methyl-1,2,3,4-tetrahydro-isoquinolin-7-yloxy]-propyl}-amine;
(4-{3-[4-(4-Methoxy-phenyl)-2-methyl-1,2,3,4-tetrahydro-isoquinolin-7-yloxyl-propyl}-piperazin-1-yl)-pyridin-4-yl-methanone;
1-(4-{3-[4-(4-Methoxy-phenyl)-2-methyl-1,2,3,4-tetrahydro-isoquinolin-7-yloxy]-propyl}-piperazin-1-yl)-2-methyl-propan-1-one;
Cyclobutyl-(4-{3-[4-(4-methoxy-phenyl)-2-methyl-1,2,3,4-tetrahydro-isoquinolin-7-yloxy]-propyl}-piperazin-1-yl)-methanone;
Cyclopropyl-(4-{3-[4-(4-methoxy-phenyl)-2-methyl-1,2,3,4-tetrahydro-isoquinolin-7-yloxy]-propyl}-piperazin-1-yl)-methanone;
7-[3-(4,4-Difluoro-piperidin-1-yl)-propoxy]-4-(4-methoxy-phenyl)-2-methyl-1,2,3,4-tetrahydro-isoquinoline;
4-(4-Methoxy-phenyl)-2-methyl-7-(3-morpholin-4-yl-propoxy)-1,2,3,4-tetrahydro-isoquinoline;
(1-{3-[4-(4-Methoxy-phenyl)-2-methyl-1,2,3,4-tetrahydro-isoquinolin-7-yloxy]-propyl}-pyrrolidin-3-yl)-dimethyl-amine;
7-[3-((2R,5R)-trans-Dimethyl-pyrrolidin-1-yl)-propoxy]-4-(4-methoxy-phenyl)-2-methyl-1,2,3,4-tetrahydro-isoquinoline;
4-{3-[4-(4-Methoxy-phenyl)-2-methyl-1,2,3,4-tetrahydro-isoquinolin-7-yloxy]-propyl}-piperazine-1-carboxylic acid ethyl ester;
(4-{3-[4-(4-Methoxy-phenyl)-2-methyl-1,2,3,4-tetrahydro-isoquinolin-7-yloxy]-propyl}-piperazin-1-yl)-(1-methyl-1H-pyrrol-2-yl)-methanone;
(4-{3-[4-(4-Methoxy-phenyl)-2-methy)-1,2,3,4-tetrahydro-isoquinolin-7-yloxy]-propyl}-piperazin-1-yl)-(1-methyl-1H-imidazol-4-yl)-methanone;
(1,3-Dimethyl-tetrahydro-pyrimidin-2-ylidene)-{3-[4-(4-methoxy-phenyl)-2-methyl-1,2,3,4-tetrahydro-isoquinolin-7-yloxy]-propyl}-amine;
7-[3-(1,3-Dihydro-isoindol-2-yl)-propoxy]-4-(4-methoxy-phenyl)-2-methyl-1,2,3,4-tetrahydro-isoquinoline;
Bis-(2-methoxy-ethyl)-{3-[4-(4-methoxy-phenyl)-2-methyl-1,2,3,4-tetrahydro-isoquinolin-7-yloxy]-propyl}-amine;
7-[3-(5,6-Dihydro-4H-pyrimidin-1-yl)-propoxy]-4-(4-methoxy-phenyl)-2-methyl-1,2,3,4-tetrahydro-isoquinoline;
Benzothiazol-2-yl-{3-[4-(4-methoxy-phenyl)-2-methyl-1,2,3,4-tetrahydro-isoquinolin-7-yloxy]-propyl}-methyl-amine;
1-{3-[4-(4-Methoxy-phenyl)-2-methyl-1,2,3,4-tetrahydro-isoquinolin-7-yloxy]-propyl}-piperidin-3-ol;
1-{3-[4-(4-Methoxy-phenyl)-2-methyl-1,2,3,4-tetrahydro-isoquinolin-7-yloxy]-propyl}-piperidin-4-ol;
(1-{3-[4-(4-Methoxy-phenyl)-2-methyl-1,2,3,4-tetrahydro-isoquinolin-7-yloxy]-propyl}-piperidin-4-yl)-methanol;
(1-{3-[4-(4-Methoxy-phenyl)-2-methyl-1,2,3,4-tetrahydro-isoquinolin-7-yloxy]-propyl}-piperidin-3-yl)-methanol;
(1-{3-[4-(4-Methoxy-phenyl)-2-methyl-1,2,3,4-tetrahydro-isoquinolin-7-yloxy]-propyl}-piperidin-2-yl)-methanol;
4-(4-Methoxy-phenyl)-2-methyl-7-[3-(3-trifluoromethyl-piperidin-1-yl)-propoxy]-1,2,3,4-tetrahydro-isoquinoline;
2-(1-{3-[4-(4-Methoxy-phenyl)-2-methyl-1,2,3,4-tetrahydro-isoquinolin-7-yloxy]-propyl}-piperidin-4-yl)-ethanol;
7-[3-(1,1-Dioxo-1λ⁶-thiomorpholin-4-yl)-propoxy]-4-(4-methoxy-phenyl)-2-methyl-1,2,3,4-tetrahydro-isoquinoline;
4-(4-Methoxy-phenyl)-2-methyl-7-[3-((2S)-trifluoromethyl-pyrrolidin-1-yl)-propoxy]-1,2,3,4-tetrahydro-isoquinoline;
7-[3-(3,3-Difluoro-piperidin-1-yl)-propoxy]-4-(4-methoxy-phenyl)-2-methyl-1,2,3,4-tetrahydro-isoquinoline;
7-[3-(3,3-Difluoro-piperidin-1-yl)-propoxy]-4-(4-methoxy-phenyl)-2-methyl-1,2,3,4-tetrahydro-isoquinoline (enantiomer 1);
7-[3-(3,3-Difluoro-piperidin-1-yl)-propoxy]-4-(4-methoxy-phenyl)-2-methyl-1,2,3,4-tetrahydro-isoquinoline (enantiomer 2);
(1-{3-[4-(4-Methoxy-phenyl)-2-methyl-1,2,3,4-tetrahydro-isoquinolin-7-yloxy]-propyl}-pyrrolidin-(2R)-yl)-methanol;
1-{3-[4-(4-Methoxy-phenyl)-2-methyl-1,2,3,4-tetrahydro-isoquinolin-7-yloxy]-propyl}-(R)-pyrrolidin-3-ol;
7-[3-(2,6-Dimethyl-morpholine-4-yl)-propoxy]-4-(4-methoxy-phenyl)-2-methyl-1,2,3,4-tetrahydro-isoquinoline;
1-{3-[4-(4-Methoxy-phenyl)-2-methyl-1,2,3,4-tetrahydro-isoquinolin-7-yloxy]-propyl}-piperidine-4-carboxylic acid ethyl ester;
7-(3-Azetidin-1-yl-propoxy)-4-(4-methoxy-phenyl)-2-methyl-1,2,3,4-tetrahydro-isoquinoline;
4-(4-Methoxy-phenyl)-2-methyl-7-[3-(2-methyl-pyrrolidin-1-yl)-propoxy]-1,2,3,4-tetrahydro-isoquinoline;
2-(1-{3-[4-(4-Methoxy-phenyl)-2-methyl-1,2,3,4-tetrahydro-isoquinolin-7-yloxy]-propyl}-piperidin-2-yl)-ethanol;
1-{3-[4-(4-Methoxy-phenyl)-2-methyl-1,2,3,4-tetrahydro-isoquinolin-7-yloxy]-propyl}-piperidine-4-carbonitrile;
1-{3-[4-(4-Methoxy-phenyl)-2-methyl-1,2,3,4-tetrahydro-isoquinolin-7-yloxy]-propyl}-piperidine-4-carbonitrile (enantiomer 1);
1-{3-[4-(4-Methoxy-phenyl)-2-methyl-1,2,3,4-tetrahydro-isoquinolin-7-yloxy]-propyl}-piperidine-4-carbonitrile (enantiomer 2);
4-(4-Methoxy-phenyl)-2-methyl-7-[3-(4-trifluoromethyl-piperidin-1-yl)-propoxy]-1,2,3,4-tetrahydro-isoquinoline;
7-[3-(3-Fluoro-piperidin-1-yl)-propoxy]-4-(4-methoxy-phenyl)-2-methyl-1,2,3,4-tetrahydro-isoquinoline;
Ethyl-(2-methoxy-ethyl)-{3-[4-(4-methoxy-phenyl)-2-methyl-1,2,3,4-tetrahydro-isoquinolin-7-yloxy]-propyl}-amine;
7-[3-(1,4-Dioxa-8-aza-spiro[4.5]dec-8-yl)-propoxy]-4-(4-methoxy-phenyl)-2-methyl-1,2,3,4-tetrahydro-isoquinoline;
7-[2-Fluoro-3-(4-fluoro-piperidin-1-yl)-propoxy]-4-(4-methoxy-phenyl)-2-methyl-1,2,3,4-tetrahydro-isoquinoline; 7-[3-(4-Fluoro-piperidin-1-yl)-propoxy]-4-(3-methoxy-phenyl)-2-methyl-1,2,3,4-tetrahydro-isoquinoline; 7-[3-(4-Fluoro-piperidin-1-yl)-propoxy]-4-(3-methoxy-phenyl)-2-methyl-1,2,3,4-tetrahydro-isoquinoline (enantiomer 1); 7-[3-(4-Fluoro-piperidin-1-yl)-propoxy]-4-(3-methoxy-phenyl)-2-methyl-1,2,3,4-tetrahydro-isoquinoline (enantiomer 2); 4-(3-Chloro-phenyl)-7-[3-(4-fluoro-piperidin-1-yl)-propoxy]-2-methyl-1,2,3,4-tetrahydro-isoquinoline; 4-(4-Chloro-phenyl)-7-[3-(4-fluoro-piperidin-1-yl)-propoxy]-2-methyl-1,2,3,4-tetrahydro-isoquinoline; 4-(3-Fluoro-phenyl)-7-[3-(4-fluoro-piperidin-1-yl)-propoxy]-2-methyl-1,2,3,4-tetrahydro-isoquinoline; 7-[3-(4-Fluoro-piperidin-1-yl)-propoxy]-2-methyl-4-(4-trifluoromethoxy-phenyl)-1,2,3,4-tetrahydro-isoquinoline; 4-(4-Difluordmethoxy-phenyl)-7-[3-(4-fluoro-piperidin-1-yl)-prppoxy]-2-methyl-1,2,3,4-tetrahydro-isoquinoline; 7-[3-(4-Fluoro-piperidin-1-yl)-propoxy]-4-(4-methanesulfonyl-phenyl)-2-methyl-1,2,3,4-tetrahydro-isoquinoline; 7-[3-(4-Fluoro-piperidin-1-yl)-propoxy]-2-methyl-4-(4-methylsulfanyl-phenyl)-1,2,3,4-tetrahydro-isoquinoline; 4-(3-Chloro-4-methoxy-phenyl)-7-[3-(4-fluoro-piperidin-1-yl)-propoxy]-2-methyl-1,2,3,4-tetrahydro-isoquinoline; 4-(2,4-Dichloro-phenyl)-7-[3-(4-fluoro-piperidin-1-yl)-propoxy]-2-methyl-1,2,3,4-tetrahydro-isoquinoline; 4-(2,5-Dichloro-phenyl)-7-[3-(4-fluoro-piperidin-1-yl)-propoxy]-2-methyl-1,2,3,4-tetrahydro-isoquinoline; 4-(3,5-Dichloro-phenyl)-7-[3-(4-fluoro-piperidin-1-yl)-propoxy]-2-mefhyl-1,2,3,4-tetrahydro-isoquinoline;
2-Methyl-7-(3-piperidin-1-yl-propoxy)-4-thiophen-3-yl-1,2,3,4-tetrahydro-isoquinoline; 4-(3,4-Dichloro-phenyl)-7-[3-(4-fluoro-piperidin-1-yl)-propoxy]-2-methyl-1,2,3,4-tetrahydro-isoquinoline; 2-Methyl-7-(3-piperidin-1-yl-propoxy)-4-pyridin-3-yl-1,2,3,4-tetrahydro-isoquinoline; 2-Methyl-7-(3-pippridin-1-yl-propoxy)-4-(trifluoromethyl-phenyl)-1,2,3,4-tetrahydro-isoquinoline; 4-(4-Methoxy-phenyl)-2-methyl-7-(3-piperidin-1-yl-propoxy)-1,2,3,4-tetrahydro-isoquinoline (racemic); 4-(4-Methoxy-phenyl)-2-methyl-7-(3-piperidin-1-yl-propoxy)-1,2,3,4-tetrahydro-isoquinoline (enantiomer 1); 4-(4-Methoxy-phenyl)-2-methyl-7-(3-piperidin-1-yl-propoxy)-1,2,3,4-tetrahydro-isoquinoline (enantiomer 2); 2-tert-Butyl-4-(4-methoxy-phenyl)-7-(3-piperidin-1-yl-propoxy)-1,2,3,4-tetrahydro-isoquinoline; 2-Benzyl-4-(4-methoxy-phenyl)-7-(3-piperidin-1-yl-propoxy)-1,2,3,4-tetrahydro-isoquinoline; 2-Ethyl-4-(4-methoxy-phenyl)-7-(3-piperidin-1-yl-propoxy)-1,2,3,4-tetrahydro-isoquinoline; 4-(4-Methoxy-phenyl)-7-(3-piperidin-1-y)-prbpoxyl-2-propyl-1,2,3,4-tetrahydro-isoquinoline; 2-Isopropyl-4-(4-methoxy-phenyl)-7-(3-piperidin-1-yl-propoxy)-1,2,3,4-tetrahydro-isoquinoline; 4-(4-Methoxy-phenyl)-7-(3-piperidin-1-yl-propoxy)-1,2,3,4-tetrahydro-isoquinoline; 3-[4-(4-Methoxy-phenyl)-7-(3-piperidin-1-yl-propoxy)-3,4-dihydro-1H-isoquinolin-2-yl]-propan-l-ol; 2-[4-(4-Methoxy-phenyl)-7-(3-piperidin-1-yl-propoxy)-3,4-dihydro-1H-isoquinolin-2-yl]-ethanol; 2-(2-Fluoro-ethyl)-4-(4-methoxy-phenyl)-7-(3-piperidin-1-yl-propoxy)-1,2,3,4-tetrahydro-isoquinoline; 2-Cyclopropyl-4-(4-methoxy-phenyl)-7-(3-piperidin-1-yl-propoxy)-1,2,3,4-tetrahydro-isoquinoline;
4-(4-Methoxy-phenyl)-7-(3-morpholin-4-yl-propoxy)-2-(1-phenyl-ethyl)-1,2,3,4-tetrahydro-isoquinoline;
4-(4-Methoxy-phenyl)-7-(3-morpholin-4-yl-propoxy)-2-(1-phenyl-ethyl)-1,2,3,4-tetrahydro-isoquinoline (diastereomer 1);
4-(4-Methoxy-phenyl)-7-(3-morpholin-4-yl-propoxy)-2-(1-phenyl-ethyl)-1,2,3,4-tetrahydro-isoquinoline (diastereomer 2);
4-(3,4-Dichloro-phenyl)-2-methyl-7-(3-piperidin-1-yl-propoxy)-1,2,3,4-tetrahydro-isoquinoline;
4-(3,4-Dichloro-phenyl)-2-methyl-7-(3-morpholin-4-yl-propoxy)-1,2,3,4-tetrahydro- , isoquinoline;
4-(4-Methoxy-phenyl)-2-methyl-7-(piperidin-4-ylmethoxy)-1,2,3,4-tetrahydro-isoquinoline;
4-(3-Chloro-4-methoxy-phenyl)-2-methyl-7-(piperidin-4-ylmethoxy)-1,2,3,4-tetrahydro-isoquinoline;
2-Methyl-4-(4-methylsulfanyl-phenyl)-7-(piperidin-4-ylmethoxy)-1,2,3,4-tetrahydro-isoquinoline;
4-(3-Fluoro-4-methoxy-phenyl)-2-methyl-7-(piperidin-4-ylmethoxy)-1,2,3,4-tetrahydro-isoquinoline;
4-(2-Fluoro-4-methoxy-phenyl)-2-methyl-7-(piperidin-4-ylmethoxy)-1,2,3,4-tetrahydro-isoquinoline;
7-(1-Isopropyl-piperidin-4-ylmethoxy)-4-(4-methoxy-phenyl)-2-methyl-1,2,3,4-tetrahydro-isoquinoline;
4-(4-Methoxy-phenyl)-2-methyl-7-(1-methyl-piperidin-4-ylmethoxy)-1,2,3,4-tetrahydro-isoquinoline;
4-(4-Methoxy-phenyl)-2-methyl-7-(1-propyl-piperidin-4-ylmethoxy)-1,2,3,4-tetrahydro-isoquinoline;
7-(1-Cyclopentyl-piperidin-4-ylmethoxy)-4-(4-methoxy-phenyl)-2-methyl-1,2,3,4-tetrahydro-isoquinoline;
7-(1-Ethyl-piperidin-4-ylmethoxy)-4-(4-methoxy-phenyl)-2-methyl-1,2,3,4-tetrahydro-isoquinoline;
4-(3-Chloro-4-methoxy-phenyl)-7-(1-isopropyl-piperidin-4-ylmethoxy)-2-methyl-1,2,3,4-tetrahydro-isoquinoline;
4-(3-Fluoro-4-methoxy-phenyl)-7-(1-isopropyl-piperidin-4-ylmethoxy)-2-methyl-1,2,3,4-tetrahydro-isoquinoline; 4-(2-Fluoro-4-methoxy-phenyl)-7-(1-isoprppyl-piperidin-4-ylmethoxy)-2-methyl-1,2,3,4-tetrahydro-isoquinoline; 7-(1-Isopropyl-piperidin-4-ylmethoxy)-4-(3-methoxy-phenyl)-2-methyl-1,2,3,4-tetrahydro-isoquinoline; 4-(3-Methoxy-phenyl)-2-methyl-7-(1-methyl-piperidin-4-ylmethoxy)-1,2,3,4-tetrahydro-isoquinoline; 4-(3-Methoxy-phenyl)-2-methyl-7-(1-propyl-piperidin-4-ylmethoxy)-1,2,3,4-tetrahydro-isoquinoline; 7-(1-Cyclopentyl-piperidin-4-ylmethoxy)-4-(3-methoxy-phenyl)-2-methyl-1,2,3,4-tetrahydro-isoquinoline; 7-(1-Ethyl-piperidin-4-ylmethoxy)-4-(3-methoxy-phenyl)-2-methyl-1,2,3,4-tetrahydro-isoquinoline; 4-(3-Methoxy-phbnyl)-2-methyl-7-(piperidin-4-yloxy)-1,2,3,4-tetrahydro-isoquinoline; 4-(3-Methoxy-phenyl)-2-methyl-7-(1-methyl-piperidin-4-yloxy)-1,2,3,4-tetrahydro-isoquinoline; 7-(1-Isopropyl-piperidin-4-yloxy)-4-(3-methoxy-phenyl)-2-methyl-1,2,3,4-tetrahydro-isoquinoline; 7-(1-Cyclobutyl-piperidin-4-yloxy)-4-(3-methoxy-phenyl)-2-methyl-1,2,3,4-tetrahydro-isoquinoline; 7-(1-Ethyl-piperidin-4-yloxy)-4-(3-methoxy-phenyl)-2-methyl-1,2,3,4-tetrahydro-isoquinoline; 7-(1-Cyclopentyl-piperidin-4-yloxy)-4-(3-methoxy-phenyl)-2-methyl-1,2,3,4-tetrahydro-isoquinoline; 4-(3-Methoxy-phenyl)-2-methyl-7-(1-propyl-piperidin-4-yloxy)-1,2,3,4-tetrahydro-isoquinoline; 7-(1-Isopropyl-piperidin-4-ylmethoxy)-2-methyl-4-(4-methylsulfanyl-phenyl)-1,2,3,4-tetrahydro-isoquinoline; 7-(1-Cyclobutyl-piperidin-4-ylmethoxy)-2-methyl-4-(4-methylsulfanyl-phenyl)-1,2,3,4-tetrahydro-isoquinoline;
7-(1-Ethyl-piperidin-4-ylmethoxy)-2-methyl-4-(4-methylsulfanyl-phenyl)-1,2,3,4-tetrahydro-isoquinoline;
4-(3-Chloro-4-methoxy-phenyl)-7-(1-cyclobutyl-piperidin-4-ylmethoxy)-2-methyl-1,2,3,4-tetrahydro-isoquinoline;
4-(3-Chloro-4-methoxy-phenyl)-7-(1-ethyl-piperidin-4-ylmethoxy)-2-methyl-1,2,3,4-tetrahydro-isoquinoline;
4-(3-Chloro-4-methoxy-phenyl)-2-methyl-7-(1-propyl-piperidin-4-ylmethoxy)-1,2,3,4-tetrahydro-isoquinoline;
2-Methyl-4-(4-methylsulfanyl-phenyl)-7-(1-propyl-piperidin-4-ylmethoxy)-1,2,3,4-tetrahydro-isoquinoline;
7-(1-Isobutyl-piperidin-4-ylmethoxy)-4-(4-methoxy-phenyl)-2-methyl-1,2,3,4-tetrahydro-isoquinoline;
7-(1-Cyclobutyl-piperidin-4-ylmethoxy)-4-(3-fluoro-4-methoxy-phenyl)-2-methyl-1,2,3,4-tetrahydro-isoquinoline;
4-(3-Fluoro-4-methoxy-phenyl)-2-methyl-7-(1-propyl-piperidin-4-ylmethoxy)-1,2,3,4-tetrahydro-isoquinoline;
7-(1-Cyclobutyl-piperidin-4-ylmethoxy)-4-(2-fluoro-4-methoxy-phenyl)-2-methyl-1,2,3,4-tetrahydro-isoquinoline;
4-(2-Fluoro-4-methoxy-phenyl)-2-methyl-7-(1-propyl-piperidin-4-ylmelhoxy)-1,2,3,4-tetrahydro-isoquinoline;
4-(2-Fluoro-4-methoxy-phenyl)-7-(1-isobutyl-piperidin-4-ylmethoxy)-2-methyl-1,2,3,4-tetrahydro-isoquinoline;
7-[1-(2-Fluoro-ethyl)-piperidin-4-ylmethoxy]-4-(4-methoxy-phenyl)-2-methyl-1,2,3,4-fetrahydro-isoquinoline;
7-(1-Isopropyl-piperidin-4-yloxy)-4-(4-methoxy-phenyl)-2-methyl-1,2,3,4-tetrahydro-isoquinoline;
7-(1-Isopropyl-piperidin-4-yloxy)-2-methyl-4-(4-methylsulfanyl-phenyl)-1,2,3,4-tetrahydro-isoquinoline;
4-(2-Fluoro-4-methoxy-phenyl)-7-(1-isopropyl-piperidin-4-yloxy)-2-methyl-1,2,3,4-tetrahydro-isoquinoline;
4-(3-Fluoro-4-methoxy-phenyl)-7-(1-isopropyl-piperidin-4-yloxy)-2-methyl-1,2,3,4-tetrahydro-isoquinoline;
4-(4-Methoxy-phenyl)-2-methyl-7-[1-(tetrahydro-pyran-4-yl)-piperidin-4-yloxy]-1,2,3,4-tetrahydro-isoquinoline; 2,2,2-Triftuoro-1-{4-[4-(4-methoxy-phenyl)-2-methyl-1,2,3,4-tetrahydro-isoquinolin-7-yloxymethyl]-piperidin-1-yl}-ethanone; 4-(4-Methoxy-phenyl)-2-methyl-7-[1-(2,2,2-trifluoro-ethyl)-piperidin-4-ylmethoxy]-1,2,3,4-tetrahydroisoquinoline; 7-[3-(4-Fluoro-piperidin-1-yl)-propoxy]-2-methyl-4-phenyl-1,2,3,4-tetrahydro-isoquinoline; 4-(2-Fluoro-phenyl)-7-[3-(4-fluoro-piperidin-1-yl)-propoxy]-2-methyl-1,2,3,4-tetrahydro-isoquinoline; 7-[3-(4-Fluoro-piperidin-1-yl)-propoxy]-2-methyl-4-p-tolyl-1,2,3,4-tetrahydro-isoquinoline; 2-Benzyl-7-[3-(4-fluoro-piperidin-1-yl)-propoxy]-4-(4-methoxy-phenyl)-1,2,3,4-tetrahydro-isoquinoline; 7-[3-(4-Fluoro-piperidin-1-yl)-propoxy]-2-methyl-4-(3-trifluoromethoxy-phenyl)-1,2,3,4-tetrahydro-isoquinoline; 7-[3-(3,3-Difluoro-pyrrolidin-1-yl)-propoxy]-4-(4-methoxy-phenyl)-2-methyl-1,2,3,4-tetrahydro-isoquinoline; 4-(4-Methoxy-phenyl)-2-methyl-7-[3-(3S-methyl-morpholin-4-yl)-propoxy]-1,2,3,4-tetrahydro-isoquinoline; Dicyclopropylmethyl-{3-[4-(4-methoxy-phenyl)-2-methyl-1,2,3,4-tetrahydro-isoquinolin-7-yloxy]-propyl}-amine; 4-(2-Chloro-phenyl)-7-[3-(4-fluoro-piperidin-1-yl)-propoxy]-2-methyl-1,2,3,4-tetrahydro-isoquinoline; 2-Methyl-7-[3-(3S-methyl-morpholin-4-yl)-propoxy]-4-(4-morpholin-4-yl-phenyl)-1,2,3,4-tetrahydro-isoquinoline; 7-[3-(4-Fluoro-piperidin-1-yl)-propoxy]-2-methyl-4-(4-morpholin-4-yl-phenyl)-1,2,3,4-tetrahydro-isoquinoline; 4-{2-Methyl-7-[3-(3S-methyl-morpholin-4-yl)-propoxy]-1,2,3,4-tetrahydro-isoquinolin-4-yl}-benzonitrile;
4-{7-[3-(4-Fluoro-piperidin-1-yl)-propoxy]-2-methyl-1,2,3,4-tetrahydro-isoquinolin-4-yl}-benzonitrile;
7-[3-(3-Benzhydryl-azetidin-1-yl)-propoxy]-4-(4-methoxy-phenyl)-2-methyl-1,2,3,4-tetrahydro-isoquinoline; 2-Methyl-4-(4-methylsulfanyl-phenyl)-7-(3-morpholin-4-yl-propoxy)-1,2,3,4-tetrahydro-isoquinoline; 4-(2-Fluoro-4-methoxy-phenyl)-7-[3-(4-fluoro-piperidin-1-yl)-propoxy]-2-methyl-1,2,3,4-tetrahydro-isoquinoline (racemate); 4-(2-Fluoro-4-methoxy-phenyl)-7-[3-(4-fluoro-piperidin-1-yl)-propoxy]-2-methyl-1,2,3,4-tetrahydro-isoquinoline (enantiomer 1); 4-(2-Fluoro-4-methoxy-phenyl)-7-[3-(4-fluoro-piperidin-1-yl)-propoxy]-2-methyl-1,2,3,4-tetrahydro-isoquinoline (enantiomer 2); 4-(3-Fluoro-4-methoxy-phenyl)-7-[3-(4-fluoro-piperidin-1-yl)-propoxy]₋2-methyl-1,2,3,4-tetrahydro-isoquinoline (racemate); 4-(3-Fluoro-4-methoxy-phenyl)-7-[3-(4-fluoro-piperidin-1-yl)-propoxy]-2-methyl-1,2,3,4-tetrahydro-isoquinoline (enantiomer 1); 4-(3-Fluoro-4-methoxy-phenyl)-7-[3-(4-fluoro-piperidin-1-yl)-ptopoxy]-2-methyl-1,2,3,4-tetrahydro-isoquinoline (enantiomer 2); 4-(4-Ethoxy-phenyl)-7-[3-(4-fluoro-piperidin-1-yl)-propoxy]-2-methyl-1,2,3,4-tetrahydro-isoquinoline; 2-Ethyl-7-[3-(4-fluoro-piperidin-1-yl)-propoxy]-4-(4-methoxy-phenyl)-1,2,3,4-tetrahydro-isoquinoline; 7-[3-(4-Fluoro-piperidin-1-yl)-propoxy]-4-(4-methoxy-phenyl)-1,2,3,4-tetrahydro-isoquinoline; 4-(4-Methoxy-phenyl)-2-methyl-7-(piperidin-4-yloxy)-1,2,3,4-tetrahydro-isoquinoline; 2-Methyl-4-(4-methylsulfanyl-phenyl)-7-(piperidin-4-yloxy)-1,2,3,4-tetrahydro-isoquinoline; 4-(2-Fluoro-4-methoxy-phenyl)-2-methyl-7-(piperidin-4-yloxy)-1,2,3,4-tetrahydro-isoquinoline; 4-(3-Fluoro-4-methoxy-phenyl)-2-methyl-7-(piperidin-4-yloxy)-1,2,3,4-tetrahydro-isoquinoline; 7-[1-(2-Fluoro-ethyl)-piperidin-4-yloxy]-4-(4-methoxy-phenyl)-2-methyl-1,2,3,4-tetrahydro-isoquinoline;
7-[3-(4-Fluoro-piperidin-1-yl)-propoxy]-2-methyl-4-(4-methylsulfanyl-phenyl)-1,2,3,4-tetrahydro-isoquinoline (enantiomer 1); 7-[3-(4-Fluoro-piperidin-1-yl)-propoxy]-2-methyl-4-(4-methylsulfanyl-phenyl)-1,2,3,4-tetrahydro-isoquinoline (enantiomer 2); 4-(4-Methoxy-phenyl)-2-methyl-7-[3-(3S-methyl-morpholin-4-yl)-propoxy]-1,2,3,4-tetrahydro-isoquinoline (enantiomer 1); 4-(4-Methoxy-phenyl)-2-methyl-7-[3-(3S-methyl-morpholin-4-yl)-propoxy]-1,2,3,4-tetrahydro-isoquinoline (enantiomer 2); 2-Methyl-4-(4-methylsulfanyl-phenyl)-7-(3-morpholin-4-yl-propoxy)-1,2,3,4-tetrahydro-isoquinoline (enantiomer 1); 2-Methyl-4-(4-methylsulfanyl-phenyl)-7-(3-morpholin-4-yl-propoxy)-1,2,3,4-tetrahydro-isoquinoline (enantiomer 2); 7-[3-(4-Fluoro-piperidin-1-yl)-propoxy]-4-(4-methylsulfanyl-phenyl)-1,2,3,4-tetrahydro-isoquinotine; 4-(4-Methylsulfanyl-phenyl)-7-(3-morpholin-4-yl-propoxy)-1,2,3,4-tetrahydro-isoquinoline; 7-[3-(3S-Methyl-morpholin-4-yl)-propoxy]-4-(4-methylsulfanyl-phenyl)-1,2,3,4-tetrahydro-isoquinoline; 4-(4-Methoxy-phenyl)-7-(3-morpholin-4-yl-propoxy)-1,2,3,4-tetrahydro-isoquinoline; 7-[3-(4-Fluoro-piperidin-1-yl)-propoxy]-4-(4-methanesulfinyl-phenyl)-2-methyl-1,2,3,4-tetrahydro-isoquinoline; 4-(4-Methanesulfinyl-phenyl)-2-methyl-7-(3-morpholin-4-yl-propoxy)-1,2,3,4-tetrahydro-isoquinoline; 4-(4-Methanesulfinyl-phenyl)-2-methyl-7-(3-morpholin-4-yl-propoxy)-1,2,3,4-tetrahydro-isoquinoline (enantiomer 1); 4-(4-Methanesulfonyl-phenyl)-2-methyl-7-(3-morpholin-4-yl-propoxy)-1,2,3,4-tetrahydro-isoquinoline; 7-[3-(4-Fluoro-piperidin-1-yl)-propoxy]-4-(4-methoxy-phenyl)-2,6-dimethyl-1,2,3,4-tetrahydro-isoquinoline; 7-[3-(4-Fluoro-piperidin-1-yl)-propoxy]-4-(4-methoxy-phenyl)-2,8-dim ethyl-1,2,3,4-tetrahydro-isoquinoline;
7-[3-(4-Fluoro-piperidin-1-yl)-propoxy]-4-(4-methoxy-phenyl)-2-methyl-1,2,3,4-tetrahydro-isoquinolin-6-ol; 7-[3-(4-Fluoro-piperidin-1-yl)-propoxy]-4-(4-methoxy-phenyl)-2-methyl-1,2,3,4-tetrahydro-isoquinolin-8-ol; 2,8-Dimethyl-4-(4-methylsulfanyl-phenyl)-7-(3-morpholin-4-yl-propoxy)-1,2,3,4-tetrahydro-isoquirioline; 2,6-Dimethyl-4-(4-methylsulfanyl-phenyl)-7-(3-morpholin-4-yl-propoxy)-1,2,3,4-tetrahydro-isoquinoline; 2-Methyl-4-(4-methylsulfanyl-phenyl)-7-(3-morpholin-4-yl-propoxy)-1,2,3,4-tetrahydro-isoquinolin-8-ol; 2-Methyl-4-(4-methylsulfanyl-phenyl)-7-(3-morpholin-4-yl-propoxy)-1,2,3,4-tetrahydro-isoquinolin-6-ol; 8-Fluoro-2-methyl-4-(4-methylsulfanyl-phenyl)-7-(3-morpholin-4-yl-propoxy)-1,2,3,4-tetrahydro-isoquinoline; 6-Fluoro-2-methyl-4-(4-methylsulfanyl-phenyl)-7-(3-morpholin-4-yl-propoxy)-1,2,3,4-tetrahydro-isoquinoline; 7-[1-(2-Fluoro-ethyl)-piperidin-4-ylmethoxy]-2-methyl-4-(4-methylsulfanyl-phenyl)-1,2,3,4-tetrahydro-isoquinoline; 7-[1-(2-Fluoro-ethyl)-piperidin-4-yloxy]-2-methyl-4-(4-methylsulfanyhphenyl)-1,2,3,4-tetrahydro-isoquinoline; 4-(4-Methoxy-phenyl)-7-[3-(3S-methyl-morpholin-4-yl)-propoxy]-1,2,3,4-tetrahydro-isoquinoline; 4-(4-Methanesulfinyl-phenyl)-2-methyl-7-[3-(3S-methyl-morpholin-4-yl)-propoxy]-1,2,3,4-tetrahydro-isoquinoline; 7-[3-(3,3-Difluoro-azetidin-1-yl)-propoxy]-4-(4-methoxy-phenyl)-2-methyl-1,2,3,4-tetrahydro-isoquinoline; (4-{3-[4-(4-Methoxy-phenyl)-2-methyl-1,2,3,4-tetrahydro-isoquinolin-7-yloxy]-propyl}-morpholin-3-yl)-methanol; (4-{3-[4-(4-Methoxy-phenyl)-2-methyl-1,2,3,4-tetrahydro-isoquinolin-7-yloxy]-propyl}-morpholin-3S-yl)-methanol; (4-{3-[4-(4-Methoxy-phenyl)-2-methyl-1,2,3,4-tetrahydro-isoquinolin-7-yloxy]-propyl}-morpholin-2-yl)-methanol;
{3-[2-Methyl-4-(4-methylsulfanyl-phenyl)-1,2,3,4-tetrahydro-isoquinolin-7-yloxy]-propyl}-(2H-pyrazol-3-yl)-amine;
4-(6-Bromo-pyridin-3-yl)-7-[3-(4-fluoro-piperidin-1-yl)-propoxy]-2-methyl-1,2,3,4-tetrahydro-isoquinoline;
7-[3-(4-Fluoro-piperidin-1-yl)-propoxy]-2-methyl-4-(6-methylsulfanyl-pyridin-3-yl)-1,2,3,4-tetrahydro-isoquinoline;
(5-{7-[3-(4-Fluoro-piperidin-1-yl)-propoxy]-2-methyl-1,2,3,4-tetrahydro-isoquinolin-4-yl}-pyridin-2-yl)-dimethyl-amine:
7-[3-(4-Fluoro-piperidin-1-yl)-propoxy]-2-methyl-4-(6-trimethylsilanylethynyl-pyridin-3-yl)-1,2,3,4-tetrahydro-isoquinoline;
4-(6-Ethynyl-pyridin-3-yl)-7-[3-(4-fluoro-piperidin-1-yl)-propoxy]-2-methyl-1,2,3,4-tetrahydro-isoquinoline;
7-[3-(4-Fluoro-piperidin-1-yl)-propoxy]-2-methyl-4-(6-phenylsulfanyl-pyridin-3-yl)-1,2,3,4-tetrahydro-isoquinoline;
7-[3-(4-Fluoro-piperidin-1-yl)-propoxy]-4-(6-imidazol-1-yl-pyridin-3-yl)-2-methyl-1,2,3,4-tetrahydro-isoquinoline;
7-[3-(4-Fluoro-piperidih-1-yl)-propoxy]-4-(6-methoxy-pyridin-3-yl)-2-methyl-1,2,3,4-tetrahydro-isoquinoline;
7-[3-(4-Fluoro-piperidin-1-yl)-propoxy]-2-methyl-4-(6-pyrazol-1-yl-pyridin-3-yl)-1,2,3,4-tetrahydro-isoquinoline;
7-[3-(4-Fluoro-piperidin-1-yl)-propoxy]-4-[6-(1H-imidazol-2-ylsulfanyl)-pyridin-3-yl]-2-methyl-1,2,3,4-tetrahydro-isoquinoline;
7-[3-(4-Fluoro-piperidin-1-yl)-propoxy]-2-methyl-4-[6-(pyrimidin-2-ylsulfanyl)-pyridin-3-yl]-1,2,3,4-tetrahydro-isoquinoline;
4-(4-Methoxy-phenyl)-2-methyl-7-[3-(4-methyl-piperazin-1-yl)-propoxy]-1,2,3,4-tetrahydro-isoquinoline;
7-[3-(4-Ethyl-piperazin-1-yl)-propoxy]-4-(4-methoxy-phenyl)-2-methyl-1,2,3,4-tetrahydro-isoquinoline;
7-[3-(4-Isopropyl-piperazin-1-yl)-propoxy]-4-(4-methoxy-phenyl)-2-methyl-1,2,3,4-tetrahydro-isoquinoline;
4-(4-Methoxy-phenyl)-2-methyl-7-[3-(4-thiazol-2-yl-piperazin-1-yl)-propoxy]-1,2,3,4-tetrahydro-isoquinoline;
4-(4-Methoxy-phenyl)-2-methyl-7-[3-(4-thiophen-2-ylmethyl-piperazin-1-yl)-propoxy]-1,2,3,4-tetrahydro-isoquinoline;
2-(4-{3-[4-(4-Methoxy-phenyl)-2-methyl-1,2,3,4-tetrahydro-isoquinolin-7-yloxy]-propyl}-piperazin-1-yl)-ethanol;
2-(4-{3-[4-(4-Methoxy-phenyl)-2-methyl-1,2,3,4-tetrahydrc-isoquinolin-7-yloxy]-propyl}-piperazin-1-yl)-phenol;
4-(4-Methoxy-phenyl)-2-methyl-7-[3-(4-pyridin-4-yl-piperazin-1-yl)-propoxy]-1,2,3,4-tetrahydro-isoquinoline;
4-(4-Methoxy-phenyl)-2-methyl-7-{3-[4-(4-trifluoromethyl-phenyl)-piperazin-1-yl]-propoxy}-1,2,3,4-tetrahydro-isoquinoline;
7-[3-(4-Allyl-piperazin-1-yl)-propoxy]-4-(4-methoxy-phenyl)-2-methyl-1,2;3,4-tetrahydro-isoquinoline;
7-[3-(4-[1,3]-Dioxolan-2-ylmethyl-piperazin-1-yl)-propoxy]-4-(4-methoxy-phenyl)-2-methyl-1,2,3,4-tetrahydro-isoquinoline;
4-(4-{3-[4-(4-Methoxy-phenyl)-2-methyl-1,2,3,4-tetrahydro-isoquinolin-7-yloxy]-propyl}-piperazin-1-yl)-benzonitrile;
7-{3-[4-(2-Methoxy-ethyl)-piperazin-1-yl]-propoxy}-4-(4-methoxy-phenyl)-2-methyl-1,2,3,4-tetrahydro-isoquinoline;
4-(4-Methoxy-phenyl)-2-methyl-7-{3-[4-(tetrahydro-furan-2-ylmethyl)-piperazin-1-yl]-propoxy}-1,2,3,4-tetrahydro-isoquinoline;
4-{3-[4-(4-Methoxy-phenyl)-2-methyl-1,2,3,4-tetrahydro-isoquinolin-7-yloxy]-propyl}-piperazine-1-carboxylic acid tert-butyl ester;
7-[3-(4-Cyclopropyl-piperazin-1-yl)-propoxy]-4-(4-methoxy-phenyl)-2-methyl-1,2,3,4-tetrahydro-isoquinoline;
4-(4-Bromo-phenyl)-7-[3-(4-fluoro-piperidin-1-yl)-propoxy]-2-methyl-1,2,3,4-tetrahydro-isoquinoline;
4-(4-Difluoromethoxy-phenyl)-2-methyl-7-(3-morpholin-4-yl-propoxy)-1,2,3,4-tetrahydro-isoquinoline;
2-Methyl-7-[3-(3S-methyl-morpholin-4-yl)-propoxy]-4-(4-methylsulfanyl-phenyl)-1,2,3,4-tetrahydro-isoquinoline;
7-[3-(4,4-Difluoro-piperidin-1-yl)-propoxy]-2-methyl-4-(4-methylsulfanyl-phenyl)-1,2,3,4-tetrahydro-isoquinoline;
7-[3-(4-Fluoro-piperidin-1-yl)-propoxy]-2-methyl-4-(4-trifluoromethylsulfanyl-phenyl)-1,2,3,4-tetrahydro-isoquinoline;
2-Methyl-7-(3-morpholin-4-yl-propoxy)-4-(4-trifluoromethylsulfanyl-phenyl)-1,2,3,4-tetrahydro-isoquinoline;
2-Methyl-7-[3-(3S-methyl-morpholin-4-yl)-propoxy]-4-(4-trifluoromethylsulfanyl-phenyl)-1,2,3,4-tetrahydro-isoquinoline;
4-(2-Fluoro-4-methoxy-phenyl)-2-methyl-7-[3-(3S-methyl-morpholin-4-yl)-propoxy]-1,2,3,4-tetrahydro-isoquinoline;
4-(3-Fluoro-4-methoxy-phenyl)-2-methyl-7-[3-(3S-methyl-morpholin-4-yl)-propoxy]-1,2,3,4-tetrahydro-isoquinoline;
2-Methyl-7-(3-piperidin-1-yl-propoxy)-4-(4-trifluoromethoxy-phenyl)-1,2,3,4-tetrahydro-isoquinoline;
{3-[4-(4-Methoxy-phenyl)-2-methyl-1,2,3,4-tetrahydro-isoquinolin-7-yloxy]-propyl}-(tetrahydro-pyran-4-yl)-amine;
Cyclopropyl-{3-[4-(4-methoxy-phenyl)-2-methyl-1,2,3,4-tetrahydro-isoquinolin-7-yloxy]-propyl}-(1-methyl-piperidin-4-yl)-amine;
(2-Methoxy-ethyl)-{3-[4-(4-methoxy-phenyl)-2-methyl-1,2,3,4-tetrahydro-isoquinolin-7-yloxy]-propyl}-amine;
3-{3-[4-(4-Methoxy-phenyl)-2-methyl-1,2,3,4,-tetrahydro-isoquinolin-7-yloxy]-propylamino}-propan-1-ol;
Allyl-{3-[4-(4-methoxy-phenyl)-2-methyl-1,2,3,4-tetrahydro-isoquinolin-7-yloxy]-propyl}-amine;
Isobutyl-{3-[4-(4-methoxy-phenyl)-2-methyl-1,2,3,4-tetrahydro-isoquinolin-7-yloxy]-propyl}-amine;
Cyclopropyl-{3-[4-(4-methoxy-phenyl)-2-methyl-1,2,3,4-tetrahydro-isoquinolin-7-yloxy]-propyl}-amine;
Isopropyl-{3-[4-(4-methoxy-phenyl)-2-methyl-1,2,3,4-tetrahydro-isoquinolin-7-yloxy]-propyl}-amine;
{3-[4-(4-Methoxy-phenyl)-2-methyl-1,2,3,4-tetrahydro-isoquinolin-7-yloxy]-propyl}-propyl-amine;
Ethyl-{3-[4-(4-methoxy-phenyl)-2-methyl-1,2,3,4-tetrahydro-isoquinolin-7-yloxy]-propyl}-amine;
Isopropyl-{3-[4-(4-methoxy-phenyl)-2-methyl-1,2,3,4-tetrahydro-isoquinolin-7-yloxy]-peopyl}-methyl-amine;
Cyclopropyl-{3-[4-(4-methoxy-phenyl)-2-methyl-1,2,3,4-tetrahydro-isoquinolin-7-yloxy]-propyl}-methyl-amine;
Dicyclopropyl-{3-[4-(4-methoxy-phenyl)-2-methyl-1,2,3,4-tetrahydro-isoquinolin-7-yloxy]-propyl}-amine;
7-(1-Isopropyl-azetidin-3-ylmethoxy)-2-methyl-4-(4-methylsulfanyl-phenyl)-1,2,3,4-tetrahydro-isoquinoline;
7-(1-Cyclopropyl-azetidin-3-ylmethoxy)-2-methyl-4-(4-methylsulfanyl-phenyl)- , 1,2,3,4-tetrahydro-isoquinoline;
7-(1-Cyclobutyl-azetidin-3-ylmethoxy)-2-methyl-4-(4-methylsulfanyl-phenyl)-1,2,3,4-tetrahydro-isoquinoline;
7-[1-(2-Fluoro-ethyl)-azetidin-3-ylmethoxy]-2-methyl-4-(4-methylsulfanyl-phenyl)-1,2,3,4-tetrahydro-isoquinoline;
4-(4-Methoxy-phenyl)-2-methyl-7-[2-(1-methyl-pyrrolidin-2-yl)-ethoxy]-1,2,3,4-tetrahydro-isoquinoline;
4-(4-Methoxy-phenyl)-2-methyl-7-[2-(1-methyl-pyrrolidin-2-yl)-ethoxy]-1,2,3,4-tetrahydro-isoquinoline.(diastereomer 1);
4-(4-Methoxy-phenyl)-2-methyl-7-[2-(1-methyl-pyriolidin-2-yl)-ethoxy]-1,2,3,4-tetrahydro-isoquinoline (diastereomer 2);
4-(3-Methoxy-phenyl)-2-methyl-7-(piperidin-4-ylmethoxy)-1,2,3,4-tetrahydro-isoquinoline;
{4-[4-(4-Methoxy-phenyl)-2-methyl-1,2,3,4-tetrahydro-isoquinolin-7-yloxymethyl]-piperidin-1-yl}-acetonitrile;
2-Methyl-7-(1-methyl-piperidin-4-yloxy)-4-(4-methylsulfanyl-phenyl)-1,2,3,4-tetrahydro-isoquinoline;
2-Methyl-4-(4-methylsulfanyl-phenyl)-7-[1-(3,3,3-trifluoro-propyl)-piperidin-4-yloxy]-1,2,3,4-tetrahydro-isoquinoline;
{4-[2-Methyl-4-(4-methylsulfanyl-phenyl)-1,2,3,4-tetrahydro-isoquinolin-7-yloxy]-piperidin-1-yl}-acetonitrile;
2-Methyl-4-(4-methylsulfanyl-phenyl)-7-[1-(tetrahydro-pyran-4-yl)-piperidin-4-yloxy]-1,2,3,4-tetrahydro-isoquinoline;
7-(1-Cyclopropyl-piperidin-4-yloxy)-2-methyl-4-(4-methylsulfanyl-phenyl)-1,2,3,4-tetrahydro-isoquinoline;
7-(1-Cyclobutyl-piperidin-4-ylmethoxy)-4-(4-methoxy-phenyl)-2-methyl-1,2,3,4-tetrahydro-isoquinoline;
7-(1-Cyclopropyl-piperidin-4-ylmethoxy)-4-(4-methoxy-phenyl)-2-methyl-1,2,3,4-tetrahydro-isoquinoline;
1-{4-[4-(4-Methoxy-phenyl)-2-methyl-1,2,3,4-tetrahydro-isoquinolin-7-yloxymethyl]-piperidin-1-yl}-2-methyl-propan-2-ol;
4-(4-Methoxy-phenyl)-2-methyl-7-(4-methyl-morpholin-2-ylmethoxy)-1,2,3,4-tetrahydro-isoquinoline;
4-(4-Methoxy-phenyl)-2-methyl-7-(morpholin-2S-ylmethoxy)-1,2,3,4-tetrahydro-isoquinoline;
4-(4-Methoxy-phenyl)-2-methyl-7-(morpholin-2R-ylmethoxy)-1,2,3,4-tetrahydro-isoquinoline;
7-(4-Isopropyl-morpholin-2-ylmethoxy)-4-(4-methoxy-phenyl)-2-methyl-1,2,3,4-tetrahydro-isoquinoline;
7-(4-Isopropyl-morpholin-2S-ylmethoxy)-4-(4-methoxy-phenyl)-2-methyl-1,2,3,4-tetrahydro-isoquinoline;
7-(4-Isopropyl-morpholin-2R-ylmethoxy)-4-(4-methoxy-phenyl)-2-methyl-1,2,3,4-tetrahydro-isoquinoline;
7-(4-Isopiopyl-morpholin-2R-ylmethoxy)-4-(4-methoxy-phenyl)-2-methyl-1,2,3,4-tetrahydro-isoquinoline (diastereomer 1);
7-(4-Isopropyl-morpholin-2R-ylmethoxy)-4-(4-methoxy-phenyl)-2-methyl-1,2,3,4-tetrahydro-isoquinoline (diastereomer 2);
7-(4-Ethyl-moroholin-2-ylmethoxy)-4-(4-methoxy-phenyl)-2-methyl-1,2,3,4-tetrahydro-isoquinoline;
7-[4-(2-Fluoro-ethyl)-morpholin-2-ylmethoxy]-4-(4-methoxy-phenyl)-2-methyl-1,2,3,4-tetrahydro-isoquinoline;
7-(4-Cyclopropyl-morpholin-2-ylmethoxy)-4-(4-methoxy-phenyl)-2-methyl-1,2,3,4-tetrahydro-isoquinoline;
7-(4-Cyclopropyl-morpholin-2S-ylmethoxy)-4-(4-methoxy-phenyl)-2-methyl-1,2,3,4-tetrahydro-isoquinoline;
7-(4-Cyclopropyl-morpholin-2S-ylmethoxy)-4-(4-methoxy-phenyl)-2-methyl-1,2,3,4-tetrahydro-isoquinoline (diastereomer 1);
7-(4-Cyclopropyl-morpholin-2S-ylmethoxy)-4-(4-methoxy-phenyl)-2-methyl-1,2,3,4-tetrahydro-isoquinoline (diastereomer 2);
7-(4-Cyclopropyl-morpholin-2R-ylmethoxy)-4-(4-methoxy-phenyl)-2-methyl-1,2,3,4-tetrahydro-isoquinoline;
7-(4-Cyclopropyl-morpholin-2R-ylmethoxy)-4-(4-methoxy-phenyl)-2-methyl-1,2,3,4-tetrahydro-isoquinoline (diastereomer 1);
7-(4-Cyclopropyl-morpholin-2R-ylmethoxy)-4-(4-methoxy-phenyl)-2-methyl-1,2,3,4-tetrahydro-isoquinoline (diastereomer 2);
7-(4-Isopropyl-morpholin-2-ylmethoxy)-2-methyl-4-(4-methylsulfanyl-phenyl)-1,2,3,4-tetrahydro-isoquinoline;
7-(4-Cyclopropyl-morpholin-2-ylmethoxy)-2-methyl-4-(4-methylsulfanyl-phenyl)-1,2,3,4-tetrahydro-isoquinoline;
2-Methyl-4-(4-methylsulfanyl-phenyl)-7-(morpholin-2-ylmethoxy)-1,2,3,4-tetrahydro-isoquinoline;
4-(4-Methoxy-phenyl)-2,6-dimethyl-7-(3-piperidin-1-yl-propoxy)-1,2,3,4-tetrahydro-isoquinoline;
4-(4-Methoxy-phenyl)-2,6-dimethyl-7-(3-morpholin-4-yl-propoxy)-1,2,3,4-tetrahydro-isoquinoline;
4-(3-Fluoro-4-methoxy-phenyl)-2,6-dimethyl-7-(3-morpholin-4-yl-propoxy)-1,2,3,4-tetrahydro-isoquinoline;
4-(3-Fluoro-4-methoxy-phenyl)-2,8-dimethyl-7-(3-morpholin-4-yl-propoxy)-1,2,3,4-tetrahydro-isoquinoline;
4-(4-Methoxy-phenyl)-2,8-dimethyl-7-(3-morpholin-4-yl-propoxy)-1,2,3,4-tetrahydro-isoquinoline;
2,6-Dimethyl-4-(4-methylsulfanyl-phenyl)-7-(3-piperldin-1-yl-propoxy)-1,2,3,4-tetrahydro-isoquinoline;
7-(4-Piperidin-1-yl-but-1-ynyl)-4-pyridin-3-yl-1,2,3,4-tetrahydro-isoquinoline;
7-(4-Piperidin-1-yl-butyl)-4-pyridin-3-yl-1,2,3,4-tetrahydro-isoquinoline;
2-Methyl-7-(4-piperidin-1-yl-butyl)-4-pyridin-3-yl-1,2,3,4-tetrahydro-isoquinoline;
7-[4-(4,4-Difluoro-piperidin-1-yl)-but-1-ynyl]-4-(4-methoxy-phenyl)-2-methyl-1,2,3,4-tetrahydro-isoquinoline;
4-(4-Methoxy-phenyl)-2-methyl-7-(4-piperidin-1-yl-but-1-ynyl)-1,2,3,4-tetrahydro-isoquinoline;
4-(4-Methoxy-phenyl)-2-methyl-7-(4-morpholin-4-yl-but-1-ynyl)-1,2,3,4-tetrahydro-isoquinoline;
4-(4-Methoxy-phenyl)-2-methyl-7-(4-thiomorpholin-4-yl-but-1-ynyl)-1,2,3,4-tetrahydro-isoquinoline;
7-[4-(4-Isopropyl-piperazin-1-yl)-but-1-ynyl]-4-(4-methoxy-phenyl)-2-methyl-1,2,3,4-tetrahydro-isoquinoline;
4-(4-Fluoro-phenyl)-2-methyl-7-(4-piperidin-1-yl-but-1-ynyl)-1,2,3,4-tetrahydro-isoquinoline;
7-[4-(4,4-Difluoro-piperidin-1-yl)-butyl]-4-(4-methoxy-phenyl)-2-methyl-1,2,3,4-tetrahydro-isoquinoline;
4-(4-Methoxy-phenyl)-2-methyl-7-(4-morpholin-4-yl-butyl)-1,2,3,4-tetrahydro-isoquinoline;
4-(4-Methoxy-phenyl)-2-methyl-7-(4-thiomorpholin-4-yl-butyl)-1,2,3,4-tetrahydro-isoquinoline;
7-[4-(4-isopropyl-piperazin-1-yl)-butyl]-4-(4-methoxy-phenyl)-2-methyl-1,2,3,4-tetrahydro-isoquinoline;
and salts thereof.

65. A pharmaceutical composition comprising a pharmaceutically acceptable carrier and an effective amount of a compound of Formula (I): 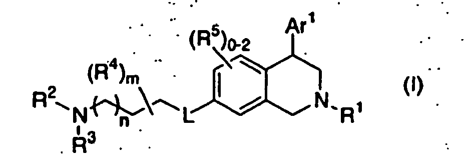 wherein
L is -O- and n is 1 or 2; or L is -C≡C- or -CH₂CH₂- and n is 0 or 1;
R¹ is -H; or is -C₁₋₆alkyl, -C₃₋₆alkenyl, -C₃₋₆alkynyl, -C₃₋₇cycloalkyl, -C₁₋₆alkylC₃₋₇cycloalkyl, -COOC₁₋₆alkyl, or -COObenryl, each optionally mono-, di-, or tri-substituted with R^{a};
where R^{a} is selected from -OH, -OC₁₋₆alkyl, phenyl optionally substituted with -OC₁₋₄alkyl or halo, -CN, -NO₂, -N(R^{b})R^{c}, -C(O)N(R^{b})R^{c}, -N(R^{b})C(O)R^{b}, -N(R^{b})SO₂C₁₋₆alkyl, -C(O)C₁₋₆alkyl, -S(O)₀₋₂-C₁₋₆alkyl, -SO₂N(R^{b})R^{c}, -SCF₃, halo, -CF₃, -OCF₃, -COOH, and -COOC₁₋₆alkyl;
wherein R^{b} and R^{c} are each independently -H or -C₁₋₆alkyl;
R² and R³ are each independently selected from the group consisting of: -H;
A) -C₁₋₆alkyl, -C₃₋₆alkenyl, -C₃₋₆alkynyl, -C₃₋₇cycloalkyl, -C₁₋₆alkylC₁₋₇cycloalkyl, -CH(C₃₋₈cycloalkyl)₂, -CH(phenyl)₂, benzyl, and -C(O)OC₁₋₄alkyl, wherein each alkyl, cycloalkyl, or benzyl is optionally substituted with -OH, -OC₁₋₄alkyl, -CN, -NH₂, -NH(C₁₋₄alkyl), -N(C₁₋₄alkyl)₂, halo, -CF₃, -OCF₃, -COOH, or -COOC₁₋₆alkyl;
B) phenyl or pyridyl, optionally fused at two adjacent carbon ring members to a three- for four-membered hydrocarbon moiety to form a fused five- or six-membered aromatic ring, which moiety has one carbon atom replaced by, >O, >S, >NH, >N(C₁₋₄alkyl), or >NC(O)OC₁₋₄alkyl, and which moiety has up to one additional carbon atom optionally replaced by -N=;
C) naphthyl,
D) a 4-8 membered heterocyclic ring, said heterocyclic ring having a carbon atom which is the point of attachment, having 1 or 2 heteroatom members selected from the group consisting of >O, >S(O)₀₋₂, >NH, >N(C₁₋₄alkyl), and >NC(O)OC₁₋₄alkyl, and having 0 or 1 double bonds; and
E) a monocyclic aromatic hydrocarbon group having five or six ring atoms, having a carbon atom which is the point of attachment, having one carbon atom replaced by >O, >S, >NH, >N(C₁₋₄alkyl), or >NC(O)OC₁₋₄alkyl, having up to one additional carbon atom optionally replaced by -N=, and optionally benzofused or pyridofused;
where each of B)-E) are optionally mono-, di-, or tri-substituted with a moiety selected from the group consisting of -C₁₋₄alkyl, -OH, -C₁₋₄alkylOH, -OC₁₋₆alkyl, -CN, -NO₂, -N(R^{d})R^{e} -C(O)N(R^{d})R^{e}, -N(R^{d})C(O)R^{d}, -N(R^{d})SO₂C₁₋₆alkyl, -C(O)C₁₋₆alkyl, -S(O)₀₋₂-C₁₋₆alkyl, -SO₂N(R^{d})R^{e}, -SCF₃, halo, -CF₃, -OCF₃, -COOH, -COOC₁₋₆alkyl, -OC(O)N(R^{d})R^{e}, and -OC(O)OC₁₋₆alkyl;
where R^{d} and R^{e} are each independently -H or -C₁₋₆alkyl;
or, alternatively,
R² and R³ may be taken together with the nitrogen to which they are attached to form tetrahydro-pyrimidin-2-ylidenyl, or a 4-8 membered heterocyclic ring, said heterocyclic ring having 0 or 1 additional heteroatom members separated from the nitrogen of attachment by at least one carbon member and selected from the group consisting of >O, >S(O)₀₋₂, >NH, and >NR^{f}, having 0 or 1 double bonds, having 0, 1 or 2 carbon members separated from the nitrogen of attachment by at least one carbon member which is a carbonyl, optionally benzo or pyrido fused, optionally having one carbon member that forms a bridge, and having 0-5 carbon member substituents R^{ff},
where R^{f} is selected from the group consisting of:
i) -C₁₋₆alkyl optionally substituted with R^{z}, -C₃₋₆alkenyl, -C₃₋₆alkynyl, -C(O)N(R^{g})R^{h}, -C(O)Rⁱ, -S(O)₀₋₂-C₁₋₆alkyl, and -COOC₁₋₆alkyl,
where R^{z} is fluoro, -CN, -OH, -OC₁₋₄alkyl, -C₃₋₇cycloalkyl, or -CF₃;
where R^{g} and R^{h} are each independently -H or -C₁₋₆alkyl; and
where Rⁱ is -C₁₋₆alkyl, -C₃₋₈cycloalkyl, phenyl, or 5- or 6-membered aromatic heterocyclyl, where each alkyl, cycloalkyl, phenyl or heterocyclyl is optionally mono-, di-, or tri-substituted with -C₁₋₄alkyl, -OH, -OC₁₋₆alkyl, -CF₃, -CN, or halo;
ii) -(CH₂)₀₋₁-RingA, where Ring A is phenyl or a 5- or 6-membered carbon-linked aromatic heterocyclyl, optionally substituted with R^{aa};
where R^{aa} is -H, -C₁₋₆alkyl, -OC₁₋₆alkyl, phenyl, -CN, -NO₂, -N(R^{g})R^{h} -C(O)N(R^{g})R^{h}, -N(R^{g})C(O)R^{h}, -N(R^{h})SO₂C₁₋₆alkyl, -C(O)C₁₋₆alkyl, -S(O)₀₋₂-C₁₋₆alkyl, -SO₂N(R^{g})R^{h}, -SCF₃, halo, -CF₃, -OCF₃, -OCHF₂, -COOH, and -COOC₁₋₆alkyl; and
iii) -(CH₂)₀₋₁-RingB, where Ring B is -C₃₋₇cycloalkyl with one or two carbon ring members optionally replaced with >O, or >NH, and optionally substituted with -C₁₋₄alkyl, fluoro, -CN, -OH, -OC₁₋₄alkyl, or -CF₃;
where R^{ff} is selected from the group consisting of -C₁₋₆alkyl optionally mono-or di-substituted with R^{z}, -C₂₋₆alkenyl, -C₂₋₆alkynyl, -C₃₋₇cycloalkyl, -CH(phenyl)₂, halo, -OH, -OC₁₋₆alkyl, -OC₂₋₃alkylO-, -CN, -NO₂, -N(R^{g})R^{h}, -C(O)N(R^{g})R^{h}, -N(R^{g})C(O)R^{g}, -N(R^{g})SP₂C₁₋₆alkyl, -C(O)Rⁱ, -S(O)₀₋₂-C₁₋₆alkyl, -SO₂N(R^{g})R^{h}, -SCF₃, -CF₃, -OCF₃, -COOH, and -COOC₁₋₆alkyl;
R⁴ is -OH, -OC₁₋₆alkyl, -CF₃, -C₁₋₆alkyl, or halo; two R⁴ substituents may be taken together to form methylene or ethylene; or one of R⁴ is taken together with R² to form methylene, ethylene, propylene, or -CH₂CH₂O-, wherein each is optionally substituted with -OH, -OC₁₋₆alkyl, -SC₁₋₆alkyl, -CF₃, -C₁₋₆alkyl, amino, or halo;
m is 0, 1, or 2;
R⁵ is selected from the group consisting of -C₁₋₆alkyl, -OH, -OC₁₋₆alkyl, -SC₁₋₆alkyl, and halo;
Ar¹ is an aryl or heteroaryl ring selected from the group consisting of:
a) phenyl, optionally mono-, di-, or tri-substituted with R^{j} and optionally di-substituted on adjacent carbons with -OC₁₋₄alkyleneO- optionally mono-or di-substituted with fluoro, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
where R^{j} is selected from the group consisting of
1) -OH, -C₁₋₆alkyl, -OC₁₋₆alkyl optionally mono-, di-, or tri-substituted with halo, -C₂₋₆alkenyl, -OC₃₋₆alkenyl, -C₂₋₆alkynyl optionally substituted with trimethylsilyl, -OC₃₋₆alkynyl, -C₃₋₆cycloalkyl, -OC₃₋₆cycloalkyl, -CN, -NO₂, -N(R^{k})R^{l}, -N(R^{k})C(O)R^{l}, -N(R^{k})SO₂C₁₋₆alkyl, -C(O)C₁₋₆alkyl, -S(O)₀₋₂-C₁₋₆alkyl, -C(O)N(R^{m})Rⁿ, -SO₂N(R^{m})Rⁿ, -SCF₃, halo, -CF₃, -COOH, -COOC₁₋₆alkyl, and -COOC₃₋₇cycloalkyl;
where R^{k} and R^{l} are each independently -H or -C₁₋₆alkyl;
where R^{m} and Rⁿ are each independently -H or -C₁₋₆alkyl, or R^{m} and Rⁿ taken together with their nitrogen of attachment form a 4-8 membered heterocyclic ring having 1 or 2 heteroatom members selected from >O, >S(O)₀₋₂, >NH, and >NC₁₋₆alkyl, having 0 or 1 double bonds, having 0 or 1 carbonyl members;
2) -G-Ar², where G is a bond, -O-, or -S-, and Ar² is phenyl or is a monocyclic aromatic hydrocarbon group having five or six ring atoms, having one carbon atom replaced by >O, >S, >NH, or >N(C₁₋₄alkyl), having up to one additional carbon atom optionally replaced by -N=, each optionally mono-, di-, or tri-substituted with R^{p};
where R^{p} is a substituent independently selected from the group consisting of: -OH, -C₁₋₆alkyl, -OC₁₋₆alkyl, phenyl, -CN, -NO₂, -N(R^{q})R^{r}, -C(O)N(R^{q})R^{r}, -N(R^{q})C(O)R^{r}, -N(R^{q})SO₂C₁₋₆alkyl, -C(O)C₁₋₆alkyl, -S(O)₀₋₂-C₁₋₆alkyl, -SO₂N(R^{q})R^{r}, -SCF₃, halo, -CF₃, -OCF₃, -OCHF₂, -COOH, and -COOC₁₋₆alkyl;
wherein R^{q} and R^{r} are each independently selected from -H, -C₁₋₆alkyl, and -C₂₋₆alkenyl; and
3) a 4-8 membered saturated or partially saturated heterocyclic ring, having 1 or 2 heteroatom members selected from >O, >S(O)₀₋₂, >NH, and >NC₁₋₆alkyl, having 0 or 1 carbonyl members, said ring optionally mono-, di-, or tri-substituted with R^{p};
b) phenyl or pyridyl fused at two adjacent carbon ring members to a three membered hydrocarbon moiety to form a fused five membered aromatic ring, which moiety has one carbon atom replaced by >O, >S, >NH, or >N(C₁₋₄alkyl), and which moiety has up to one additional carbon atom optionally replaced by -N=, the fused rings optional mono-, di-, or tri-substituted with R^{t};
where R^{t} is a substituent independently selected from the group consisting of: -OH, -C₁₋₆alkyl, -OC₁₋₆alkyl, phenyl, -CN, -NO₂, -N(R^{u})R^{v}, -C(O)N(R^{u})R^{v}, -N(R^{u})C(O)R^{v}, -N(R^{u})SO₂C₁₋₆alkyl, -C(O)C₁₋₆alkyl, -S(O)₀₋₂-C₁₋₆alkyl, -SO₂N(R^{u})R^{v}, -SCF₃, halo, -CF₃, -OCF₃, -OCHF₂, -COOH, and -COOC₁₋₆alkyl;
where R^{u} and R^{v} are each independently -H or -C₁₋₆alkyl;
c) phenyl fused at two adjacent ring members to a four membered hydrocarbon moiety to form a fused six membered aromatic ring, which moiety has one or two carbon atoms replaced by -N=, the fused rings optionally mono-, di-, or tri-substituted with R^{t};
d) naphthyl, optionally mono-, di-, or tri-substituted with R^{t};
e) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atom optionally replaced by -N=, optionally mono- or di-substituted with R^{j} and optionally benzofused or pyridofused at two adjacent carbon atoms, where the benzofused or pyridofused moiety is optionally mono-, di-, or tri-substituted with R^{t}; and
f) a monocyclic aromatic hydrocarbon group having six ring atoms, having a carbon atom which is the point of attachment, having one or two carbon atoms replaced by -N=, optionally mono- or di-substituted with R^{j} and optionally benzofused or pyridofused at two adjacent carbon atoms, where the benzofused or pyridofused moiety is optionally mono- or di-substituted with R^{l};
and enantiomers, diastereomers, hydrates, solvates and pharmaceutically acceptable salts, esters and amides thereof,
with the proviso:
when n is 1, L is -O-, Ar is unsubstituted phenyl and R² and R³ are both methyl, then R⁴ is not -OH.

## Patentansprüche

1. Verbindung von Formel (I): 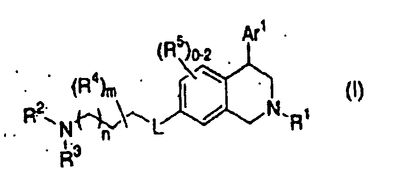 worin
L -O- ist und n 1 oder 2 ist; oder L -C≡C- oder -CH₂CH₂- ist und n 0 oder 1 ist;
R¹ -H ist; oder -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -COOC₁₋₆-Alkyl oder -COOBenzyl ist, von denen jedes gegebenenfalls einfach, zweifach oder dreifach mit R^{a} substituiert ist;
wobei R^{a} ausgewählt ist aus -OH, -OC₁₋₆-Alkyl, Phenyl, gegebenenfalls substituiert mit -OC₁₋₄-Alkyl oder Halo, -CN, -NO₂, -N(R^{b})R^{c}, -C(O)N(R^{b})R^{c}, -N(R^{b})C(O)R^{b}, -N(R^{b})SO₂C₁₋₆-Alkyl, -C(O)C₁₋₆-Alkyl, -S(O)₀₋₂-C₁₋₆-Alkyl, -SO₂N(R^{b})R^{c}, -SCF₃, Halo, -CF₃, -OCF₃, -COOH und -COOC₁₋₆-Alkyl;
wobei R^{b} und R^{c} jeweils unabhängig -H oder -C₁₋₆-Alkyl sind;
R² und R³ jeweils unabhängig ausgewählt sind aus der Gruppe, bestehend aus : -H;
A) -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -CH(C₃₋₈-Cycloalkyl)₂, -CH(Phenyl)₂, Benzyl und -C(O)OC₁₋₄-Alkyl, wobei jedes Alkyl, Cycloalkyl oder Benzyl gegebenenfalls mit -OH, -OC₁₋₄-Alkyl, -CN, -NH₂, -NH(C₁₋₄-Alkyl), -N(C₁₋₄-Alkyl)₂, Halo, -CF₃, -OCF₃, -COOH oder -COOC₁₋₆-Alkyl substituiert ist;
B) Phenyl oder Pyridyl, gegebenenfalls an zwei benachbarten Kohlenstoffringgliedern an eine drei- oder viergliedrige Kohlenwasserstoffeinheit kondensiert, um einen kondensierten fünf- oder sechsgliedrigen aromatischen Ring zu bilden, wobei in der Einheit ein Kohlenstoffatom durch >O, >S, >NH, >N(C₁₋₄-Alkyl) oder >NC(O)OC₁₋₄-Alkyl ersetzt ist und wobei in der Einheit bis zu ein zusätzliches Kohlenstoffatom gegebenenfalls durch -N= ersetzt ist;
C) Naphthyl;
D) einem 4- bis 8-gliedrigen heterocyclischen Ring, wobei der heterocyclische Ring ein Kohlenstoffatom aufweist, das der Bindungspunkt ist, 1 oder 2 Heteroatomglieder aufweist, die ausgewählt sind aus der Gruppe, bestehend aus >O, >S(O)₀₋₂, >NH, >N(C₁₋₄-Alkyl) und >NC(O)OC₁₋₄-Alkyl, und 0 oder 1 Doppelbindungen aufweist; und
E) einer monocyclischen aromatischen Kohlenwasserstoffgruppe, die fünf oder sechs Ringatome aufweist, die ein Kohlenstoffatom aufweist, das der Bindungspunkt ist, in der ein Kohlenstoffatom durch >O, >S, >NH, >N(C₁₋₄-Alkyl) oder >NC(O)OC₁₋₄-Alkyl ersetzt ist, in der bis zu ein zusätzliches Kohlenstoffatom gegebenenfalls durch -N= ersetzt ist und die gegebenenfalls benzokondensiert oder pyridokondensiert ist;
wobei B)-E) jeweils gegebenenfalls einfach, zweifach oder dreifach mit einer Einheit substituiert sind, die ausgewählt ist aus der Gruppe, bestehend aus -C₁₋₄-Alkyl, -OH, -C₁₋₄-AlkylOH, -OC₁₋₆-Alkyl, -CN, -NO₂, -N(R^{d})R^{e}, -C(O)N(R^{d})R^{e}, -N(R^{d})C(O)R^{d}, -N(R^{d})SO₂C₁₋₆-Alkyl, -C(O)C₁₋₆-Alkyl, -S(O)₀₋₂-C₁₋₆-Alkyl, -SO₂N(R^{d})R^{e}, -SCF₃, Halo, -CF₃, -OCF₃, -COOH, -COOC₁₋₆-Alkyl, -OC(O)N(R^{d})R^{e} und -OC(O)OC₁₋₆-Alkyl;
wobei R^{d} und R^{e} jeweils unabhängig -H oder -C₁₋₆-Alkyl sind,
oder, alternativ,
R² und R³ mit dem Stickstoff, an das sie gebunden sind, zusammengenommen sein können, um Tetrahydropyrimidin-2-ylidenyl oder einen 4- bis 8-gliedrigen heterocyclischen Ring zu bilden, wobei der heterocyclische Ring 0 oder 1 zusätzliche Heteroatomglieder aufweist, die vom Bindungsstickstoff durch wenigstens ein Kohlenstoffglied getrennt sind und ausgewählt sind aus der Gruppe, bestehend aus >O, >S(O)₀₋₂, >NH und >NR^{f}, 0 oder 1 Doppelbindungen aufweist, 0, 1 oder 2 Kohlenstoffglieder aufweist, die vom Bindungsstickstoff durch wenigstens ein Kohlenstoffglied getrennt sind, das ein Carbonyl ist, gegebenenfalls benzo- oder pyridokondensiert ist, gegebenenfalls ein Kohlenstoffglied aufweist, das eine Brücke bildet, und 0-5 Kohlenstoffgliedsubstituenten R^{ff} aufweist,
wobei R^{f} ausgewählt ist aus der Gruppe, bestehend aus:
i) -C₁₋₆-Alkyl, gegebenenfalls substituiert mit R^{z}, -C₃₋₆-Alkenyl, -C₃₋₆-Alkinyl, -C(O)N(R^{g})R^{h}, -C(O)Rⁱ, -S(O)₀₋₂-C₁₋₆-Alkyl und -COOC₁₋₆-Alkyl,
wobei R^{z} Fluor, -CN, -OH, -OC₁₋₄-Alkyl, -C₃₋₇-Cycloalkyl oder -CF₃ ist;
wobei R^{g} und R^{h} jeweils unabhängig -H oder -C₁₋₆-Alkyl sind; und
wobei Rⁱ -C₁₋₆-Alkyl, -C₃₋₈-Cycloalkyl, Phenyl oder 5-oder 6-gliedriges aromatisches Heterocyclyl ist, wobei jedes Alkyl, Cycloalkyl, Phenyl oder Heterocyclyl gegebenenfalls einfach, zweifach oder dreifach mit -C₁₋₄-Alkyl, -OH, -OC₁₋₆-Alkyl, -CF₃, -CN oder Halo substituiert ist;
ii) -(CH₂)₀₋₁-RingA, wobei Ring A Phenyl oder ein 5- oder 6-gliedriges kohlenstoffverknüpftes aromatisches Heterocyclyl ist, gegebenenfalls substituiert mit R^{aa};
wobei R^{aa} -OH, -C₁₋₆-Alkyl, -OC₁₋₆-Alkyl, Phenyl, -CN, -NO₂, -N(R^{g})R^{h}, -C(O)N(R^{g})R^{h}, -N(R^{g})C(O)R^{h}, -N(R^{h})SO₂C₁₋₆-Alkyl, -C(O)C₁₋₆-Alkyl, -S(O)₀₋₂-C₁₋₆-Alkyl, -SO₂N(R^{g})R^{h}, -SCF₃, Halo, -CF₃, -OCF₃, -OCHF₂, -COOH und -COOC₁₋₆-Alkyl ist; und
iii) -(CH₂)₀₋₁-RingB, wobei Ring B -C₃₋₇-Cycloalkyl ist, in dem ein oder zwei Kohlenstoffringglieder gegebenenfalls durch >O oder >NH ersetzt sind und das gegebenenfalls mit -C₁₋₄-Alkyl, Fluor, -CN, -OH, -OC₁₋₄-Alkyl oder -CF₃ substituiert ist;
wobei R^{ff} ausgewählt ist aus der Gruppe, bestehend aus -C₁₋₆-Alkyl, gegebenenfalls einfach oder zweifach substituiert mit R^{z}, -C₂₋₆-Alkenyl, -C₂₋₆-Alkinyl, -C₃₋₇-Cycloalkyl, -CH(Phenyl)₂, Halo, -OH, -OC₁₋₆-Alkyl, -OC₂₋₃-Alkyl-O-, -CN, -NO₂, -N(R^{g})R^{h}, -C(O)N(R^{g})R^{h}, -N(R^{g})C(O)R^{g}, -N(R^{g})SO₂C₁₋₆-Alkyl, -C(O)Rⁱ, -S(O)₀₋₂-C₁₋₆-Alkyl, -SO₂N(R^{g})R^{h}, -SCF₃, -CF₃, -OCF₃, -COOH und -COOC₁₋₆-Alkyl;
R⁴ -OH, -OC₁₋₆-Alkyl, -CF₃, -C₁₋₆-Alkyl oder Halo ist; zwei R⁴-Substituenten zusammengenommen sein können, um Methylen oder Ethylen zu bilden; oder eines von R⁴ mit R² zusammengenommen ist, um Methylen, Ethylen, Propylen oder -CH₂CH₂O- zu bilden, wobei jedes gegebenenfalls mit -OH, -OC₁₋₆-Alkyl, -SC₁₋₆-Alkyl, -CF₃, -C₁₋₆-Alkyl, Amino oder Halo substituiert ist;
m 0, 1 oder 2 ist;
R⁵ ausgewählt ist aus der Gruppe, bestehend aus -C₁₋₆-Alkyl, -OH, -OC₁₋₆-Alkyl, -SC₁₋₆-Alkyl und Halo;
Ar¹ ein Aryl- oder Heteroarylring ist, der ausgewählt ist aus der Gruppe, bestehend aus:
a) Phenyl, gegebenenfalls einfach, zweifach oder dreifach substituiert mit R^{j} und gegebenenfalls auf benachbarten Kohlenstoffen zweifach substituiert mit -OC₁₋₄-AlkylenO-, gegebenenfalls einfach oder zweifach substituiert mit Fluor, -(CH₂)₂₋₃NH-, -(CH₂)₁₋₂-NH(CH₂)-, -(CH₂)₂₋₃N(C₁₋₄-Alkyl)- oder -(CH₂)₁₋₂N(C₁₋₄-Alkyl)(CH₂)-;
wobei R^{j} ausgewählt ist aus der Gruppe, bestehend aus
1) -OH, -C₁₋₆-Alkyl, -OC₁₋₆-Alkyl, gegebenenfalls einfach, zweifach oder dreifach substituiert mit Halo, -C₂₋₆-Alkenyl, -OC₃₋₆-Alkenyl, -C₂₋₆-Alkinyl, gegebenenfalls substituiert mit Trimethylsilyl, -OC₃₋₆-Alkinyl, -C₃₋₆-Cycloalkyl, -OC₃₋₆-Cycloalkyl, -CN, -NO₂, -N(R^{k})R^{l}, -N(R^{k})C(O)R^{l}, -N(R^{k})SO₂C₁₋₆-Alkyl, -C(O)C₁₋₆-Alkyl, -S(O)₀₋₂-C₁₋₆-Alkyl, -C(O)N(R^{m})Rⁿ, -SO₂N(R^{m})Rⁿ, -SCF₃, Halo, -CF₃, -COOH, -COOC₁₋₆-Alkyl und -COOC₃₋₇-Cycloalkyl;
wobei R^{k} und R^{l} jeweils unabhängig -H oder -C₁₋₆-Alkyl sind;
wobei R^{m} und Rⁿ jeweils unabhängig -H oder -C₁₋₆-Alkyl sind oder R^{m} und Rⁿ mit ihrem Bindungsstickstoff zusammengenommen einen 4- bis 8-gliedrigen heterocyclischen Ring bilden, der 1 oder 2 Heteroatomglieder aufweist, die ausgewählt sind aus >O, >S(O)₀₋₂, >NH und >NC₁₋₆-Alkyl, der 0 oder 1 Doppelbindungen aufweist, der 0 oder 1 Carbonylglieder aufweist;
2) -G-Ar², wobei G eine Bindung, -O- oder -S- ist und Ar² Phenyl ist oder eine monocyclische aromatische Kohlenwasserstoffgruppe ist, die fünf oder sechs Ringatome aufweist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die jeweils gegebenenfalls einfach, zweifach oder dreifach mit R^{p} substituiert ist;
wobei R^{p} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{q})R^{r}, -C(O)N(R^{q})R^{r}, -N(R^{q})C(O)R^{r}, -N(R^{q})SO₂C₁₋₆-Alkyl, -C(O)C₁₋₆-Alkyl, -S(O)₀₋₂-C₁₋₆-Alkyl, -SO₂N(R^{q})R^{r}, -SCF₃, Halo, -CF₃, -OCF₃, -OCHF₂, -COOH und -COOC₁₋₆-Alkyl;
wobei R^{q} und R^{r} jeweils unabhängig ausgewählt sind aus -H, -C₁₋₆-Alkyl und -C₂₋₆-Alkenyl; und
3) einem 4- bis 8-gliedrigen gesättigten oder teilweise gesättigten heterocyclischen Ring, der 1 oder 2 Heteroatomglieder aufweist, die ausgewählt sind aus >O, >S(O)₀₋₂, >NH und >NC₁₋₆-Alkyl, der 0 oder 1 Carbonylglieder aufweist, wobei der Ring gegebenenfalls einfach, zweifach oder dreifach mit R^{p} substituiert ist;
b) Phenyl oder Pyridyl, an zwei benachbarten Kohlenstoffringgliedern an eine dreigliedrige Kohlenwasserstoffeinheit kondensiert, um einen kondensierten fünfgliedrigen aromatischen Ring zu bilden, wobei in der Einheit ein Kohlenstoffatom durch >O, >S, >NH oder >N(C₁₋₄-Alkyl) ersetzt ist und wobei in der Einheit bis zu ein zusätzliches Kohlenstoffatom gegebenenfalls durch -N= ersetzt ist, wobei die kondensierten Ringe gegebenenfalls einfach, zweifach oder dreifach mit R^{t} substituiert sind;
wobei R^{t} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{u})R^{v}, -C(O)N(R^{u})R^{v}, -N(R^{u})C(O)R^{v}, -N(R^{u})SO₂C₁₋₆-Alkyl, -C(O)C₁₋₆-Alkyl, -S(O)₀₋₂-C₁₋₆-Alkyl, -SO₂N(R^{u})R^{v}, -SCF₃, Halo, -CF₃, -OCF₃, -OCHF₂, -COOH und -COOC₁₋₆-Alkyl;
wobei R^{u} und R^{v} jeweils unabhängig -H oder -C₁₋₆-Alkyl sind;
c) Phenyl, an zwei benachbarten Ringgliedern an eine viergliedrige Kohlenwasserstoffeinheit kondensiert, um einen kondensierten sechsgliedrigen aromatischen Ring zu bilden, wobei in der Einheit ein oder zwei Kohlenstoffatome durch -N= ersetzt sind, wobei die kondensierten Ringe gegebenenfalls einfach, zweifach oder dreifach mit R^{t} substituiert sind;
d) Naphthyl, gegebenenfalls einfach, zweifach oder dreifach mit R^{t} substituiert;
e) einer monocyclischen aromatischen Kohlenwasserstoffgruppe, die fünf Ringatome aufweist, die ein Kohlenstoffatom aufweist, das der Bindungspunkt ist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die gegebenenfalls einfach oder zweifach mit R^{j} substituiert ist und die gegebenenfalls an zwei benachbarten Kohlenstoffatomen benzokondensiert oder pyridokondensiert ist, wobei die benzokondensierte oder pyridokondensierte Einheit gegebenenfalls einfach, zweifach oder dreifach mit R^{t} substituiert ist; und
f) einer monocyclischen aromatischen Kohlenwasserstoffgruppe, die sechs Ringatome aufweist, die ein Kohlenstoffatom aufweist, das der Bindungspunkt ist, in der ein oder zwei Kohlenstoffatome durch -N= ersetzt sind, die gegebenenfalls einfach oder zweifach mit R^{j} substituiert ist und die gegebenenfalls an zwei benachbarten Kohlenstoffatomen benzokondensiert oder pyridokondensiert ist, wobei die benzokondensierte oder pyridokondensierte Einheit gegebenenfalls einfach oder zweifach mit R^{t} substituiert ist;
und Enantiomere, Diastereomere, Hydrate, Solvate und pharmazeutisch verträgliche Salze, Ester und Amide davon, mit der Maßgabe:
wenn n 1 ist, L -O- ist, Ar unsubstituiertes Phenyl ist und R² und R³ beide Methyl sind, dann ist R⁴ nicht -OH,
zur Verwendung bei der Behandlung oder Verhinderung einer ZNS-Störung, die ausgewählt ist aus der Gruppe, bestehend aus: Schlaf/Wach- und Aufwach/Vigilanz-Störungen, Schlaflosigkeit, Jetlag, gestörtem Schlaf, Aufmerksamkeitsdefizitstörungen mit Hyperaktivität (ADHD), Aufmerksamkeitsdefizitstörungen, Lern- und Gedächtnisstörungen, Lernbeeinträchtigung, Gedächtnisbeeinträchtigung, Gedächtnisverlust, kognitiver Dysfunktion, Migräne, neurogener Entzündung, Demenz, milder kognitiver Beeinträchtigung, Prädemenz, Alzheimer-Krankheit, Epilepsie, Narkolepsie mit oder ohne assoziierte Kataplexie, Kataplexie, Störungen der Schlaf/Wach-Homoeostase, idiopathischer Somnolenz, übermäßiger Tagschläfrigkeit (EDS), Störungen des Tag/Nacht-Rhythmus, Schlaf/Müdigkeitsstörungen, Müdigkeit, Benommenheit im Zusammenhang mit Schlafapnoe, Schlafbeeinträchtigung aufgrund perimenopausaler hormonaler Verschiebungen, Müdigkeit im Zusammenhang mit Parkinson, Müdigkeit im Zusammenhang mit MS, Müdigkeit im Zusammenhang mit Depression, durch Chemotherapie induzierter Müdigkeit, Müdigkeit im Zusammenhang mit Arbeit, Lethargie, Essstörungen, Fettleibigkeit, Bewegungskrankheit, Schwindel, Schizophrenie, Substanzmissbrauch, bipolaren Störungen, manischen Störungen und Depression, in Säugern.

2. Verbindung nach Anspruch 1, wobei L -O- ist und n 1 ist.

3. Verbindung nach Anspruch 1, wobei L -C≡C- ist und n 0 ist.

4. Verbindung nach Anspruch 1, wobei L -CH₂CH₂- ist und n 0 ist.

5. Verbindung nach Anspruch 1, wobei R¹ Methyl, Ethyl, Propyl, t-Butyl, Allyl, Propargyl oder Benzyl ist.

6. Verbindung nach Anspruch 1, wobei R¹ Wasserstoff oder Methyl ist.

7. Verbindung nach Anspruch 1, wobei, wenn R² Methyl ist, R³ nicht Methyl ist.

8. Verbindung nach Anspruch 1, wobei R² und R³ mit dem Stickstoff zusammengenommen sein können, um einen 4- bis 8-gliedrigen heterocyclischen Ring zu bilden, wobei der heterocyclische Ring ausgewählt ist aus Piperidin, Pyrrolidin und Morpholin, wobei der Ring mit 1 oder 2 Substituenten R^{ff} substituiert ist.

9. Verbindung nach Anspruch 1, ausgewählt aus der Gruppe, bestehend aus:
7-[3-(4-Fluorpiperidin-1 -yl)-propoxy]-4-(4-methoxyphenyl)-2-methyl-1,2,3,4-tetrahydroisochinolin;
7-[3-(4-Fluorpiperidin-1-yl)-propoxy]-4-(4-methoxyphenyl)-2-methyl-1,2,3,4-tetrahydroisochinolin (Enantiomer 1);
7-[3-(4-Fluorpiperidin-1-yl)-propoxy]-4-(4-methoxyphenyl)-2-methyl-1,2,3,4-tetrahydroisochinolin (Enantiomer 2);
1-(4-{3-[4-(4-Methoxyphenyl)-2-methyl-1,2,3,4-tetrahydroisochinolin-7-yloxy]-propyl}-piperazin-1-yl)-ethanon;
Diethyl-{3-[4-(4-methoxyphenyl)-2-methyl-1,2,3,4-tetrahydroisochinolin-7-yloxy]-propyl}-amin;
(4-{3-[4-(4-Methoxyphenyl)-2-methyl-1,2,3,4-tetrahydroisochinolin-7-yloxy]-propyl}-piperazin-1-yl)-pyridin-4-ylmethanon;
1-(4-{3-[4-(4-Methoxyphenyl)-2-methyl-1,2,3,4-tetrahydroisochinolin-7-yloxy]-propyl}-piperazin-1-yl)-2-methylpropan-1-on;
Cyclobutyl-(4-{3-[4-(4-methoxyphenyl)-2-methyl-1,2,3,4-tetrahydroisochinolin-7-yloxy]-propyl}-piperazin-1-yl)-methanon;
Cyclopropyl-(4-{3-[4-(4-methoxyphenyl)-2-methyl-1,2,3,4-tetrahydroisochinolin-7-yloxy]-propyl}-piperazin-1-yl)-methanon;
7-[3-(4,4-Difluorpiperidin-1-yl)-propoxy]-4-(4-methoxyphenyl)-2-methyl-1,2,3,4-tetrahydroisochinolin;
4-(4-Methoxyphenyl)-2-methyl-7-(3-morpholin-4-ylpropoxy)-1,2,3,4-tetrahydroisochinolin;
(1-{3-[4-(4-Methoxyphenyl)-2-methyl-1,2,3,4-tetrahydroisochinolin-7-yloxy]-propyl}-pyrrolidin-3-yl)-dimethylamin;
7-[3-((2R,5R)-trans-Dimethylpyrrolidin-1-yl)-propoxy]-4-(4-methoxyphenyl)-2-methyl-1,2,3,4-tetrahydroisochinolin;
4-{3-[4-(4-Methoxyphenyl)-2-methyl-1,2,3,4-tetrahydroisochinolin-7-yloxy]-propyl}-piperazin-1-carbonsäureethylester;
(4-{3-[4-(4-Methoxyphenyl)-2-methyl-1,2,3,4-tetrahydroisochinolin-7-yloxy]-propyl}-piperazin-1-yl)-(1-methyl-1H-pyrrol-2-yl)-methanon;
(4-{3-[4-(4-Methoxyphenyl)-2-methyl-1,2,3,4-tetrahydroisochinolin-7-yloxy]-propyl}-piperazin-1-yl)-(1-methyl-1H-imidazol-4-yl)-methanon;
(1,3-Dimethyltetrahydropyrimidin-2-yliden)-{3-[4-(4-methoxyphenyl)-2-methyl-1,2,3,4-tetrahydroisochinolin-7-yloxy]-propyl}-amin;
7-[3-(1,3-Dihydroisoindol-2-yl)-propoxy]-4-(4-methoxyphenyl)-2-methyl-1,2,3,4-tetrahydroisochinolin;
Bis-(2-methoxyethyl)-{3-[4-(4-methoxyphenyl)-2-methyl-1,2,3,4-tetrahydroisochinolin-7-yloxy]-propyl}-amin;
7-[3-(5,6-Dihydro-4H-pyrimidin-1-yl)-propoxy]-4-(4-methoxyphenyl)-2-methyl-1,2,3,4-tetrahydroisochinolin;
Benzothiazol-2-yl-{3-[4-(4-methoxyphenyl)-2-methyl-1,2,3,4-tetrahydroisochinolin-7-yloxy]-propyl}-methylamin;
1-{3-[4-(4-Methoxyphenyl)-2-methyl-1,2,3,4-tetrahydroisochinolin-7-yloxy]-propyl}-piperidin-3-ol;
1-{3-[4-(4-Methoxyphenyl)-2-methyl-1,2,3,4-tetrahydroisochinolin-7-yloxy]-propyl}-piperidin-4-ol;
(1-{3-[4-(4-Methoxyphenyl)-2-methyl-1,2,3,4-tetrahydroisochinolin-7-yloxy]-propyl}-piperidin-4-yl)-methanol;
(1-{3-[4-(4-Methoxyphenyl)-2-methyl-1,2,3,4-tetrahydroisochinolin-7-yloxy]-propyl}-piperidin-3-yl)-methanol;
(1-{3-[4-(4-Methoxyphenyl)-2-methyl-1,2,3,4-tetrahydroisochinolin-7-yloxy]-propyl}-piperidin-2-yl)-methanol;
4-(4-Methoxyphenyl)-2-methyl-7-[3-(3-trifluormethylpiperidin-1-yl)-propoxy]-1,2,3,4-tetrahydroisochinolin;
2-(1-{3-[4-(4-Methoxyphenyl)-2-methyl-1,2,3,4-tetrahydroisochinolin-7-yloxy]-propyl}-piperidin-4-yl)-ethanol;
7-[3-(1,1-Dioxo-1λ⁶-thiomorpholin-4-yl)-propoxy]-4-(4-methoxyphenyl)-2-methyl-1,2,3,4-tetrahydroisochinolin;
4-(4-Methoxyphenyl)-2-methyl-7-[3-((2S)-trifluormethylpyrrolidin-1-yl)-propoxy]-1,2,3,4-tetrahydroisochinolin;
7-[3-(3,3-Difluorpiperidin-1-yl)-propoxy]-4-(4-methoxyphenyl)-2-methyl-1,2,3,4-tetrahydroisochinolin;
7-[3-(3,3-Difluorpiperidin-1-yl)-propoxy]-4-(4-methoxyphenyl)-2-methyl-1,2,3,4-tetrahydroisochinolin (Enantiomer 1);
7-[3-(3,3-Difluorpiperidin-1-yl)-propoxy]-4-(4-methoxyphenyl)-2-methyl-1,2,3,4-tetrahydroisochinolin (Enantiomer 2);
(1-{3-[4-(4-Methoxyphenyl)-2-methyl-1,2,3,4-tetrahydroisochinolin-7-yloxy]-propyl}-pyrrolidin-(2R)-yl)-methanol;
1-{3-[4-(4-Methoxyphenyl)-2-methyl-1,2,3,4-tetrahydroisochinolin-7-yloxy]-propyl}-(R)-pyrrolidin-3-ol;
7-[3-(2,6-Dimethylmorpholin-4-yl)-propoxy]-4-(4-methoxyphenyl)-2-methyl-1,2,3,4-tetrahydroisochinolin;
1-{3-[4-(4-Methoxyphenyl)-2-methyl-1,2,3,4-tetrahydroisochinolin-7-yloxy]-propyl}-piperidin-4-carbonsäureethylester;
7-(3-Azetidin-1-ylpropoxy)-4-(4-methoxyphenyl)-2-methyl-1,2,3,4-tetrahydroisochinolin;
4-(4-Methoxyphenyl)-2-methyl-7-[3-(2-methylpyrrolidin-1-yl)-propoxy]-1,2,3,4-tetrahydroisochinolin;
2-(1-{3-[4-(4-Methoxyphenyl)-2-methyl-1,2,3,4-tetrahydroisochinolin-7-yloxy]-propyl}-piperidin-2-yl)-ethanol;
1-{3-[4-(4-Methoxyphenyl)-2-methyl-1,2,3,4-tetrahydroisochinolin-7-yloxy]-propyl}-piperidin-4-carbonitril;
1-{3-[4-(4-Methoxyphenyl)-2-methyl-1,2,3,4-tetrahydroisochinolin-7-yloxy]-propyl}-piperidin-4-carbonitril (Enantiomer 1);
1-{3-[4-(4-Methoxyphenyl)-2-methyl-1,2,3,4-tetrahydroisochinolin-7-yloxy]-propyl}-piperidin-4-carbonitril (Enantiomer 2);
4-(4-Methoxyphenyl)-2-methyl-7-[3-(4-trifluormethylpiperidin-1-yl)-propoxy]-1,2,3,4-tetrahydroisochinolin;
7-[3-(3-Fluorpiperidin-1-yl)-propoxy]-4-(4-methoxyphenyl)-2-methyl-1,2,3,4-tetrahydroisochinolin;
Ethyl-(2-methoxyethyl)-{3-[4-(4-methoxyphenyl)-2-methyl-1,2,3,4-tetrahydroisochinolin-7-yloxy]-propyl}-amin;
7-[3-(1,4-Dioxa-8-azaspiro[4.5]dec-8-yl)-propoxy]-4-(4-methoxyphenyl)-2-methyl-1,2,3,4-tetrahydroisochinolin;
1-[4-(4-Methoxyphenyl)-2-methyl-1,2,3,4-tetrahydroisochinolin-7-yloxy]-3-piperidin-1-ylpropan-2-ol;
7-[2-Fluor-3-(4-fluorpiperidin-1-yl)-propoxy]-4-(4-methoxyphenyl)-2-methyl-1,2,3,4-tetrahydroisochinolin;
7-[3-(4-Fluorpiperidin-1-yl)-propoxy]-4-(3-methoxyphenyl)-2-methyl-1,2,3,4-tetrahydroisochinolin;
7-[3-(4-Fluorpiperidin-1-yl)-propoxy]-4-(3-methoxyphenyl)-2-methyl-1,2,3,4-tetrahydroisochinolin (Enantiomer 1);
7-[3-(4-Fluorpiperidin-1-yl)-propoxy]-4-(3-methoxyphenyl)-2-methyl-1,2,3,4-tetrahydroisochinolin (Enantiomer 2);
4-(3-Chlorphenyl)-7-[3-(4-fluorpiperidin-1-yl)-propoxy]-2-methyl-1,2,3,4-tetrahydroisochinolin;
4-(4-Chlorphenyl)-7-[3-(4-fluorpiperidin-1-yl)-propoxy]-2-methyl-1,2,3,4-tetrahydroisochinolin;
4-(3-Fluorphenyl)-7-[3-(4-fluorpiperidin-1-yl)-propoxy]-2-methyl-1,2,3,4-tetrahydroisochinolin;
7-[3-(4-Fluorpiperidin-1-yl)-propoxy]-2-methyl-4-(4-trifluormethoxyphenyl)-1,2,3,4-tetrahydroisochinolin;
4-(4-Difluormethoxyphenyl)-7-[3-(4-fluorpiperidin-1-yl)-propoxy]-2-methyl-1,2,3,4-tetrahydroisochinolin;
7-[3-(4-Fluorpiperidin-1-yl)-propoxy]-4-(4-methansulfonylphenyl)-2-methyl-1,2,3,4-tetrahydroisochinolin;
7-[3-(4-Fluorpiperidin-1-yl)-propoxy]-2-methyl-4-(4-methylsulfanylphenyl)-1,2,3,4-tetrahydroisochinolin;
4-(3-Chlor-4-methoxyphenyl)-7-[3-(4-fluorpiperidin-1-yl)-propoxy]-2-methyl-1,2,3,4-tetrahydroisochinolin;
4-(2,4-Dichlorphenyl)-7-[3-(4-fluorpiperidin-1-yl)-propoxy]-2-methyl-1,2,3,4-tetrahydroisochinolin;
4-(2,5-Dichlorphenyl)-7-[3-(4-fluorpiperidin-1-yl)-propoxy]-2-methyl-1,2,3,4-tetrahydroisochinolin;
4-(3,5-Dichlorphenyl)-7-[3-(4-fluorpiperidin-1-yl)-propoxy]-2-methyl-1,2,3,4-tetrahydroisochinolin;
2-Methyl-7-(3-piperidin-1-ylpropoxy)-4-thiophen-3-yl-1,2,3,4-tetrahydroisochinolin;
4-(3,4-Dichlorphenyl)-7-[3-(4-fluorpiperidin-1-yl)-propoxy]-2-methyl-1,2,3,4-tetrahydroisochinolin;
2-Methyl-7-(3-piperidin-1-ylpropoxy)-4-pyridin-3-yl-1,2,3,4-tetrahydroisochinolin;
2-Methyl-7-(3-piperidin-1-ylpropoxy)-4-(trifluormethylphenyl)-1,2,3,4-tetrahydroisochinolin;
4-(4-Methoxyphenyl)-2-methyl-7-(3-piperidin-1-ylpropoxy)-1,2,3,4-tetrahydroisochinolin (razemisch);
4-(4-Methoxyphenyl)-2-methyl-7-(3-piperidin-1-ylpropoxy)-1,2,3,4-tetrahydroisochinolin (Enantiomer 1);
4-(4-Methoxyphenyl)-2-methyl-7-(3-piperidin-1-ylpropoxy)-1,2,3,4-tetrahydroisochinolin (Enantiomer 2);
2-tert-Butyl-4-(4-m ethoxyphenyl)-7-(3-piperidin-1-ylpropoxy)-1,2,3,4-tetrahydroisochinolin;
2-Benzyl-4-(4-methoxyphenyl)-7-(3-piperidin-1-ylpropoxy)-1,2,3,4-tetrahydroisochinolin;
2-Ethyl-4-(4-methoxyphenyl)-7-(3-piperidin-1-ylpropoxy)-1,2,3,4-tetrahydroisochinolin;
4-(4-Methoxyphenyl)-7-(3-piperidin-1-ylpropoxy)-2-propyl-1,2,3,4-tetrahydroisochinolin;
2-Isopropyl-4-(4-methoxyphenyl)-7-(3-piperidin-1-ylpropoxy)-1,2,3,4-tetrahydroisochinolin;
4-(4-Methoxyphenyl)-7-(3-piperidin-1-ylpropoxy)-1,2,3,4-tetrahydroisochinolin;
3-[4-(4-Methoxyphenyl)-7-(3-piperidin-1-ylpropoxy)-3,4-dihydro-1H-isochinolin-2-yl]-propan-1-ol;
2-[4-(4-Methoxyphenyl)-7-(3-piperidin-1-ylpropoxy)-3,4-dihydro-1H-isochinolin-2-yl]-ethanol;
2-(2-Fluorethyl)-4-(4-methoxyphenyl)-7-(3-piperidin-1-ylpropoxy)-1,2,3,4-tetrahydroisochinolin;
2-Cyclopropyl-4-(4-methoxyphenyl)-7-(3-piperidin-1-ylpropoxy)-1,2,3,4-tetrahydroisochinolin;
4-(4-Methoxyphenyl)-7-(3-morpholin-4-ylpropoxy)-2-(1-phenylethyl)-1,2,3,4-tetrahydroisochinolin;
4-(4-Methoxyphenyl)-7-(3-morpholin-4-ylpropoxy)-2-(1-phenylethyl)-1,2,3,4-tetrahydroisochinolin (Diastereomer 1);
4-(4-Methoxyphenyl)-7-(3-morpholin-4-ylpropoxy)-2-(1-phenylethyl)-1,2,3,4-tetrahydroisochinolin (Diastereomer 2);
4-(3,4-Dichlorphenyl)-2-methyl-7-(3-piperidin-1-ylpropoxy)-1,2,3,4-tetrahydroisochinolin;
4-(3,4-Dichlorphenyl)-2-methyl-7-(3-morpholin-4-ylpropoxy)-1,2,3,4-tetrahydroisochinolin;
4-(4-Methoxyphenyl)-2-methyl-7-(piperidin-4-ylmethoxy)-1,2,3,4-tetrahydroisochinolin;
4-(3-Chlor-4-methoxyphenyl)-2-methyl-7-(piperidin-4-ylmethoxy)-1,2,3,4-tetrahydroisochinolin;
2-Methyl-4-(4-methylsulfanylphenyl)-7-(piperidin-4-ylmethoxy)-1,2,3,4-tetrahydroisochinolin;
4-(3-Fluor-4-methoxyphenyl)-2-methyl-7-(piperidin-4-ylmethoxy)-1,2,3,4-tetrahydroisochinolin;
4-(2-Fluor-4-methoxyphenyl)-2-methyl-7-(piperidin-4-ylmethoxy)-1,2,3,4-tetrahydroisochinolin;
7-(1-Isopropylpiperidin-4-ylmethoxy)-4-(4-methoxyphenyl)-2-methyl-1,2,3,4-tetrahydroisochinolin;
4-(4-Methoxyphenyl)-2-methyl-7-(1-methylpiperidin-4-ylmethoxy)-1,2,3,4-tetrahydroisochinolin;
4-(4-Methoxyphenyl)-2-methyl-7-(1-propylpiperidin-4-ylmethoxy)-1,2,3,4-tetrahydroisochinolin;
7-(1-Cyclopentylpiperidin-4-ylmethoxy)-4-(4-methoxyphenyl)-2-methyl-1,2,3,4-tetrahydroisochinolin;
7-(1-Ethylpiperidin-4-ylmethoxy)-4-(4-methoxyphenyl)-2-methyl-1,2,3,4-tetrahydroisochinolin;
4-(3-Chlor-4-methoxyphenyl)-7-(1-isopropylpiperidin-4-ylmethoxy)-2-methyl-1,2,3,4-tetrahydroisochinolin;
4-(3-Fluor-4-methoxyphenyl)-7-(1-isopropylpiperidin-4-ylmethoxy)-2-methyl-1,2,3,4-tetrahydroisochinolin;
4-(2-Fluor-4-methoxyphenyl)-7-(1-isopropylpiperidin-4-ylmethoxy)-2-methyl-1,2,3,4-tetrahydroisochinolin;
7-(1-Isopropylpiperidin-4-ylmethoxy)-4-(3-methoxyphenyl)-2-methyl-1,2,3,4-tetrahydroisochinolin;
4-(3-Methoxyphenyl)-2-methyl-7-(1-methylpiperidin-4-ylmethoxy)-1,2,3,4-tetrahydroisochinolin;
4-(3-Methoxyphenyl)-2-methyl-7-(1-propylpiperidin-4-ylmethoxy)-1,2,3,4-tetrahydroisochinolin;
7-(1-Cyclopentylpiperidin-4-ylmethoxy)-4-(3-methoxyphenyl)-2-methyl-1,2,3,4-tetrahydroisochinolin;
7-(1-Ethylpiperidin-4-ylmethoxy)-4-(3-methoxyphenyl)-2-methyl-1,2,3,4-tetrahydroisochinolin;
4-(3-Methoxyphenyl)-2-methyl-7-(piperidin-4-yloxy)-1,2,3,4-tetrahydroisochinolin;
4-(3-Methoxyphenyl)-2-methyl-7-(1-methylpiperidin-4-yloxy)-1,2,3,4-tetrahydroisochinolin;
7-(1-Isopropylpiperidin-4-yloxy)-4-(3-methoxyphenyl)-2-methyl-1,2,3,4-tetrahydroisochinolin;
7-(1-Cyclobutylpiperidin-4-yloxy)-4-(3-methoxyphenyl)-2-methyl-1,2,3,4-tetrahydroisochinolin;
7-(1-Ethylpiperidin-4-yloxy)-4-(3-methoxyphenyl)-2-methyl-1,2,3,4-tetrahydroisochinolin;
7-(1-Cyclopentylpiperidin-4-yloxy)-4-(3-methoxyphenyl)-2-methyl-1,2,3,4-tetrahydroisochinolin;
4-(3-Methoxyphenyl)-2-methyl-7-(1-propylpiperidin-4-yloxy)-1,2,3,4-tetrahydroisochinolin;
7-(1-Isopropylpiperidin-4-ylmethoxy)-2-methyl-4-(4-methylsulfanylphenyl)-1,2,3,4-tetrahydroisochinolin;
7-(1-Cyclobutylpiperidin-4-ylmethoxy)-2-methyl-4-(4-methylsulfanylphenyl)-1,2,3,4-tetrahydroisochinolin;
7-(1-Ethylpiperidin-4-ylmethoxy)-2-methyl-4-(4-methylsulfanylphenyl)-1,2,3,4-tetrahydroisochinolin;
4-(3-Chlor-4-methoxyphenyl)-7-(1-cyclobutylpiperidin-4-ylmethoxy)-2-methyl-1,2,3,4-tetrahydroisochinolin;
4-(3-Chlor-4-methoxyphenyl)-7-(1-ethylpiperidin-4-ylmethoxy)-2-methyl-1,2,3,4-tetrahydroisochinolin;
4-(3-Chlor-4-methoxyphenyl)-2-methyl-7-(1-propylpiperidin-4-ylmethoxy)-1,2,3,4-tetrahydroisochinolin;
2-Methyl-4-(4-methylsulfanylphenyl)-7-(1-propylpiperidin-4-ylmethoxy)-1,2,3,4-tetrahydroisochinolin;
7-(1-Isobutylpiperidin]-4-ylmethoxy)-4-(4-methoxyphenyl)-2-methyl-1,2,3,4-tetrahydroisochinolin;
7-(1-Cyclobutylpiperidin-4-ylmethoxy)-4-(3-fluor-4-methoxyphenyl)-2-methyl-1,2,3,4-tetrahydroisochinolin;
4-(3-Fluor-4-methoxyphenyl)-2-methyl-7-(1-propylpiperidin-4-ylmethoxy)-1,2,3,4-tetrahydroisochinolin;
7-(1-Cyclobutylpiperidin-4-ylmethoxy)-4-(2-fluor-4-methoxyphenyl)-2-methyl-1,2,3,4-tetrahydroisochinolin;
4-(2-Fluor-4-methoxyphenyl)-2-methyl-7-(1-propylpiperidin-4-ylmethoxy)-1,2,3,4-tetrahydroisochinolin;
4-(2-Fluor-4-methoxyphenyl)-7-(1-isobutylpiperidin-4-ylmethoxy)-2-methyl-1,2,3,4-tetrahydroisochinolin;
7-[1-(2-Fluorethyl)-piperidin-4-ylmethoxy]-4-(4-methoxyphenyl)-2-methyl-1,2,3,4-tetrahydroisochinolin;
7-(1-Isopropylpiperidin-4-yloxy)-4-(4-methoxyphenyl)-2-methyl-1,2,3,4-tetrahydroisochinolin;
7-(1-Isopropylpiperidin-4-yloxy)-2-methyl-4-(4-methylsulfanylphenyl)-1,2,3,4-tetrahydroisochinolin;
4-(2-Fluor-4-methoxyphenyl)-7-(1-isopropylpiperidin-4-yloxy)-2-methyl-1,2,3,4-tetrahydroisochinolin;
4-(3-Fluor-4-methoxyphenyl)-7-(1-isopropylpiperidin-4-yloxy)-2-methyl-1,2,3,4-tetrahydroisochinolin;
4-(4-Methoxyphenyl)-2-methyl-7-[1-(tetrahydropyran-4-yl)-piperidin-4-yloxy]-1,2,3,4-tetrahydroisochinolin;
2,2,2-Trifluor-1-{4-[4-(4-methoxyphenyl)-2-methyl-1,2,3,4-tetrahydroisochinolin-7-yloxymethyl]-piperidin-1-yl}-ethanon;
4-(4-Methoxyphenyl)-2-methyl-7-[1-(2,2,2-trifluorethyl)-piperidin-4-ylmethoxy]-1,2,3,4-tetrahydroisochinolin;
7-[3-(4-Fluorpiperidin-1-yl)-propoxy]-2-methyl-4-phenyl-1,2,3,4-tetrahydroisochinolin;
4-(2-Fluorphenyl)-7-[3-(4-fluorpiperidin-1-yl)-propoxy]-2-methyl-1,2,3,4-tetrahydroisochinolin;
7-[3-(4-Fluorpiperidin-1-yl)-propoxy]-2-methyl-4-p-tolyl-1,2,3,4-tetrahydroisochinolin;
2-Benzyl-7-[3-(4-fluorpiperidin-1-yl)-propoxy]-4-(4-methoxyphenyl)-1,2,3,4-tetrahydroisochinolin;
7-[3-(4-Fluorpiperidin-1-yl)-propoxy]-2-methyl-4-(3-trifluormethoxyphenyl)-1,2,3,4-tetrahydroisochinolin;
7-[3-(3,3-Difluorpyrrolidin-1-yl)-propoxy]-4-(4-methoxyphenyl)-2-methyl-1,2,3,4-tetrahydroisochinolin;
4-(4-Methoxyphenyl)-2-methyl-7-[3-(3S-methylmorpholin-4-yl)-propoxy]-1,2,3,4-tetrahydroisochinolin;
Dicyclopropylmethyl-{3-[4-(4-methoxyphenyl)-2-methyl-1,2,3,4-tetrahydroisochinolin-7-yloxy]-propyl}-amin;
4-(2-Chlorphenyl)-7-[3-(4-fluorpiperidin-1-yl)-propoxy]-2-methyl-1,2,3,4-tetrahydroisochinolin;
2-Methyl-7-[3-(3S-methylmorpholin-4-yl)-propoxy]-4-(4-morpholin-4-ylphenyl)-1,2,3,4-tetrahydroisochinolin;
7-[3-(4-Fluorpiperidin-1-yl)-propoxy]-2-methyl-4-(4-morpholin-4-ylphenyl)-1,2,3,4-tetrahydroisochinolin;
4-{2-Methyl-7-[3-(3S-methylmorpholin-4-yl)-propoxy]-1,2,3,4-tetrahydroisochinolin-4-yl}-benzonitril;
4-{7-[3-(4-Fluorpiperidin-1-yl)-propoxy]-2-methyl-1,2,3,4-tetrahydroisochinolin-4-yl}-benzonitril;
7-[3-(3-Benzhydrylazetidin-1-yl)-propoxy]-4-(4-methoxyphenyl)-2-methyl-1,2,3,4-tetrahydroisochinolin;
2-Methyl-4-(4-methylsulfanylphenyl)-7-(3-morpholin-4-ylpropoxy)-1,2,3,4-tetrahydroisochinolin;
4-(2-Fluor-4-methoxyphenyl)-7-[3-(4-fluorpiperidin-1-yl)-propoxy]-2-methyl-1,2,3,4-tetrahydroisochinolin (Razemat);
4-(2-Fluor-4-methoxyphenyl)-7-[3-(4-fluorpiperidin-1-yl)-propoxy]-2-methyl-1,2,3,4-tetrahydroisochinolin (Enantiomer 1);
4-(2-Fluor-4-methoxyphenyl)-7-[3-(4-fluorpiperidin-1-yl)-propoxy]-2-methyl-1,2,3,4-tetrahydroisochinolin (Enantiomer 2);
4-(3-Fluor-4-methoxyphenyl)-7-[3-(4-fluorpiperidin-1-yl)-propoxy]-2-methyl-1,2,3,4-tetrahydroisochinolin (Razemat);
4-(3-Fluor-4-methoxyphenyl)-7-[3-(4-fluorpiperidin-1-yl)-propoxy]-2-methyl-1,2,3,4-tetrahydroisochinolin (Enantiomer 1);
4-(3-Fluor-4-methoxyphenyl)-7-[3-(4-fluorpiperidin-1-yl)-propoxy]-2-methyl-1,2,3,4-tetrahydroisochinolin (Enantiomer 2);
4-(4-Ethoxyphenyl)-7-[3-(4-fluorpiperidin-1-yl)-propoxy]-2-methyl-1,2,3,4-tetrahydroisochinolin;
2-Ethyl-7-[3-(4-fluorpiperidin-1-yl)-propoxy]-4-(4-methoxyphenyl)-1,2,3,4-tetrahydroisochinolin;
7-[3-(4-Fluorpiperidin-1-yl)-propoxy]-4-(4-methoxyphenyl)-1,2,3,4-tetrahydroisochinolin;
4-(4-Methoxyphenyl)-2-methyl-7-(piperidin-4-yloxy)-1,2,3,4-tetrahydroisochinolin;
2-Methyl-4-(4-methylsulfanylphenyl)-7-(piperidin-4-yloxy)-1,2,3,4-tetrahydroisochinolin;
4-(2-Fluor-4-methoxyphenyl)-2-methyl-7-(piperidin-4-yloxy)-1,2,3,4-tetrahydroisochinolin;
4-(3-Fluor-4-methoxyphenyl)-2-methyl-7-(piperidin-4-yloxy)-1,2,3,4-tetrahydroisochinolin;
7-[1-(2-Fluorethyl)-piperidin-4-yloxy]-4-(4-methoxyphenyl)-2-methyl-1,2,3,4-tetrahydroisochinolin;
7-[3-(4-Fluorpiperidin-1-yl)-propoxy]-2-methyl-4-(4-methylsulfanylphenyl)-1,2,3,4-tetrahydroisochinolin (Enantiomer 1);
7-[3-(4-Fluorpiperidin-1-yl)-propoxy]-2-methyl-4-(4-methylsulfanylphenyl)-1,2,3,4-tetrahydroisochinolin (Enantiomer 2);
4-(4-Methoxyphenyl)-2-methyl-7-[3-(3S-methylmorpholin-4-yl)-propoxy]-1,2,3,4-tetrahydroisochinolin (Enantiomer 1);
4-(4-Methoxyphenyl)-2-methyl-7-[3-(3S-methylmorpholin-4-yl)-propoxy]-1,2,3,4-tetrahydroisochinolin (Enantiomer 2);
2-Methyl-4-(4-methylsulfanylphenyl)-7-(3-morpholin-4-ylpropoxy)-1,2,3,4-tetrahydroisochinolin (Enantiomer 1);
2-Methyl-4-(4-methylsulfanylphenyl)-7-(3-morpholin-4-ylpropoxy)-1,2,3,4-tetrahydroisochinolin (Enantiomer 2);
7-[3-(4-Fluorpiperidin-1-yl)-propoxy]-4-(4-methylsulfanylphenyl)-1,2,3,4-tetrahydroisochinolin;
4-(4-Methylsulfanylphenyl)-7-(3-morpholin-4-ylpropoxy)-1,2,3,4-tetrahydroisochinolin;
7-[3-(3S-Methylmorpholin-4-yl)-propoxy]-4-(4-methylsulfanylphenyl)-1,2,3,4-tetrahydroisochinolin;
4-(4-Methoxyphenyl)-7-(3-morpholin-4-ylpropoxy)-1,2,3,4-tetrahydroisochinolin;
7-[3-(4-Fluorpiperidin-1-yl)-propoxy]-4-(4-methansulfinylphenyl)-2-methyl-1,2,3,4-tetrahydroisochinolin;
4-(4-Methansulfinylphenyl)-2-methyl-7-(3-morpholin-4-ylpropoxy)-1,2,3,4-tetrahydroisochinolin;
4-(4-Methansulfinylphenyl)-2-methyl-7-(3-morpholin-4-ylpropoxy)-1,2,3,4-tetrahydroisochinolin (Enantiomer 1);
4-(4-Methansulfonylphenyl)-2-methyl-7-(3-morpholin-4-ylpropoxy)-1,2,3,4-tetrahydroisochinolin;
7-[3-(4-Fluorpiperidin-1-yl)-propoxy]-4-(4-methoxyphenyl)-2,6-dimethyl-1,2,3,4-tetrahydroisochinolin;
7-[3-(4-Fluorpiperidin-1-yl)-propoxy]-4-(4-methoxyphenyl)-2,8-dimethyl-1,2,3,4-tetrahydroisochinolin;
7-[3-(4-Fluorpiperidin-1-yl)-propoxy]-4-(4-methoxyphenyl)-2-methyl-1,2,3,4-tetrahydroisochinolin-6-ol;
7-[3-(4-Fluorpiperidin-1-yl)-propoxy]-4-(4-methoxyphenyl)-2-methyl-1,2,3,4-tetrahydroisochinolin-8-ol;
2,8-Dimethyl-4-(4-methylsulfanylphenyl)-7-(3-morpholin-4-ylpropoxy)-1,2,3,4-tetrahydroisochinolin;
2,6-Dimethyl-4-(4-methylsulfanylphenyl)-7-(3-morpholin-4-ylpropoxy)-1,2,3,4-tetrahydroisochinolin;
2-Methyl-4-(4-methylsulfanylphenyl)-7-(3-morpholin-4-ylpropoxy)-1,2,3,4-tetrahydroisochinolin-8-ol;
2-Methyl-4-(4-methylsulfanylphenyl)-7-(3-morpholin-4-ylpropoxy)-1,2,3,4-tetrahydroisochinolin-6-ol;
8-Fluor-2-methyl-4-(4-methylsulfanylphenyl)-7-(3-morpholin-4-ylpropoxy)-1,2,3,4-tetrahydroisochinolin;
6-Fluor-2-methyl-4-(4-methylsulfanylphenyl)-7-(3-morpholin-4-ylpropoxy)-1,2,3,4-tetrahydroisochinolin;
7-[1-(2-Fluorethyl)-piperidin-4-ylmethoxy]-2-methyl-4-(4-methylsulfanylphenyl)-1,2,3,4-tetrahydroisochinolin;
7-[1-(2-Fluorethyl)-piperidin-4-yloxy]-2-methyl-4-(4-methylsulfanylphenyl)-1,2,3,4-tetrahydroisochinolin;
4-(4-Methoxyphenyl)-7-[3-(3S-methylmorpholin-4-yl)-propoxy]-1,2,3,4-tetrahydroisochinolin;
4-(4-Methansulfinylphenyl)-2-methyl-7-[3-(3S-methylmorpholin-4-yl)-propoxy]-1,2,3,4-tetrahydroisochinolin;
7-[3-(3,3-Difluorazetidin-1-yl)-propoxy]-4-(4-methoxyphenyl)-2-methyl-1,2,3,4-tetrahydroisochinolin;
(4-{3-[4-(4-Methoxyphenyl)-2-methyl-1,2,3,4-tetrahydroisochinolin-7-yloxy]-propyl}-morpholin-3-yl)-methanol;
(4-{3-[4-(4-Methoxyphenyl)-2-methyl-1,2,3,4-tetrahydroisochinolin-7-yloxy]-propyl}-morpholin-3S-yl)-methanol;
(4-{3-[4-(4-Methoxyphenyl)-2-methyl-1,2,3,4-tetrahydroisochinolin-7-yloxy]-propyl}-morpholin-2-yl)-methanol;
{3-[2-Methyl-4-(4-methylsulfanylphenyl)-1,2,3,4-tetrahydroisochinolin-7-yloxy]-propyl}-(2H-pyrazol-3-yl)-amin;
4-(6-Brompyridin-3-yl)-7-[3-(4-fluorpiperidin-1-yl)-propoxy]-2-methyl-1,2,3,4-tetrahydroisochinolin;
7-[3-(4-Fluorpiperidin-1-yl)-propoxy]-2-methyl-4-(6-methylsulfanylpyridin-3-yl)-1,2,3,4-tetrahydroisochinolin;
5-{7-[3-(4-Fluorpiperidin-1-yl)-propoxy]-2-methyl-1,2,3,4-tetrahydroisochinolin-4-yl}-pyridin-2-yl)-dimethylamin;
7-[3-(4-Fluorpiperidin-1-yl)-propoxy]-2-methyl-4-(6-trimethylsilanylethinylpyridin-3-yl)-1,2,3,4-tetrahydroisochinolin;
4-(6-Ethinylpyridin-3-yl)-7-[3-(4-fluorpiperidin-1-yl)-propoxy]-2-methyl-1,2,3,4-tetrahydroisochinolin;
7-[3-(4-Fluorpiperidin-1-yl)-propoxy]-2-methyl-4-(6-phenylsulfanylpyridin-3-yl)-1,2,3,4-tetrahydroisochinolin;
7-[3-(4-Fluorpiperidin-1-yl)-propoxy]-4-(6-imidazol-1-ylpyridin-3-yl)-2-methyl-1,2,3,4-tetrahydroisochinolin;
7-[3-(4-Fluorpiperidin-1-yl)-propoxy]-4-(6-methoxypyridin-3-yl)-2-methyl-1,2,3,4-tetrahydroisochinolin;
7-[3-(4-Fluorpiperidin-1-yl)-propoxy]-2-methyl-4-(6-pyrazol-1-ylpyridin-3-yl)-1,2,3,4-tetrahydroisochinolin;
7-[3-(4-Fluorpiperidin-1-yl)-propoxy]-4-[6-(1H-imidazol-2-ylsulfanyl)-pyridin-3-yl]-2-methyl-1,2,3,4-tetrahydroisochinolin;
7-[3-(4-Fluorpiperidin-1-yl)-propoxy]-2-methyl-4-[6-(pyrimidin-2-ylsulfanyl)-pyridin-3-yl]-1,2,3,4-tetrahydroisochinolin;
4-(4-Methoxyphenyl)-2-methyl-7-[3-(4-methylpiperazin-1-yl)-propoxy]-1,2,3,4-tetrahydroisochinolin;
7-[3-(4-Ethylpiperazin-1-yl)-propoxy]-4-(4-methoxyphenyl)-2-methyl-1,2,3,4-tetrahydroisochinolin;
7-[3-(4-Isopropylpiperazin-1-yl)-propoxy]-4-(4-methoxyphenyl)-2-methyl-1,2,3,4-tetrahydroisochinolin;
4-(4-Methoxyphenyl)-2-methyl-7-[3-(4-thiazol-2-ylpiperazin-1-yl)-propoxy]-1,2,3,4-tetrahydroisochinolin;
4-(4-Methoxyphenyl)-2-methyl-7-[3-(4-thiophen-2-ylmethylpiperazin-1-yl)-propoxy]-1,2,3,4-tetrahydroisochinolin;
2-(4-{3-[4-(4-Methoxyphenyl)-2-methyl-1,2,3,4-tetrahydroisochinolin-7-yloxy]-propyl}-piperazin-1-yl)-ethanol;
2-(4-{3-[4-(4-Methoxyphenyl)-2-methyl-1,2,3,4-tetrahydroisochinolin-7-yloxy]-propyl}-piperazin-1-yl)-phenol;
4-(4-Methoxyphenyl)-2-methyl-7-[3-(4-pyridin-4-ylpiperazin-1-yl)-propoxy]-1,2,3,4-tetrahydroisochinolin;
4-(4-Methoxyphenyl)-2-methyl-7-{3-[4-(4-trifluormethylphenyl)-piperazin-1-yl]-propoxy}-1,2,3,4-tetrahydroisochinolin;
7-[3-(4-Allylpiperazin-1-yl)-propoxy]-4-(4-methoxyphenyl)-2-methyl-1,2,3,4-tetrahydroisochinolin;
7-[3-(4-[1,3]-Dioxolan-2-ylmethylpiperazin-1-yl)-propoxy]-4-(4-methoxyphenyl)-2-methyl-1,2,3,4-tetrahydroisochinolin;
4-(4-{3-[4-(4-Methoxyphenyl)-2-methyl-1,2,3,4-tetrahydroisochinolin-7-yloxy]-propyl}-piperazin-1-yl)-benzonitril;
7-{3-[4-(2-Methoxyethyl)-piperazin-1-yl]-propoxy}-4-(4-methoxyphenyl)-2-methyl-1,2,3,4-tetrahydroisochinolin;
4-(4-Methoxyphenyl)-2-methyl-7-{3-[4-(tetrahydrofuran-2-ylmethyl)-piperazin-1-yl]-propoxy}-1,2,3,4-tetrahydroisochinolin;
4-{3-[4-(4-Methoxyphenyl)-2-methyl-1,2,3,4-tetrahydroisochinolin-7-yloxy]-propyl}-piperazin-1-carbonsäure-tert-butylester;
7-[3-(4-Cyclopropylpiperazin-1-yl)-propoxy]-4-(4-methoxyphenyl)-2-methyl-1,2,3,4-tetrahydroisochinolin;
4-(4-Bromphenyl)-7-[3-(4-fluorpiperidin-1-yl)-propoxy]-2-methyl-1,2,3,4-tetrahydroisochinolin;
4-(4-Difluormethoxyphenyl)-2-methyl-7-(3-morpholin-4-ylpropoxy)-1,2,3,4-tetrahydroisochinolin;
2-Methyl-7-[3-(3S-methylmorpholin-4-yl)-propoxy]-4-(4-methylsulfanylphenyl)-1,2,3,4-tetrahydroisochinolin;
7-[3-(4,4-Difluorpiperidin-1-yl)-propoxy]-2-methyl-4-(4-methylsulfanylphenyl)-1,2,3,4-tetrahydroisochinolin;
7-[3-(4-Fluorpiperidin-1-yl)-propoxy]-2-methyl-4-(4-trifluormethylsulfanylphenyl)-1,2,3,4-tetrahydroisochinolin;
2-Methyl-7-(3-morpholin-4-ylpropoxy)-4-(4-trifluormethylsulfanylphenyl)-1,2,3,4-tetrahydroisochinolin;
2-Methyl-7-[3-(3S-methylmorpholin-4-yl)-propoxy]-4-(4-trifluormethylsulfanylphenyl)-1,2,3,4-tetrahydroisochinolin;
4-(2-Fluor-4-methoxyphenyl)-2-methyl-7-[3-(3S-methylmorpholin-4-yl)-propoxy]-1,2,3,4-tetrahydroisochinolin;
4-(3-Fluor-4-methoxyphenyl)-2-methyl-7-[3-(3S-methylmorpholin-4-yl)-propoxy]-1,2,3,4-tetrahydroisochinolin;
2-Methyl-7-(3-piperidin-1-ylpropoxy)-4-(4-trifluormethoxyphenyl)-1,2,3,4-tetrahydroisochinolin;
3-[4-(4-Methoxyphenyl)-2-methyl-1,2,3,4-tetrahydroisochinolin-7-yloxy]-propyl}-(tetrahydropyran-4-yl)-amin;
Cyclopropyl-{3-[4-(4-methoxyphenyl)-2-methyl-1,2,3,4-tetrahydroisochinolin-7-yloxy]-propyl}-(1-methylpiperidin-4-yl)-amin;
(2-Methoxyethyl)-{3-[4-(4-methoxyphenyl)-2-methyl-1,2,3,4-tetrahydroisochinolin-7-yloxy]-propyl}-amin;
3-{3-[4-(4-Methoxyphenyl)-2-methyl-1,2,3,4-tetrahydroisochinolin-7-yloxy]-propylamino}-propan-1-ol;
Allyl-{3-[4-(4-methoxyphenyl)-2-methyl-1,2,3,4-tetrahydroisochinolin-7-yloxy]-propyl}-amin;
Isobutyl-{3-[4-(4-methoxyphenyl)-2-methyl-1,2,3,4-tetrahydroisochinolin-7-yloxy]-propyl}-amin;
Cyclopropyl-{3-[4-(4-methoxyphenyl)-2-methyl-1,2,3,4-tetrahydroisochinolin-7-yloxy]-propyl}-amin;
Isopropyl-{3-[4-(4-methoxyphenyl)-2-methyl-1,2,3,4-tetrahydroisochinolin-7-yloxy]-propyl}-amin;
3-[4-(4-Methoxyphenyl)-2-methyl-1,2,3,4-tetrahydroisochinolin-7-yloxy]-propyl}-propylamin;
Ethyl-*{3-[4-(4-methoxyphenyl)-2-methyl-1,2,3,4-tetrahydroisochinolin-7-yloxy]-propyl}-amin;
Isopropyl-{3-[4-(4-methoxyphenyl)-2-methyl-1,2,3,4-tetrahydroisochinolin-7-yloxyl-propyl}-methylamin;
Cyclopropyl-{3-[4-(4-methoxyphenyl)-2-methyl-1,2,3,4-tetrahydroisochinolin-7-yloxy]-propyl}-methylamin;
Dicyclopropyl-{3-[4-(4-methoxyphenyl)-2-methyl-1,2,3,4-tetrahydroisochinolin-7-yloxy]-propyl}-amin;
7-(1-Isopropylazetidin-3-ylmethoxy)-2-methyl-4-(4-methylsulfanylphenyl)-1,2,3,4-tetrahydroisochinolin;
7-(1-Cyclopropylazetidin-3-ylmethoxy)-2-methyl-4-(4-methylsulfanylphenyl)-1,2,3,4-tetrahydroisochinolin;
7-(1-Cyclobutylazetidin-3-ylmethoxy)-2-methyl-4-(4-methylsulfanylphenyl)-1,2,3,4-tetrahydroisochinolin;
7-[1-(2-Fluorethyl)-azetidin-3-ylmethoxy]-2-methyl-4-(4-methylsulfanylphenyl)-1,2,3,4-tetrahydroisochinolin;
4-(4-Methoxyphenyl)-2-methyl-7-[2-(1-methylpyrrolidin-2-yl)-ethoxy]-1,2,3,4-tetrahydroisochinolin;
4-(4-Methoxyphenyl)-2-methyl-7-[2-(1-methylpyrrolidin-2-yl)-ethoxy]-1,2,3,4-tetrahydroisochinolin (Diastereomer 1);
4-(4-Methoxyphenyl)-2-methyl-7-[2-(1-methylpyrrolidin-2-yl)-ethoxy]-1,2,3,4-tetrahydroisochinolin (Diastereomer 2);
4-(3-Methoxyphenyl)-2-methyl-7-(piperidin-4-ylmethoxy)-1,2,3,4-tetrahydroisochinolin;
{4-[4-(4-Methoxyphenyl)-2-methyl-1,2,3,4-tetrahydroisochinolin-7-yloxymethyl]-piperidin-1-yl}-acetonitril;
2-Methyl-7-(1-methylpiperidin-4-yloxy)-4-(4-methylsulfanylphenyl)-1,2,3,4-tetrahydroisochinolin;
2-Methyl-4-(4-methylsulfanylphenyl)-7-[1-(3,3,3-trifluorpropyl)-piperidin-4-yloxy]-1,2,3,4-tetrahydroisochinolin;
{4-[2-Methyl-4-(4-methylsulfanylphenyl)-1,2,3,4-tetrahydroisochinolin-7-yloxy]-piperidin-1-yl}-acetonitril;
2-Methyl-4-(4-methylsulfanylphenyl)-7-[1-(tetrahydropyran-4-yl)-piperidin-4-yloxy]-1,2,3,4-tetrahydroisochinolin;
7-(1-Cyclopropylpiperidin-4-yloxy)-2-methyl-4-(4-methylsulfanylphenyl)-1,2,3,4-tetrahydroisochinolin;
7-(1-Cyclobutylpiperidin-4-ylmethoxy)-4-(4-methoxyphenyl)-2-methyl-1,2,3,4-tetrahydroisochinolin;
7-(1-Cyclopropylpiperidin-4-ylmethoxy)-4-(4-methoxyphenyl)-2-methyl-1,2,3,4-tetrahydroisochinolin;
1-{4-[4-(4-Methoxyphenyl)-2-methyl-1,2,3,4-tetrahydroisochinolin-7-yloxymethyl]-piperidin-1-yl}-2-methylpropan-2-ol;
4-(4-Methoxyphenyl)-2-methyl-7-(4-methylmorpholin-2-ylmethoxy)-1,2,3,4-tetrahydroisochinolin;
4-(4-Methoxyphenyl)-2-methyl-7-(morpholin-2S-ylmethoxy)-1,2,3,4-tetrahydroisochinolin;
4-(4-Methoxyphenyl)-2-methyl-7-(morpholin-2R-ylmethoxy)-1,2,3,4-tetrahydroisochinolin;
7-(4-Isopropylmorpholin-2-ylmethoxy)-4-(4-methoxyphenyl)-2-methyl-1,2,3,4-tetrahydroisochinolin;
7-(4-Isopropylmorpholin-2S-ylmethoxy)-4-(4-methoxyphenyl)-2-methyl-1,2,3,4-tetrahydroisochinolin;
7-(4-Isopropylmorpholin-2R-ylmethoxy)-4-(4-methoxyphenyl)-2-methyl-1,2,3,4-tetrahydroisochinolin;
7-(4-Isopropylmorpholin-2R-ylmethoxy)-4-(4-methoxyphenyl)-2-methyl-1,2,3,4-tetrahydroisochinolin (Diastereomer 1);
7-(4-Isopropylmorpholin-2R-ylmethoxy)-4-(4-methoxyphenyl)-2-methyl-1,2,3,4-tetrahydroisochinolin (Diastereomer 2);
7-(4-Ethylmorpholin-2-ylmethoxy)-4-(4-methoxyphenyl)-2-methyl-1,2,3,4-tetrahydroisochinolin;
7-[4-(2-Fluorethyl)-morpholin-2-ylmethoxy]-4-(4-methoxyphenyl)-2-methyl-1,2,3,4-tetrahydroisochinolin;
7-(4-Cyclopropylmorpholin-2-ylmethoxy)-4-(4-methoxyphenyl)-2-methyl-1,2,3,4-tetrahydroisochinolin;
7-(4-Cyclopropylmorpholin-2S-ylmethoxy)-4-(4-methoxyphenyl)-2-methyl-1,2,3,4-tetrahydroisochinolin;
7-(4-Cyclopropylmorpholin-2S-ylmethoxy)-4-(4-methoxyphenyl)-2-methyl-1,2,3,4-tetrahydroisochinolin (Diastereomer 1);
7-(4-Cyclopropylmorpholin-2S-ylmethoxy)-4-(4-methoxyphenyl)-2-methyl-1,2,3,4-tetrahydroisochinolin (Diastereomer 2);
7-(4-Cyclopropylmorpholin-2R-ylmethoxy)-4-(4-methoxyphenyl)-2-methyl-1,2,3,4-tetrahydroisochinolin;
7-(4-Cyclopropylmorpholin-2R-ylmethoxy)-4-(4-methoxyphenyl)-2-methyl-1,2,3,4-tetrahydroisochinolin (Diastereomer 1);
7-(4-Cyclopropylmorpholin-2R-ylmethoxy)-4-(4-methoxyphenyl)-2-methyl-1,2,3,4-tetrahydroisochinolin (Diastereomer 2);
7-(4-Isopropylmorpholin-2-ylmethoxy)-2-methyl-4-(4-methylsulfanylphenyl)-1,2,3,4-tetrahydroisochinolin;
7-(4-Cyclopropylmorpholin-2-ylmethoxy)-2-methyl-4-(4-methylsulfanylphenyl)-1,2,3,4-tetrahydroisochinolin;
2-Methyl-4-(4-methylsulfanylphenyl)-7-(morpholin-2-ylmethoxy)-1,2,3,4-tetrahydroisochinolin;
4-(4-Methoxyphenyl)-2,6-dimethyl-7-(3-piperidin-1-ylpropoxy)-1,2,3,4-tetrahydroisochinolin;
4-(4-Methoxyphenyl)-2,6-dimethyl-7-(3-morpholin-4-ylpropoxy)-1,2,3,4-tetrahydroisochinolin;
4-(3-Fluor-4-methoxyphenyl)-2,6-dimethyl-7-(3-morpholin-4-ylpropoxy)-1,2,3,4-tetrahydroisochinolin;
4-(3-Fluor-4-methoxyphenyl)-2,8-dimethyl-7-(3-morpholin-4-ylpropoxy)-1,2,3,4-tetrahydroisochinolin;
4-(4-Methoxyphenyl)-2,8-dimethyl-7-(3-morpholin-4-ylpropoxy)-1,2,3,4-tetrahydroisochinolin;
2,6-Dimethyl-4-(4-methylsulfanylphenyl)-7-(3-piperidin-1-ylpropoxy)-1,2,3,4-tetrahydroisochinolin;
7-(4-Piperidin-1-ylbut-1-inyl)-4-pyridin-3-yl-1,2,3,4-tetrahydroisochinolin;
7-(4-Piperidin-1-ylbutyl)-4-pyridin-3-yl-1,2,3,4-tetrahydroisochinolin;
2-Methyl-7-(4-piperidin-1-ylbutyl)-4-pyridin-3-yl-1,2,3,4-tetrahydroisochinolin;
7-[4-(4,4-Difluorpiperidin-1-yl)-but-1-inyl]-4-(4-methoxyphenyl)-2-methyl-1,2,3,4-tetrahydroisochinolin;
4-(4-Methoxyphenyl)-2-methyl-7-(4-piperidin-1-ylbut-1-inyl)-1,2,3,4-tetrahydroisochinolin;
4-(4-Methoxyphenyl)-2-methyl-7-(4-morpholin-4-ylbut-1-inyl)-1,2,3,4-tetrahydroisochinolin;
4-(4-Methoxyphenyl)-2-methyl-7-(4-thiomorpholin-4-ylbut-1-inyl)-1,2,3,4-tetrahydroisochinolin;
7-[4-(4-Isopropylpiperazin-1-yl)-but-1-inyl]-4-(4-methoxyphenyl)-2-methyl-1,2,3,4-tetrahydroisochinolin;
4-(4-Fluorphenyl)-2-methyl-7-(4-piperidin-1-ylbut-1-inyl)-1,2,3,4-tetrahydroisochinolin;
7-[4-(4,4-Difluorpiperidin-1-yl)-butyl]-4-(4-methoxyphenyl)-2-methyl-1,2,3,4-tetrahydroisochinolin;
4-(4-Methoxyphenyl)-2-methyl-7-(4-morpholin-4-ylbutyl)-1,2,3,4-tetrahydroisochinolin;
4-(4-Methoxyphenyl)-2-methyl-7-(4-thiomorpholin-4-ylbutyl)-1,2,3,4-tetrahydroisochinolin;
7-[4-(4-Isopropylpiperazin-1-yl)-butyl]-4-(4-methoxyphenyl)-2-methyl-1,2,3,4-tetrahydroisochinolin;
und Salze davon.

10. Verbindung von Formel (I): 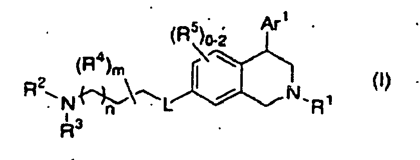 worin
L -O- ist und n 1 oder 2 ist; oder L -C≡C- oder -CH₂CH₂- ist und n 0 oder 1 ist;
R¹ -H ist; oder -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -COOC₁₋₆-Alkyl oder -COOBenzyl ist, von denen jedes gegebenenfalls einfach, zweifach oder dreifach mit R^{a} substituiert ist;
wobei R^{a} ausgewählt ist aus -OH, -OC₁₋₆-Alkyl, Phenyl, gegebenenfalls substituiert mit -OC₁₋₄-Alkyl oder Halo, -CN, -NO₂, -N(R^{b})R^{c}, -C(O)N(R^{b})R^{c}, -N(R^{b})C(O)R^{b}, -N(R^{b})SO₂C₁₋₆-Alkyl, -C(O)C₁₋₆-Alkyl, -S(O)₀₋₂-C₁₋₆-Alkyl, -SO₂N(R^{b})R^{c}, -SCF₃, Halo, -CF₃, -OCF₃, -COOH und -COOC₁₋₆-Alkyl;
wobei R^{b} und R^{c} jeweils unabhängig -H oder -C₁₋₆-Alkyl sind;
R² und R³ jeweils unabhängig ausgewählt sind aus der Gruppe, bestehend aus : -H;
A) -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -CH(C₃₋₈-Cycloalkyl)₂, -CH(Phenyl)₂, Benzyl und -C(O)OC₁₋₄-Alkyl, wobei jedes Alkyl, Cycloalkyl oder Benzyl gegebenenfalls mit -OH, -OC₁₋₄-Alkyl, -CN, -NH₂, -NH(C₁₋₄-Alkyl), -N(C₁₋₄-Alkyl)₂, Halo, -CF₃, -OCF₃, -COOH oder -COOC₁₋₆-Alkyl substituiert ist;
B) Phenyl oder Pyridyl, gegebenenfalls an zwei benachbarten Kohlenstoffringgliedern an eine drei- oder viergliedrige Kohlenwasserstoffeinheit kondensiert, um einen kondensierten fünf- oder sechsgliedrigen aromatischen Ring zu bilden, wobei in der Einheit ein Kohlenstoffatom durch >O, >S, >NH, >N(C₁₋₄-Alkyl) oder >NC(O)OC₁₋₄-Alkyl ersetzt ist und wobei in der Einheit bis zu ein zusätzliches Kohlenstoffatom gegebenenfalls durch -N= ersetzt ist;
C) Naphthyl;
D) einem 4- bis 8-gliedrigen heterocyclischen Ring, wobei der heterocyclische Ring ein Kohlenstoffatom aufweist, das der Bindungspunkt ist, 1 oder 2 Heteroatomglieder aufweist, die ausgewählt sind aus der Gruppe, bestehend aus >O, >S(O)₀₋₂, >NH, >N(C₁₋₄-Alkyl) und >NC(O)OC₁₋₄-Alkyl, und 0 oder 1 Doppelbindungen aufweist; und
E) einer monocyclischen aromatischen Kohlenwasserstoffgruppe, die fünf oder sechs Ringatome aufweist, die ein Kohlenstoffatom aufweist, das der Bindungspunkt ist, in der ein Kohlenstoffatom durch >O, >S, >NH, >N(C₁₋₄-Alkyl) oder >NC(O)OC₁₋₄-Alkyl ersetzt ist, in der bis zu ein zusätzliches Kohlenstoffatom gegebenenfalls durch -N= ersetzt ist und die gegebenenfalls benzokondensiert oder pyridokondensiert ist;
wobei jedes von B)-E) gegebenenfalls einfach, zweifach oder dreifach mit einer Einheit substituiert sind, die ausgewählt ist aus der Gruppe, bestehend aus -C₁₋₄-Alkyl, -OH, -C₁₋₄-AlkylOH, -OC₁₋₆-Alkyl, -CN, -NO₂, -N(R^{d})R^{e}, -C(O)N(R^{d})R^{e}, -N(R^{d})C(O)R^{d}, -N(R^{d})SO₂C₁₋₆-Alkyl, -C(O)C₁₋₆-Alkyl, -S(O)₀₋₂-C₁₋₆-Alkyl, -SO₂N(R^{d})R^{e}, -SCF₃, Halo, -CF₃, -OCF₃, -COOH, -COOC₁₋₆-Alkyl, -OC(O)N(R^{d})R^{e} und -OC(O)OC₁₋₆-Alkyl;
wobei R^{d} und R^{e} jeweils unabhängig -H oder -C₁₋₆-Alkyl sind,
oder, alternativ,
R² und R³ mit dem Stickstoff, an das sie gebunden sind, zusammengenommen sein können, um Tetrahydropyrimidin-2-ylidenyl oder einen 4- bis 8-gliedrigen heterocyclischen Ring zu bilden, wobei der heterocyclische Ring 0 oder 1 zusätzliche Heteroatomglieder aufweist, die vom Bindungsstickstoff durch wenigstens ein Kohlenstoffglied getrennt sind und ausgewählt sind aus der Gruppe, bestehend aus >O, >S(O)₀₋₂, >NH und >NR^{f}, 0 oder 1 Doppelbindungen aufweist, 0, 1 oder 2 Kohlenstoffglieder aufweist, die vom Bindungsstickstoff durch wenigstens ein Kohlenstoffglied getrennt sind, das ein Carbonyl ist, gegebenenfalls benzo- oder pyridokondensiert ist, gegebenenfalls ein Kohlenstoffglied aufweist, das eine Brücke bildet, und 0-5 Kohlenstoffgliedsubstituenten R^{ff} aufweist,
wobei R^{f} ausgewählt ist aus der Gruppe, bestehend aus:
i) -C₁₋₆-Alkyl, gegebenenfalls substituiert mit R^{z}, -C₃₋₆-Alkenyl, -C₃₋₆-Alkinyl, -C(O)N(R^{g})R^{h}, -C(O)Rⁱ, -S(O)₀₋₂-C₁₋₆-Alkyl und -COOC₁₋₆-Alkyl,
wobei R^{z} Fluor, -CN, -OH, -OC₁₋₄-Alkyl, -C₃₋₇-Cycloalkyl oder -CF₃ ist;
wobei R^{g} und R^{h} jeweils unabhängig -H oder -C₁₋₆-Alkyl sind; und
wobei Rⁱ -C₁₋₆-Alkyl, -C₃₋₈-Cycloalkyl, Phenyl oder 5-oder 6-gliedriges aromatisches Heterocyclyl ist, wobei jedes Alkyl, Cycloalkyl, Phenyl oder Heterocyclyl gegebenenfalls einfach, zweifach oder dreifach mit -C₁₋₄-Alkyl, -OH, -OC₁₋₆-Alkyl, -CF₃, -CN oder Halo substituiert ist;
ii) -(CH₂)₀₋₁-RingA, wobei Ring A Phenyl oder ein 5- oder 6-gliedriges kohlenstoffverknüpftes aromatisches Heterocyclyl ist, gegebenenfalls substituiert mit R^{aa};
wobei R^{aa} -OH, -C₁₋₆-Alkyl, -OC₁₋₆-Alkyl, Phenyl, -CN, -NO₂, -N(R^{g})R^{h}, -C(O)N(R^{g})R^{h}, -N(R^{g})C(O)R^{h}, -N(R^{h})SO₂C₁₋₆-Alkyl, -C(O)C₁₋₆-Alkyl, -S(O)₀₋₂-C₁₋₆-Alkyl, -SO₂N(R^{g})R^{h}, -SCF₃, Halo, -CF₃, -OCF₃, -OCHF₂, -COOH und -COOC₁₋₆-Alkyl ist; und iii) -(CH₂)₀₋₁-RingB, wobei Ring B -C₃₋₇-Cycloalkyl ist, in dem ein oder zwei Kohlenstoffringglieder gegebenenfalls durch >O oder >NH ersetzt sind und das gegebenenfalls mit -C₁₋₄-Alkyl, Fluor, -CN, -OH, -OC₁₋₄-Alkyl oder -CF₃ substituiert ist;
wobei R^{ff} ausgewählt ist aus der Gruppe, bestehend aus -C₁₋₆-Alkyl, gegebenenfalls einfach oder zweifach substituiert mit R^{z}, -C₂₋₆-Alkenyl, -C₂₋₆-Alkinyl, -C₃₋₇-Cycloalkyl, -CH(Phenyl)₂, Halo, -OH, -OC₁₋₆-Alkyl, -OC₂₋₃-Alkyl-O-, -CN, -NO₂, -N(R^{g})R^{h}, -C(O)N(R^{g})R^{h}, -N(R^{g})C(O)R^{g}, -N(R^{g})SO₂C₁₋₆-Alkyl, -C(O)Rⁱ, -S(O)₀₋₂-C₁₋₆-Alkyl, -SO₂N(R^{g})R^{h}, -SCF₃, -CF₃, -OCF₃, -COOH und -COOC₁₋₆-Alkyl;
R⁴ -OH, -OC₁₋₆-Alkyl, -CF₃, -C₁₋₆-Alkyl oder Halo ist; zwei R⁴-Substituenten zusammengenommen sein können, um Methylen oder Ethylen zu bilden; oder eines von R⁴ mit R² zusammengenommen ist, um Methylen, Ethylen, Propylen oder -CH₂CH₂O- zu bilden, wobei jedes gegebenenfalls mit -OH, -OC₁₋₆-Alkyl, -SC₁₋₆-Alkyl, -CF₃, -C₁₋₆-Alkyl, Amino oder Halo substituiert ist;
m 0, 1 oder 2 ist;
R⁵ ausgewählt ist aus der Gruppe, bestehend aus -C₁₋₆-Alkyl, -OH, -OC₁₋₆-Alkyl, -SC₁₋₆-Alkyl und Halo;
Ar¹ ein Aryl- oder Heteroarylring ist, der ausgewählt ist aus der Gruppe, bestehend aus:
a) Phenyl, gegebenenfalls einfach, zweifach oder dreifach substituiert mit R^{j} und gegebenenfalls auf benachbarten Kohlenstoffen zweifach substituiert mit -OC₁₋₄-AlkylenO-, gegebenenfalls einfach oder zweifach substituiert mit Fluor, -(CH₂)₂₋₃NH-, -(CH₂)₁₋₂-NH(CH₂)-, -(CH₂)₂₋₃N(C₁₋₄-Alkyl)- oder -(CH₂)₁₋₂N(C₁₋₄-Alkyl)(CH₂)-;
wobei Rⁱ ausgewählt ist aus der Gruppe, bestehend aus
1) -OH, -C₁₋₆-Alkyl, -OC₁₋₆-Alkyl, gegebenenfalls einfach, zweifach oder dreifach substituiert mit Halo, -C₂₋₆-Alkenyl, -OC₃₋₆-Alkenyl, -C₂₋₆-Alkinyl, gegebenenfalls substituiert mit Trimethylsilyl, -OC₃₋₆-Alkinyl, -C₃₋₆-Cycloalkyl, -OC₃₋₆-Cycloalkyl, -CN, -NO₂, -N(R^{k})R^{l}, -N(R^{k})C(O)R^{l}, -N(R^{k})SO₂C₁₋₆-Alkyl, -C(O)C₁₋₆-Alkyl, -S(O)₀₋₂-C₁₋₆-Alkyl, -C(O)N(R^{m})Rⁿ, -SO₂N(R^{m})Rⁿ, -SCF₃, Halo, -CF₃, -COOH, -COOC₁₋₆-Alkyl und -COOC₃₋₇-Cycloalkyl;
wobei R^{k} und R^{l} jeweils unabhängig -H oder -C₁₋₆-Alkyl sind;
wobei R^{m} und Rⁿ jeweils unabhängig -H oder -C₁₋₆-Alkyl sind oder R^{m} und Rⁿ mit ihrem Bindungsstickstoff zusammengenommen einen 4- bis 8-gliedrigen heterocyclischen Ring bilden, der 1 oder 2 Heteroatomglieder aufweist, die ausgewählt sind aus >O, >S(O)₀₋₂, >NH und >NC₁₋₆-Alkyl, der 0 oder 1 Doppelbindungen aufweist, der 0 oder 1 Carbonylglieder aufweist;
2) -G-Ar², wobei G eine Bindung, -O- oder -S- ist und Ar² Phenyl ist oder eine monocyclische aromatische Kohlenwasserstoffgruppe ist, die fünf oder sechs Ringatome aufweist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die jeweils gegebenenfalls einfach, zweifach oder dreifach mit R^{p} substituiert ist;
wobei R^{p} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{q})R^{r}, -C(O)N(R^{q})R^{r}, -N(R^{q})C(O)R^{r}, -N(R^{q})SO₂C₁₋₆-Alkyl, -C(O)C₁₋₆-Alkyl, -S(O)₀₋₂-C₁₋₆-Alkyl, -SO₂N(R^{q})R^{r}, -SCF₃, Halo, -CF₃, -OCF₃, -OCHF₂, -COOH und -COOC₁₋₆-Alkyl;
wobei R^{q} und R^{r} jeweils unabhängig ausgewählt sind aus -H, -C₁₋₆-Alkyl und -C₂₋₆-Alkenyl; und
3) einem 4- bis 8-gliedrigen gesättigten oder teilweise gesättigten heterocyclischen Ring, der 1 oder 2 Heteroatomglieder aufweist, die ausgewählt sind aus >O, >S(O)₀₋₂, >NH und >NC₁₋₆-Alkyl, der 0 oder 1 Carbonylglieder aufweist, wobei der Ring gegebenenfalls einfach, zweifach oder dreifach mit R^{p} substituiert ist;
b) Phenyl oder Pyridyl, an zwei benachbarten Kohlenstoffringgliedern an eine dreigliedrige Kohlenwasserstoffeinheit kondensiert, um einen kondensierten fünfgliedrigen aromatischen Ring zu bilden, wobei in der Einheit ein Kohlenstoffatom durch >O, >S, >NH oder >N(C₁₋₄-Alkyl) ersetzt ist und wobei in der Einheit bis zu ein zusätzliches Kohlenstoffatom gegebenenfalls durch -N= ersetzt ist, wobei die kondensierten Ringe gegebenenfalls einfach, zweifach oder dreifach mit R^{t} substituiert sind;
wobei R^{t} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{u})R^{v}, -C(O)N(R^{u})R^{v}_{,} -N(R^{u})C(O)R^{v}, -N(R^{u})SO₂C₁₋₆-Alkyl, -C(O)C₁₋₆-Alkyl, -S(O)₀₋₂-C₁₋₆-Alkyl, -SO₂N(R^{u})R^{v}, -SCF₃, Halo, -CF₃, -OCF₃, -OCHF₂, -COOH und -COOC₁₋₆-Alkyl;
wobei R^{u} und R^{v} jeweils unabhängig -H oder -C₁₋₆-Alkyl sind;
c) Phenyl, an zwei benachbarten Ringgliedern an eine viergliedrige Kohlenwasserstoffeinheit kondensiert, um einen kondensierten sechsgliedrigen aromatischen Ring zu bilden, wobei in der Einheit ein oder zwei Kohlenstoffatome durch -N= ersetzt sind, wobei die kondensierten Ringe gegebenenfalls einfach, zweifach oder dreifach mit R^{t} substituiert sind;
d) Naphthyl, gegebenenfalls einfach, zweifach oder dreifach mit R^{t} substituiert;
e) einer monocyclischen aromatischen Kohlenwasserstoffgruppe, die fünf Ringatome aufweist, die ein Kohlenstoffatom aufweist, das der Bindungspunkt ist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die gegebenenfalls einfach oder zweifach mit R^{j} substituiert ist und die gegebenenfalls an zwei benachbarten Kohlenstoffatomen benzokondensiert oder pyridokondensiert ist, wobei die benzokondensierte oder pyridokondensierte Einheit gegebenenfalls einfach, zweifach oder dreifach mit R^{t} substituiert ist; und
f) einer monocyclischen aromatischen Kohlenwasserstoffgruppe, die sechs Ringatome aufweist, die ein Kohlenstoffatom aufweist, das der Bindungspunkt ist, in der ein oder zwei Kohlenstoffatome durch -N= ersetzt sind, die gegebenenfalls einfach oder zweifach mit R^{j} substituiert ist und die gegebenenfalls an zwei benachbarten Kohlenstoffatomen benzokondensiert oder pyridokondensiert ist, wobei die benzokondensierte oder pyridokondensierte Einheit gegebenenfalls einfach oder zweifach mit R^{t} substituiert ist;
und Enantiomere, Diastereomere, Hydrate, Solvate und pharmazeutisch verträgliche Salze, Ester und Amide davon, mit der Maßgabe:
wenn n 1 ist, L -O- ist, Ar unsubstituiertes Phenyl ist und R² und R³ beide Methyl sind, dann ist R⁴ nicht -OH,
zur Verwendung bei der Behandlung einer ZNS-Störung, die ausgewählt ist aus der Gruppe, bestehend aus: Depression, gestörtem Schlaf, Müdigkeit, Lethargie, kognitiver Beeinträchtigung, Gedächtnisbeeinträchtigung, Gedächtnisverlust, Lernbeeinträchtigung und Aufmerksamkeitsdefizitstörungen, in Säugern.

11. Verbindung von Formel (II): 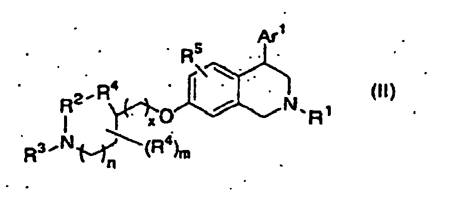 worin
n 1 oder 2 ist;
x 0 oder 1 ist;
wobei n + x 1 oder 2 ist;
R¹ -H ist; oder -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -COOC₁₋₆-Alkyl oder -COOBenzyl ist, von denen jedes gegebenenfalls einfach, zweifach oder dreifach mit R^{a} substituiert ist;
wobei R^{a} ausgewählt ist aus -OH, -OC₁₋₆-Alkyl, Phenyl, gegebenenfalls substituiert mit -OC₁₋₄-Alkyl oder Halo, -CN, -NO₂, -N(R^{b})R^{c}, -C(O)N(R^{b})R^{c}, -N(R^{b})C(O)R^{b}, -N(R^{b})SO₂C₁₋₆-Alkyl, -C(O)C₁₋₆-Alkyl, -S(O)₀₋₂-C₁₋₆-Alkyl, -SO₂N(R^{b})R^{c}, -SCF₃, Halo, -CF₃, -OCF₃, -COOH und -COOC₁₋₆-Alkyl;
wobei R^{b} und R^{c} jeweils unabhängig -H oder -C₁₋₆-Alkyl sind;
R³ ist ausgewählt aus der Gruppe, bestehend aus: -H;
A) -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -CH(C₃₋₈-Cycloalkyl)₂, -CH(Phenyl)₂, Benzyl und -C(O)OC₁₋₄-Alkyl, wobei jedes Alkyl, Cycloalkyl oder Benzyl gegebenenfalls mit -OH, -OC₁₋₄-Alkyl, -CN, -NH₂, -NH(C₁₋₄-Alkyl), -N(C₁₋₄-Alkyl)₂, Halo, -CF₃, -OCF₃, -COOH oder -COOC₁₋₆-Alkyl substituiert ist;
B) Phenyl oder Pyridyl, gegebenenfalls an zwei benachbarten Kohlenstoffringgliedern an eine drei- oder viergliedrige Kohlenwasserstoffeinheit kondensiert, um einen kondensierten fünf- oder sechsgliedrigen aromatischen Ring zu bilden, wobei in der Einheit ein Kohlenstoffatom durch >O, >S, >NH, >N(C₁₋₄-Alkyl) oder >NC(O)OC₁₋₄-Alkyl ersetzt ist und wobei in der Einheit bis zu ein zusätzliches Kohlenstoffatom gegebenenfalls durch -N= ersetzt ist;
C) Naphthyl;
D) einem 4- bis 8-gliedrigen heterocyclischen Ring, wobei der heterocyclische Ring ein Kohlenstoffatom aufweist, das der Bindungspunkt ist, 1 oder 2 Heteroatomglieder aufweist, die ausgewählt sind aus der Gruppe, bestehend aus >O, >S(O)₀₋₂, >NH, >N(C₁₋₄-Alkyl) und >NC(O)OC₁₋₄-Alkyl, und 0 oder 1 Doppelbindungen aufweist; und
E) einer monocyclischen aromatischen Kohlenwasserstoffgruppe, die fünf oder sechs Ringatome aufweist, die ein Kohlenstoffatom aufweist, das der Bindungspunkt ist, in der ein Kohlenstoffatom durch >O, >S, >NH, >N(C₁₋₄-Alkyl) oder >NC(O)OC_{1- 4}-Alkyl ersetzt ist, in der bis zu ein zusätzliches Kohlenstoffatom gegebenenfalls durch -N= ersetzt ist und die gegebenenfalls benzokondensiert oder pyridokondensiert ist;
wobei B)-E) jeweils gegebenenfalls einfach, zweifach oder dreifach mit einer Einheit substituiert sind, die ausgewählt ist aus der Gruppe, bestehend aus -C₁₋₄-Alkyl, -OH, -C₁₋₄-AlkylOH, -OC₁₋₆-Alkyl, -CN, -NO₂, -N(R^{d})R^{e}, -C(O)N(R^{d})R^{e}, -N(R^{d})C(O)R^{d}, -N(R^{d})SO₂C₁₋₆-Alkyl, -C(O)C₁₋₆-Alkyl, -S(O)₀₋₂-C₁₋₆-Alkyl, -SO₂N(R^{d})R^{e}, -SCF₃, Halo, -CF₃, -OCF₃, -COOH, -COOC₁₋₆-Alkyl, -OC(O)N(R^{d})R^{e} und -OC(O)OC₁₋₆-Alkyl;
wobei R^{d} und R^{e} jeweils unabhängig -H oder -C₁₋₆-Alkyl sind;
-R²-R⁴- Methylen, Ethylen, Propylen oder -CH₂CH₂O- ist, wobei jedes gegebenenfalls mit -OH, -OC₁₋₆-Alkyl, -SC₁₋₆-Alkyl, -CF₃, -C₁₋₆-Alkyl, Amino oder Halo substituiert ist;
R⁴ -OH, -OC₁₋₆-Alkyl, -CF₃, -C₁₋₆-Alkyl oder Halo ist;
m 0, 1 oder 2 ist;
R⁵ ausgewählt ist aus der Gruppe, bestehend aus -C₁₋₆-Alkyl, -OH, -OC₁₋₆-Alkyl, -SC₁₋₆-Alkyl und Halo;
Ar¹ ein Aryl- oder Heteroarylring ist, der ausgewählt ist aus der Gruppe, bestehend aus:
a) Phenyl, gegebenenfalls einfach, zweifach oder dreifach substituiert mit R^{j} und gegebenenfalls auf benachbarten Kohlenstoffen zweifach substituiert mit -OC₁₋₄-AlkylenO-, gegebenenfalls einfach oder zweifach substituiert mit Fluor, -(CH₂)₂₋₃NH-, -(CH₂)₁₋₂-NH(CH₂)-, -(CH₂)₂₋₃N(C₁₋₄-Alkyl)- oder -(CH₂)₁₋₂N(C₁₋₄-Alkyl)(CH₂)-;
wobei R^{j} ausgewählt ist aus der Gruppe, bestehend aus
1) -OH, -C₁₋₆-Alkyl, -OC₁₋₆-Alkyl, gegebenenfalls einfach, zweifach oder dreifach substituiert mit Halo, -C₂₋₆-Alkenyl, -OC₃₋₆-Alkenyl, -C₂₋₆-Alkinyl, gegebenenfalls substituiert mit Trimethylsilyl, -OC₃₋₆-Alkinyl, -C₃₋₆-Cycloalkyl, -OC₃₋₆-Cycloalkyl, -CN, -NO₂, -N(R^{k})R^{l}, -N(R^{k})C(O)R^{l}, -N(R^{k})SO₂C₁₋₆-Alkyl, -C(O)C₁₋₆-Alkyl, -S(O)₀₋₂-C₁₋₆-Alkyl, -C(O)N(R^{m})Rⁿ, -SO₂N(R^{m})Rⁿ, -SCF₃, Halo, -CF₃, -COOH, -COOC₁₋₆-Alkyl und -COOC₃₋₇-Cycloalkyl;
wobei R^{k} und R^{l} jeweils unabhängig -H oder -C₁₋₆-Alkyl sind;
wobei R^{m} und Rⁿ jeweils unabhängig -H oder -C₁₋₆-Alkyl sind oder R^{m} und Rⁿ mit ihrem Bindungsstickstoff zusammengenommen einen 4- bis 8-gliedrigen heterocyclischen Ring bilden, der 1 oder 2 Heteroatomglieder aufweist, die ausgewählt sind aus >O, >S(O)₀₋₂, >NH und >NC₁₋₆-Alkyl, der 0 oder 1 Doppelbindungen aufweist, der 0 oder 1 Carbonylglieder aufweist;
2) -G-Ar², wobei G eine Bindung, -O- oder -S- ist und Ar² Phenyl ist oder eine monocyclische aromatische Kohlenwasserstoffgruppe ist, die fünf oder sechs Ringatome aufweist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die jeweils gegebenenfalls einfach, zweifach oder dreifach mit R^{p} substituiert ist;
wobei R^{p} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{q})R^{r}, -C(O)N(R^{q})R^{r}, -N(R^{q})C(O)R^{r}, -N(R^{q})SO₂C₁₋₆-Alkyl, -C(O)C₁₋₆-Alkyl, -S(O)₀-₂-C₁₋₆-Alkyl, -SO₂N(R^{q})R^{r}, -SCF₃, Halo, -CF₃, -OCF₃, -OCHF₂, -COOH und -COOC₁₋₆-Alkyl;
wobei R^{q} und R^{r} jeweils unabhängig ausgewählt sind aus -H, -C₁₋₆-Alkyl und -C₂₋₆-Alkenyl; und
3) einem 4- bis 8-gliedrigen gesättigten oder teilweise gesättigten heterocyclischen Ring, der 1 oder 2 Heteroatomglieder aufweist, die ausgewählt sind aus >O, >S(O)₀₋₂, >NH und >NC₁₋₆-Alkyl, der 0 oder 1 Carbonylglieder aufweist, wobei der Ring gegebenenfalls einfach, zweifach oder dreifach mit R^{p} substituiert ist;
b) Phenyl oder Pyridyl, an zwei benachbarten Kohlenstoffringgliedern an eine dreigliedrige Kohlenwasserstoffeinheit kondensiert, um einen kondensierten fünfgliedrigen aromatischen Ring zu bilden, wobei in der Einheit ein Kohlenstoffatom durch >O, >S, >NH oder >N(C₁₋₄-Alkyl) ersetzt ist und wobei in der Einheit bis zu ein zusätzliches Kohlenstoffatom gegebenenfalls durch -N= ersetzt ist, wobei die kondensierten Ringe gegebenenfalls einfach, zweifach oder dreifach mit R^{t} substituiert sind;
wobei R^{t} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{u})R^{v}, -C(O)N(R^{u})R^{v}, -N(R^{u})C(O)R^{v}, -N(R^{u})SO₂C₁₋₆-Alkyl, -C(O)C₁₋₆-Alkyl, -S(O)₀₋₂-C₁₋₆-Alkyl, -SO₂N(R^{u})R^{v}, -SCF₃, Halo, -CF₃, -OCF₃, -OCHF₂, -COOH und -COOC₁₋₆-Alkyl;
wobei R^{u} und R^{v} jeweils unabhängig -H oder -C₁₋₆-Alkyl sind;
c) Phenyl, an zwei benachbarten Ringgliedern an eine viergliedrige Kohlenwasserstoffeinheit kondensiert, um einen kondensierten sechsgliedrigen aromatischen Ring zu bilden, wobei in der Einheit ein oder zwei Kohlenstoffatome durch -N= ersetzt sind, wobei die kondensierten Ringe gegebenenfalls einfach, zweifach oder dreifach mit R^{t} substituiert sind;
d) Naphthyl, gegebenenfalls einfach, zweifach oder dreifach mit R^{t} substituiert;
e) einer monocyclischen aromatischen Kohlenwasserstoffgruppe, die fünf Ringatome aufweist, die ein Kohlenstoffatom aufweist, das der Bindungspunkt ist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die gegebenenfalls einfach oder zweifach mit R^{j} substituiert ist und gegebenenfalls an zwei benachbarten Kohlenstoffatomen benzokondensiert oder pyridokondensiert ist, wobei die benzokondensierte oder pyridokondensierte Einheit gegebenenfalls einfach, zweifach oder dreifach mit R^{t} substituiert ist; und
f) einer monocyclischen aromatischen Kohlenwasserstoffgruppe, die sechs Ringatome aufweist, die ein Kohlenstoffatom aufweist, das der Bindungspunkt ist, in der ein oder zwei Kohlenstoffatome durch -N= ersetzt sind, die gegebenenfalls einfach oder zweifach mit R^{j} substituiert ist und die gegebenenfalls an zwei benachbarten Kohlenstoffatomen benzokondensiert oder pyridokondensiert ist, wobei die benzokondensierte oder pyridokondensierte Einheit gegebenenfalls einfach oder zweifach mit R^{t} substituiert ist;
und Enantiomere, Diastereomere, Hydrate, Solvate und pharmazeutisch verträgliche Salze, Ester und Amide davon zur Verwendung bei den Behandlungen einer ZNS-Störung, ausgewählt aus der Gruppe, bestehend aus: Schlaf/Wach- und Aufwach/Vigilanz-Störungen, Schlaflosigkeit, Jetlag, gestörtem Schlaf, Aufinerksamkeitsdefizitstörungen mit Hyperaktivität (ADHD), Aufinerksamkeitsdefizitstörungen, Lern- und Gedächtnisstörungen, Lernbeeinträchtigung, Gedächtnisbeeinträchtigung, Gedächtnisverlust, kognitiver Dysfunktion, Migräne, neurogener Entzündung, Demenz, milder kognitiver Beeinträchtigung, Prädemenz, Alzheimer-Krankheit, Epilepsie, Narkolepsie mit oder ohne assoziierte Kataplexie, Kataplexie, Störungen der Schlaf/Wach-Homoeostase, idiopathischer Somnolenz, übermäßiger Tagschläfrigkeit (EDS), Störungen des Tag/Nacht-Rhythmus, Schlaf/Müdigkeitsstörungen, Müdigkeit, Benommenheit im Zusammenhang mit Schlafapnoe, Schlafbeeinträchtigung aufgrund perimenopausaler hormonaler Verschiebungen, Müdigkeit im Zusammenhang mit Parkinson, Müdigkeit im Zusammenhang mit MS, Müdigkeit im Zusammenhang mit Depression, durch Chemotherapie induzierter Müdigkeit, Müdigkeit im Zusammenhang mit Arbeit, Lethargie, Essstörungen, Fettleibigkeit, Bewegungskrankheit, Schwindel, Schizophrenie, Substanzmissbrauch, bipolaren Störungen, manischen Störungen und Depression, in Säugern.

12. Verbindung von Formel (II): 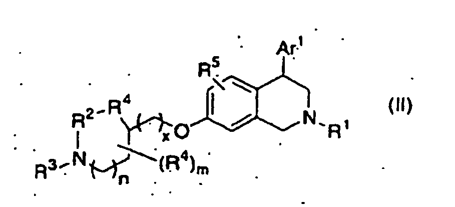 worin
n 1 oder 2 ist;
x 0 oder 1 ist;
wobei n + x 1 oder 2 ist;
R¹ -H ist; oder -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -COOC₁₋₆-Alkyl oder -COOBenzyl ist, von denen jedes gegebenenfalls einfach, zweifach oder dreifach mit R^{a} substituiert ist;
wobei R^{a} ausgewählt ist aus -OH, -OC₁₋₆-Alkyl, Phenyl, gegebenenfalls substituiert mit -OC₁₋₄-Alkyl oder Halo, -CN, -NO₂, -N(R^{b})R^{c}, -C(O)N(R^{b})R^{c}, -N(R^{b})C(O)R^{b}, -N(R^{b})SO₂C₁₋₆-Alkyl, -C(O)C₁₋₆-Alkyl, -S(O)₀₋₂-C₁₋₆-Alkyl, -SO₂N(R^{b})R^{c}, -SCF₃, Halo, -CF₃, -OCF₃, -COOH und -COOC₁₋₆-Alkyl;
wobei R^{b} und R^{c} jeweils unabhängig -H oder -C₁₋₆-Alkyl sind;
R³ ist ausgewählt aus der Gruppe, bestehend aus: -H;
A) -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -CH(C₃₋₈-Cycloalkyl)₂, -CH(Phenyl)₂, Benzyl und -C(O)OC₁₋₄-Alkyl, wobei jedes Alkyl, Cycloalkyl oder Benzyl gegebenenfalls mit -OH, -OC₁₋₄-Alkyl, -CN, -NH₂, -NH(C₁₋₄-Alkyl), -N(C₁₋₄-Alkyl)₂, Halo, -CF₃, -OCF₃, -COOH oder -COOC₁₋₆-Alkyl substituiert ist;
B) Phenyl oder Pyridyl, gegebenenfalls an zwei benachbarten Kohlenstoffringgliedern an eine drei- oder viergliedrige Kohlenwasserstoffeinheit kondensiert, um einen kondensierten fünf- oder sechsgliedrigen aromatischen Ring zu bilden, wobei in der Einheit ein Kohlenstoffatom durch >O, >S, >NH, >N(C₁₋₄-Alkyl) oder >NC(O)OC₁₋₄-Alkyl ersetzt ist und wobei in der Einheit bis zu ein zusätzliches Kohlenstoffatom gegebenenfalls durch -N= ersetzt ist;
C) Naphthyl;
D) einem 4- bis 8-gliedrigen heterocyclischen Ring, wobei der heterocyclische Ring ein Kohlenstoffatom aufweist, das der Bindungspunkt ist, 1 oder 2 Heteroatomglieder aufweist, die ausgewählt sind aus der Gruppe, bestehend aus >O, >S(O)₀₋₂, >NH, >N(C₁₋₄-Alkyl) und >NC(O)OC₁₋₄-Alkyl, und 0 oder 1 Doppelbindungen aufweist; und
E) einer monocyclischen aromatischen Kohlenwasserstoffgruppe, die fünf oder sechs Ringatome aufweist, die ein Kohlenstoffatom aufweist, das der Bindungspunkt ist, in der ein Kohlenstoffatom durch >O, >S, >NH, >N(C₁₋₄-Alkyl) oder >NC(O)OC₁₋₄-Alkyl ersetzt ist, in der bis zu ein zusätzliches Kohlenstoffatom gegebenenfalls durch -N= ersetzt ist und die gegebenenfalls benzokondensiert oder pyridokondensiert ist;
wobei B)-E) jeweils gegebenenfalls einfach, zweifach oder dreifach mit einer Einheit substituiert sind, die ausgewählt ist aus der Gruppe, bestehend aus -C₁₋₄-Alkyl, -OH, -C₁₋₄-AlkylOH, -OC₁₋₆-Alkyl, -CN, -NO₂, -N(R^{d})R^{e}, -C(O)N(R^{d})R^{e}, -N(R^{d})C(O)R^{d}, -N(R^{d})SO₂C₁₋₆-Alkyl, -C(O)C₁₋₆-Alkyl, -S(O)₀₋₂-C₁₋₆-Alkyl, -SO₂N(R^{d})R^{e}, -SCF₃, Halo, -CF₃, -OCF₃, -COOH, -COOC₁₋₆-Alkyl, -OC(O)N(R^{d})R^{e} und -OC(O)OC₁₋₆-Alkyl;
wobei R^{d} und R^{e} jeweils unabhängig -H oder -C₁₋₆-Alkyl sind;
-R²-R⁴- Methylen, Ethylen, Propylen oder -CH₂CH₂O- ist, wobei jedes gegebenenfalls mit -OH, -OC₁₋₆-Alkyl, -SC₁₋₆-Alkyl, -CF₃, -C₁₋₆-Alkyl, Amino oder Halo substituiert ist;
R⁴ -OH, -OC₁₋₆-Alkyl, -CF₃, -C₁₋₆-Alkyl oder Halo ist;
m 0, 1 oder 2 ist;
R⁵ ausgewählt ist aus der Gruppe, bestehend aus -C₁₋₆-Alkyl, -OH, -OC₁₋₆-Alkyl, -SC₁₋₆-Alkyl und Halo;
Ar¹ ein Aryl- oder Heteroarylring ist, der ausgewählt ist aus der Gruppe, bestehend aus:
a) Phenyl, gegebenenfalls einfach, zweifach oder dreifach substituiert mit R^{j} und gegebenenfalls auf benachbarten Kohlenstoffen zweifach substituiert mit -OC₁₋₄-AlkylenO-, gegebenenfalls einfach oder zweifach substituiert mit Fluor, -(CH₂)₂₋₃NH-, -(CH₂)₁₋₂-NH(CH₂)-, -(CH₂)₂₋₃N(C₁₋₄-Alkyl)- oder -(CH₂)₁₋₂N(C₁₋₄-Alkyl)(CH₂)-;
wobei R^{j} ausgewählt ist aus der Gruppe, bestehend aus
1) -OH, -C₁₋₆-Alkyl, -OC₁₋₆-Alkyl, gegebenenfalls einfach, zweifach oder dreifach substituiert mit Halo, -C₂₋₆-Alkenyl, -OC₃₋₆-Alkenyl, -C₂₋₆-Alkinyl, gegebenenfalls substituiert mit Trimethylsilyl, -OC₃₋₆-Alkinyl, -C₃₋₆-Cycloalkyl, -OC₃₋₆-Cycloalkyl, -CN, -NO₂, -N(R^{k})R^{l}, -N(R^{k})C(O)R^{l}, -N(R^{k})SO₂C₁₋₆-Alkyl, -C(O)C₁₋₆-Alkyl, -S(O)₀₋₂-C₁₋₆-Alkyl, -C(O)N(R^{m})Rⁿ, -SO₂N(R^{m})Rⁿ, -SCF₃, Halo, -CF₃, -COOH, -COOC₁₋₆-Alkyl und -COOC₃₋₇-Cycloalkyl;
wobei R^{k} und R^{l} jeweils unabhängig -H oder -C₁₋₆-Alkyl sind;
wobei R^{m} und Rⁿ jeweils unabhängig -H oder -C₁₋₆-Alkyl sind oder R^{m} und Rⁿ mit ihrem Bindungsstickstoff zusammengenommen einen 4- bis 8-gliedrigen heterocyclischen Ring bilden, der 1 oder 2 Heteroatomglieder aufweist, die ausgewählt sind aus >O, >S(O)₀₋₂, >NH und >NC₁₋₆-Alkyl, der 0 oder 1 Doppelbindungen aufweist, der 0 oder 1 Carbonylglieder aufweist;
2) -G-Ar², wobei G eine Bindung, -O- oder -S- ist und Ar² Phenyl ist oder eine monocyclische aromatische Kohlenwasserstoffgruppe ist, die fünf oder sechs Ringatome aufweist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die jeweils gegebenenfalls einfach, zweifach oder dreifach mit R^{p} substituiert ist;
wobei R^{p} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{q})R^{r}, -C(O)N(R^{q})R^{r}, -N(R^{q})C(O)R^{r}, -N(R^{q})SO₂C₁₋₆-Alkyl, -C(O)C₁₋₆-Alkyl, -S(O)₀₋₂-C₁₋₆-Alkyl, -SO₂N(R^{q})R^{r}, -SCF₃, Halo, -CF₃, -OCF₃, -OCHF₂, -COOH und -COOC₁₋₆-Alkyl;
wobei R^{q} und R^{r} jeweils unabhängig ausgewählt sind aus -H, -C₁₋₆-Alkyl und -C₂₋₆-Alkenyl; und
3) einem 4- bis 8-gliedrigen gesättigten oder teilweise gesättigten heterocyclischen Ring, der 1 oder 2 Heteroatomglieder aufweist, die ausgewählt sind aus >O, >S(O)₀₋₂, >NH und >NC₁₋₆-Alkyl, der 0 oder 1 Carbonylglieder aufweist, wobei der Ring gegebenenfalls einfach, zweifach oder dreifach mit R^{p} substituiert ist;
b) Phenyl oder Pyridyl, an zwei benachbarten Kohlenstoffringgliedern an eine dreigliedrige Kohlenwasserstoffeinheit kondensiert, um einen kondensierten fünfgliedrigen aromatischen Ring zu bilden, wobei in der Einheit ein Kohlenstoffatom durch >O, >S, >NH oder >N(C₁₋₄-Alkyl) ersetzt ist und wobei in der Einheit bis zu ein zusätzliches Kohlenstoffatom gegebenenfalls durch -N= ersetzt ist, wobei die kondensierten Ringe gegebenenfalls einfach, zweifach oder dreifach mit R^{t} substituiert sind;
wobei R^{t} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{u})R^{v}, -C(O)N(R^{u})R^{v}, -N(R^{u})C(O)R^{v}, -N(R^{u})SO₂C₁₋₆-Alkyl, -C(O)C₁₋₆-Alkyl, -S(O)₀₋₂-C₁₋₆-Alkyl, -SO₂N(R^{u})R^{v}, -SCF₃, Halo, -CF₃, -OCF₃, -OCHF₂, -COOH und -COOC₁₋₆-Alkyl;
wobei R^{u} und R^{v} jeweils unabhängig -H oder -C₁₋₆-Alkyl sind;
c) Phenyl, an zwei benachbarten Ringgliedern an eine viergliedrige Kohlenwasserstoffeinheit kondensiert, um einen kondensierten sechsgliedrigen aromatischen Ring zu bilden, wobei in der Einheit ein oder zwei Kohlenstoffatome durch -N= ersetzt sind, wobei die kondensierten Ringe gegebenenfalls einfach, zweifach oder dreifach mit R^{t} substituiert sind;
d) Naphthyl, gegebenenfalls einfach, zweifach oder dreifach mit R^{t} substituiert;
e) einer monocyclischen aromatischen Kohlenwasserstoffgruppe, die fünf Ringatome aufweist, die ein Kohlenstoffatom aufweist, das der Bindungspunkt ist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die gegebenenfalls einfach oder zweifach mit R^{j} substituiert ist und gegebenenfalls an zwei benachbarten Kohlenstoffatomen benzokondensiert oder pyridokondensiert ist, wobei die benzokondensierte oder pyridokondensierte Einheit gegebenenfalls einfach, zweifach oder dreifach mit R^{t} substituiert ist; und
f) einer monocyclischen aromatischen Kohlenwasserstoffgruppe, die sechs Ringatome aufweist, die ein Kohlenstoffatom aufweist, das der Bindungspunkt ist, in der ein oder zwei Kohlenstoffatome durch -N= ersetzt sind, die gegebenenfalls einfach oder zweifach mit R^{j} substituiert ist und die gegebenenfalls an zwei benachbarten Kohlenstoffatomen benzokondensiert oder pyridokondensiert ist, wobei die benzokondensierte oder pyridokondensierte Einheit gegebenenfalls einfach oder zweifach mit R^{t} substituiert ist;
und Enantiomere, Diastereomere, Hydrate, Solvate und pharmazeutisch verträgliche Salze, Ester und Amide davon.

13. Verbindung von Formel (I): 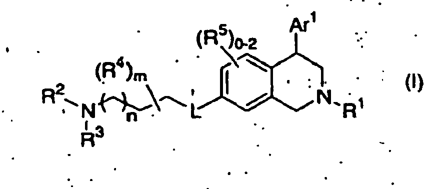 worin
L -O- ist und n 1 oder 2 ist; oder L -C≡C- oder -CH₂CH₂- ist und n 0 oder 1 ist;
R¹ -H ist; oder -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -COOC₁₋₆-Alkyl oder -COOBenzyl ist, von denen jedes gegebenenfalls einfach, zweifach oder dreifach mit R^{a} substituiert ist;
wobei R^{a} ausgewählt ist aus -OH, -OC₁₋₆-Alkyl, Phenyl, gegebenenfalls substituiert mit -OC₁₋₄-Alkyl oder Halo, -CN, -NO₂, -N(k^{b})R^{c}, -C(O)N(R^{b})R^{c}, -N(R^{b})C(O)R^{b}, -N(R^{b})SO₂C₁₋₄-Alkyl, -C(O)C₁₋₆-Alkyl, -S(O)₀₋₂-C₁₋₆-Alkyl, -SO₂N(R^{b})R^{c}, -SCF₃, Halo, -CF₃, -OCF₃, -COOH und -COOC₁₋₆-Alkyl;
wobei R^{b} und R^{c} jeweils unabhängig -H oder -C₁₋₆-Alkyl sind;
R² und R³ jeweils unabhängig ausgewählt sind aus der Gruppe, bestehend aus : -H;
A) -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -CH(C₃₋₈-Cycloalkyl)₂, -CH(Phenyl)₂, Benzyl und -C(O)OC₁₋₄-Alkyl, wobei jedes Alkyl, Cycloalkyl oder Benzyl gegebenenfalls mit -OH, -OC₁₋₄-Alkyl, -CN, -NH₂, -NH(C₁₋₄-Alkyl), -N(C₁₋₄-Alkyl)₂, Halo, -CF₃, -OCF₃, -COOH oder -COOC₁₋₆-Alkyl substituiert ist;
B) Phenyl oder Pyridyl, gegebenenfalls an zwei benachbarten Kohlenstoffringgliedern an eine drei- oder viergliedrige Kohlenwasserstoffeinheit kondensiert, um einen kondensierten fünf- oder sechsgliedrigen aromatischen Ring zu bilden, wobei in der Einheit ein Kohlenstoffatom durch >O, >S, >NH, >N(C₁₋₄-Alkyl) oder >NC(O)OC₁₋₄-Alkyl versetzt ist und wobei in der Einheit bis zu ein zusätzliches Kohlenstoffatom gegebenenfalls durch -N= ersetzt ist;
C) Naphthyl;
D) einem 4- bis 8-gliedrigen heterocyclischen Ring, wobei der heterocyclische Ring ein Kohlenstoffatom aufweist, das der Bindungspunkt ist, 1 oder 2 Heteroatomglieder aufweist, die ausgewählt sind aus der Gruppe, bestehend aus >O, >S(O)₀₋₂, >NH, >N(C₁₋₄-Alkyl) und >NC(O)OC₁₋₄-Alkyl, und 0 oder 1 Doppelbindungen aufweist; und
E) einer monocyclischen aromatischen Kohlenwasserstoffgruppe, die fünf oder sechs Ringatome aufweist, die ein Kohlenstoffatom aufweist, das der Bindungspunkt ist, in der ein Kohlenstoffatom durch >O, >S, >NH, >N(C₁₋₄-Alkyl) oder >NC(O)OC₁₋₄-Alkyl ersetzt ist, in der bis zu ein zusätzliches Kohlenstoffatom gegebenenfalls durch -N= ersetzt ist und die gegebenenfalls benzokondensiert oder pyridokondensiert ist;
wobei B)-E) jeweils gegebenenfalls einfach, zweifach oder dreifach mit einer Einheit substituiert sind, die ausgewählt ist aus der Gruppe, bestehend aus -C₁₋₄-Alkyl, -OH, -C₁₋₄-AlkylOH, -OC₁₋₆-Alkyl, -CN, -NO₂, -N(R^{d})R^{e}, -C(O)N(R^{d})R^{e}, -N(R^{d})C(O)R^{d}, -N(R^{d})SO₂C₁₋₆-Alkyl, -C(O)C₁₋₆-Alkyl, -S(O)₀₋₂-C₁₋₆-Alkyl, -SO₂N(R^{d})R^{e}, -SCF₃, Halo, -CF₃, -OCF₃, -COOH, -COOC₁₋₆-Alkyl, -OC(O)N(R^{d})R^{e} und -OC(O)OC₁₋₆-Alkyl;
wobei R^{d} und R^{e} jeweils unabhängig -H oder -C₁₋₆-Alkyl sind,
oder, alternativ,
R² und R³ mit dem Stickstoff, an das sie gebunden sind, zusammengenommen sein können, um Tetrahydropyrimidin-2-ylidenyl oder einen 4- bis 8-gliedrigen heterocyclischen Ring zu bilden, wobei der heterocyclische Ring 0 oder 1 zusätzliche Heteroatomglieder aufweist, die vom Bindungsstickstoff durch wenigstens ein Kohlenstoffglied getrennt sind und ausgewählt sind aus der Gruppe, bestehend aus >O, >S(O)₀₋₂, >NH und >NR^{f}, 0 oder 1 Doppelbindungen aufweist, 0, 1 oder 2 Kohlenstoffglieder aufweist, die vom Bindungsstickstoff durch wenigstens einen Kohlenstoffglied getrennt sind, das ein Carbonyl ist, gegebenenfalls benzo- oder pyridokondensiert ist, gegebenenfalls ein Kohlenstoffglied aufweist, das eine Brücke bildet, und 0-5 Kohlenstoffgliedsubstituenten R^{ff} aufweist,
wobei R^{f} ausgewählt ist aus der Gruppe, bestehend aus:
i) -C₁₋₆-Alkyl, gegebenenfalls substituiert mit R^{z}, -C₃₋₆-Alkenyl, -C₃₋₆-Alkinyl, -C(O)N(R^{g})R^{h}, -C(O)Rⁱ, -S(O)₀₋₂-C₁₋₆-Alkyl und -COOC₁₋₆-Alkyl,
wobei R^{z} Fluor, -CN, -OH, -OC₁₋₄-Alkyl, -C₃₋₇-Cycloalkyl oder -CF₃ ist;
wobei R^{g} und R^{h} jeweils unabhängig -H oder -C₁₋₆-Alkyl sind; und
wobei Rⁱ -C₁₋₆-Alkyl, -C₃₋₈-Cycloalkyl, Phenyl oder 5-oder 6-gliedriges aromatisches Heterocyclyl ist, wobei jedes Alkyl, Cycloalkyl, Phenyl oder Heterocyclyl gegebenenfalls einfach, zweifach oder dreifach mit -C₁₋₄-Alkyl, -OH, -OC₁₋₆-Alkyl, -CF₃, -CN oder Halo substituiert ist;
ii) -(CH₂)₀₋₁-RingA, wobei Ring A Phenyl oder ein 5- oder 6-gliedriges kohlenstoffverknüpftes aromatisches Heterocyclyl ist, gegebenenfalls substituiert mit R^{aa};
wobei R^{aa} -OH, -C₁₋₆-Alkyl, -OC₁₋₆-Alkyl, Phenyl, -CN, -NO₂, -N(R^{g})R^{h}, -C(O)N(R^{g})R^{h}, -N(R^{g})C(O)R^{h}, -N(R^{h})SO₂C₁₋₆-Alkyl, -C(O)C₁₋₆-Alkyl, -S(O)₀₋₂-C₁₋₆-Alkyl, -SO₂N(R^{g})R^{h}, -SCF₃, Halo, -CF₃, -OCF₃, -OCHF₂, -COOH und -COOC₁₋₆-Alkyl ist; und
iii) -(CH₂)₀₋₂-RingB, wobei Ring B -C₃₋₇-Cycloalkyl ist, in dem ein oder zwei Kohlenstoffringglieder gegebenenfalls durch >O oder >NH ersetzt sind und das gegebenenfalls mit -C₁₋₄-Alkyl, Fluor, -CN, -OH, -OC₁₋₄-Alkyl oder -CF₃ substituiert ist;
wobei R^{ff} ausgewählt ist aus der Gruppe, bestehend aus -C₁₋₆-Alkyl, gegebenenfalls einfach oder zweifach substituiert mit R^{z}, -C₂₋₆-Alkenyl, -C₂₋₆-Alkinyl, -C₃₋₇-Cycloalkyl, -CH(Phenyl)₂, Halo, -OH, -OC₁₋₆-Alkyl, -OC₂₋₃-Alkyl-O-, -CN, -NO₂, -N(R^{g})R^{h}, -C(O)N(R^{g})R^{h}, -N(R^{g})C(O)R^{g}, -N(R^{g})SO₂C₁₋₆-Alkyl, -C(O)R¹, -S(O)₀₋₂-C₁₋₆-Alkyl, -SO₂N(R^{g})R^{h}, -SCF₃, -CF₃, -OCF₃, -COOH und -COOC₁₋₆-Alkyl;
R⁴ -OH, -OC₁₋₆-Alkyl, -CF₃, -C₁₋₆-Alkyl oder Halo ist; zwei R⁴-Substituenten zusammengenommen sein können, um Methylen oder Ethylen zu bilden; oder eines von R⁴ mit R² zusammengenommen ist, um Methylen, Ethylen, Propylen oder -CH₂CH₂O- zu bilden, wobei jedes gegebenenfalls mit -OH, -OC₁₋₆-Alkyl, -SC₁₋₆-Alkyl, -CF₃, -C₁₋₆-Alkyl, Amino oder Halo substituiert ist;
m 0, 1 oder 2 ist;
R⁵ ausgewählt ist aus der Gruppe, bestehend aus -C₁₋₆-Alkyl, -OH, -OC₁₋₆-Alkyl, -SC₁₋₆-Alkyl und Halo;
Ar¹ ein Aryl- oder Heteroarylring ist, der ausgewählt ist aus der Gruppe, bestehend aus:
a) Phenyl, gegebenenfalls einfach, zweifach oder dreifach substituiert mit R^{j} und gegebenenfalls auf benachbarten Kohlenstoffen zweifach substituiert mit -OC₁₋₄-AlkylenO-, gegebenenfalls einfach oder zweifach substituiert mit Fluor, -(CH₂)₂₋₃NH-, -(CH₂)₁₋₂-NH(CH₂)-, -(CH₂)₂-₃N(C₁₋₄-Alkyl)- oder -(CH₂)₁₋₂N(C₁₋₄-Alkyl)(CH₂)-;
wobei R^{j} ausgewählt ist aus der Gruppe, bestehend aus
1) -OH, -C₁₋₆-Alkyl, -OC₁₋₆-Alkyl, gegebenenfalls einfach, zweifach oder dreifach substituiert mit Halo, -C₂₋₆-Alkenyl, -OC₃₋₆-Alkenyl, -C₂₋₆-Alkinyl, gegebenenfalls substituiert mit Trimethylsilyl, -OC₃₋₆-Alkinyl, -C₃₋₆-Cycloalkyl, -OC₃₋₆-Cycloalkyl, -CN, -NO₂, -N(R^{k})R^{l}, -N(R^{k})C(O)R^{l}, -N(R^{k})SO₂C₁₋₆-Alkyl, -C(O)C₁₋₆-Alkyl, -S(O)₀₋₂-C₁₋₆-Alkyl, -C(O)N(R^{m})Rⁿ, -SO₂N(R^{m})Rⁿ, -SCF₃, Halo, -CF₃, -COOH, -COOC₁₋₆-Alkyl und -COOC₃₋₇-Cycloalkyl;
wobei R^{k} und R^{l} jeweils unabhängig -H oder -C₁₋₆-Alkyl sind;
wobei R^{m} und Rⁿ jeweils unabhängig -H oder -C₁₋₆-Alkyl sind oder R^{m} und Rⁿ mit ihrem Bindungsstickstoff zusammengenommen einen 4- bis 8-gliedrigen heterocyclischen Ring bilden, der 1 oder 2 Heteroatomglieder aufweist, die ausgewählt sind aus >O, >S(O)₀₋₂, >NH und >NC₁₋₆-Alkyl, der 0 oder 1 Doppelbindungen aufweist, der 0 oder 1 Carbonylglieder aufweist;
2) -G-Ar², wobei G eine Bindung, -O- oder -S- ist und Ar² Phenyl ist oder eine monocyclische aromatische Kohlenwasserstoffgruppe ist, die fünf oder sechs Ringatome aufweist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die jeweils gegebenenfalls einfach, zweifach oder dreifach mit R^{p} substituiert ist;
wobei R^{p} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{q})R^{r}, -C(O)N(R^{q})R^{r}, -N(R^{q})C(O)R^{r}, -N(R^{q})SO₂C₁₋₆-Alkyl, -C(O)C₁₋₆-Alkyl, -S(O)₀₋₂-C₁₋₆-Alkyl, -SO₂N(R^{q})R^{r}, -SCF₃, Halo, -CF₃, -OCF₃, -OCHF₂, -COOH und -COOC₁₋₆-Alkyl;
wobei R^{q} und R^{r} jeweils unabhängig ausgewählt sind aus -H, -C₁₋₆-Alkyl und -C₂₋₆-Alkenyl; und
3) einem 4- bis 8-gliedrigen gesättigten oder teilweise gesättigten heterocyclischen Ring, der 1 oder 2 Heteroatomglieder aufweist, die ausgewählt sind aus >O, >S(O)₀₋₂, >NH und >NC₁₋₆-Alkyl, der 0 oder 1 Carbonylglieder aufweist, wobei der Ring gegebenenfalls einfach, zweifach oder dreifach mit R^{p} substituiert ist;
b) Phenyl oder Pyridyl, an zwei benachbarten Kohlenstoffringgliedern an eine dreigliedrige Kohlenwasserstoffeinheit kondensiert, um einen kondensierten fünfgliedrigen aromatischen Ring zu bilden, wobei in der Einheit ein Kohlenstoffatom durch >O, >S, >NH oder >N(C₁₋₄-Alkyl) ersetzt ist und wobei in der Einheit bis zu ein zusätzliches Kohlenstoffatom gegebenenfalls durch -N= ersetzt ist, wobei die kondensierten Ringe gegebenenfalls einfach, zweifach oder dreifach mit R^{t} substituiert sind;
wobei R^{t} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{u})R^{v}, -C(O)N(R^{u})R^{v}, -N(R^{u})C(O)R^{v}, -N(R^{u})SO₂C₁₋₆-Alkyl, -C(O)C₁₋₆-Alkyl, -S(O)₀₋₂-C₁₋₆-Alkyl, -SO₂N(R^{u})R^{v}, -SCF₃, Halo, -CF₃, -OCF₃, -OCHF₂, -COOH und -COOC₁₋₆-Alkyl;
wobei R^{u} und R^{v} jeweils unabhängig -H oder -C₁₋₆-Alkyl sind;
c) Phenyl, an zwei benachbarten Ringgliedern an eine viergliedrige Kohlenwasserstoffeinheit kondensiert, um einen kondensierten sechsgliedrigen aromatischen Ring zu bilden, wobei in der Einheit ein oder zwei Kohlenstoffatome durch -N= ersetzt sind, wobei die kondensierten Ringe gegebenenfalls einfach, zweifach oder dreifach mit R^{t} substituiert sind;
d) Naphthyl, gegebenenfalls einfach, zweifach oder dreifach mit R^{t} substituiert;
e) einer monocyclischen aromatischen Kohlenwasserstoffgruppe, die fünf Ringatome aufweist, die ein Kohlenstoffatom aufweist, das der Bindungspunkt ist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die gegebenenfalls einfach oder zweifach mit R^{j} substituiert ist und gegebenenfalls an zwei benachbarten Kohlenstoffatomen benzokondensiert oder pyridokondensiert ist, wobei die benzokondensierte oder pyridokondensierte Einheit gegebenenfalls einfach, zweifach oder dreifach mit R^{t} substituiert ist; und
f) einer monocyclischen aromatischen Kohlenwasserstoffgruppe, die sechs Ringatome aufweist, die ein Kohlenstoffatom aufweist, das der Bindungspunkt ist, in der ein oder zwei Kohlenstoffatome durch -N= ersetzt sind, die gegebenenfalls einfach oder zweifach mit R^{j} substituiert ist und die gegebenenfalls an zwei benachbarten Kohlenstoffatomen benzokondensiert oder pyridokondensiert ist, wobei die benzokondensierte oder pyridokondensierte Einheit gegebenenfalls einfach oder zweifach mit R^{t} substituiert ist;
und Enantiomere, Diastereomere, Hydrate, Solvate und pharmazeutisch verträgliche Salze, Ester und Amide davon;
mit der folgenden Maßgabe:
wenn n 1 ist, L -O- ist, Ar unsubstituiertes Phenyl ist und R² und R³ beide Methyl sind, dann ist R⁴ nicht -OH.

14. Verbindung nach Anspruch 13, wobei L -O- ist und n 1 ist.

15. Verbindung nach Anspruch 13, wobei L -C≡C- ist und n 0 ist.

16. Verbindung nach Anspruch 13, wobei L -CH₂CH₂- ist und n 0 ist.

17. Verbindung nach Anspruch 13, wobei R¹ -C₁₋₄-Alkyl ist.

18. Verbindung nach Anspruch 13, wobei R¹ ausgewählt ist aus der Gruppe, bestehend aus Wasserstoff, Methyl, Ethyl, Propyl, Isopropyl, Butyl, t-Butyl, Pentyl, Hexyl, Benzyl, Vinyl, Allyl, Propargyl, Cyclopropyl, Cyclobutyl, Cyclopentyl, Cyclohexyl, Cyclopropylmethyl, -COOCH₃, -COO-t-Butyl und -COOBenzyl.

19. Verbindung nach Anspruch 13, wobei R¹ Methyl, Ethyl, Propyl, t-Butyl, Allyl, Propargyl oder Benzyl ist.

20. Verbindung nach Anspruch 13, wobei R¹ Wasserstoff oder Methyl ist.

21. Verbindung nach Anspruch 13, wobei R¹ Methyl ist.

22. Verbindung nach Anspruch 13, wobei, wenn R² Methyl ist, R³ nicht Methyl ist.

23. Verbindung nach Anspruch 13, wobei R² und R³ Wasserstoff sind oder, gegebenenfalls substituiert, unabhängig ausgewählt sind aus der Gruppe, bestehend aus:
A) Methyl, Ethyl, Propyl, Isopropyl, sec-Butyl, Butyl, Pentyl, Hexyl, Vinyl, Allyl, Propargyl, Cyclopropyl, Cyclobutyl, Cyclopentyl, Cyclohexyl, Cyclopropylmethyl, Dicyclopropylmethyl, Benzhydryl, Benzyl, -C(O)O-t-Butyl,
B) Phenyl, Pyridyl, 4-, 5-, 6- oder 7-Benzoxazolyl, 4-, 5-, 6- oder 7-Benzothiophenyl, 4-, 5-, 6- oder 7-Benzofuranyl, 4-, 5-, 6- oder 7-Indolyl, 4-, 5-, 6- oder 7-Benzthiazolyl, 4-, 5-, 6- oder 7-Benzimidazolyl, 4-, 5-, 6- oder 7-Indazolyl, Imidazo[1,2-a]pyridin-5-,-6-,-7- oder -8-yl, Pyrazolo[1,5-a]pyridin-4-,-5-,-6- oder -7-yl, 1H-Pyrrolo[2,3-b]pyridin-4-,-5- oder -6-yl, 1H-Pyrrolo[3,2-c]pyridin-4-,-6- oder -7-yl, 1H-Pyrrolo[2,3-c]pyridin-4-,-5- oder -7-yl, 1H-Pyrrolo[3,2-b]pyridin-5-,-6- oder-7-yl,
C) Napththyl,
D) Azetidinyl, Pyrrolidinyl, Piperidinyl, Tetrahydropyranyl und
E) Furanyl, Oxazolyl, Isoxazolyl, 1,2,3-Oxadiazolyl, 1,2,4-Oxadiazolyl, 1,2,5-Oxadiazolyl, 1,3,4-Oxadiazolyl, Thiophenyl, Thiazolyl, Isothiazolyl, Pyrrolyl, Imidazolyl, Pyrazolyl, 1,2,3-Triazolyl, 1,2,4-Triazolyl, 3-Indoxazinyl, 2-Benzoxazolyl, 2- oder 3-Benzothiophenyl, 2- oder 3-Benzofuranyl, 2- oder 3-Indolyl, 2-Benzothiazolyl, 2-Benzimidazolyl und 3-Indazolyl.

24. Verbindung nach Anspruch 13, wobei R² und R³ unabhängig ausgewählt sind aus der Gruppe, bestehend aus Methyl, Ethyl, Propyl, Isopropyl, sec-Butyl, Hydroxyethyl, 2-Hydroxy-2-methylpropyl, Allyl, Cyclopropyl, Cyclobutyl, Cyclopentyl, Dicyclopropylmethyl, Pyrrolidinyl, Piperidinyl, N-Methylpiperidinyl, Tetrahydropyranyl, 2-Benzothiazolyl und Methoxyethyl.

25. Verbindung nach 13, wobei R² und R³ jeweils unabhängig Wasserstoff, Ethyl, Isopropyl, Methoxyethyl, 2-Benzothiazolyl, Cyclopropyl, Cyclobutyl oder Cyclopentyl sind.

26. Verbindung nach Anspruch 13, wobei R² und R³, gegebenenfalls substituiert, mit dem Stickstoff, an das sie gebunden sind, zusammengenommen sind, um Tetrahydropyrimidin-2-ylidenyl oder einen Ring zu bilden, der ausgewählt ist aus der Gruppe, bestehend aus Azetidinyl, Pyrrolidinyl, Piperidinyl, Morpholinyl, Thiomorpholinyl, Piperazinyl, Homopiperidinyl, 1,3-Dihydroisoindol-2-yl, 5,6-Dihydro-4H-pyrimidin-1-yl und 1,1-Dioxo-1λ⁶-thiomorpholin-4-yl.

27. Verbindung nach Anspruch 13, wobei R^{f}, gegebenenfalls substituiert, Methyl, Ethyl, Isopropyl, Allyl, Cyclopropyl, tert-Butoxycarbonyl, Tetrahydrofuranylmethyl, [1,3]-Dioxolanylmethyl, Thiazolyl, Thiophenyl, Thiophenylmethyl, Pyridinyl, Phenyl, Acetyl, Isobutyl, Cyclopropancarbonyl, Cyclobutancarbonyl, Pyridinyl, Pyridincarbonyl, 1H-Pyrrolcarbonyl und 1H-Imidazolcarbonyl ist.

28. Verbindung nach Anspruch 13, wobei R² und R³ mit dem Stickstoff, an das sie gebunden sind, zusammengenommen sind, um einen 4- bis 8-gliedrigen heterocyclischen Ring zu bilden, wobei der heterocyclische Ring ausgewählt ist aus Piperidin, Pyrrolidin und Morpholin, wobei der Ring mit 1 oder 2 Substituenten R^{ff} substituiert ist.

29. Verbindung nach Anspruch 13, wobei R^{ff} ausgewählt ist aus der Gruppe, bestehend aus Methyl, Ethyl, Isopropyl, Butyl, Hexyl, -CHF₂, Benzhydryl, -CF₃, Vinyl, Allyl, Propargyl, Cyclopropyl, Cyclopentyl, Cyclopropylmethyl, Cyclobutylethyl, Brom, Chlor, Fluor, Iod, -OH, Hydroxymethyl, Hydroxyethyl, Methoxy, Ethoxy, Isopropoxy, Pentyloxy, -O(CH₂)₂O-, -O(CH₂)₃O-, -CN, Amino, Methylamino, Dimethylamino, Diethylamino, Diethylcarbamoyl, Methansulfanyl, Methansulfonyl, Methansulfonamido, -C(O)Rⁱ, -COOH und Ethoxycarbonyl.

30. Verbindung nach Anspruch 13, wobei R^{ff} ausgewählt ist aus der Gruppe, bestehend aus Methyl, Fluor, -OH, -CF₃, Hydroxymethyl, Hydroxyethyl, Benzhydryl, Dimethylamino, Ethoxycarbonyl, Cyano und -O(CH₂)₂O-.

31. Verbindung nach Anspruch 13, wobei Rⁱ ausgewählt ist aus der Gruppe, bestehend aus Methyl, Pyridyl, Isopropyl, Cyclobutyl, Cyclopropyl, N-Methylpyrrolyl und 1-Methylimidazolyl.

32. Verbindung nach Anspruch 13, wobei R² und R³ mit dem Stickstoff, an das sie gebunden sind, zusammengenommen sind, um Azetidinyl, 3,3-Difluorazetidinyl, 3-Benzhydrylazetidinyl, 2-Methylpyrrolidinyl, 3-Hydroxypyrrolidinyl, 3-Dimethylaminopyrrolidinyl, 2,5-Dimethylpyrrolidinyl, 2-Trifluormethylpyrrolidinyl, 2-Hydroxymethylpyrrolidinyl, 3,3-Difluorpyrrolidinyl, Piperidinyl, 4-Fluorpiperidinyl, 3-Fluorpiperidinyl, 3,3-Difluorpiperidinyl, 4,4-Difluorpiperidinyl, 3-Trifluormethylpiperidinyl, 4-Trifluormethylpiperidinyl, 1,4-Dioxa-8-azaspiro[4.5]dec-8-yl, 4-Cyanopiperidinyl, 4-Carboethoxypiperidinyl, 3-Hydroxypiperidinyl, 4-Hydroxypiperidinyl, 2-Hydroxymethylpiperidinyl, 3-Hydroxymethylpiperidinyl, 4-Hydroxymethylpiperidinyl, 3-Hydroxyethylpiperidinyl, 4-Hydroxyethylpiperidinyl, Morpholinyl, 2-Methylmorpholin-4-yl, 3-Methylmorpholin-4-yl, 3-Hydroxymethylmorpholin-4-yl, 2-Hydroxymethylmorpholin-4-yl, 4-Methylpiperazin-1-yl, 4-Ethylpiperazin-1-yl, 4-Isopropylpiperazinyl, 4-Allylpiperazin-1-yl, 4-Cyclopropylpiperazin-1-yl, 4-(2-Hydroxyethyl)piperazin-1-yl, 4-(2-Methoxyethyl)-piperazin-1-yl, 4-(tert-Butoxycarbonyl)piperazin-1-yl, 4-(Tetrahydrofuran-2-ylmethyl)piperazin-1-yl, 4-[1,3]-Dioxolan-2-ylmethylpiperazin-1-yl, 4-Thiazol-2-ylpiperazin-1-yl, 4-(2-Thiophenyl)piperazinyl, 4-Thiophen-2-ylmethylpiperazin-1-yl, 4-(Pyridin-4-yl)-piperazin-1-yl, 4-Phenylpiperazin-1-yl, 4-(2-Hydroxyphenyl)piperazinyl, 4-(4-Trifluormethylphenyl)-piperazin-1-yl, 4-(4-Cyanophenyl)piperazin-1-yl, 4-Acetylpiperazin-1-yl, 4-Isobutyrylpiperazin-1-yl, 4-Cyclopropancarbonylpiperazin-1-yl), 4-Cyclobutancarbonylpiperazin-1-yl, 4-Pyridin-4-ylpiperazin-1-yl, 4-(Pyridin-4-carbonyl)-piperazin-1-yl, 4-(1-Methyl-1H-pyrrol-2-carbonyl)-piperazin-1-yl, 4-(1-Methyl-1H-imidazol-4-carbonyl)-piperazin-1-yl, 1,1-Dioxo-1λ⁶-thiomorpholin-4-yl, 2,6-Dimethylmorpholin-4-yl und 1,3-Dihydroisoindol-2-yl, 5,6-Dihydro-4H-pyrimidin-1-yl zu bilden.

33. Verbindung nach Anspruch 13, wobei R² und R³ mit dem Stickstoff, an das sie gebunden sind, zusammengenommen sind, um Piperidinyl, 4-Fluorpiperidinyl, 4,4-Difluorpiperidinyl, Morpholinyl oder 3-Methylmorpholin-4-yl zu bilden.

34. Verbindung nach Anspruch 13, wobei R⁴ Hydroxy, Methoxy, Ethoxy, Isopropoxy, Pentyloxy, -CF₃, Methyl, Ethyl, Propyl, Isobutyl, Pentyl, Chlor oder Fluor ist.

35. Verbindung nach Anspruch 13, wobei R⁴ Hydroxy, Methyl, Methoxy, Fluor oder -CF₃ ist.

36. Verbindung nach Anspruch 13, wobei zwei R⁴ zusammengenommen sind, um Methylen zu bilden.

37. Verbindung nach Anspruch 13, wobei R² und eines von R⁴ zusammengenommen sind, um Methylen, Ethylen oder -CH₂CH₂O- zu bilden.

38. Verbindung nach Anspruch 13, wobei m 0 ist.

39. Verbindung nach Anspruch 13, wobei R⁵ Methyl, Ethyl, Isopropyl, Hexyl, Hydroxy, Methoxy, Ethoxy, Isopropoxy, Methylsulfanyl, Brom, Chlor, Fluor oder Iod ist.

40. Verbindung nach Anspruch 13, wobei R⁵ Methyl, Hydroxy oder Fluor ist.

41. Verbindung nach Anspruch 13, wobei Ar¹ gegebenenfalls substituiert, ausgewählt ist aus der Gruppe, bestehend aus:
a) Phenyl, 5-, 6-, 7-, 8-Benzo-1,4-dioxanyl, 4-, 5-, 6-, 7-Benzo-1,3-dioxolyl, 4-, 5-, 6-, 7-Indolinyl, 4-, 5-, 6-, 7-Isoindolinyl, 1,2,3,4-Tetrahydroisochinolin-4-, -5-, -6- oder -7-yl, 1,2,3,4-Tetrahydroisochinolin-4-, -5-, -6- oder -7-yl,
b) 4-, 5-, 6- oder 7-Benzoxazolyl, 4-, 5-, 6- oder 7-Benzothiophenyl, 4-, 5-, 6- oder 7-Benzofuranyl, 4-, 5-, 6- oder 7-Indolyl, 4-, 5-, 6- oder 7-Benzthiazolyl, 4-, 5-, 6- oder 7-Benzimidazolyl, 4-, 5-, 6- oder 7-Indazolyl, Imidazo[1,2-a]pyridin-5-, -6-, -7- oder -8-yl, Pyrazolo[1,5-a]pyridin-4-, -5-, -6- oder -7-yl, 1H-Pyrrolo[2,3-b]pyridin-4-, -5-oder -6-yl, 1H-Pyrrolo[3,2-c]pyridin-4-, -6- oder -7-yl, 1H-Pyrrolo[2,3-c]pyridin-4-, -5- oder -7-yl, 1H-Pyrrolo[3,2-b]pyridin-5-, -6- oder -7-yl,
c) 5-, 6-, 7- oder 8-Isochinolinyl, 5-, 6-, 7- oder 8-Chinolinyl, 5-, 6-, 7- oder 8-Chinoxalinyl, 5-, 6-, 7- oder 8-Chinazolinyl,
d) Naphthyl,
e) Furanyl, Oxazolyl, Isoxazolyl, 1,2,3-Oxadiazolyl, 1,2,4-Oxadiazolyl, 1,2,5-Oxadiazolyl, 1,3,4-Oxadiazolyl, Thiophenyl, Thiazolyl, Isothiazolyl, Pyrrolyl, Imidazolyl, Pyrazolyl, 1,2,3-Triazolyl, 1,2,4-Triazolyl, 3-Indoxazinyl, 2-Benzoxazolyl, 2- oder 3-Benzothiophenyl, 2- oder 3-Benzofuranyl, 2- oder 3-Indolyl, 2-Benzthiazolyl, 2-Benzimidazolyl, 3-Indazolyl und
f) Pyridinyl, Pyridinyl-N-oxid, Pyrazinyl, Pyrimidinyl, Pyridazinyl, 1-, 3- oder 4-Isochinolinyl, 2-, 3- oder 4-Chinolinyl, 2- oder 3-Chinoxalinyl, 2- oder 4-Chinazolinyl, [1,5]-, [1,6]-, [1,7]- oder [1,8]Naphthyridin-2-, -3- oder -4-yl, [2,5]-, [2,6]-, [2,7]-, [2,8]Naphthyridin-1- , -3- oder -4-yl.

42. Verbindung nach Anspruch 13, wobei Ar¹, gegebenenfalls substituiert, ausgewählt ist aus der Gruppe, bestehend aus Phenyl, Pyridyl, Pyrazinyl, Thiozolyl, Pyrazolyl und Thiophenyl.

43. Verbindung nach Anspruch 13, wobei G eine Bindung oder -S- ist.

44. Verbindung nach Anspruch 13, wobei Ar² ausgewählt ist aus der Gruppe, bestehend aus Phenyl, Pyrrolyl, Furanyl, Thiophenyl, Pyrazolyl, Imidazolyl, Oxazolyl, Thiazolyl, Isoxazolyl, Triazolyl, Tetrazolyl, Pyridinyl, Pyrimidinyl oder Pyrazinyl.

45. Verbindung nach Anspruch 13, wobei Ar¹ ausgewählt ist aus der Gruppe, bestehend aus Phenyl, 2-Methoxyphenyl, 3-Methoxyphenyl, 4-Methoxyphenyl, 4-Ethoxyphenyl, 2,4-Dimethoxyphenyl, 2,5-Dimethoxyphenyl, 3,4-Dimethoxyphenyl, 3,5-Dimethoxyphenyl, 3,4,5-Trimethoxyphenyl, 2-Methylphenyl, 3-Methylphenyl, 4-Methylphenyl, 4-Ethylphenyl, 3-Ethinylphenyl, 4-Ethinylphenyl, 2-Chlorphenyl, 3-Chlorphenyl, 4-Chlorphenyl, 2-Fluorphenyl, 3-Fluorphenyl, 4-Fluorphenyl, 2-Bromphenyl, 3-Bromphenyl, 4-Bromphenyl, 3-Iodphenyl, 4-Iodphenyl, 2-Trifluormethylphenyl, 3-Trifluormethylphenyl, 4-Trifluormethylphenyl, 3-Trifluormethoxyphenyl, 4-Trifluormethoxyphenyl, 4-Difluormethoxyphenyl, 3-Cyanophenyl, 4-Cyanophenyl, 3-Acetylphenyl, 4-Acetylphenyl, 3,4-Difluorphenyl, 3,4-Dichlorphenyl, 2,3-Difluorphenyl, 2,3-Dichlorphenyl, 2,4-Difluorphenyl, 2,4-Dichlorphenyl, 2,5-Dichlorphenyl, 3,5-Dichlorphenyl, 3-Nitrophenyl, 4-Nitrophenyl, 3-Chlor-4-fluorphenyl, 3-Chlor-4-methoxyphenyl, 3-Chlor-4-difluormethoxyphenyl, 3-Fluor-4-chlorphenyl, 2-Fluor-4-methoxyphenyl, 3-Fluor-4-methoxyphenyl, Benzo[1,3]dioxol-4- oder -5-yl, 2-Hydroxyphenyl, 3-Hydroxyphenyl, 4-Hydroxyphenyl, 4-Hydroxy-2-methylphenyl, 4-Hydroxy-3-fluorphenyl, 3,4-Dihydroxyphenyl, 4-Aminophenyl, 4-Dimethylaminophenyl, 4-Morpholin-4-ylphenyl, 4-Carbamoylphenyl, 4-Fluor-3-methylphenyl, 4-Methansulfanylphenyl, 4-Methansulfinylphenyl, 4-Methansulfonylphenyl, 4-Trifluormethansulfanylphenyl, Thiophen-2-yl, Thiophen-3-yl, 2-Pyridinyl, 3-Pyridinyl, 4-Pyridinyl, 2-Chlor-5-pyridinyl, 2-Dimethylamino-5-pyridinyl, 6-Methoxypyridin-3-yl, 6-Methylsulfanylpyridin-3-yl, 2-Hydroxy-5-pyridinyl, 6-Pyrazol-1-ylpyridin-3-yl, 6-Brompyridin-3-yl, 6-Ethinylpyridin-3-yl, 6-Trimethylsilanylethinylpyridin-3-yl, 6-Phenylsulfanylpyridin-3-yl, 6-Imidazol-1-ylpyridin-3-yl, 6-(1H-Imidazol-2-ylsulfanyl)-pyridin-3-yl, 6-(Pyrimidin-2-ylsulfanyl)-pyridin-3-yl, Oxazol-5-yl, Thiazol-5-yl, Thiazol-2-yl, 2H-Pyrazol-3-yl, Pyrazin-2-yl, 1-Naphthyl, 2-Naphthyl, 4-Imidazol-1-ylphenyl, 4-Pyrazol-1-ylphenyl, 1H-Indol-5-yl, 1H-Benzimidazol-5-yl, Benzo[b]thiophen-7-yl und 4-Biphenyl.

46. Verbindung nach Anspruch 13, wobei Ar¹, gegebenenfalls substituiert mit Halo, 4-Methoxyphenyl, 4-Methansulfanylphenyl oder 4-Chlorphenyl ist.

47. Verbindung nach Anspruch 13, wobei das pharmazeutisch verträgliche Salz ein wirksames Aminoadditionssalz ist.

48. Verbindung nach Anspruch 13, wobei das pharmazeutisch verträgliche Salz ausgewählt ist aus der Gruppe, bestehend aus Hydrobromid, Hydrochlorid, Sulfat, Bisulfat, Nitrat, Acetat, Oxalat, Valerat, Oleat, Palmitat, Stearat, Laurat, Borat, Benzoat, Lactat, Phosphat, Tosylat, Citrat, Maleat, Fumarat, Succinat, Tartrat, Naphthylat, Mesylat, Glucoheptonat, Lactiobionat und Laurylsulfonat.

49. Verbindung nach Anspruch 13, die isotopisch markiert ist, um mit PET oder SPECT nachweisbar zu sein.

50. Verfahren zur Untersuchung von Histamin-H3-Rezeptor- und Serotonin-vermittelten Störungen, das den Schritt der Verwendung einer mit ¹⁸F markierten oder mit ¹¹C markierten Verbindung nach Anspruch 13 als eine Positronenemissions-tomographie(PET)-Molekülsonde umfasst.

51. Verbindung von Formel (II): 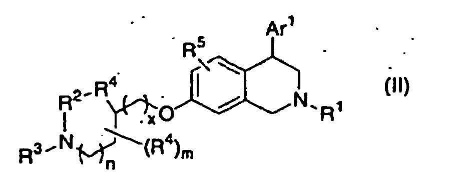 worin
n 1 oder 2 ist;
x 0 oder 1 ist;
wobei n+x 1 oder 2 ist;
R¹ -H ist; oder -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -COOC₁₋₆-Alkyl oder -COOBenzyl ist, von denen jedes gegebenenfalls einfach, zweifach oder dreifach mit R^{a} substituiert ist;
wobei R^{a} ausgewählt ist aus -OH, -OC₁₋₆-Alkyl, Phenyl, gegebenenfalls substituiert mit -OC₁₋₄-Alkyl oder Halo, -CN, -NO₂, -N(k^{b})R^{c}, -C(O)N(R^{b})R^{c}, -N(R^{b})C(O)R^{b}, -N(R^{b})SO₂C₁₋₆-Alkyl, -C(O)C₁₋₆-Alkyl, -S(O)₀₋₂-C₁₋₆-Alkyl, -SO₂N(R^{b})R^{c}, -SCF₃, Halo, -CF₃, -OCF₃, -COOH und -COOC₁₋₆-Alkyl;
wobei R^{b} und R^{c} jeweils unabhängig -H oder -C₁₋₆-Alkyl sind;
R³ ausgewählt ist aus der Gruppe, bestehend aus: -H;
A) -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -CH(C₃₋₈-Cycloalkyl)₂, -CH(Phenyl)₂, Benzyl und -C(O)OC₁₋₄-Alkyl, wobei jedes Alkyl, Cycloalkyl oder Benzyl gegebenenfalls mit -OH, -OC₁₋₄-Alkyl, -CN, -NH₂, -NH(C₁₋₄-Alkyl), -N(C₁₋₄-Alkyl)₂, Halo, -CF₃, -OCF₃, -COOH oder -COOC₁₋₆-Alkyl substituiert ist;
B) Phenyl oder Pyridyl, gegebenenfalls an zwei benachbarten Kohlenstoffringgliedern an eine drei- oder viergliedrige Kohlenwasserstoffeinheit kondensiert, um einen kondensierten fünf- oder sechsgliedrigen aromatischen Ring zu bilden, wobei in der Einheit ein Kohlenstoffatom durch >O, >S, >NH, >N(C₁₋₄-Alkyl) oder >NC(O)OC₁₋₄-Alkyl ersetzt ist und wobei in der Einheit bis zu ein zusätzliches Kohlenstoffatom gegebenenfalls durch -N= ersetzt ist;
C) Naphthyl;
D) einem 4- bis 8-gliedrigen heterocyclischen Ring, wobei der heterocyclische Ring ein Kohlenstoffatom aufweist, das der Bindungspunkt ist, 1 oder 2 Heteroatomglieder aufweist, die ausgewählt sind aus der Gruppe, bestehend aus >O, >S(O)₀₋₂, >NH, >N(C₁₋₄-Alkyl) und >NC(O)OC₁₋₄-Alkyl, und 0 oder 1 Doppelbindungen aufweist; und
E) einer monocyclischen aromatischen Kohlenwasserstoffgruppe, die fünf oder sechs Ringatome aufweist, die ein Kohlenstoffatom aufweist, das der Bindungspunkt ist, in der ein Kohlenstoffatom durch >O, >S, >NH, >N(C₁₋₄-Alkyl) oder >NC(O)OC₁₋₄-Alkyl ersetzt ist, in der bis zu ein zusätzliches Kohlenstoffatom gegebenenfalls durch -N= ersetzt ist und die gegebenenfalls benzokondensiert oder pyridokondensiert ist;
wobei B)-E) jeweils gegebenenfalls einfach, zweifach oder dreifach mit einer Einheit substituiert sind, die ausgewählt ist aus der Gruppe, bestehend aus -C₁₋₄-Alkyl, -OH, -C₁₋₄-AlkylOH, -OC₁₋₆-Alkyl, -CN, -NO₂, -N(R^{d})R^{e}, -C(O)N(R^{d})R^{e}, -N(R^{d})C(O)R^{d}, -N(R^{d})SO₂C₁₋₆-Alkyl, -C(O)C₁₋₆-Alkyl, -S(O)₀₋₂-C₁₋₆-Alkyl, -SO₂N(R^{d})R^{e}, -SCF₃, Halo, -CF₃, -OCF₃, -COOH, -COOC₁₋₆-Alkyl, -OC(O)N(R^{d})R^{e} und -OC(O)OC₁₋₆-Alkyl;
wobei R^{d} und R^{e} jeweils unabhängig -H oder -C₁₋₆-Alkyl sind;
-R²-R⁴- Methylen, Ethylen, Propylen oder -CH₂CH₂O- ist, wobei jedes gegebenenfalls mit -OH, -OC₁₋₆-Alkyl, -SC₁₋₆-Alkyl, -CF₃, -C₁₋₆-Alkyl, Amino oder Halo substituiert ist;
R⁴ -OH, -OC₁₋₆-Alkyl, -CF₃, -C₁₋₆-Alkyl oder Halo ist;
m 0, 1 oder 2 ist;
R⁵ ausgewählt ist aus der Gruppe, bestehend aus -C₁₋₆-Alkyl, -OH, -OC₁₋₆-Alkyl, -SC₁₋₆-Alkyl und Halo;
Ar¹ ein Aryl- oder Heteroarylring ist, der ausgewählt ist aus der Gruppe, bestehend aus:
a) Phenyl, gegebenenfalls einfach, zweifach oder dreifach substituiert mit R^{j} und gegebenenfalls auf benachbarten Kohlenstoffen zweifach substituiert mit -OC₁₋₄-AlkylenO-, gegebenenfalls einfach oder zweifach substituiert mit Fluor, -(CH₂)₂₋₃NH-, -(CH₂)₁₋₂NH(CH₂)-, -(CH₂)₂₋₃N(C₁₋₄-Alkyl)- oder -(CH₂)₁₋₂N(C₁₋₄-Alkyl)(CH₂)-;
wobei R^{j} ausgewählt ist aus der Gruppe, bestehend aus
1) -OH, -C₁₋₆-Alkyl, -OC₁₋₆-Alkyl, gegebenenfalls einfach, zweifach oder dreifach substituiert mit Halo, -C₂₋₆-Alkenyl, -OC₃₋₆-Alkenyl, -C₂₋₆-Alkinyl, gegebenenfalls substituiert mit Trimethylsilyl, -OC₃₋₆-Alkinyl, -C₃₋₆-Cycloalkyl, -OC₃₋₆-Cycloalkyl, -CN, -NO₂, -N(R^{k})R^{l}, -N(R^{k})C(O)R^{l}, -N(R^{k})SO₂C₁₋₆-Alkyl, -C(O)C₁₋₆-Alkyl, -S(O)₀₋₂-C₁₋₆-Alkyl, -C(O)N(R^{m})Rⁿ, -SO₂N(R^{m})Rⁿ, -SCF₃, Halo, -CF₃, -COOH, -COOC₁₋₆-Alkyl und -COOC₃₋₇-Cycloalkyl;
wobei R^{k} und R^{l} jeweils unabhängig -H oder -C₁₋₆-Alkyl sind;
wobei R^{m} und Rⁿ jeweils unabhängig -H oder -C₁₋₆-Alkyl sind oder R^{m} und Rⁿ mit ihrem Bindungsstickstoff zusammengenommen einen 4- bis 8-gliedrigen heterocyclischen Ring bilden, der 1 oder 2 Heteroatomglieder aufweist, die ausgewählt sind aus >O, >S(O)₀₋₂, >NH und >NC₁₋₆-Alkyl, der 0 oder 1 Doppelbindungen aufweist, der 0 oder 1 Carbonylglieder aufweist;
2) -G-Ar², wobei G eine Bindung, -O- oder -S- ist und Ar² Phenyl ist oder eine monocyclische aromatische Kohlenwasserstoffgruppe ist, die fünf oder sechs Ringatome aufweist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die jeweils gegebenenfalls einfach, zweifach oder dreifach mit R^{p} substituiert ist;
wobei R^{p} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{q})R^{r}, -C(O)N(R^{q})R^{r}, -N(R^{q})C(O)R^{r}, -N(R^{q})SO₂C₁₋₆-Alkyl, -C(O)C₁₋₆-Alkyl, -S(O)₀₋₂-C₁₋₆-Alkyl, -SO₂N(R^{q})R^{r}, -SCF₃, Halo, -CF₃, -OCF₃, -OCHF₂, -COOH und -COOC₁₋₆-Alkyl;
wobei R^{q} und R^{r} jeweils unabhängig ausgewählt sind aus -H, -C₁₋₆-Alkyl und -C₂₋₆-Alkenyl; und
3) einem 4- bis 8-gliedrigen gesättigten oder teilweise gesättigten heterocyclischen Ring, der 1 oder 2 Heteroatomglieder aufweist, die ausgewählt sind aus >O, >S(O)₀₋₂, >NH und >NC₁₋₆-Alkyl, der 0 oder 1 Carbonylglieder aufweist, wobei der Ring gegebenenfalls einfach, zweifach oder dreifach mit R^{p} substituiert ist;
b) Phenyl oder Pyridyl, an zwei benachbarten Kohlenstoffringgliedern an eine dreigliedrige Kohlenwasserstoffeinheit kondensiert, um einen kondensierten fünfgliedrigen aromatischen Ring zu bilden, wobei in der Einheit ein Kohlenstoffatom durch >O, >S, >NH oder >N(C₁₋₄-Alkyl) ersetzt ist und wobei in der Einheit bis zu ein zusätzliches Kohlenstoffatom gegebenenfalls durch -N= ersetzt ist, wobei die kondensierten Ringe gegebenenfalls einfach, zweifach oder dreifach mit R^{t} substituiert sind;
wobei R^{t} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{u})R^{v}, -C(O)N(R^{u})R^{v}, -N(R^{u})C(O)R^{v}, -N(R^{u})SO₂C₁₋₆-Alkyl, -C(O)C₁₋₆-Alkyl, -S(O)₀₋₂-C₁₋₆-Alkyl, -SO₂N(R^{u})R^{v}, -SCF₃, Halo, -CF₃, -OCF₃, -OCHF₂, -COOH und -COOC₁₋₆-Alkyl;
wobei R^{u} und R^{v} jeweils unabhängig -H oder -C₁₋₆-Alkyl sind;
c) Phenyl, an zwei benachbarten Ringgliedern an eine viergliedrige Kohlenwasserstoffeinheit kondensiert, um einen kondensierten sechsgliedrigen aromatischen Ring zu bilden, wobei in der Einheit ein oder zwei Kohlenstoffatome durch -N= ersetzt sind, wobei die kondensierten Ringe gegebenenfalls einfach, zweifach oder dreifach mit R^{t} substituiert sind;
d) Naphthyl, gegebenenfalls einfach, zweifach oder dreifach mit R^{t} substituiert;
e) einer monocyclischen aromatischen Kohlenwasserstoffgruppe, die fünf Ringatome aufweist, die ein Kohlenstoffatom aufweist, das der Bindungspunkt ist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die gegebenenfalls einfach oder zweifach mit R^{j} substituiert ist und gegebenenfalls an zwei benachbarten Kohlenstoffatomen benzokondensiert oder pyridokondensiert ist, wobei die benzokondensierte oder pyridokondensierte Einheit gegebenenfalls einfach, zweifach oder dreifach mit R^{t} substituiert ist; und
f) einer monocyclischen aromatischen Kohlenwasserstoffgruppe, die sechs Ringatome aufweist, die ein Kohlenstoffatom aufweist, das der Bindungspunkt ist, in der ein oder zwei Kohlenstoffatome durch -N= ersetzt sind, die gegebenenfalls einfach oder zweifach mit R^{j} substituiert ist und die gegebenenfalls an zwei benachbarten Kohlenstoffatomen benzokondensiert oder pyridokondensiert ist, wobei die benzokondensierte oder pyridokondensierte Einheit gegebenenfalls einfach oder zweifach mit R^{t} substituiert ist;
und Enantiomere, Diastereomere, Hydrate, Solvate und pharmazeutisch verträgliche Salze, Ester und Amide davon.

52. Verbindung nach Anspruch 51, wobei n 1 ist und x 0 ist.

53. Verbindung nach Anspruch 51, wobei n 1 ist und x 1 ist.

54. Verbindung nach Anspruch 51, wobei n 2 ist und x 0 ist.

55. Verbindung nach Anspruch 51, wobei R¹ Methyl ist.

56. Verbindung nach Anspruch 51, wobei R³ Wasserstoff, Ethyl, Isopropyl, Methoxyethyl, 2-Benzothiazolyl, Cyclopropyl, Cyclobutyl oder Cyclopentyl ist.

57. Verbindung nach Anspruch 51, wobei R⁴ Methyl, Hydroxy, Methoxy, -CF₃, Methyl, Fluor oder Chlor ist.

58. Verbindung nach Anspruch 51, wobei m 0 ist.

59. Verbindung nach Anspruch 51, wobei R⁵ Methyl, Hydroxy, Methoxy, Methylsulfanyl, Fluor oder Chlor ist.

60. Verbindung nach Anspruch 51, wobei Ar¹, gegebenenfalls substituiert mit Halo, 4-Methoxyphenyl, 4-Methansulfanylphenyl oder 4-Chlorphenyl ist.

61. Verbindung nach Anspruch 51, wobei -R²-R⁴- Methylen ist.

62. Verbindung nach Anspruch 51, wobei -R²-R⁴- Ethylen ist.

63. Verbindung nach Anspruch 51, wobei -R²-R⁴- -CH₂CH₂O- ist.

64. Verbindung, ausgewählt aus der Gruppe, bestehend aus:
7-[3-(4-Fluorpiperidin-1-yl)-propoxy]-4-(4-methoxyphenyl)-2-methyl-1,2,3,4-tetrahydroisochinolin;
7-[3-(4-Fluorpiperidin-1-yl)-propoxy]-4-(4-methoxyphenyl)-2-methyl-1,2,3,4-tetrahydroisochinolin (Enantiomer 1);
7-[3-(4-Fluorpiperidin-1-yl)-propoxy]-4-(4-methoxyphenyl)-2-methyl-1,2,3,4-tetrahydroisochinolin (Enantiomer 2);
1-(4-{3-[4-(4-Methoxyphenyl)-2-methyl-1,2,3,4-tetrahydroisochinolin-7-yloxy]-propyl}-piperazin-1-yl)-ethanon;
Diethyl-{3-[4-(4-methoxyphenyl)-2-methyl-1,2,3,4-tetrahydroisochinolin-7-yloxy]-propyl}-amin;
(4-{3-[4-(4-Methoxyphenyl)-2-methyl-1,2,3,4-tetrahydroisochinolin-7-yloxy]-propyl}-piperazin-1-yl)-pyridin-4-ylmethanon;
1-(4-{3-[4-(4-Methoxyphenyl)-2-methyl-1,2,3,4-tetrahydroisochinolin-7-yloxy]-propyl}-piperazin-1-yl)-2-methylpropan-1-on;
Cyclobutyl-(4-{3-[4-(4-methoxyphenyl)-2-methyl-1,2,3,4-tetrahydroisochinolin-7-yloxy]-propyl}-piperazin-1-yl)-methanon;
Cyclopropyl-(4-{3-[4-(4-methoxyphenyl)-2-methyl-1,2,3,4-tetrahydroisochinolin-7-yloxy]-propyl}-piperazin-1-yl)-methanon;
7-[3-(4,4-Difluorpiperidin-1-yl)-propoxy]-4-(4-methoxyphenyl)-2-methyl-1,2,3,4-tetrahydroisochinolin;
4-(4-Methoxyphenyl)-2-methyl-7-(3-morpholin-4-ylpropoxy)-1,2,3,4-tetrahydroisochinolin;
(1-{3-[4-(4-Methoxyphenyl)-2-methyl-1,2,3,4-tetrahydroisochinolin-7-yloxy]-propyl}-pyrrolidin-3-yl)-dimethylamin;
7-[3-((2R,5R)-trans-Dimethylpyrrolidin-1-yl)-propoxy]-4-(4-methoxyphenyl)-2-methyl-1,2,3,4-tetrahydroisochinolin;
4-{3-[4-(4-Methoxyphenyl)-2-methyl-1,2,3,4-tetrahydroisochinolin-7-yloxy]-propyl}-piperazin-1-carbonsäureethylester;
(4-{3-[4-(4-Methoxyphenyl)-2-methyl-1,2,3,4-tetrahydroisochinolin-7-yloxy]-propyl}-piperazin-1-yl)-(1-methyl-1H-pyrrol-2-yl)-methanon;
(4-{3-[4-(4-Methoxyphenyl)-2-methyl-1,2,3,4-tetrahydroisochinolin-7-yloxy]-propyl}-piperazin-1-yl)-(1-methyl-1H-imidazol-4-yl)-methanon;
(1,3-Dimethyltetrahydropyrimidin-2-yliden)-{3-[4-(4-methoxyphenyl)-2-methyl-1,2,3,4-tetrahydroisochinolin-7-yloxy]-propyl}-amin;
7-[3-(1,3-Dihydroisoindol-2-yl)-propoxy]-4-(4-methoxyphenyl)-2-methyl-1,2,3,4-tetrahydroisochinolin;
Bis-(2-methoxyethyl)-{3-[4-(4-methoxyphenyl)-2-methyl-1,2,3,4-tetrahydroisochinolin-7-yloxy]-propyl}-amin;
7-[3-(5,6-Dihydro-4H-pyrimidin-1-yl)-propoxy]-4-(4-methoxyphenyl)-2-methyl-1,2,3,4-tetrahydroisochinolin;
Benzothiazol-2-yl-{3-[4-(4-methoxyphenyl)-2-methyl-1,2,3,4-tetrahydroisochinolin-7-yloxy]-propyl}-methylamin;
1-{3-[4-(4-Methoxyphenyl)-2-methyl-1,2,3,4-tetrahydroisochinolin-7-yloxy]-propyl}-piperidin-3-ol;
1-{3-[4-(4-Methoxyphenyl)-2-methyl-1,2,3,4-tetrahydroisochinolin-7-yloxy]-propyl}-piperidin-4-ol;
(1-{3-[4-(4-Methoxyphenyl)-2-methyl-1,2,3,4-tetrahydroisochinolin-7-yloxy]-propyl}-piperidin-4-yl)-methanol;
(1-{3-[4-(4-Methoxyphenyl)-2-methyl-1,2,3,4-tetrahydroisochinolin-7-yloxy]-propyl}-piperidin-3-yl)-methanol;
(1-{3-[4-(4-Methoxyphenyl)-2-methyl-1,2,3,4-tetrahydroisochinolin-7-yloxy]-propyl}-piperidin-2-yl)-methanol;
4-(4-Methoxyphenyl)-2-methyl-7-[3-(3-trifluormethylpiperidin-1-yl)-propoxy]-1,2,3,4-tetrahydroisochinolin;
2-(1-{3-[4-(4-Methoxyphenyl)-2-methyl-1,2,3,4-tetrahydroisochinolin-7-yloxy]-propyl}-piperidin-4-yl)-ethanol;
7-[3-(1,1-Dioxo-1λ⁶-thiomorpholin-4-yl)-propoxy]-4-(4-methoxyphenyl)-2-methyl-1,2,3,4-tetrahydroisochinolin;
4-(4-Methoxyphenyl)-2-methyl-7-[3-((2S)-trifluormethylpyrrolidin-1-yl)-propoxy]-1,2,3,4-tetrahydroisochinolin;
7-[3-(3,3-Difluorpiperidin-1-yl)-propoxy]-4-(4-methoxyphenyl)-2-methyl-1,2,3,4-tetrahydroisochinolin;
7-[3-(3,3-Difluorpiperidin-1-yl)-propoxy]-4-(4-methoxyphenyl)-2-methyl-1,2,3,4-tetrahydroisochinolin (Enantiomer 1);
7-[3-(3,3-Difluorpiperidin-1-yl)-propoxy]-4-(4-methoxyphenyl)-2-methyl-1,2,3,4-tetrahydroisochinolin (Enantiomer 2);
(1-{3-[4-(4-Methoxyphenyl)-2-methyl-1,2,3,4-tetrahydroisochinolin-7-yloxy]-propyl}-pyrrolidin-(2R)-yl)-methanol;
1-{3-[4-(4-Methoxyphenyl)-2-methyl-1,2,3,4-tetrahydroisochinolin-7-yloxy]-propyl}-(R)-pyrrolidin-3-ol;
7-[3-(2,6-Dimethylmorpholin-4-yl)-propoxy]-4-(4-methoxyphenyl)-2-methyl-1,2,3,4-tetrahydroisochinolin;
1-{3-[4-(4-Methoxyphenyl)-2-methyl-1,2,3,4-tetrahydroisochinolin-7-yloxy]-propyl}-piperidin-4-carbonsäureethylester;
7-(3-Azetidin-1-ylpropoxy)-4-(4-methoxyphenyl)-2-methyl-1,2,3,4-tetrahydroisochinolin;
4-(4-Methoxyphenyl)-2-methyl-7-[3-(2-methylpyrrolidin-1-yl)-propoxy]-1,2,3,4-tetrahydroisochinolin;
2-(1-{3-[4-(4-Methoxyphenyl)-2-methyl-1,2,3,4-tetrahydroisochinolin-7-yloxy]-propyl}-piperidin-2-yl)-ethanol;
1-{3-[4-(4-Methoxyphenyl)-2-methyl-1,2,3,4-tetrahydroisochinolin-7-yloxy]-propyl}-piperidin-4-carbonitril;
1-{3-[4-(4-Methoxyphenyl)-2-methyl-1,2,3,4-tetrahydroisochinolin-7-yloxy]-propyl}-piperidin-4-carbonitril (Enantiomer 1);
1-{3-[4-(4-Methoxyphenyl)-2-methyl-1,2,3,4-tetrahydroisochinolin-7-yloxy]-propyl}-piperidin-4-carbonitril (Enantiomer 2);
4-(4-Methoxyphenyl)-2-methyl-7-[3-(4-trifluormethylpiperidin-1-yl)-propoxy]-1,2,3,4-tetrahydroisochinolin;
7-[3-(3-Fluorpiperidin-1-yl)-propoxy]-4-(4-methoxyphenyl)-2-methyl-1,2,3,4-tetrahydroisochinolin;
Ethyl-(2-methoxyethyl)-{3-[4-(4-methoxyphenyl)-2-methyl-1,2,3,4-tetrahydroisochinolin-7-yloxy]-propyl -amin;
7-[3-(1,4-Dioxa-8-azaspiro[4.5]dec-8-yl)-propoxy]-4-(4-methoxyphenyl)-2-methyl-1,2,3,4-tetrahydroisochinolin;
1-[4-(4-Methoxyphenyl)-2-methyl-1,2,3,4-tetrahydroisochinolin-7-yloxy]-3-piperidin-1-yl-propan-2-ol;
7-[2-Fluor-3-(4-fluorpiperidin-1-yl)-propoxy]-4-(4-methoxyphenyl)-2-methyl-1,2,3,4-tetrahydroisochinolin;
7-[3-(4-Fluorpiperidin-1-yl)-propoxy]-4-(3-methoxyphenyl)-2-methyl-1,2,3,4-tetrahydroisochinolin;
7-[3-(4-Fluorpiperidin-1-yl)-propoxy]-4-(3-methoxyphenyl)-2-methyl-1,2,3,4-tetrahydroisochinolin (Enantiomer 1);
7-[3-(4-Fluorpiperidin-1-yl)-propoxy]-4-(3-methoxyphenyl)-2-methyl-1,2,3,4-tetrahydroisochinolin (Enantiomer 2);
4-(3-Chlorphenyl)-7-[3-(4-fluorpiperidin-1-yl)-propoxy]-2-methyl-1,2,3,4-tetrahydroisochinolin;
4-(4-Chlorphenyl)-7-[3-(4-fluorpiperidin-1-yl)-propoxy]-2-methyl-1,2,3,4-tetrahydroisochinolin;
4-(3-Fluorphenyl)-7-[3-(4-fluorpiperidin-1-yl)-propoxy]-2-methyl-1,2,3,4-tetrahydroisochinolin;
7-[3-(4-Fluorpiperidin-1-yl)-propoxy]-2-methyl-4-(4-trifluormethoxyphenyl)-1,2,3,4-tetrahydroisochinolin;
4-(4-Difluormethoxyphenyl)-7-[3-(4-fluorpiperidin-1-yl)-propoxy]-2-methyl-1,2,3,4-tetrahydroisochinolin;
7-[3-(4-Fluorpiperidin-1-yl)-propoxy]-4-(4-methansulfonylphenyl)-2-methyl-1,2,3,4-tetrahydroisochinolin;
7-[3-(4-Fluorpiperidin-1-yl)-propoxy]-2-methyl-4-(4-methylsulfanylphenyl)-1,2,3,4-tetrahydroisochinolin;
4-(3-Chlor-4-methoxyphenyl)-7-[3-(4-fluorpiperidin-1-yl)-propoxy]-2-methyl-1,2,3,4-tetrahydroisochinolin;
4-(2,4-Dichlorphenyl)-7-[3-(4-fluorpiperidin-1-yl)-propoxy]-2-methyl-1,2,3,4-tetrahydroisochinolin;
4-(2,5-Dichlorphenyl)-7-[3-(4-fluorpiperidin-1-yl)-propoxy]-2-methyl-1,2,3,4-tetrahydroisochinolin;
4-(3,5-Dichlorphenyl)-7-[3-(4-fluorpiperidin-1-yl)-propoxy]-2-methyl-1,2,3,4-tetrahydroisochinolin;
2-Methyl-7-(3-piperidin-1-ylpropoxy)-4-thiophen-3-yl-1,2,3,4-tetrahydroisochinolin;
4-(3,4-Dichlorphenyl)-7-[3-(4-fluorpiperidin-1-yl)-propoxy]-2-methyl-1,2,3,4-tetrahydroisochinolin;
2-Methyl-7-(3-piperidin-1-ylpropoxy)-4-pyridin-3-yl-1,2,3,4-tetrahydroisochinolin;
2-Methyl-7-(3-piperidin-1-ylpropoxy)-4-(trifluormethylphenyl)-1,2,3,4-tetrahydroisochinolin;
4-(4-Methoxyphenyl)-2-methyl-7-(3-piperidin-1-ylpropoxy)-1,2,3,4-tetrahydroisochinolin (razemisch);
4-(4-Methoxyphenyl)-2-methyl-7-(3-piperidin-1-ylpropoxy)-1,2,3,4-tetrahydroisochinolin (Enantiomer 1);
4-(4-Methoxyphenyl)-2-methyl-7-(3-piperidin-1-ylpropoxy)-1,2,3,4-tetrahydroisochinolin (Enantiomer 2);
2-tert-Butyl-4-(4-methoxyphenyl)-7-(3-piperidin-1-ylpropoxy)-1,2,3,4-tetrahydroisochinolin;
2-Benzyl-4-(4-methoxyphenyl)-7-(3-piperidin-1-ylpropoxy)-1,2,3,4-tetrahydroisochinolin;
2-Ethyl-4-(4-methoxyphenyl)-7-(3-piperidin-1-ylpropoxy)-1,2,3,4-tetrahydroisochinolin;
4-(4-Methoxyphenyl)-7-(3-piperidin-1-ylpropoxy)-2-propyl-1,2,3,4-tetrahydroisochinolin;
2-Isopropyl-4-(4-methoxyphenyl)-7-(3-piperidin-1-ylpropoxy)-1,2,3,4-tetrahydroisochinolin;
4-(4-Methoxyphenyl)-7-(3-piperidin-1-ylpropoxy)-1,2,3,4-tetrahydroisochinolin;
3-[4-(4-Methoxyphenyl)-7-(3-piperidin-1-ylpropoxy)-3,4-dihydro-1H-isochinolin-2-yl]-propan-1-ol;
2-[4-(4-Methoxyphenyl)-7-(3-piperidin-1-ylpropoxy)-3,4-dihydro-1H-isochinolin-2-yl]-ethanol;
2-(2-Fluorethyl)-4-(4-methoxyphenyl)-7-(3-piperidin-1-ylpropoxy)-1,2,3,4-tetrahydroisochinolin;
2-Cyclopropyl-4-(4-methoxyphenyl)-7-(3-piperidin-1-ylpropoxy)-1,2,3,4-tetrahydroisochinolin;
4-(4-Methoxyphenyl)-7-(3-morpholin-4-ylpropoxy)-2-(1-phenylethyl)-1,2,3,4-tetrahydroisochinolin;
4-(4-Methoxyphenyl)-7-(3-morpholin-4-ylpropoxy)-2-(1-phenylethyl)-1,2,3,4-tetrahydroisochinolin (Diastereomer 1);
4-(4-Methoxyphenyl)-7-(3-morpholin-4-ylpropoxy)-2-(1-phenylethyl)-1,2,3,4-tetrahydroisochinolin (Diastereomer 2);
4-(3,4-Dichlorphenyl)-2-methyl-7-(3-piperidin-1-ylpropoxy)-1,2,3,4-tetrahydroisochinolin;
4-(3,4-Dichlorphenyl)-2-methyl-7-(3-morpholin-4-ylpropoxy)-1,2,3,4-tetrahydroisochinolin;
4-(4-Methoxyphenyl)-2-methyl-7-(piperidin-4-ylmethoxy)-1,2,3,4-tetrahydroisochinolin;
4-(3-Chlor-4-methoxyphenyl)-2-methyl-7-(piperidin-4-ylmethoxy)-1,2,3,4-tetrahydroisochinolin;
2-Methyl-4-(4-methylsulfanylphenyl)-7-(piperidin-4-ylmethoxy)-1,2,3,4-tetrahydroisochinolin;
4-(3-Fluor-4-methoxyphenyl)-2-methyl-7-(piperidin-4-ylmethoxy)-1,2,3,4-tetrahydroisochinolin;
4-(2-Fluor-4-methoxyphenyl)-2-methyl-7-(piperidin-4-ylmethoxy)-1,2,3,4-tetrahydroisochinolin;
7-(1-Isopropylpiperidin-4-ylmethoxy)-4-(4-methoxyphenyl)-2-methyl-1,2,3,4-tetrahydroisochinolin;
4-(4-Methoxyphenyl)-2-methyl-7-(1-methylpiperidin-4-ylmethoxy)-1,2,3,4-tetrahydroisochinolin;
4-(4-Methoxyphenyl)-2-methyl-7-(1-propylpiperidin-4-ylmethoxy)-1,2,3,4-tetrahydroisochinolin;
7-(1-Cyclopentylpiperidin-4-ylmethoxy)-4-(4-methoxyphenyl)-2-methyl-1,2,3,4-tetrahydroisochinolin;
7-(1-Ethylpiperidin-4-ylmethoxy)-4-(4-methoxyphenyl)-2-methyl-1,2,3,4-tetrahydroisochinolin;
4-(3-Chlor-4-methoxyphenyl)-7-(1-isopropylpiperidin-4-ylmethoxy)-2-methyl-1,2,3,4-tetrahydroisochinolin;
4-(3-Fluor-4-methoxyphenyl)-7-(1-isopropylpiperidin-4-ylmethoxy)-2-methyl-1,2,3,4-tetrahydroisochinolin;
4-(2-Fluor-4-methoxyphenyl)-7-(1-isopropylpiperidin-4-ylmethoxy)-2-methyl-1,2,3,4-tetrahydroisochinolin;
7-(1-Isopropylpiperidin-4-ylmethoxy)-4-(3-methoxyphenyl)-2-methyl-1,2,3,4-tetrahydroisochinolin;
4-(3-Methoxyphenyl)-2-methyl-7-(1-methylpiperidin-4-ylmethoxy)-1,2,3,4-tetrahydroisochinolin;
4-(3-Methoxyphenyl)-2-methyl-7-(1-propylpiperidin-4-ylmethoxy)-1,2,3,4-tetrahydroisochinolin;
7-(1-Cyclopentylpiperidin-4-ylmethoxy)-4-(3-methoxyphenyl)-2-methyl-1,2,3,4-tetrahydroisochinolin;
7-(1-Ethylpiperidin-4-ylmethoxy)-4-(3-methoxyphenyl)-2-methyl-1,2,3,4-tetrahydroisochinolin;
4-(3-Methoxyphenyl)-2-methyl-7-(piperidin-4-yloxy)-1,2,3,4-tetrahydroisochinolin;
4-(3-Methoxyphenyl)-2-methyl-7-(1-methylpiperidin-4-yloxy)-1,2,3,4-tetrahydroisochinolin;
7-(1-Isopropylpiperidin-4-yloxy)-4-(3-methoxyphenyl)-2-methyl-1,2,3,4-tetrahydroisochinolin;
7-(1-Cyclobutylpiperidin-4-yloxy)-4-(3-methoxyphenyl)-2-methyl-1,2,3,4-tetrahydroisochinolin;
7-(1-Ethylpiperidin-4-yloxy)-4-(3-methoxyphenyl)-2-methyl-1,2,3,4-tetrahydroisochinolin;
7-(1-Cyclopentylpiperidin-4-yloxy)-4-(3-methoxyphenyl)-2-methyl-1,2,3,4-tetrahydroisochinolin;
4-(3-Methoxyphenyl)-2-methyl-7-(1-propylpiperidin-4-yloxy)-1,2,3,4-tetrahydroisochinolin;
7-(1-Isopropylpiperidin-4-ylmethoxy)-2-methyl-4-(4-methylsulfanylphenyl)-1,2,3,4-tetrahydroisochinolin;
7-(1-Cyclobutylpiperidin-4-ylmethoxy)-2-methyl-4-(4-methylsulfanylphenyl)-1,2,3,4-tetrahydroisochinolin;
7-(1-Ethylpiperidin-4-ylmethoxy)-2-methyl-4-(4-methylsulfanylphenyl)-1,2,3,4-tetrahydroisochinolin;
4-(3-Chlor-4-methoxyphenyl)-7-(1-cyclobutylpiperidin-4-ylmethoxy)-2-methyl-1,2,3,4-tetrahydroisochinolin;
4-(3-Chlor-4-methoxyphenyl)-7-(1-ethylpiperidin-4-ylmethoxy)-2-methyl-1,2,3,4-tetrahydroisochinolin;
4-(3-Chlor-4-methoxyphenyl)-2-methyl-7-(1-propylpiperidin-4-ylmethoxy)-1,2,3,4-tetrahydroisochinolin;
2-Methyl-4-(4-methylsulfanylphenyl)-7-(1-propylpiperidin-4-ylmethoxy)-1,2,3,4-tetrahydroisochinolin;
7-(1-Isobutylpiperidin]-4-ylmethoxy)-4-(4-methoxyphenyl)-2-methyl-1,2,3,4-tetrahydroisochinolin;
7-(1-Cyclobutylpiperidin-4-ylmethoxy)-4-(3-fluor-4-methoxyphenyl)-2-methyl-1,2,3,4-tetrahydroisochinolin;
4-(3-Fluor-4-methoxyphenyl)-2-methyl-7-(1-propylpiperidin-4-ylmethoxy)-1,2,3,4-tetrahydroisochinolin;
7-(1-Cyclobutylpiperidin-4-ylmethoxy)-4-(2-fluor-4-methoxyphenyl)-2-methyl-1,2,3,4-tetrahydroisochinolin;
4-(2-Fluor-4-methoxyphenyl)-2-methyl-7-(1-propylpiperidin-4-ylmethoxy)-1,2,3,4-tetrahydroisochinolin;
4-(2-Fluor-4-methoxyphenyl)-7-(1-isobutylpiperidin-4-ylmethoxy)-2-methyl-1,2,3,4-tetrahydroisochinolin;
7-[1-(2-Fluorethyl)-piperidin-4-ylmethoxy]-4-(4-methoxyphenyl)-2-methyl-1,2,3,4-tetrahydroisochinolin;
7-(1-Isopropylpiperidin-4-yloxy)-4-(4-methoxyphenyl)-2-methyl-1,2,3,4-tetrahydroisochinolin;
7-(1-Isopropylpiperidin-4-yloxy)-2-methyl-4-(4-methylsulfanylphenyl)-1,2,3,4-tetrahydroisochinolin;
4-(2-Fluor-4-methoxyphenyl)-7-(1-isopropylpiperidin-4-yloxy)-2-methyl-1,2,3,4-tetrahydroisochinolin;
4-(3-Fluor-4-methoxyphenyl)-7-(1-isopropylpiperidin-4-yloxy)-2-methyl-1,2,3,4-tetrahydroisochinolin;
4-(4-Methoxyphenyl)-2-methyl-7-[1-(tetrahydropyran-4-yl)-piperidin-4-yloxy]-1,2,3,4-tetrahydroisochinolin;
2,2,2-Trifluor-1-{4-[4-(4-methoxyphenyl)-2-methyl-1,2,3,4-tetrahydroisochinolin-7-yloxymethyl]-piperidin-1-yl}-ethanon;
4-(4-Methoxyphenyl)-2-methyl-7-[1-(2,2,2-trifluorethyl)-piperidin-4-ylmethoxy]-1,2,3,4-tetrahydroisochinolin;
7-[3-(4-Fluorpiperidin-1-yl)-propoxy]-2-methyl-4-phenyl-1,2,3,4-tetrahydroisochinolin;
4-(2-Fluorphenyl)-7-[3-(4-fluorpiperidin-1-yl)-propoxy]-2-methyl-1,2,3,4-tetrahydroisochinolin;
7-[3-(4-Fluorpiperidin-1-yl)-propoxy]-2-methyl-4-p-tolyl-1,2,3,4-tetrahydroisochinolin;
2-Benzyl-7-[3-(4-fluorpiperidin-1-yl)-propoxy]-4-(4-methoxyphenyl)-1,2,3,4-tetrahydroisochinolin;
7-[3-(4-Fluorpiperidin-1-yl)-propoxy]-2-methyl-4-(3-trifluormethoxyphenyl)-1,2,3,4-tetrahydroisochinolin;
7-[3-(3,3-Difluorpyrrolidin-1-yl)-propoxy]-4-(4-methoxyphenyl)-2-methyl-1,2,3,4-tetrahydroisochinolin;
4-(4-Methoxyphenyl)-2-methyl-7-[3-(3S-methylmorpholin-4-yl)-propoxy]-1,2,3,4-tetrahydroisochinolin;
Dicyclopropylmethyl-{3-[4-(4-methoxyphenyl)-2-methyl-1,2,3,4-tetrahydroisochinolin-7-yloxy]-propyl}-amin;
4-(2-Chlorphenyl)-7-[3-(4-fluorpiperidin-1-yl)-propoxy]-2-methyl-1,2,3,4-tetrahydroisochinolin;
2-Methyl-7-[3-(3 S-methylmorpholin-4-yl)-propoxy]-4-(4-morpholin-4-ylphenyl)-1,2,3,4-tetrahydroisochinolin;
7-[3-(4-Fluorpiperidin-1-yl)-propoxy]-2-methyl-4-(4-morpholin-4-ylphenyl)-1,2,3,4-tetrahydroisochinolin;
4-{2-Methyl-7-[3-(3S-methylmorpholin-4-yl)-propoxy]-1,2,3,4-tetrahydroisochinolin-4-yl}-benzonitril;
4-{7-[3-(4-Fluorpiperidin-1-yl)-propoxy]-2-methyl-1,2,3,4-tetrahydroisochinolin-4-yl}-benzonitril;
7-[3-(3-Benzhydrylazetidin-1-yl)-propoxy]-4-(4-methoxyphenyl)-2-methyl-1,2,3,4-tetrahydroisochinolin;
2-Methyl-4-(4-methylsulfanylphenyl)-7-(3-morpholin-4-ylpropoxy)-1,2,3,4-tetrahydroisochinolin;
4-(2-Fluor-4-methoxyphenyl)-7-[3-(4-fluorpiperidin-1-yl)-propoxy]-2-methyl-1,2,3,4-tetrahydroisochinolin (Razemat);
4-(2-Fluor-4-methoxyphenyl)-7-[3-(4-fluorpiperidin-1-yl)-propoxy]-2-methyl-1,2,3,4-tetrahydroisochinolin (Enantiomer 1);
4-(2-Fluor-4-methoxyphenyl)-7-[3-(4-fluorpiperidin-1-yl)-propoxy]-2-methyl-1,2,3,4-tetrahydroisochinolin (Enantiomer 2);
4-(3-Fluor-4-methoxyphenyl)-7-[3-(4-fluorpiperidin-1-yl)-propoxy]-2-methyl-1,2,3,4-tetrahydroisochinolin (Razemat);
4-(3-Fluor-4-methoxyphenyl)-7-[3-(4-fluorpiperidin-1-yl)-propoxy]-2-methyl-1,2,3,4-tetrahydroisochinolin (Enantiomer 1);
4-(3-Fluor-4-methoxyphenyl)-7-[3-(4-fluorpiperidin-1-yl)-propoxy]-2-methyl-1,2,3,4-tetrahydroisochinolin (Enantiomer 2);
4-(4-Ethoxyphenyl)-7-[3-(4-fluorpiperidin-1-yl)-propoxy]-2-methyl-1,2,3,4-tetrahydroisochinolin;
2-Ethyl-7-[3-(4-fluorpiperidin-1-yl)-propoxy]-4-(4-methoxyphenyl)-1,2,3,4-tetrahydroisochinolin;
7-[3-(4-Fluorpiperidin-1-yl)-propoxy]-4-(4-methoxyphenyl)-1,2,3,4-tetrahydroisochinolin;
4-(4-Methoxyphenyl)-2-methyl-7-(piperidin-4-yloxy)-1,2,3,4-tetrahydroisochinolin;
2-Methyl-4-(4-methylsulfanylphenyl)-7-(piperidin-4-yloxy)-1,2,3,4-tetrahydroisochinolin;
4-(2-Fluor-4-methoxyphenyl)-2-methyl-7-(piperidin-4-yloxy)-1,2,3,4-tetrahydroisochinolin;
4-(3-Fluor-4-methoxyphenyl)-2-methyl-7-(piperidin-4-yloxy)-1,2,3,4-tetrahydroisochinolin;
7-[1-(2-Fluorethyl)-piperidin-4-yloxy]-4-(4-methoxyphenyl)-2-methyl-1,2,3,4-tetrahydroisochinolin;
7-[3-(4-Fluorpiperidin-1-yl)-propoxy]-2-methyl-4-(4-methylsulfanylphenyl)-1,2,3,4-tetrahydroisochinolin (Enantiomer 1);
7-[3-(4-Fluorpiperidin-1-yl)-propoxy]-2-methyl-4-(4-methylsulfanylphenyl)-1,2,3,4-tetrahydroisochinolin (Enantiomer 2);
4-(4-Methoxyphenyl)-2-methyl-7-[3-(3S-methylmorpholin-4-yl)-propoxy]-1,2,3,4-tetrahydroisochinolin (Enantiomer 1);
4-(4-Methoxyphenyl)-2-methyl-7-[3-(3 S-methylmorpholin-4-yl)-propoxy]-1,2,3,4-tetrahydroisochinolin (Enantiomer 2);
2-Methyl-4-(4-methylsulfanylphenyl)-7-(3-morpholin-4-ylpropoxy)-1,2,3,4-tetrahydroisochinolin (Enantiomer 1);
2-Methyl-4-(4-methylsulfanylphenyl)-7-(3-morpholin-4-ylpropoxy)-1,2,3,4-tetrahydroisochinolin (Enantiomer 2);
7-[3-(4-Fluorpiperidin-1-yl)-propoxy]-4-(4-methylsulfanylphenyl)-1,2,3,4-tetrahydroisochinolin;
4-(4-Methylsulfanylphenyl)-7-(3-morpholin-4-ylpropoxy)-1,2,3,4-tetrahydroisochinolin;
7-[3-(3S-Methylmorpholin-4-yl)-propoxy]-4-(4-methylsulfanylphenyl)-1,2,3,4-tetrahydroisochinolin;
4-(4-Methoxyphenyl)-7-(3-morpholin-4-ylpropoxy)-1,2,3,4-tetrahydroisochinolin;
7-[3-(4-Fluorpiperidin-1 -yl)-propoxy]-4-(4-methansulfinylphenyl)-2-methyl-1,2,3,4-tetrahydroisochinolin;
4-(4-Methansulfinylphenyl)-2-methyl-7-(3-morpholin-4-ylpropoxy)-1,2,3,4-tetrahydroisochinolin;
4-(4-Methansulfinylphenyl)-2-methyl-7-(3-morpholin-4-ylpropoxy)-1,2,3,4-tetrahydroisochinolin (Enantiomer 1);
4-(4-Methansulfonylphenyl)-2-methyl-7-(3-morpholin-4-ylpropoxy)-1,2,3,4-tetrahydroisochinolin;
7-[3-(4-Fluorpiperidin-1-yl)-propoxy]-4-(4-methoxyphenyl)-2,6-dimethyl-1,2,3,4-tetrahydroisochinolin;
7-[3-(4-Fluorpiperidin-1-yl)-propoxy]-4-(4-methoxyphenyl)-2,8-dimethyl-1,2,3,4-tetrahydroisochinolin;
7-[3-(4-Fluorpiperidin-1-yl)-propoxy]-4-(4-methoxyphenyl)-2-methyl-1,2,3,4-tetrahydroisochinolin-6-ol;
7-[3-(4-Fluorpiperidin-1-yl)-propoxy]-4-(4-methoxyphenyl)-2-methyl-1,2,3,4-tetrahydroisochinolin-8-ol;
2,8-Dimethyl-4-(4-methylsulfanylphenyl)-7-(3-morpholin-4-ylpropoxy)-1,2,3,4-tetrahydroisochinolin;
2,6-Dimethyl-4-(4-methylsulfanylphenyl)-7-(3-morpholin-4-ylpropoxy)-1,2,3,4-tetrahydroisochinolin;
2-Methyl-4-(4-methylsulfanylphenyl)-7-(3-morpholin-4-ylpropoxy)-1,2,3,4-tetrahydroisochinolin-8-ol;
2-Methyl-4-(4-methylsulfanylphenyl)-7-(3-morpholin-4-ylpropoxy)-1,2,3,4-tetrahydroisochinolin-6-ol;
8-Fluor-2-methyl-4-(4-methylsulfanylphenyl)-7-(3-morpholin-4-ylpropoxy)-1,2,3,4-tetrahydroisochinolin;
6-Fluor-2-methyl-4-(4-methylsulfanylphenyl)-7-(3-morpholin-4-ylpropoxy)-1,2,3,4-tetrahydroisochinolin;
7-[1-(2-Fluorethyl)-piperidin-4-ylmethoxy]-2-methyl-4-(4-methylsulfanylphenyl)-1,2,3,4-tetrahydroisochinolin;
7-[1-(2-Fluorethyl)-piperidin-4-yloxy]-2-methyl-4-(4-methylsulfanylphenyl)-1,2,3,4-tetrahydroisochinolin;
4-(4-Methoxyphenyl)-7-[3-(3 S-methylmorpholin-4-yl)-propoxy]-1,2,3,4-tetrahydroisochinolin;
4-(4-Methansulfinylphenyl)-2-methyl-7-[3-(3 S-methylmorpholin-4-yl)-propoxy]-1,2,3,4-tetrahydroisochinolin;
7-[3-(3,3-Difluorazetidin-1-yl)-propoxy]-4-(4-methoxyphenyl)-2-methyl-1,2,3,4-tetrahydroisochinolin;
(4-{3-[4-(4-Methoxyphenyl)-2-methyl-1,2,3,4-tetrahydroisochinolin-7-yloxy]-propyl}-morpholin-3-yl)-methanol;
(4-{3-[4-(4-Methoxyphenyl)-2-methyl-1,2,3,4-tetrahydroisochinolin-7-yloxy]-propyl}-morpholin-3S-yl)-methanol;
(4-{3-[4-(4-Methoxyphenyl)-2-methyl-1,2,3,4-tetrahydroisochinolin-7-yloxy]-propyl}-morpholin-2-yl)-methanol;
{3-[2-Methyl-4-(4-methylsulfanylphenyl)-1,2,3,4-tetrahydroisochinolin-7-yloxy]-propyl}-(2H-pyrazol-3-yl)-amin;
4-(6-Brompyridin-3-yl)-7-[3-(4-fluorpiperidin-1-yl)-propoxy]-2-methyl-1,2,3,4-tetrahydroisochinolin;
7-[3-(4-Fluorpiperidin-1-yl)-propoxy]-2-methyl-4-(6-methylsulfanylpyridin-3-yl)-1,2,3,4-tetrahydroisochinolin;
5-{7-[3-(4-Fluorpiperidin-1-yl)-propoxy]-2-methyl-1,2,3,4-tetrahydroisochinolin-4-yl}-pyridin-2-yl)-dimethylamin;
7-[3-(4-Fluorpiperidin-1-yl)-propoxy]-2-methyl-4-(6-trimethylsilanylethinylpyridin-3-yl)-1,2,3,4-tetrahydroisochinolin;
4-(6-Ethinylpyridin-3-yl)-7-[3-(4-fluorpiperidin-1-yl)-propoxy]-2-methyl-1,2,3,4-tetrahydroisochinolin;
7-[3-(4-Fluorpiperidin-1-yl)-propoxy]-2-methyl-4-(6-phenylsulfanylpyridin-3-yl)-1,2,3,4-tetrahydroisochinolin;
7-[3-(4-Fluorpiperidin-1-yl)-propoxy]-4-(6-imidazol-1-ylpyridin-3-yl)-2-methyl-1,2,3,4-tetrahydroisochinolin;
7-[3-(4-Fluorpiperidin-1-yl)-propoxy]-4-(6-methoxypyridin-3-yl)-2-methyl-1,2,3,4-tetrahydroisochinolin;
7-[3-(4-Fluorpiperidin-1-yl)-propoxy]-2-methyl-4-(6-pyrazol-1-ylpyridin-3-yl)-1,2,3,4-tetrahydroisochinolin;
7-[3-(4-Fluorpiperidin-1-yl)-propoxy]-4-[6-(1H-imidazol-2-ylsulfanyl)-pyridin-3-yl]-2-methyl-1,2,3,4-tetrahydroisochinolin;
7-[3-(4-Fluorpiperidin-1-yl)-propoxy]-2-methyl-4-[6-(pyrimidin-2-ylsulfanyl)-pyridin-3-yl]-1,2,3,4-tetrahydroisochinolin;
4-(4-Methoxyphenyl)-2-methyl-7-[3-(4-methylpiperazin-1-yl)-propoxy]-1,2,3,4-tetrahydroisochinolin;
7-[3-(4-Ethylpiperazin-1-yl)-propoxy]-4-(4-methoxyphenyl)-2-methyl-1,2,3,4-tetrahydroisochinolin;
7-[3-(4-Isopropylpiperazin-1-yl)-propoxy]-4-(4-methoxyphenyl)-2-methyl-1,2,3,4-tetrahydroisochinolin;
4-(4-Methoxyphenyl)-2-methyl-7-[3-(4-thiazol-2-ylpiperazin-1-yl)-propoxy]-1,2,3,4-tetrahydroisochinolin;
4-(4-Methoxyphenyl)-2-methyl-7-[3-(4-thiophen-2-ylmethylpiperazin-1-yl)-propoxy]-1,2,3,4-tetrahydroisochinolin;
2-(4-{3-[4-(4-Methoxyphenyl)-2-methyl-1,2,3,4-tetrahydroisochinolin-7-yloxy]-propyl}-piperazin-1-yl)-ethanol;
2-(4-{3-[4-(4-Methoxyphenyl)-2-methyl-1,2,3,4-tetrahydroisochinolin-7-yloxy]-propyl}-piperazin-1-yl)-phenol;
4-(4-Methoxyphenyl)-2-methyl-7-[3-(4-pyridin-4-ylpiperazin-1-yl)-propoxy]-1,2,3,4-tetrahydroisochinolin;
4-(4-Methoxyphenyl)-2-methyl-7-{3-[4-(4-trifluormethylphenyl)-piperazin-1-yl]-propoxy}-1,2,3,4-tetrahydroisochinolin;
7-[3-(4-Allylpiperazin-1-yl)-propoxy]-4-(4-methoxyphenyl)-2-methyl-1,2,3,4-tetrahydroisochinolin;
7-[3-(4-[1,3]-Dioxolan-2-ylmethylpiperazin-1-yl)-propoxy]-4-(4-methoxyphenyl)-2-methyl-1,2,3,4-tetrahydroisochinolin;
4-(4-{3-[4-(4-Methoxyphenyl)-2-methyl-1,2,3,4-tetrahydroisochinolin-7-yloxy]-propyl}-piperazin-1-yl)-benzonitril;
7-{3-[4-(2-Methoxyethyl)-piperazin-1-yl]-propoxy}-4-(4-methoxyphenyl)-2-methyl-1,2,3,4-tetrahydroisochinolin;
4-(4-Methoxyphenyl)-2-methyl-7-{3-4-(tetrahydrofuran-2-ylmethyl)-piperazin-1-yl]-propoxy}-1,2,3,4-tetrahydroisochinolin;
4-{3-[4-(4-Methoxyphenyl)-2-methyl-1,2,3,4-tetrahydroisochinolin-7-yloxy]-propyl}-piperazin-1-carbonsäure-tert-butylester;
7-[3-(4-Cyclopropylpiperazin-1-yl)-propoxy]-4-(4-methoxyphenyl)-2-methyl-1,2,3,4-tetrahydroisochinolin;
4-(4-Bromphenyl)-7-[3-(4-fluorpiperidin-1-yl)-propoxy]-2-methyl-1,2,3,4-tetrahydroisochinolin;
4-(4-Difluormethoxyphenyl)-2-methyl-7-(3-morpholin-4-ylpropoxy)-1,2,3,4-tetrahydroisochinolin;
2-Methyl-7-[3-(3S-methylmorpholin-4-yl)-propoxy]-4-(4-methylsulfanylphenyl)-1,2,3,4-tetrahydroisochinolin;
7-[3-(4,4-Difluorpiperidin-1-yl)-propoxy]-2-methyl-4-(4-methylsulfanylphenyl)-1,2,3,4-tetrahydroisochinolin;
7-[3-(4-Fluorpiperidin-1-yl)-propoxy]-2-methyl-4-(4-trifluormethylsulfanylphenyl)-1,2,3,4-tetrahydroisochinolin;
2-Methyl-7-(3-morpholin-4-ylpropoxy)-4-(4-trifluormethylsulfanylphenyl)-1,2,3,4-tetrahydroisochinolin;
2-Methyl-7-[3-(3S-methylmorpholin-4-yl)-propoxy]-4-(4-trifluormethylsulfanylphenyl)-1,2,3,4-tetrahydroisochinolin;
4-(2-Fluor-4-methoxyphenyl)-2-methyl-7-[3-(3S-methylmorpholin-4-yl)-propoxy]-1,2,3,4-tetrahydroisochinolin;
4-(3-Fluor-4-methoxyphenyl)-2-methyl-7-[3-(3 S-methylmorpholin-4-yl)-propoxy]-1,2,3,4-tetrahydroisochinolin;
2-Methyl-7-(3-piperidin-1-ylpropoxy)-4-(4-trifluormethoxyphenyl)-1,2,3,4-tetrahydroisochinolin;
3-[4-(4-Methoxyphenyl)-2-methyl-1,2,3,4-tetrahydroisochinolin-7-yloxy]-propyl}-(tetrahydropyran-4-yl)-amin;
Cyclopropyl-{3-[4-(4-methoxyphenyl)-2-methyl-1,2,3,4-tetrahydroisochinolin-7-yloxy]-propyl}-(1-methylpiperidin-4-yl)-amin;
(2-Methoxyethyl)-{3-[4-(4-methoxyphenyl)-2-methyl-1,2,3,4-tetrahydroisochinolin-7-yloxy]-propyl}-amin;
3-{3-[4-(4-Methoxyphenyl)-2-methyl-1,2,3,4-tetrahydroisochinolin-7-yloxy]-propylamino}-propan-1-ol;
Allyl-{3-[4-(4-methoxyphenyl)-2-methyl-1,2,3,4-tetrahydroisochinolin-7-yloxy]-propyl}-amin;
Isobutyl-{3-[4-(4-methoxyphenyl)-2-methyl-1,2,3,4-tetrahydroisochinolin-7-yloxy]-propyl}-amin;
Cyclopropyl-{3-[4-(4-methoxyphenyl)-2-methyl-1,2,3,4-tetrahydroisochinolin-7-yloxy]-propyl}-amin;
Isopropyl-{3-[4-(4-methoxyphenyl)-2-methyl-1,2,3,4-tetrahydroisochinolin-7-yloxy]-propyl}-amin;
3-[4-(4-Methoxyphenyl)-2-methyl-1,2,3,4-tetrahydroisochinolin-7-yloxy]-propyl}-propylamin;
Ethyl-{3-[4-(4-methoxyphenyl)-2-methyl-1,2,3,4-tetrahydroisochinolin-7-yloxy]-propyl}-amin;
Isopropyl-{3-[4-(4-methoxyphenyl)-2-methyl-1,2,3,4-tetrahydroisochinolin-7-yloxy]-propyl}-methylamin;
Cyclopropyl-{3-[4-(4-methoxyphenyl)-2-methyl-1,2,3,4-tetrahydroisochinolin-7-yloxy]-propyl}-methylamin;
Dicyclopropyl-{3-[4-(4-methoxyphenyl)-2-methyl-1,2,3,4-tetrahydroisochinolin-7-yloxy]-propyl}-amin;
7-(1-Isopropylazetidin-3-ylmethoxy)-2-methyl-4-(4-methylsulfanylphenyl)-1,2,3,4-tetrahydroisochinolin;
7-(1-Cyclopropylazetidin-3-ylmethoxy)-2-methyl-4-(4-methylsulfanylphenyl)-1,2,3,4-tetrahydroisochinolin;
7-(1-Cyclobutylazetidin-3-ylmethoxy)-2-methyl-4-(4-methylsulfanylphenyl)-1,2,3,4-tetrahydroisochinolin;
7-[1-(2-Fluorethyl)-azetidin-3-ylmethoxy]-2-methyl-4-(4-methylsulfanylphenyl)-1,2,3,4-tetrahydroisochinolin;
4-(4-Methoxyphenyl)-2-methyl-7-[2-(1-methylpyrrolidin-2-yl)-ethoxy]-1,2,3,4-tetrahydroisochinolin;
4-(4-Methoxyphenyl)-2-methyl-7-[2-(1-methylpyrrolidin-2-yl)-ethoxy]-1,2,3,4-tetrahydroisochinolin (Diastereomer 1);
4-(4-Methoxyphenyl)-2-methyl-7-[2-(1-methylpyrrolidin-2-yl)-ethoxy]-1,2,3,4-tetrahydroisochinolin (Diastereomer 2);
4-(3-Methoxyphenyl)-2-methyl-7-(piperidin-4-ylmethoxy)-1,2,3,4-tetrahydroisochinolin;
{4-[4-(4-Methoxyphenyl)-2-methyl-1,2,3,4-tetrahydroisochinolin-7-yloxymethyl]-piperidin-1-yl}-acetonitril;
2-Methyl-7-(1-methylpiperidin-4-yloxy)-4-(4-methylsulfanylphenyl)-1,2,3,4-tetrahydroisochinolin;
2-Methyl-4-(4-methylsulfanylphenyl)-7-[1-(3,3,3-trifluorpropyl)-piperidin-4-yloxy]-1,2,3,4-tetrahydroisochinolin;
{4-[2-Methyl-4-(4-methylsulfanylphenyl)-1,2,3,4-tetrahydroisochinolin-7-yloxy]-piperidin-1-yl}-acetonitril;
2-Methyl-4-(4-methylsulfanylphenyl)-7-[1-(tetrahydropyran-4-yl)-piperidin-4-yloxy]-1,2,3,4-tetrahydroisochinolin;
7-(1-Cyclopropylpiperidin-4-yloxy)-2-methyl-4-(4-methylsulfanylphenyl)-1,2,3,4-tetrahydroisochinolin;
7-(1-Cyclobutylpiperidin-4-ylmethoxy)-4-(4-methoxyphenyl)-2-methyl-1,2,3,4-tetrahydroisochinolin;
7-(1-Cyclopropylpiperidin-4-ylmethoxy)-4-(4-methoxyphenyl)-2-methyl-1,2,3,4-tetrahydroisochinolin;
1-{4-[4-(4-Methoxyphenyl)-2-methyl-1,2,3,4-tetrahydroisochinolin-7-yloxymethyl]-piperidin-1-yl}-2-methylpropan-2-ol;
4-(4-Methoxyphenyl)-2-methyl-7-(4-methylmorpholin-2-ylmethoxy)-1,2,3,4-tetrahydroisochinolin;
4-(4-Methoxyphenyl)-2-methyl-7-(morpholin-2S-ylmethoxy)-1,2,3,4-tetrahydroisochinolin;
4-(4-Methoxyphenyl)-2-methyl-7-(morpholin-2R-ylmethoxy)-1,2,3,4-tetrahydroisochinolin;
7-(4-Isopropylmorpholin-2-ylmethoxy)-4-(4-methoxyphenyl)-2-methyl-1,2,3,4-tetrahydroisochinolin;
7-(4-Isopropylmorpholin-2S-ylmethoxy)-4-(4-methoxyphenyl)-2-methyl-1,2,3,4-tetrahydroisochinolin;
7-(4-Isopropylmorpholin-2R-ylmethoxy)-4-(4-methoxyphenyl)-2-methyl-1,2,3,4-tetrahydroisochinolin;
7-(4-Isopropylmorpholin-2R-ylmethoxy)-4-(4-methoxyphenyl)-2-methyl-1,2,3,4-tetrahydroisochinolin (Diastereomer 1);
7-(4-Isopropylmorpholin-2R-ylmethoxy)-4-(4-methoxyphenyl)-2-methyl-1,2,3,4-tetrahydroisochinolin (Diastereomer 2);
7-(4-Ethylmorpholin-2-ylmethoxy)-4-(4-methoxyphenyl)-2-methyl-1,2,3,4-tetrahydroisochinolin;
7-[4-(2-Fluorethyl)-morpholin-2-ylmethoxy]-4-(4-methoxyphenyl)-2-methyl-1,2,3,4-tetrahydroisochinolin;
7-(4-Cyclopropylmorpholin-2-ylmethoxy)-4-(4-methoxyphenyl)-2-methyl-1,2,3,4-tetrahydroisochinolin;
7-(4-Cyclopropylmorpholin-2S-ylmethoxy)-4-(4-methoxyphenyl)-2-methyl-1,2,3,4-tetrahydroisochinolin;
7-(4-Cyclopropylmorpholin-2S-ylmethoxy)-4-(4-methoxyphenyl)-2-methyl-1,2,3,4-tetrahydroisochinolin (Diastereomer 1);
7-(4-Cyclopropylmorpholin-2S-ylmethoxy)-4-(4-methoxyphenyl)-2-methyl-1,2,3,4-tetrahydroisochinolin (Diastereomer 2);
7-(4-Cyclopropylmorpholin-2R-ylmethoxy)-4-(4-methoxyphenyl)-2-methyl-1,2,3,4-tetrahydroisochinolin;
7-(4-Cyclopropylmorpholin-2R-ylmethoxy)-4-(4-methoxyphenyl)-2-methyl-1,2,3,4-tetrahydroisochinolin (Diastereomer 1);
7-(4-Cyclopropylmorpholin-2R-ylmethoxy)-4-(4-methoxyphenyl)-2-methyl-1,2,3,4-tetrahydroisochinolin (Diastereomer 2);
7-(4-Isopropylmorpholin-2-ylmethoxy)-2-methyl-4-(4-methylsulfanylphenyl)-1,2,3,4-tetrahydroisochinolin;
7-(4-Cyclopropylmorpholin-2-ylmethoxy)-2-methyl-4-(4-methylsulfanylphenyl)-1,2,3,4-tetrahydroisochinolin;
2-Methyl-4-(4-methylsulfanylphenyl)-7-(morpholin-2-ylmethoxy)-1,2,3,4-tetrahydroisochinolin;
4-(4-Methoxyphenyl)-2,6-dimethyl-7-(3-piperidin-1-ylpropoxy)-1,2,3,4-tetrahydroisochinolin;
4-(4-Methoxyphenyl)-2,6-dimethyl-7-(3-morpholin-4-ylpropoxy)-1,2,3,4-tetrahydroisochinolin;
4-(3-Fluor-4-methoxyphenyl)-2,6-dimethyl-7-(3-morpholin-4-ylpropoxy)-1,2,3,4-tetrahydroisochinolin;
4-(3-Fluor-4-methoxyphenyl)-2,8-dimethyl-7-(3-morpholin-4-ylpropoxy)-1,2,3,4-tetrahydroisochinolin;
4-(4-Methoxyphenyl)-2,8-dimethyl-7-(3-morpholin-4-ylpropoxy)-1,2,3,4-tetrahydroisochinolin;
2,6-Dimethyl-4-(4-methylsulfanylphenyl)-7-(3-piperidin-1-ylpropoxy)-1,2,3,4-tetrahydroisochinolin;
7-(4-Piperidin-1-ylbut-1-inyl)-4-pyridin-3-yl-1,2,3,4-tetrahydroisochinolin;
7-(4-Piperidin-1-ylbutyl)-4-pyridin-3-yl-1,2,3,4-tetrahydroisochinolin;
2-Methyl-7-(4-piperidin-1-ylbutyl)-4-pyridin-3-yl-1,2,3,4-tetrahydroisochinolin;
7-[4-(4,4-Difluorpiperidin-1-yl)-but-1-inyl]-4-(4-methoxyphenyl)-2-methyl-1,2,3,4-tetrahydroisochinolin;
4-(4-Methoxyphenyl)-2-methyl-7-(4-piperidin-1-ylbut-1-inyl)-1,2,3,4-tetrahydroisochinolin;
4-(4-Methoxyphenyl)-2-methyl-7-(4-morpholin-4-ylbut-1-inyl)-1,2,3,4-tetrahydroisochinolin;
4-(4-Methoxyphenyl)-2-methyl-7-(4-thiomorpholin-4-ylbut-1-inyl)-1,2,3,4-tetrahydroisochinolin;
7-[4-(4-Isopropylpiperazin-1-yl)-but-1-inyl]-4-(4-methoxyphenyl)-2-methyl-1,2,3,4-tetrahydroisochinolin;
4-(4-Fluorphenyl)-2-methyl-7-(4-piperidin-1-ylbut-1-inyl)-1,2,3,4-tetrahydroisochinolin;
7-[4-(4,4-Difluorpiperidin-1-yl)-butyl]-4-(4-methoxyphenyl)-2-methyl-1,2,3,4-tetrahydroisochinolin;
4-(4-Methoxyphenyl)-2-methyl-7-(4-morpholin-4-ylbutyl)-1,2,3,4-tetrahydroisochinolin;
4-(4-Methoxyphenyl)-2-methyl-7-(4-thiomorpholin-4-ylbutyl)-1,2,3,4-tetrahydroisochinolin;
7-[4-(4-Isopropylpiperazin-1-yl)-butyl]-4-(4-methoxyphenyl)-2-methyl-1,2,3,4-tetrahydroisochinolin;
und Salze davon.

65. Pharmazeutische Zusammensetzung, die einen pharmazeutisch verträglichen Trägerstoff und eine wirksame Menge einer Verbindung von Formel (I) umfasst: 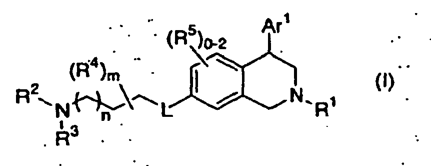 worin
L -O- ist und n 1 oder 2 ist; oder L -C≡C- oder -CH₂CH₂- ist und n 0 oder 1 ist;
R¹ -H ist; oder -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -COOC₁₋₆-Alkyl oder -COOBenzyl ist, von denen jedes gegebenenfalls einfach, zweifach oder dreifach mit R^{a} substituiert ist;
wobei R^{a} ausgewählt ist aus -OH, -OC₁₋₆-Alkyl, Phenyl, gegebenenfalls substituiert mit -OC₁₋₄-Alkyl oder Halo, -CN, -NO₂, -N(R^{b})R^{c}, -C(O)N(R^{b})R^{c}, -N(R^{b})C(O)R^{b}, -N(R^{b})SO₂C₁₋₆-Alkyl, -C(O)C₁₋₆-Alkyl, -S(O)₀₋₂-C₁₋₆-Alkyl, -SO₂N(R^{b})R^{c}, -SCF₃, Halo, -CF₃, -OCF₃, -COOH und -COOC₁₋₆-Alkyl;
wobei R^{b} und R^{c} jeweils unabhängig -H oder -C₁₋₆-Alkyl sind;
R² und R³ jeweils unabhängig ausgewählt sind aus der Gruppe, bestehend aus : -H;
A) -C₁₋₆-Alkyl, -C₃₋₆-Alkenyl, -C₃₋₆-Alkinyl, -C₃₋₇-Cycloalkyl, -C₁₋₆-Alkyl-C₃₋₇-cycloalkyl, -CH(C₃₋₈-Cycloalkyl)₂, -CH(Phenyl)₂, Benzyl und -C(O)OC₁₋₄-Alkyl, wobei jedes Alkyl, Cycloalkyl oder Benzyl gegebenenfalls mit -OH, -OC₁₋₄-Alkyl, -CN, -NH₂, -NH(C₁₋₄-Alkyl), -N(C₁₋₄-Alkyl)₂, Halo, -CF₃, -OCF₃, -COOH oder -COOC₁₋₆-Alkyl substituiert ist;
B) Phenyl oder Pyridyl, gegebenenfalls an zwei benachbarten Kohlenstoffringgliedern an eine drei- oder viergliedrige Kohlenwasserstoffeinheit kondensiert, um einen kondensierten fünf- oder sechsgliedrigen aromatischen Ring zu bilden, wobei in der Einheit ein Kohlenstoffatom durch >O, >S, >NH, >N(C₁₋₄-Alkyl) oder >NC(O)OC₁₋₄-Alkyl ersetzt ist und wobei in der Einheit bis zu ein zusätzliches Kohlenstoffatom gegebenenfalls durch -N= ersetzt ist;
C) Naphthyl;
D) einem 4- bis 8-gliedrigen heterocyclischen Ring, wobei der heterocyclische Ring ein Kohlenstoffatom aufweist, das der Bindungspunkt ist, 1 oder 2 Heteroatomglieder aufweist, die ausgewählt sind aus der Gruppe, bestehend aus >O, >S(O)₀₋₂, >NH, >N(C₁₋₄-Alkyl) und >NC(O)OC₁₋₄-Alkyl, und 0 oder 1 Doppelbindungen aufweist; und
E) einer monocyclischen aromatischen Kohlenwasserstoffgruppe, die fünf oder sechs Ringatome aufweist, die ein Kohlenstoffatom aufweist, das der Bindungspunkt ist, in der ein Kohlenstoffatom durch >O, >S, >NH, >N(C₁₋₄-Alkyl) oder >NC(O)OC₁₋₄-Alkyl ersetzt ist, in der bis zu ein zusätzliches Kohlenstoffatom gegebenenfalls durch -N= ersetzt ist und die gegebenenfalls benzokondensiert oder pyridokondensiert ist;
wobei B)-E) jeweils gegebenenfalls einfach, zweifach oder dreifach mit einer Einheit substituiert sind, die ausgewählt ist aus der Gruppe, bestehend aus -C₁₋₄-Alkyl, -OH, -C₁₋₄-AlkylOH, -OC₁₋₆-Alkyl, -CN, -NO₂, -N(R^{d})R^{e}, -C(O)N(R^{d})R^{e}, -N(R^{d})C(O)R^{d}, -N(R^{d})SO₂C₁₋₆-Alkyl, -C(O)C₁₋₆-Alkyl, -S(O)₀₋₂-C₁₋₆-Alkyl, -SO₂N(R^{d})R^{e}, -SCF₃, Halo, -CF₃, -OCF₃, -COOH, -COOC₁₋₆-Alkyl, -OC(O)N(R^{d})R^{e} und -OC(O)OC₁₋₆-Alkyl;
wobei R^{d} und R^{e} jeweils unabhängig -H oder -C₁₋₆-Alkyl sind,
oder, alternativ,
R² und R³ mit dem Stickstoff, an das sie gebunden sind, zusammengenommen sein können, um Tetrahydropyrimidin-2-ylidenyl oder einen 4- bis 8-gliedrigen heterocyclischen Ring zu bilden, wobei der heterocyclische Ring 0 oder 1 zusätzliche Heteroatomglieder aufweist, die vom Bindungsstickstoff durch wenigstens ein Kohlenstoffglied getrennt sind und ausgewählt sind aus der Gruppe, bestehend aus >O, >S(O)₀₋₂, >NH und >NR^{f}, 0 oder 1 Doppelbindungen aufweist, 0, 1 oder 2 Kohlenstoffglieder aufweist, die vom Bindungsstickstoff durch wenigstens ein Kohlenstoffglied getrennt sind, das ein Carbonyl ist, gegebenenfalls benzo- oder pyridokondensiert ist, gegebenenfalls ein Kohlenstoffglied aufweist, das eine Brücke bildet, und 0-5 Kohlenstoffgliedsubstituenten R^{ff} aufweist,
wobei R^{f} ausgewählt ist aus der Gruppe, bestehend aus:
i) -C₁₋₆-Alkyl, gegebenenfalls substituiert mit R^{z}, -C₃₋₆-Alkenyl, -C₃₋₆-Alkinyl, -C(O)N(R^{g})R^{h}, -C(O)Rⁱ, -S(O)₀₋₂-C₁₋₆-Alkyl und -COOC₁₋₆-Alkyl,
wobei R^{z} Fluor, -CN, -OH, -OC₁₋₄-Alkyl, -C₃₋₇-Cycloalkyl oder -CF₃ ist;
wobei R^{g} und R^{h} jeweils unabhängig -H oder -C₁₋₆-Alkyl sind; und
wobei Rⁱ -C₁₋₆-Alkyl, -C₃₋₈-Cycloalkyl, Phenyl oder 5-oder 6-gliedriges aromatisches Heterocyclyl ist, wobei jedes Alkyl, Cycloalkyl, Phenyl oder Heterocyclyl gegebenenfalls einfach, zweifach oder dreifach mit -C₁₋₄-Alkyl, -OH, -OC₁₋₆-Alkyl, -CF₃, -CN oder Halo substituiert ist;
ii) -(CH₂)₀₋₁-RingA, wobei Ring A Phenyl oder ein 5- oder 6-gliedriges kohlenstoffverknüpftes aromatisches Heterocyclyl ist, gegebenenfalls substituiert mit R^{aa};
wobei R^{aa} -OH, -C₁₋₆-Alkyl, -OC₁₋₆-Alkyl, Phenyl, -CN, -NO₂, -N(R^{g})R^{h}, -C(O)N(R^{g})R^{h}, -N(R^{g})C(O)R^{h}, -N(R^{h})SO₂C₁₋₆-Alkyl, -C(O)C₁₋₆-Alkyl, -S(O)₀₋₂-C₁₋₆-Alkyl, -SO₂N(R^{g})R^{h}, -SCF₃, Halo, -CF₃, -OCF₃, -OCHF₂, -COOH und -COOC₁₋₆-Alkyl ist; und
iii) -(CH₂)₀₋₁-RingB, wobei Ring B -C₃₋₇-Cycloalkyl ist, in dem ein oder zwei Kohlenstoffringglieder gegebenenfalls durch >O oder >NH ersetzt sind und das gegebenenfalls mit -C₁₋₄-Alkyl, Fluor, -CN, -OH, -OC₁₋₄-Alkyl oder -CF₃ substituiert ist;
wobei R^{ff} ausgewählt ist aus der Gruppe, bestehend aus -C₁₋₆-Alkyl, gegebenenfalls einfach oder zweifach substituiert mit R^{z}, -C₂₋₆-Alkenyl, -C₂₋₆-Alkinyl, -C₃₋₇-Cycloalkyl, -CH(Phenyl)₂, Halo, -OH, -OC₁₋₆-Alkyl, -OC₂₋₃-Alkyl-O-, -CN, -NO₂, -N(R^{g})R^{h}, -C(O)N(R^{g})R^{h}, -N(R^{g})C(O)R^{g}, -N(R^{g})SO₂C₁₋₆-Alkyl, -C(O)R¹, -S(O)₀₋₂-C₁₋₆-Alkyl, -SO₂N(R^{g})R^{h}, -SCF₃, -CF₃, -OCF₃, -COOH und -COOC₁₋₆-Alkyl;
R⁴ -OH, -OC₁₋₆-Alkyl, -CF₃, -C₁₋₆-Alkyl oder Halo ist; zwei R⁴-Substituenten zusammengenommen sein können, um Methylen oder Ethylen zu bilden; oder eines von R⁴ mit R² zusammengenommen ist, um Methylen, Ethylen, Propylen oder -CH₂CH₂O- zu bilden, wobei jedes gegebenenfalls mit -OH, -OC₁₋₆-Alkyl, -SC₁₋₆-Alkyl, -CF₃, -C₁₋₆-Alkyl, Amino oder Halo substituiert ist;
m 0, 1 oder 2 ist;
R⁵ ausgewählt ist aus der Gruppe, bestehend aus -C₁₋₆-Alkyl, -OH, -OC₁₋₆-Alkyl, -SC₁₋₆-Alkyl und Halo;
Ar¹ ein Aryl- oder Heteroarylring ist, der ausgewählt ist aus der Gruppe, bestehend aus:
a) Phenyl, gegebenenfalls einfach, zweifach oder dreifach substituiert mit R^{j} und gegebenenfalls auf benachbarten Kohlenstoffen zweifach substituiert mit -OC₁₋₄-AlkylenO-, gegebenenfalls einfach oder zweifach substituiert mit Fluor, -(CH₂)₂₋₃NH-, -(CH₂)₁₋₂NH(CH₂)-, -(CH₂)₂₋₃N(C₁₋₄-Alkyl)- oder -(CH₂)₁₋₂N(C₁₋₄-Alkyl)(CH₂)-;
wobei R^{j} ausgewählt ist aus der Gruppe, bestehend aus
1) -OH, -C₁₋₆-Alkyl, -OC₁₋₆-Alkyl, gegebenenfalls einfach, zweifach oder dreifach substituiert mit Halo, -C₂₋₆-Alkenyl, -OC₃₋₆-Alkenyl, -C₂₋₆-Alkinyl, gegebenenfalls substituiert mit Trimethylsilyl, -OC₃₋₆-Alkinyl, -C₃₋₆-Cycloalkyl, -OC₃₋₆-Cycloalkyl, -CN, -NO₂, -N(R^{k})R^{l}, -N(R^{k})C(O)R^{l}, -N(R^{k})SO₂C₁₋₆-Alkyl, -C(O)C₁₋₆-Alkyl, -S(O)₀₋₂-C₁₋₆-Alkyl, -C(O)N(R^{m})Rⁿ, -SO₂N(R^{m})Rⁿ, -SCF₃, Halo, -CF₃, -COOH, -COOC₁₋₆-Alkyl und -COOC₃₋₇-Cycloalkyl;
wobei R^{k} und R^{l} jeweils unabhängig -H oder -C₁₋₆-Alkyl sind;
wobei R^{m} und Rⁿ jeweils unabhängig -H oder -C₁₋₆-Alkyl sind oder R^{m} und Rⁿ mit ihrem Bindungsstickstoff zusammengenommen einen 4- bis 8-gliedrigen heterocyclischen Ring bilden, der 1 oder 2 Heteroatomglieder aufweist, die ausgewählt sind aus >O, >S(O)₀₋₂, >NH und >NC₁₋₆-Alkyl, der 0 oder 1 Doppelbindungen aufweist, der 0 oder 1 Carbonylglieder aufweist;
2) -G-Ar², wobei G eine Bindung, -O- oder -S- ist und Ar² Phenyl ist oder eine monocyclische aromatische Kohlenwasserstoffgruppe ist, die fünf oder sechs Ringatome aufweist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die jeweils gegebenenfalls einfach, zweifach oder dreifach mit R^{p} substituiert ist;
wobei R^{p} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{q})R^{r}, -C(O)N(R^{q})R^{r}, -N(R^{q})C(O)R^{r}, -N(R^{q})SO₂C₁₋₆-Alkyl, -C(O)C₁₋₆-Alkyl, -S(O)₀₋₂-C₁₋₆-Alkyl, -SO₂N(R^{q})R^{r}, -SCF₃, Halo, -CF₃, -OCF₃, -OCHF₂, -COOH und -COOC₁₋₆-Alkyl;
wobei R^{q} und R^{r} jeweils unabhängig ausgewählt sind aus -H, -C₁₋₆-Alkyl und -C₂₋₆-Alkenyl; und
3) einem 4- bis 8-gliedrigen gesättigten oder teilweise gesättigten heterocyclischen Ring, der 1 oder 2 Heteroatomglieder aufweist, die ausgewählt sind aus >O, >S(O)₀₋₂, >NH und >NC₁₋₆-Alkyl, der 0 oder 1 Carbonylglieder aufweist, wobei der Ring gegebenenfalls einfach, zweifach oder dreifach mit R^{p} substituiert ist;
b) Phenyl oder Pyridyl, an zwei benachbarten Kohlenstoffringgliedern an eine dreigliedrige Kohlenwasserstoffeinheit kondensiert, um einen kondensierten fünfgliedrigen aromatischen Ring zu bilden, wobei in der Einheit ein Kohlenstoffatom durch >O, >S, >NH oder >N(C₁₋₄-Alkyl) ersetzt ist und wobei in der Einheit bis zu ein zusätzliches Kohlenstoffatom gegebenenfalls durch -N= ersetzt ist, wobei die kondensierten Ringe gegebenenfalls einfach, zweifach oder dreifach mit R^{t} substituiert sind;
wobei R^{t} ein Substituent ist, der unabhängig ausgewählt ist aus der Gruppe, bestehend aus: -OH, -C₁₋₆-Alkyl, -OC₁₋₆-Alkyl, Phenyl, -CN, -NO₂, -N(R^{u})R^{v}, -C(O)N(R^{u})R^{v} -N(R^{u})C(O)R^{v}, -N(R^{u})SO₂C₁₋₆-Alkyl, -C(O)C₁₋₆-Alkyl, -S(O)₀₋₂-C₁₋₆-Alkyl, -SO₂N(R^{u})R^{v}, -SCF₃, Halo, -CF₃, -OCF₃, -OCHF₂, -COOH und -COOC₁₋₆-Alkyl;
wobei R^{u} und R^{v} jeweils unabhängig -H oder -C₁₋₆-Alkyl sind;
c) Phenyl, an zwei benachbarten Ringgliedern an eine viergliedrige Kohlenwasserstoffeinheit kondensiert, um einen kondensierten sechsgliedrigen aromatischen Ring zu bilden, wobei in der Einheit ein oder zwei Kohlenstoffatome durch -N= ersetzt sind, wobei die kondensierten Ringe gegebenenfalls einfach, zweifach oder dreifach mit R^{t} substituiert sind;
d) Naphthyl, gegebenenfalls einfach, zweifach oder dreifach mit R^{t} substituiert;
e) einer monocyclischen aromatischen Kohlenwasserstoffgruppe, die fünf Ringatome aufweist, die ein Kohlenstoffatom aufweist, das der Bindungspunkt ist, in der ein Kohlenstoffatom durch >O, >S, >NH oder >N(C₁₋₄-Alkyl) ersetzt ist, in der bis zu ein zusätzliches Kohlenstoffatom gegebenenfalls durch -N= ersetzt ist, die gegebenenfalls einfach oder zweifach mit R^{j} substituiert ist und gegebenenfalls an zwei benachbarten Kohlenstoffatomen benzokondensiert oder pyridokondensiert ist, wobei die benzokondensierte oder pyridokondensierte Einheit gegebenenfalls einfach, zweifach oder dreifach mit R^{t} substituiert ist; und
f) einer monocyclischen aromatischen Kohlenwasserstoffgruppe, die sechs Ringatome aufweist, die ein Kohlenstoffatom aufweist, das der Bindungspunkt ist, in der ein oder zwei Kohlenstoffatome durch -N= ersetzt sind, die gegebenenfalls einfach oder zweifach mit R^{j} substituiert ist und die gegebenenfalls an zwei benachbarten Kohlenstoffatomen benzokondensiert oder pyridokondensiert ist, wobei die benzokondensierte oder pyridokondensierte Einheit gegebenenfalls einfach oder zweifach mit R^{t} substituiert ist;
und Enantiomere, Diastereomere, Hydrate, Solvate und pharmazeutisch verträgliche Salze, Ester und Amide davon,
mit der Maßgabe:
wenn n 1 ist, L -O- ist, Ar unsubstituiertes Phenyl ist und R² und R³ beide Methyl sind, dann ist R⁴ nicht -OH.

## Revendications

1. Composé de formule (I): 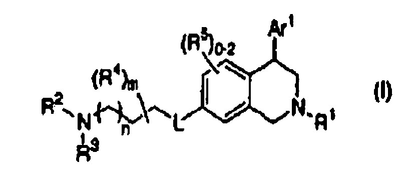 dans laquelle
L est -0- et n vaut 1 ou 2; ou L est -C=C- ou -CH₂CH₂- et n vaut 0 ou 1;
R¹ est -H; ou est un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyle en C_{3 à 7}, -COOalkyle en C_{1 à 6} ou -COObenzyle, chacun facultativement mono-, di- ou tri-substitué par R^{a};
où R^{a} est choisi parmi un groupe -OH, -Oalkyle en C_{1 à 6}, phényle facultativement substitué par un groupe -Oalkyle en C_{1 à 4} ou halogéno, -CN, -NO₂-, -N(R^{b})R^{c}, -C(O)N(R^{b})R^{c}, -N(R^{b})C(O)R^{b}, -N(R^{b})SO₂alkyle en C_{1 à 6}, -C(O)alkyle en C_{1 à 6}, -S(O)_{0 à 2}-alkyle en C_{1 à 6}, -SO₂N(R^{b})R^{c}, -SCF₃, halogéno, -CF₃, -OCF₃, -COOH et -COOalkyle en C_{1 à 6};
où R^{b} et R^{c} sont chacun indépendamment -H ou un groupe -alkyle en C_{1 à 6};
R² et R³ sont chacun indépendamment choisis dans le groupe consistant en: -H;
A) un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyle en C_{3 à 7}, -CH (cycloalkyle en C_{3 à 8})₂, -CH(phényle)₂, benzyle et -C(O)Oalkyle en C_{1 à 4}, où chaque groupe alkyle, cycloalkyle ou benzyle est facultativement substitué par un groupe -OH, -Oalkyle en C_{1 à 4}, -CN, -NH₂, -NH (alkyle en C_{1 à 4}), -N (alkyle en C_{1 à 4})₂, halogéno, -CF₃, -OCF₃, -COOH ou -COOalkyle en C_{1 à 6};
B) un groupe phényle ou pyridyle, facultativement condensé au niveau de deux chaînons de cycle de carbone adjacents à un fragment hydrocarbure à trois ou quatre chaînons pour former un cycle aromatique condensé à cinq ou six chaînons, lequel fragment a un atome de carbone remplacé par >O, >S, >NH, >N (alkyle en C_{1 à 4}) ou >NC(O)Oalkyle en C_{1 à 4}, et lequel fragment a jusqu'à un atome de carbone additionnel facultativement remplacé par -N=;
C) un groupe naphtyle,
D) un cycle hétérocyclique à 4 à 8 chaînons, ledit cycle hétérocyclique ayant un atome de carbone qui est le point d'attache, comportant 1 ou 2 chaînons hétéroatome choisis dans le groupe consistant en >O, >S(O)_{0 à 2}, >NH, >N(alkyle en C_{1 à 4}), et >NC (O)Oalkyle en C_{1 à 4}, et comportant 0 ou 1 double liaison; et
E) un groupe hydrocarbure aromatique monocyclique comportant cinq ou six atomes de cycle, ayant un atome de carbone qui est le point d'attache, ayant un atome de carbone remplacé par >O, >S, >NH, >N(alkyle en C_{1 à 4}) ou >NC(O)Oalkyle en C_{1 à 4}, ayant jusqu'à un atome de carbone additionnel facultativement remplacé par -N=, et facultativement benzocondensé ou pyridocondensé;
où chacun de B) à E) est facultativement mono-, di- ou tri-substitué par un fragment choisi dans le groupe consistant en un groupe -alkyle en C_{1 à 4}, -OH, -alkyl en C_{1 à 4}OH, -Oalkyle en C_{1 à 6}, -CN, -NO₂, -N(R^{d})R^{e}, -C(O)N(R^{d})R^{e}, -N(R^{d})C(O)R^{d}, -N(R^{d})SO₂alkyle en C_{1 à 6}, -C(O)alkyle en C_{1 à 6}, -S(O)_{0 à 2}-alkyle en C_{1 à 6}, -SO₂N(R^{d})R^{e}, -SCF₃, halogéno, -CF₃, -OCF₃, -COOH, -COOalkyle en C_{1 à 4}, -OC(O)N(R^{d})R^{e} et -OC(O)Oalkyle en C_{1 à 6};
où R^{d} et R^{e} sont chacun indépendamment -H ou un groupe -alkyle en C_{1 à 6};
ou, en variante,
R² et R³ peuvent être pris conjointement avec l'atome d'azote auquel ils sont attachés pour former un groupe tétrahydro-pyrimidin-2-ylidényle, ou un cycle hétérocyclique à 4 à 8 chaînons, ledit cycle hétérocyclique ayant 0 ou 1 chaînon hétéroatome additionnel séparé de l'azote d'attache par au moins un chaînon carbone et choisi dans le groupe consistant en >O, >S(O)_{0 à 2}, >NH et >NR^{f}, ayant 0 ou 1 double liaison, ayant 0, 1 ou 2 chaînons carbone séparés de l'atome d'azote d'attache par au moins un chaînon carbone qui est un groupe carbonyle, facultativement benzo ou pyridocondensé, comportant facultativement un chaînon carbone qui forme un pont, et comportant 0 à 5 substituants de chaînon carbone R^{ff},
où R^{f} est choisi dans le groupe consistant en:
i) un groupe -alkyle en C_{1 à 6} facultativement substitué par R^{z}, -alcényle en C_{3 à 6}, -alcynyle en C_{3 à 6}, -C(O)N(R^{g})R^{h}, -C(O)Rⁱ, -S(O)_{0 à 2}-alkyle en C_{1 à 6} et -COOalkyle en C_{1 à 6},
où R^{z} est un groupe fluoro, -CN, -OH, -Oalkyle en C_{1 à 4}, -cycloalkyle en C_{3 à 7} ou -CF_{3;}
où R^{g} et R^{h} sont chacun indépendamment -H ou -alkyle en C_{1 à 6}; et
où Rⁱ est un groupe -alkyle en C_{1 à 6}, -cycloalkyle en C_{3 à 8}, phényle, ou hétérocyclyle aromatique à 5 ou 6 chaînons, où chaque groupe alkyle, cycloalkyle, phényle ou hétérocyclyle est facultativement mono-, di- ou tri-substitué par un groupe -alkyle en C_{1 à 4}, -OH, -Oalkyle en C_{1 à 6}, -CF₃, -CN ou halogéno;
ii) -(CH₂)_{0 à 1}-Cycle A, où Cycle A est un groupe phényle ou un groupe hétérocyclyle aromatique à 5 ou 6 chaînons à liaison carbone, facultativement substitué par R^{aa};
où R^{aa} est un groupe -OH, -alkyle en C_{1 à 6}, -Oalkyle en C_{1 à 6}, phényle, -CN, -NO₂, -N(R^{g})R^{h}, -C(O)N(R^{g})R^{h}, -(R^{g})C(O)R^{h}, -N(R^{h})SO₂-alkyle en C_{1 à 6}, -C(O)alkyle en C_{1 à 6}, -S(O)_{0 à 2}-alkyle en C_{1 à 6}, -SO₂N(R^{g})R^{h}, -SCF₃, halogéno, -CF₃, -OCF₃, -OCHF₂, -COOH et -COOalkyle en C_{1 à 6}; et
iii) -(CH₂)_{0 à 1}-Cycle B, où Cycle B est un groupe -cycloalkyle en C_{3 à 7} avec un ou deux chaînons de cycle carboné facultativement remplacés par >0 ou >NH, et facultativement substitué par un groupe -alkyle en C_{1 à 4}, fluoro, -CN, -OH, -Oalkyle en C_{1 à 4} ou -CF_{3;}
où R^{ff} est choisi dans le groupe consistant en un groupe -alkyle en C_{1 à 6} facultativement mono- ou di-substitué par R^{z}, un groupe -alcényle en C_{2 à 6}, -alcynyle en C_{2 à 6}, -cycloalkyle en C_{3 à 7}, -CH(phényle)₂, halogéno, -OH, -Oalkyle en C_{1 à 6}, -Oalkyl en C_{2 à 3}O-, -CN, -NO₂, -N(R^{g})R^{h}, -C(O)N(R^{g})R^{h}, -N(R^{g})C(O)R^{g}, -N(R^{g})SO₂alkyle en C_{1 à 6}, -C(O)Rⁱ, -S (0) _{0 à 2}-alkyle en C_{1 à 6}, -SO₂N(R^{g})R^{h}, -SCF₃, -CF₃, -OCF₃, -COOH et -COOalkyle en C_{1 à 6};
R⁴ est un groupe -OH, -Oalkyle en C_{1 à 6}, -CF₃, -alkyle en C_{1 à 6} ou halogéno; deux substituants R⁴ peuvent être pris ensemble pour former un groupe méthylène ou éthylène; ou l'un de R⁴ est pris conjointement avec R² pour former un groupe méthylène, éthylène, propylène ou -CH₂CH₂O-, où chacun est facultativement substitué par un groupe -OH, -Oalkyle en C_{1 à 6}, -Salkyle en C_{1 à 6}, -CF₃, -alkyle en C_{1 à 6}, amino ou halogéno;
m vaut 0, 1 ou 2;
R⁵ est choisi dans le groupe consistant en un groupe -alkyle en C_{1 à 6}, -OH, -Oalkyle en C_{1 à 6}, -Salkyle en C_{1 à 6} et halogéno;
Ar¹ est un cycle aryle ou hétéroaryle choisi dans le groupe consistant en:
a) un groupe phényle, facultativement mono-, di- ou tri-substitué par R^{j} et facultativement di-substitué sur des atomes de carbone adjacents par un groupe -Oalkylène en C_{1 à 4}O- facultativement mono- ou di-substitué par un groupe fluoro, -(CH₂) _{2 à 3}-NH-, -(CH₂)_{1 à 2}NH(CH₂)-, -(CH₂)_{2 à 3}-N(alkyle en C_{1 à 4})-, ou -(CH₂)_{1 à 2}N(alkyl en C_{1 à 4})(CH₂)-;
où R^{j} est choisi dans le groupe consistant en
1) un groupe -OH, -alkyle en C_{1 à 6}, -Oalkyle en C_{1 à 6} facultativement mono-, di- ou tri-substitué par un groupe halogéno, -alcényle en C_{2 à 6}, -Oalcényle en C_{3 à 6}, -alcynyle en C_{2 à 6} facultativement substitué par un groupe triméthylsilyle, -Oalcynyle en C_{3 à 6}, -cycloalkyle en C_{3 à 6}, -Ocycloalkyle en C_{3 à 6}, -CN, -NO₂, -N(R^{k})R^{j}, -N(R^{k})C(O)R^{l}, -N(R^{k})SO₂alkyle en C_{1 à 6}, -C(O)alkyle en C_{1 à 6}, -S(O)_{0 à 2}-alkyle en C_{1 à 6}, -C(O)N(R^{m})Rⁿ, -SO₂N(R^{m})Rⁿ, -SCF₃, halogéno, -CF₃, -COOH, -COOalkyle en C_{1 à 6} et -COOcycloalkyle en C_{3 à 7};
où R^{k} et R^{l} sont chacun indépendamment -H ou un groupe -alkyle en C_{1 à 6};
où R^{m} et Rⁿ sont chacun indépendamment -H ou un groupe -alkyle en C_{1 à 6}, ou R^{m} et Rⁿ pris conjointement avec leur atome d'azote d'attache forment un cycle hétérocyclique à 4 à 8 chaînons comportant 1 ou 2 chaînons hétéroatome choisis parmi >O, >S(O)_{0 à 2}, >NH et >Nalkyle en C_{1 à 6}, comportant 0 ou 1 double liaison, comportant 0 ou 1 chaînon carbonyle;
2) -G-Ar², où G est une liaison, -O- ou -S-, et Ar² est un groupe phényle ou est un groupe hydrocarbure aromatique monocyclique comportant cinq ou six atomes de cycle, comportant un atome de carbone remplacé par >O, >S, >NH, ou >N (alkyle en C_{1 à 4}), comportant jusqu'à un atome de carbone additionnel facultativement remplacé par -N=, chacun facultativement mono-, di- ou tri-substitué par R^{p};
où R^{p} est un substituant indépendamment choisi dans le groupe consistant en: un groupe -OH, -alkyle en C_{1 à 6}, -Oalkyle en C_{1 à 6}, phényle, -CN, -NO₂, -N(R^{q})R^{r}, -C(O)N(R^{q})R^{r}, -N(R^{q})C(O)R^{r}, -N(R^{q})SO₂alkyle en C_{1 à 6}, -C(O)alkyle en C_{1 à 6}, -S(O)_{0 à 2}-alkyle en C_{1 à 6}, -SO₂N(R^{q})R^{r}, -SCF₃, halogéno, -CF₃, -OCF₃, -OCHF₂, -COOH et -COOalkyle en C_{1 à 6};
où R^{q} et R^{r} sont chacun indépendamment choisis parmi -H, un groupe -alkyle en C_{1 à 6} et -alcényle en C_{2 à 6}; et
3) un cycle hétérocyclique saturé ou partiellement saturé à 4 à 8 chaînons, comportant 1 ou 2 chaînons hétéroatome choisis parmi >O, >S(O)_{0 à 2}, >NH et >Nalkyle en C_{1 à 6}, comportant 0 ou 1 chaînon carbonyle, ledit cycle étant facultativement mono-, di- ou tri-substitué par R^{p;}
b) un groupe phényle ou pyridyle condensé au niveau de deux chaînons de cycle de carbone adjacents à un fragment hydrocarbure à trois chaînons pour former un cycle aromatique condensé à cinq chaînons, lequel fragment a un atome de carbone remplacé par >O, >S, >NH ou >N (alkyle en C_{1 à 4}), et lequel fragment a jusqu'à un atome de carbone additionnel facultativement remplacé par -N=, les cycles condensés étant facultativement mono-, di- ou tri-substitués par R^{t};
où R^{t} est un substituant indépendamment choisi dans le groupe consistant en: un groupe -OH, -alkyle en C_{1 à 6}, -Oalkyle en C_{1 à 6}, phényle, -CN, -NO₂, -N(R^{u})R^{v}, -C(O)N(R^{u})R^{v}, -N(R^{u})C(O)R^{v}, -N(R^{u})SO₂alkyle en C_{1 à 6,} -C(O)alkyle en C_{1 à 6}, -S(O)_{0 à 2}-alkyle en C_{1 à 6}, -SO₂N(R^{u})R^{v}, -SCF₃, halogéno, -CF₃, -OCF₃, -OCHF₂, -COOH et -COOalkyle en C_{1 à 6};
où R^{u} et R^{v} sont chacun indépendamment -H ou un groupe -alkyle en C_{1 à 6};
c) un groupe phényle condensé au niveau de deux chaînons de cycle adjacents à un fragment hydrocarbure à quatre chaînons pour former un cycle aromatique condensé à six chaînons, lequel fragment a un ou deux atomes de carbone remplacés par -N=, les cycles condensés étant facultativement mono-, di- ou tri-substitués par R^{t};
d) un groupe naphtyle, facultativement mono-, di- ou tri-substitué par R^{t};
e) un groupe hydrocarbure aromatique monocyclique comportant cinq atomes de cycle, comportant un atome de carbone qui est le point d'attache, ayant un atome de carbone remplacé par >0, >S, >NH ou >N(alkyle en C_{1 à 4}), ayant jusqu'à un atome de carbone additionnel facultativement remplacé par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono-, di- ou tri-substitué par R^{t}; et
f) un groupe hydrocarbure aromatique monocyclique comportant six atomes de cycle, ayant un atome de carbone qui est le point d'attache, ayant un ou deux atomes de carbone remplacés par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono- ou di-substitué par R^{t};
et ses énantiomères, diastéréoisomères, hydrates, solvates, et sels, esters et amides pharmaceutiquement acceptables, à condition que:
lorsque n vaut 1, L est -O-, Ar est un groupe phényle non substitué et R² et R³ sont tous deux un groupe méthyle, alors R⁴ ne soit pas -OH,
à utiliser dans le traitement ou la prévention d'un trouble du SNC choisi dans le groupe consistant en: les troubles du sommeil et de l'éveil et de la stimulation et de la vigilance, l'insomnie, le décalage horaire, le sommeil perturbé, les troubles déficitaires de l'attention avec hyperactivité (TDAH), les troubles déficitaires de l'attention, les troubles d'apprentissage et de la mémoire, l'altération de l'apprentissage, l'altération de la mémoire, la perte de mémoire, le dysfonctionnement cognitif, la migraine, l'inflammation neurogène, la démence, l'altération cognitive douce, la prédémence, la maladie d'Alzheimer, l'épilepsie, la narcolepsie avec ou sans cataplexie associée, la cataplexie, les troubles de l'homéostase du sommeil et de l'éveil, la somnolence idiopathique, la somnolence diurne excessive (EDS), les troubles du rythme circadien, les troubles du sommeil et de la fatigue, la fatigue, l'assoupissement associé aux apnées du sommeil, l'altération du sommeil due aux changements hormonaux périménopausiques, la fatigue liée à Parkinson, la fatigue liée à MS, la fatigue liée à la dépression, la fatigue induite par chimiothérapie, la fatigue liée au travail, la léthargie, les troubles de l'alimentation, l'obésité, la cinétose, le vertige, la schizophrénie, l'abus de substances, les troubles bipolaires, les troubles maniaques et la dépression chez les mammifères.

2. Composé de la revendication 1, dans lequel L est -0-et n vaut 1.

3. Composé de la revendication 1, dans lequel L est -C≡C et n vaut 0.

4. Composé de la revendication 1, dans lequel L est -CH₂-CH₂- et n vaut 0.

5. Composé de la revendication 1, dans lequel R¹ est un groupe méthyle, éthyle, propyle, t-butyle, allyle, propargyle ou benzyle.

6. Composé de la revendication 1, dans lequel R¹ est un atome d'hydrogène ou un groupe méthyle.

7. Composé de la revendication 1, dans lequel R² est un groupe méthyle, R³ n'est pas un groupe méthyle.

8. Composé de la revendication 1, dans lequel R² et R³ peuvent être pris conjointement avec l'atome d'azote pour former un cycle hétérocyclique à 4 à 8 chaînons, ledit cycle hétérocyclique étant choisi parmi la pipéridine, la pyrrolidine et la morpholine, ledit cycle étant substitué par 1 ou 2 substituants R^{ff}.

9. Composé de la revendication 1, choisi dans le groupe consistant en:
La 7-[3-(4-fluoro-pipéridin-1-yl)-propoxy]-4-(4-méthoxy-phényl)-2-méthyl-1,2,3,4-tétrahydro-isoquinoléine;
La 7-[3-(4-fluoro-pipéridin-1-yl)-propoxy]-4-(4-méthoxy-phényl)-2-méthyl-1,2,3,4-tétrahydro-isoquinoléine (énantiomère 1);
La 7-[3-(4-fluoro-pipéridin-1-yl)-propoxy]-4-(4-méthoxy-phényl)-2-méthyl-1,2,3,4-tétrahydro-isoquinoléine (énantiomère 2);
La 1-(4-{3-[4-(4-méthoxy-phényl)-2-méthyl-1,2,3,4-tétrahydro-isoquinoléin-7-yloxy]-propyl}-pipérazin-1-yl)-éthanone;
La diéthyl-{3-[4-(4-méthoxy-phényl)-2-méthyl-1,2,3,4-tétrahydro-isoquinoléin-7-yloxy]propyl}-amine;
la (4-{3-[4-(4-méthoxy-phényl)-2-méthyl-1,2,3,4-tétrahydro-isoquinoléin-7-yloxy]-propyl}-pipérazin-1-yl)-pyridin-4-yl-méthanone;
la 1-(4-{3-[4-(4-méthoxy-phényl)-2-méthyl-1,2,3,4-tétrahydro-isoquinoléin-7-yloxy]-propyl}-pipérazin-1-yl)-2-méthyl-propan-1-one;
la cyclobutyl-(4-{3-[4-(4-méthoxy-phényl)-2-méthyl-1,2,3,4-tétrahydro-isoquinoléin-7-yloxy]-propyl}-pipérazin-1-yl)-méthanone;
la cyclopropyl-(4-{3-[4-(4-méthoxy-phényl)-2-méthyl-1,2,3,4-tétrahydro-isoquinoléin-7-yloxy]-propyl}-pipérazin-1-yl)-méthanone;
la 7-[3-(4,4-difluoro-pipéridin-1-yl)-propoxy]-4-(4-méthoxy-phényl)-2-méthyl-1,2,3,4-tétrahydro-isoquinoléine;
la 4-(4-méthoxy-phényl)-2-méthyl-7-(3-morpholin-4-yl-propoxy)-1,2,3,4-tétrahydro-isoquinoléine;
la (1-{3-[4-(4-méthoxy-phényl)-2-méthyl-1,2,3,4-tétrahydro-isoquinoléin-7-yloxy]-propyl}-pyrrolidin-3-yl)-diméthyl-amine;
la 7-[3-((2R,5R)-trans-diméthyl-pyrrolidin-1-yl)-propoxy]-4-(4-méthoxy-phényl)-2-méthyl-1,2,3,4-tétrahydro-isoquinoléine;
l'ester d'éthyle d'acide 4-{3-[4-(4-méthoxy-phényl)-2-méthyl-1,2,3,4-tétrahydro-isoquinoléin-7-yloxy]-propyl}-pipérazine-1-carboxylique;
la (4-{3-[4-(4-méthoxy-phényl)-2-méthyl-1,2,3,4-tétrahydro-isoquinoléin-7-yloxy-propyl}-pipérazin-1-yl)-(1-méthyl-1H-pyrrol-2-yl)-méthanone;
la (4-{3-[4-(4-méthoxy-phényl)-2-méthyl-1,2,3,4-tétrahydro-isoquinoléin-7-yloxy]propyl}-pipérazin-1-yl)-(1-méthyl-1H-imidazol-4-yl)-méthanone;
la (1,3-diméthyl-tétrahydro-pyrimidin-2-ylidène)-{3-[4-(4-méthoxy-phényl)-2-méthyl-1,2,3,4-tétrahydro-isoquinoléin-7-yloxy]-propyl}-amine;
la 7-[3-(1,3-dihydro-isoindol-2-yl)-propoxy]-4-(4-méthoxy-phényl)-2-méthyl-1,2,3,4-tétrahydro-isoquinoléine;
la bis-(2-méthoxy-éthyl)-{3-[4-(4-méthoxy-phényl)-2-méthyl-1,2,3,4-tétrahydro-isoquinoléin-7-yloxy]-propyl}-amine;
la 7-[3-(5,6-dihydro-4H-pyrimidin-1-yl)-propoxy]-4-(4-méthoxy-phényl)-2-méthyl-1,2,3,4-tétrahydro-isoquinoléine;
la benzothiazol-2-yl-{3-[4-(4-méthoxy-phényl)-2-méthyl-1,2,3,4-tétrahydro-isoquinoléin-7-yloxy]-propyl}-méthyl-amine;
le 1-{3-[4-(4-méthoxy-phényl)-2-méthyl-1,2,3,4-tétrahydro-isoquinoléin-7-yloxy]-propyl}-pipéridin-3-ol;
le 1-{3-[4-(4-méthoxy-phényl)-2-méthyl-1,2,3,4-tétrahydro-isoquinoléin-7-yloxy]-propyl}-pipéridin-4-ol;
le (1-{3-[4-(4-méthoxy-phényl)-2-méthyl-1,2,3,4-tétrahydro-isoquinoléin-7-yloxy]-propyl}-pipéridin-4-yl)-méthanol;
le (1-{3-[4-(4-méthoxy-phényl)-2-méthyl-1,2,3,4-tétrahydro-isoquinoléin-7-yloxy]-propyl}-pipéridin-3-yl)-méthanol;
le (1-{3-[4-(4-méthoxy-phényl)-2-méthyl-1,2,3,4-tétrahydro-isoquinoléin-7-yloxy]-propyl}-pipéridin-2-yl)-méthanol;
la 4-(4-méthoxy-phényl)-2-méthyl-7-[3-(3-trifluorométhyl-pipéridin-1-yl)-propoxy]-1,2,3,4-tétrahydro-isoquinoléine;
le 2-(1-{3-[4-(4-méthoxy-phényl)-2-méthyl-1,2,3,4-tétrahydro-isoquinoléin-7-yloxy]-propyl}-pipéridin-4-yl)-éthanol;
la 7-[3-(1,1-dioxo-1λ⁶-thiomorpholin-4-yl)-propoxy]-4-(4-méthoxy-phényl)-2-méthyl-1,2,3,4-tétrahydro-isoquinoléine;
la 4-(4-méthoxy-phényl)-2-méthyl-7-[3-((2S)-trifluorométhyl-pyrrolidin-1-yl)-propoxy]-1,2,3,4-tétrahydro-isoquinoléine;
la 7-[3-(3,3-difluoro-pipéridin-1-yl)-propoxy]-4-[4-méthoxy-phényl)-2-méthyl-1,2,3,4-tétrahydro-isoquinoléine;
la 7-[3-(3,3-difluoro-pipéridin-1-yl)-propoxy]-4-(4-méthoxy-phényl)-2-méthyl-1,2,3,4-tétrahydro-isoquinoléine (énantiomère 1);
la 7-[3-(3,3-difluoro-pipéridin-1-yl)-propoxy]-4-(4-méthoxy-phényl)-2-méthyl-1,2,3,4-tétrahydro-isoquinoléine (énantiomère 2);
le (1-{3-[4-(4-méthoxy-phényl)-2-méthyl-1,2,3,4-tétrahydro-isoquinoléin-7-yloxy]-propyl}-pyrrolidin-(2R)-yl)-méthanol;
le 1-{3-[4-(4-méthoxy-phényl)-2-méthyl-1,2,3,4-tétrahydro-isoquinoléin-7-yloxy]-propyl}-(R)-pyrrolidin-3-ol;
la 7-[3-(2,6-diméthyl-morpholin-4-yl)-propoxy]-4-(4-méthoxy-phényl)-2-méthyl-1,2,3,4-tétrahydro-isoquinoléine;
l'ester d'éthyle d'acide 1-{3-[4-(4-méthoxy-phényl)-2-méthyl-1,2,3,4-tétrahydro-isoquinoléin-7-yloxy]-propyl}-pipéridine-4-carboxylique;
la 7-(3-azétidin-1-yl-propoxy)-4-(4-méthoxy-phényl)-2-méthyl-1,2,3,4-tétrahydro-isoquinoléine;
la 4-(4-méthoxy-phényl)-2-méthyl-7-[3-(2-méthyl-pyrrolidin-1-yl)-propoxy]-1,2,3,4-tétrahydro-isoquinoléine;
le 2-(1-[3-{4-(4-méthoxy-phényl)-2-méthyl-1,2,3,4-tétrahydro-isoquinoléin-7-yloxy]-propyl}-pipéridin-2-yl)-éthanol;
le 1-{3-[4-(4-méthoxy-phényl)-2-méthyl-1,2,3,4-tétrahydro-isoquinoléin-7-yloxy]-propyl}-pipéridine-4-carbonitrile;
le 1-{3-[4-(4-méthoxy-phényl)-2-méthyl-1,2,3,4-tétrahydro-isoquinoléin-7-yloxy]-propyl}-pipéridine-4-carbonitrile (énantiomère 1);
le 1-{3-[4-(4-méthoxy-phényl)-2-méthyl-1,2,3,4-tétrahydro-isoquinoléin-7-yloxy]propyl}-pipéridine-4-carbonitrile (énantiomère 2);
la 4-(4-méthoxy-phényl)-2-méthyl-7-[3-(4-trifluorométhyl-pipéridin-1-yl)-propoxy]-1,2,3,4-tétrahydro-isoquinoléine;
la 7-[3-(3-fluoro-pipéridin-1-yl)-propoxy]-4-(4-méthoxy-phényl)-2-méthyl-1,2,3,4-tétrahydro-isoquinoléine;
l'éthyl-(2-méthoxy-éthyl)-{3-[4-(4-méthoxy-phényl)-2-méthyl-1,2,3,4-tétrahydro-isoquinoléin-7-yloxy]-propyl}-amine;
la 7-[3-(1,4-dioxa-8-aza-spiro[4.5]déc-8-yl)-propoxy]-4-(4-méthoxy-phényl)-2-méthyl-1,2,3,4-tétrahydro-isoquinoléine;
le 1-[4-(4-méthoxy-phényl)-2-méthyl-1,2,3,4-tétrahydro-isoquinoléin-7-yloxy]-3-pipéridin-1-yl-propan-2-ol;
la 7-[2-fluoro-3-(4-fluoro-pipéridin-1-yl)-propoxy]-4-(4-méthoxy-phényl)-2-méthyl-1,2,3,4-tétrahydro-isoquinoléine;
la 7-[3-(4-fluoro-pipéridin-1-yl)-propoxy]-4-(3-méthoxy-phényl)-2-méthyl-1,2,3,4-tétrahydro-isoquinoléine;
la 7-[3-(4-fluoro-pipéridin-1-yl)-propoxy]-4-(3-méthoxy-phényl)-2-méthyl-1,2,3,4-tétrahydro-isoquinoléine (énantiomère 1);
la 7-[3-(4-fluoro-pipéridin-1-yl)-propoxy]-4-(3-méthoxy-phényl)-2-méthyl-1,2,3,4-tétrahydro-isoquinoléine (énantiomère 2);
la 4-(3-chloro-phényl)-7-[3-(4-fluoro-pipéridin-1-yl)-propoxy]-2-méthyl-1,2,3,4-tétrahydro-isoquinoléine;
la 4-(4-chloro-phényl)-7-[3-(4-fluoro-pipéridin-1-yl)-propoxy]-2-méthyl-1,2,3,4-tétrahydro-isoquinoléine;
la 4-(3-fluoro-phényl)-7-[3-(4-fluoro-pipéridin-1-yl)-propoxy]-2-méthyl-1,2,3,4-tétrahydro-isoquinoléine;
la 7-[3-(4-fluoro-pipéridin-1-yl)-propoxy]-2-méthyl-4-(4-trifluorométhoxy-phényl)-1,2,3,4-tétrahydro-isoquinoléine;
la 4-(4-difluorométhoxy-phényl)-7-[3-(4-fluoro-pipéridin-1-yl)-propoxy]-2-méthyl-1,2,3,4-tétrahydro-isoquinoléine;
la 7-[3-(4-fluoro-pipéridin-1-yl)-propoxy]-4-(4-méthanesulfonyl-phényl)-2-méthyl-1,2,3,4-tétrahydro-isoquinoléine;
la 7-[3-(4-fluoro-pipéridin-1-yl)-propoxy]2-méthyl-4-(4-méthylsulfanyl-phényl)-1,2,3,4-tétrahydro-isoquinoléine;
la 4-(3-chloro-4-méthoxy-phényl)-7-[3-(4-fluoro-pipéridin-1-yl)-propoxy]-2-méthyl-1,2,3,4-tétrahydro-isoquinoléine;
la 4-(2,4-dichloro-phényl)-7-[3-(4-fluoro-pipéridin-1-yl)-propoxy]-2-méthyl-1,2,3,4-tétrahydro-isoquinoléine;
la 4-(2,5-dichloro-phényl)-7-[3-(4-fluoro-pipéridin-1-yl)-propoxy]-2-méthyl-1,2,3,4-tétrahydro-isoquinoléine;
la 4-(3,5-dichloro-phényl)-7-[3-(4-fluoro-pipéridin-1-yl)-propoxy]-2-méthyl-1,2,3,4-tétrahydro-isoquinoléine;
la 2-méthyl-7-(3-pipéridin-1-yl-propoxy)-4-thiophén-3-yl-1,2,3,4-tétrahydro-isoquinoléine;
la 4-(3,4-dichloro-phényl)-7-[3-(4-fluoro-pipéridin-1-yl)-propoxy]-2-méthyl-1,2,3,4-tétrahydro-isoquinoléine;
la 2-méthyl-7-(3-pipéridin-1-yl-propoxy)-4-pyridin-3-yl-1,2,3,4-tétrahydro-isoquinoléine;
la 2-méthyl-7-(3-pipéridin-1-yl-propoxy)-4-(trifluorométhyl-phényl)-1,2,3,4-tétrahydro-isoquinoléine;
la 4-(4-méthoxy-phényl)-2-méthyl-7-(3-pipéridin-1-yl-propoxy)-1,2,3,4-tétrahydro-isoquinoléine (racémique);
la 4-(4-méthoxy-phényl)-2-méthyl-7-(3-pipéridin-1-yl-propoxy)-1,2,3,4-tétrahydro-isoquinoléine (énantiomère 1);
la 4-(4-méthoxy-phényl)-2-méthyl-7-(3-pipéridin-1-yl-propoxy)-1,2,3,4-tétrahydro-isoquinoléine (énantiomère 2);
la 2-tert-butyl-4-(4-méthoxy-phényl)-7-(3-pipéridin-1-yl-propoxy)-1,2,3,4-tétrahydro-isoquinoléine;
la 2-benzyl-4-(4-méthoxy-phényl)-7-(3-pipéridin-1-yl-propoxy)-1,2,3,4-tétrahydro-isoquinoléine;
la 2-éthyl-4-(4-méthoxy-phényl)-7-(3-pipéridin-1-yl-propoxy)-1,2,3,4-tétrahydro-isoquinoléine;
la 4-(4-méthoxy-phényl)-7-(3-pipéridin-1-yl-propoxy)-2-propyl-1,2,3,4-tétrahydro-isoquinoléine;
la 2-isopropyl-4-(4-méthoxy-phényl)-7-(3-pipéridin-1-yl-propoxy)-1,2,3,4-tétrahydro-isoquinoléine;
la 4-(4-méthoxy-phényl)-7-(3-pipéridin-1-yl-propoxy)-1,2,3,4-tétrahydro-isoquinoléine;
le 3-[4-(4-méthoxy-phényl)-7-(3-pipéridin-1-yl-propoxy)-3,4-dihydro-1H-isoquinoléin-2-yl]-propan-1-ol;
le 2-[4-(4-méthoxy-phényl)-7-(3-pipéridin-1-yl-propoxy)-3,4-dihydro-1H-isoquinoléin-2-yl]-éthanol;
la 2-(2-fluoro-éthyl)-4-(4-méthoxy-phényl)-7-(3-pipéridin-1-yl-propoxy)-1,2,3,4-tétrahydro-isoquinoléine;
la 2-cyclopropyl-4-(4-méthoxy-phényl)-7-(3-pipéridin-1-yl-propoxy)-1,2,3,4-tétrahydro-isoquinoléine;
la 4-(4-méthoxy-phényl)-7-(3-morpholin-4-yl-propoxy)-2-(1-phényl-éthyl)-1,2,3,4-tétrahydro-isoquinoléine;
la 4-(4-méthoxy-phényl)-7-(3-morpholin-4-yl-propoxy)-2-(1-phényl-éthyl)-1,2,3,4-tétrahydro-isoquinoléine (diastéréoisomère 1);
la 4-(4-méthoxy-phényl)-7-(3-morpholin-4-yl-propoxy)-2-(1-phényl-éthyl)-1,2,3,4-tétrahydro-isoquinoléine (diastéréoisomère 2);
la 4-(3,4-dichloro-phényl)-2-méthyl-7-(3-pipéridin-1-yl-propoxy)-1,2,3,4-tétrahydro-isoquinoléine;
la 4-(3,4-dichloro-phényl)-2-méthyl-7-(3-morpholin-4-yl-propoxy)-1,2,3,4-tétrahydro-isoquinoléine;
la 4-(4-méthoxy-phényl)-2-méthyl-7-(pipéridin-4-ylméthoxy)-1,2,3,4-tétrahydro-isoquinoléine;
la 4-(3-chloro-4-méthoxy-phényl)-2-méthyl-7-(pipéridin-4-ylméthoxy)-1,2,3,4-tétrahydro-isoquinoléine;
la 2-méthyl-4-(4-méthylsulfanyl-phényl)-7-(pipéridin-4-ylméthoxy)-1,2,3,4-tétrahydro-isoquinoléine;
la 4-(3-fluoro-4-méthoxy-phényl)-2-méthyl-7-(pipéridin-4-ylméthoxy)-1,2,3,4-tétrahydro-isoquinoléine;
la 4-(2-fluoro-4-méthoxy-phényl)-2-méthyl-7-(pipéridin-4-ylméthoxy)-1,2,3,4-tétrahydro-isoquinoléine;
la 7-(1-isopropyl-pipéridin-4-ylméthoxy)-4-(4-méthoxy-phényl)-2-méthyl-1,2,3,4-tétrahydro-isoquinoléine;
la 4-(4-méthoxy-phényl)-2-méthyl-7-(1-méthyl-pipéridin-4-ylméthoxy)-1,2,3,4-tétrahydro-isoquinoléine;
la 4-(4-méthoxy-phényl)-2-méthyl-7-(1-propyl-pipéridin-4-ylméthoxy)-1,2,3,4-tétrahydro-isoquinoléine;
la 7-(1-cyclopentyl-pipéridin-4-ylméthoxy)4-(4-méthoxy-phényl)-2-méthyl-1,2,3,4-tétrahydro-isoquinoléine;
la 7-(1-éthyl-pipéridin-4-ylméthoxy)-4-(4-méthoxy-phényl)-2-méthyl-1,2,3,4-tétrahydro-isoquinoléine;
la 4-(3-chloro-4-méthoxy-phényl)-7-(1-isopropyl-pipéridin-4-ylméthoxy)-2-méthyl-1,2,3,4-tétrahydro-isoquinoléine;
la 4-(3-fluoro-4-méthoxy-phényl)-7-(1-isopropyl-pipéridin-4-ylméthoxy)-2-méthyl-1,2,3,4-tétrahydro-isoquinoléine;
la 4-(2-fluoro-4-méthoxy-phényl)-7-(1-isopropyl-pipéridin-4-ylméthoxy)-2-méthyl-1,2,3,4-tétrahydro-isoquinoléine;
la 7-(1-isopropyl-pipéridin-4-ylméthoxy)-4-(3-méthoxy-phényl)-2-méthyl-1,2,3,4-tétrahydro-isoquinoléine;
la 4-(3-méthoxy-phényl)-2-méthyl-7-(1-méthyl-pipéridin-4-ylméthoxy)-1,2,3,4-tétrahydro-isoquinoléine;
la 4-(3-méthoxy-phényl)-2-méthyl-7-(1-propyl-pipéridin-4-ylméthoxy)-1,2,3,4-tétrahydro-isoquinoléine;
la 7-(1-cyclopentyl-pipéridin-4-ylméthoxy)-4-(3-méthoxy-phényl)-2-méthyl-1,2,3,4-tétrahydro-isoquinoléine;
la 7-(1-éthyl-pipéridin-4-ylméthoxy)-4-(3-méthoxy-phényl)-2-méthyl-1,2,3,4-tétrahydro-isoquinoléine;
la 4-(3-méthoxy-phényl)-2-méthyl-7-(pipéridin-4-yloxy)-1,2,3,4-tétrahydro-isoquinoléine;
la 4-(3-méthoxy-phényl)-2-méthyl-7-(1-méthyl-pipéridin-4-yloxy)-1,2,3,4-tétrahydro-isoquinoléine;
la 7-(1-isopropyl-pipéridin-4-yloxy)-4-(3-méthoxy-phényl)-2-méthyl-1,2,3,4-tétrahydro-isoquinoléine;
la 7-(1-cyclobutyl-pipéridin-4-yloxy)-4-(3-méthoxy-phényl)-2-méthyl-1,2,3,4-tétrahydro-isoquinoléine;
la 7-(1-éthyl-pipéridin-4-yloxy)-4-(3-méthoxy-phényl)-2-méthyl-1,2,3,4-tétrahydro-isoquinoléine;
la 7-(1-cyclopentyl-pipéridin-4-yloxy)-4-(3-méthoxy-phényl)-2-méthyl-1,2,3,4-tétrahydro-isoquinoléine;
la 4-(3-méthoxy-phényl)-2-méthyl-7-(1-propyl-pipéridin-4-yloxy)-1,2,3,4-tétrahydro-isoquinoléine;
la 7-(1-isopropyl-pipéridin-4-ylméthoxy)-2-méthyl-4-(4-méthylsulfanyl-phényl)-1,2,3,4-tétrahydro-isoquinoléine;
la 7-(1-cyclobutyl-pipéridin-4-ylméthoxy)-2-méthyl-4-(4-méthylsulfanyl-phényl)-1,2,3,4-tétrahydro-isoquinoléine;
la 7-(1-éthyl-pipéridin-4-ylméthoxy)-2-méthyl-4-(4-méthylsulfanyl-phényl)-1,2,3,4-tétrahydro-isoquinoléine;
la 4-(3-chloro-4-méthoxy-phényl)-7-(1-cyclobutyl-pipéridin-4-ylméthoxy)-2-méthyl-1,2,3,4-tétrahydro-isoquinoléine;
la 4-(3-chloro-4-méthoxy-phényl)-7-(1-éthyl-pipéridin-4-ylméthoxy)-2-méthyl-1,2,3,4-tétrahydro-isoquinoléine la 4-(3-chloro-4-méthoxy-phényl)-2-méthyl-7-(1-propyl-pipéridin-4-ylméthoxy)-1,2,3,4-tétrahydro-isoquinoléine;
la 2-méthyl-4-(4-méthylsulfanyl-phényl)-7-(1-propyl-pipéridin-4-ylméthoxy)-1,2,3,4-tétrahydro-isoquinoléine;
la 7-(1-isobutyl-pipéridin)-4-ylméthoxy)-4-(4-méthoxy-phényl)-2-méthyl-1,2,3,4-tétrahydro-isoquinoléine;
la 7-(1-cyclobutyl-pipéridin-4-ylméthoxy)-4-(3-fluoro-4-méthoxy-phényl)-2-méthyl-1,2,3,4-tétrahydro-isoquinoléine;
la 4-(3-fluoro-4-méthoxy-phényl)-2-méthyl-7-(1-propyl-pipéridin-4-ylméthoxy)-1,2,3,4-tétrahydro-isoquinoléine;
la 7-(1-cyclobutyl-pipéridin-4-ylméthoxy)-4-(2-fluoro-4-méthoxy-phényl)-2-méthyl-1,2,3,4-tétrahydro-isoquinoléine;
la 4-(2-fluoro-4-méthoxy-phényl)-2-méthyl-7-(1-propyl-pipéridin-4-ylméthoxy)-1,2,3,4-tétrahydro-isoquinoléine;
la 4-(2-fluoro-4-méthoxy-phényl)-7-(1-isobutyl-pipéridin-4-ylméthoxy)-2-méthyl-1,2,3,4-tétrahydro-isoquinoléine;
la 7-[1-(2-fluoro-éthyl)-pipéridin-4-ylméthoxyl-4-(4-méthoxy-phényl)-2-méthyl-1,2,3,4-tétrahydro-isoquinoléine;
la 7-(1-isopropyl-pipéridin-4-yloxy)-4-(4-méthoxy-phényl)-2-méthyl-1,2,3,4-tétrahydro-isoquinoléine;
la 7-(1-isopropyl-pipéridin-4-yloxy)-2-méthyl-4-(4-méthylsulfanyl-phényl)-1,2,3,4-tétrahydro-isoquinoléine;
la 4-(2-fluoro-4-méthoxy-phényl)-7-(1-isopropyl-pipéridin-4-yloxy)-2-méthyl-1,2,3,4-tétrahydro-isoquinoléine;
la 4-(3-fluoro-4-méthoxy-phényl)-7-(1-isopropyl-pipéridin-4-yloxy)-2-méthyl-1,2,3,4-tétrahydro-isoquinoléine;
la 4-(4-méthoxy-phényl)-2-méthyl-7-[1-(tétrahydro-pyran-4-yl)-pipéridin-4-yloxy]-1,2,3,4-tétrahydro-isoquinoléine;
la 2,2,2-trifluoro-1-{4-[4-(4-méthoxy-phényl)-2-méthyl-1,2,3,4-tétrahydro-isoquinoléin-7-yloxyméthyl]-pipéridin-1-yl}-éthanone;
la 4-(4-méthoxy-phényl)-2-méthyl-7-[1-(2,2,2-trifluoro-éthyl)-pipéridin-4-ylméthoxy]-1,2,3,4-tétrahydro-isoquinoléine;
la 7-[3-(4-fluoro-pipéridin-1-yl)-propoxy]-2-méthyl-4-phényl-1,2,3,4-tétrahydro-isoquinoléine;
la 4-(2-fluoro-phényl)-7-[3-(4-fluoro-pipéridin-1-yl)-propoxy]-2-méthyl-1,2,3,4-tétrahydro-isoquinoléine;
la 7-[3-(4-fluoro-pipéridin-1-yl)-propoxy]-2-méthyl-4-p-tolyl-1,2,3,4-tétrahydro-isoquinoléine;
la 2-benzyl-7-[3-(4-fluoro-pipéridin-1-yl)-propoxy]-4-(4-méthoxy-phényl)-1,2,3,4-tétrahydro-isoquinoléine;
la 7-[3-(4-fluoro-pipéridin-1-yl)-propoxy]-2-méthyl-4-(3-trifluorométhoxy-phényl)-1,2,3,4-tétrahydro-isoquinoléine;
la 7-[3-(3,3-difluoro-pyrrolidin-1-yl)-propoxy]-4-(4-méthoxy-phényl)-2-méthyl-1,2,3,4-tétrahydro-isoquinoléine;
la 4-(4-méthoxy-phényl)-2-méthyl-7-[3-(3S-méthyl-morpholin-4-yl)-propoxy]-1,2,3,4-tétrahydro-isoquinoléine;
la dicyclopropylméthyl-{3-[4-(4-méthoxy-phényl)-2-méthyl-1,2,3,4-tétrahydro-isoquinoléine-7-yloxy]-propyl}-amine;
la 4-(2-chloro-phényl)-7-[3-(4-fluoro-pipéridin-1-yl)-propoxy]-2-méthyl-1,2,3,4-tétrahydro-isoquinoléine;
la 2-méthyl-7-[3-(3S-méthyl-morpholin-4-yl)-propoxy]-4(4-morpholin-4-yl-phényl)-1,2,3,4-tétrahydro-isoquinoléine;
la 7-[3-(4-fluoro-pipéridin-1-yl)-propoxy]-2-méthyl-4-(4-morpholin-4-yl-phényl)-1,2,3,4-tétrahydro-isoquinoléine;
le 4-{2-méthyl-7-[3-(3S-méthyl-morpholin-4-yl)-propoxy]-1,2,3,4-tétrahydro-isoquinoléin-4-yl)-benzonitrile;
le 4-{7-[3-(4-fluoro-pipéridin-1-yl)-propoxy]-2-méthyl-1,2,3,4-tétrahydro-isoquinoléin-4-yl}-benzonitrile;
la 7-[3-(3-benzohydryl-azétidin-1-yl)-propoxy]-4-(4-méthoxy-phényl)-2-méthyl-1,2,3,4-tétrahydro-isoquinoléine;
la 2-méthyl-4-(4-méthylsulfanyl-phényl)-7-(3-morpholin-4-yl-propoxy)-1,2,3,4-tétrahydro-isoquinoléine;
la 4-(2-fluoro-4-méthoxy-phényl)-7-[3-(4-fluoro-pipéridin-1-yl)-propoxy]-2-méthyl-1,2,3,4-tétrahydro-isoquinoléine (racémate);
la 4-(2-fluoro-4-méthoxy-phényl}-7-[3-(4-fluoro-pipéridin-1-yl)-propoxy]-2-méthyl-1,2,3,4-tétrahydro-isoquinoléine (énantiomère 1);
la 4-(2-fluoro-4-méthoxy-phényl)-7-[3-(4-fluoro-pipéridin-1-yl)-propoxy]-2-méthyl-1,2,3,4-tétrahydro-isoquinoléine (énantiomère 2);
la 4-(3-fluoro-4-méthoxy-phényl)-7-[3-(4-fluoro-pipéridin-1-yl)-propoxy]-2-méthyl-1,2,3,4-tétrahydro-isoquinoléine (racémate);
la 4-(3-fluoro-4-méthoxy-phényl)-7-[3-(4-fluoro-pipéridin-1-yl)-propoxy]-2-méthyl-1,2,3,4-tétrahydro-isoquinoléine (énantiomère 1);
la 4-(3-fluoro-4-méthoxy-phényl)-7-[3-(4-fluoro-pipéridin-1-yl)-propoxy]-2-méthyl-1,2,3,4-tétrahydro-isoquinoléine (énantiomère 2);
la 4-(4-éthoxy-phényl)-7-[3-(4-fluoro-pipéridin-1-yl)-propoxy]-2-méthyl-1,2,3,4-tétrahydro-isoquinoléine;
la 2-éthyl-7-[3-(4-fluoro-pipéridin-1-yl)-propoxy]-4-(4-méthoxy-phényl)-1,2,3,4-tétrahydro-isoquinoléine;
la 7-[3-(4-fluoro-pipéridin-1-yl)-propoxy]-4-(4-méthoxy-phényl)-1,2,3,4-tétrahydro-isoquinoléine;
la 4-(4-méthoxy-phényl)-2-méthyl-7-(pipéridin-4-yloxy)-1,2,3,4-tétrahydro-isoquinoléine;
la 2-méthyl-4-(4-méthylsulfanyl-phényl)-7-(pipéridin-4-yloxy)-1,2,3,4-tétrahydro-isoquinoléine;
la 4-(2-fluoro-4-méthoxy-phényl)-2-méthyl-7-(pipéridin-4-yloxy)-1,2,3,4-tétrahydro-isoquinoléine;
la 4-(3-fluoro-4-méthoxy-phényl)-2-méthyl-7-(pipéridin-4-yloxy)-1,2,3,4-tétrahydro-isoquinoléine;
la 7-[1-(2-fluoro-éthyl)-pipéridin-4-yloxy]-4-(4-méthoxy-phényl)-2-méthyl-1,2,3,4-tétrahydro-isoquinoléine;
la 7-[3-(4-fluoro-pipéridin-1-yl)-propoxy]-2-méthyl-4-(4-méthylsulfanyl-phényl)-1,2,3,4-tétrahydro-isoquinoléine (énantiomère 1);
la 7-[3-(4-fluoro-pipéridin-1-yl)-propoxy]-2-méthyl-4-(4-méthylsulfanyl-phényl)-1,2,3,4-tétrahydro-isoquinoléine (énantiomère 2);
la 4-(4-méthoxy-phényl)-2-méthyl-7-[3-(3S-méthyl-morpholin-4-yl)-propoxy]-1,2,3,4-tétrahydro-isoquinoléine (énantiomère 1);
la 4-(4-méthoxy-phényl)-2-méthyl-7-[3-(3S-méthyl-morpholin-4-yl)-propoxy]-1,2,3,4-tétrahydro-isoquinoléine (énantiomère 2);
la 2-méthyl-4-(4-méthylsulfanyl-phényl)-7-(3-morpholin-4-yl-propoxy)-1,2,3,4-tétrahydro-isoquinoléine (énantiomère 1) ;
la 2-méthyl-4-(4-méthylsulfanyl-phényl)-7-(3-morpholin-4-yl-propoxy)-1,2,3,4-tétrahydro-isoquinoléine (énantiomère 2);
la 7-[3-(4-fluoro-pipéridin-1-yl)-propoxy]-4-(4-méthylsulfanyl-phényl)-1,2,3,4-tétrahydro-isoquinoléine;
la 4-(4-méthylsulfanyl-phényl)-7-(3-morpholin-4-yl-propoxy)-1,2,3,4-tétrahydro-isoquinoléine;
la 7-[3-(3S-méthyl-morpholin-4-yl)-propoxy]-4-(4-méthylsulfanyl-phényl)-1,2,3,4-tétrahydro-isoquinoléine;
la 4-(4-méthoxy-phényl)-7-(3-morpholin-4-yl-propoxy)-1,2,3,4-tétrahydro-isoquinoléine;
la 7-[3-(4-fluoro-pipéridin-1-yl)-propoxy]-4-(4-méthanesulfinyl-phényl)-2-méthyl-1,2,3,4-tétrahydro-isoquinoléine;
la 4-(4-méthanesulfinyl-phényl)-2-méthyl-7-(3-morpholin-4-yl-propoxy)-1,2,3,4-tétrahydro-isoquinoléine;
la 4-(4-méthanesulfinyl-phényl)-2-méthyl-7-(3-morpholin-4-yl-propoxy)-1,2,3,4-tétrahydro-isoquinoléine (énantiomère 1);
la 4-(4-méthanesulfonyl-phényl)-2-méthyl-7-(3-morpholin-4-yl-propoxy)-1,2,3,4-tétrahydro-isoquinoléine;
la 7-[3-(4-fluoro-pipéridin-1-yl)-propoxy]-4-(4-méthoxy-phényl)-2,6-diméthyl-1,2,3,4-tétrahydro-isoquinoléine;
la 7-[3-(4-fluoro-pipéridin-1-yl)-propoxy]-4-(4-méthoxy-phényl)-2,8-diméthyl-1,2,3,4-tétrahydro-isoquinoléine;
le 7-[3-(4-fluoro-pipéridin-1-yl)-propoxy]-4-(4-méthoxy-phényl)-2-méthyl-1,2,3,4-tétrahydro-isoquinoléin-6-ol;
le 7-[3-(4-fluoro-pipéridin-1-yl)-propoxy]-4-(4-méthoxy-phényl)-2-méthyl-1,2,3,4-tétrahydro-isoquinoléin-8-ol;
la 2,8-diméthyl-4-(4-méthylsulfanyl-phényl)-7-(3-morpholin-4-yl-propoxy)-1,2,3,4-tétrahydro-isoquinoléine;
la 2,6-diméthyl-4-(4-méthylsulfanyl-phényl)-7-(3-morpholin-4-yl-propoxy)-1,2,3,4-tétrahydro-isoquinoléine;
le 2-méthyl-4-(4-méthylsulfanyl-phényl)-7-(3-morpholin-4-yl-propoxy)-1,2,3,4-tétrahydro-isoquinoléin-8-ol;
le 2-méthyl-4-(4-méthylsulfanyl-phényl)-7-(3-morpholin-4-yl-propoxy)-1,2,3,4-tétrahydro-isoquinoléin-6-ol;
le 8-fluoro-2-méthyl-4-(4-méthylsulfanyl-phényl)-7-(3-morpholin-4-yl-propoxy)-1,2,3,4-tétrahydro-isoquinoléine;
la 6-fluoro-2-méthyl-4-(4-méthylsulfanyl-phényl)-7-(3-morpholin-4-yl-propoxy)-1,2,3,4-tétrahydro-isoquinoléine;
la 7-[1-(2-fluoro-éthyl)-pipéridin-4-ylméthoxy]-2-méthyl-4-(4-méthylsulfanyl-phényl)-1,2,3,4-tétrahydro-isoquinoléine;
la 7-[1-(2-fluoro-éthyl)-pipéridin-4-yloxyl-2-méthyl-4-(4-méthylsulfanyl-phényl)-1,2,3,4-tétrahydro-isoquinoléine;
la 4-(4-méthoxyphényl)-7-[3-(3S-méthyl-morpholin-4-yl)-propoxy]-1,2,3,4-tétrahydro-isoquinoléine;
la 4-(4-méthanesulfinyl-phényl)-2-méthyl]-7-[3-(3S-méthyl-morpholin-4-yl)-propoxy]-1,2,3,4-tétrahydro-isoquinoléine;
la 7-[3-(3,3-difluoro-azétidin-1-yl)-propoxy]-4-(4-méthoxy-phényl)-2-méthyl-1,2,3,4-tétrahydro-isoquinoléine;
le (4-{3-[4-(4-méthoxy-phényl)-2-méthyl-1,2,3,4-tétrahydro-isoquinoléin-7-yloxy]-propyl}-morpholin-3-yl)-méthanol;
le (4-{3-[4-(4-méthoxy-phényl)-2-méthyl-1,2,3,4-tétrahydro-isoquinoléin-7-yloxy]-propyl}-morpholin-3S-yl)-méthanol;
le (4-{3-[4-(4-méthoxy-phényl)-2-méthyl-1,2,3,4-tétrahydro-isoquinoléin-7-yloxy]-propyl}-morpholin-2-yl)-méthanol;
la {3-[2-méthyl-4-(4-méthylsulfanyl-phényl)-1,2,3,4-tétrahydro-isoquinoléin-7-yloxy]-propyl}-(2H-pyrazol-3-yl)-amine;
la 4-(6-bromo-pyridin-3-yl)-7-[3-(4-fluoro-pipéridin-1-yl)-propoxy]-2-méthyl-1,2,3,4-tétrahydro-isoquinoléine;
la 7-[3-(4-fluoro-pipéridin-1-yl)-propoxy]-2-méthyl-4-(6-méthylsulfanyl-pyridin-3-yl)-1,2,3,4-tétrahydro-isoquinoléine;
la (5-{7-[3-(4-fluoro-pipéridin-1-yl)-propoxy]-2-méthyl-1,2,3,4-tétrahydro-isoquinoléin-4-yl}-pyridin-2-yl)-diméthyl-amine;
la 7-[3-(4-fluoro-pipéridin-1-yl)-propoxy]-2-méthyl-4-(6-triméthylsilanyléthynyl-pyridin-3-yl)-1,2,3,4-tétrahydro-isoquinoléine;
la 4-(6-éthynyl-pyridin-3-yl)-7-[3-(4-fluoro-pipéridin-1-yl)-propoxy]-2-méthyl-1,2,3,4-tétrahydro-isoquinoléine;
la 7-[3-(4-fluoro-pipéridin-1-yl)-propoxy]-2-méthyl-4-(6-phénylsulfanyl-pyridin-3-yl)-1,2,3,4-tétrahydro-isoquinoléine;
la 7-[3-(4-fluoro-pipéridin-1-yl)-propoxy]-4-(6-imidazol-1-yl-pyridin-3-yl)-2-méthyl-1,2,3,4-tétrahydro-isoquinoléine;
la 7-[3-(4-fluoro-pipéridin-1-yl)-propoxy]-4-(6-méthoxy-pyridin-3-yl)-2-méthyl-1,2,3,4-tétrahydro-isoquinoléine;
la 7-[3-(4-fluoro-pipéridin-1-yl)-propoxy]-2-méthyl-4-(6-pyrazol-1-yl-pyridin-3-yl)-1,2,3,4-tétrahydro-isoquinoléine;
la 7-[3-(4-fluoro-pipéridin-1-yl)-propoxy]-4-[6-(1H-imidazol-2-ylsulfanyl)-pyridin-3-yl)-2-méthyl-1,2,3,4-tétrahydro-isoquinoléine;
la 7-[3-(4-fluoro-pipéridin-1-yl)-propoxy]-2-méthyl-4-[6-(pyrimidin-2-ylsulfanyl)-pyridin-3-yl]-1,2,3,4-tétrahydro-isoquinoléine;
la 4-[4-méthoxy-phényl)-2-méthyl-7-[3-(4-méthyl-pipérazin-1-yl)-propoxy]-1,2,3,4-tétrahydro-isoquinoléine;
la 7-[3-(4-éthyl-pipérazin-1-yl)-propoxy]-4-(4-méthoxy-phényl)-2-méthyl-1,2,3,4-tétrahydro-isoquinoléine;
la 7-[3-(4-isopropyl-pipérazin-1-yl)-propoxy]-4-(4-méthoxy-phényl)-2-méthyl-1,2,3,4-tétrahydro-isoquinoléine;
la 4-(4-méthoxy-phényl)-2-méthyl-7-[3-(4-thiazol-2-yl-pipérazin-1-yl)-propoxy]-1,2,3,4-tétrahydro-isoquinoléine;
la 4-(4-méthoxy-phényl)-2-méthyl-7-[3-(4-thiophén-2-ylméthyl-pipérazin-1-yl)-propoxy]-1,2,3,4-tétrahydro-isoquinoléine;
le 2-(4-{3-[4-(4-méthoxy-phényl)-2-méthyl-1,2,3,4-tétrahydro-isoquinoléin-7-yloxy]-propyl}-pipérazin-1-yl)-éthanol;
le 2-(4-{3-[4-(4-méthoxy-phényl)-2-méthyl-1,2,3,4-tétrahydro-isoquinoléin-7-yloxy]-propyl}-pipérazin-1-yl)-phénol;
la 4-(4-méthoxy-phényl)-2-méthyl-7-[3-(4-pyridin-4-yl-pipérazin-1-yl)-propoxy]-1,2,3,4-tétrahydro-isoquinoléine;
la 4-(4-méthoxy-phényl)-2-méthyl-7-{3-[4-(4-trifluorométhyl-phényl)-pipérazin-1-yl]-propoxy}-1,2,3,4-tétrahydro-isoquinoléine;
la 7-[3-(4-allyl-pipérazin-1-yl)-propoxy]-4-(4-méthoxy-phényl)-2-méthyl-1,2,3,4-tétrahydro-isoquinoléine;
la 7-[3-(4-[1,3]-dioxolan-2-ylméthyl-pipérazin-1-yl)-propoxy]-4-(4-méthoxy-phényl)-2-méthyl-1,2,3,4-tétrahydro-isoquinoléine;
le 4-(4-{3-[4-(4-méthoxy-phényl)-2-méthyl-1,2,3,4-tétrahydro-isoquinoléin-7-yloxy]-propyl}-pipérazin-1-yl)-benzonitrile;
la 7-{3-[4-(2-méthoxy-éthyl)-pipérazin-1-yl]-propoxy}-4-(4-méthoxy-phényl)-2-méthyl-1,2,3,4-tétrahydro-isoquinoléine;
la 4-(4-méthoxy-phényl)-2-méthyl-7-{3-[4-(tétrahydro-furan-2-ylméthyl)-pipérazin-1-yl]-propoxy}-1,2,3,4-tétrahydro-isoquinoléine;
l'ester de tert-butyle d'acide 4-{3-[4-(4-méthoxy-phényl)-2-méthyl-1,2,3,4-tétrahydro-isoquinoléin-7-yloxy]-propyl}-pipérazine-1-carboxylique;
la 7-[3-(4-cyclopropyl-pipérazin-1-yl)-propoxy]-4-(4-méthoxy-phényl)-2-méthyl-1,2,3,4-tétrahydro-isoquinoléine;
la 4-(4-bromo-phényl)-7-[3-(4-fluoro-pipéridin-1-yl)-propoxy]-2-méthyl-1,2,3,4-tétrahydro-isoquinoléine;
la 4-(4-difluorométhoxy-phényl)-2-méthyl-7-(3-morpholin-4-yl-propoxy)-1,2,3,4-tétrahydro-isoquinoléine;
la 2-méthyl-7-[3-(3S-méthyl-morpholin-4-yl)-propoxy]-4-(4-méthylsulfanyl-phényl)-1,2,3,4-tétrahydro-isoquinoléine;
la 7-[3-(4,4-difluoro-pipéridin-1-yl)-propoxy]-2-méthyl-4-(4-méthylsulfanyl-phényl)-1,2,3,4-tétrahydro-isoquinoléine;
la 7-[3-(4-fluoro-pipéridin-1-yl)-propoxy]-2-méthyl-4-(4-trifluorométhylsulfanyl-phényl)-1,2,3,4-tétrahydro-isoquinoléine;
la 2-méthyl-7-(3-morpholin-4-yl-propoxy)-4-(4-trifluorométhylsulfanyl-phényl)-1,2,3,4-tétrahydro-isoquinoléine;
la 2-méthyl-7-[3-(3S-méthyl-morpholin-4-yl)-propoxy]-4-(4-trifluorométhylsulfanyl-phényl)-1,2,3,4-tétrahydro-isoquinoléine;
la 4-(2-fluoro-4-méthoxy-phényl)-2-méthyl-7-[3-(3S-méthyl-morpholin-4-yl)-propoxy]-1,2,3,4-tétrahydro-isoquinoléine;
la 4-(3-fluoro-4-méthoxy-phényl)-2-méthyl-7-[3-(3S-méthyl-morpholin-4-yl)-propoxy]-1,2,3,4-tétrahydro-isoquinoléine;
la 2-méthyl-7-(3-pipéridin-1-yl-propoxy)-4-(4-trifluorométhoxy-phényl)-1,2,3,4-tétrahydro-isoquinoléine;
la {3-[4-(4-méthoxy-phényl)-2-méthyl-1,2,3,4-tétrahydro-isoquinoléin-7-yloxy]-propyl}-(tétrahydro-pyran-4-yl)-amine;
la cyclopropyl-{3-[4-(4-méthoxy-phényl)-2-méthyl-1,2,3,4-tétrahydro-isoquinoléin-7-yloxy]-propyl}-(1-méthyl-pipéridin-4-yl)-amine;
la (2-méthoxy-éthyl)-{3-[4-(4-méthoxy-phényl)-2-méthyl-1,2,3,4-tétrahydro-isoquinoléin-7-yloxy]-propyl}-amine;
le 3-{3-[4-(4-méthoxy-phényl)-2-méthyl-1,2,3,4-tétrahydro-isoquinoléin-7-yloxy]-propylamino}-propan-1-ol;
l'allyl-{3-[4-(4-méthoxy-phényl)-2-méthyl-1,2,3,4-tétrahydro-isoquinoléin-7-yloxy]-propyl}-amine;
l'isobutyl-{3-[4-(4-méthoxy-phényl)-2-méthyl-1,2,3,4-tétrahydro-isoquinoléin-7-yloxy]-propyl}-amine;
la cyclopropyl-{3-[4-(4-méthoxy-phényl)-2-méthyl-1,2,3,4-tétrahydro-isoquinoléin-7-yloxy]-propyl}-amine;
l'isopropyl-{3-[4-(4-méthoxy-phényl)-2-méthyl-1,2,3,4-tétrahydro-isoquinoléin-7-yloxy]-propyl}-amine;
la {3-[4-(4-méthoxy-phényl)-2-méthyl-1,2,3,4-tétrahydro-isoquinoléin-7-yloxy]-propyl}-propyl-amine;
l'éthyl-{3-[4-(4-méthoxy-phényl)-2-méthyl-1,2,3,4-tétrahydro-isoquinoléin-7-yloxy]-propyl}-amine;
l'isopropyl-{3-[4-(4-méthoxy-phényl)-2-méthyl-1,2,3,4-tétrahydro-isoquinoléin-7-yloxy]-propyl}-méthyl-amine;
la cyclopropyl-{3-[4-(4-méthoxy-phényl)-2-méthyl-1,2,3,4-tétrahydro-isoquinoléin-7-yloxy]-propyl}-méthyl-amine;
la dicyclopropyl-{3-[4-(4-méthoxy-phényl)-2-méthyl-1,2,3,4-tétrahydro-isoquinoléin-7-yloxy]-propyl}-amine;
la 7-(1-isopropyl-azétidin-3-ylméthoxy)-2-méthyl-4-(4-méthylsulfanyl-phényl)-1,2,3,4-tétrahydro-isoquinoléine;
la 7-(1-cyclopropyl-azétidin-3-ylméthoxy)-2-méthyl-4-(4-méthylsulfanyl-phényl)-1,2,3,4-tétrahydro-isoquinoléine;
la 7-(1-cyclobutyl-azétidin-3-ylméthoxy)-2-méthyl-4-(4-méthylsulfanyl-phényl)-1,2,3,4-tétrahydro-isoquinoléine;
la 7-[1-(2-fluoro-éthyl)-azétidin-3-ylméthoxy]-2-méthyl-4-(4-méthylsulfanyl-phényl)-1,2,3,4-tétrahydro-isoquinoléine;
la 4-(4-méthoxy-phényl)-2-méthyl-7-[2-(1-méthyl-pyrrolidin-2-yl)-éthoxy]-1,2,3,4-tétrahydro-isoquinoléine;
la 4-(4-méthoxy-phényl)-2-méthyl-7-[2-(1-méthyl-pyrrolidin-2-yl)-éthoxy]-1,2,3,4-tétrahydro-isoquinoléine (diastéréoisomère 1);
la 4-(4-méthoxy-phényl)-2-méthyl-7-[2-(1-méthyl-pyrrolidin-2-yl)-éthoxy]-1,2,3,4-tétrahydro-isoquinoléine (diastéréoisomère 2);
la 4-(3-méthoxy-phényl)-2-méthyl-7-(pipéridin-4-ylméthoxy)-1,2,3,4-tétrahydro-isoquinoléine;
le {4-[4-(4-méthoxy-phényl)-2-méthyl-1,2,3,4-tétrahydro-isoquinoléin-7-yloxyméthyl]-pipéridin-1-yl}-acétonitrile;
la 2-méthyl-7-(1-méthyl-pipéridin-4-yloxy)-4-(4-méthylsulfanyl-phényl)-1,2,3,4-tétrahydro-isoquinoléine;
la 2-méthyl-4-(4-méthylsulfanyl-phényl)-7-[1-(3,3,3-trifluoro-propyl)-pipéridin-4-yloxy]-1,2,3,4-tétrahydro-isoquinoléine;
le {4-[2-méthyl-4-(4-méthylsulfanyl-phényl)-1,2,3,4-tétrahydro-isoquinoléin-7-yloxy]-pipéridin-1-yl}-acétonitrile;
la 2-méthyl-4-(4-méthylsulfanyl-phényl)-7-[1-(tétrahydro-pyran-4-yl)-pipéridin-4-yloxy]-1,2,3,4-tétrahydro-isoquinoléine;
la 7-(1-cyclopropyl-pipéridin-4-yloxy)-2-méthyl-4-(4-méthylsulfanylphényl)-1,2,3,4-tétrahydro-isoquinoléine;
la 7-(1-cyclobutyl-pipéridin-4-ylméthoxy)-4-(4-méthoxy-phényl)-2-méthyl-1,2,3,4-tétrahydro-isoquinoléine;
la 7-(1-cyclopropyl-pipéridin-4-ylméthoxy)-4-(4-méthoxy-phényl)-2-méthyl-1,2,3,4-tétrahydro-isoquinoléine;
le 1-{4-[4-(4-méthoxy-phényl)-2-méthyl-1,2,3,4-tétrahydro-isoquinoléin-7-yloxyméthyl]-pipéridin-1-yl}-2-méthyl-propan-2-ol;
la 4-(4-méthoxy-phényl)-2-méthyl-7-(4-méthyl-morpholin-2-ylméthoxy)-1,2,3,4 tétrahydro-isoquinoléine;
la 4-(4-méthoxy-phényl)-2-méthyl-7-(morpholin-2S-ylméthoxy)-1,2,3,4-tétrahydro-isoquinoléine;
la 4-(4-méthoxy-phényl)-2-méthyl-7-(morpholin-2R-ylméthoxy)-1,2,3,4-tétrahydro-isoquinoléine;
la 7-(4-isopropyl-morpholin-2-ylméthoxy)-4-(4-méthoxy-phényl)-2-méthyl-1,2,3,4-tétrahydro-isoquinoléine;
la 7-(4-isopropyl-morpholin-2S-ylméthoxy)-4-(4-méthoxy-phényl)-2-méthyl-1,2,3,4-tétrahydro-isoquinoléine;
la 7-(4-isopropyl-morpholin-2R-ylméthoxy)-4-(4-méthoxy-phényl)-2-méthyl-1,2,3,4-tétrahydro-isoquinoléine;
la 7-(4-isopropyl-morpholin-2R-ylméthoxy)-4-(4-méthoxy-phényl)-2-méthyl-1,2,3,4-tétrahydro-isoquinoléine (diastéréoisomère 1);
la 7-(4-isopropyl-morpholin-2R-ylméthoxy)-4-(4-méthoxy-phényl)-2-méthyl-1,2,3,4-tétrahydro-isoquinoléine (diastéréoisomère 2)
la 7-(4-éthyl-morpholin-2-ylméthoxy)-4-(4-méthoxy-phényl)-2-méthyl-1,2,3,4-tétrahydro-isoquinoléine;
la 7-[4-(2-fluoro-éthyl)-morpholin-2-ylméthoxy]-4-(4-méthoxy-phényl)-2-méthyl-1,2,3,4-tétrahydro-isoquinoléine;
la 7-(4-cyclopropyl-morpholin-2-ylméthoxy)-4-(4-méthoxy-phényl)-2-méthyl-1,2,3,4-tétrahydro-isoquinoléine;
la 7-(4-cyclopropyl-morpholin-2S-ylméthoxy)-4-(4-méthoxy-phényl)-2-méthyl-1,2,3,4-tétrahydro-isoquinoléine;
la 7-(4-cyclopropyle-morpholin-2S-ylméthoxy)-4-(4-méthoxy-phényl)-2-méthyl-1,2,3,4-tétrahydro-isoquinoléine (diastéréoisomère 1);
la 7-(4-cyclopropyl-morpholin-2S-ylméthoxy)-4-(4-méthoxy-phényl)-2-méthyl-1,2,3,4-tétrahydro-isoquinoléine (diastéréoisomère 2) ;
la 7-(4-cyclopropyl-morpholin-2R-ylméthoxy)-4-(4-méthoxy-phényl)-2-méthyl-1,2,3,4-tétrahydro-isoquinoléine;
la 7-(4-cyclopropyl-morpholin-2R-ylméthoxy)-4-(4-méthoxy-phényl)-2-méthyl-1,2,3,4-tétrahydro-isoquinoléine (diastéréoisomère 1) ;
la 7-(4-cyclopropyl-morpholin-2R-ylméthoxy)-4-(4-méthoxy-phényl)-2-méthyl-1,2,3,4-tétrahydro-isoquinoléine (diastéréoisomère 2) ;
la 7-(4-isopropyl-morpholin-2-ylméthoxy)-2-méthyl-4-(4-méthylsulfanyl-phényl)-1,2,3,4-tétrahydro-isoquinoléine;
la 7-(4-cyclopropyl-morpholin-2-ylméthoxy)-2-méthyl-4-(4-méthylsulfanyl-phényl)-1,2,3,4-tétrahydro-isoquinoléine;
la 2-méthyl-4-(4-méthylsulfanyl-phényl)-7-(morpholin-2-ylméthoxy)-1,2,3,4-tétrahydro-isoquinoléine;
la 4-(4-méthoxy-phényl)-2,6-diméthyl-7-(3-pipéridin-1-yl-propoxy)-1,2,3,4-tétrahydro-isoquinoléine;
la 4-(4-méthoxy-phényl)-2,6-diméthyl-7-(3-morpholin-4-yl-propoxy)-1,2,3,4-tétrahydro-isoquinoléine;
la 4-(3-fluoro-4-méthoxy-phényl)-2,6-diméthyl-7-(3-morpholin-4-yl-propoxy)-1,2,3,4-tétrahydro-isoquinoléine;
la 4-(3-fluoro-4-méthoxy-phényl)-2,8-diméthyl-7-(3-morpholin-4-yl-propoxy)-1,2,3,4-tétrahydro-isoquinoléine;
la 4-(4-méthoxy-phényl)-2,8-diméthyl-7-(3-morpholin-4-yl-propoxy)-1,2,3,4-tétrahydro-isoquinoléine;
la 2,6-diméthyl-4-(4-méthylsulfanyl-phényl)-7-(3-pipéridin-1-yl-propoxy)-1,2,3,4-tétrahydro-isoquinoléine;
la 7-(4-pipéridin-1-yl-but-1-ynyl)-4-pyridin-3-yl-1,2,3,4-tétrahydro-isoquinoléine;
la 7-(4-pipéridin-1-yl-butyl)-4-pyridin-3-yl-1,2,3,4-tétrahydro-isoquinoléine;
la 2-méthyl-7-(4-pipéridin-1-yl-butyl)-4-pyridin-3-yl-1,2,3,4-tétrahydro-isoquinoléine;
la 7-[4-(4,4-difluoro-pipéridin-1-yl)-but-1-ynyl]-4-(4-méthoxy-phényl)-2-méthyl-1,2,3,4-tétrahydro-isoquinoléine;
la 4-(4-méthoxy-phényl)-2-méthyl-7-(4-pipéridin-1-yl-but-1-ynyl)-1,2,3,4-tétrahydro-isoquinoléine;
la 4-(4-méthoxy-phényl)-2-méthyl-7-(4-morpholin-4-yl-but-1-ynyl)-1,2,3,4-tétrahydro-isoquinoléine;
la 4-(4-méthoxy-phényl)-2-méthyl-7-(4-thiomorpholin-4-yl-but-1-ynyl)-1,2,3,4-tétrahydro-isoquinoléine;
la 7-[4-(4-isopropyl-pipérazin-1-yl)-but-1-ynyl)-4-(4-méthoxy-phényl)-2-méthyl-1,2,3,4-tétrahydro-isoquinoléine;
la 4-(4-fluoro-phényl)-2-méthyl-7-(4-pipéridin-1-yl-but-1-ynyl)-1,2,3,4-tétrahydro-isoquinoléine;
la 7-[4-(4,4-difluoro-pipéridin-1-yl)-butyl]-4-(4-méthoxy-phényl)-2-méthyl-1,2,3,4-tétrahydro-isoquinoléine;
la 4-(4-méthoxy-phényl)-2-méthyl-7-(4-morpholin-4-yl-butyl)-1,2,3,4-tétrahydro-isoquinoléine;
la 4-(4-méthoxy-phényl)-2-méthyl-7-(4-thiomorpholin-4-yl-butyl)-1,2,3,4-tétrahydro-isoquinoléine;.
la 7-[4-(4-isopropyl-pipérazin-1-yl)-butyl]-4-(4-méthoxy-phényl)-2-méthyl-1,2,3,4-tétrahydro-isoquinoléine;
et ses sels.

10. Composé de formule (I): 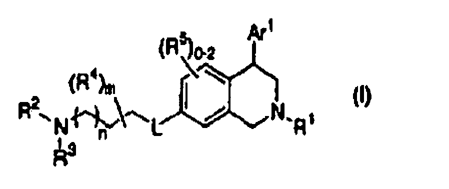 dans laquelle
L est -O- et n vaut 1 ou 2; ou L est -C=C- ou -CH₂CH₂- et n vaut 0 ou 1;
R¹ est -H; ou est un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyle en C_{3 à 7}, -COOalkyle en C_{1 à 6} ou -COObenzyle, chacun facultativement mono-, di- ou tri-substitué par R^{a};
où R^{a} est choisi parmi un groupe -OH, -Oalkyle en C_{1 à 6}, phényle facultativement substitué par un groupe -Oalkyle en C_{1 à 4} ou halogéno, -CN, -NO₂-, -N(R^{b})R^{c}, -C(O)N(R^{b})R^{c}, -N(R^{b})C(O)R^{b}, -N(R^{b})SO₂alkyle en C_{1 à 6,} -C(O)alkyle en C_{1 à 6,} -S(O)_{0 à 2}-alkyle en C_{1 à 6,} -SO₂N(R^{b})R^{c}, -SCF₃, halogéno, -CF₃, -OCF₃, -COOH et -COOalkyle en C_{1 à 6;}
où R^{b} et R^{c} sont chacun indépendamment -H ou un groupe -alkyle en C_{1 à 6;}
R² et R³ sont chacun indépendamment choisis dans le groupe consistant en: -H;
A) un groupe -alkyle en C_{1 à 6,} -alcényle en C_{3 à 6}, -alcynyle en C_{3 à 6}, -cycloalkyle en C_{3 à 7}, -alkyl en C_{1 à 6}-cycloalkyle en C_{3 à 7,} -CH (cycloalkyle en C_{3 à 8})₂, -CH(phényle)₂, benzyle et -C(O)Oalkyle en C_{1 à 4}, où chaque groupe alkyle, cycloalkyle ou benzyle est facultativement substitué par un groupe -OH, -Oalkyle en C_{1 à 4}, -CN, -NH₂, -NH (alkyle en C_{1 à 4}), -N(alkyle en C_{1 à 4})₂, halogéno, -CF₃, -OCF₃, -COOH ou -COOalkyle en C_{1 à 6;}
B) un groupe phényle ou pyridyle, facultativement condensé au niveau de deux chaînons de cycle de carbone adjacents à un fragment hydrocarbure à trois ou quatre chaînons pour former un cycle aromatique condensé à cinq ou six chaînons, lequel fragment a un atome de carbone remplacé par >O, >S, >NH, >N(alkyle en C_{1 à 4}) ou >NC(O)Oalkyle en C_{1 à 4}, et lequel fragment a jusqu'à un atome de carbone additionnel facultativement remplacé par -N=;
C) un groupe naphtyle,
D) un cycle hétérocyclique à 4 à 8 chaînons, ledit cycle hétérocyclique ayant un atome de carbone qui est le point d'attache, comportant 1 ou 2 chaînons hétéroatome choisis dans le groupe consistant en >O, >S(O)_{0 à 2}, >NH, >N(alkyle en C_{1 à 4}), et >NC(O)Oalkyle en C_{1 à 4}, et comportant 0 ou 1 double liaison; et
E) un groupe hydrocarbure aromatique monocyclique comportant cinq ou six atomes de cycle, ayant un atome de carbone qui est le point d'attache, ayant un atome de carbone remplacé par >O, >S, >NH, >N(alkyle en C_{1 à 4}) ou >NC(O)Oalkyle en C_{1 à 4}, ayant jusqu'à un atome de carbone additionnel facultativement remplacé par -N=, et facultativement benzocondensé ou pyridocondensé;
où chacun de B) à E) est facultativement mono-, di- ou tri-substitué par un fragment choisi dans le groupe consistant en un groupe -alkyle en C_{1 à 4}, -OH, -alkyl en C_{1 à 4}OH, -Oalkyle en C_{1 à 6}, -CN, -NO₂, -N(R^{d})R^{e}, -C(O)N(R^{d})R^{e}, -N(R^{d})C(O)R^{d}, -N(R^{d})SO₂alkyle en C_{1 à 6}, -C(O)alkyle en C_{1 à 6,} -S(O)_{0 à} 2-alkyle en C_{1 à 6}, -SO₂N(R^{d})R^{e}, -SCF₃, halogéno, -CF₃, -OCF₃, -COOH, -COOalkyle en C_{1 à 4}, -OC(O)N(R^{d})R^{e} et -OC(O)Oalkyle en C_{1 à 6};
où R^{d} et R^{e} sont chacun indépendamment -H ou un groupe -alkyle en C_{1 à 6};
ou, en variante,
R² et R³ peuvent être pris conjointement avec l'atome d'azote auquel ils sont attachés pour former un groupe tétrahydro-pyrimidin-2-ylidényle, ou un cycle hétérocyclique à 4 à 8 chaînons, ledit cycle hétérocyclique ayant 0 ou 1 chaînon hétéroatome additionnel séparé de l'azote d'attache par au moins un chaînon carbone et choisi dans le groupe consistant en >O, >S(O)_{0 à 2}, >NH et >NR^{f}, ayant 0 ou 1 double liaison, ayant 0, 1 ou 2 chaînons carbone séparés de l'atome d'azote d'attache par au moins un chaînon carbone qui est un groupe carbonyle, facultativement benzo ou pyridocondensé, comportant facultativement un chaînon carbone qui forme un pont, et comportant 0 à 5 substituants de chaînon carbone R^{ff},
où R^{f} est choisi dans le groupe consistant en:
i) un groupe -alkyle en C_{1 à 6} facultativement substitué par R^{z}, -alcényle en C_{3 à 6}, -alcynyle en C_{3 à 6}, -C(O)N(R^{g})R^{h}, -C(O)Rⁱ, -S(O)_{0 à 2}-alkyle en C_{1 à 6} et -COOalkyle en C_{1 à 6},
où R^{z} est un groupe fluoro, -CN, -OH, -Oalkyle en C_{1 à 4}, -cycloalkyle en C_{3 à 7} ou -CF_{3;}
où R^{g} et R^{h} sont chacun indépendamment -H ou -alkyle en C₁ à ₆; et
où Rⁱ est un groupe -alkyle en C_{1 à 6}, -cycloalkyle en C_{3 à 8}, phényle, ou hétérocyclyle aromatique à 5 ou 6 chaînons, où chaque groupe alkyle, cycloalkyle, phényle ou hétérocyclyle est facultativement mono-, di- ou tri-substitué par un groupe -alkyle en C_{1 à 4}, -OH, -Oalkyle en C_{1 à 6}, -CF₃, -CN ou halogéno;
ii) -(CH₂)_{0 à 1}-Cycle A, où Cycle A est un groupe phényle ou un groupe hétérocyclyle aromatique à 5 ou 6 chaînons à liaison carbone, facultativement substitué par R^{aa}_{;}
où R^{aa} est un groupe -OH, -alkyle en C_{1 à 6}, -Oalkyle en C_{1 à 6}, phényle, -CN, -NO₂, -N(R^{g})R^{h}, -C(O)N(R^{g})R^{h}, -(R^{g})C(O)R^{h}, -N(R^{h})SO₂-alkyle en C_{1 à 6}, -C(O) alkyle en C_{1 à 6}, -S(O)_{0 à 2}-alkyle en C_{1 à 6}, -SO₂N(R^{g})R^{h}, -SCF₃, halogéno, -CF₃, -OCF₃, -OCHF₂, -COOH et -COOalkyle en C_{1 à 6}; et
iii) -(CH₂)_{0 à 1}-Cycle B, où Cycle B est un groupe -cycloalkyle en C_{3 à 7} avec un ou deux chaînons de cycle carboné facultativement remplacés par >O ou >NH, et facultativement substitué par un groupe -alkyle en C_{1 à 4}, fluoro, -CN, -OH, -Oalkyle en C_{1 à 4} ou -CF_{3;}
où R^{ff} est choisi dans le groupe consistant en un groupe -alkyle en C_{1 à 6} facultativement mono- ou di-substitué par R^{z}, un groupe -alcényle en C_{2 à 6}, -alcynyle en C_{2 à 6}, -cycloalkyle en C_{3 à 7}, -CH(phényle)₂, halogéno, -OH, -Oalkyle en C_{1 à 6}, -Oalkyl en C_{2 à 3}O-, -CN, -NO₂, -N(R^{g})R^{h}, -C(O)N(R^{g})R^{h}, -N(R^{g})C(O)R^{g}, -N(R^{g})SO₂alkyle en C_{1 à 6}, -C(O)Rⁱ, -S(O)_{0 à 2}-alkyle en C_{1 à 6}, -SO₂N(R^{g})R^{h}, -SCF₃, -CF₃, -OCF₃, -COOH et -COOalkyle en C_{1 à 6};
R⁴ est un groupe -OH, -Oalkyle en C_{1 à 6}, -CF₃, -alkyle en C_{1 à 6} ou halogéno; deux substituants R⁴ peuvent être pris ensemble pour former un groupe méthylène ou éthylène; ou l'un de R⁴ est pris conjointement avec R² pour former un groupe méthylène, éthylène, propylène ou -CH₂CH₂O-, où chacun est facultativement substitué par un groupe -OH, -Oalkyle en C_{1 à 6}, -Salkyle en C_{1 à 6}, -CF₃, -alkyle en C_{1 à 6}, amino ou halogéno;
m vaut 0, 1 ou 2;
R⁵ est choisi dans le groupe consistant en un groupe -alkyle en C_{1 à 6}, -OH, -Oalkyle en C_{1 à 6}, -Salkyle en C_{1 à 6} et halogéno;
Ar¹ est un cycle aryle ou hétéroaryle choisi dans le groupe consistant en:
a) un groupe phényle, facultativement mono-, di- ou tri-substitué par R^{j} et facultativement di-substitué sur des atomes de carbone adjacents par un groupe -Oalkylène en C_{1 à 4}O- facultativement mono- ou di-substitué par un groupe fluoro, -(CH₂)_{2 à 3}-NH-, -(CH₂)_{1 à 2}NH(CH₂)-, -(CH₂)_{2 à 3}-N(alkyle en C_{1 à 4})-, ou -(CH₂) _{1 à 2}N(alkyl en C_{1 à 4}) (CH₂)- ;
où R^{j} est choisi dans le groupe consistant en
1) un groupe -OH, -alkyle en C_{1 à 6}, -Oalkyle en C_{1 à 6} facultativement mono-, di- ou tri-substitué par un groupe halogéno, -alcényle en C_{1 à 6}, -Oalcényle en C_{3 à 6}, -alcynyle en C_{2 à 6} facultativement substitué par un groupe triméthylsilyle, -Oalcynyle en C_{3 à 6}, -cycloalkyle en C_{3 à 6}, -Ocycloalkyle en C_{3 à 6}, -CN, -NO₂, -N(R^{k})R^{j}, -N(R^{k})C(O)R¹, -N(R^{k})SO₂alkyle en C_{1 à 6}, -C(O)alkyle en C_{1 à 6}, -S(O)_{0 à 2}-alkyle en C_{1 à 6}, -C(O)N(R^{m})Rⁿ, -SO₂N(R^{m})Rⁿ, -SCF₃, halogéno, -CF₃, -COOH, -COOalkyle en C_{1 à 6} et -COOcycloalkyle en C_{3 à 7};
où R^{k} et R^{l} sont chacun indépendamment -H ou un groupe -alkyle en C_{1 à 6};
où R^{m} et Rⁿ sont chacun indépendamment -H ou un groupe -alkyle en C_{1 à 6}, ou R^{m} et Rⁿ pris conjointement avec leur atome d'azote d'attache forment un cycle hétérocyclique à 4 à 8 chaînons comportant 1 ou 2 chaînons hétéroatome choisis parmi >O, >S(O)_{0 à 2}, >NH et >Nalkyle en C_{1 à 6}, comportant 0 ou 1 double liaison, comportant 0 ou 1 chaînon carbonyle;
2) -G-Ar², où G est une liaison, -O- ou -S-, et Ar² est un groupe phényle ou est un groupe hydrocarbure aromatique monocyclique comportant cinq ou six atomes de cycle, comportant un atome de carbone remplacé par >O, >S, >NH, ou >N(alkyle en C_{1 à 4}), comportant jusqu'à un atome de carbone additionnel facultativement remplacé par -N=, chacun facultativement mono-, di- ou tri-substitué par R^{p};
où R^{p} est un substituant indépendamment choisi dans le groupe consistant en: un groupe -OH, -alkyle en C_{1 à 6}, -Oalkyle en C_{1 à 6}, phényle, -CN, -NO₂, -N(R^{q})R^{r}, -C(O)N(R^{q})R^{r}, -N(R^{q})C(O)R^{r}, -N(R^{q})SO₂alkyle en C_{1 à 6}, -C(O)alkyle en C_{1 à 6}, -S(O)_{0 à 2}-alkyle en C_{1 à 6}, -SO₂N(R^{q})R^{r}, -SCF₃, halogéno, -CF₃, -OCF₃, -OCHF₂, -COOH et -COOalkyle en C_{1 à 6};
où R^{q} et R^{r} sont chacun indépendamment choisis parmi -H, un groupe -alkyle en C_{1 à 6} et -alcényle en C_{2 à 6}; et
3) un cycle hétérocyclique saturé ou partiellement saturé à 4 à 8 chaînons, comportant 1 ou 2 chaînons hétéroatome choisis parmi >O, >S(O)_{0 à 2}, >NH et >Nalkyle en C_{1 à 6}, comportant 0 ou 1 chaînon carbonyle, ledit cycle étant facultativement mono-, di- ou tri-substitué par R^{p;}
b) un groupe phényle ou pyridyle condensé au niveau de deux chaînons de cycle de carbone adjacents à un fragment hydrocarbure à trois chaînons pour former un cycle aromatique condensé à cinq chaînons, lequel fragment a un atome de carbone remplacé par >O, >S, >NH ou >N(alkyle en C_{1 à 4}), et lequel fragment a jusqu'à un atome de carbone additionnel facultativement remplacé par -N=, les cycles condensés étant facultativement mono-, di- ou tri-substitués par R^{t};
où R^{t} est un substituant indépendamment choisi dans le groupe consistant en: un groupe -OH, -alkyle en C_{1 à 6}, -Oalkyle en C_{1 à 6}, phényle, -CN, -NO₂, -N(R^{u})R^{v}, -C(O)N(R^{u})R^{v}, -N(R^{u})C(O)R^{v}, -N(R^{u})SO₂alkyle en C_{1 à 6}, -C(O)alkyle en C_{1 à 6}, -S(O)_{0 à 2}-alkyle en C_{1 à 6}, -SO₂N(R^{u})R^{v}, -SCF₃, halogéno, -CF₃, -OCF₃, -OCHF₂, -COOH et -COOalkyle en C_{1 à 6};
où R^{u} et R^{v} sont chacun indépendamment -H ou un groupe -alkyle en C_{1 à 6};
c) un groupe phényle condensé au niveau de deux chaînons de cycle adjacents à un fragment hydrocarbure à quatre chaînons pour former un cycle aromatique condensé à six chaînons, lequel fragment a un ou deux atomes de carbone remplacés par -N=, les cycles condensés étant facultativement mono-, di- ou tri-substitués par R^{t};
d) un groupe naphtyle, facultativement mono-, di- ou tri-substitué par R^{t};
e) un groupe hydrocarbure aromatique monocyclique comportant cinq atomes de cycle, comportant un atome de carbone qui est le point d'attache, ayant un atome de carbone remplacé par >O, >S, >NH ou >N(alkyle en C_{1 à 4}), ayant jusqu'à un atome de carbone additionnel facultativement remplacé par -N=, facultativement mono- ou di-substitué par Rⁱ et facultativement benzocondensé ou pyridocondensé au niveau de deux atomes de carbone adjacents, où le fragment benzocondensé ou pyridocondensé est facultativement mono-, di- ou tri-substitué par R^{t}; et
f) un groupe hydrocarbure aromatique monocyclique comportant six atomes de cycle, ayant un atome de carbone qui est le point d'attache, ayant un ou deux atomes de carbone remplacés par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono- ou di-substitué par R^{t};
et ses énantiomères, diastéréoisomères, hydrates, solvates, et sels, esters et amides pharmaceutiquement acceptables, à condition que:
lorsque n vaut 1, L est -O-, Ar est un groupe phényle non substitué et R² et R³ sont tous deux un groupe méthyle, alors R⁴ ne soit pas -OH, à utiliser dans le traitement d'un trouble du SNC choisi dans le groupe consistant en: la dépression, le sommeil perturbé, la fatigue, la léthargie, l'altération cognitive, l'altération de la mémoire, la perte de mémoire, l'altération de l'apprentissage et les troubles déficitaires de l'attention chez les mammifères.

11. Composé de formule (II): 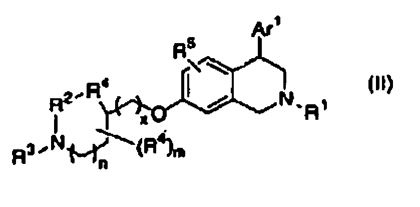 dans laquelle
n vaut 1 ou 2;
x vaut 0 ou 1;
où n + x vaut 1 ou 2;
R¹ est -H; ou est un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyl, en C_{3 à 7}, -COOalkyle en C_{1 à 6} ou -COObenzyle, chacun facultativement mono-, di- ou tri-substitué par R^{a};
où R^{a} est choisi parmi un groupe -OH, -Oalkyle en C_{1 à 6}, phényle facultativement substitué par un groupe -Oalkyle en C_{1 à 4} ou halogéno, -CN, -NO₂-, -N(R^{b})R^{c}, -C(O)N(R^{b})R^{c}, -N(R^{b})C(O)R^{b}, -N(R^{b})SO₂alkyle en C_{1 à 6}, -C(O)alkyle en C_{1 à 6}, -S(O)_{0 à 2}-alkyle en C_{1 à 6}, -SO₂N(R^{b})R^{c}, -SCF₃, halogéno, -CF₃, -OCF₃, -COOH et -COOalkyle en C_{1 à 6;}
où R^{b} et R^{c} sont chacun indépendamment -H ou un groupe -alkyle en C_{1 à 6};
R³ est choisi dans le groupe consistant en: -H;
A) un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyle en C_{3 à 7}, -CH(cycloalkyle en C_{3 à 8})₂, -CH(phényle)₂, benzyle et -C(O)Oalkyle en C_{1 à 4}, où chaque groupe alkyle, cycloalkyle ou benzyle est facultativement substitué par un groupe -OH, -Oalkyle en C_{1 à 4}, -CN, -NH₂, -NH (alkyle en C_{1 à 4}), -N(alkyle en C_{1 à 4})₂, halogéno, -CF₃, -OCF₃, -COOH ou -COOalkyle en C_{1 à 6};
B) un groupe phényle ou pyridyle, facultativement condensé au niveau de deux chaînons de cycle de carbone adjacents à un fragment hydrocarbure à trois ou quatre chaînons pour former un cycle aromatique condensé à cinq ou six chaînons, lequel fragment a un atome de carbone remplacé par >O, >S, >NH, >N(alkyle en C_{1 à 4}) ou >NC(O)Oalkyle en C_{1 à 4}, et lequel fragment a jusqu'à un atome de carbone additionnel facultativement remplacé par -N=;
C) un groupe naphtyle,
D) un cycle hétérocyclique à 4 à 8 chaînons, ledit cycle hétérocyclique ayant un atome de carbone qui est le point d'attache, comportant 1 ou 2 chaînons hétéroatome choisis dans le groupe consistant en >O, >S(O)_{0 à 2}, >NH, >N(alkyle en C_{1 à 4}), et >NC (O)Oalkyle en C_{1 à 4}, et comportant 0 ou 1 double liaison; et
E) un groupe hydrocarbure aromatique monocyclique comportant cinq ou six atomes de cycle, ayant un atome de carbone qui est le point d'attache, ayant un atome de carbone remplacé par >O, >S, >NH, >N(alkyle en C_{1 à 4}) ou >NC(O)Oalkyle en C_{1 à 4}, ayant jusqu'à un atome de carbone additionnel facultativement remplacé par -N=, et facultativement benzocondensé ou pyridocondensé;
où chacun de B) à E) est facultativement mono-, di- ou tri-substitué par un fragment choisi dans le groupe consistant en un groupe -alkyle en C_{1 à 4}, -OH, -alkyl en C_{1 à 4}0H, -Oalkyle en C_{1 à 6}, -CN, -NO₂, -N(R^{d})R^{e}, -C(O)N(R^{d})R^{e}, -N(R^{d})C(O)R^{d}, -N(R^{d})SO₂alkyle en C_{1 à 6}, -C(O)alkyle en C_{1 à 6}, -S(O)_{0 à 2}-alkyle en C_{1 à 6}, -SO₂N(R^{d})R^{e}, -SCF₃, halogéno, -CF₃, -OCF₃, -COOH, -COOalkyle en C_{1 à 4}, -OC(O)N(R^{d})R^{e} et -OC(O)Oalkyle en C_{1 à 6};
où R^{d} et R^{e} sont chacun indépendamment -H ou un groupe -alkyle en C_{1 à 6};
-R²-R⁴- est un groupe méthylène, éthylène, propylène, ou -CH₂CH₂O-, où chacun est facultativement substitué par un groupe -OH, -Oalkyle en C_{1 à 6}, -CF₃, -alkyle en C_{1 à 6}, amino ou halogéno;
R⁴ est un groupe -OH, -Oalkyle en C_{1 à 6}, -CF₃, -alkyle en C_{1 à 6} ou halogéno,
m vaut 0, 1 ou 2;
R⁵ est choisi dans le groupe consistant en un groupe -alkyle en C_{1 à 6}, -OH, -Oalkyle en C_{1 à 6}, -Salkyle en C_{1 à 6} et halogéno;
Ar¹ est un cycle aryle ou hétéroaryle choisi dans le groupe consistant en:
a) un groupe phényle, facultativement mono-, di- ou tri-substitué par R^{j} et facultativement di-substitué sur des atomes de carbone adjacents par un groupe -Oalkylène en C_{1 à 4} O- facultativement mono- ou di-substitué par un groupe fluoro, -(CH₂)_{2 à 3}-NH-, -(CH₂)_{1 à 2}NH(CH₂)-, -(CH₂)_{2 à 3}-N(alkyle en C_{1 à 4})-, ou -(CH₂)_{1 à 2}N(alkyl en C_{1 à 4}) (CH₂)- ;
où Rⁱ est choisi dans le groupe consistant en
1) un groupe -OH, -alkyle en C_{1 à 6}, -Oalkyle en C_{1 à 6} facultativement mono-, di- ou tri-substitué par un groupe halogéno, -alcényle en C_{2 à 6}, -Oalcényle en C_{3 à 6}, -alcynyle en C_{2 à 6} facultativement substitué par un groupe triméthylsilyle, -Oalcynyle en C_{3 à 6}, -cycloalkyle en C_{3 à 6}, -Ocycloalkyle en C_{3 à 6}, -CN, -NO₂, -N(R^{k})R^{j}, -N(R^{k})C(O)R^{l}, -N(R^{k})SO₂alkyle en C_{1 à 6}, -C(O)alkyle en C_{1 à 6}, -S(O)_{0 à 2}-alkyle en C_{1 à 6}, -C(O)N(R^{m})Rⁿ, -SO₂N(R^{m})Rⁿ, -SCF₃, halogéno, -CF₃, -COOH, -COOalkyle en C_{1 à 6} et -COOcycloalkyle en C_{3 à 7};
où R^{k} et R^{l} sont chacun indépendamment -H ou un groupe -alkyle en C_{1 à 6};
où R^{m} et Rⁿ sont chacun indépendamment -H ou un groupe -alkyle en C_{1 à 6}, ou R^{m} et Rⁿ pris conjointement avec leur atome d'azote d'attache forment un cycle hétérocyclique à 4 à 8 chaînons comportant 1 ou 2 chaînons hétéroatome choisis parmi >O, >S(O)_{0 à 2}, >NH et >Nalkyle en C_{1 à 6}, comportant 0 ou 1 double liaison, comportant 0 ou 1 chaînon carbonyle;
2) -G-Ar², où G est une liaison, -O- ou -S-, et Ar² est un groupe phényle ou est un groupe hydrocarbure aromatique monocyclique comportant cinq ou six atomes de cycle, comportant un atome de carbone remplacé par >O, >S, >NH, ou >N(alkyle en C_{1 à 4})_{,} comportant jusqu'à un atome de carbone additionnel facultativement remplacé par -N=, chacun facultativement mono-, di- ou tri-substitué par R^{p};
où R^{p} est un substituant indépendamment choisi dans le groupe consistant en: un groupe -OH, -alkyle en C_{1 à 6}, -Oalkyle en C_{1 à 6}, phényle, -CN, -NO₂, -N(R^{q})R^{r}, -C(O)N(R^{q})R^{r}, -N(R^{q})C(O)R^{r}, -N(R^{q})SO₂alkyle en C_{1 à 6}, -C(O)alkyle en C_{1 à 6,} -S(O)_{0 à 2}-alkyle en C_{1 à 6}, -SO₂N(R^{q})R^{r}, -SCF₃, halogéno, -CF₃, -OCF₃, -OCHF₂, -COOH et -COOalkyle en C_{1 à 6};
où R^{q} et R^{r} sont chacun indépendamment choisis parmi -H, un groupe -alkyle en C_{1 à 6} et -alcényle en C_{2 à 6}; et
3) un cycle hétérocyclique saturé ou partiellement saturé à 4 à 8 chaînons, comportant 1 ou 2 chaînons hétéroatome choisis parmi >O, >S(O)_{0 à 2}, >NH et >Nalkyle en C_{1 à 6}, comportant 0 ou 1 chaînon carbonyle, ledit cycle étant facultativement mono-, di- ou tri-substitué par R^{p;}
b) un groupe phényle ou pyridyle condensé au niveau de deux chaînons de cycle de carbone adjacents à un fragment hydrocarbure à trois chaînons pour former un cycle aromatique condensé à cinq chaînons, lequel fragment a un atome de carbone remplacé par >O, >S, >NH ou >N(alkyle en C_{1 à 4}), et lequel fragment a jusqu'à un atome de carbone additionnel facultativement remplacé par -N=, les cycles condensés étant facultativement mono-, di- ou tri-substitués par R^{t};
où R^{t} est un substituant indépendamment choisi dans le groupe consistant en: un groupe -OH, -alkyle en C_{1 à 6}, -Oalkyle en C_{1 à 6}, phényle, -CN, -NO₂, -N(R^{u})R^{v}, -C(O)N(R^{u})R^{v}, -N(R^{u})C(O)R^{v}, -N(R^{u})SO₂alkyle en C_{1 à 6}, -C(O)alkyle en C_{1 à 6}, -S(O)_{0 à 2}-alkyle en C_{1 à 6}, -SO₂N(R^{u})R^{v}, -SCF₃, halogéno, -CF₃, -OCF₃, -OCHF₂, -COOH et -COOalkyle en C_{1 à 6};
où R^{u} et R^{v} sont chacun indépendamment -H ou un groupe -alkyle en C_{1 à 6};
c) un groupe phényle condensé au niveau de deux chaînons de cycle adjacents à un fragment hydrocarbure à quatre chaînons pour former un cycle aromatique condensé à six chaînons, lequel fragment a un ou deux atomes de carbone remplacés par -N=, les cycles condensés étant facultativement mono-, di- ou tri-substitués par R^{t};
d) un groupe naphtyle, facultativement mono-, di- ou tri-substitué par R^{t};
e) un groupe hydrocarbure aromatique monocyclique comportant cinq atomes de cycle, comportant un atome de carbone qui est le point d'attache, ayant un atome de carbone remplacé par >O, >S, >NH ou >N(alkyle en C_{1 à 4}), ayant jusqu'à un atome de carbone additionnel facultativement remplacé par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono-, di- ou tri-substitué par R^{t}; et
f) un groupe hydrocarbure aromatique monocyclique comportant six atomes de cycle, ayant un atome de carbone qui est le point d'attache, ayant un ou deux atomes de carbone remplacés par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono- ou di-substitué par R^{t};
et ses énantiomères, diastéréoisomères, hydrates, solvates, et sels, esters et amides pharmaceutiquement acceptables, à utiliser dans le traitement d'un trouble du SNC choisi dans le groupe consistant en: les troubles du sommeil et de l'éveil et de la stimulation et de la vigilance, l'insomnie, le décalage horaire, le sommeil perturbé, les troubles déficitaires de l'attention avec hyperactivité (TDAH), les troubles déficitaires de l'attention, les troubles d'apprentissage et de la mémoire, l'altération de l'apprentissage, l'altération de la mémoire, la perte de mémoire, le dysfonctionnement cognitif, la migraine, l'inflammation neurogène, la démence, l'altération cognitive douce, la prédémence, la maladie d'Alzheimer, l'épilepsie, la narcolepsie avec ou sans cataplexie associée, la cataplexie, les troubles de l'homéostase du sommeil et de l'éveil, la somnolence idiopathique, la somnolence diurne excessive (EDS), les troubles du rythme circadien, les troubles du sommeil et de la fatigue, la fatigue, l'assoupissement associé aux apnées du sommeil, l'altération du sommeil due aux changements hormonaux périménopausiques, la fatigue liée à Parkinson, la fatigue liée à MS, la fatigue liée à la dépression, la fatigue induite par chimiothérapie, la fatigue liée au travail, la léthargie, les troubles de l'alimentation, l'obésité, la cinétose, le vertige, la schizophrénie, l'abus de substances, les troubles bipolaires, les troubles maniaques et la dépression chez les mammifères.

12. Composé de formule (II): 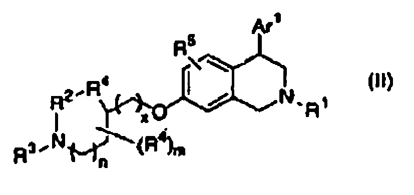 dans laquelle
n vaut 1 ou 2;
x vaut 0 ou 1;
où n + x vaut 1 ou 2;
R¹ est -H; ou est un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyle en C_{3 à 7}, -COOalkyle en C_{1 à 6} ou -COObenzyle, chacun facultativement mono-, di- ou tri-substitué par R^{a};
où R^{a} est choisi parmi un groupe -OH, -Oalkyle en C_{1 à 6}, phényle facultativement substitué par un groupe -Oalkyle en C_{1 à 4} ou halogéno, -CN, -NO₂-, -N(R^{b})R^{c}, -C(O)N(R^{b})R^{c}, -N(R^{b})C(O)R^{b}, -N(R^{b})SO₂alkyle en C_{1 à 6,} -C(O)alkyle en C_{1 à 6}, -S(O)_{0 à 2}-alkyle en C_{1 à 6,} -SO₂N(R^{b})R^{c}, -SCF₃, halogéno, -CF₃, -OCF₃, -COOH et -COOalkyle en C_{1 à 6};
où R^{b} et R^{c} sont chacun indépendamment -H ou un groupe -alkyle en C_{1 à 6};
R³ est choisi dans le groupe consistant en: -H;
A) un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyle en C_{3 à 7}, -CH(cycloalkyle en C_{3 à 8})₂, -CH(phényle)₂, benzyle et -C(O)Oalkyle en C_{1 à 4}, où chaque groupe alkyle, cycloalkyle ou benzyle est facultativement substitué par un groupe -OH, -Oalkyle en C_{1 à 4}, -CN, -NH₂, -NH (alkyle en C_{1 à 4}), -N(alkyle en C_{1 à 4})₂, halogéno, -CF₃, -OCF₃, -COOH ou -COOalkyle en C_{1 à 6};
B) un groupe phényle ou pyridyle, facultativement condensé au niveau de deux chaînons de cycle de carbone adjacents à un fragment hydrocarbure à trois ou quatre chaînons pour former un cycle aromatique condensé à cinq ou six chaînons, lequel fragment a un atome de carbone remplacé par >O, >S, >NH, >N(alkyle en C_{1 à 4}) ou >NC(O)Oalkyle en C_{1 à 4}, et lequel fragment a jusqu'à un atome de carbone additionnel facultativement remplacé par -N=;
C) un groupe naphtyle,
D) un cycle hétérocyclique à 4 à 8 chaînons, ledit cycle hétérocyclique ayant un atome de carbone qui est le point d'attache, comportant 1 ou 2 chaînons hétéroatome choisis dans le groupe consistant en >O, >S(O)_{0 à 2}, >NH, >N (alkyle en C_{1 à 4}, et >NC (O)Oalkyle en C_{1 à 4}, et comportant 0 ou 1 double liaison; et
E) un groupe hydrocarbure aromatique monocyclique comportant cinq ou six atomes de cycle, ayant un atome de carbone qui est le point d'attache, ayant un atome de carbone remplacé par >O, >S, >NH, >N(alkyle en C_{1 à 4}) ou >NC(O)Oalkyle en C_{1 à 4}, ayant jusqu'à un atome de carbone additionnel facultativement remplacé par -N=, et facultativement benzocondensé ou pyridocondensé;
où chacun de B) à E) est facultativement mono-, di- ou tri-substitué par un fragment choisi dans le groupe consistant en un groupe -alkyle en C_{1 à 4}. -OH, -alkyl en C_{1 à 4}OH, -Oalkyle en Là6, -CN, -NO₂, -N(R^{d})R^{e}, -C(O)N(R^{d})R^{e}, -N(R^{d})C(O)R^{d}, -N(R^{d})SO₂alkyle en C_{1 à 6}, -C(O)alkyle en C_{1 à 6}, -S(O)_{0 à 2}-alkyle en C_{1 à 6}. -SO₂N(R^{d})R^{e}, -SCF₃, halogéno, -CF₃, -OCF₃, -COOH, -COOalkyle en C_{1 à 4}, -OC(O)N(R^{d})R^{e} et -OC(O)Oalkyle en C_{1 à 6};
où R^{d} et R^{e} sont chacun indépendamment -H ou un groupe -alkyle en C_{1 à 6}; -R²-R⁴- est un groupe méthylène, éthylène, propylène, ou -CH₂CH₂O-, où chacun est facultativement substitué par un groupe -OH, -Oalkyle en C_{1 à 6}, -CF₃, -alkyle en C_{1 à 6}, amino ou halogéno;
R⁴ est un groupe -OH, -Oalkyle en C_{1 à 6}, -CF₃, -alkyle en C_{1 à 6} ou halogéno,
m vaut 0, 1 ou 2;
R⁵ est choisi dans le groupe consistant en un groupe -alkyle en C_{1 à 6}, -OH, -Oalkyle en C_{1 à 6}, -Salkyle en C_{1 à 6} et halogéno;
Ar¹ est un cycle aryle ou hétéroaryle choisi dans le groupe consistant en:
a) un groupe phényle, facultativement mono-, di- ou tri-substitué par R^{j} et facultativement di-substitué sur des atomes de carbone adjacents par un groupe -Oalkylène en C_{1 à 4}O- facultativement mono- ou di-substitué par un groupe fluoro, -(CH₂)_{2 à 3}-NH-, -(CH₂)_{1 à 2}NH(CH₂)-, -(CH₂)_{2 à 3}-N(alkyle en C_{1 à 4})-, ou -(CH₂)_{1 à 2}N(alkyl en C_{1 à 4})(CH₂)-;
où R^{j} est choisi dans le groupe consistant en
1) un groupe -OH, -alkyle en C_{1 à 6}, -Oalkyle en C_{1 à 6} facultativement mono-, di- ou tri-substitué par un groupe halogéno, -alcényle en C_{2 à 6}, -Oalcényle en C_{3 à 6}, -alcynyle en C_{2 à 6} facultativement substitué par un groupe triméthylsilyle, -Oalcynyle en C_{3 à 6}, -cycloalkyle en C_{3 à 6}, -Ocycloalkyle en C_{3 à 6}, -CN, -NO₂, -N(R^{k})R^{j}, -N(R^{k})C(O)R^{l}, -N(R^{k})SO₂alkyle en C_{1 à 6}, -C(O)alkyle en C_{1 à 6}, -S(O)_{0 à 2}-alkyle en C_{1 à 6}, -C(O)N(R^{m})Rⁿ, -SO₂N(R^{m})Rⁿ_{,} -SCF₃, halogéno, -CF₃, -COOH, -COOalkyle en C_{1 à 6} et -COOcycloalkyle en C_{3 à 7};
où R^{k} et R^{l} sont chacun indépendamment -H ou un groupe -alkyle en C_{1 à 6}; où R^{m} et Rⁿ sont chacun indépendamment -H ou un groupe -alkyle en C_{1 à 6}, ou R^{m} et Rⁿ pris conjointement avec leur atome d'azote d'attache forment un cycle hétérocyclique à 4 à 8 chaînons comportant 1 ou 2 chaînons hétéroatome choisis parmi >O, >S(O)_{0 à 2}, >NH et >Nalkyle en C_{1 à 6}, comportant 0 ou 1 double liaison, comportant 0 ou 1 chaînon carbonyle;
2) -G-Ar², où G est une liaison, -O- ou -S-, et Ar² est un groupe phényle ou est un groupe hydrocarbure aromatique monocyclique comportant cinq ou six atomes de cycle, comportant un atome de carbone remplacé par >O, >S, >NH, ou >N(alkyle en C_{1 à 4}), comportant jusqu'à un atome de carbone additionnel facultativement remplacé par -N=, chacun facultativement mono-, di- ou tri-substitué par R^{p};
où R^{p} est un substituant indépendamment choisi dans le groupe consistant en: un groupe -OH, -alkyle en C_{1 à 6}, -Oalkyle en C_{1 à 6}, phényle, -CN, -NO₂, -N(R^{q})R^{r}, -C(O)N(R^{q})R^{r}, -N(R^{q})C(O)R^{r}, -N(R^{q})SO₂alkyle en C_{1 à 6}, -C(O)alkyle en C_{1 à 6}, -S(O)_{0 à 2}-alkyle en C_{1 à 6}, -SO₂N(R^{q})R^{r}, -SCF₃, halogéno, -CF₃, -OCF₃, -OCHF₂, -COOH et -COOalkyle en C_{1 à 6};
où R^{q} et R^{r} sont chacun indépendamment choisis parmi -H, un groupe -alkyle en C_{1 à 6} et -alcényle en C_{2 à 6}; et
3) un cycle hétérocyclique saturé ou partiellement saturé à 4 à 8 chaînons, comportant 1 ou 2 chaînons hétéroatome choisis parmi >O, >S(O)_{0 à 2}, >NH et >Nalkyle en C_{1 à 6}, comportant 0 ou 1 chaînon carbonyle, ledit cycle étant facultativement mono-, di- ou tri-substitué par R^{p};
b) un groupe phényle ou pyridyle condensé au niveau de deux chaînons de cycle de carbone adjacents à un fragment hydrocarbure à trois chaînons pour former un cycle aromatique condensé à cinq chaînons, lequel fragment a un atome de carbone remplacé par >O, >S, >NH ou >N (alkyle en C_{1 à 4}), et lequel fragment a jusqu'à un atome de carbone additionnel facultativement remplacé par -N=, les cycles condensés étant facultativement mono-, di- ou tri-substitués par R^{t};
où R^{t} est un substituant indépendamment choisi dans le groupe consistant en: un groupe -OH, -alkyle en C_{1 à 6}, -Oalkyle en C_{1 à 6}, phényle, -CN, -NO₂, -N(R^{u})R^{v}, -C(O)N(R^{u})R^{v}, -N(R^{u})C(O)R^{v}, -N(R^{u})SO₂alkyle en C_{1 à 6}, -C(O)alkyle en C_{1 à 6}, -S(O)_{0 à 2}-alkyle en C_{1 à 6}, -SO₂N(R^{u})R^{v}, -SCF₃, halogéno, -CF₃, -OCF₃, -OCHF₂, -COOH et -COOalkyle en C_{1 à 6};
où R^{u} et R^{v} sont chacun indépendamment -H ou un groupe -alkyle en C_{1 à 6};
c) un groupe phényle condensé au niveau de deux chaînons de cycle adjacents à un fragment hydrocarbure à quatre chaînons pour former un cycle aromatique condensé à six chaînons, lequel fragment a un ou deux atomes de carbone remplacés par -N=, les cycles condensés étant facultativement mono-, di- ou tri-substitués par R^{t};
d) un groupe naphtyle, facultativement mono-, di- ou tri-substitué par R^{t};
e) un groupe hydrocarbure aromatique monocyclique comportant cinq atomes de cycle, comportant un atome de carbone qui est le point d'attache, ayant un atome de carbone remplacé par >O, >S, >NH ou >N(alkyle en C_{1 à 4}), ayant jusqu'à un atome de carbone additionnel facultativement remplacé par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono-, di- ou tri-substitué par R^{t}; et
f) un groupe hydrocarbure aromatique monocyclique comportant six atomes de cycle, ayant un atome de carbone qui est le point d'attache, ayant un ou deux atomes de carbone remplacés par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono- ou di-substitué par R^{t};
et ses énantiomères, diastéréoisomères, hydrates, solvates, et sels, esters et amides pharmaceutiquement acceptables.

13. Composé de formule (I): 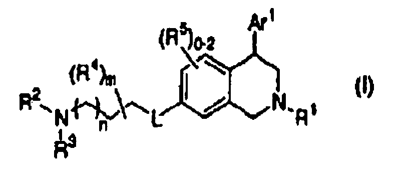 dans laquelle
L est -O- et n vaut 1 ou 2; ou L est -C=C- ou -CH₂CH₂- et n vaut 0 ou 1;
R¹ est -H; ou est un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyle en C_{3 à 7}, -COOalkyle en C_{1 à 6} ou -COObenzyle, chacun facultativement mono-, di- ou tri-substitué par R^{a};
où R^{a} est choisi parmi un groupe -OH, -Oalkyle en C_{1 à 6}, phényle facultativement substitué par un groupe -Oalkyle en C_{1 à 4} ou halogéno, -CN, -NO₂-, -N(R^{b})R^{c}, -C(O)N(R^{b})R^{c}, -N(R^{b})C(O)R^{b}, -N(R^{b})SO₂alkyle en C_{1 à 6}, -C(O)alkyle en C_{1 à 6}, -S(O)_{0 à 2}-alkyle en C_{1 à 6}, -SO₂N(R^{b})R^{c}, -SCF₃, halogéno, -CF₃, -OCF₃, -COOH et -COOalkyle en C_{1 à 6};
où R^{b} et R^{c} sont chacun indépendamment -H ou un groupe -alkyle en C_{1 à 6};
R² et R³ sont chacun indépendamment choisis dans le groupe consistant en: -H;
A) un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyle en C_{3 à 7}, -CH (cycloalkyle en C_{3 à 8})₂, -CH(phényle)₂, benzyle et -C(O)Oalkyle en C_{1 à 4}, où chaque groupe alkyle, cycloalkyle ou benzyle est facultativement substitué par un groupe -OH, -Oalkyle en C_{1 à 4}, -CN, -NH₂, -NH (alkyle en C_{1 à 4}), -N (alkyle en C_{1 à 4})₂, halogéno, -CF₃, -OCF₃, -COOH ou -COOalkyle en C_{1 à 6;}
B) un groupe phényle ou pyridyle, facultativement condensé au niveau de deux chaînons de cycle de carbone adjacents à un fragment hydrocarbure à trois ou quatre chaînons pour former un cycle aromatique condensé à cinq ou six chaînons, lequel fragment a un atome de carbone remplacé par >O, >S, >NH, >N(alkyle en C_{1 à 4}) ou >NC(O)Oalkyle en C_{1 à 4}, et lequel fragment a jusqu'à un atome de carbone additionnel facultativement remplacé par -N=;
C) un groupe naphtyle,
D) un cycle hétérocyclique à 4 à 8 chaînons, ledit cycle hétérocyclique ayant un atome de carbone qui est le point d'attache, comportant 1 ou 2 chaînons hétéroatome choisis dans le groupe consistant en >O, >S(O)_{0 à 2}, >NH, >N (alkyle en C_{1 à 4}), et >NC(O)Oalkyle en C_{1 à 4}, et comportant 0 ou 1 double liaison; et
E) un groupe hydrocarbure aromatique monocyclique comportant cinq ou six atomes de cycle, ayant un atome de carbone qui est le point d'attache, ayant un atome de carbone remplacé par >O, >S, >NH, >N(alkyle en C_{1 à 4}) ou >NC(O)Oalkyle en C_{1 à 4}, ayant jusqu'à un atome de carbone additionnel facultativement remplacé par -N=, et facultativement benzocondensé ou pyridocondensé;
où chacun de B) à E) est facultativement mono-, di- ou tri-substitué par un fragment choisi dans le groupe consistant en un groupe -alkyle en C_{1 à 4}, -OH, -alkyl en C_{1 à 4}OH, -Oalkyle en C_{1 à 6}, -CN, -NO₂, -N(R^{d})R^{e}, -C(O)N(R^{d})R^{e}, -N(R^{d})C(O)R^{d}, -N(R^{d})SO₂alkyle en C_{1 à 6}, -C(O)alkyle en C_{1 à 6}, -S(O)_{0 à 2}-alkyle en C_{1 à 6}, -SO₂N(R^{d})R^{e}, -SCF₃, halogéno, -CF₃, -OCF₃, -COOH, -COOalkyle en C_{1 à 4}, -OC(O)N(R^{d})R^{e} et -OC(O)Oalkyle en C_{1 à 6};
où R^{d} et R^{e} sont chacun indépendamment -H ou un groupe -alkyle en C_{1 à 6};
ou, en variante,
R² et R³ peuvent être pris conjointement avec l'atome d'azote auquel ils sont attachés pour former un groupe tétrahydro-pyrimidin-2-ylidényle, ou un cycle hétérocyclique à 4 à 8 chaînons, ledit cycle hétérocyclique ayant 0 ou 1 chaînon hétéroatome additionnel séparé de l'azote d'attache par au moins un chaînon carbone et choisi dans le groupe consistant en >O, >S(O)_{0 à 2}, >NH et >NR^{f}, ayant 0 ou 1 double liaison, ayant 0, 1 ou 2 chaînons carbone séparés de l'atome d'azote d'attache par au moins un chaînon carbone qui est un groupe carbonyle, facultativement benzo ou pyridocondensé, comportant facultativement un chaînon carbone qui forme un pont, et comportant 0 à 5 substituants de chaînon carbone R^{ff},
où R^{f} est choisi dans le groupe consistant en:
i) un groupe -alkyle en C_{1 à 6} facultativement substitué par R², -alcényle en C_{3 à 6}, -alcynyle en C_{3 à 6}, -C(O)N(R^{g})R^{h}, -C(O)Rⁱ, -S(O)_{0 à 2}-alkyle en C_{1 à 6} et -COOalkyle en C_{1 à 6},
où R^{z} est un groupe fluoro, -CN, -OH, -Oalkyle en C_{1 à 4}, -cycloalkyle en C_{3 à 7} ou -CF_{3;}
où R^{g} et R^{h} sont chacun indépendamment -H ou -alkyle en C_{1 à 6}; et
où Rⁱ est un groupe -alkyle en C_{1 à 6}, -cycloalkyle en C_{3 à 8}, phényle, ou hétérocyclyle aromatique à 5 ou 6 chaînons, où chaque groupe alkyle, cycloalkyle, phényle ou hétérocyclyle est facultativement mono-, di- ou tri-substitué par un groupe -alkyle en C_{1 à 4}, -OH, -Oalkyle en C_{1 à 6}, -CF₃, -CN ou halogéno;
ii) -(CH₂)_{0 à 1}-Cycle A, où Cycle A est un groupe phényle ou un groupe hétérocyclyle aromatique à 5 ou 6 chaînons à liaison carbone, facultativement substitué par R^{aa};
où R^{aa} est un groupe -OH, -alkyle en C_{1 à 6}, -Oalkyle en C_{1 à 6}, phényle, -CN, -NO₂, -N(R^{g})R^{h}, -C(O)N(R^{g})R^{h}, -(R^{g})C(O)R^{h}, -N(R^{h})SO₂-alkyle en C_{1 à 6}, -C(O)alkyle en C_{1 à 6}, -S(O)_{0 à 2}-alkyle en C_{1 à 6}, -SO₂N(R^{g})R^{h}, -SCF₃, halogéno, -CF₃, -OCF₃, -OCHF₂, -COOH et -COOalkyle en C_{1 à 6}; et
iii) -(CH₂)_{0 à 1}-Cycle B, où Cycle B est un groupe -cycloalkyle en C_{3 à 7} avec un ou deux chaînons de cycle carboné facultativement remplacés par >O ou >NH, et facultativement substitué par un groupe -alkyle en C_{1 à 4}, fluoro, -CN, -OH, -Oalkyle en C_{1 à 4} ou -CF_{3;}
où R^{ff} est choisi dans le groupe consistant en un groupe -alkyle en C_{1 à 6} facultativement mono- ou di-substitué par R², un groupe -alcényle en C_{2 à 6}, -alcynyle en C_{2 à 6}, -cycloalkyle en C_{3 à 7}, -CH(phényle)₂, halogéno, -OH, -Oalkyle en C_{1 à 6}, -Oalkyl en C_{2 à 3}O-, -CN, -NO₂, -N(R^{g})R^{h}, -C(O)N(R^{g})R^{h}, -N(R^{g})C(O)R^{g}, -N(R^{g})SO₂alkyle en C_{1 à 6}, -C(O)Rⁱ, -S(O)_{0 à 2}-alkyle en C_{1 à 6}, -SO₂N(R^{g})R^{h}, -SCF₃, -CF₃, -OCF₃, -COOH et -COOalkyle en C_{1 à 6};
R⁴ est un groupe -OH, -Oalkyle en C_{1 à 6}, -CF₃, -alkyle en C_{1 à 6} Ou halogéno; deux substituants R⁴ peuvent être pris ensemble pour former un groupe méthylène ou éthylène; ou l'un de R⁴ est pris conjointement avec R² pour former un groupe méthylène, éthylène, propylène ou -CH₂CH₂O-, où chacun est facultativement substitué par un groupe -OH, -Oalkyle en C_{1 à 6}, -Salkyle en C_{1 à 6}, -CF₃, -alkyle en C_{1 à 6}, amino ou halogéno;
m vaut 0, 1 ou 2;
R⁵ est choisi dans le groupe consistant en un groupe -alkyle en C_{1 à 6}, -OH, -Oalkyle en C_{1 à 6}, -Salkyle en C_{1 à 6} et halogéno;
Ar¹ est un cycle aryle ou hétéroaryle choisi dans le groupe consistant en:
a) un groupe phényle, facultativement mono-, di- ou tri-substitué par R^{j} et facultativement di-substitué sur des atomes de carbone adjacents par un groupe -Oalkylène en C_{1 à 4}O- facultativement mono- ou di-substitué par un groupe fluoro, -(CH₂)_{2 à 3}-NH-, -(CH₂)_{1 à 2}NH(CH₂)-, -(CH₂)_{2 à 3}-N(alkyle en C_{1 à 4})-, ou -(CH₂)_{1 à 2}N(alkyl en C_{1 à 4})(CH₂)-;
où R^{j} est choisi dans le groupe consistant en
1) un groupe -OH, -alkyle en C_{1 à 6}, -Oalkyle en C_{1 à 6} facultativement mono-, di- ou tri-substitué par un groupe halogéno, -alcényle en C_{2 à 6}, -Oalcényle en C_{3 à 6}, -alcynyle en C_{2 à 6} facultativement substitué par un groupe triméthylsilyle, -Oalcynyle en C_{3 à 6}, -cycloalkyle en C_{3 à 6}, -Ocycloalkyle en C_{3 à 6}, -CN, -NO₂, -N(R^{k})R^{j}, -N(R^{k})C(O)R^{l}, -N(R^{k})SO₂alkyle en C_{1 à 6}, -C(O)alkyle en C_{1 à 6}, -S(O)_{0 à 2}-alkyle en C_{1 à 6}, -C(O)N(R^{m})Rⁿ, -SO₂N(R^{m})Rⁿ, -SCF₃, halogéno, -CF₃, -COOH, -COOalkyle en C_{1 à 6} et -COOcycloalkyle en C_{3 à 7};
où R^{k} et R^{l} sont chacun indépendamment -H ou un groupe -alkyle en C_{1 à 6};
où R^{m} et Rⁿ sont chacun indépendamment -H ou un groupe -alkyle en C_{1 à 6}, ou R^{m} et Rⁿ pris conjointement avec leur atome d'azote d'attache forment un cycle hétérocyclique à 4 à 8 chaînons comportant 1 ou 2 chaînons hétéroatome choisis parmi >0, >S(O)_{0 à 2}, >NH et >Nalkyle en C_{1 à 6}, comportant 0 ou 1 double liaison, comportant 0 ou 1 chaînon carbonyle;
2) -G-Ar², où G est une liaison, -0- ou -S-, et Ar² est un groupe phényle ou est un groupe hydrocarbure aromatique monocyclique comportant cinq ou six atomes de cycle, comportant un atome de carbone remplacé par >O, >S, >NH, ou >N(alkyle en C_{1 à 4}), comportant jusqu'à un atome de carbone additionnel facultativement remplacé par -N=, chacun facultativement mono-, di- ou tri-substitué par R^{p};
où R^{p} est un substituant indépendamment choisi dans le groupe consistant en: un groupe -OH, -alkyle en C_{1 à 6}, -Oalkyle en C_{1 à 6}, phényle, -CN, -NO₂, -N(R^{q})R^{r}, -C(O)N(R^{q})R^{r}, -N(R^{q})C(O)R^{r}, -N(R^{q})SO₂alkyle en C_{1 à 6}, -C(O)alkyle en C_{1 à 6}, -S(O)_{0 à 2}-alkyle en C_{1 à 6}, -SO₂N(R^{q})R^{r}, -SCF₃, halogéno, -CF₃, -OCF₃, -OCHF₂, -COOH et -COOalkyle en C_{1 à 6};
où R^{q} et R^{r} sont chacun indépendamment choisis parmi -H, un groupe -alkyle en C_{1 à 6} et -alcényle en C_{2 à 6}; et
3) un cycle hétérocyclique saturé ou partiellement saturé à 4 à 8 chaînons, comportant 1 ou 2 chaînons hétéroatome choisis parmi >O, >S(O)_{0 à 2}, >NH et >Nalkyle en C_{1 à 6}, comportant 0 ou 1 chaînon carbonyle, ledit cycle étant facultativement mono-, di- ou tri-substitué par R^{p};
b) un groupe phényle ou pyridyle condensé au niveau de deux chaînons de cycle de carbone adjacents à un fragment hydrocarbure à trois chaînons pour former un cycle aromatique condensé à cinq chaînons, lequel fragment a un atome de carbone remplacé par >O, >S, >NH ou >N (alkyle en C_{1 à 4}), et lequel fragment a jusqu'à un atome de carbone additionnel facultativement remplacé par -N=, les cycles condensés étant facultativement mono-, di- ou tri-substitués par R^{t};
où R^{t} est un substituant indépendamment choisi dans le groupe consistant en: un groupe -OH, -alkyle en C_{1 à 6}, -Oalkyle en C_{1 à 6}, phényle, -CN, -NO₂, -N(R^{u})R^{v}, -C(O)N(R^{u})R^{v}, -N(R^{u})C(O)R^{v}, -N(R^{u})SO₂alkyle en C_{1 à 6}, -C(O)alkyle en C_{1 à 6}, -S(O)_{0 à 2}-alkyle en C_{1 à 6}, -SO₂N(R^{u})R^{v}, -SCF₃, halogéno, -CF₃, -OCF₃, -OCHF₂, -COOH et -COOalkyle en C_{1 à 6};
où R^{u} et R^{v} sont chacun indépendamment -H ou un groupe -alkyle en C_{1 à 6};
c) un groupe phényle condensé au niveau de deux chaînons de cycle adjacents à un fragment hydrocarbure à quatre chaînons pour former un cycle aromatique condensé à six chaînons, lequel fragment a un ou deux atomes de carbone remplacés par -N=, les cycles condensés étant facultativement mono-, di- ou tri-substitués par R^{t};
d) un groupe naphtyle, facultativement mono-, di- ou tri-substitué par R^{t};
e) un groupe hydrocarbure aromatique monocyclique comportant cinq atomes de cycle, comportant un atome de carbone qui est le point d'attache, ayant un atome de carbone remplacé par >O, >S, >NH ou >N(alkyle en C_{1 à 4}), ayant jusqu'à un atome de carbone additionnel facultativement remplacé par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono-, di- ou tri-substitué par R^{t}; et
f) un groupe hydrocarbure aromatique monocyclique comportant six atomes de cycle, ayant un atome de carbone qui est le point d'attache, ayant un ou deux atomes de carbone remplacés par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono- ou di-substitué par R^{t};
et ses énantiomères, diastéréoisomères, hydrates, solvates, et sels, esters et amides pharmaceutiquement acceptables, à condition que:
lorsque n vaut 1, L est -O-, Ar est un groupe phényle non substitué et R² et R³ sont tous deux un groupe méthyle, alors R⁴ ne soit pas -OH.

14. Composé de la revendication 13, dans lequel L est -0-et n vaut 1.

15. Composé de la revendication 13, dans lequel L est -C≡C-et n vaut 0.

16. Composé de la revendication 13 dans lequel L est -CH₂CH₂- et n vaut 0.

17. Composé de la revendication 13, dans lequel R¹ est un groupe -alkyle en C_{1 à 4}.

18. Composé de la revendication 13, dans lequel R¹ est choisi dans le groupe consistant en un atome d'hydrogène, un groupe méthyle, éthyle, propyle, isopropyle, butyle, t-butyle, pentyle, hexyle, benzyle, vinyle, allyle, propargyle, cyclopropyle, cyclobutyle, cyclopentyle, cyclohexyle, cyclopropylméthyle, -COOCH₃, -COO-t-butyle et -COObenzyle.

19. Composé de la revendication 13, dans lequel R¹ est un groupe méthyle, éthyle, propyle, t-butyle, allyle, propargyle ou benzyle.

20. Composé de la revendication 13, dans lequel R¹ est un atome d'hydrogène ou un groupe méthyle.

21. Composé de la revendication 13, dans lequel R¹ est un groupe méthyle.

22. Composé de la revendication 13, dans lequel lorsque R² est un groupe méthyle, R³ n'est pas un groupe méthyle.

23. Composé de la revendication 13, dans lequel R² et R³ sont un atome d'hydrogène, ou, facultativement substitués, sont indépendamment choisis dans le groupe consistant en:
A) un groupe méthyle, éthyle, propyle, isopropyle, sec-butyle, butyle, pentyle, hexyle, vinyle, allyle, propargyle, cyclopropyle, cyclobutyle, cyclopentyle, cyclohexyle, cyclopropylméthyle, dicyclopropylméthyle, benzhydryle, benzyle, -C(O)O-t-butyle,
B) un groupe phényle, pyridyle, 4-, 5-, 6- ou 7-benzoxazolyle, 4-, 5-, 6- ou 7-benzothiophényle, 4-, 5-, 6-ou 7-benzofuranyle, 4-, 5-, 6- ou 7-indolyle, 4-, 5-, 6- ou 7-benzthiazolyle, 4-, 5-, 6- ou 7-benzimidazolyle, 4-, 5-, 6- ou 7-indazolyle, imidazo[1,2-a]pyridin-5, 6, 7 ou 8-yle, pyrazolo[1,5-a]pyridin-4, 5, 6 ou 7-yle, 1H-pyrrolo[2,3-b]pyridin-4, 5 ou 6-yle, 1H-pyrrolo[3,2-c]pyridin-4, 6 ou 7-yle, 1H-pyrrolo[2,3-c]pyridin-4, 5 ou 7-yle, 1H-pyrrolo[3,2-b]pyridin-5, 6 ou 7-yle,
C) un groupe naphtyle,
D) un groupe azétidinyle, pyrrolidinyle, pipéridinyle, tétrahydropyranyle, et
E) un groupe furanyle, oxazolyle, 1,2,3-oxadiazolyle, 1,2,4-oxadiazolyle, 1,2,5-oxadiazolyle, 1,3,4-oxadiazolyle, thiophényle, thiazolyle, isothiazolyle, pyrrolyle, imidazolyle, pyrazolyle, 1,2,3-triazolyle, 1,2,4-triazolyle, 3-indoxazinyle, 2-benzoxazolyle, 2- ou 3-benzothiophényle, 2- ou 3-benzofuranyle, 2- ou 3-indolyle, 2-benzthiazolyle, 2-benzimidazolyle et 3-indazolyle.

24. Composé de la revendication 13, dans lequel R² et R³ sont indépendamment choisis dans le groupe consistant en un groupe méthyle, éthyle, propyle, isopropyle, sec-butyle, hydroxyéthyle, 2-hydroxy-2-méthylpropyle, allyle, cyclopropyle, cyclobutyle, cyclopentyle, dicyclopropylméthyle, pyrrolidinyle, pipéridinyle, N-méthylpipéridinyle, tétrahydropyranyle, 2-benzothiazolyle et méthoxyéthyle.

25. Composé de la revendication 13, dans lequel R² et R³ sont chacun indépendamment un atome d'hydrogène, un groupe éthyle, isopropyle, méthoxyéthyle, 2-benzothiazolyle, cyclopropyle, cyclobutyle ou cyclopentyle.

26. Composé de la revendication 13, dans lequel R² et R³, facultativement substitués, sont pris conjointement avec l'atome d'azote auquel ils sont attachés pour former un groupe tétrahydropyrimidin-2-ylidényle, ou un cycle choisi dans le groupe consistant en un groupe azétidinyle, pyrrolidinyle, pipéridinyle, morpholinyle, thiomorpholinyle, pipérazinyle, homopipéridinyle, 1,3-dihydro-isoindol-2-yle, 5,6-dihydro-4H-pyrimidin-1-yle et 1,1-dioxo-1λ⁶-thiomorpholin-4-yle.

27. Composé de la revendication 13, dans lequel R^{f}, facultativement substitué, est un groupe méthyle, éthyle, isopropyle, allyle, cyclopropyle, *tert*-butoxycarbonyle, tétrahydrofuranylméthyle, [1,3]-dioxolan-ylméthyle, thiazolyle, thiophényle, thiophénylméthyle, pyridinyle, phényle, acétyle, isobutyryle, cyclopropanecarbonyle, cyclobutanecarbonyle, pyridinyle, pyridine-carbonyle, 1H-pyrrole-carbonyle et 1H-imidazole-carbonyle.

28. Composé de la revendication 13, dans lequel R² et R³ sont pris conjointement avec l'atome d'azote auquel ils sont attachés pour former un cycle hétérocyclique à 4 à 8 chaînons, ledit cycle hétérocyclique étant choisi parmi la pipéridine, la pyrrolidine et la morpholine, ledit cycle étant substitué par 1 ou 2 substituants R^{ff}.

29. Composé de la revendication 13, dans lequel R^{ff} est choisi dans le groupe consistant en un groupe méthyle, éthyle, isopropyle, butyle, hexyle, -CHF₂, benzhydryle, -CF₃, vinyle, allyle, propargyle, cyclopropyle, cyclopentyle, cyclopropylméthyle, cyclobutyléthyle, bromo, chloro, fluoro, iodo, -OH, hydroxyméthyle, hydroxyéthyle, méthoxy, éthoxy, isopropoxy, pentyloxy, -O(CH₂)₂O-, O(CH₂)₃O-, -CN, amino, méthylamino, diméthylamino, diéthylamino, diéthylcarbamoyle, méthanesulfanyle, méthanesulfonyle, méthanesulfonamido, -C(O)Rⁱ, -COOH et éthoxycarbonyle.

30. Composé de la revendication 13, dans lequel R^{ff} est choisi dans le groupe consistant en un groupe méthyle, fluoro, -OH, -CF₃, hydroxyméthyle, hydroxyéthyle, benzhydryle, diméthylamino, éthoxycarbonyle, cyano et -O(CH₂)₂O-.

31. Composé de la revendication 13, dans lequel Rⁱ est choisi dans le groupe consistant en un groupe méthyle, pyridyle, isopropyle, cyclobutyle, cyclopropyle, N-méthylpyrrolyle et 1-méthylimidazolyle.

32. Composé de la revendication 13, dans lequel R² et R³ sont pris conjointement avec l'atome d'azote auquel ils sont attachés pour former un groupe azétidinyle, 3,3-difluoroazétidinyle, 3-benzhydryl-azétidinyle, 2-méthyl-pyrrolidinyle, 3-hydroxypyrrolidinyle, 3-diméthyl-aminopyrrolidinyle, 2,5-diméthylpyrrolidinyle, 2-trifluoro-méthylpyrrolidinyle, 2-hydroxyméthylpyrrolidinyle, 3,3-difluoropyrrolidinyle, pipéridinyle, 4-fluoropipéridinyle, 3-fluoropipéridinyle, 3,3-difluoropipéridinyle, 4,4-difluoropipéridinyle, 3-trifluorométhylpipéridinyle, 4-trifluorométhylpipéridinyle, 1,4-dioxa-8-aza-spiro[4.5]déc-8-yle, 4-cyanopipéridinyle, 4-carboéthoxypipéridinyle, 3-hydroxypipéridinyle, 4-hydroxypipéridinyle, 2-hydroxyméthylpipéridinyle, 3-hydroxyméthylpipéridinyle, 4-hydroxyméthylpipéridinyle, 3-hydroxyéthylpipéridinyle, 4-hydroxyéthylpipéridinyle, morpholinyle, 2-méthylmorpholin-4-yle, 3-méthylmorpholin-4-yle, 3-hydroxyméthylmorpholin-4-yle, 2-hydroxyméthylmorpholin-4-yle, 4-méthyl-pipérazin-1-yle, 4-éthyl-pipérazin-1-yle, 4-isopropyl-pipérazin-yle, 4-allyl-pipérazin-1-yle, 4-cyclopropyl-pipérazin-1-yle, 4-(2-hydroxyéthyl)pipérazin-1-yle, 4-(2-méthoxyéthyl)-pipérazin-1-yle, 4-(tert-butoxycarbonyl)pipérazin-1-yle, 4-(tétrahydrofuran-2-ylméthyl)pipérazin-1-yle, 4-[1,3]-dioxolan-2-ylméthyl-pipérazin-1-yle, 4-thiazol-2-yl-pipérazin-1-yle, 4-(2-thiophényl)pipérazinyle, 4-thiophén-2-ylméthyl-pipérazin-1-yle, 4-(pyridin-4-yl)-pipérazin-1-yle, 4-phénylpipérazin-1-yle, 4-(2-hydroxy-phényl)pipérazinyle, 4-(4-trifluorométhyl-phényl)-pipérazin-1-yle, 4-(4-cyanophényl)pipérazin-1-yle, 4-acétylpipérazin-1-yle, 4-isobutyryl-pipérazin-1-yle, 4-cyclopropanecarbonyl-pipérazin-1-yle, 4-cyclobutanecarbonyl-pipérazin-1-yle, 4-pyridin-4-yl-pipérazin-1-yle, 4-(pyridine-4-carbonyl)-pipérazin-1-yle, 4-(1-méthyl-1H-pyrrole-2-carbonyl)-pipérazin-1-yle, 4-(1-méthyl-1H-imidazole-4-carbonyl)-pipérazin-1-yle, 1,1-dioxo-1λ⁶-thiomorpholin-4-yle, 2,6-diméthylmorpholin-4-yle et 1,3-dihydro-isoindol-2-yle, 5,6-dihydro-4H-pyrimidin-1-yle.

33. Composé de la revendication 13, dans lequel R² et R³ sont pris conjointement avec l'atome d'azote auquel ils sont attachés pour former un groupe pipéridinyle, 4-fluoropipéridinyle, 4,4-difluoropipéridinyle, morpholinyle ou 3-méthylmorpholin-4-yle.

34. Composé de la revendication 13, dans lequel R⁴ est un groupe hydroxy, méthoxy, éthoxy, isopropoxy, pentyloxy, -CF₃, méthyle, éthyle, propyle, isobutyle, pentyle, chloro ou fluoro.

35. Composé de la revendication 13, dans lequel R⁴ est un groupe hydroxy, méthyle, méthoxy, fluoro ou -CF₃.

36. Composé de la revendication 13, dans lequel deux R⁴ sont pris ensemble pour former un groupe méthylène.

37. Composé de la revendication 13, dans lequel R² et l'un de R⁴ sont pris ensemble pour former un groupe méthylène, éthylène ou -CH₂-CH₂O-.

38. Composé de la revendication 13, dans lequel m vaut 0.

39. Composé de la revendication 13, dans lequel R⁵ est un groupe méthyle, éthyle, isopropyle, hexyle, hydroxy, méthoxy, éthoxy, isopropoxy, méthylsulfanyle, bromo, chloro, fluoro ou iodo.

40. Composé de la revendication 13, dans lequel R⁵ est un groupe méthyle, hydroxy ou fluoro.

41. Composé de la revendication 13, dans lequel Ar¹, facultativement substitué, est choisi dans le groupe consistant en:
a) un groupe phényle, 5-, 6-, 7-, 8-benzo-1,4-dioxanyle, 4-, 5-, 6-, 7-benzo-1,3-dioxolyle, 4-, 5-, 6-, 7-indolinyle, 4-, 5-, 6-, 7-isoindolinyle, 1,2,3,4-tétrahydro-quinoléin-4, 5, 6 ou 7-yle, 1,2,3,4-tétrahydro-isoquinoléin-4, 5, 6 ou 7-yle,
b) un groupe 4-, 5-, 6- ou 7-benzoxazolyle, 4-, 5-, 6-ou 7-benzothiophényle, 4-, 5-, 6- ou 7-benzofuranyle, 4-, 5-, 6- ou 7-indolyle, 4-, 5-, 6- ou 7-benzthiazolyle, 4-, 5-, 6- ou 7-benzimidazolyle, 4-, 5-, 6- ou 7-indazolyle, imidazo[1,2-a]pyridin-5, 6, 7 ou 8-yle, pyrazolo[1,5-a]pyridin-4, 5, 6 ou 7-yle, 1H-pyrrolo[2,3-b]pyridin-4, 5 ou 6-yle, 1H-pyrrolo[3,2-c]pyridin-4, 6 ou 7-yle, 1H-pyrrolo[2,3-c]pyridin-4, 5 ou 7-yle, 1H-pyrrolo[3,2-b]pyridin-5, 6 ou 7-yle,
c) un groupe 5-, 6-, 7- ou 8-isoquinoléinyle, 5-, 6-, 7- ou 8-quinoléinyle, 5-, 6-, 7- ou 8-quinoxalinyle, 5-, 6-, 7- ou 8-quinazolinyle,
d) un groupe naphtyle,
e) un groupe furanyle, oxazolyle, isoxazolyle, 1,2,3-oxadiazolyle, 1,2,4-oxadiazolyle, 1,2,5-oxadiazolyle, 1,3,4-oxadiazolyle, thiophényle, thiazolyle, isothiazolyle, pyrrolyle, imidazolyle, pyrazoyle, 1,2,3-triazolyle, 1,2,4-triazolyle, 3-indoxazinyle, 2-benzoxazolyle, 2- ou 3-benzothiophényle, 2- ou 3-benzofuranyle, 2- ou 3-indolyle, 2-benzthiazolyle, 2-benzimidazolyle, 3-indazolyle, et
f) un groupe pyridinyle, pyridinyl-N-oxyde, pyrazinyle, pyrimidinyle, pyridazinyle, 1-, 3- ou 4-isoquinoléinyle, 2-, 3- ou 4-quinoléinyle, 2- ou 3-quinoxalinyle, 2- ou 4-quinazolinyle, [1,5], [1,6], [1,7] ou [1,8]naphtyridin-2-, 3- ou 4-yle, [2,5], [2,6], [2,7], [2,8]naphtyridin-1-, 3- ou 4-yle.

42. Composé de la revendication 13, dans lequel Ar¹, facultativement substitué, est choisi dans le groupe consistant en un groupe phényle, pyridyle, pyrazinyle, thiazolyle, pyrazolyle et thiophényle.

43. Composé de la revendication 13, dans lequel G est une liaison ou -S-.

44. Composé de la revendication 13, dans lequel Ar² est choisi dans le groupe consistant en un groupe phényle, pyrrolyle, furanyle, thiophényle, pyrazolyle, imidazolyle, oxazolyle, thiazolyle, isoxazolyle, triazolyle, tétrazolyle, pyridinyle, pyrimidinyle ou pyrazinyle.

45. Composé de la revendication 13, dans lequel Ar¹ est choisi dans le groupe consistant en un groupe phényle, 2-méthoxyphényle, 3-méthoxyphényle, 4-méthoxyphényle, 4-éthoxyphényle, 2,4-diméthoxyphényle, 2,5-diméthoxyphényle, 3,4-diméthoxyphényle, 3,5-diméthoxyphényle, 3,4,5-triméthoxyphényle, 2-méthylphényle, 3-méthylphényle, 4-méthylphényle, 4-éthylphényle, 3-éthynylphényle, 4-éthynylphényle, 2-chlorophényle, 3-chlorophényle, 4-chlorophényle, 2-fluorophényle, 3-fluorophényle, 4-fluorophényle, 2-bromophényle, 3-bromophényle, 4-bromophényle, 3-iodophényle, 4-iodophényle, 2-trifluorométhylphényle, 3-trifluorométhylphényle, 4-trifluorométhylphényle, 3-trifluorométhoxyphényle, 4-trifluorométhoxyphényle, 4-difluorométhoxyphényle, 3-cyanophényle, 4-cyanophényle, 3-acétylphényle, 4-acétylphényle, 3,4-difluorophényle, 3,4-dichlorophényle, 2,3-difluorophényle, 2,3-dichlorophényle, 2,4-difluorophényle, 2,4-dichlorophényle, 2,5-dichlorophényle, 3,5-dichlorophényle, 3-nitrophényle, 4-nitrophényle, 3-chloro-4-fluorophényle, 3-chloro-4-méthoxyphényle, 3-chloro-4-difluorométhoxyphényle, 3-fluoro-4-chlorophényle, 2-fluoro-4-méthoxyphényle, 3-fluoro-4-méthoxyphényle, benzo[1,3]dioxol-4 ou 5-yle, 2-hydroxyphényle, 3-hydroxyphényle, 4-hydroxyphényle, 4-hydroxy-2-méthylphényle, 4-hydroxy-3-fluorophényle, 3,4-dihydroxyphényle, 4-aminophényle, 4-diméthylaminophényle, 4-morpholin-4-yl-phényle, 4-carbamoylphényle, 4-fluoro-3-méthylphényle, 4-méthanesulfanylphényle, 4-méthanesulfinyl-phényle, 4-méthanesulfonylphényle, 4-trifluorométhane-sulfanylphényle, thiophén-2-yle, thiophén-3-yle, 2-pyridinyle, 3-pyridinyle, 4-pyridinyle, 2-chloro-5-pyridinyle, 2-diméthylamino-5-pyridinyle, 6-méthoxy-pyridin-3-yle, 6-méthylsulfanyl-pyridin-3-yle, 2-hydroxy-5-pyridinyle, 6-pyrazol-1-yl-pyridin-3-yle, 6-bromo-pyridin-3-yle, 6-éthynyl-pyridin-3-yle, 6-triméthylsilanyléthynyl-pyridin-3-yle, 6-phénylsulfanyl-pyridin-3-yle, 6-imidazol-1-yl-pyridin-3-yle, 6-(1H-imidazol-2-ylsulfanyl)-pyridin-3-yle, 6-(pyrimidin-2-ylsulfanyl)-pyridin-3-yle, oxazol-5-yle, thiazol-5-yle, thiazol-2-yle, 2H-pyrazol-3-yle, pyrazin-2-yle, 1-naphtyle, 2-naphtyle, 4-imidazol-1-ylphényle, 4-pyrazol-1-ylphényle, 1H-indol-5-yle, 1H-benzimidazol-5-yle, benzo[b]thiophén-7-yle et 4-biphényle.

46. Composé de la revendication 13, dans lequel Ar¹, facultativement substitué avec un halogéno, est un groupe 4-méthoxyphényle, 4-méthanesulfanylphényle ou 4-chlorophényle.

47. Composé de la revendication 13, dans lequel ledit sel pharmaceutiquement acceptable est un sel d'addition amino efficace.

48. Composé de la revendication 13, dans lequel ledit sel pharmaceutiquement acceptable est choisi dans le groupe consistant en le bromhydrate, le chlorhydrate, le sulfate, le bisulfate, le nitrate, l'acétate, l'oxalate, le valérate, l'oléate, le palmitate, le stéarate, le laurate, le borate, le benzoate, le lactate, le phosphate, le tosylate, le citrate, le maléate, le fumarate, le succinate, le tartrate, le naphtylate, le mésylate, le glucoheptonate, le lactiobionate et le laurylsulfonate.

49. Composé de la revendication 13, marqué isotopiquement pour être détectable par PET ou SPECT.

50. Procédé pour étudier des troubles véhiculés par récepteur histaminique H₃ et sérotonine comprenant l'étape d'utilisation d'un composé ¹⁸F marqué ou ¹¹C marqué de la revendication 13 en tant que sonde moléculaire de tomographie par émission de positons (PET).

51. Composé de formule (II): 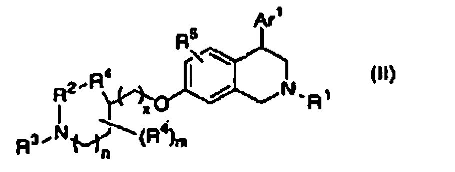 dans laquelle
n vaut 1 ou 2;
x vaut 0 ou 1;
où n + x vaut 1 ou 2;
R¹ est -H; ou est un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyle en C_{3 à 7}, -COOalkyle en C_{1 à 6} ou -COObenzyle, chacun facultativement mono-, di- ou tri-substitué par R^{a};
où R^{a} est choisi parmi un groupe -OH, -Oalkyle en C_{1 à 6}, phényle facultativement substitué par un groupe -Oalkyle en C_{1 à 4} ou halogéno, -CN, -NO₂-, -N(R^{b})R^{c}, -C(O)N(R^{b})R^{c}, -N(R^{b})C(O)R^{b}, -N(R^{b})SO₂alkyle en C_{1 à 6}, -C(O)alkyle en C_{1 à 6}, -S(O)_{0 à 2}-alkyle en C_{1 à 6}, -SO₂N(R^{b})R^{c}, -SCF₃, halogéno, -CF₃, -OCF₃, -COOH et -COOalkyle en C_{1 à 6;}
où R^{b} et R^{c} sont chacun indépendamment -H ou un groupe -alkyle en C_{1 à 6};
R³ est choisi dans le groupe consistant en: -H;
A) un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyl, en C_{3 à 7}, -CH(cycloalkyle en C_{3 à 8})₂, -CH(phényle)₂, benzyle et -C(O)Oalkyle en C_{1 à 4}, où chaque groupe alkyle, cycloalkyle ou benzyle est facultativement substitué par un groupe -OH, -Oalkyle en C_{1 à 4}, -CN, -NH₂, -NH (alkyle en C_{1 à 4}), -N(alkyle en C_{1 à 4})₂, halogéno, -CF₃, -OCF₃, -COOH ou -COOalkyle en C_{1 à 6};
B) un groupe phényle ou pyridyle, facultativement condensé au niveau de deux chaînons de cycle de carbone adjacents à un fragment hydrocarbure à trois ou quatre chaînons pour former un cycle aromatique condensé à cinq ou six chaînons, lequel fragment a un atome de carbone remplacé par >O, >S, >NH, >N(alkyle en C_{1 à 4}) ou >NC(O)Oalkyle en C_{1 à 4}, et lequel fragment a jusqu'à un atome de carbone additionnel facultativement remplacé par -N=;
C) un groupe naphtyle,
D) un cycle hétérocyclique à 4 à 8 chaînons, ledit cycle hétérocyclique ayant un atome de carbone qui est le point d'attache, comportant 1 ou 2 chaînons hétéroatome choisis dans le groupe consistant en >O, >S(O)_{0 à 2}, >NH, >N(alkyle en C_{1 à 4}), et >NC(O)Oalkyle en C_{1 à 4}, et comportant 0 ou 1 double liaison; et
E) un groupe hydrocarbure aromatique monocyclique comportant cinq ou six atomes de cycle, ayant un atome de carbone qui est le point d'attache, ayant un atome de carbone remplacé par >O, >S, >NH, >N(alkyle en C_{1 à 4}) ou >NC(O)Oalkyle en C_{1 à 4}, ayant jusqu'à un atome de carbone additionnel facultativement remplacé par -N=, et facultativement benzocondensé ou pyridocondensé;
où chacun de B) à E) est facultativement mono-, di- ou tri-substitué par un fragment choisi dans le groupe consistant en un groupe -alkyle en C_{1 à 4}, -OH, -alkyl en C_{1 à 4}OH, -Oalkyle en C_{1 à 6}, -CN, -NO₂, -N(R^{d})R^{e}, -C(O)N(R^{d})R^{e}, -N(R^{d})C(O)R^{d}, -N(R^{d})SO₂alkyle en C_{1 à 6}, -C(O)alkyle en C_{1 à 6}, -S(O)_{0 à 2}-alkyle en C_{1 à 6}, -SO₂N(R^{d})R^{e}, -SCF₃, halogéno, -CF₃, -OCF₃, -COOH, -COOalkyle en C_{1 à 4}, -OC(O)N(R^{d})R^{e} et -OC(O)Oalkyle en C_{1 à 6};
où R^{d} et R^{e} sont chacun indépendamment -H ou un groupe -alkyle en C_{1 à 6;}
-R²-R⁴- est un groupe méthylène, éthylène, propylène, ou -CH₂CH₂O-, où chacun est facultativement substitué par un groupe -OH, -Oalkyle en C_{1 à 6}, -CF₃, -alkyle en C_{1 à 6}, amino ou halogéno;
R⁴ est un groupe -OH, -Oalkyle en C_{1 à 6}, -CF₃, -alkyle en C_{1 à 6} ou halogéno,
m vaut 0, 1 ou 2;
R⁵ est choisi dans le groupe consistant en un groupe -alkyle en C_{1 à 6}, -OH, -Oalkyle en C_{1 à 6}, -Salkyle en C_{1 à 6} et halogéno;
Ar¹ est un cycle aryle ou hétéroaryle choisi dans le groupe consistant en:
a) un groupe phényle, facultativement mono-, di- ou tri-substitué par R^{j} et facultativement di-substitué sur des atomes de carbone adjacents par un groupe -Oalkylène en C_{1 à 4}O- facultativement mono- ou di-substitué par un groupe fluoro, -(CH₂)_{2 à 3}-NH-, -(CH₂)_{1 à 2}NH(CH₂)-, -(CH₂)_{2 à 3}-N(alkyle en C_{1 à 4})-, ou -(CH₂)_{1 à 2}N(alkyl en C_{1 à 4})(CH₂)-;
où R^{j} est choisi dans le groupe consistant en
1) un groupe -OH, -alkyle en C_{1 à 6}, -Oalkyle en C_{1 à 6} facultativement mono-, di- ou tri-substitué par un groupe halogéno, -alcényle en C_{2 à 6}, -Oalcényle en C_{3 à 6}, -alcynyle en C_{2 à 6} facultativement substitué par un groupe triméthylsilyle, -Oalcynyle en C_{3 à 6}, -cycloalkyle en C_{3 à 6}, -Ocycloalkyle en C_{3 à 6}, -CN, -NO₂, -N(R^{k})R^{j}, -N(R^{k})C(O)R^{l}, -N(R^{k})SO₂alkyle en C_{1 à 6}, -C(O)alkyle en C_{1 à 6}, -S(O)_{0 à 2}-alkyle en C_{1 à 6}, -C(O)N(R^{m})Rⁿ, -SO₂N(R^{m})Rⁿ, -SCF₃, halogéno, -CF₃, -COOH, -COOalkyle en C_{1 à 6} et -COOcycloalkyle en C_{3 à 7};
où R^{k} et P^{l} sont chacun indépendamment -H ou un groupe -alkyle en C_{1 à 6};
où R^{m} et Rⁿ sont chacun indépendamment -H ou un groupe -alkyle en C_{1 à 6}, ou R^{m} et Rⁿ pris conjointement avec leur atome d'azote d'attache forment un cycle hétérocyclique à 4 à 8 chaînons comportant 1 ou 2 chaînons hétéroatome choisis parmi >O, >S(O)_{0 à 2}, >NH et >Nalkyle en C_{1 à 6}, comportant 0 ou 1 double liaison, comportant 0 ou 1 chaînon carbonyle;
2) -G-Ar², où G est une liaison, -0- ou -S-, et Ar² est un groupe phényle ou est un groupe hydrocarbure aromatique monocyclique comportant cinq ou six atomes de cycle, comportant un atome de carbone remplacé par >O, >S, >NH, ou >N(alkyle en C_{1 à 4}), comportant jusqu'à un atome de carbone additionnel facultativement remplacé par -N=, chacun facultativement mono-, di- ou tri-substitué par R^{p};
où R^{p} est un substituant indépendamment choisi dans le groupe consistant en: un groupe -OH, -alkyle en C_{1 à 6}, -Oalkyle en C_{1 à 6}, phényle, -CN, -NO₂, -N(R^{q})R^{r}, -C(O)N(R^{q})R^{r}, -N(R^{q})C(O)R^{r}, -N(R^{q})SO₂alkyle en C_{1 à 6}, -C (O)alkyle en C_{1 à 6}, -S(O)_{0 à 2}-alkyle en C_{1 à 6}, -SO₂N(R^{q})R^{r}, -SCF₃, halogéno, -CF₃, -OCF₃, -OCHF₂, -COOH et -COOalkyle en C_{1 à 6};
où R^{q} et R^{r} sont chacun indépendamment choisis parmi -H, un groupe -alkyle en C_{1 à 6} et -alcényle en C_{2 à 6}; et
3) un cycle hétérocyclique saturé ou partiellement saturé à 4 à 8 chaînons, comportant 1 ou 2 chaînons hétéroatome choisis parmi >O, >S(O)_{0 à 2}, >NH et >Nalkyle en C_{1 à 6}, comportant 0 ou 1 chaînon carbonyle, ledit cycle étant facultativement mono-, di- ou tri-substitué par R^{p;}
b) un groupe phényle ou pyridyle condensé au niveau de deux chaînons de cycle de carbone adjacents à un fragment hydrocarbure à trois chaînons pour former un cycle aromatique condensé à cinq chaînons, lequel fragment a un atome de carbone remplacé par >O, >S, >NH ou >N(alkyle en C_{1 à 4}), et lequel fragment a jusqu'à un atome de carbone additionnel facultativement remplacé par -N=, les cycles condensés étant facultativement mono-, di- ou tri-substitués par _{R}^{t};
où R^{t} est un substituant indépendamment choisi dans le groupe consistant en: un groupe -OH, -alkyle en C_{1 à 6}, -Oalkyle en C_{1 à 6}, phényle, -CN, -NO₂, -N(R^{u})R^{v}, -C(O)N(R^{u})R^{v}, -N(R^{u})C(O)R^{v}, -N(R^{u})SO₂alkyle en C_{1 à 6}, -C(O)alkyle en C_{1 à 6}, -S(O)_{0 à 2}-alkyle en C_{1 à 6}, -SO₂N(R^{u})R^{v}, -SCF₃, halogéno, -CF₃, -OCF₃, -OCHF₂, -COOH et -COOalkyle en C_{1 à 6};
où R^{u} et R^{v} sont chacun indépendamment -H ou un groupe -alkyle en C_{1 à 6};
c) un groupe phényle condensé au niveau de deux chaînons de cycle adjacents à un fragment hydrocarbure à quatre chaînons pour former un cycle aromatique condensé à six chaînons, lequel fragment a un ou deux atomes de carbone remplacés par -N=, les cycles condensés étant facultativement mono-, di- ou tri-substitués par R^{t};
d) un groupe naphtyle, facultativement mono-, di- ou tri-substitué par R^{t};
e) un groupe hydrocarbure aromatique monocyclique comportant cinq atomes de cycle, comportant un atome de carbone qui est le point d'attache, ayant un atome de carbone remplacé par >O, >S, >NH ou >N(alkyle en C_{1 à 4}), ayant jusqu'à un atome de carbone additionnel facultativement remplacé par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono-, di- ou tri-substitué par R^{t}; et
f) un groupe hydrocarbure aromatique monocyclique comportant six atomes de cycle, ayant un atome de carbone qui est le point d'attache, ayant un ou deux atomes de carbone remplacés par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono- ou di-substitué par R^{t};
et ses énantiomères, diastéréoisomères, hydrates, solvates, et sels, esters et amides pharmaceutiquement acceptables.

52. Composé de la revendication 51, dans lequel n vaut 1 et x vaut 0.

53. Composé de la revendication 51, dans lequel n vaut 1 et x vaut 1.

54. Composé de la revendication 51, dans lequel n vaut 2 et x vaut 0.

55. Composé de la revendication 51, dans lequel R¹ est un groupe méthyle.

56. Composé de la revendication 51, dans lequel R³ est un atome d'hydrogène, un groupe éthyle, isopropyle, méthoxyéthyle, 2-benzothiazolyle, cyclopropyle, cyclobutyle ou cyclopentyle.

57. Composé de la revendication 51, dans lequel R⁴ est un groupe méthyle, hydroxy, méthoxy, -CF₃, méthyle, fluoro ou chloro.

58. Composé de la revendication 51, dans lequel m vaut 0.

59. Composé de la revendication 51, dans lequel R⁵ est un groupe méthyle, hydroxy, méthoxy, méthylsulfanyle, fluoro ou chloro.

60. Composé de la revendication 51, dans lequel Ar¹, facultativement substitué par un halogéno, est un groupe 4-méthoxyphényle, 4-méthanesulfanylphényle ou 4-chlorophényle.

61. Composé de la revendication 51, dans lequel -R²-R⁴- est un groupe méthylène.

62. Composé de la revendication 51, dans lequel -R²-R⁴- est un groupe éthylène.

63. Composé de la revendication 51, dans lequel -R²-R⁴- est -CH₂-CH₂O-.

64. Composé choisi dans le groupe consistant en:
la 7-[3-(4-fluoro-pipéridin-1-yl)-propoxy]-4-(4-méthoxy-phényl)-2-méthyl-1,2,3,4-tétrahydro-isoquinoléine;
la 7-[3-(4-fluoro-pipéridin-1-yl)-propoxy]-4-(4-méthoxy-phényl)-2-méthyl-1,2,3,4-tétrahydro-isoquinoléine (énantiomère 1);
la 7-[3-(4-fluoro-pipéridin-1-yl)-propoxy]-4-(4-méthoxy-phényl)-2-méthyl-1,2,3,4-tétrahydro-isoquinoléine (énantiomère 2);
la 1-(4-{3-[4-(4-méthoxy-phényl)-2-méthyl-1,2,3,4-tétrahydro-isoquinoléin-7-yloxy]-propyl}-pipérazin-1-yl)-éthanone;
la diéthyl-{3-[4-(4-méthoxy-phényl)-2-méthyl-1,2,3,4-tétrahydro-isoquinoléin-7-yloxy]propyl}-amine;
la (4-{3-[4-(4-méthoxy-phényl)-2-méthyl-1,2,3,4-tétrahydro-isoquinoléin-7-yloxy]-propyl}-pipérazin-1-yl)-pyridin-4-yl-méthanone;
la 1-(4-{3-[4-(4-méthoxy-phényl)-2-méthyl-1,2,3,4-tétrahydro-isoquinoléin-7-yloxy]-propyl}-pipérazin-1-yl)-2-méthyl-propan-1-one;
la cyclobutyl-(4-{3-[4-(4-méthoxy-phényl)-2-méthyl-1,2,3,4-tétrahydro-isoquinoléin-7-yloxy]-propyl}-pipérazin-1-yl)-méthanone;
la cyclopropyl-(4-{3-[4-(4-méthoxy-phényl)-2-méthyl-1,2,3,4-tétrahydro-isoquinoléin-7-yloxy]-propyl}-pipérazin-1-yl)-méthanone;
la 7-[3-(4,4-difluoro-pipéridin-1-yl)-propoxy]-4-(4-méthoxy-phényl)-2-méthyl-1,2,3,4-tétrahydro-isoquinoléine;
la 4-(4-méthoxy-phényl)-2-méthyl-7-(3-morpholin-4-yl-propoxy)-1,2,3,4-tétrahydro-isoquinoléine;
la (1-{3-[4-(4-méthoxy-phényl)-2-méthyl-1,2,3,4-tétrahydro-isoquinoléin-7-yloxy]-propyl}-pyrrolidin-3-yl)-diméthyl-amine;
la 7-[3-((2R,5R)-trans-diméthyl-pyrrolidin-1-yl)-propoxy]-4-(4-méthoxy-phényl)-2-méthyl-1,2,3,4-tétrahydro-isoquinoléine;
l'ester d'éthyle d'acide 4-{3-[4-(4-méthoxy-phényl)-2-méthyl-1,2,3,4-tétrahydro-isoquinoléin-7-yloxy]-propyl}-pipérazine-1-carboxylique;
la (4-{3-[4-(4-méthoxy-phényl)-2-méthyl-1,2,3,4-tétrahydro-isoquinoléin-7-yloxy-propyl}-pipérazin-1-yl)-(1-méthyl-1H-pyrrol-2-yl)-méthanone;
la (4-{3-[4-(4-méthoxy-phényl)-2-méthyl-1,2,3,4-tétrahydro-isoquinoléin-7-yloxy]propyl}-pipérazin-1-yl)-(1-méthyl-1H-imidazol-4-yl)-méthanone;
la (1,3-diméthyl-tétrahydro-pyrimidin-2-ylidène)-{3-[4-(4-méthoxy-phényl)-2-méthyl-1,2,3,4-tétrahydro-isoquinoléin-7-yloxy]-propyl}-amine;
la 7-[3-(1,3-dihydro-isoindol-2-yl)-propoxy]-4-(4-méthoxy-phényl)-2-méthyl-1,2,3,4-tétrahydro-isoquinoléine;
la bis-(2-méthoxy-éthyl)-{3-[4-(4-méthoxy-phényl)-2-méthyl-1,2,3,4-tétrahydro-isoquinoléin-7-yloxy]-propyl}-amine;
la 7-[3-(5,6-dihydro-4H-pyrimidin-1-yl)-propoxy]-4-(4-méthoxy-phényl)-2-méthyl-1,2,3,4-tétrahydro-isoquinoléine;
la benzothiazol-2-yl-{3-[4-(4-méthoxy-phényl)-2-méthyl-1,2,3,4-tétrahydro-isoquinoléin-7-yloxy]-propyl}-méthyl-amine;
le 1-{3-[4-(4-méthoxy-phényl)-2-méthyl-1,2,3,4-tétrahydro-isoquinoléin-7-yloxy]-propyl}-pipéridin-3-ol;
le 1-{3-[4-(4-méthoxy-phényl)-2-méthyl-1,2,3,4-tétrahydro-isoquinoléin-7-yloxy]-propyl}-pipéridin-1-ol;
le (1-{3-[4-(4-méthoxy-phényl)-2-méthyl-1,2,3,4-tétrahydro-isoquinoléin-7-yloxy]-propyl}-pipéridin-4-yl)-méthanol;
(1-{3-[4-(4-méthoxy-phényl)-2-méthyl-1,2,3,4-tétrahydro-isoquinoléin-7-yloxy]-propyl)-pipéridin-3-yl)-méthanol;
le (1-{3-[4-(4-méthoxy-phényl)-2-méthyl-1,2,3,4-tétrahydro-isoquinoléin-7-yloxy]-propyl}-pipéridin-2-yl)-méthanol;
la 4-(4-méthoxy-phényl)-2-méthyl-7-[3-(3-trifluorométhyl-pipéridin-1-yl)-propoxy]-1,2,3,4-tétrahydro-isoquinoléine;
le 2-(1-{3-[4-(4-méthoxy-phényl)-2-méthyl-1,2,3,4-tétrahydro-isoquinoléin-7-yloxy]-propyl}-pipéridin-4-yl)-éthanol;
la 7-[3-(1,1-dioxo-1λ⁶-thiomorpholin-4-yl)-propoxy]-4-(4-méthoxy-phényl)-2-méthyl-1,2,3,4-tétrahydro-isoquinoléine;
la 4-(4-méthoxy-phényl)-2-méthyl-7-[3-((2S)-trifluorométhyl-pyrrolidin-1-yl)-propoxy]-1,2,3,4-tétrahydro-isoquinoléine;
la 7-[3-(3,3-difluoro-pipéridin-1-yl)-propoxy]-4-[4-méthoxy-phényl)-2-méthyl-1,2,3,4-tétrahydro-isoquinoléine;
la 7-[3-(3,3-difluoro-pipéridin-1-yl)-propoxy]-4-(4-méthoxy-phényl)-2-méthyl-1,2,3,4-tétrahydro-isoquinoléine (énantiomère 1);
la 7-[3-(3,3-difluoro-pipéridin-1-yl)-propoxy]-4-(4-méthoxy-phényl)-2-méthyl-1,2,3,4-tétrahydro-isoquinoléine (énantiomère 2);
le (1-{3-[4-(4-méthoxy-phényl)-2-méthyl-1,2,3,4-tétrahydro-isoquinoléin-7-yloxy]-propyl}-pyrrolidin-(2R)-yl)-méthanol;
le 1-{3-[4-(4-méthoxy-phényl)-2-méthyl-1,2,3,4-tétrahydro-isoquinoléin-7-yloxy]-propyl}-(R)-pyrrolidin-3-ol;
la 7-[3-(2,6-diméthyl-morpholin-4-yl)-propoxy]-4-(4-méthoxy-phényl)-2-méthyl-1,2,3,4-tétrahydro-isoquinoléine;
l'ester d'éthyle d'acide 1-{3-[4-(4-méthoxy-phényl)-2-méthyl-1,2,3,4-tétrahydro-isoquinoléin-7-yloxy]-propyl}-pipéridine-4-carboxylique;
la 7-(3-azétidin-1-yl-propoxy)-4-(4-méthoxy-phényl)-2-méthyl-1,2,3,4-tétrahydro-isoquinoléine;
la 4-(4-méthoxy-phényl)-2-méthyl-7-[3-(2-méthyl-pyrrolidin-1-yl)-propoxy]-1,2,3,4-tétrahydro-isoquinoléine;
le 2-(1-{3-[4-(4-méthoxy-phényl)-2-méthyl-1,2,3,4-tétrahydro-isoquinoléin-7-yloxyl-propyl}-pipéridin-2-yl)-éthanol;
le 1-{3-[4-(4-méthoxy-phényl)-2-méthyl-1,2,3,4-tétrahydro-isoquinoléin-7-yloxy]-propyl}-pipéridine-4-carbonitrile;
le 1-{3-[4-(4-méthoxy-phényl)-2-méthyl-1,2,3,4-tétrahydro-isoquinoléin-7-yloxy]-propyl}-pipéridine-4-carbonitrile (énantiomère 1);
le 1-{3-[4-(4-méthoxy-phényl)-2-méthyl-1,2,3,4-tétrahydro-isoquinoléin-7-yloxy]propyl}-pipéridine-4-carbonitrile (énantiomère 2);
la 4-(4-méthoxy-phényl)-2-méthyl-7-[3-(4-trifluorométhyl-pipéridin-1-yl)-propoxy]-1,2,3,4-tétrahydro-isoquinoléine;
la 7-[3-(3-fluoro-pipéridin-1-yl)-propoxy]-4-(4-méthoxy-phényl)-2-méthyl-1,2,3,4-tétrahydro-isoquinoléine;
l'éthyl-(2-méthoxy-éthyl)-{3-[4-(4-méthoxy-phényl)-2-méthyl-1,2,3,4-tétrahydro-isoquinoléin-7-yloxy]-propyl}-amine;
la 7-[3-(1,4-dioxa-8-aza-spiro[4.5]déc-8-yl)-propoxy]-4-(4-méthoxy-phényl)-2-méthyl-1,2,3,4-tétrahydro-isoquinoléine;
le 1-[4-(4-méthoxy-phényl)-2-méthyl-1,2,3,4-tétrahydro-isoquinoléin-7-yloxy]-3-pipéridin-1-yl-propan-2-ol;
la 7-[2-fluoro-3-(4-fluoro-pipéridin-1-yl)-propoxy]-4-(4-méthoxy-phényl)-2-méthyl-1,2,3,4-tétrahydro-isoquinoléine;
la 7-[3-(4-fluoro-pipéridin-1-yl)-propoxy]-4-(3-méthoxy-phényl)-2-méthyl-1,2,3,4-tétrahydro-isoquinoléine;
la 7-[3-(4-fluoro-pipéridin-1-yl)-propoxy]-4-(3-méthoxy-phényl)-2-méthyl-1,2,3,4-tétrahydro-isoquinoléine (énantiomère 1);
la 7-[3-(4-fluoro-pipéridin-1-yl)-propoxy]-4-(3-méthoxy-phényl)-2-méthyl-1,2,3,4-tétrahydro-isoquinoléine (énantiomère 2);
la 4-(3-chloro-phényl)-7-[3-(4-fluoro-pipéridin-1-yl)-propoxy]-2-méthyl-1,2,3,4-tétrahydro-isoquinoléine;
la 4-(4-chloro-phényl)-7-[3-(4-fluoro-pipéridin-1-yl)-propoxy]-2-méthyl-1,2,3,4-tétrahydro-isoquinoléine;
la 4-(3-fluoro-phényl)-7-[3-(4-fluoro-pipéridin-1-yl)-propoxy]-2-méthyl-1,2,3,4-tétrahydro-isoquinoléine;
la 7-[3-(4-fluoro-pipéridin-1-yl)-propoxy]-2-méthyl-4-(4-trifluorométhoxy-phényl)-1,2,3,4-tétrahydro-isoquinoléine;
la 4-(4-difluorométhoxy-phényl)-7-[3-(4-fluoro-pipéridin-1-yl)-propoxy]-2-méthyl-1,2,3,4-tétrahydro-isoquinoléine;
la 7-[3-(4-fluoro-pipéridin-1-yl)-propoxy]-4-(4-méthanesulfonyl-phényl)-2-méthyl-1,2,3,4-tétrahydro-isoquinoléine;
la 7-[3-(4-fluoro-pipéridin-1-yl)-propoxy]2-méthyl-4-(4-méthylsulfanyl-phényl)-1,2,3,4-tétrahydro-isoquinoléine;
la 4-(3-chloro-4-méthoxy-phényl)-7-[3-(4-fluoro-pipéridin-1-yl)-propoxy]-2-méthyl-1,2,3,4-tétrahydro-isoquinoléine;
la 4-(2,4-dichloro-phényl)-7-[3-(4-fluoro-pipéridin-1-yl)-propoxy]-2-méthyl-1,2,3,4-tétrahydro-isoquinoléine;
la 4-(2,5-dichloro-phényl)-7-[3-(4-fluoro-pipéridin-1-yl)-propoxy]-2-méthyl-1,2,3,4-tétrahydro-isoquinoléine;
la 4-(3,5-dichloro-phényl)-7-[3-(4-fluoro-pipéridin-1-yl)-propoxy]-2-méthyl-1,2,3,4-tétrahydro-isoquinoléine;
la 2-méthyl-7-(3-pipéridin-1-yl-propoxy)-4-thiophén-3-yl-1,2,3,4-tétrahydro-isoquinoléine;
la 4-(3,4-dichloro-phényl)-7-[3-(4-fluoro-pipéridin-1-yl)-propoxy]-2-méthyl-1,2,3,4-tétrahydro-isoquinoléine;
la 2-méthyl-7-(3-pipéridin-1-yl-propoxy)-4-pyridin-3-yl-1,2,3,4-tétrahydro-isoquinoléine;
la 2-méthyl-7-(3-pipéridin-1-yl-propoxy)-4-(trifluorométhyl-phényl)-1,2,3,4-tétrahydro-isoquinoléine;
la 4-(4-méthoxy-phényl)-2-méthyl-7-(3-pipéridin-1-yl-propoxy)-1,2,3,4-tétrahydro-isoquinoléine (racémique);
la 4-(4-méthoxy-phényl)-2-méthyl-7-(3-pipéridin-1-yl-propoxy)-1,2,3,4-tétrahydro-isoquinoléine (énantiomère 1);
la 4-(4-méthoxy-phényl)-2-méthyl-7-(3-pipéridin-1-yl-propoxy)-1,2,3,4-tétrahydro-isoquinoléine (énantiomère 2);
la 2-tert-butyl-4-(4-méthoxy-phényl)-7-(3-pipéridin-1-yl-propoxy)-1,2,3,4-tétrahydro-isoquinoléine;
la 2-benzyl-4-(4-méthoxy-phényl)-7-(3-pipéridin-1-yl-propoxy)-1,2,3,4-tétrahydro-isoquinoléine;
la 2-éthyl-4-(4-méthoxy-phényl)-7-(3-pipéridin-1-yl-propoxy)-1,2,3,4-tétrahydro-isoquinoléine;
la 4-(4-méthoxy-phényl)-7-(3-pipéridin-1-yl-propoxy)-2-propyl-1,2,3,4-tétrahydro-isoquinoléine;
la 2-isopropyl-4-(4-méthoxy-phényl)-7-(3-pipéridin-1-yl-propoxy)-1,2,3,4-tétrahydro-isoquinoléine;
la 4-(4-méthoxy-phényl)-7-(3-pipéridin-1-yl-propoxy)-1,2,3,4-tétrahydro-isoquinoléine;
le 3-[4-(4-méthoxy-phényl)-7-(3-pipéridin-1-yl-propoxy)-3,4-dihydro-1H-isoquinoléin-2-yl]-propan-1-ol;
le 2-[4-(4-méthoxy-phényl)-7-(3-pipéridin-1-yl-propoxy)-3,4-dihydro-1H-isoquinoléin-2-yl]-éthanol;
la 2-(2-fluoro-éthyl)-4-(4-méthoxy-phényl)-7-(3-pipéridin-1-yl-propoxy)-1,2,3,4-tétrahydro-isoquinoléine;
la 2-cyclopropyl-4-(4-méthoxy-phényl)-7-(3-pipéridin-1-yl-propoxy)-1,2,3,4-tétrahydro-isoquinoléine;
la 4-(4-méthoxy-phényl)-7-(3-morpholin-4-yl-propoxy)-2-(1-phényl-éthyl)-1,2,3,4-tétrahydro-isoquinoléine;
la 4-(4-méthoxy-phényl)-7-(3-morpholin-4-yl-propoxy)-2-(1-phényl-éthyl)-1,2,3,4-tétrahydro-isoquinoléine (diastéréoisomère 1);
la 4-(4-méthoxy-phényl)-7-(3-morpholin-4-yl-propoxy)-2-(1-phényl-éthyl)-1,2,3,4-tétrahydro-isoquinoléine (diastéréoisomère 2);
la 4-(3,4-dichloro-phényl)-2-méthyl-7-(3-pipéridin-1-yl-propoxy)-1,2,3,4-tétrahydro-isoquinoléine;
la 4-(3,4-dichloro-phényl)-2-méthyl-7-(3-morpholin-4-yl-propoxy)-1,2,3,4-tétrahydro-isoquinoléine;
la 4-(4-méthoxy-phényl)-2-méthyl-7-(pipéridin-4-ylméthoxy)-1,2,3,4-tétrahydro-isoquinoléine;
la 4-(3-chloro-4-méthoxy-phényl)-2-méthyl-7-(pipéridin-4-ylméthoxy)-1,2,3,4-tétrahydro-isoquinoléine;
la 2-méthyl-4-(4-méthylsulfanyl-phényl)-7-(pipéridin-4-ylméthoxy)-1,2,3,4-tétrahydro-isoquinoléine;
la 4-(3-fluoro-4-méthoxy-phényl)-2-méthyl-7-(pipéridin-4-ylméthoxy)-1,2,3,4-tétrahydro-isoquinoléine;
la 4-(2-fluoro-4-méthoxy-phényl)-2-méthyl-7-(pipéridin-4-ylméthoxy)-1,2,3,4-tétrahydro-isoquinoléine;
la 7-(1-isopropyl-pipéridin-4-ylméthoxy)-4-(4-méthoxy-phényl)-2-méthyl-1,2,3,4-tétrahydro-isoquinoléine;
la 4-(4-méthoxy-phényl)-2-méthyl-7-(1-méthyl-pipéridin-4-ylméthoxy)-1,2,3,4-tétrahydro-isoquinoléine;
la 4-(4-méthoxy-phényl)-2-méthyl-7-(1-propyl-pipéridin-4-ylméthoxy)-1,2,3,4-tétrahydro-isoquinoléine;
la 7-(1-cyclopentyl-pipéridin-4-ylméthoxy)4-(4-méthoxy-phényl)-2-méthyl-1,2,3,4-tétrahydro-isoquinoléine;
la 7-(1-éthyl-pipéridin-4-ylméthoxy)-4-(4-méthoxy-phényl)-2-méthyl-1,2,3,4-tétrahydro-isoquinoléine;
la 4-(3-chloro-4-méthoxy-phényl)-7-(1-isopropyl-pipéridin-4-ylméthoxy)-2-méthyl-1,2,3,4-tétrahydro-isoquinoléine;
la 4-(3-fluoro-4-méthoxy-phényl)-7-(1-isopropyl-pipéridin-4-ylméthoxy)-2-méthyl-1,2,3,4-tétrahydro-isoquinoléine;
la 4-(2-fluoro-4-méthoxy-phényl)-7-(1-isopropyl-pipéridin-4-ylméthoxy)-2-méthyl-1,2,3,4-tétrahydro-isoquinoléine;
la 7-(1-isopropyl-pipéridin-4-ylméthoxy)-4-(3-méthoxy-phényl)-2-méthyl-1,2,3,4-tétrahydro-isoquinoléine;
la 4-(3-méthoxy-phényl)-2-méthyl-7-(1-méthyl-pipéridin-4-ylméthoxy)-1,2,3,4-tétrahydro-isoquinoléine;
la 4-(3-méthoxy-phényl)-2-méthyl-7-(1-propyl-pipéridin-4-ylméthoxy)-1,2,3,4-tétrahydro-isoquinoléine;
la 7-(1-cyclopentyl-pipéridin-4-ylméthoxy)-4-(3-méthoxy-phényl)-2-méthyl-1,2,3,4-tétrahydro-isoquinoléine;
la 7-(1-éthyl-pipéridin-4-ylméthoxy)-4-(3-méthoxy-phényl)-2-méthyl-1,2,3,4-tétrahydro-isoquinoléine;
la 4-(3-méthoxy-phényl)-2-méthyl-7-(pipéridin-4-yloxy)-1,2,3,4-tétrahydro-isoquinoléine;
la 4-(3-méthoxy-phényl)-2-méthyl-7-(1-méthyl-pipéridin-4-yloxy)-1,2,3,4-tétrahydro-isoquinoléine;
la 7-(1-isopropyl-pipéridin-4-yloxy)-4-(3-méthoxy-phényl)-2-méthyl-1,2,3,4-tétrahydro-isoquinoléine;
la 7-(1-cyclobutyl-pipéridin-4-yloxy)-4-(3-méthoxy-phényl)-2-méthyl-1,2,3,4-tétrahydro-isoquinoléine;
la 7-(1-éthyl-pipéridin-4-yloxy)-4-(3-méthoxy-phényl)-2-méthyl-1,2,3,4-tétrahydro-isoquinoléine;
la 7-(1-cyclopentyl-pipéridin-4-yloxy)-4-(3-méthoxy-phényl)-2-méthyl-1,2,3,4-tétrahydro-isoquinoléine;
la 4-(3-méthoxy-phényl)-2-méthyl-7-(1-propyl-pipéridin-4-yloxy)-1,2,3,4-tétrahydro-isoquinoléine;
la 7-(1-isopropyl-pipéridin-4-ylméthoxy)-2-méthyl-4-(4-méthylsulfanyl-phényl)-1,2,3,4-tétrahydro-isoquinoléine;
la 7-(1-cyclobutyl-pipéridin-4-ylméthoxy)-2-méthyl-4-(4-méthylsulfanyl-phényl)-1,2,3,4-tétrahydro-isoquinoléine;
la 7-(1-éthyl-pipéridin-4-ylméthoxy)-2-méthyl-4-(4-méthylsulfanyl-phényl)-1,2,3,4-tétrahydro-isoquinoléine;
la 4-(3-chloro-4-méthoxy-phényl)-7-(1-cyclobutyl-pipéridin-4-ylméthoxy)-2-méthyl-1,2,3,4-tétrahydro-isoquinoléine;
la 4-(3-chloro-4-méthoxy-phényl)-7-(1-éthyl-pipéridin-4-ylméthoxy)-2-méthyl-1,2,3,4-tétrahydro-isoquinoléine ;.
la 4-(3-chloro-4-méthoxy-phényl)-2-méthyl-7-(1-propyl-pipéridin-4-ylméthoxy)-1,2,3,4-tétrahydro-isoquinoléine;
la 2-méthyl-4-(4-méthylsulfanyl-phényl)-7-(1-propyl-pipéridin-4-ylméthoxy)-1,2,3,4-tétrahydro-isoquinoléine;
la 7-(1-isobutyl-pipéridin)-4-ylméthoxy)-4-(4-méthoxy-phényl)-2-méthyl-1,2,3,4-tétrahydro-isoquinoléine;
la 7-(1-cyclobutyl-pipéridin-4-ylméthoxy)-4-(3-fluoro-4-méthoxy-phényl)-2-méthyl-1,2,3,4-tétrahydro-isoquinoléine;
la 4-(3-fluoro-4-méthoxy-phényl)-2-méthyl-7-(1-propyl-pipéridin-4-ylméthoxy)-1,2,3,4-tétrahydro-isoquinoléine;
la 7-(1-cyclobutyl-pipéridin-4-ylméthoxy)-4-(2-fluoro-4-méthoxy-phényl)-2-méthyl-1,2,3,4-tétrahydro-isoquinoléine;
la 4-(2-fluoro-4-méthoxy-phényl)-2-méthyl-7-(1-propyl-pipéridin-4-ylméthoxy)-1,2,3,4-tétrahydro-isoquinoléine;
la 4-(2-fluoro-4-méthoxy-phényl)-7-(1-isobutyl-pipéridin-4-ylméthoxy)-2-méthyl-1,2,3,4-tétrahydro-isoquinoléine;
la 7-[1-(2-fluoro-éthyl)-pipéridin-4-ylméthoxy]-4-(4-méthoxy-phényl)-2-méthyl-1,2,3,4-tétrahydro-isoquinoléine;
la 7-(1-isopropyl-pipéridin-4-yloxy)-4-(4-méthoxy-phényl)-2-méthyl-1,2,3,4-tétrahydro-isoquinoléine;
la 7-(1-isopropyl-pipéridin-4-yloxy)-2-méthyl-4-(4-méthylsulfanyl-phényl)-1,2,3,4-tétrahydro-isoquinoléine;
la 4-(2-fluoro-4-méthoxy-phényl)-7-(1-isopropyl-pipéridin-4-yloxy)-2-méthyl-1,2,3,4-tétrahydro-isoquinoléine;
la 4-(3-fluoro-4-méthoxy-phényl)-7-(1-isopropyl-pipéridin-4-yloxy)-2-méthyl-1,2,3,4-tétrahydro-isoquinoléine;
la 4-(4-méthoxy-phényl)-2-méthyl-7-[1-(tétrahydro-pyran-4-yl)-pipéridin-4-yloxy]-1,2,3,4-tétrahydro-isoquinoléine;
la 2,2,2-trifluoro-1-{4-[4-(4-méthoxy-phényl)-2-méthyl-1,2,3,4-tétrahydro-isoquinoléin-7-yloxyméthyl]-pipéridin-1-yl}-éthanone;
la 4-(4-méthoxy-phényl)-2-méthyl-7-[1-(2,2,2-trifluoro-éthyl)-pipéridin-4-ylméthoxy]-1,2,3,4-tétrahydro-isoquinoléine;
la 7-[3-(4-fluoro-pipéridin-1-yl)-propoxy]-2-méthyl-4-phényl-1,2,3,4-tétrahydro-isoquinoléine;
la 4-(2-fluoro-phényl)-7-[3-(4-fluoro-pipéridin-1-yl)-propoxy]-2-méthyl-1,2,3,4-tétrahydro-isoquinoléine;
la 7-[3-(4-fluoro-pipéridin-1-yl)-propoxy]-2-méthyl-4-p-tolyl-1,2,3,4-tétrahydro-isoquinoléine;
la 2-benzyl-7-[3-(4-fluoro-pipéridin-1-yl)-propoxy]-4-(4-méthoxy-phényl)-1,2,3,4-tétrahydro-isoquinoléine;
la 7-[3-(4-fluoro-pipéridin-1-yl)-propoxy]-2-méthyl-4-(3-trifluorométhoxy-phényl)-1,2,3,4-tétrahydro-isoquinoléine;
la 7-[3-(3,3-difluoro-pyrrolidin-1-yl)-propoxy]-4-(4-méthoxy-phényl)-2-méthyl-1,2,3,4-tétrahydro-isoquinoléine;
la 4-(4-méthoxy-phényl)-2-méthyl-7-[3-(3S-méthyl-morpholin-4-yl)-propoxy]-1,2,3,4-tétrahydro-isoquinoléine;
la dicyclopropylméthyl-{3-[4-(4-méthoxy-phényl)-2-méthyl-1,2,3,4-tétrahydro-isoquinoléine-7-yloxy]-propyl}-amine;
la 4-(2-chloro-phényl)-7-[3-(4-fluoro-pipéridin-1-yl)-propoxy]-2-méthyl-1,2,3,4-tétrahydro-isoquinoléine;
la 2-méthyl-7-[3-(3S-méthyl-morpholin-4-yl)-propoxy]-4(4-morpholin-4-yl-phényl)-1,2,3,4-tétrahydro-isoquinoléine;
la 7-[3-(4-fluoro-pipéridin-1-yl)-propoxy]-2-méthyl-4-(4-morpholin-4-yl-phényl)-1,2,3,4-tétrahydro-isoquinoléine;
le 4-{2-méthyl-7-[3-(3S-méthyl-morpholin-4-yl)-propoxy]-1,2,3,4-tétrahydro-isoquinoléin-4-yl}-benzonitrile;
le 4-{7-[3-(4-fluoro-pipéridin-1-yl)-propoxy]-2-méthyl-1,2,3,4-tétrahydro-isoquinoléin-4-yl)-benzonitrile;
la 7-[3-(3-benzohydryl-azétidin-1-yl)-propoxy]-4-(4-méthoxy-phényl)-2-méthyl-1,2,3,4-tétrahydro-isoquinoléine;
la 2-méthyl-4-(4-méthylsulfanyl-phényl)-7-(3-morpholin-4-yl-propoxy)-1,2,3,4-tétrahydro-isoquinoléine;
la 4-(2-fluoro-4-méthoxy-phényl)-7-[3-(4-fluoro-pipéridin-1-yl)-propoxy]-2-méthyl-1,2,3,4-tétrahydro-isoquinoléine (racémate);
la 4-(2-fluoro-4-méthoxy-phényl}-7-[3-(4-fluoro-pipéridin-1-yl)-propoxy]-2-méthyl-1,2,3,4-tétrahydro-isoquinoléine (énantiomère 1);
la 4-(2-fluoro-4-méthoxy-phényl)-7-[3-(4-fluoro-pipéridin-1-yl)-propoxy]-2-méthyl-1,2,3,4-tétrahydro-isoquinoléine (énantiomère 2);
la 4-(3-fluoro-4-méthoxy-phényl)-7-[3-(4-fluoro-pipéridin-1-yl)-propoxy]-2-méthyl-1,2,3,4-tétrahydro-isoquinoléine (racémate);
la 4-(3-fluoro-4-méthoxy-phényl)-7-[3-(4-fluoro-pipéridin-1-yl)-propoxy]-2-méthyl-1,2,3,4-tétrahydro-isoquinoléine (énantiomère 1);
la 4-(3-fluoro-4-méthoxy-phényl)-7-[3-(4-fluoro-pipéridin-1-yl)-propoxy]-2-méthyl-1,2,3,4-tétrahydro-isoquinoléine (énantiomère 2);
la 4-(4-éthoxy-phényl)-7-[3-(4-fluoro-pipéridin-1-yl)-propoxy]-2-méthyl-1,2,3,4-tétrahydro-isoquinoléine;
la 2-éthyl-7-[3-(4-fluoro-pipéridin-1-yl)-propoxy]-4-(4-méthoxy-phényl)-1,2,3,4-tétrahydro-isoquinoléine;
la 7-[3-(4-fluoro-pipéridin-1-yl)-propoxy]-4-(4-méthoxy-phényl)-1,2,3,4-tétrahydro-isoquinoléine;
la 4-(4-méthoxy-phényl)-2-méthyl-7-(pipéridin-4-yloxy)-1,2,3,4-tétrahydro-isoquinoléine;
la 2-méthyl-4-(4-méthylsulfanyl-phényl)-7-(pipéridin-4-yloxy)-1,2,3,4-tétrahydro-isoquinoléine;
la 4-(2-fluoro-4-méthoxy-phényl)-2-méthyl-7-(pipéridin-4-yloxy)-1,2,3,4-tétrahydro-isoquinoléine;
la 4-(3-fluoro-4-méthoxy-phényl)-2-méthyl-7-(pipéridin-4-yloxy)-1,2,3,4-tétrahydro-isoquinoléine;
la 7-[1-(2-fluoro-éthyl)-pipéridin-4-yloxy]-4-(4-méthoxy-phényl)-2-méthyl-1,2,3,4-tétrahydro-isoquinoléine;
la 7-[3-(4-fluoro-pipéridin-1-yl)-propoxy]-2-méthyl-4-(4-méthylsulfanyl-phényl)-1,2,3,4-tétrahydro-isoquinoléine (énantiomère 1);
la 7-[3-(4-fluoro-pipéridin-1-yl)-propoxy]-2-méthyl-4-(4-méthylsulfanyl-phényl)-1,2,3,4-tétrahydro-isoquinoléine (énantiomère 2);
la 4-(4-méthoxy-phényl)-2-méthyl-7-[3-(3S-méthyl-morpholin-4-yl)-propoxy]-1,2,3,4-tétrahydro-isoquinoléine (énantiomère 1);
la 4-(4-méthoxy-phényl)-2-méthyl-7-[3-(3S-méthyl-morpholin-4-yl)-propoxy]-1,2,3,4-tétrahydro-isoquinoléine (énantiomère 2);
la 2-méthyl-4-(4-méthylsulfanyl-phényl)-7-(3-morpholin-4-yl-propoxy)-1,2,3,4-tétrahydro-isoquinoléine (énantiomère 1);
la 2-méthyl-4-(4-méthylsulfanyl-phényl)-7-(3-morpholin-4-yl-propoxy)-1,2,3,4-tétrahydro-isoquinoléine (énantiomère 2);
la 7-[3-(4-fluoro-pipéridin-1-yl)-propoxy]-4-(4-méthylsulfanyl-phényl)-1,2,3,4-tétrahydro-isoquinoléine;
la 4-(4-méthylsulfanyl-phényl)-7-(3-morpholin-4-yl-propoxy)-1,2,3,4-tétrahydro-isoquinoléine;
la 7-[3-(3S-méthyl-morpholin-4-yl)-propoxy]-4-(4-méthylsulfanyl-phényl)-1,2,3,4-tétrahydro-isoquinoléine;
la 4-(4-méthoxy-phényl)-7-(3-morpholin-4-yl-propoxy)-1,2,3,4-tétrahydro-isoquinoléine;
la 7-[3-(4-fluoro-pipéridin-1-yl)-propoxy]-4-(4-méthanesulfinyl-phényl)-2-méthyl-1,2,3,4-tétrahydro-isoquinoléine;
la 4-(4-méthanesulfinyl-phényl)-2-méthyl-7-(3-morpholin-4-yl-propoxy)-1,2,3,4-tétrahydro-isoquinoléine;
la 4-(4-méthanesulfinyl-phényl)-2-méthyl-7-(3-morpholin-4-yl-propoxy)-1,2,3,4-tétrahydro-isoquinoléine (énantiomère 1) ;
la 4-(4-méthanesulfonyl-phényl)-2-méthyl-7-(3-morpholin-4-yl-propoxy)-1,2,3,4-tétrahydro-isoquinoléine;
la 7-[3-(4-fluoro-pipéridin-1-yl)-propoxy]-4-(4-méthoxy-phényl)-2,6-diméthyl-1,2,3,4-tétrahydro-isoquinoléine;
la 7-[3-(4-fluoro-pipéridin-1-yl)-propoxy]-4-(4-méthoxy-phényl)-2,8-diméthyl-1,2,3,4-tétrahydro-isoquinoléine;
le 7-[3-(4-fluoro-pipéridin-1-yl)-propoxy]-4-(4-méthoxy-phényl)-2-méthyl-1,2,3,4-tétrahydro-isoquinoléin-6-ol;
le 7-[3-(4-fluoro-pipéridin-1-yl)-propoxy]-4-(4-méthoxy-phényl)-2-méthyl-1,2,3,4-tétrahydro-isoquinoléin-8-ol;
la 2,8-diméthyl-4-(4-méthylsulfanyl-phényl)-7-(3-morpholin-4-yl-propoxy)-1,2,3,4-tétrahydro-isoquinoléine;
la 2,6-diméthyl-4-(4-méthylsulfanyl-phényl)-7-(3-morpholin-4-yl-propoxy)-1,2,3,4-tétrahydro-isoquinoléine;
le 2-méthyl-4-(4-méthylsulfanyl-phényl)-7-(3-morpholin-4-yl-propoxy)-1,2,3,4-tétrahydro-isoquinoléin-8-ol;
le 2-méthyl-4-(4-méthylsulfanyl-phényl)-7-(3-morpholin-4-yl-propoxy)-1,2,3,4-tétrahydro-isoquinoléin-6-ol;
la 8-fluoro-2-méthyl-4-(4-méthylsulfanyl-phényl)-7-(3-morpholin-4-yl-propoxy)-1,2,3,4-tétrahydro-isoquinoléine;
la 6-fluoro-2-méthyl-4-(4-méthylsulfanyl-phényl)-7-(3-morpholin-4-yl-propoxy)-1,2,3,4-tétrahydro-isoquinoléine;
la 7-[1-(2-fluoro-éthyl)-pipéridin-4-ylméthoxy]-2-méthyl-4-(4-méthylsulfanyl-phényl)-1,2,3,4-tétrahydro-isoquinoléine;
la 7-[1-(2-fluoro-éthyl)-pipéridin-4-yloxy]-2-méthyl-4-(4-méthylsulfanyl-phényl)-1,2,3,4-tétrahydro-isoquinoléine;
la 4-(4-méthoxyphényl)-7-[3-(3S-méthyl-morpholin-4-yl)-propoxy]-1,2,3,4-tétrahydro-isoquinoléine;
la 4-(4-méthanesulfinyl-phényl)-2-méthyl]-7-[3-(3S-méthyl-morpholin-4-yl)-propoxy]-1,2,3,4-tétrahydro-isoquinoléine;
la 7-[3-(3,3-difluoro-azétidin-1-yl)-propoxy]-4-(4-méthoxy-phényl)-2-méthyl-1,2,3,4-tétrahydro-isoquinoléine;
le (4-{3-[4-(4-méthoxy-phényl)-2-méthyl-1,2,3,4-tétrahydro-isoquinoléin-7-yloxy]-propyl}-morpholin-3-yl)-méthanol;
le (4-{3-[4-(4-méthoxy-phényl)-2-méthyl-1,2,3,4-tétrahydro-isoquinoléin-7-yloxy]-propyl}-morpholin-3S-yl)-méthanol;
le (4-{3-[4-(4-méthoxy-phényl)-2-méthyl-1,2,3,4-tétrahydro-isoquinoléin-7-yloxy]-propyl}-morpholin-2-yl)-méthanol;
la {3-[2-méthyl-4-(4-méthylsulfanyl-phényl)-1,2,3,4-tétrahydro-isoquinoléin-7-yloxy]-propyl}-(2H-pyrazol-3-yl)-amine;
la 4-(6-bromo-pyridin-3-yl)-7-[3-(4-fluoro-pipéridin-1-yl)-propoxy]-2-méthyl-1,2,3,4-tétrahydro-isoquinoléine;
la 7-[3-(4-fluoro-pipéridin-1-yl)-propoxy]-2-méthyl-4-(6-méthylsulfanyl-pyridin-3-yl)-1,2,3,4-tétrahydro-isoquinoléine;
la (5-{7-[3-(4-fluoro-pipéridin-1-yl)-propoxy]-2-méthyl-1,2,3,4-tétrahydro-isoquinoléin-4-yl}-pyridin-2-yl)-diméthyl-amine;
la 7-[3-(4-fluoro-pipéridin-1-yl)-propoxy]-2-méthyl-4-(6-triméthylsilanyléthynyl-pyridin-3-yl)-1,2,3,4-tétrahydro-isoquinoléine;
la 4-(6-éthynyl-pyridin-3-yl)-7-[3-(4-fluoro-pipéridin-1-yl)-propoxy]-2-méthyl-1,2,3,4-tétrahydro-isoquinoléine;
la 7-[3-(4-fluoro-pipéridin-1-yl)-propoxy]-2-méthyl-4-(6-phénylsulfanyl-pyridin-3-yl)-1,2,3,4-tétrahydro-isoquinoléine;
la 7-[3-(4-fluoro-pipéridin-1-yl)-propoxy]-4-(6-imidazol-1-yl-pyridin-3-yl)-2-méthyl-1,2,3,4-tétrahydro-isoquinoléine;
la 7-[3-(4-fluoro-pipéridin-1-yl)-propoxy]-4-(6-méthoxy-pyridin-3-yl)-2-méthyl-1,2,3,4-tétrahydro-isoquinoléine;
la 7-[3-(4-fluoro-pipéridin-1-yl)-propoxy]-2-méthyl-4-(6-pyrazol-1-yl-pyridin-3-yl)-1,2,3,4-tétrahydro-isoquinoléine;
la 7-[3-(4-fluoro-pipéridin-1-yl)-propoxy]-4-[6-(1H-imidazol-2-ylsulfanyl)-pyridin-3-yl)-2-méthyl-1,2,3,4-tétrahydro-isoquinoléine;
la 7-[3-(4-fluoro-pipéridin-1-yl)-propoxy]-2-méthyl-4-[6-(pyrimidin-2-ylsulfanyl)-pyridin-3-yl]-1,2,3,4-tétrahydro-isoquinoléine;
la 4-[4-méthoxy-phényl)-2-méthyl-7-[3-(4-méthyl-pipérazin-1-yl)-propoxy]-1,2,3,4-tétrahydro-isoquinoléine;
la 7-[3-(4-éthyl-pipérazin-1-yl)-propoxy]-4-(4-méthoxy-phényl)-2-méthyl-1,2,3,4-tétrahydro-isoquinoléine;
la 7-[3-(4-isopropyl-pipérazin-1-yl)-propoxy]-4-(4-méthoxy-phényl)-2-méthyl-1,2,3,4-tétrahydro-isoquinoléine;
la 4-(4-méthoxy-phényl)-2-méthyl-7-[3-(4-thiazol-2-yl-pipérazin-1-yl)-propoxy]-1,2,3,4-tétrahydro-isoquinoléine;
la 4-(4-méthoxy-phényl)-2-méthyl-7-[3-(4-thiophén-2-ylméthyl-pipérazin-1-yl)-propoxy]-1,2,3,4-tétrahydro-isoquinoléine;
le 2-(4-{3-[4-(4-méthoxy-phényl)-2-méthyl-1,2,3,4-tétrahydro-isoquinoléin-7-yloxy]-propyl}-pipérazin-1-yl)-éthanol;
le 2-(4-{3-[4-(4-méthoxy-phényl)-2-méthyl-1,2,3,4-tétrahydro-isoquinoléin-7-yloxy]-propyl}-pipérazin-1-yl)-phénol;
la 4-(4-méthoxy-phényl)-2-méthyl-7-[3-(4-pyridin-4-yl-pipérazin-1-yl)-propoxy]-1,2,3,4-tétrahydro-isoquinoléine;
la 4-(4-méthoxy-phényl)-2-méthyl-7-{3-[4-(4-trifluorométhyl-phényl)-pipérazin-1-yl]-propoxy}-1,2,3,4-tétrahydro-isoquinoléine;
la 7-[3-(4-allyl-pipérazin-1-yl)-propoxy]-4-(4-méthoxy-phényl)-2-méthyl-1,2,3,4-tétrahydro-isoquinoléine;
la 7-[3-(4-[1,3]-dioxolan-2-ylméthyl-pipérazin-1-yl)-propoxy]-4-(4-méthoxy-phényl)-2-méthyl-1,2,3,4-tétrahydro-isoquinoléine;
le 4-(4-{3-[4-(4-méthoxy-phényl)-2-méthyl-1,2,3,4-tétrahydro-isoquinoléin-7-yloxy]-propyl}-pipérazin-1-yl)-benzonitrile;
la 7-{3-[4-(2-méthoxy-éthyl)-pipérazin-1-yl]-propoxy}-4-(4-méthoxy-phényl)-2-méthyl-1,2,3,4-tétrahydro-isoquinoléine;
la 4-(4-méthoxy-phényl)-2-méthyl-7-{3-[4-(tétrahydro-furan-2-ylméthyl)-pipérazin-1-yl]-propoxy}-1,2,3,4-tétrahydro-isoquinoléine;
l'ester de tert-butyle d'acide 4-{3-[4-(4-méthoxy-phényl)-2-méthyl-1,2,3,4-tétrahydro-isoquinoléin-7-yloxy]-propyl}-pipérazine-1-carboxylique;
la 7-[3-(4-cyclopropyl-pipérazin-1-yl)-propoxy]-4-(4-méthoxy-phényl)-2-méthyl-1,2,3,4-tétrahydro-isoquinoléine;
la 4-(4-bromo-phényl)-7-[3-(4-fluoro-pipéridin-1-yl)-propoxy]-2-méthyl-1,2,3,4-tétrahydro-isoquinoléine;
la 4-(4-difluorométhoxy-phényl)-2-méthyl-7-(3-morpholin-4-yl-propoxy)-1,2,3,4-tétrahydro-isoquinoléine;
la 2-méthyl-7-[3-(3S-méthyl-morpholin-4-yl)-propoxy]-4-(4-méthylsulfanyl-phényl)-1,2,3,4-tétrahydro-isoquinoléine;
la 7-[3-(4,4-difluoro-pipéridin-1-yl)-propoxy]-2-méthyl-4-(4-méthylsulfanyl-phényl)-1,2,3,4-tétrahydro-isoquinoléine;
la 7-[3-(4-fluoro-pipéridin-1-yl)-propoxy)-2-méthyl-4-(4-trifluorométhylsulfanyl-phényl)-1,2,3,4-tétrahydro-isoquinoléine;
la 2-méthyl-7-(3-morpholin-4-yl-propoxy)-4-(4-trifluorométhylsulfanyl-phényl)-1,2,3,4-tétrahydro-isoquinoléine;
la 2-méthyl-7-[3-(3S-méthyl-morpholin-4-yl)-propoxy]-4-(4-trifluorométhylsulfanyl-phényl)-1,2,3,4-tétrahydro-isoquinoléine;
la 4-(2-fluoro-4-méthoxy-phényl)-2-méthyl-7-[3-(3S-méthyl-morpholin-4-yl)-propoxy]-1,2,3,4-tétrahydro-isoquinoléine;
la 4-(3-fluoro-4-méthoxy-phényl)-2-méthyl-7-[3-(3S-méthyl-morpholin-4-yl)-propoxy]-1,2,3,4-tétrahydro-isoquinoléine;
la 2-méthyl-7-(3-pipéridin-1-yl-propoxy)-4-(4-trifluorométhoxy-phényl)-1,2,3,4-tétrahydro-isoquinoléine;
la {3-[4-(4-méthoxy-phényl)-2-méthyl-1,2,3,4-tétrahydro-isoquinoléin-7-yloxy]-propyl}-(tétrahydro-pyran-4-yl)-amine;
la cyclopropyl-{3-[4-(4-méthoxy-phényl)-2-méthyl-1,2,3,4-tétrahydro-isoquinoléin-7-yloxy]-propyl}-(1-méthyl-pipéridin-4-yl)-amine;
la (2-méthoxy-éthyl)-{3-[4-(4-méthoxy-phényl)-2-méthyl-1,2,3,4-tétrahydro-isoquinoléin-7-yloxy]-propyl}-amine;
le 3-{3-[4-(4-méthoxy-phényl)-2-méthyl-1,2,3,4-tétrahydro-isoquinoléin-7-yloxy]-propylamino}-propan-1-ol;
l'allyl-{3-[4-(4-méthoxy-phényl)-2-méthyl-1,2,3,4-tétrahydro-isoquinoléin-7-yloxy]-propyl}-amine;
l'isobutyl-{3-[4-(4-méthoxy-phényl)-2-méthyl-1,2,3,4-tétrahydro-isoquinoléin-7-yloxy]-propyl}-amine;
la cyclopropyl-{3-[4-(4-méthoxy-phényl)-2-méthyl-1,2,3,4-tétrahydro-isoquinoléin-7-yloxy]-propyl}-amine;
l'isopropyl-{3-[4-(4-méthoxy-phényl)-2-méthyl-1,2,3,4-tétrahydro-isoquinoléin-7-yloxy]-propyl}-amine;
la {3-[4-(4-méthoxy-phényl)-2-méthyl-1,2,3,4-tétrahydro-isoquinoléin-7-yloxy]-propyl}-propyl-amine;
l'éthyl-{3-[4-(4-méthoxy-phényl)-2-méthyl-1,2,3,4-tétrahydro-isoquinoléin-7-yloxy]-propyl}-amine;
l'isopropyl-{3-[4-(4-méthoxy-phényl)-2-méthyl-1,2,3,4-tétrahydro-isoquinoléin-7-yloxy]-propyl}-méthyl-amine;
la cyclopropyl-{3-[4-(4-méthoxy-phényl)-2-méthyl-1,2,3,4-tétrahydro-isoquinoléin-7-yloxy]-propyl}-méthyl-amine;
la dicyclopropyl-{3-[4-(4-méthoxy-phényl)-2-méthyl-1,2,3,4-tétrahydro-isoquinoléin-7-yloxy]-propyl}-amine;
la 7-(1-isopropyl-azétidin-3-ylméthoxy)-2-méthyl-4-(4-méthylsulfanyl-phényl)-1,2,3,4-tétrahydro-isoquinoléine;
la 7-(1-cyclopropyl-azétidin-3-ylméthoxy)-2-méthyl-4-(4-méthylsulfanyl-phényl)-1,2,3,4-tétrahydro-isoquinoléine;
la 7-(1-cyclobutyl-azétidin-3-ylméthoxy)-2-méthyl-4-(4-méthylsulfanyl-phényl)-1,2,3,4-tétrahydro-isoquinoléine;
la 7-[1-(2-fluoro-éthyl)-azétidin-3-ylméthoxy]-2-méthyl-4-(4-méthylsulfanyl-phényl)-1,2,3,4-tétrahydro-isoquinoléine;
la 4-(4-méthoxy-phényl)-2-méthyl-7-[2-(1-méthyl-pyrrolidin-2-yl)-éthoxy]-1,2,3,4-tétrahydro-isoquinoléine;
la 4-(4-méthoxy-phényl)-2-méthyl-7-[2-(1-méthyl-pyrrolidin-2-yl)-éthoxy]-1,2,3,4-tétrahydro-isoquinoléine (diastéréoisomère 1);
la 4-(4-méthoxy-phényl)-2-méthyl-7-[2-(1-méthyl-pyrrolidin-2-yl)-éthoxy]-1,2,3,4-tétrahydro-isoquinoléine (diastéréoisomère 2);
la 4-(3-méthoxy-phényl)-2-méthyl-7-(pipéridin-4-ylméthoxy)-1,2,3,4-tétrahydro-isoquinoléine;
le {4-[4-(4-méthoxy-phényl)-2-méthyl-1,2,3,4-tétrahydro-isoquinoléin-7-yloxyméthyl]-pipéridin-1-yl}-acétonitrile;
la 2-méthyl-7-(1-méthyl-pipéridin-4-yloxy)-4-(4-méthylsulfanyl-phényl)-1,2,3,4-tétrahydro-isoquinoléine;
la 2-méthyl-4-(4-méthylsulfanyl-phényl)-7-[1-(3,3,3-trifluoro-propyl)-pipéridin-4-yloxy]-1,2,3,4-tétrahydro-isoquinoléine;
le {4-[2-méthyl-4-(4-méthylsulfanyl-phényl)-1,2,3,4-tétrahydro-isoquinoléin-7-yloxy]-pipéridin-1-yl}-acétonitrile;
la 2-méthyl-4-(4-méthylsulfanyl-phényl)-7-[1-(tétrahydro-pyran-4-yl)-pipéridin-4-yloxy]-1,2,3,4-tétrahydro-isoquinoléine;
la 7-(1-cyclopropyl-pipéridin-4-yloxy)-2-méthyl-4-(4-méthylsulfanylphényl)-1,2,3,4-tétrahydro-isoquinoléine;
la 7-(1-cyclobutyl-pipéridin-4-ylméthoxy)-4-(4-méthoxy-phényl)-2-méthyl-1,2,3,4-tétrahydro-isoquinoléine;
la 7-(1-cyclopropyl-pipéridin-4-ylméthoxy)-4-(4-méthoxy-phényl)-2-méthyl-1,2,3,4-tétrahydro-isoquinoléine;
le 1-{4-[4-(4-méthoxy-phényl)-2-méthyl-1,2,3,4-tétrahydro-isoquinoléin-7-yloxyméthyl]-pipéridin-1-yl}-2-méthyl-propan-2-ol;
la 4-(4-méthoxy-phényl)-2-méthyl-7-(4-méthyl-morpholin-2-ylméthoxy)-1,2,3,4 tétrahydro-isoquinoléine;
la 4-(4-méthoxy-phényl)-2-méthyl-7-(morpholin-2S-ylméthoxy)-1,2,3,4-tétrahydro-isoquinoléine;
la 4-(4-méthoxy-phényl)-2-méthyl-7-(morpholin-2R-ylméthoxy)-1,2,3,4-tétrahydro-isoquinoléine;
la 7-(4-isopropyl-morpholin-2-ylméthoxy)-4-(4-méthoxy-phényl)-2-méthyl-1,2,3,4-tétrahydro-isoquinoléine;
la 7-(4-isopropyl-morpholin-2S-ylméthoxy)-4-(4-méthoxy-phényl)-2-méthyl-1,2,3,4-tétrahydro-isoquinoléine;
la 7-(4-isopropyl-morpholin-2R-ylméthoxy)-4-(4-méthoxy-phényl)-2-méthyl-1,2,3,4-tétrahydro-isoquinoléine;
la 7-(4-isopropyl-morpholin-2R-ylméthoxy)-4-(4-méthoxy-phényl)-2-méthyl-1,2,3,4-tétrahydro-isoquinoléine (diastéréoisomère 1);
la 7-(4-isopropyl-morpholin-2R-ylméthoxy)-4-(4-méthoxy-phényl)-2-méthyl-1,2,3,4-tétrahydro-isoquinoléine (diastéréoisomère 2)
la 7-(4-éthyl-morpholin-2-ylméthoxy)-4-(4-méthoxy-phényl)-2-méthyl-1,2,3,4-tétrahydro-isoquinoléine;
la 7-[4-(2-fluoro-éthyl)-morpholin-2-ylméthoxy]-4-(4-méthoxy-phényl)-2-méthyl-1,2,3,4-tétrahydro-isoquinoléine;
la 7-(4-cyclopropyl-morpholin-2-ylméthoxy)-4-(4-méthoxy-phényl)-2-méthyl-1,2,3,4-tétrahydro-isoquinoléine;
la 7-(4-cyclopropyl-morpholin-2S-ylméthoxy)-4-(4-méthoxy-phényl)-2-méthyl-1,2,3,4-tétrahydro-isoquinoléine;
la 7-(4-cyclopropyl-morpholin-2S-ylméthoxy)-4-(4-méthoxy-phényl)-2-méthyl-1,2,3,4-tétrahydro-isoquinoléine (diastéréoisomère 1);
la 7-(4-cyclopropyl-morpholin-2S-ylméthoxy)-4-(4-méthoxy-phényl)-2-méthyl-1,2,3,4-tétrahydro-isoquinoléine (diastéréoisomère 2);
la 7-(4-cyclopropyl-morpholin-2R-ylméthoxy)-4-(4-méthoxy-phényl)-2-méthyl-1,2,3,4-tétrahydro-isoquinoléine;
la 7-(4-cyclopropyl-morpholin-2R-ylméthoxy)-4-(4-méthoxy-phényl)-2-méthyl-1,2,3,4-tétrahydro-isoquinoléine (diastéréoisomère 1);
la 7-(4-cyclopropyl-morpholin-2R-ylméthoxy)-4-(4-méthoxy-phényl)-2-méthyl-1,2,3,4-tétrahydro-isoquinoléine (diastéréoisomère 2);
la 7-(4-isopropyl-morpholin-2-ylméthoxy)-2-méthyl-4-(4-méthylsulfanyl-phényl)-1,2,3,4-tétrahydro-isoquinoléine;
la 7-(4-cyclopropyl-morpholin-2-ylméthoxy)-2-méthyl-4-(4-méthylsulfanyl-phényl)-1,2,3,4-tétrahydro-isoquinoléine;
la 2-méthyl-4-(4-méthylsulfanyl-phényl)-7-(morpholin-2-ylméthoxy)-1,2,3,4-tétrahydro-isoquinoléine;
la 4-(4-méthoxy-phényl)-2,6-diméthyl-7-(3-pipéridin-1-yl-propoxy)-1,2,3,4-tétrahydro-isoquinoléine;
la 4-(4-méthoxy-phényl)-2,6-diméthyl-7-(3-morpholin-4-yl-propoxy)-1,2,3,4-tétrahydro-isoquinoléine;
la 4-(3-fluoro-4-méthoxy-phényl)-2,6-diméthyl-7-(3-morpholin-4-yl-propoxy)-1,2,3,4-tétrahydro-isoquinoléine;
la 4-(3-fluoro-4-méthoxy-phényl)-2,8-diméthyl-7-(3-morpholin-4-yl-propoxy)-1,2,3,4-tétrahydro-isoquinoléine;
la 4-(4-méthoxy-phényl)-2,8-diméthyl-7-(3-morpholin-4-yl-propoxy)-1,2,3,4-tétrahydro-isoquinoléine;
la 2,6-diméthyl-4-(4-méthylsulfanyl-phényl)-7-(3-pipéridin-1-yl-propoxy)-1,2,3,4-tétrahydro-isoquinoléine;
la 7-(4-pipéridin-1-yl-but-1-ynyl)-4-pyridin-3-yl-1,2,3,4-tétrahydro-isoquinoléine;
la 7-(4-pipéridin-1-yl-butyl)-4-pyridin-3-yl-1,2,3,4-tétrahydro-isoquinoléine;
la 2-méthyl-7-(4-pipéridin-1-yl-butyl)-4-pyridin-3-yl-1,2,3,4-tétrahydro-isoquinoléine;
la 7-[4-(4,4-difluoro-pipéridin-1-yl)-but-1-ynyl]-4-(4-méthoxy-phényl)-2-méthyl-1,2,3,4-tétrahydro-isoquinoléine;
la 4-(4-méthoxy-phényl)-2-méthyl-7-(4-pipéridin-1-yl-but-1-ynyl)-1,2,3,4-tétrahydro-isoquinoléine;
la 4-(4-méthoxy-phényl)-2-méthyl-7-(4-morpholin-4-yl-but-1-ynyl)-1,2,3,4-tétrahydro-isoquinoléine;
la 4-(4-méthoxy-phényl)-2-méthyl-7-(4-thiomorpholin-4-yl-but-1-ynyl)-1,2,3,4-tétrahydro-isoquinoléine;
la 7-[4-(4-isopropyl-pipérazin-1-yl)-but-1-ynyl)-4-(4-méthoxy-phényl)-2-méthyl-1,2,3,4-tétrahydro-isoquinoléine;
la 4-(4-fluoro-phényl)-2-méthyl-7-(4-pipéridin-1-yl-but-1-ynyl)-1,2,3,4-tétrahydro-isoquinoléine;
la 7-[4-(4,4-difluoro-pipéridin-1-yl)-butyl]-4-(4-méthoxy-phényl)-2-méthyl-1,2,3,4-tétrahydro-isoquinoléine;
la 4-(4-méthoxy-phényl)-2-méthyl-7-(4-morpholin-4-yl-butyl)-1,2,3,4-tétrahydro-isoquinoléine;
la 4-(4-méthoxy-phényl)-2-méthyl-7-(4-thiomorpholin-4-yl-butyl)-1,2,3,4-tétrahydro-isoquinoléine;.
la 7-[4-(4-isopropyl-pipérazin-1-yl)-butyl]-4-(4-méthoxy-phényl)-2-méthyl-1,2,3,4-tétrahydro-isoquinoléine;
et ses sels.

65. Composition pharmaceutique comprenant un support pharmaceutiquement acceptable et une quantité efficace d'un composé de formule (I): 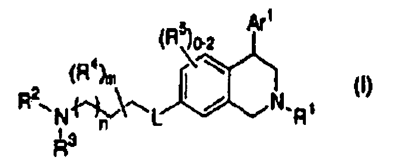 dans laquelle
L est -0- et n vaut 1 ou 2; ou L est -C≡C- ou -CH₂CH₂- et n vaut 0 ou 1;
R¹ est -H; ou est un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyle en C_{3 à 7}, -COOalkyle en C_{1 à 6} ou -COObenzyle, chacun facultativement mono-, di- ou tri-substitué par R^{a};
où R^{a} est choisi parmi un groupe -OH, -Oalkyle en C_{1 à 6}, phényle facultativement substitué par un groupe -Oalkyle en C_{1 à 4} ou halogéno, -CN, -NO₂-, -N(R^{b})R^{c}, -C(O)N(R^{b})R^{c}, -N(R^{b})C(O)R^{b}, -N(R^{b})SO₂alkyle en C_{1 à 6}, -C(O)alkyle en C_{1 à 6}, -S(O)_{0 à 2}-alkyle en C_{1 à 6}, -SO₂N(R^{b})R^{c}, -SCF₃, halogéno, -CF₃, -OCF₃, -COOH et -COOalkyle en C_{1 à 6;}
où R^{b} et R^{c} sont chacun indépendamment -H ou un groupe -alkyle en C_{1 à 6};
R² et R³ sont chacun indépendamment choisis dans le groupe consistant en: -H;
A) un groupe -alkyle en C_{1 à 6}, -alcényle en C_{3 à 6}, -alcynyle en C_{3 à 6}, -cycloalkyle en C_{3 à 7}, -alkyl en C_{1 à 6}-cycloalkyle en C_{3 à 7}, -CH (cycloalkyle en C_{3 à 8})₂, -CH(phényle)₂, benzyle et -C(O)Oalkyle en C_{1 à 4}, où chaque groupe alkyle, cycloalkyle ou benzyle est facultativement substitué par un groupe -OH, -Oalkyle en C_{1 à 4}, -CN, -NH₂, -NH (alkyle en C_{1 à 4}), -N(alkyle en C_{1 à 4})₂, halogéno, -CF₃, -OCF₃, -COOH ou -COOalkyle en C_{1 à 6};
B) un groupe phényle ou pyridyle, facultativement condensé au niveau de deux chaînons de cycle de carbone adjacents à un fragment hydrocarbure à trois ou quatre chaînons pour former un cycle aromatique condensé à cinq ou six chaînons, lequel fragment a un atome de carbone remplacé par >O, >S, >NH, >N(alkyle en C_{1 à 4}) ou >NC(O)Oalkyle en C_{1 à 4}, et lequel fragment a jusqu'à un atome de carbone additionnel facultativement remplacé par -N=;
C) un groupe naphtyle,
D) un cycle hétérocyclique à 4 à 8 chaînons, ledit cycle hétérocyclique ayant un atome de carbone qui est le point d'attache, comportant 1 ou 2 chaînons hétéroatome choisis dans le groupe consistant en >O, >S(O)_{0 à 2}, >NH, >N(alkyle en C_{1 à 4}), et >NC(O)Oalkyle en C_{1 à 4}, et comportant 0 ou 1 double liaison; et
E) un groupe hydrocarbure aromatique monocyclique comportant cinq ou six atomes de cycle, ayant un atome de carbone qui est le point d'attache, ayant un atome de carbone remplacé par >O, >S, >NH, >N(alkyle en C_{1 à 4}) ou >NC(O)Oalkyle en C_{1 à 4}, ayant jusqu'à un atome de carbone additionnel facultativement remplacé par -N=, et facultativement benzocondensé ou pyridocondensé;
où chacun de B) à E) est facultativement mono-, di- ou tri-substitué par un fragment choisi dans le groupe consistant en un groupe -alkyle en C_{1 à 4}, -OH, -alkyl en C_{1 à 4}OH, -Oalkyle en C_{1 à 6}, -CN, -NO₂, -N(R^{d})R^{e}, -C(O)N(R^{d})R^{e}, -N(R^{d})C(O)R^{d}, -N(R^{d})SO₂alkyle en C_{1 à 6}, -C(O)alkyle en C_{1 à 6}, -S(O)_{0 à 2}-alkyle en C_{1 à 6}, -SO₂N(R^{d})R^{e}, -SCF₃, halogéno, -CF₃, -OCF₃, -COOH, -COOalkyle en C_{1 à 4}, -OC(O)N(R^{d})R^{e} et -OC(O)Oalkyle en C_{1 à 6};
où R^{d} et R^{e} sont chacun indépendamment -H ou un groupe -alkyle en C_{1 à 6};
ou, en variante,
R² et R³ peuvent être pris conjointement avec l'atome d'azote auquel ils sont attachés pour former un groupe tétrahydro-pyrimidin-2-ylidényle, ou un cycle hétérocyclique à 4 à 8 chaînons, ledit cycle hétérocyclique ayant 0 ou 1 chaînon hétéroatome additionnel séparé de l'azote d'attache par au moins un chaînon carbone et choisi dans le groupe consistant en >O, >S(O)_{0 à 2}, >NH et >NR^{f}, ayant 0 ou 1 double liaison, ayant 0, 1 ou 2 chaînons carbone séparés de l'atome d'azote d'attache par au moins un chaînon carbone qui est un groupe carbonyle, facultativement benzo ou pyridocondensé, comportant facultativement un chaînon carbone qui forme un pont, et comportant 0 à 5 substituants de chaînon carbone R^{ff},
où R^{f} est choisi dans le groupe consistant en:
i) un groupe -alkyle en C_{1 à 6} facultativement substitué par R^{z}, -alcényle en C_{3 à 6}, -alcynyle en C_{3 à 6}, -C(O)N(R^{g})R^{h}, -C(O)Rⁱ, -S(O)_{0 à 2}-alkyle en C_{1 à 6} et -COOalkyle en C_{1 à 6},
où R² est un groupe fluoro, -CN, -OH, -Oalkyle en C_{1 à 4}, -cycloalkyle en C_{3 à 7} ou -CF_{3;}
où R^{g} et R^{h} sont chacun indépendamment -H ou -alkyle en C_{1 à 6}; et
où Rⁱ est un groupe -alkyle en C_{1 à 6}, -cycloalkyle en C_{3 à 8}, phényle, ou hétérocyclyle aromatique à 5 ou 6 chaînons, où chaque groupe alkyle, cycloalkyle, phényle ou hétérocyclyle est facultativement mono-, di- ou tri-substitué par un groupe -alkyle en C_{1 à 4}, -OH, -Oalkyle en C_{1 à 6}, -CF₃, -CN ou halogéno;
ii) -(CH₂)_{0 à 1}-Cycle A, où Cycle A est un groupe phényle ou un groupe hétérocyclyle aromatique à 5 ou 6 chaînons à liaison carbone, facultativement substitué par R^{aa}_{;}
où R^{aa} est un groupe -OH, -alkyle en C_{1 à 6}, -Oalkyle en C_{1 à 6}, phényle, -CN, -NO₂, -N(R^{g})R^{h}, -C(O)N(R^{g})R^{h}, -(R^{g})C(O)R^{h}, -N(R^{h})SO₂-alkyle en C_{1 à 6}, -C(O)alkyle en C_{1 à 6}, -S(O)_{0 à 2}-alkyle en C_{1 à 6}, -SO₂N(R^{g})R^{h}, -SCF₃, halogéno, -CF₃, -OCF₃, -OCHF₂, -COOH et -COOalkyle en C_{1 à 6}; et
iii) -(CH₂)_{0 à 1}-Cycle B, où Cycle B est un groupe -cycloalkyle en C_{3 à 7} avec un ou deux chaînons de cycle carboné facultativement remplacés par >O ou >NH, et facultativement substitué par un groupe -alkyle en C_{1 à 4}, fluoro, -CN, -OH, -Oalkyle en C_{1 à 4} ou -CF_{3;}
où R^{ff} est choisi dans le groupe consistant en un groupe -alkyle en C_{1 à 6} facultativement mono- ou di-substitué par R^{z}, un groupe -alcényle en C_{2 à 6}, -alcynyle en C_{2 à 6}, -cycloalkyle en C_{3 à 7}, -CH(phényle)₂, halogéno, -OH, -Oalkyle en C_{1 à 6}, -Oalkyl en C_{2 à 3}O-, -CN, -NO₂, -N(R^{g})R^{h}, -C(O)N(R^{g})R^{h}, -N(R^{g})C(O)R^{g}, -N(R^{g})SO₂alkyle en C_{1 à 6}, -C(O)Rⁱ, -S(O)_{0 à 2}-alkyle en C_{1 à 6}, -SO₂N(R^{g})R^{h}, -SCF₃, -CF₃, -OCF₃, -COOH et -COOalkyle en C_{1 à 6};
R⁴ est un groupe -OH, -Oalkyle en C_{1 à 6}, -CF₃, -alkyle en C_{1 à 6} ou halogéno; deux substituants R⁴ peuvent être pris ensemble pour former un groupe méthylène ou éthylène; ou l'un de R⁴ est pris conjointement avec R² pour former un groupe méthylène, éthylène, propylène ou -CH₂CH₂O-, où chacun est facultativement substitué par un groupe -OH, -Oalkyle en C_{1 à 6}, -Salkyle en C_{1 à 6}, -CF₃, -alkyle en C_{1 à 6}, amino ou halogéno;
m vaut 0, 1 ou 2;
R⁵ est choisi dans le groupe consistant en un groupe -alkyle en C_{1 à 6}, -OH, -Oalkyle en C_{1 à 6}, -Salkyle en C_{1 à 6} et halogéno;
Ar¹ est un cycle aryle ou hétéroaryle choisi dans le groupe consistant en:
a) un groupe phényle, facultativement mono-, di- ou tri-substitué par R^{j} et facultativement di-substitué sur des atomes de carbone adjacents par un groupe -Oalkylène en C_{1 à 4}O- facultativement mono- ou di-substitué par un groupe fluoro, -(CH₂)_{2 à 3}-NH-, -(CH₂)_{1 à 2}NH(CH₂)-, -(CH₂)_{2 à 3}-N(alkyle en C_{1 à 4})-, Ou -(CH₂)_{1 à 2}N(alkyl en C_{1 à 4}) (CH₂)-;
où R^{j} est choisi dans le groupe consistant en
1) un groupe -OH, -alkyle en C_{1 à 6}, -Oalkyle en C_{1 à 6} facultativement mono-, di- ou tri-substitué par un groupe halogéno, -alcényle en C_{2 à 6}, -Oalcényle en C_{3 à 6}, -alcynyle en C_{2 à 6} facultativement substitué par un groupe triméthylsilyle, -Oalcynyle en C_{3 à 6}, -cycloalkyle en C_{3 à 6}, -Ocycloalkyle en C_{3 à 6}, -CN, -NO₂, -N(R^{k})R^{j}, -N(R^{k})C(O)R^{l}, -N(R^{k})SO₂alkyle en C_{1 à 6}, -C(O)alkyle en C_{1 à 6}, -S(O)_{0 à 2}-alkyle en C_{1 à 6}, -C(O)N(R^{m})Rⁿ, -SO₂N(R^{m})Rⁿ, -SCF₃, halogéno, -CF₃, -COOH, -COOalkyle en C_{1 à 6} et -COOcycloalkyle en C_{3 à 7;}
où R^{k} et R^{l} sont chacun indépendamment -H ou un groupe -alkyle en C_{1 à 6};
où R^{m} et Rⁿ sont chacun indépendamment -H ou un groupe -alkyle en C_{1 à 6}, ou R^{m} et Rⁿ pris conjointement avec leur atome d'azote d'attache forment un cycle hétérocyclique à 4 à 8 chaînons comportant 1 ou 2 chaînons hétéroatome choisis parmi >O, >S(O)_{0 à 2}, >NH et >Nalkyle en C_{1 à 6}, comportant 0 ou 1 double liaison, comportant 0 ou 1 chaînon carbonyle;
2) -G-Ar², où G est une liaison, -O- ou -S-, et Ar² est un groupe phényle ou est un groupe hydrocarbure aromatique monocyclique comportant cinq ou six atomes de cycle, comportant un atome de carbone remplacé par >O, >S, >NH, ou >N(alkyle en C_{1 à 4}), comportant jusqu'à un atome de carbone additionnel facultativement remplacé par -N=, chacun facultativement mono-, di- ou tri-substitué par R^{p};
où R^{p} est un substituant indépendamment choisi dans le groupe consistant en: un groupe -OH, -alkyle en C_{1 à 6}, -Oalkyle en C_{1 à 6}, phényle, -CN, -NO₂, -N(R^{q})R^{r}, -C(O)N(R^{q})R^{r}, -N(R^{q})C(O)R^{r}, -N(R^{q})SO₂alkyle en C_{1 à 6}, -C(O)alkyle en C_{1 à 6}, -S(O)_{0 à 2}-alkyle en C_{1 à 6}, -SO₂N(R^{q})R^{r}, -SCF₃, halogéno, -CF₃, -OCF₃, -OCHF₂, -COOH et -COOalkyle en C_{2 à 6};
où R^{q} et R^{r} sont chacun indépendamment choisis parmi -H, un groupe -alkyle en C_{1 à 6} et -alcényle en C_{1 à 6}; et
3) un cycle hétérocyclique saturé ou partiellement saturé à 4 à 8 chaînons, comportant 1 ou 2 chaînons hétéroatome choisis parmi >O, >S(O)_{0 à 2}, >NH et >Nalkyle en C_{1 à 6}, comportant 0 ou 1 chaînon carbonyle, ledit cycle étant facultativement mono-, di- ou tri-substitué par R^{p};
b) un groupe phényle ou pyridyle condensé au niveau de deux chaînons de cycle de carbone adjacents à un fragment hydrocarbure à trois chaînons pour former un cycle aromatique condensé à cinq chaînons, lequel fragment a un atome de carbone remplacé par >O, >S, >NH ou >N(alkyle en C_{1 à 4}) et lequel fragment a jusqu'à un atome de carbone additionnel facultativement remplacé par -N=, les cycles condensés étant facultativement mono-, di- ou tri-substitués par R^{t};
où R^{t} est un substituant indépendamment choisi dans le groupe consistant en: un groupe -OH, -alkyle en C_{1 à 6}, -Oalkyle en C_{1 à 6}, phényle, -CN, -NO₂, -N(R^{u})R^{v}, -C(O)N(R^{u})R^{v}, -N(R^{u})C(O)R^{v}, -N(R^{u})SO₂alkyle en C_{1 à 6}, -C(O)alkyle en C_{1 à 6}, -S(O)_{0 à 2}-alkyle en C_{1 à 6}, -SO₂N(R^{u})R^{v}, -SCF₃, halogéno, -CF₃, -OCF₃, -OCHF₂, -COOH et -COOalkyle en C_{1 à 6};
où R^{u} et R^{v} sont chacun indépendamment -H ou un groupe -alkyle en C_{1 à 6};
c) un groupe phényle condensé au niveau de deux chaînons de cycle adjacents à un fragment hydrocarbure à quatre chaînons pour former un cycle aromatique condensé à six chaînons, lequel fragment a un ou deux atomes de carbone remplacés par -N=, les cycles condensés étant facultativement mono-, di- ou tri-substitués par R^{t};
d) un groupe naphtyle, facultativement mono-, di- ou tri-substitué par _{R}^{t};
e) un groupe hydrocarbure aromatique monocyclique comportant cinq atomes de cycle, comportant un atome de carbone qui est le point d'attache, ayant un atome de carbone remplacé par >O, >S, >NH ou >N(alkyle en C_{1 à 4}), ayant jusqu'à un atome de carbone additionnel facultativement remplacé par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono-, di- ou tri-substitué par R^{t}; et
f) un groupe hydrocarbure aromatique monocyclique comportant six atomes de cycle, ayant un atome de carbone qui est le point d'attache, ayant un ou deux atomes de carbone remplacés par -N=, facultativement mono- ou di-substitué par R^{j} et facultativement benzocondensé ou pyridocondensé au niveau de deux atomes de carbone adjacents, où le fragment benzocondensé ou pyridocondensé est facultativement mono- ou di-substitué par R^{t};
et ses énantiomères, diastéréoisomères, hydrates, solvates, et sels, esters et amides pharmaceutiquement acceptables, à condition que:
lorsque n vaut 1, L est -O-, Ar est un groupe phényle non substitué et R² et R³ sont tous deux un groupe méthyle, alors R⁴ ne soit pas -OH.
